# EUROPEAN PATENT APPLICATION

(11) **EP 3 696 189 A1**
(43) Date of publication of application: **19.08.2020**
(21) Application number: 19157257.7
(22) Date of filing: 14.02.2019
(51) Int. Cl.: C07K 14/435, C12N 9/00

(54) **MEANS AND METHODS FOR PREPARING ENGINEERED TARGET PROTEINS BY GENETIC CODE EXPANSION IN A TARGET PROTEIN SELECTIVE MANNER**

(71) Applicant: European Molecular Biology Laboratory, 69117 Heidelberg (DE)
(72) Inventor: The designation of the inventor has not yet been filed
(74) Representative: Reitstötter Kinzebach

(57) **Abstract**

The present invention is concerned with orthogonal translation systems which allow for the site-specific introduction of non-canonical amino acid residues into a target protein (POI) in a POI-mRNA-selective manner. Specifically, the present invention relates to assembler fusion proteins which bring an RNA-targeting polypeptide (RNA-TP) segment and an orthogonal aminoacyl tRNA synthetase (O-RS) segment into spatial proximity of one another, either by direct linkage in RNA-TP/O-RS fusion proteins, or though the action of "assemblers" fused to each of these segments in assembler fusion proteins (AFPs). The invention also relates to AFP combinations and nucleic acid molecules comprising a POI-encoding sequence together with a targeting nucleotide sequence that is able to interact with an RNA-TP. The invention further relates to nucleic acid molecules, expression cassettes and expression vectors encoding said RNA-TP/O-RS fusion proteins or AFPs, cells comprising same, as well as methods and kits for translationally preparing POIs.

## Description

### FIELD OF THE INVENTION

The present invention is concerned with orthogonal translation systems which allow for the site-specific introduction of non-canonical amino acid (ncAA) residues into a polypeptide of interest (POI) in a POI-mRNA-selective manner. Specifically, the present invention relates to fusion proteins which bring an RNA-targeting polypeptide (RNA-TP) segment and an orthogonal aminoacyl tRNA synthetase (O-RS) segment into spatial proximity of one another. This is achieved by combining an RNA-TP segment and an O-RS segment in one and the same fusion protein (RNA-TP/O-RS fusion protein), or by the action of one or more polypeptide segments which act as "assemblers" (APs) and facilitate a local enrichment of assembler fusion proteins (AFPs) comprising the one or more APs together with an RNA-TP segment or an O-RS segment, thus bringing said RNA-TP and O-RS segments into close proximity of one another. The invention also relates to AFP combinations and nucleic acid molecules comprising a POI-encoding sequence together with a targeting nucleotide sequence (TN) that is able to interact with an RNA-TP. The invention further relates to nucleic acid molecules, expression cassettes and expression vectors encoding said RNA-TP/O-RS fusion proteins or AFPs, cells comprising same, as well as methods and kits for translationally preparing POIs.

### BACKGROUND OF THE INVENTION

The ability to engineer orthogonal (i.e. non-crossreactive) translation systems site-specifically into living cells enables the introduction of new functionality into proteins. However, this is a herculean task, as translation is a complex multistep process in which at least 20 different aminoacylated tRNAs, their cognate aminoacyl tRNA synthetases (RS), ribosomes and diverse other factors work in concert to synthesize a polypeptide chain from the RNA transcript. An ideal orthogonal system would show no cross-reactivity with factors of the host machinery, minimizing its impact on the housekeeping translational activity and normal physiology of the cell.

Towards this goal, genetic code expansion (GCE) is a method that enables reprogramming of a specific codon. With GCE, an orthogonal (suppressor) RS (O-RS) can aminoacylate its cognate suppressor tRNA with non-canonical amino acids (ncAAs). These ncAAs are typically custom designed and harbor chemical functionalities that can, for example, enable protein function to be photocontrolled, encode posttranslational modifications or allow the introduction of fluorescent labels for microscopy studies using click chemistry. To introduce ncAAs site-specifically into a polypeptide of interest (POI), the anticodon loop of the tRNA is chosen to decode and thus suppress one of the stop codons (see, e.g., Liu et al., Annu Rev Biochem 2010, 79:413-444; Lemke, ChemBioChem 2014, 15:1691-1694; Chin, Nature 2017, 550;53-60). To minimize the impact on the host cell machinery, the Amber stop codon (corresponding tRNA_{CUA}) is often utilized, owing to its particularly low abundance in E.COLI, to terminate endogenous proteins (<10%). Nevertheless, in principle any Amber codon in the genome can be suppressed, potentially leading to unwanted background suppression of non-targeted host proteins. If ncAA-modified proteins are recombinantly produced for *in vitro* applications, this background incorporation might be tolerable as long as the yields of purified full-length protein are acceptable. However, the challenge is different if the host is considered more than just a bioreactor vessel that can be sacrificed for its protein. In order to study the function of a host-cell POI *in situ,* the physiological condition of that host cell is an important factor. In that context, minimization of background incorporation of the ncAA is particularly required to ensure well-controlled experiments.

At least three elegant approaches have been developed to enable orthogonal translation in *E.coli,* that is, to decode a specific codon only for the RNA of the POI and not the entire genome. i) Orthogonal ribosomes recognizing a unique Shine-Dalgarno sequence have been developed to decode quadruplet codons, which are then used instead of stop codons to site-specifically encode an ncAA into a POI. (See, e.g., Heumann et al., Nature 2010, 464:441:444; Orelle et al., Nature 2015, 524:119-124; Fried et al., Angew Chem 2015, 54:12791-12794.) ii) Recently, genome engineering has advanced to the stage that *E.coli* strains can be depleted of selected native codons, providing a genetically clean (e.g. Amber codon free) host background for selective decoding of specific codons only in the POI. (See, e.g., Isaacs et al., Science 2011, 333:348-353; Lajoie et al., Science 2013, 342:357-360; Ostrov et al., Science 2016, 353:819-822; Wang et al., Nature 2016, 539:59-64.) iii) Unique non-canonical codons have been designed using an artificial base pair encoded only in the coding sequence of the POI. This lowers the risk of nonspecific decoding in other parts of the genome (see Zhang et al., Nature 2017, 551:644-647). However, due to genome complexity, it is not straightforward to transfer these orthogonal translation approaches to eukaryotes (see, e.g., Thompson et al., ACS Chem Biol 2018, 13:313-325), in which additionally the Amber codon is highly abundant (20% in mammalian cells).

There is therefore a high demand for strategies for POI-selective orthogonal translation which are versatile and work not only for well-characterized prokaryotes such as *E.coli,* which are relatively easy to handle and manipulate, but are also applicable to eukaryotic cells. It was therefore an object of the present invention to address this challenge.

### SUMMARY OF THE INVENTION

The inventors found that orthogonal translation systems (OT systems) which are able to selectively translate the mRNA of a POI can be created by generating spatial proximity between the mRNA of the POI and the O-RSs which allow for translationally introducing the ncAA residues into the growing polypeptide chain of the POI. The inventors demonstrated for a variety of POIs, including membrane proteins, that their OT systems allow for site-specifically introducing ncAA residues into a POI in a mammalian cell with selectivity for the mRNA of the POI compared to other mRNAs in the cytoplasm that contain the same stop codon (that is used as selector codon for encoding the ncAA residue of the POI).

In the orthogonal translation systems of the invention, the spatial proximity is achieved by including a targeting sequence (TN) in the mRNA of the POI that can selectively interact with an RNA-targeting polypeptide (RNA-TP), and linking the O-RS with such RNA-TP. Said linkage can be in a fusion protein comprising both, the O-RS and the RNA-TP (RNA-TP/O-RS fusion protein) or can be achieved by the action of one or more polypeptide segments which act as "assemblers" (APs) in facilitating a local enrichment of assembler fusion proteins (AFPs) comprising the one or more APs and an RNA-TP segment or an O-RS segment, thus bringing said RNA-TP and O-RS segments into close proximity of one another. The local enrichment of the AFPs allows for the formation of assemblies (OT assemblies, also termed "OT organelles" herein) which can act as artificial orthogonally translating organelles. The inventors demonstrated that different types of APs can be used, including APs which drive local enrichment at (previously existing) intracellular structures (such as, e.g., microtubules or the cytoplasmic side of membranes such as the cell membrane or the nuclear membrane), termed intracellular targeting polypeptide (IC-TP) segments, as well as APs which generate high local AFP concentrations by self-association in the cytoplasm (in particular by phase separation) termed phase separation polypeptide (PSP) segments herein. The inventors further found that AFPs combining different AP types are particularly useful.

Thus, in a first aspect, the present invention relates to an assembler fusion protein (AFP) comprising:
(a) at least one first polypeptide segment acting as assembler (AP) that is selected from:
   (a1) a polypeptide segment derived from an intracellular targeting polypeptide (IC-TP segment), wherein said intracellular targeting polypeptide targets, and thus becomes locally enriched at, an intracellular structural element within or directly adjacent to the cytoplasm; and
   (a2) a polypeptide segment derived from a phase separation polypeptide (PSP segment), wherein said phase separation polypeptide has the ability to undergo self-association in the cytoplasm of a cell so as to create sites of high local concentration in the cytoplasm, and
(b) at least one second polypeptide segment acting as an effector (EP) that is selected from:
   b1) an RNA-targeting polypeptide (RNA-TP) segment, and
   b2) an orthogonal aminoacyl tRNA synthetase (O-RS) segment;
   wherein said polypeptide segments are functionally linked in said AFP.

In a second aspect, the present invention relates to an assembler fusion protein (AFP) combination comprising at least two AFPs of the present invention as described herein. Preferably, the AFP combination comprises at least one AFP comprising a RNA-TP segment and at least one AFP comprising an O-RS segment.

In a third aspect, the present invention relates to a fusion protein (RNA-TP/O-RS fusion protein) comprising:
(i) at least one RNA-targeting polypeptide (RNA-TP) segment; and
(ii) at least one orthogonal aminoacyl tRNA synthetase (O-RS) segment,
wherein said polypeptide segments are functionally linked in said RNA-TP/O-RS fusion protein.

In a further aspect, the present invention provides a nucleic acid molecule, or a combination of two or more nucleic acid molecules, comprising:
(i) a nucleotide sequence that encodes at least one RNA-TP/O-RS fusion protein of the present invention as described herein, or
(ii) a nucleic acid sequence complementary to (i), or
(iii) both of (i) and (ii).

In a further aspect, the present invention provides a nucleic acid molecule, or a combination of two or more nucleic acid molecules, comprising:
(i) a nucleotide sequence that encodes at least one AFP of the present invention as described herein, or
(ii) a nucleic acid sequence complementary to (i), or
(iii) both of (i) and (ii).

In a further aspect, the present invention provides a nucleic acid molecule, or a combination of two or more nucleic acid molecules, comprising:
(i) a nucleotide sequence that encodes at least one AFP combination of the present invention as described herein, or
(ii) a nucleic acid sequence complementary to (i), or
(iii) both of (i) and (ii).

In further aspects, the present invention provides an expression cassette comprising the nucleotide sequence of the nucleic acid molecule, or the combination of nucleic acid molecules, of the present invention as described herein.

In particular embodiments, the present invention provides an expression cassette comprising:
(i) a nucleotide sequence that encodes at least one RNA-TP/O-RS fusion protein of the present invention as described herein, or
(ii) a nucleic acid sequence complementary to (i), or
(iii) both of (i) and (ii).

In further particular embodiments, the present invention provides an expression cassette comprising:
(i) a nucleotide sequence that encodes at least one AFP of the present invention as described herein, or
(ii) a nucleic acid sequence complementary to (i), or
(iii) both of (i) and (ii).

In further particular embodiments, the present invention provides an expression cassette comprising:
(i) a nucleotide sequence that encodes at least one AFP combination of the present invention as described herein, or
(ii) a nucleic acid sequence complementary to (i), or
(iii) both of (i) and (ii).

In further aspects, the present invention provides an expression vector comprising at least one expression cassette of the present invention as described herein.

In further aspects, the present invention provides a cell comprising at least one nucleic acid molecule, or combination of nucleic acid molecules, of the present invention as described herein. In particular embodiments, the cell comprises at least one expression cassette or at least one expression vector of the present invention as described herein.

In a further aspect, the present invention relates to a method for preparing a polypeptide of interest (POI) comprising in its amino acid sequence one or more non-canonical amino acid (ncAA) residues. Said method comprises expressing the POI in a cell of the present invention in the presence of said one or more ncAAs, wherein the cell comprises:
(i) at least one AFP comprising a RNA-TP segment and at least one AFP comprising an O-RS segment as described herein;
(ii) a POI-encoding nucleotide sequence (CS^{POI}) wherein said one or more ncAA residues of the POI are encoded by selector codon(s),
(iii) a targeting nucleotide sequence (TN) that is functionally linked to the CS^{POI} and is able to interact with an RNA-TP segment of at least one of the AFPs in the cell;
(iv) one or more orthogonal tRNA^{ncAA} (O-tRNA^{ncAA}) molecules which carry the anticodon(s) complementary to the selector codon(s) of the CS^{POI}, and wherein said O-tRNA^{ncAA} molecules together with one or more O-RS segments of the AFPs in the cell form one or more orthogonal O-RS/O-tRNA^{ncAA} pairs which allow for the introduction of said one or more ncAA residues into the amino acid sequence of the POI;
and wherein the method optionally further comprises recovering the expressed POI. Said at least one AFP comprising a RNA-TP segment and said at least one AFP comprising an O-RS segment recited in (i) can be one and the same type of AFP, i.e. an AFP comprising both a RNA-TP segment and an O-RS segment. Alternatively, said at least one AFP comprising a RNA-TP segment and said at least one AFP comprising an O-RS segment recited in (i) can be different AFPs.

In a further aspect, the present invention relates to a method for preparing a polypeptide of interest (POI) comprising in its amino acid sequence one or more non-canonical amino acid (ncAA) residues. Said method comprises expressing the POI in a cell of the present invention in the presence of said one or more ncAAs, wherein the cell comprises:
(i) RNA-TP/O-RS fusion proteins of the present invention as described herein;
(ii) a POI-encoding nucleotide sequence (CS^{POI}) wherein said one or more ncAA residues of the POI are encoded by selector codon(s),
(iii) a targeting nucleotide sequence (TN) that is functionally linked to the CS^{POI} and is able to interact with an RNA-TP segment of at least one of the RNA-TP/O-RS fusion proteins in the cell;
(iv) one or more orthogonal tRNA^{ncAA} (O-tRNA^{ncAA}) molecules which carry the anticodon(s) complementary to the selector codon(s) of the CS^{POI}, and wherein said O-tRNA^{ncAA} molecules together with one or more O-RS segments of the RNA-TP/O-RS fusion proteins in the cell form one or more orthogonal O-RS/O-tRNA^{ncAA} pairs which allow for the introduction of said one or more ncAA residues into the amino acid sequence of the POI;
and wherein the method optionally further comprises recovering the expressed POI.

In a further aspect, the present invention relates to a method for preparing a polypeptide of interest (POI) comprising in its amino acid sequence one or more non-canonical amino acid (ncAA) residues. Said method comprises the steps of:
(a) expressing in a cell one or more AFPs comprising at least one RNA-TP segment and one or more AFPs comprising at least one O-RS segment as described herein;
(b) expressing in said cell one or more orthogonal tRNA^{ncAA} (O-tRNA^{ncAA}) molecules, wherein
   - said orthogonal tRNA^{ncAA} molecules and one or more of the O-RS segments of the AFPs in the cell form one or more orthogonal aminoacyl tRNA synthetase/tRNA^{ncAA} (O-RS/O-tRNA^{ncAA}) pairs,
   - said O-RS/O-tRNA^{ncAA} pairs allow for introducing said one or more ncAA residues into the amino acid sequence of said POI,
   wherein steps (a) and (b) can be concomitantly or sequentially in any order;
(c) then, expressing said POI in said cell in the presence of said one or more ncAAs, wherein
   - the POI-encoding nucleotide sequence (CS^{POI}) comprises one or more selector codons encoding said one or more ncAA residues,
   - said selector codons match the anticodons of said one or more O-tRNA^{ncAA} molecules;
   - said CS^{POI} is functionally linked to a targeting nucleotide sequence (TN), thus forming a CS^{POI}/TN fusion sequence,
   - said CS^{POI}/TN fusion sequence is able to interact, via its TN, with an RNA-TP segment of at least one of the AFPs in the cell;
   and
(d) optionally recovering the expressed POI.

In a further aspect, the present invention relates to a method for preparing a polypeptide of interest (POI) comprising in its amino acid sequence one or more non-canonical amino acid (ncAA) residues. Said method comprises the steps of:
(a) expressing in a cell RNA-TP/O-RS fusion proteins of the present invention as described herein;
(b) expressing in said cell one or more orthogonal tRNA^{ncAA} (O-tRNA^{ncAA}) molecules, wherein
   - said orthogonal tRNA^{ncAA} molecules and one or more of the O-RS segments of the RNA-TP/O-RS fusion proteins in the cell form one or more orthogonal aminoacyl tRNA synthetase/tRNA^{ncAA} (O-RS/O-tRNA^{ncAA}) pairs,
   - said O-RS/O-tRNA^{ncAA} pairs allow for introducing said one or more ncAA residues into the amino acid sequence of said POI,
   wherein steps (a) and (b) can be concomitantly or sequentially in any order;
(c) then, expressing said POI in said cell in the presence of said one or more ncAAs, wherein
   - the POI-encoding nucleotide sequence (CS^{POI}) comprises one or more selector codons encoding said one or more ncAA residues,
   - said selector codons match the anticodons of said one or more O-tRNA^{ncAA} molecules;
   - said CS^{POI} is functionally linked to a targeting nucleotide sequence (TN), thus forming a CS^{POI}/TN fusion sequence,
   - said CS^{POI}/TN fusion sequence is able to interact, via its TN, with an RNA-TP segment of at least one of the RNA-TP/O-RS fusion proteins in the cell;
   and
(d) optionally recovering the expressed POI.

In a further aspect, the present invention relates to a nucleic acid molecule comprising:
(i) a nucleotide sequence (CS^{POI}) that encodes a polypeptide of interest (POI), said POI comprising one or more, identical or different, non-canonical amino acid (ncAA) residues which are encoded in the CS^{POI} by selector codons, and
(ii) a targeting nucleotide sequence (TN), wherein an RNA molecule comprising said TN is able to interact via said TN with an RNA-targeting polypeptide (RNA-TP).

In a further aspect, the present invention relates to a kit for preparing a polypeptide of interest (POI) having at least one non-canonical amino acid (ncAA) residue, the kit comprising:
- at least one ncAA, or salt thereof, corresponding to the at least one ncAA residue of the POI, and
- at least one expression vector of the present invention as described herein.

Said expression vector comprises at least one expression cassette comprising:
(i) a nucleotide sequence that encodes at least one RNA-TP/O-RS fusion protein of the present invention, at least one AFP of the present invention, or at least one AFP combination of the present invention, or
(ii) a nucleic acid sequence complementary to (i), or
(iii) both of (i) and (ii).

### BRIEF DESCRIPTION OF THE FIGURES

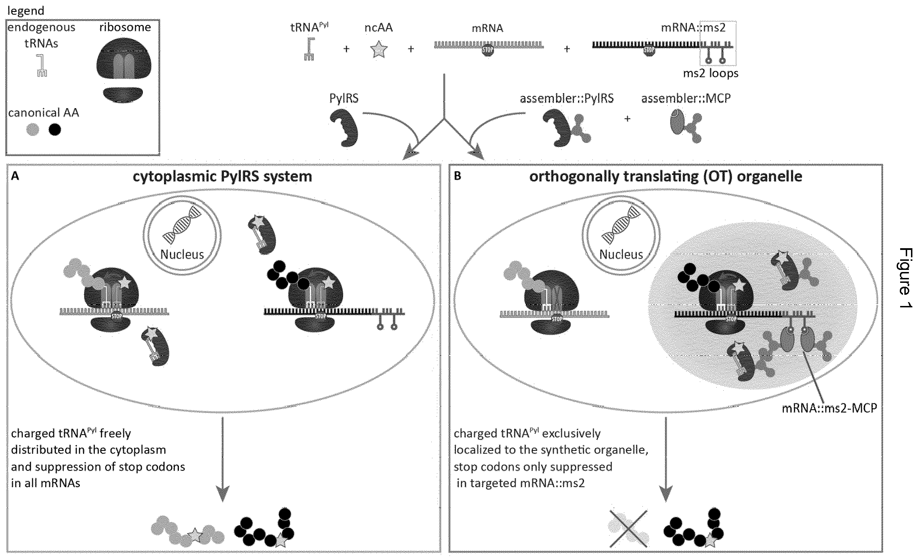

Figure 1 shows a schematic representation of the spatial separation of the components which allow for orthogonal translation so as to decode a specific stop codon in a uniquely tagged mRNA. (A) Expression of the synthetase PylRS leads to aminoacylation of its cognate stop codon suppressor tRNA^{Pyl} with a custom designed ncAA. This leads to site-specific ncAA incorporation whenever the respective stop codon occurs in mRNA of the POI. Given that many endogenous mRNAs terminate on the same stop codon, utilizing this approach in the cytoplasm potentially leads to misincorporation of the ncAA into unwanted proteins (left box). (B) To avoid this, the mRNA encoding the POI and the orthogonal aminoacyl-tRNA synthetase (e.g., PylRS) can be brought into close proximity to one antoher through the use of an RNA-targeting polypeptide segment (e.g., MCP) and assemblers" which allows for spatial enrichment of all components so as to create an OT assembly ("OT organelle"), including the mRNA encoding the POI, the orthogonal aminoacyl-tRNA synthetase, the tRNA, and ribosomes (right box). Here aminoacylated tRNA^{Pyl} is particularly available in direct proximity of the OT organelle, so that particularly here stop codon suppression (of the POI mRNA) can occur. This leads to a selective suppression of stop codons (and thus expression) of the POI mRNA over corresponding stop codons in mRNAs that are not targeted to the OT assembly. While in (A) GCE occurs stop codon-specific, in (B) it should occur stop codon-specific and mRNA-specific.

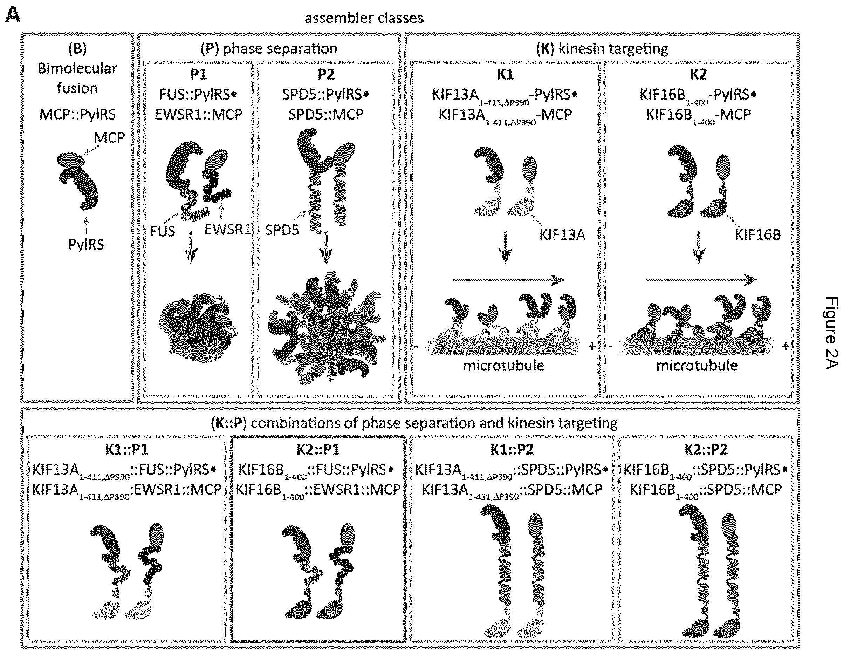

Figure 2A shows a schematic representation of different assembler classes. **B** = bimolecular MCP::PylRS fusion, **P1** = fusions to FUS and EWSR1, **P2** = SPD5, **K1** = truncation of kinesin KIF13A (KIF13A_{1-411,ΔP390}), **K2** = truncation of kinesin KIF16B (KIF16B₁₋₄₀₀) and combinations thereof **(K1::P1, K1::P2, K2::P1, K2::P2).**

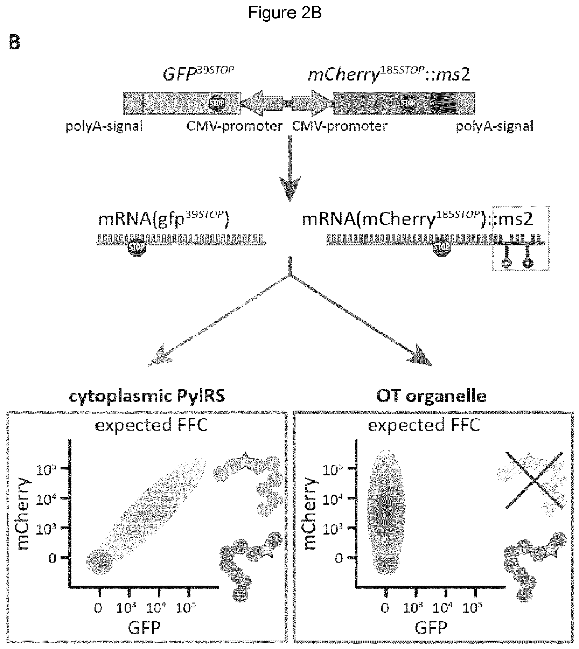

Figure 2B shows a schematic representation of the dual-color reporter. mRNAs encoding the fluorescent proteins GFP and mCherry, containing stop codons at permissive sites, are expressed from one plasmid, each with its own CMV promoter, ensuring a constant ratio of mRNA throughout each experiment. The mRNA of the mCherry reporter is tagged with two MS2 RNA stem-loops ("ms2", also referred to as MS2-tag herein), mRNA(mCherry)::ms2. In the presence of ncAA and tRNA^{Pyl}, in the case of cytoplasmic PylRS, both GFP^{39*STOP*} and mCherry^{185*STOP*} are produced, leading to a diagonal in fluorescence flow cytometry (FFC) analysis (left box). However, under the same conditions, orthogonal translation in OT organelles enables selective stop codon suppression of mRNA(mCherry)::ms2, resulting in an mCherry-positive and GFP-negative population (drawn schematically as a vertical population in the right box). In both schemes, non-transfected HEK293T cells are represented by a gray circle at the bottom.

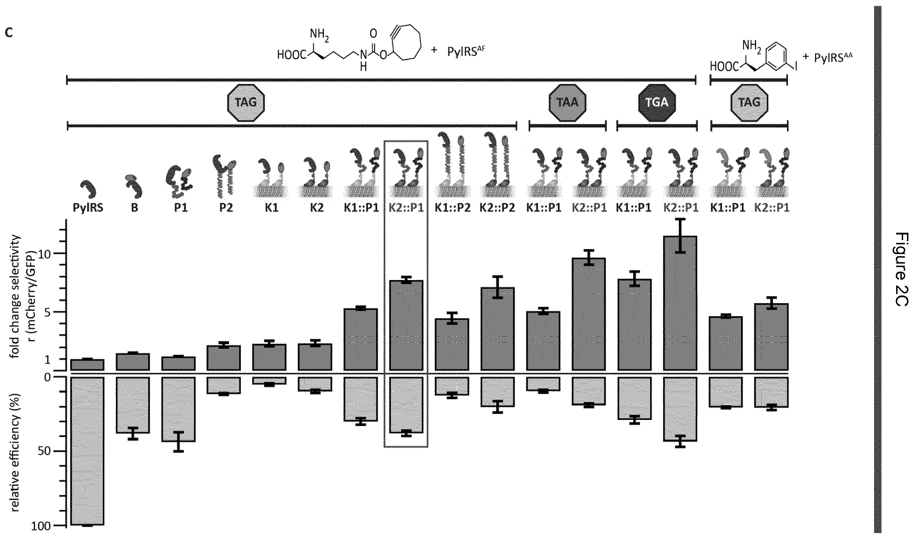

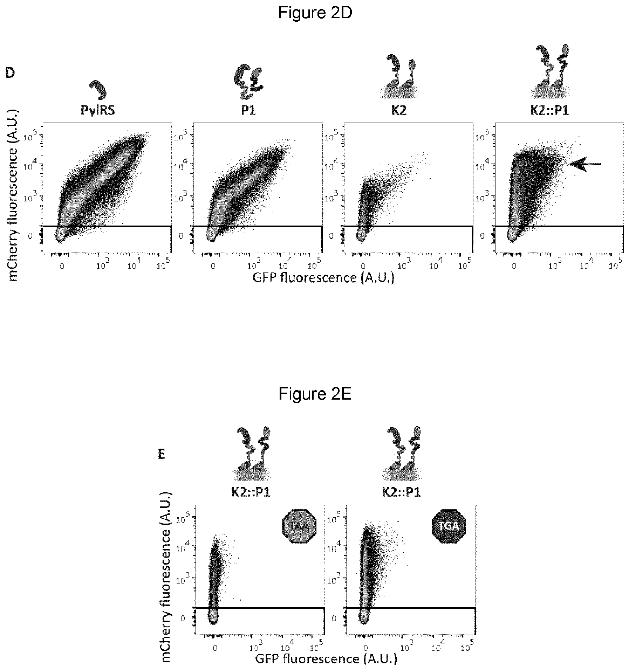

Figure 2C shows the selectivity and relative efficiency of various exemplary OT systems. For all experiments the indicated constructs were co-expressed with tRNA^{Pyl} (anticodon corresponding to the indicated codon) and the dual reporter (*GFP^{39STOP}, mCherry^{185STOP}::ms2*). GCE was performed in presence of the indicated ncAAs, and cells were analyzed by FFC. The dark gray bars (normalized to cytoplasmic PylRS) represent the fold change in the ratios *r* of the mean fluorescence intensities of mCherry versus GFP (derived from FFC, see Fig. 2D, E) for all the systems tested. The light-gray bars represent the relative efficiency as defined by the mean fluorescence intensity of mCherry for each condition divided by cytoplasmic PylRS control (derived from FFC, see Fig. 2D, E). Shown are the mean values of at least three independent experiments; error bars represent the SEM. The box highlights the best performing OT organelle (OT^{K2::P1}).
Figure 2D shows the results of the FFC analysis of the dual-color reporter expressed with the four indicated systems in transfected HEK293T cells and tRNA^{Pyl} in the presence of the ncAA SCO, a lysine derivative with a cyclooctyne side chain. Highly selective and efficient orthogonal translation was observed for the OT assembly (the black arrow indicates a bright, highly mCherry-positive population). Shown in the dot plots are the sums of at least three independent experiments. Axes indicate fluorescence intensity in arbitrary units.
Figure 2E shows FFC plots for the OT assembly selectively translating Opal and Ochre codons only of recruited mRNA(mCherry^{185TGA})::ms2 and mRNA(mCherry^{185TAA})::ms2, respectively.

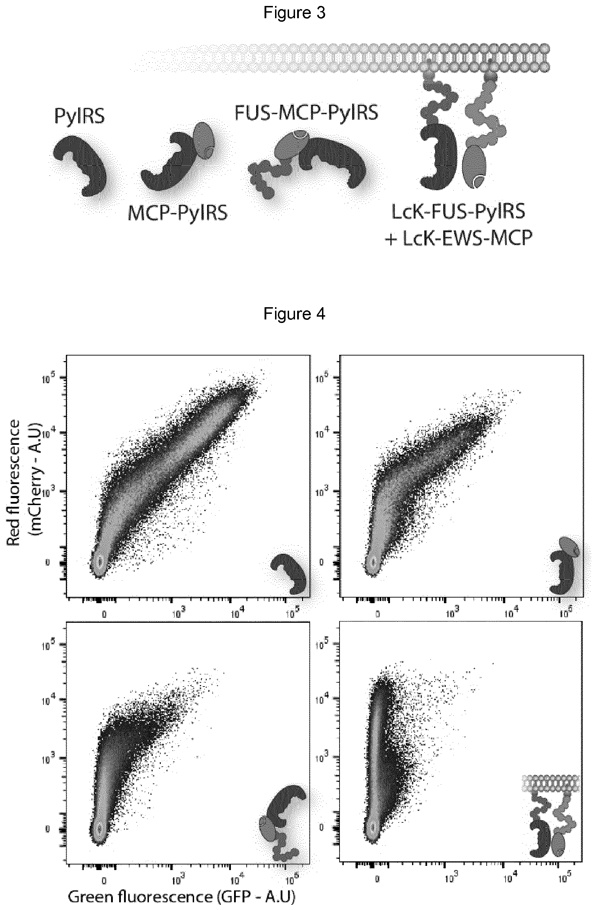

Figure 3 shows a schematic representation of the constructs composing the following systems: PylRS, MCP::PylRS, FUS::MCP::PylRS and LcK::FUS::PylRS•LcK::EWS::MCP.
Figure 4 shows the flow cytometry analysis of the dual reporter expression with the 4 different systems depicted in Figure 3. HEK293T cells were transfected with constructs encoding the dual reporter, tRNA, LcK::FUS::PylRS and LcK::EWS::MCP or PylRS, MCP::PylRS, FUS::MCP::PylRS and pcDNA3.1. Shown is the sum of at least three independent experiments. Axes indicate fluorescence intensity in arbitrary units.

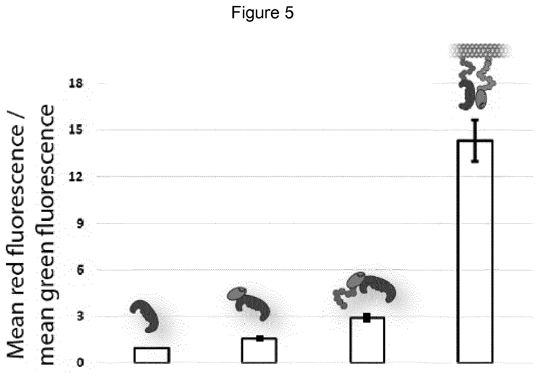

Figure 5 shows a bar plot with the ratios of the mean fluorescence intensity of mCherry vs. GFP fluorescence for all the tested systems. Plots represent mean values of at least 3 biological replicates, error bars indicate standard error of means.

### DETAILED DESCRIPTION OF THE INVENTION

Unless otherwise defined herein, scientific and technical terms used in connection with the present invention shall have the meanings that are commonly understood by those of ordinary skill in the art. The meaning and scope of the terms should be clear. However, in the event of any latent ambiguity, definitions provided herein take precedent over any dictionary or extrinsic definition. Further, unless otherwise required by context, singular terms shall include pluralities and plural terms shall include the singular.

If not otherwise stated, nucleotide sequences are depicted herein in the 5' to 3' direction. If not otherwise stated, amino acid sequences are depicted herein in the direction from N-terminus to C-terminus.

If not otherwise stated, the polypeptide of interest (POI) that is translationally expressed by the OT system according to the present invention comprises one or more ncAA residues which are encoded in the nucleotide sequence encoding the POI (CS^{POI}) by selector codons.

### 1. Fusion proteins

The fusion proteins of the invention include fusion proteins wherein at least one RNA-TP and at least one O-RS are comprised by one and the same fusion protein (RNA-TP/O-RS fusion proteins) as well as fusion proteins which comprise at least one AP and at least one EP selected from RNA-TP segments and O-RS segments (AFPs). AFPs can also comprise both RNA-TP and O-RS segments, such as one or more RNA-TP segments and one or more O-RS segments, in addition to the at least one AP.

The term "segment" as used herein in the context of fusion proteins indicates that the thus designated element (e.g., RNA-TP, O-RS, IC-TP, PSP) is part of the fusion protein, i.e. linked to the remainder of the fusion protein. The segments of the fusion proteins of the invention are functionally linked, i.e. linked such that they still function as RNA-TP, O-RS, IC-TP and PSP, respectively. Said linkage is preferably covalent, and in particular is a peptidic linkage.

For example, the RNA-TP segment comprised in the fusion proteins of the present invention is a segment of the fusion protein that is derived from, and functions in the context of the fusion protein as, an RNA-TP, thus allowing the fusion protein to interact with (bind to) the targeted RNA, wherein said interaction is expediently a specific one. Thus, an RNA-TP segment may comprise the (entire) amino acid sequence, or a functional fragment, of an RNA-targeting polypeptide as described herein.

Analogously, an O-RS segment comprised by the fusion proteins of the present invention is a segment of the fusion protein that is derived from, and functions in the context of the fusion protein as, an O-RS, thus conferring to the fusion protein O-RS enzymatic activity, that is the ability to catalyze the aminoacylation of an O-tRNA with an ncAA. Thus, an O-RS segment may comprise the (entire) amino acid sequence, or a functional fragment, of an O-RS as described herein.

The assembler fusion proteins (AFPs) described herein comprise at least one polypeptide segment acting as an assembler (AP). As used herein the term AP refers to any polypeptide segment that allows for enrichment of AFPs comprising said segment at spatially distinct sites within a living cell. Expediently said spatially distinct sites are located within, or directly adjacent to, the cytoplasm of the cell and readily accessible to the translational machinery of the cell (which includes canonical aminoacylated tRNAs, translation factors, ribosomal subunits, etc.) as well as the O-tRNAs which allow for the introduction of the ncAA residues into the POI.

There are different types of polypeptide segments which can serve as APs in the present invention. One type of APs are polypeptide segments which are derived from, and function in the context of the fusion protein as, intracellular targeting polypeptides (IC-TPs). These IC-TP segments may comprise the (entire) amino acid sequence, or a function fragment, of an IC-TP. IC-TPs target, and thus become locally enriched at, intracellular structural elements within, or directly adjacent to, the cytoplasm. Examples of such structural elements include microtubules, the cytoplasmic side of membranes such as the cell membrane, the nuclear membrane, the mitochondrial membrane, etc.

Accordingly, in particular embodiments, the fusion protein of the present invention comprises at least one IC-TP segment that targets, and facilitates local enrichment of the fusion protein at, microtubules, in particular the plus end or the minus end of the microtubules). For instance, dyneins and kinesins (proteins of the dynein or kinesin family of proteins), and functional fragments and mutants thereof, can be used as IC-TPs for such function.

In further particular embodiments, the fusion protein of the present invention comprises at least one IC-TP segment that is derived from, and functions as, a membrane anchor. For example, the fusion protein of the present invention comprises at least one IC-TP segment that targets, and facilitates local enrichment of the fusion protein at, the (inner) cell membrane (in particular the cytoplasmic side of the cell membrane). In another example, the fusion protein of the present invention comprises at least one IC-TP segment that targets, and facilitates local enrichment of the fusion protein at, the (outer) nuclear membrane (in particular the cytoplasmic side of the nuclear membrane). In further particular embodiments, the fusion protein of the present invention comprises at least one IC-TP segment that targets, and facilitates local enrichment of the fusion protein at, the outer mitochondrial membrane (in particular the cytoplasmic side of the mitochondrial membrane). For instance, the transmembrane domain of membrane proteins, and functional fragments and mutants thereof, can be used as IC-TPs for such function.

Polypeptides which target, and thus become locally enriched at, intracellular structural elements as described above, are known in the art and are useful as IC-TPs in the present invention. Specific examples of suitable IC-TPs include, but are not limited to:
- optionally truncated kinesin polypeptides which constitutively move towards, and become locally enriched at, microtubule-plus ends in living cells, for example optionally truncated kinesin family member 16B (KIF16B), e.g. optionally truncated *Homo sapiens* KIF16B (Uniprot: Q96L93), in particular the fragment covering KIF16B amino acid residues 1-400 (KIF16B₁₋₄₀₀) comprising the amino acid sequence of SEQ ID NO:20; or optionally truncated kinesin family member 13A (KIF13A), e.g. optionally truncated *Homo sapiens* KIF13A (Uniprot: Q9H1H9), in particular the KIF13A fragment covering amino acid residues 1-411 wherein P390 is deleted (KIF13A_{1-411,Δ390}) comprising the amino acid sequence of SEQ ID NO:22;
- polypeptides targeting the outer mitochondrial membrane derived from transmembrane-proteins such as, e.g., optionally truncated translocase of outer mitochondrial membrane 20 (TOMM20), for example optionally truncated *Homo sapiens* TOMM20 (Uniprot: Q15388), in particular the fragment covering amino acid residues 1-70 of TOMM20 (TOMM20₁₋₇₀) comprising the amino acid sequence of SEQ ID NO:24;
- cell membrane-targeting polypeptides derived from transmembrane-proteins such as, e.g., lymphocyte-specific protein tyrosine kinase (LcK; e.g., *Mus musculus* LcK, Uniprot: P06240), CD4 (e.g., *Mus musculus* CD4, Uniprot: P06332), FRB (similar to *Homo sapiens* mTOR; Uniprot: P42345), CD28 (e.g., *Mus musculus* CD28, Uniprot: P31041) and combinations thereof, in particular polypeptides comprising the amino acid sequence of SEQ ID NO:26, SEQ ID NO:28 or SEQ ID NO:30;
as well as functional fragments and mutants of these polypeptides. Said functional fragments and mutants may have at least 60%, at least 70%, at least 80%, at least 90%, at least 95%, at least 96%, at least 97%, at least 98% or at least 99% amino acid sequence identity to the amino acid of the polypeptide they are derived from.

A further type of APs are polypeptide segments which are derived from, and function in the context of the fusion protein as, phase separation polypeptides (PSPs). PSPs are polypeptides which have the ability to self-assemble in the cytoplasm of a cell so as to create sites of high local concentration in the cytoplasm. Specifically, PSPs are able to drive phase separation (in particular liquid-liquid phase separation) leading to the formation of membrane-less compartments in the cytoplasm. Said compartments may take the form of droplets, aggregates, condensates or a dense phase. In particular, PSPs include intrinsically disordered proteins (IDPs) which are an important class of proteins that drive phase separation (see, e.g., Alberti et al., Bioessays 2016, 38:959-968 and references cited therein such as Patel et al., Cell 2015, 162:1066-1077; Han et al., Cell 2012, 149:768-779; Kato et a/., Cell 2012, 149:753-767). There are three different classes of ICPs, proteins of each, or functional fragments or mutants thereof, can be used as PSPs in the present invention. One prominent class of IDPs contains so called prion-like domains which are devoid of charges and contain polar amino acid residues (Q, N, S, G) with interspersed aromatic residues (F, Y). See, e.g., Malinovska et al., Biochim Biophys Acta 2013, 1834:918-931; Alberti et al., 2009, Cell 137:146-158, Malinovska et al., Prion 2015, 9:339-346. Another class of IDPs is also characterized by low sequence complexity but frequently contains acidic and basic amino acid side chains, e.g. RGG repeat containing IDPs such as Ddx4. See Nott et al., Cell 2015, 57:936-947. Specific examples of suitable IC-TPs include, but are not limited to:
- spindle-defective protein 5 (SPD5) (e.g., *Caenorhabditis elegans* SPD5; Uniprot: P91349), in particular a polypeptide comprising the amino acid sequence of SEQ ID NO:32;
- fused-in sarcoma (FUS) (e.g., *Homo sapiens* FUS; Uniprot: P35637), in particular a polypeptide comprising the amino acid sequence of SEQ ID NO:34;
- Ewing sarcoma breakpoint region 1 (EWSR1) (e.g., *Homo sapiens EWSR1*; Uniprot: Q01844), in particular a polypeptide comprising the amino acid sequence of SEQ ID NO:36;
as well as functional fragments and mutants of these polypeptides. Said functional fragments and mutants may comprise at least 60%, at least 70%, at least 80%, at least 90%, at least 95%, at least 96%, at least 97%, at least 98% or at least 99% amino acid sequence identity to the amino acid of the polypeptide they are derived from.

The number of APs comprised by fusion proteins of the present invention is not particularly limited, i.e. a fusion protein may comprise 1, 2, 3, 4, 5, 6, 7, 8, 9, 10 or more same or different APs. Fusion proteins of the present invention which comprise at least one AP selected from IC-TP segments and at least one AP selected from PSP segments are particularly preferred. Likewise, the number of RNA-TP segments is not particularly limited and may be independently selected from 1, 2, 3, 4, 5 or more different or same RNA-TP segments. Likewise, the number of O-RS segments is not particularly limited and may be independently selected from 1, 2, 3, 4, 5 or more different or same O-RS segments. This applies to both AFPs as well as to RNA-TP/O-RS fusion proteins. The number of segments in the fusion proteins of the present invention of course influences the size of the fusion protein that is not particularly limited but typically less than 3500 amino acid residues, such as less than 3000 amino acid residues.

The order of the segments within the fusion proteins of the invention is not particularly limited either. The RNA-TP, O-RS and/or AP segments may thus be functionally linked in any order. Examples of RNA-TP/O-RS fusion protein structures include, but are not limited to,

[RNA-TP]ₓ- [O-RS]_{y}

[O-RS]_{y}- [RNA-TP]ₓ

wherein x and y, independently of each other, are integers selected from 1, 2, 3, 4 and 5;
"-" designates a peptidic linkage.
[RNA-TP]ₓ for x≥2 may include the same or different RNA-TP segments. [O-RS]_{y} for y≥2 may include the same or different O-RS segments.

Examples of RNA-TP/O-RS fusion protein structures include, but are not limited to:

[IC-TP]ₘ - [EP]ₒ

[EP]ₒ - [IC-TP]ₘ

[PSP]ₙ - [EP]ₒ

[EP]ₒ - [PSP]ₙ

[IC-TP]ₘ - [EP]ₒ - [PSP]ₙ

[PSP]ₙ - [EP]ₒ - [IC-TP]ₘ

[IC-TP]ₘ - [PSP]ₙ - [EP]ₒ

[EP]ₒ - [PSP]ₙ - [IC-TP]ₘ

[PSP]ₙ - [IC-TP]ₘ - [EP]ₒ

[EP]ₒ - [IC-TP]ₘ - [PSP]ₙ

wherein m, n and o, independently of each other, are integers selected from 1, 2, 3, 4 or 5,
and "-" designates a peptidic linkage.
[IC-TP]ₘ for m≥2 may include the same or different IC-TP segments. [PSP]ₙ for n≥2 may include the same or different PSP segments. [EP]ₒ for o≥2 may include the same or different EPs. Where [EP]ₒ includes different EPs, for example at least one EP may be a RNA-TP segment and at least one an O-RS segment.

The fusion proteins of the present invention provide an orthogonal translation (OT) system wherein the one or more O-RS (segments) required for the introduction of the one or more ncAA residues into the POI are brought into spatial proximity to at least one RNA-targeting polypeptide (RNA-TP) segment. The mRNA of the POI comprises at least one targeting nucleotide sequence (TN) that is able to interact with an RNA-TP segment of at least one of the fusion proteins of the OT system. Said interaction is expediently a specific one. The RNA-TP segments of the fusion proteins of the invention are preferably mRNA-targeting polypeptide segments. The RNA-TP segment of the fusion protein and the TN of the POI mRNA are expediently chosen so as to specifically interact with (bind to) one another. Suitable pairs of RNA-TP segment and TN for this purpose can be selected from coat proteins of RNA viruses and the nucleic acid motifs bound by said coat proteins. Such viral coat proteins and protein-bound RNA motifs are known in the art.

Specific examples of suitable RNA-TPs include, but are not limited to:
- MCP (coat protein of *Enterobacteria* phage MS2), in particular a polypeptide comprising the amino acid sequence of SEQ ID NO:14;
- λ_{N22} (22 amino acid RNA-binding domain of lambda phage antiterminator protein N), in particular a polypeptide comprising the amino acid sequence of SEQ ID NO:16;
as well as functional fragments and mutants of these polypeptides. Said functional fragments and mutants may comprise at least 60%, at least 70%, at least 80%, at least 90%, at least 95%, at least 96%, at least 97%, at least 98% or at least 99% amino acid sequence identity to the amino acid of the polypeptide they are derived from.

Specific examples of suitable TNs include, but are not limited to:
- *Enterobacteria phage* MS2 RNA stem-loop, in particular a polynucleotide having an RNA sequence corresponding to (encoded by) the nucleotide (DNA) sequence of SEQ ID NO:17;
- BoxB (lambda phase RNA stem-loop, specific binding site of λ_{N22}), in particular a polynucleotide having an RNA sequence corresponding to (encoded by) the nucleotide (DNA) sequence of SEQ ID NO:18;
as well as functional fragments and mutants thereof. Said functional fragments and mutants may comprise at least 60%, at least 70%, at least 80%, at least 90%, at least 95%, at least 96%, at least 97%, at least 98% or at least 99% nucleotide sequence identity to the polynucleotide sequences they are derived from.

MCP specifically interacts with MS2 RNA stem-loops. Thus, where the RNA-TP segment(s) of the fusion protein(s) comprise (consist of) segments which are derived from, and function as, MCP, the mRNA of the POI expediently comprises one or more MS2 RNA stem-loops, e.g. two MS2 RNA stem-loops. λ_{N22} specifically interacts with BoxB. Thus, where the RNA-TP segment(s) of the fusion protein(s) comprise (or consist of) segments which are derived from, and function as, λ_{N22}, the mRNA of the POI expediently comprises one or more BoxB motifs, e.g. one, two, three, four or more BoxB motifs.

Several RSs have been used for genetic code expansion including the *Methanococcus jannaschii* tyrosyl-tRNA synthetase, *E.coli* tyrosyl-tRNA synthetase, *E.coli* leucyl-tRNA synthetase pyrrolysyl-tRNA synthetases from certain *Methanosarcina* (such as *M. mazei, M. barkeri, M. acetivorans, M. thermophila*), *Methanococcoides* (*M. burtonii*) or *Desulfitobacterium* (*D. hafniense*). Corresponding orthogonal RS/tRNA pairs have been used to genetically encode a variety of functionalities in polypeptides (Chin, Annu Rev Biochem 2014, 83:379-408; Chin et al., J Am Chem Soc 2001, 124:9026; Chin et al., Science 2003, 301:964; Nguyen et al., J Am Chem Soc 2009, 131:8720; Yanagisawa et al., Chem Biol 2008, 15:1187). Depending on the cell used for the translation of the POI, these RS can be used as O-RS in the present invention.

Pyrrolysyl tRNA synthetases (PylRSs) which can be used in methods and fusion proteins of the invention may be wildtype or genetically engineered PylRSs. Examples for wildtype PylRSs include, but are not limited to PylRSs from archaebacteria and eubacteria such as *Methanosarcina maize, Methanosarcina barkeri, Methanococcoides burtonii, Methanosarcina acetivorans, Methanosarcina thermophila* and *Desulfitobacterium hafniense.* Genetically engineered PylRSs have been described, for example, by Neumann et al. (Nat Chem Biol 2008, 4:232), by Yanagisawa et al. (Chem Biol 2008, 15:1187), and in EP2192185A1. The efficiency of genetic code expansion using PylRS can be increased by modifying the amino acid sequence of the PylRS such that it is not directed to the nucleus. To this end, the nuclear localization signal (NLS) can be removed from the PylRS or can be overridden by introducing a suitable nuclear export signal (NES). PylRSs which are used in the fusion proteins and methods of the present invention may be PylRSs lacking the NLS and/or comprising a NES as described, e.g., in WO 2018/069481.

Accordingly, examples of O-RS segment(s) which can be used in the fusion proteins of the present invention include, but are not limited to:
- *Methanococcus jannaschii* tyrosyl-tRNA synthetase;
- *Escherichia coli* tyrosyl-tRNA synthetase;
- *Escherichia coli* leucyl-tRNA synthetase;
- *Methanosarcina mazei* pyrrolysyl-tRNA synthetase;
- *Methanosarcina barkeri* pyrrolysyl-tRNA synthetase;
- *Methanosarcina acetivorans* pyrrolysyl-tRNA synthetase;
- *Methanosarcina thermophila* pyrrolysyl-tRNA synthetase;
- *Methanococcoides burtonii* pyrrolysyl-tRNA synthetase;
- *Desulfitobacterium hafniense* pyrrolysyl-tRNA synthetase;
as well as functional (i.e., enzymatically active) fragments and mutants of these polypeptides. Said functional fragments and mutants may comprise at least 60%, at least 70%, at least 80%, at least 90%, at least 95%, at least 96%, at least 97%, at least 98% or at least 99% amino acid sequence identity to the aminoacyl tRNA synthetase they are derived from.

Particular examples of O-RS segments useful as in the present invention which are derived from *M. mazei* pyrrolysyl-tRNA synthetases include, but are not limited to:
- O-RS segments derived from PylRS^{AF} (*Methanosarcina mazei* pyrrolysyl tRNA synthetase double mutant: Y306A, Y384F; Uniprot: Q8PWY1), for example O-RS segments comprising the amino acid sequence of SEQ ID NO:8;
- O-RS segments derived from PylRS^{AA} (*Methanosarcina mazei* pyrrolysyl tRNA synthetase double mutant: N346A, C348A; Uniprot: Q8PWY1), for example O-RS segments comprising the amino acid sequence of SEQ ID NO:10;
- O-RS segments derived from PylRS^{AAAF} (*Methanosarcina mazei* pyrrolysyl tRNA synthetase quadruple mutant: Y306A, N346A, C348A, Y384F; Uniprot: Q8PWY1), for example O-RS segments comprising the amino acid sequence of SEQ ID NO:12;
as well as functional (i.e., enzymatically active) fragments and mutants of these polypeptide segments. Said functional fragments and mutants may comprise at least 60%, at least 70%, at least 80%, at least 90%, at least 95%, at least 96%, at least 97%, at least 98% or at least 99% amino acid sequence identity to the aminoacyl tRNA synthetase they are derived from.

According to particular embodiments, wild-type and mutant *M. mazei* PylRSs as described herein are used for aminoacylation of tRNA with ncAAs as described in WO2012/104422 or WO2015/107064. Exemplary ncAAs for this purpose include, but are not limited to, 2-amino-6-(cyclooct-2-yn-1-yloxycarbonylamino)hexanoic acid (SCO), 2-amino-6-(cyclooct-2-yn-1-yloxyethoxycarbonylamino)hexanoid acid, 2-amino-6[(4*E*-cyclooct-4-en-1-yl)oxycarbonyl-amino]hexanoic acid (TCO), 2-amino-6[(2*E*-cyclooct-2-en-1-yl)oxycarbonylamino]hexanoic acid (TCO*), 2-amino-6-(prop-2-ynoxycarbonylamino)hexanoic acid (PrK) and 2-amino-6-(9-biocyclo[6.1.0]non-4-ynylmethoxycarbonylamino)hexanoid acid (BCN).

### 2. Functional fragments and mutants

Described herein are fragments and mutants of particular RNA-TPs, O-RSs, IC-TPs, PSPs and TNs which are functional (i.e. have the RNA-binding activity of the parent RNA-TP, the targeting activity for intracellular structures of the parent IC-TP, the self-assembly activity of the parent PSP, the binding activity for RNA-TP of the parent TN or the enzymatic activity of the parent O-RS, respectively). Such fragments and mutants can be characterized by a minimum degree of sequence identity as described herein. Said amino acid or nucleotide sequence identity means identity over the entire length of the thus characterized amino acid or nucleotide sequence, respectively. The percentage identity values can be determined as known in the art on the basis of BLAST alignments, blastp algorithms (protein-protein BLAST), or using the Clustal method (Higgins et al., Comput Appl. Biosci. 1989, 5(2):151-1).

Fragments and mutants of particular RNA-TPs, O-RSs, IC-TPs or PSPs which are useful in the present invention retain the relevant function (binding, self-assembly or enzymatic activity, respectively) of the parent polypeptide and can be obtained, e.g., by conservative amino acid substitution, i.e. the replacement of an amino acid residue with different amino acid residues having similar biochemical properties (e.g. charge, hydrophobicity and size) as known in the art. Typical examples are substitution of Leu by Ile or *vice versa,* substitution of Asp by Glu or *vice versa,* substitution of Asn by Gln or *vice versa,* and others.

### 3. Orthogonal translation, tRNAs and POI coding sequences

The term "translation system" generally refers to a set of components necessary to incorporate a naturally occurring amino acid in a growing polypeptide chain (protein). Components of a translation system can include, e.g., ribosomes, tRNAs, aminoacyl tRNA synthetases, mRNA and the like. An aminoacyl tRNA synthetase (RS) is an enzyme capable of aminoacylating a tRNA with an amino acid or an amino acid analog. An RS used in processes of the invention is capable of aminoacylating a tRNA with the corresponding ncAA, i.e. aminoacylating a tRNA^{ncAA}. The term "orthogonal" as used herein refers to an element of a translation system (e.g., an orthogonal tRNA (O-tRNA) and/or an orthogonal aminoacyl tRNA synthetase (O-RS)) that is used with reduced efficiency by a translation system of interest (e.g., a cell). "Orthogonal" refers to the inability or reduced efficiency, e.g., less than 20% efficient, less than 10% efficient, less than 5% efficient, or e.g., less than 1 % efficient, of an O-tRNA or an O-RS to function with the endogenous RS or endogenous tRNAs, respectively, of a translation system of interest. For example, an O-tRNA in a translation system of interest is aminoacylated by any endogenous RA of the translation system with reduced or even zero efficiency, when compared to aminoacylation of an endogenous tRNA by the endogenous RS. In another example, an O-RS aminoacylates any endogenous tRNA in the translation system of interest with reduced or even zero efficiency, as compared to aminoacylation of the endogenous tRNA by an endogenous RS. Specifically, the term "orthogonal translation system" or "OT system" is used herein to refer to a translation system using an O-RS/O-tRNA^{ncAA} pair that allows for introducing ncAA residues into a growing polypeptide chain.

O-RS/O-tRNA^{ncAA} pairs used in the invention preferably have following properties: the O-tRNA^{ncAA} is preferentially aminoacylated with the ncAA by the O-RS. In addition, the orthogonal pair functions in the translation system of interest (e.g, the cell) such that the O-tRNA^{ncAA} is used to incorporate the ncAA residue into the growing polypeptide chain of a POI. Incorporation occurs in a site specific manner. Specifically, the O-tRNA^{ncAA} recognizes a selector codon (e.g., an Amber, Ochre or Opal stop codon) in the mRNA coding for the POI.

The term "preferentially aminoacylates" refers to an efficiency of, e.g., about 50% efficient, about 70% efficient, about 75% efficient, about 85% efficient, about 90% efficient, about 95% efficient, or about 99% or more efficient, at which an O-RS aminoacylates an O-tRNA with an unnatural amino acid compared to an endogenous tRNA or amino acid of a translation system of interest (e.g., a cell). The unnatural amino acid is then incorporated into a growing polypeptide chain with high fidelity, e.g., at greater than about 75% efficiency for a given selector codon, at greater than about 80% efficiency for a given selector codon, at greater than about 90% efficiency for a given selector codon, at greater than about 95% efficiency for a given selector codon, or at greater than about 99% or more efficiency for a given selector codon.

tRNAs which can be used for being aminoacylated by a fusion protein of the present invention comprising at least one O-RS segment derived from a *M. mazei* pyrrolysyl tRNA synthetase include, but are not limited to pyrrolysyl tRNA of *M. mazei* and functional mutants thereof wherein the anticodon is the anticodon to a selector codon such as, e.g., the CUA anticodon to the Amber stop codon TAG, the anticodon UCA to the Opal stop codon TGA, and the anticodon UUA to the Ochre stop codon TAA. Examples for such pyrrolysyl tRNAs include, but are not limited to, those encoded by the nucleotide sequence of SEQ ID NO:4 (tRNA^{Pyl,CUA}), SEQ ID NO:5 (tRNA^{Pyl,UCA}) or SEQ ID NO:6 (tRNA^{Pyl,UUA}).

The term "selector codon" as used herein refers to a codon that is recognized (i.e. bound) by the O-tRNA^{ncAA} in the translation process. The term is also used for the corresponding codons in polypeptide-encoding sequences of polynucleotides which are not messenger RNAs (mRNAs), e.g. DNA plasmids. The new OT systems described herein allow for orthogonal translation of POIs in a manner that is selective for the mRNA of said POIs compared to other mRNAs present in the cytoplasm of the cell. Nevertheless, it is preferable that the selector codon is a codon of low abundance in the cell chosen for expression, for example a codon of low abundance in naturally occurring eukaryotic cells. The new OT systems bring the mRNA of the POIs, the O-RS and the tRNA^{ncAA} into proximity to one another, thus supporting the introduction of the ncAA (rather than the introduction of an amino acid of a different tRNA that might potentially bind to the selector codon) at the selector codon-encoded amino acid position of the POI. Thus, the selector codon can be a sense codon. Nevertheless, in preferred embodiments, the selector codon is a codon that is not recognized by endogenous tRNAs of the cell used for preparing the POI.

The anticodon of the O-tRNA^{ncAA} binds to a selector codon within an mRNA (the mRNA of the POI) and thus incorporates the ncAA site-specifically into the growing chain of the polypeptide (POI) encoded by said mRNA. Examples for selector codons which are useful in the new OT systems described herein include, but are not limited to:
- nonsense codons, such as stop codons, *e.g.,* Amber (UAG), Ochre (UAA), and Opal (UGA) codons;
- codons consisting of more than three bases (e.g., four base codons);
- codons derived from natural or unnatural base pairs; and
- sense codons.

Where a selector codon is used that is a sense codon (i.e., a natural three base codon), it is preferable that the endogenous translation system of the cell used for POI expression according to a method of the present invention does not (or only scarcely) use said natural three base codon, *e.g.,* a cell that is lacking, or has a reduced abundance of, a tRNA that recognizes the natural three base codon or a cell wherein the natural three base codon is a rare codon. The use of one or more stop codons, such as one or more of Amber, Ochre and Opal, as selector codons in the present invention is particularly preferred.

A number of selector codons can be introduced into a polynucleotide encoding a desired polypeptide (target polypeptide, POI), e.g., one or more, two or more, more than three, etc. selector codons. A POI can carry two or more ncAA residues. Said ncAA residues can be the same and encoded by the same type of selector codon, or can be different and encoded by different selector codons.

An anticodon has the reverse complement sequence of the corresponding codon.

A suppressor tRNA is a tRNA (such as an O-tRNA^{ncAA}) that alters the reading of a messenger RNA (mRNA) in a given translation system (e.g., a cell). A suppressor tRNA can read through, e.g., a stop codon, a four base codon, or a rare codon.

The O-tRNA is preferentially aminoacylated by O-RS (rather than endogenous synthetases) and is capable of decoding a selector codon, as described herein. The O-RS recognizes the O-tRNA, e.g., with an extended anticodon loop, and preferentially aminoacylates the O-tRNA with an ncAA.

The O-tRNA and the O-RS used in the methods and/or fusion proteins of the invention can be naturally occurring or can be derived by mutation of a naturally occurring tRNA and/or RS from a variety of organisms. In various embodiments, the tRNA and RS are derived from at least one organism. In another embodiment, the tRNA is derived from a naturally occurring or mutated naturally occurring tRNA from a first organism and the RS is derived from naturally occurring or mutated naturally occurring RS from a second organism.

A suitable (orthogonal) tRNA/RS pair may be selected from libraries of mutant tRNA and RS, e.g.. based on the results of a library screening. Alternatively, a suitable tRNA/RS pair may be a heterologous tRNA/synthetase pair that is imported from a source species into the translation system. Preferably, the cell used as translation system is different from said source species. Methods for evolving tRNA/RS pairs are described, e.g., in WO 02/085923 and WO 02/06075.

Conventional site-directed mutagenesis can be used to introduce selector codons into the coding sequence of a POI.

### 4. Nucleic acid molecules

The invention also relates to nucleic acid molecules (single-stranded or double-stranded DNA and RNA sequences, for example cDNA, mRNA), or combinations of such nucleic acid molecules, comprising a nucleotide sequence that encodes for at least one of the fusion proteins of the present invention, and/or a nucleotide sequence complementary thereto.

Further, the invention relates to nucleic acid molecules (single-stranded or double-stranded DNA and RNA sequences, for example cDNA, mRNA), or combinations of such nucleic acid molecules, comprising (i) a nucleotide sequences (CS^{POI}) that encodes at least one POI, said POI comprising one or more ncAA residues which are encoded in the CS^{POI} by selector codons, and (ii) a targeting nucleotide sequence (TN) as described herein, wherein an RNA molecule comprising (the RNA version of) said TN is able to interact via said TN with an RNA-targeting polypeptide (RNA-TP).

The nucleic acid molecules of the invention can in addition contain untranslated sequences of the 3'- and/or 5'-end of the coding gene region. The TN is preferably located at the 3' end of the nucleic acid molecule encoding the POI(s). For example, nucleic acid molecules of the invention encoding the POI(s) can be prepared by introducing at least one TN at (in particular 3' of) the 3' untranslated region using common cloning techniques known in the art.

The nucleic acid molecules of the invention can in addition contain untranslated sequences of the 3'- and/or 5'-end of the coding gene region.

The invention further relates to, in particular recombinant, expression constructs or expression cassettes, containing, under the genetic control of regulatory nucleic acid sequences the nucleic acid sequence of the nucleic acid molecule, or combination of nucleic acid molecules, of the invention as described herein. The expression cassettes of the invention thus comprise the nucleic acid sequence coding for at least one POI (plus TN) or at least one fusion protein of the invention, and/or a nucleic acid sequence complementary thereto. The invention also relates to, in particular recombinant, vectors, comprising at least one of these expression constructs (expression vectors).

An expression cassette typically comprises a promoter sequence that is located 5' (upstream) of, and functionally linked with, the nucleic acid sequence encoding the to-be-expressed POI(s) or fusion protein(s), a terminator sequence 3' (downstream) of said encoding sequence and optionally further regulatory elements. Examples of such further regulatory elements include, but are not limited to, targeting sequences, enhancers, polyadenylation signals, selectable markers, amplification signals, replication origins and the like. Suitable regulatory sequences are described for example in Goeddel, Gene Expression Technology: Methods in Enzymology 185, Academic Press, San Diego, CA (1990).

In addition to these regulatory sequences, the natural regulation of these sequences can still be present before the actual structural genes and optionally can have been genetically altered, so that the natural regulation has been switched off and expression of the genes has been increased. The nucleic acid construct can, however, also be of simpler construction, i.e. no additional regulatory signals have been inserted before the coding sequence and the natural promoter, with its regulation, has not been removed. Instead, the natural regulatory sequence is mutated so that regulation no longer takes place and gene expression is increased.

A "functional" linkage of elements of nucleic acid molecules, such as promotor, polypeptide-encoding sequence, terminator, regulators, means that these elements are arranged such that the encoding sequence can be transcribed and the optional regulatory elements can perform their regulation of said transcription. This can be achieved by a direct linkage of the elements in one and the same nucleic acid molecule. However, such direct linkage is not necessarily required. Genetic control sequences, for example enhancer sequences, can even exert their function on the target sequence from more remote positions or even from other DNA molecules. Arrangements are preferred in which the nucleic acid sequence to be transcribed is positioned downstream (i.e. at the 3'-end of) the promoter sequence, so that the two sequences are joined together covalently. The distance between the promoter sequence and the nucleic acid sequence to be expressed can be smaller than 200 base pairs, or smaller than 100 base pairs or smaller than 50 base pairs.

For expression in a cell, the expression cassette is advantageously inserted into an expression vector. Expression vectors are chosen according to the cell to be used for expression which makes optimal expression of the encoding nucleotide sequences in the cell possible. Vectors are well known by a person skilled in the art and are given for example in "Cloning vectors" (Pouwels P. H. et al., Ed., Elsevier, Amsterdam-New York-Oxford, 1985). Examples of expression vectors include, but are not limited to, plasmids, viral vectors (phages), e.g. SV40, CMV, baculovirus and adenovirus, transposons, IS elements, phasmids, cosmids, and linear or circular DNA. See, e.g., the book "Cloning Vectors" (Eds. Pouwels P. H. et al. Elsevier, Amsterdam-New York-Oxford, 1985, ISBN 0 444 904018). These vectors can be replicated autonomously in the (host) cell or can be replicated chromosomally. Expression vectors comprising at least one expression cassette of the present invention represent a further aspect of the invention.

For the expression of a POI in a cell according to the present invention, it is possible, e.g., to introduce a nucleic acid molecule which encodes the POI (e.g. an expression vector of the invention) into the cell. Alternatively, an existing gene of the cell can be modified so as to comprise selector codons at those amino acid positions where the POI is intended to carry ncAA residues. Methods for introducing (recombinant) polypeptide-encoding nucleic acid molecules into, or for modifying existing genes of, a cell are known in the art.

The term "expression" describes, in the context of the invention, the production of polypeptides encoded by the corresponding nucleic acid sequence in a cell. The term "expression" is also used for the production of tRNA molecules encoded by nucleic acid sequences in the cell.

The nucleic acid molecules of the invention, including the expression cassettes and expression vectors of the invention can be prepared using common cloning techniques known in the art. Common recombination and cloning techniques are used, as described for example in T. Maniatis, E.F. Fritsch and J. Sambrook, Molecular Cloning: A Laboratory Manual, Cold Spring Harbor Laboratory, Cold Spring Harbor, NY (1989) and in T.J. Silhavy, M.L. Berman and L.W. Enquist, Experiments with Gene Fusions, Cold Spring Harbor Laboratory, Cold Spring Harbor, NY (1984) and in Ausubel, F.M. et al., Current Protocols in Molecular Biology, Greene Publishing Assoc. and Wiley Interscience (1987).

The nucleic acid molecules, or combinations of nucleic acid molecules, of the invention, including expression cassettes and expression vectors of the invention, can be isolated, for example by methods known in the art.

An "isolated" nucleic acid molecule is separated from other nucleic acid molecules that are present in the natural source of the nucleic acid, and moreover can be essentially free of other cellular material or culture medium, when it is produced by recombinant techniques, or free of chemical precursors or other chemicals, when it is chemically synthesized.

A nucleic acid molecule according to the invention can be isolated by standard techniques of molecular biology and the sequence information provided according to the invention. For example, cDNA can be isolated from a suitable cDNA-bank, using one of the concretely disclosed complete sequences or a segment thereof as hybridization probe and standard hybridization techniques (as described for example in Sambrook, J., Fritsch, E.F. and Maniatis, T. Molecular Cloning: A Laboratory Manual. 2nd edition, Cold Spring Harbor Laboratory, Cold Spring Harbor Laboratory Press, Cold Spring Harbor, NY, 1989). Moreover, a nucleic acid molecule, comprising one of the disclosed sequences or a segment thereof, can be isolated by polymerase chain reaction, using the oligonucleotide primers that were constructed on the basis of this sequence. The nucleic acid thus amplified can be cloned into a suitable vector and can be characterized by DNA sequence analysis. The oligonucleotides according to the invention can moreover be produced by standard methods of synthesis, e.g. with an automatic DNA synthesizer.

### 5. ncAAs and post-translational POI modifications

The abbreviation "ncAA" refers generally to any non-canonical or non-natural amino acid, or amino acid residue, that is not among the 22 naturally occurring proteinogenic amino acids. Numerous ncAAs are well known in the art (see, e.g., Liu et al., Annu Rev Biochem 2010, 79:413-444; Lemke, ChemBioChem 2014, 15:1691-1694). The term "ncAA" also refers to amino acid derivatives, for example α-hydroxy acids (rather than α-amino acids). Such derivatives have been shown to be translationally incorporable as well. See, e.g., Ohta et al., 2008, ChemBioChem 9:2773-2778. Accordingly, the meaning of terms such as "aminoacylate" or "aminoacylation" used herein is not limited to the RS-catalyzed linkage of a tRNA and an α-amino acid but also includes the RS-catalyzed linkage of a tRNA and a ncAA derivative such as an α-hydroxy acid.

Particular preferred ncAAs for use in the present invention are those which can be post-translationally further modified, for example using click chemistry reactions. Such click reactions include strain-promoted inverse-electron-demand Diels-Alder cycloadditions (SPIEDAC; see, e.g., Devaraj et al., Angew Chem Int Ed Engl 2009, 48:7013)) as well as cycloadditions between strained cycloalkynyl groups, or strained cycloalkynyl analog groups having one or more of the ring atoms not bound by the triple bond substituted by amino groups), with azides, nitrile oxides, nitrones and diazocarbonyl reagents (see, e.g., Sanders et al., J Am Chem Soc 2010, 133:949; Agard et al., J Am Chem Soc 2004, 126:15046), for example strain promoted alkyne-azide cycloadditions (SPAAC). Such click reactions allow for ultrafast and biorthogonal covalent site-specific coupling of ncAA-labeling groups of target polypeptides with suitable groups of coupling partner molecule. Pairs of docking and labeling groups which can react via the above-mentioned click reactions are known in the art. Examples of suitable ncAAs for use in the present invention comprising docking groups include, but are not limited to, the ncAAs ("unnatural amino acids", "UAAs") described, e.g., in WO 2012/104422 and WO 2015/107064. Optionally substituted strained alkynyl groups include, but are not limited to, optionally substituted *trans*-cyclooctenyl groups, such as those described in. Optionally substituted strained alkenyl groups include, but are not limited to, optionally substituted cyclooctynyl groups, such as those described in WO 2012/104422 and WO 2015/107064. Optionally substituted tetrazinyl groups include, but are not limited to, those described in WO 2012/104422 and WO 2015/107064.

The ncAAs used in the context of the present invention can be used in the form of their salt. Salts of an ncAA as described herein means acid or base addition salts, especially addition salts with physiologically tolerated acids or bases. Physiologically tolerated acid addition salts can be formed by treatment of the base form of an ncAA with appropriate organic or inorganic acids. ncAAs containing an acidic proton may be converted into their non-toxic metal or amine addition salt forms by treatment with appropriate organic and inorganic bases. Salts of carboxyl groups of ncAAs can be produced in a manner known in the art and comprise inorganic salts, for example sodium, calcium, ammonium, iron and zinc salts, and salts with organic bases, for example amines, such as triethanolamine, arginine, lysine, piperidine, etc. ncAAs may also be used in the form of salts of acid addition, for example salts with mineral acids, such as hydrochloric acid or sulfuric acid and salts with organic acids, such as acetic acid and oxalic acid. The ncAAs and salts thereof which are useful in the present invention also comprise the hydrates and solvent addition forms thereof, *e.g.* hydrates, alcoholates and the like.

Physiologically tolerated acids or bases are in particular those which are tolerated by the translation system used for preparation of POI with ncAA residues, *e.g.* are substantially non-toxic to living eukaryotic cells.

ncAAs, and salts thereof, useful in the context of the present the invention can be prepared by analogy to methods which are well known in the art and are described, e.g., in the various publications cited herein.

The nature of the coupling partner molecule depends on the intended use. For example, the target polypeptide may be coupled to a molecule suitable for imaging methods or may be functionalized by coupling to a bioactive molecule. For instance, in addition to the docking group, a coupling partner molecule may comprise a group selected from, but not limited to, dyes (e.g. fluorescent, luminescent, or phosphorescent dyes, such as dansyl, coumarin, fluorescein, acridine, rhodamine, silicon-rhodamine, BODIPY, or cyanine dyes), molecules able to emit fluorescence upon contact with a reagent, chromophores (e.g., phytochrome, phycobilin, bilirubin, etc.), radiolabels (e.g. radioactive forms of hydrogen, fluorine, carbon, phosphorous, sulphur, or iodine, such as tritium, ¹⁸F, ¹¹C, ¹⁴C, ³²P, ³³P, ³³S, ³⁵S, ¹¹In ¹²⁵I, ¹²³I, ¹³¹I, ²¹²B, ⁹⁰Y or ¹⁸⁶Rh), MRI-sensitive spin labels, affinity tags (e.g. biotin, His-tag, Flag-tag, strep-tag, sugars, lipids, sterols, PEG-linkers, benzylguanines, benzylcytosines, or cofactors), polyethylene glycol groups (e.g., a branched PEG, a linear PEG, PEGs of different molecular weights, etc.), photocrosslinkers (such as p-azidoiodoacetanilide), NMR probes, X-ray probes, pH probes, IR probes, resins, solid supports and bioactive compounds (e.g. synthetic drugs). Suitable bioactive compounds include, but are not limited to, cytotoxic compounds (*e.g.,* cancer chemotherapeutic compounds), antiviral compounds, biological response modifiers (*e.g.,* hormones, chemokines, cytokines, interleukins, etc.), microtubule affecting agents, hormone modulators, and steroidal compounds. Specific examples of useful coupling partner molecules include, but are not limited to, a member of a receptor/ligand pair; a member of an antibody/antigen pair; a member of a lectin/carbohydrate pair; a member of an enzyme/substrate pair; biotin/avidin; biotin/streptavidin and digoxin/antidigoxin.

The ability of certain (labeling groups of) ncAA residues to be coupled covalently *in situ* to (the docking groups of) conjugation partner molecules, in particular by a click reaction as described herein, can be used for detecting a target polypeptide having such ncAA residue(s) within a eukaryotic cell or tissue expressing the target polypeptide, and for studying the distribution and fate of the target polypeptides. Specifically, the method of the present invention for preparing a POI by expression in (e.g., eukaryotic) cells can be combined with super-resolution microscopy (SRM) to detect the POI within the cell or a tissue of such cells. Several SRM methods are known in the art and can be adapted so as to utilize click chemistry for detecting a target polypeptide expressed by a eukaryotic cell of the present invention. Specific examples of such SRM methods include DNA-PAINT (DNA point accumulation for imaging in nanoscale topography; described, e.g., by Jungmann et al., Nat Methods 11:313-318, 2014), dSTORM (direct stochastic optical reconstruction microscopy) and STED (stimulated emission depletion) microscopy.

### 6. Translational preparation of POIs in cells

The OT systems provided by the invention allow for the translational preparation of a POI in a cell.

The cell used for preparing a POI according to the invention can be a prokaryotic cell. Alternatively, the cell used for preparing a POI according to the invention can be a eukaryotic cell. The cell used for preparing a POI according to the invention can be a separate cell such as, e.g., a single-cell microorganism or a cell line derived from cells of multicellular organisms. Alternatively, the cell used for preparing a POI according to the invention can be present in (and part of) a tissue, an organ, a body part or an entire multicellular organism. Thus, the methods of the invention for preparing a POI can be performed with a separate cell or a cell culture, or with a tissue or tissue culture, organ, body part or (entire multicellular) organism.

Eukaryotic cells are often more difficult to handle and manipulate compared to prokaryotes such as, e.g., *E.coli,* and therefore not or only very difficult accessible to known approaches for POI-selective orthogonal translation such as those described in the "Background of the invention" section above. The OT system and the methods of the invention are therefore particular advantageous when use for POI expression in eukaryotic cells (including, e.g., single- and multicellular eukaryotic organisms, and eukaryotic cell lines).

In principle, all prokaryotic or eukaryotic cells can be used for preparing a POI according to a method of the present invention. Microorganisms such as, e.g., bacteria, fungi or yeasts can be used, as well as eukaryotic cells, such as, e.g., mammalian cells, insect cells, yeast cells and plant cells. Eukaryotic cells and in particular mammalian cells are particularly preferred.

The cell used for preparing a POI according to the invention carries a POI-encoding nucleotide sequence (CS^{POI}) wherein the ncAA residue(s) of the POI are encoded by selector codon(s). Said CS^{POI} is functionally linked with one or more targeting sequences (TNs). Translation yields an mRNA comprising the CS^{POI} and the TN(s). The cell further comprises one or more fusion proteins of the present invention, wherein said fusion protein(s) comprise at least one O-RS segment and at least one RNA-TP segment. Said O-RS and RNA-TP can be on separate fusion proteins (e.g. AFPs) of the invention. Alternatively, said O-RS and RNA-TP can be on one and the same fusion protein (e.g. on an RNA-TP/O-RS fusion protein or an AFP) of the invention. *Via* (at least one of) its TN(s) said mRNA can interact with (bind to) at least one of the RNA-TP segments of the fusion proteins of the invention in the cell. The cell further comprises one or more orthogonal tRNA^{ncAA} molecules (O-tRNA^{ncAA}) which carry the anticodon(s) to the selector codon(s) of the CS^{POI}. Said O-tRNA^{ncAA} molecules and one or more of the O-RS segments of the fusion proteins in the cell form one or more orthogonal O-RS/O-tRNA^{ncAA} pairs which allow for introducing the ncAA residue(s) into the amino acid sequence of the (translationally prepared) POI.

The interaction of the mRNA comprising CS^{POI} and TN(s) with the RNA-TP segment(s), the aminoacylation of the O-tRNA^{ncAA} with the ncAAs by the O-RS segment(s), and the translational preparation of the POI including the introduction of the ncAA residue(s) thought to take place in the cytoplasm, more particularly in the OT assembly (OT organelle), of the cell in the presence of the ncAAs.

The mRNA comprising CS^{POI} and TN(s) (mRNA^{POI}) can be generated from a recombinant construct (e.g. expression vector) introduced into the cell. Alternatively, one or more endogenous genes of the cell can be modified so as to comprise one or more selector codons and one or more TNs. Techniques for introducing recombinant constructs into a cell as well as methods for modifying endogenous genes of a cell are well known in the art.

The tRNA^{ncAA} molecules and fusion proteins of the invention can be generated from a recombinant construct (e.g. expression vector) introduced into the cell.

Using expression vectors according to the invention, recombinant cells can be produced which can be used for preparing a POI using a method of the present invention. Advantageously, the recombinant vectors according to the invention, described above, are introduced into a suitable cell and expressed.

The cell used for preparing a POI as described herein can be prepared by introducing nucleotide sequences encoding the fusion protein(s), the tRNA^{ncAA} molecule(s) and the POI into the cell. Said nucleotide sequences can be located on separate nucleic acid molecules (vectors) or on the same nucleic acid molecule (e.g., vector), in any combination, and can be introduced into the cell in combination or sequentially.

Preferably common cloning and transfection techniques, known by a person skilled in the art, are used, for example co-precipitation, protoplast fusion, electroporation, virus-mediated gene delivery, lipofection, microinjection or others, for introducing the stated nucleic acid molecules in the respective cell. Suitable techniques are described for example in Current Protocols in Molecular Biology, F. Ausubel et al., Ed., Wiley Interscience, New York 1997, or Sambrook et al. Molecular Cloning: A Laboratory Manual. 2nd edition, Cold Spring Harbor Laboratory, Cold Spring Harbor Laboratory Press, Cold Spring Harbor, NY, 1989.

For the methods of the present invention, the cell used for POI expression is grown or cultured in a manner known by a person skilled in the art. Depending on the type of cell, a liquid medium can be used for culturing. Culture can be batchwise, semi-batchwise or continuous. Nutrients can be present at the beginning of the culturing or can be supplied later, semi-continuously or continuously.

The expressed POIs can be purified by known techniques, such as, e.g., molecular sieve chromatography (gel filtration), such as Q-sepharose chromatography, ion exchange chromatography and hydrophobic chromatography, and other common protein purification techniques such as ultrafiltration, crystallization, salting-out, dialysis and native gel electrophoresis. Suitable methods are described, for example, in Cooper, T. G., Biochemische Arbeitsmethoden [Biochemical processes], Verlag Walter de Gruyter, Berlin, New York or in Scopes, R., Protein Purification, Springer Verlag, New York, Heidelberg, Berlin.

For isolating a POI, it can be advantageous to link the POI with a tag that can serve for easier purification. This can be achieved by introducing a corresponding tag-encoding sequence into the CS^{POI}. Suitable tags for protein purification are well known in the art and include, e.g., histidine tags (e.g., His₆ tag) and epitopes that can be recognized as antigens of antibodies (described for example in Harlow, E. and Lane, D., 1988, Antibodies: A Laboratory Manual. Cold Spring Harbor (N.Y.) Press). These tags can serve for attaching the proteins to a solid carrier, for example a polymer matrix, which can for example be used as packing in a chromatography column, or can be used on a microtiter plate or on some other carrier.

A tag linked to a POI can also serve for detecting the POI. Tags for protein detection are well known in the art and include, e.g., fluorescent dyes, enzyme markers, which form a detectable reaction product after reaction with a substrate, and others.

For preparing a POI according to a method of the present invention, the expression can be achieved by culturing the cell in the presence of one or more ncAAs corresponding to the ncAA residue(s) of the POI (wherein said ncAAs may expediently be comprised in the culture medium) for a time suitable to allow translation of the POI. Depending on the nucleic acid(s) encoding the POI (and optionally the fusion proteins of the invention and/or the tRNA^{ncAA} molecules), it may be required to induce expression by adding a compound inducing transcription, such as, e.g., arabinose, isopropyl *β*-D-thiogalactoside (IPTG) or tetracycline that allows transcription.

After translation, the POI may optionally be recovered from the translation system. For this purpose, the POI can be recovered and purified, either partially or substantially to homogeneity, according to procedures known to and used by those of skill in the art. Unless the target polypeptide is secreted into the culture medium, recovery usually requires cell disruption. Methods of cell disruption are well known in the art and include physical disruption, e.g., by (ultrasound) sonication, liquid-sheer disruption (e.g., via French press), mechanical methods (such as those utilizing blenders or grinders) or freeze-thaw cycling, as well as chemical lysis using agents which disrupt lipid-lipid, protein-protein and/or protein-lipid interactions (such as detergents), and combinations of physical disruption techniques and chemical lysis. Standard procedures for purifying polypeptides from cell lysates or culture media are also well known in the art and include, *e.g.,* ammonium sulfate or ethanol precipitation, acid or base extraction, column chromatography, affinity column chromatography, anion or cation exchange chromatography, phosphocellulose chromatography, hydrophobic interaction chromatography, hydroxylapatite chromatography, lectin chromatography, gel electrophoresis and the like. Protein refolding steps can be used, as desired, in making correctly folded mature proteins. High performance liquid chromatography (HPLC), affinity chromatography or other suitable methods can be employed in final purification steps where high purity is desired. Antibodies made against the polypeptides of the invention can be used as purification reagents, *i.e.* for affinity-based purification of the polypeptides. A variety of purification/protein folding methods are well known in the art, including, *e.g.,* those set forth in Scopes, Protein Purification, Springer, Berlin (1993); and Deutscher, Methods in Enzymology Vol. 182: Guide to Protein Purification, Academic Press (1990); and the references cited therein.

As noted, those of skill in the art will recognize that, after synthesis, expression and/or purification, polypeptides can possess a conformation different from the desired conformations of the relevant polypeptides. For example, polypeptides produced by prokaryotic systems often are optimized by exposure to chaotropic agents to achieve proper folding. During purification from, *e.g.,* cell lysates, the expressed polypeptide is optionally denatured and then renatured. This is accomplished, *e.g.,* by solubilizing the proteins in a chaotropic agent such as guanidine HCI. In general, it is occasionally desirable to denature and reduce expressed polypeptides and then to cause the polypeptides to re-fold into the preferred conformation. For example, guanidine, urea, DTT, DTE, and/or a chaperonin can be added to a translation product of interest. Methods of reducing, denaturing and renaturing proteins are well known to those of skill in the art. Polypeptides can be refolded in a redox buffer containing, *e.g.,* oxidized glutathione and L-arginine.

Also described are polypeptides produced by the methods of the invention. Such polypeptides can be prepared by a method of the invention that makes use of the OT system described herein.

### 7. Kits

The present invention also provides kits for preparing a POI having at least one non-canonical amino acid (ncAA) residue. The kit of the invention may comprise at least one expression vector for at least one fusion protein of the present invention. The fusion protein(s) encoded by the expression vector(s) in the kit may comprise at least one O-RS segment and at least one RNA-TP segment. The kit may further comprise at least one ncAA, or salt thereof, corresponding to the at least one ncAA residue of the POI. Expediently said O-RS segment is capable of aminoacylating a tRNA with the at least one ncAA. The kit may further comprise at least one expression vector for an orthogonal tRNA^{ncAA} (O-tRNA^{ncAA}) molecule. Further components of the kit may include at least one expression vector comprising a multiple cloning site and a targeting nucleotide sequence (TN), wherein an RNA molecule comprising said TN is able to interact via said TN with an RNA-targeting polypeptide (RNA-TP). Expediently said TN is a sequence which, when present in an RNA molecule, is able to interact with an RNA-TP segment of at least one of the fusion protein(s) encoded by the expression vector(s) comprised by the kit. The kit may further comprise at least one reporter construct encoding an easily detectable (e.g. fluorescent) reporter polypeptide having at least one non-canonical amino acid (ncAA) residue such that the mRNA translated from said construct comprises a TN as described herein.

The kits of the present invention can be used in methods of the invention for preparing ncAA-residue containing POIs as described herein.

### PARTICULAR EMBODIMENTS

The present invention further provides the following non-limiting embodiments E1 to E.
E1: An assembler fusion protein (AFP) comprising:
   (a) at least one first polypeptide segment acting as assembler (AP) that is selected from:
      (a1) a polypeptide segment derived from an intracellular targeting polypeptide (IC-TP segment), wherein said intracellular targeting polypeptide targets, and thus becomes locally enriched at, an intracellular structural element within or directly adjacent to the cytoplasm; and
      (a2) a polypeptide segment derived from a phase separation polypeptide (PSP segment), wherein said phase separation polypeptide has the ability to undergo self-association in the cytoplasm of a cell so as to create sites of high local concentration in the cytoplasm, and
   (b) at least one second polypeptide segment acting as an effector (EP) that is selected from:
      b1) an RNA-targeting polypeptide (RNA-TP) segment, and
      b2) an orthogonal aminoacyl tRNA synthetase (O-RS) segment;
      wherein said polypeptide segments are functionally linked in said AFP.
E2: The AFP of E1 comprising at least two APs, preferably at least one IC-TP segment and at least one PSP segment.
E3: The AFP of E1 or E2 having one of the following structures (from the N-terminus to the C-terminus):

   [IC-TP]ₘ - [EP]ₒ

   [EP]ₒ - [IC-TP]ₘ

   [PSP]ₙ - [EP]ₒ

   [EP]ₒ - [PSP]ₙ

   [IC-TP]ₘ - [EP]ₒ - [PSP]ₙ

   [PSP]ₙ - [EP]ₒ - [IC-TP]ₘ

   [IC-TP]ₘ - [PSP]ₙ - [EP]ₒ

   [EP]ₒ - [PSP]ₙ - [IC-TP]ₘ

   [PSP]ₙ - [IC-TP]ₘ - [EP]ₒ

   [EP]ₒ - [IC-TP]ₘ - [PSP]ₙ

   wherein m, n and o, independently of each other, are integers selected from 1, 2, 3, 4 or 5, and "-" designates a peptidic linkage.
E4: The AFP of any one of E1-E3, wherein the at least one EP is selected from RNA-TP segments.
E5: The AFP of any one of E1-E3, wherein the at least one EP is selected from O-RS segments.
E6: The AFP of any one of E1-E3 comprising at least one EP selected from RNA-TP segments and at least one EP selected from O-RS segments.
E7: The AFP of any one of E1-E6 comprising at least one IC-TP segment selected from dyneins and kinesins, and fragments and mutants of dyneins and kinesins which retain the ability to target, and become enriched at, the plus or the minus end of microtubules.
E8: The AFP of any one of E1-E6 comprising at least one IC-TP segment selected from transmembrane domains of membrane proteins, and functional fragments and mutants of transmembrane domains which retain the ability to target, and become enriched at, the cytoplasmic side of membranes, in particular membranes selected from the cell membrane, nuclear membrane and mitochondrial membrane.
E9: The AFP of any one of E1-E8 comprising at least one IC-TP segment selected from:
   - KIF16B₁₋₄₀₀ comprising the amino acid sequence of SEQ ID NO:20, or a functional fragment or mutant thereof having at least 60% sequence identity to the amino acid sequence of SEQ ID NO:20;
   - KIF13A_{1-411,Δ390} comprising the amino acid sequence of SEQ ID NO:22, or a functional fragment or mutant thereof having at least 60% sequence identity to the amino acid sequence of SEQ ID NO:22;
   - TOMM20₁₋₇₀ comprising the amino acid sequence of SEQ ID NO:24, or a functional fragment or mutant thereof having at least 60% sequence identity to the amino acid sequence of SEQ ID NO:24;
   - LcK comprising the amino acid sequence of SEQ ID NO:26, or a functional fragment or mutant thereof having at least 60% sequence identity to the amino acid sequence of SEQ ID NO:26;
   - FRB-CD28 comprising the amino acid sequence of SEQ ID NO:28, or a functional fragment or mutant thereof having at least 60% sequence identity to the amino acid sequence of SEQ ID NO:28; and
   - FUS-CD28 comprising the amino acid sequence of SEQ ID NO:30, or a functional fragment or mutant thereof having at least 60% sequence identity to the amino acid sequence of SEQ ID NO:30.
E10: The AFP of any one of E1-E9 comprising at least one PSP segment selected from intrinsically disordered proteins (IDPs), in particular prion-like domains, and functional fragments and mutants of IDPs, or prio-like domains which retain the ability to undergo self-association in the cytoplasm of a cell so as to create sites of high local concentration in the cytoplasm.
E11: The AFP of any one of E1-E10 comprising at least one PSP segment selected from:
   - SPD5 comprising the amino acid sequence of SEQ ID NO:32, or a functional fragment or mutant thereof having at least 60% sequence identity to the amino acid sequence of SEQ ID NO:32;
   - FUS comprising the amino acid sequence of SEQ ID NO:34, or a functional fragment or mutant thereof having at least 60% sequence identity to the amino acid sequence of SEQ ID NO:34; and
   - EWSR1 comprising the amino acid sequence of SEQ ID NO:36, or a functional fragment or mutant thereof having at least 60% sequence identity to the amino acid sequence of SEQ ID NO:36.
E12: The AFP of any one of E1-E11 comprising at least one RNA-TP segment selected from RNA-binding segments of viral coat proteins, and functional fragments and mutants of RNA-binding segments of viral coat proteins which retain the ability to interact specifically with an RNA motif of the virus.
E13: The AFP of any one of E1-E12 comprising at least one RNA-TP segment selected from:
   - MCP comprising the amino acid sequence of SEQ ID NO:14, or a functional fragment or mutant thereof having at least 60% sequence identity to the amino acid sequence of SEQ ID NO:14; and
   - λ_{N22} comprising the amino acid sequence of SEQ ID NO:16, or a functional fragment or mutant thereof having at least 60% sequence identity to the amino acid sequence of SEQ ID NO:16.
E14: The AFP of any one of E1-E13 comprising at least one O-RS segment selected from:
   - *Methanococcus jannaschii* tyrosyl-tRNA synthetase;
   - *Escherichia coli* tyrosyl-tRNA synthetase;
   - *Escherichia coli* leucyl-tRNA synthetase;
   - *Methanosarcina mazei* pyrrolysyl-tRNA synthetase;
   - *Methanosarcina barkeri* pyrrolysyl-tRNA synthetase;
   - *Methanosarcina acetivorans* pyrrolysyl-tRNA synthetase;
   - *Methanosarcina thermophila* pyrrolysyl-tRNA synthetase;
   - *Methanococcoides burtonii* pyrrolysyl-tRNA synthetase;
   - *Desulfitobacterium hafniense* pyrrolysyl-tRNA synthetase; and
   and functional fragments and mutants thereof which retain aminoacyl-tRNA synthetase enzymatic activity.
E15: The AFP of any one of E1-E14 comprising at least one O-RS segment selected from:
   - PylRS^{AF} comprising the amino acid sequence of SEQ ID NO:8, or a functional fragment or mutant thereof having at least 60% sequence identity to the amino acid sequence of SEQ ID NO:8;
   - PylRS^{AA} comprising the amino acid sequence of SEQ ID NO:10, or a functional fragment or mutant thereof having at least 60% sequence identity to the amino acid sequence of SEQ ID NO:10;
   - PylRS^{AAAF} comprising the amino acid sequence of SEQ ID NO:12, or a functional fragment or mutant thereof having at least 60% sequence identity to the amino acid sequence of SEQ ID NO:12.
E16: An assembler fusion protein (AFP) combination comprising at least two AFPs of any one of E1-E15.
E17: The AFP combination of E16 comprising at least one first AFP comprising at least one RNA-TP segment, and at least one second AFP comprising at least one O-RS segment.
E18: A fusion protein (RNA-TP/O-RS fusion protein) comprising:
   (i) at least one RNA-targeting polypeptide (RNA-TP) segment; and
   (ii) at least one orthogonal aminoacyl tRNA synthetase (O-RS) segment,
   wherein said polypeptide segments are functionally linked in said RNA-TP/O-RS fusion protein.
E19: The RNA-TP/O-RS fusion protein of E18 having one of the following structures (from the N-terminus to the C-terminus):

   [RNA-TP]ₓ- [O-RS]_{y}

   [O-RS]_{y}- [RNA-TP]ₓ

   wherein x and y, independently of each other, are integers selected from 1, 2, 3, 4 and 5; and "-" designates a peptidic linkage.
E20: The RNA-TP/O-RS fusion protein of E18 or E19 comprising at least one RNA-TP segment selected from RNA-binding segments of viral coat proteins, and functional fragments and mutants of RNA-binding segments of viral coat proteins which retain the ability to interact specifically with an RNA motif of the virus.
E21: The RNA-TP/O-RS fusion protein of any one of E18-E20 comprising at least one RNA-TP segment selected from:
   - MCP comprising the amino acid sequence of SEQ ID NO:14, or a functional fragment or mutant thereof having at least 60% sequence identity to the amino acid sequence of SEQ ID NO:14; and
   - λ_{N22} comprising the amino acid sequence of SEQ ID NO:16, or a functional fragment or mutant thereof having at least 60% sequence identity to the amino acid sequence of SEQ ID NO:16.
E22: The RNA-TP/O-RS fusion protein of any one of E18-E21 comprising at least one O-RS segment selected from:
   - *Methanococcus jannaschii* tyrosyl-tRNA synthetase;
   - *Escherichia coli* tyrosyl-tRNA synthetase;
   - *Escherichia coli* leucyl-tRNA synthetase;
   - *Methanosarcina mazei* pyrrolysyl-tRNA synthetase;
   - *Methanosarcina barkeri* pyrrolysyl-tRNA synthetase;
   - *Methanosarcina acetivorans* pyrrolysyl-tRNA synthetase;
   - *Methanosarcina thermophila* pyrrolysyl-tRNA synthetase;
   - *Methanococcoides burtonii* pyrrolysyl-tRNA synthetase;
   - *Desulfitobacterium hafniense* pyrrolysyl-tRNA synthetase; and
   and functional fragments and mutants thereof which retain aminoacyl-tRNA synthetase enzymatic activity.
E23: The RNA-TP/O-RS fusion protein of any one of E18-E22 comprising at least one O-RS segment selected from:
   - PylRS^{AF} comprising the amino acid sequence of SEQ ID NO:8, or a functional fragment or mutant thereof having at least 60% sequence identity to the amino acid sequence of SEQ ID NO:8;
   - PylRS^{AA} comprising the amino acid sequence of SEQ ID NO:10, or a functional fragment or mutant thereof having at least 60% sequence identity to the amino acid sequence of SEQ ID NO:10;
   - PylRS^{AAAF} comprising the amino acid sequence of SEQ ID NO:12, or a functional fragment or mutant thereof having at least 60% sequence identity to the amino acid sequence of SEQ ID NO:12.
E24: A nucleic acid molecule, or a combination of two or more nucleic acid molecules, comprising:
   (i) a nucleotide sequence that encodes at least one AFP of any one of E1-E15, or at least one AFP combination of E16 or E17, or
   (ii) a nucleic acid sequence complementary to the nucleotide sequence of (i).
   (iii) both of (i) and (ii).
E25: A nucleic acid molecule, or a combination of two or more nucleic acid molecules, comprising:
   (i) a nucleotide sequence that encodes at least one RNA-TP/O-RS fusion protein of any one of E18-E23, or
   (ii) a nucleic acid sequence complementary to (i), or
   (iii) both of (i) and (ii).
E26: An expression cassette comprising the nucleotide sequence of the nucleic acid molecule, or the combination of nucleic acid molecules, of E24 or E25.
E27: An expression vector comprising at least one expression cassette of E26.
E28: A cell comprising at least one nucleic acid molecule, or combination of nucleic acid molecules, of E24 or E25, at least one expression cassette of E26, or at least one expression vector of E27.
E29: The cell of E28 which is a eukaryotic cell.
E30: The cell of E28 which is a mammalian cell.
E31: The cell of any one of E28-E30 comprising at least one nucleic acid molecule, or combination of nucleic acid molecules, of E24, or at least one expression cassette comprising the nucleotide sequence of said nucleic acid molecule, or combination of nucleic acid molecules, or at least one expression vector comprising said expression cassette.
E32: The cell of E31 comprising a nucleotide sequence that encodes, or is complementary to a nucleotide sequence encoding, at least one AFP of any one of E1-E3 and E7-E15 comprising at least one EP selected from RNA-TP segments and at least one EP selected from O-RS segments.
E33: The cell of E31 comprising a nucleotide sequence that encodes, or is complementary to a nucleotide sequence encoding, at least one AFP of any one of E1-E3 and E7-E15 comprising at least one EP selected from RNA-TP segments, and at least one AFP of any one of E1-E3 and E7-E15 comprising at least one EP selected O-RS segments.
E34: The cell of any one of E28-E30 comprising at least one nucleic acid molecule, or combination of nucleic acid molecules, of E25, or at least one expression cassette comprising the nucleotide sequence of said nucleic acid molecule, or combination of nucleic acid molecules, or at least one expression vector comprising said expression cassette.
E35: The cell of any one E28-E34, wherein the cell expresses the at least one AFP, the at least one AFP combination or the at least one RNA-TP/O-RS fusion protein, respectively, that is encoded by the nucleotide sequence of said nucleic acid molecule, or combination of nucleic acid molecules.
E36: A method for preparing a polypeptide of interest (POI) comprising in its amino acid sequence one or more non-canonical amino acid (ncAA) residues, wherein the method comprises expressing the POI in a cell of any one of E31-E33 in the presence of said one or more ncAAs, wherein the cell comprises:
   (i) a POI-encoding nucleotide sequence (CS^{POI}) wherein said one or more ncAA residues of the POI are encoded by selector codon(s),
   (ii) a targeting nucleotide sequence (TN) that is functionally linked to the CS^{POI} and is able to interact with an RNA-TP segment of at least one of the AFPs in the cell;
   (iii) one or more orthogonal tRNA^{ncAA} (O-tRNA^{ncAA}) molecules which carry the anticodon(s) complementary to the selector codon(s) of the CS^{POI}, and wherein said O-tRNA^{ncAA} molecules together with one or more O-RS segments of at least one of the AFPs in the cell form one or more orthogonal O-RS/O-tRNA^{ncAA} pairs which allow for the introduction of said one or more ncAA residues into the amino acid sequence of the POI;
   and wherein the method optionally further comprises recovering the expressed POI.
E37: A method for preparing a polypeptide of interest (POI) comprising in its amino acid sequence one or more non-canonical amino acid (ncAA) residues, wherein the method comprises expressing the POI in a cell of E35 in the presence of said one or more ncAAs, wherein the cell comprises:
   (i) a POI-encoding nucleotide sequence (CS^{POI}) wherein said one or more ncAA residues of the POI are encoded by selector codon(s),
   (ii) a targeting nucleotide sequence (TN) that is functionally linked to the CS^{POI} and is able to interact with an RNA-TP segment of at least one of the RNA-TP/O-RS fusion proteins in the cell;
   (iii) one or more orthogonal tRNA^{ncAA} (O-tRNA^{ncAA}) molecules which carry the anticodon(s) complementary to the selector codon(s) of the CS^{POI}, and wherein said O-tRNA^{ncAA} molecules together with one or more O-RS segments of the RNA-TP/O-RS fusion proteins in the cell form one or more orthogonal O-RS/O-tRNA^{ncAA} pairs which allow for the introduction of said one or more ncAA residues into the amino acid sequence of the POI;
   and wherein the method optionally further comprises recovering the expressed POI.
E38: A method for preparing a polypeptide of interest (POI) comprising in its amino acid sequence one or more non-canonical amino acid (ncAA) residues, said method comprising the steps of:
   (a) expressing in a cell one or more AFPs of any one of E1-E3 and E7-E15 comprising at least one RNA-TP segment and one or more AFPs of any one of E1-E3 and E7-E15 comprising at least one O-RS segment;
   (b) expressing in said cell one or more orthogonal tRNA^{ncAA} (O-tRNA^{ncAA}) molecules, wherein
      - said orthogonal tRNA^{ncAA} molecules and one or more of the O-RS segments of the AFPs in the cell form one or more orthogonal aminoacyl tRNA synthetase/tRNA^{ncAA} (O-RS/O-tRNA^{ncAA}) pairs,
      - said O-RS/O-tRNA^{ncAA} pairs allow for introducing said one or more ncAA residues into the amino acid sequence of said POI,
      wherein steps (a) and (b) can be concomitantly or sequentially in any order;
   (c) then, expressing said POI in said cell in the presence of said one or more ncAAs, wherein
      - the POI-encoding nucleotide sequence (CS^{POI}) comprises one or more selector codons encoding said one or more ncAA residues,
      - said selector codons match the anticodons of said one or more O-tRNA^{ncAA} molecules;
      - said CS^{POI} is functionally linked to a targeting nucleotide sequence (TN), thus forming a CS^{POI}/TN fusion sequence,
      - said CS^{POI}/TN fusion sequence is able to interact, via its TN, with an RNA-TP segment of at least one of the AFPs in the cell;
      and
   (d) optionally recovering the expressed POI.
E39: A method for preparing a polypeptide of interest (POI) comprising in its amino acid sequence one or more non-canonical amino acid (ncAA) residues, said method comprising the steps of:
   (a) expressing in a cell RNA-TP/O-RS fusion proteins of any one of E18-E23;
   (b) expressing in said cell one or more orthogonal tRNA^{ncAA} (O-tRNA^{ncAA}) molecules, wherein
      - said orthogonal tRNA^{ncAA} molecules and one or more of the O-RS segments of the RNA-TP/O-RS fusion proteins in the cell form one or more orthogonal aminoacyl tRNA synthetase/tRNA^{ncAA} (O-RS/O-tRNA^{ncAA}) pairs,
      - said O-RS/O-tRNA^{ncAA} pairs allow for introducing said one or more ncAA residues into the amino acid sequence of said POI,
      wherein steps (a) and (b) can be concomitantly or sequentially in any order;
   (c) then, expressing said POI in said cell in the presence of said one or more ncAAs, wherein
      - the POI-encoding nucleotide sequence (CS^{POI}) comprises one or more selector codons encoding said one or more ncAA residues,
      - said selector codons match the anticodons of said one or more O-tRNA^{ncAA} molecules;
      - said CS^{POI} is functionally linked to a targeting nucleotide sequence (TN), thus forming a CS^{POI}/TN fusion sequence,
      - said CS^{POI}/TN fusion sequence is able to interact, via its TN, with an RNA-TP segment of at least one of the RNA-TP/O-RS fusion proteins in the cell;
      and
   (d) optionally recovering the expressed POI.
E40: The method of any one of E36-E39, wherein the TN is selected from viral RNA motifs bound by a viral coat protein, and functional fragments and mutants thereof which retain the ability to be bound by a viral coat protein.
E41: The method of any one of E36-E40, wherein the TN is selected from:
   - MS2 RNA stem-loop comprising the RNA sequence encoded by the nucleotide sequence of SEQ ID NO:17, or a functional fragment or mutant thereof having at least 60% sequence identity to the amino acid sequence of SEQ ID NO:17; and
   - BoxB comprising the RNA sequence encoded by the nucleotide sequence of SEQ ID NO:18, or a functional fragment or mutant thereof having at least 60% sequence identity to the amino acid sequence of SEQ ID NO:18.
E42: The method of any one of E36-E41, wherein the selector codon(s) encoding the ncAA residue(s) of the POI are selected from Amber, Ochre and Opal stop codons.
E43: A nucleic acid molecule comprising:
   (i) a nucleotide sequence (CS^{POI}) that encodes a polypeptide of interest (POI), said POI comprising one or more non-canonical amino acid (ncAA) residues which are encoded in the CS^{POI} by selector codons, and
   (ii) a targeting nucleotide sequence (TN), wherein an RNA molecule comprising said TN is able to interact via said TN with an RNA-targeting polypeptide (RNA-TP).
E44: The nucleic acid molecule of E43, wherein the TN is selected from viral RNA motifs bound by a viral coat protein, and functional fragments and mutants thereof which retain the ability to be bound by a viral coat protein.
E45: The nucleic acid molecule of E43 or E44, wherein the TN is selected from:
   - MS2 RNA stem-loop comprising the RNA sequence encoded by the nucleotide sequence of SEQ ID NO:17, or a functional fragment or mutant thereof having at least 60% sequence identity to the amino acid sequence of SEQ ID NO:17; and
   - BoxB comprising the RNA sequence encoded by the nucleotide sequence of SEQ ID NO:18, or a functional fragment or mutant thereof having at least 60% sequence identity to the amino acid sequence of SEQ ID NO:18.
E46: The nucleic acid molecule of any one of E43-E45, wherein the selector codon(s) encoding the ncAA residue(s) of the POI are selected from Amber, Ochre and Opal stop codons.
E47: A kit for preparing a polypeptide of interest (POI) having at least one non-canonical amino acid (ncAA) residue, the kit comprising:
   - at least one ncAA, or salt thereof, corresponding to the at least one ncAA residue of the POI, and
   - at least one expression vector of E27.
E48: The kit of E47, wherein the expression vector encodes a fusion protein comprising at least one O-RS segment and at least one RNA-TP segment.
E49: The kit of E47 or E48 further comprising at least one expression vector for an orthogonal tRNA^{ncAA} (O-tRNA^{ncAA}) molecule.
E50: The kit of any one of E47-E49 further comprising at least one expression vector comprising a multiple cloning site and a targeting nucleotide sequence (TN), wherein an RNA molecule comprising said TN is able to interact via said TN with an RNA-targeting polypeptide (RNA-TP).

The invention is further illustrated by the following non-limiting examples.

### EXAMPLES

### Methods

### (A) Cell culture, transfections and feeding with ncAAs

HEK293T cells (ATCC CRL-3216) and COS-7 cells (ATCC, CRL-1651) were maintained in Dulbecco's modified Eagle's medium (Life Technologies, 41965-039) supplemented with 1% penicillin-streptomycin (Sigma, 10,000 U/ml penicillin, 10 mg/ml streptomycin, 0.9% NaCl), 2 mM L-glutamine (Sigma), 1 mM sodium pyruvate (Life Technologies) and 10% FBS (Sigma). Cells were cultured at 37°C in a 5% CO₂ atmosphere and passaged every 2-3 days up to 15-20 passages.

In all cases, cells were seeded 15-20h prior to transfection at a density resulting in 70-80% confluency at the time of transfection. Flow cytometry was performed using 24-well plates with plastic bottom (Nunclon Delta Surface ThermoScientific). Immunofluorescence labeling and FISH were performed on 24-well plates with glass bottom (Greiner Bio-One) or four-well Lab-Tek #1.0 borosilicate coverglass (ThermoFisher).

Transfections of HEK293T cells were performed with polyethylenimine (PEI, Sigma-Aldrich) using 3 µg PEI per 1 µg DNA. COS-7 cells were transfected using the JetPrime reagent (PeqLab) according to the manufacturer's recommendations at a ratio of 1:2.

For Amber suppression system test, cells were transfected at a ratio of a 1:1:1:1 with POI^{TAG} vectors, tRNA^{Pyl}, synthetase and MCP or mock constructs. 4-6 hours after transfection the medium to a fresh containing ncAA.

Stock and working solutions for all of the used ncAAs were prepared as described in Nikic et al. (Nat Protoc 10(5):780-791, 2015). SCO (cyclooctyne lysine, SiChem SC-8000) was used at a final concentration of 250 µM. 3-lodophenylalanine (Chem-Impex International Inc.) was used at a final concentration of 1 mM. SCO is efficiently recognized by PylRS^{AF} (Y306A, Y384F) (see Plass et al., Angew Chem 2011, 50:3878-3881). 3-lodophenylalanine is recognized by PylRS^{AA} (C346A, N348A) (see Wang et al., ACS Chem Biol 2013, 8:405-415).

### (B) Flow cytometry

HEK293T cells were harvested after one day after transfection, resuspended in 1x PBS and passed through 100 µm nylon mesh. Co-transfections for flow cytometry were performed at a 1:1:1:1 ratio with 1.2 µg total DNA with:
- a reporter plasmid encoding the POI (a stop codon encoding the amino acid position to be occupied by the ncAA),
- a plasmid encoding the tRNA^{Pyl} having the anticodon which matches (i.e., is the reverse complement) of the stop codon in the POI-encoding sequence (hereinafter simply referred to as tRNA^{Pyl}),
- a plasmid encoding the PylRS or functional mutant thereof, respectively, and
- either a plasmid encoding an MCP fusion polypeptide or a mock plasmid.
Cell culture medium was exchanged for fresh medium containing the ncAA to be incorporated into the POI 4-6 h post-transfection and left until the time of harvesting.

Data acquisition and analysis were performed using an LSRFortessa SORP Cell Analyzer (Becton, Dickinson and Company) and the FlowJo software (FlowJo). Cells were first gated by cell type using forward scatter area (FSC-A) and side scatter area (SSC-A) parameters. Subsequently, single cells were identified based on SSC-A and side scatter width (SSC-W). Each shown FFC plot is the sum of three independent biological replicates from which the mean and the SEM were calculated. At least 130,000 single cells were analyzed per condition. GFP fluorescence was acquired in the 488-530/30 channel and mCherry fluorescence in the 561-610/20 channel.

### (C) PylRS immunostaining and imaging, fluorescence in situ hybridization (FISH)

For immune-labelling experiments, the cells were rinsed with 1x PBS, fixed in 2%

paraformaldehyde in 1x PBS for 10 min at RT, rinsed with 1x PBS again and then permeabilized in 0.5% Triton X in 1x PBS for 15 min at RT. After rinsing the permeabilized cell samples twice with 1x PBS, said samples were incubated for 90 min in blocking solution (3% BSA in 1x PBS for 90 min at RT), and then with 1 µg/ml primary antibody (polyclonal rat anti-PyIRS, prepared as described in Nikic et al. (Angew Chem Int Ed Engl 2016, 55(52):16172-16176) and/or polyclonal rabbit anti-MCP (Merck, ABE76) and/or monoclonal rabbit anti-RPL26L1 antibody (EPR8478, Abcam, ab137046)) in blocking solution overnight at 4°C. The next day, the cell samples were rinsed with 1x PBS and incubated with 2 µg/ml secondary antibody (chicken anti-rat IgG(H+L) cross-adsorbed Alexa Fluor 594 conjugated antibody (Thermo Fisher Scientific, A-21471) and/or goat anti-rabbit IgG(H+L) cross-adsorbed Alexa Fluor 647 conjugated F(ab')₂ (Thermo Fisher Scientific, A-21246)) in blocking solution for 60 min at RT. DNA was stained with Hoechst 33342 (1 µg/ml in 1xPBS) for 10 min at RT. If only DNA was stained, the cells were fixed and permeabilized as described above and then stained with Hoechst 33342 (1 µg/ml in 1xPBS) for 10 min at RT. Finally, the cells were rinsed twice with 1x PBS.

FISH experiments were performed one day after transfection analogously to the FISH experiments described in Nikic et al. (Angew Chem Int Ed Engl 2016, 55(52):16172-16176). The hybridization protocol was adapted for 24-well plates from Pierce et al. (Methods Cell Biol 122:415-436, 2014).

For imaging of only tRNA^{Pyl}, the hybridization probe 5'-CTAACCCGGCTGAACGGATTTAGAGTCCATTCGATC-3' (labelled at the 5' terminus with Cy5; SEQ ID NO:1) was used at 0.25 µM. After four washes with SSC and one wash with TN buffer (0.1 M TrisHCl, 150 mM NaCl), cells were incubated for 1 h at RT with 3% BSA in TN buffer prior to standard immunofluorescence labeling as described above.

For imaging of both tRNA^{Pyl} and MS2 RNA stem-loop sequence, the hybridization probe for tRNA^{Pyl} (5'-CTAACCCGGCTGAACGGATTTAGAGTCCATTCGATC-3', labelled at the 5' terminus with digoxigenin; SEQ ID NO:2) was used at 0.16 µM, and the hybridization probe for the MS2 RNA stem-loop sequence (5'-CTGCAGACATGGGTGATCCTCATGTTTTCTA-3', labelled at the 5' terminus with Alexa Fluor 647; SEQ ID NO:3) was used at 0.75 µM. After four washes with SSC, the cells were incubated for 1h at RT in blocking buffer (0.1 M TrisHCl, 150 mM NaCl, 1x blocking reagent (Sigma 11096176001). Then, the cells were incubated with fluorescein conjugated sheep anti-digoxigenin Fab (Sigma 11207741910) at a 1:200 dilution in blocking buffer overnight at 4°C. The next day, 3 washes of 5 minutes were done in Tween buffer (0.1 M TrisHCl, 150 mM NaCl, 0.5% Tween20). DNA was stained with Hoechst 33342 (1 µg/ml in 1xPBS) for 10 min at RT.

Confocal images were acquired on a Leica SP8 STED 3X microscope equipped with a 63x/1.40 oil immersion objective using the following laser lines for excitation: 405 nm for Hoechst 33342, 488 nm for fluorescein and GFP, 548 nm for mOrange, 594 nm for Alexa Fluor 594, 647 nm for Alexa Fluor 647 and Cy5. Emission light was collected with HyD detectors at 420-500 nm and 605-680 nm respectively.

Ribosomal immunofluorescence images were taken on an Olympus Fluoroview FV3000 microscope equipped with a 60x/1.40 oil immersion objective using the following laser lines for excitation: 488 nm for GFP, 594 nm for Alexa Fluor 594, 640 nm for Alexa Fluor 647.

Images were processed using FIJI software.

### (D) Constructs, cloning and mutagenesis

Two different fluorescent protein reporters (dual color reporter) were cloned into a pBI-CMV1 vector (Clontech 631630), one protein in one multiple cloning site and the other reporter in the other multiple cloning site. The CDS for one of the reporters encoded an mRNA carrying two MS2 RNA stem-loops fused to the 3' untranslated region ("MS2-tag"), while the encoded mRNA of the other reporter was not MS2-tagged.

For examination of Amber suppression, The reporters GFP^{39TAG} and mCherry^{185TAG} were used as N-terminal fusion with NLS. For examination of Ochre and Opal suppression, analogous constructs were prepared (with GFP^{39TAA} and mCherry^{185TAA}, GFP^{39TGA} and mCherry^{185TGA}, respectively).

NLS::GFP^{39TAG}::MS2-tag reporter: NLS::GFP^{39TAG} was cloned with two copies of MS2 RNA stem-loops into the pBI-CMV1 vector as a reporter for successful Amber suppression in imaging experiments.

For examination of suppression of multiple Amber codons, pBI-CMV constructs for GFP^{39,149TAG} and GFP^{39,149,182TAG} were prepared which did not contain a second (e.g. mCherry) reporter in the second multiple cloning site.

Constructs for OT assemblies were prepared as follows: tRNA^{Pyl} was cloned under the control of a human U6 promoter, and all other constructs were under CMV promoters cloned in the pcDNA3.1 (Invitrogen V86020) vector. MCP protein was cloned from the addgene plasmid #31230 and FUS from the Addgene plasmid #26374. In all FUS fusions, amino acids 1-478(S108N) were used, replacing the C-terminal NLS region by a Flag-tag. In all RS fusions the previously reported efficient NES::PylRS^{AF} (Y306A, Y384F) sequence was used (see, e.g., Nikic et al., Angew Chem Int Ed Engl 2016, 55(52):16172-16176). The PylRS mutant PylRS^{AA} (N346A, C348A) was cloned via site-directed mutagenesis starting from wildtype PylRS. The SPD5 gene was ordered from Genewiz and fused to MCP and PylRS^{AF} via restriction cloning. KIF13A₁₋₄₁₁ and KIF16B₁₋₄₀₀ were cloned from human cDNA and inserted into pcDNA3.1 via restriction cloning. P390 of KIF13A₁₋₄₁₁ was removed via side directed mutagenesis. KIF13A₁₋₄₁₁,ΔP390 and KIF16B₋₄₀₀ fusions with MCP, PylRS^{AF}, EWSR1::MCP, FUS::PylRS^{AF}, FUS::PylRS^{AA}, SPD5::MCP and SPD5::PylRS^{AF} were assembled via Gibson assembly (see Gibson et al., Nat Methods 2009, 6:343-345).

Constructs for differential imaging experiments: To selectively express Nup153-EGFP^{149TAG} and Vim^{116TAG}-mOrange, one gene was first inserted together with an MS2-tag into pBI-CMV1 (compare Nikic et al., Angew Chem Int Ed Engl 2016, 55(52):16172-16176). Subsequently, the other gene was inserted without MS2-tag. INSR^{676TAG}::mOrange was fused to an MS2-tag by replacing Vim^{116TAG}-mOrange in the pBI vector bearing Nup153::EGFP^{149TAG} and Vim^{116TAG}::mOrange::MS2-tag to yield a bicistronic vector with INSR^{676TAG}::mOrange in one and Nup153::EGFP^{149TAG} in the other cassette.

Multicistronic Amber suppression vectors for COS-7 cell experiments: As COS-7 cells have lower transfection efficiency; we generated multicistronic vectors harboring the components of an OT assembly. To assemble multicistronic Amber suppression vectors, first one copy of tRNA^{Pyl} under the control of a human U6 promoter was inserted into the pBI-CMV1 vector via Gibson assembly. Subsequently, first the AFP CDS KIF16B::FUS::PylRS^{AF} and finally the AFP CDS KIF16B::EWSR1::MCP were inserted via Gibson assembly. Alternatively, a previously published pcDNA3.1 based construct (see Nikic et al., Angew Chem Int Ed Engl 2016, 55(52):16172-16176) expressing NES::PylRS^{AF} under a CMV promoter and tRNA^{Pyl} under a human U6 promoter was used. Constructs with U6-tRNA^{Pyl}, KIF16B::FUS::PylRS^{AF} and KIF16B::EWSR1::MCP, or NES::PylRS^{AF} were alternatively inserted into a pDonor vector (GeneCopoeia).

### EXAMPLE 1 - RNA-TP/O-RS fusion and AFPs comprising a single AP

An OT assembly ("OT organelle", Fig. 1) was engineered having the following components:
i) An mRNA-targeting system in which two MS2 RNA stem-loops (MS2-tag) were fused to the mRNA of choice coding for the POI, creating an mRNA::ms2 fusion. The MS2-tag binds specifically to the MS2 bacteriophage coat protein (MCP) (see Bertrand et al., Mol Cell 1998, 2:437-445), which will thus form a stable and specific mRNA::ms2-MCP complex in cells. The MS2-tag was always fused to the 3' untranslated region (3' UTR) of the mRNA, which ensures translation to yield a scar-less final POI.
ii) A tRNA/RS suppressor pair. The orthogonal tRNA/RS pair from the *Methanosarcina mazei* pyrrolysyl system (tRNA^{Pyl}/PylRS) was chosen because it has enabled the encoding of more than 200 ncAAs with diverse functionalities into proteins using GCE in a multitude of cell types and species, including *E. coli,* mammalian cells and even living mice (see, e.g., Liu et al., Annu Rev Biochem 2010, 79:413-444; Lemke, ChemBioChem 2014, 15:1691-1694; Chin, Nature 2017, 550;53-60).
iii) The assembler (AP) was the key component required to form an OT assembly. The purpose of the assembler was to create membrane-less structures in the form of a dense phase, aggregate, droplet or condensate, in which the mRNA::ms2-MCP complex is brought into close proximity of the tRNA^{Pyl}/PylRS pair.

The simplest strategy tested was the bimolecular fusion of MCP::PylRS (termed B, Fig. 2). In addition, strategies were tested which were expected to yield much larger assemblies. All of those assembly systems were composed of an assembler fusion to PylRS co-expressed with an assembler fusion to MCP. Assembler::PylRS•assembler::MCP were expected to form large aggregates (co-expression herein denoted with "•"). One tested assembly strategy was based on phase separation of proteins and one based on the assembly of kinesins, which are abbreviated herein as **P** and **K,** respectively (Fig. 2A). Furthermore, for each **P** and **K** approach two different molecular designs **(P1, P2** and **K1, K2,** respectively) were tested which are summarized as follows:
**P1.** Previous studies have established the capacity of the proteins fused-in sarcoma (FUS) and Ewing sarcoma breakpoint regions 1 (EWSR1) to form mixed droplet-like structures by phase separation. They both contain a prion-like disordered domain that facilitates phase separation into liquid, gel and solid states (see, e.g., Altmeyer et al., Nat Commun 2015, 6:8088; Patel et al., Cell 2015, 162:1066-1077). In a phase-separated state, these proteins are locally highly concentrated (several orders of magnitude) compared to the remaining soluble fraction in the cytoplasm. FUS was fused to PylRS and EWSR1 was fused to MCP. It was expected that this would lead to the formation of droplets in which MCP and PylRS are highly enriched. **P1** is denoted FUS::PylRS•EWSR1::MCP.
**P2.** The *Caenorhabditis elegans* protein spindle-defective protein 5 (SPD5) has been shown to phase separate into particularly large (several micron-sized) droplets (see Woodruff et al., Cell 2017, 169:1066-1077, e1010). In a phase-separated state, SPD5 is locally highly concentrated compared to the remaining soluble fraction in the cytoplasm (by several orders of magnitude). It was expected that a protein fused to SPD5 would condense into droplets. Similarly to FUS-EWSR1 droplets, PylRS fused to SPD5 and MCP fused to SPD5 were expected to be highly enriched. **P2** is denoted SPD5::PylRS•SPD5::MCP.
**K1.** Certain kinesin truncations constitutively move towards microtubule-plus ends in living cells (Soppina et al., Proc Natl Acad Sci U.S.A. 2014, 111:5562-5567). One such truncated kinesin is KIF13A_{1-411,ΔP390}, and it was expected that PylRS and MCP, respectively, fused to this kinesin truncation and co-expressed would be locally enriched, due to spatial targeting to microtubule-plus ends. **K1** is denoted KIF13A_{1-411,ΔP390}::PylRS•KIF13A_{1-411,ΔP390}::MCP.
**K2.** By analogy to **K1,** the truncated kinesin KIF16B₁₋₄₀₀ was also tested. **K2** is denoted KIF16B₁₋₄₀₀::PylRS•KIF16B₁₋₄₀₀::MCP.

In order to evaluate these assemblers for facilitating functional orthogonal translation of an MS2-tagged mRNA, a dual-reporter construct was designed, in which GFP and mCherry mutants are simultaneously expressed from two different expression cassettes from one plasmid, ensuring that the mRNA ratio between them is constant across all experiments. Stop codons were introduced at permissive sites into GFP at position 39 (GFP^{39STOP}) and into mCherry at position 185 (mCherry^{185STOP}; Fig. 2B). Only if stop codon suppression is successful will the corresponding green or red fluorescent protein be produced. Transfected cells (tRNA^{Pyl} and ncAA were always present unless specifically noted otherwise) were analyzed by fluorescence flow cytometry (FFC); settings were adjusted so that an approximate diagonal results in the FFC plots if GFP and mCherry are expressed from this plasmid using the conventional cytoplasmic PylRS system, which cannot differentiate mRNAs. A selective and functional OT organelle should selectively express mCherry only if the MS2-tag is fused to the 3' UTR of the mCherry mRNA, leading to the appearance of a vertical line in the cytometry plot (Fig. 2B). Unless otherwise reported, all experiments where performed in the presence of tRNA^{Pyl} and the ncAA SCO, a widely used and well characterized lysine derivative, the side chain of which carries a cyclooctyne that can be used in a variety of click-chemistry reactions to install diverse chemical groups onto the protein. As previously reported, this ncAA is efficiently encoded by a Y306A, Y384F double mutant of PylRS (for simplicity this mutant is designated PylRS herein, unless otherwise specified) (see Nikic et al., Angew Chem 2014, 53:2245-2249; Plass, Angew Chem 2012, 51:4166-4170; Plass et al., Angew Chem 2011, 50:3878-3881). Omission of the ncAA served as a standard negative control and lead to no expression of GFP or mCherry.

The performance of each OT system was evaluated according to its selectivity and relative efficiency. Selectivity is defined as the ratio *r* of the mean mCherry FFC signal divided by the mean GFP signal. Final values are expressed as fold selectivity relative to that of cytoplasmic PylRS. Relative efficiency is defined as the mean mCherry signal of each system divided by the mean mCherry signal of the cytoplasmic PylRS system, which serves as the reference (here defined as 100%). All results on selectivity (dark-gray positive bars) and efficiency (light-gray negative bars) are summarized in the bar plot in Figure 2C. Selected FFC data is also shown in Figure 2D.

The simplest strategy **B** (MCP fused to PylRS) showed an about 1.5-fold selectivity gain (Fig. 2C). The OT system **P1** (based on phase separation of FUS/EWSR1) had a somewhat lower selectivity gain (Fig. 2C, D). The **P2** system (based on SPD5) showed an approximate twofold selectivity gain (Fig. 2C). For **K1** a twofold increase in selectivity was observed (Fig. 2C). The **K2** system behaved similarly (Fig. 2C,D). In total, the selectivity gains were relatively small, but robustly detected and distinguishable from a simple efficiency drop. The observed selectivity effect (data not shown) was robust across a titration of Amber suppression efficiencies (specifically, 0.48 ng, 2.4 ng, 12 ng, 60 ng or 300 ng tRNA^{Pyl} construct, respectively, were used), indicating that bringing the ncAA aminoacylation activity (i.e. the tRNAPyl/PylRS in the presence of ncAA) in direct proximity of the target mRNA represents a pathway to more selective codon suppression.

### EXAMPLE 2 - AFPs comprising a combination of two APs

AFPs comprising combinations of the APs described in example 1 were tested in an analogous manner, those were:
**K1::P1** = KIF13A_{1-411,ΔP390}::FUS::PylRS•KIF13A_{1-411,ΔP390}::EWSR1::MCP,
**K2::P1** = KIF16B₁₋₄₀₀::FUS::PylRS•KIF16B₁₋₄₀₀::EWSR1::MCP,
**K1::P2** = KIF13A_{1-411,ΔP390}::SPD5::PylRS•KIF13A_{1-411,ΔP390}::SPD5::MCP,
**K2::P2** = KIF16B₁₋₄₀₀::SPD5::PylRS-KIF16B₁₋₄₀₀::SPD5::MCP.

For all combinations an at least fivefold selectivity gain was observed indicating orthogonal translation. The best performing of these systems was based on the fusion of FUS/EWSR1 with KIF16B₁₋₄₀₀, **K2::P1** and exhibited a selectivity of eightfold (box in Fig. 2C). This was also directly obvious from the FFC data, in which the bright, mCherry-positive cell population was clearly retained, whereas GFP expression was minimal (arrow in Fig. 2D).

### EXAMPLE 3 - AFPs comprising a combination of APs including a membrane-targeting AP

AFPs comprising combinations of APs derived from phase separation polypeptides (PSPs), FUS and EWSR1 (also termed EWS herein) and an AP which acts as a membrane-targeting signal, LcK, were tested in a manner analogous to example 2. LcK is a cell membrane-targeting signal (Resh, Bba-Mol Cell Res 1999, 1451:1-16) that adds an amphipathic helix post translationally to the POI. For these experiments, the AFPs LcK::FUS::PyIRS and LcK::EWSR1::MCP were co-expressed in HE293T cells (see Fig. 3). Testing of this system with the same dual reporter resulted in a dramatic shift in the signal and a strong selectivity for the expression solely of the MS2-tagged mCherry compared to the control PylRS. See Fig. 4 and Fig. 5 showing a 26-fold selectivity gain as compared to the control. IF and FISH for MCP, PylRS and tRNA show a clear membrane signal with appearance of occasional droplet-like structures and a perfect co-localization of all the components.

Without wishing to be bound by theory it is assumed that targeting the OT system to a membrane results in a confinement of the components to a 2D surface (i.e. a film), offering an even higher spatial segregation than a cytoplasmic droplet. In accordance with such a cumulative effect of the two combined assembler strategies (LcK for membrane targeting, and FUS/EWSR1 for droplet generation), it was shown that the presence of the FUS/EWSR1 "assemblers" is not a requirement in an LcK-fused (and thus membrane-anchored system) for obtaining selective Amber suppression (data not shown). Nevertheless, the combination of the LcK-targeting with FUS/EWSR1 resulted in a higher selectivity of the system. Further, it was found that swapping the MS2-tag on the fluorescent reporters, yielded a swapped selectivity in the FFC data, underlining the selective (orthogonal) translation of the MS2-tagged mRNA.

### EXAMPLE 4 - Validation of the selectivity gain being specific to the interaction of the mRNA MS2-tag and MCP

To validate that the observed selectivity gain was specific to the interaction of the MCP segment with the MS2-tag of the mRNA, all the OT systems were characterized by expressing the RS assembler fusion of each OT system without MCP. As expected, no selective orthogonal translation of MS2-tagged mRNA was observed in those cases (data not shown). Additionally, a reporter inversion was performed by moving the MS2-tag from the mCherry to the GFP cassette in the dual-color reporter, which as expected inverted selectivity of the system towards dominant GFP expression (data not shown). This established that the OT systems acted selectively on the MS2-tagged RNA.

### EXAMPLE 5 - Introduction of multiple ncAAs into the same POI

GCE can also be used to introduce multiple ncAAs into the same POI (see, e.g., Liu et al., Annu Rev Biochem 2010, 79:413-444; Lemke, ChemBioChem 2014, 15:1691-1694; Chin, Nature 2017, 550;53-60). However, only very few publications report on more than one, that is, two- or three-codon suppression in the same protein in eukaryotes, as yields typically suffer compared to single-codon suppression (see Xiao et al., Angew Chem 2013, 52:14080-14083; Schmied et al., J Am Chem Soc 2014, 136:15577-15583; Zhang et al., Biochem Biophys Res Co 2017, 489:490-496). Notably, even dual- and triple-Amber proteins were still suppressed with the OT organelle (data not shown).

### EXAMPLE 6 - OT with 3-iodophenylalanine

To ensure that also other ncAAs can be translated by the OT assembly, another structurally different ncAA (3-iodophenylalanine) was tested which is a phenylalanine derivative instead of a lysine derivative (such as SCO) and is encoded by a different PylRS mutant (N346A, C348A) (see Wang et al., ACS Chem Biol 2013, 8:405-415). Consistent results were also observed for this system (Fig. 2C).

### EXAMPLE 7 - OT with different selector codons

As Opal and Ochre codons are highly abundant in eukaryotic genomes (52% Opal, 28% Ochre in the human genome), the Amber codon is by far the most used for GCE in eukaryotes. In addition, genomic approaches to orthogonal translation by removing those codons in the entire eukaryotic genome would be even more challenging then for the Amber codon and are currently beyond the state of the art. However, in the OT systems of the present invention, a simple mutation in the anticodon loop of the tRNA^{Pyl}, as well as in the respective codon in the MS2-tagged POI-encoding mRNA allows for orthogonal translation of those codons. FFC analysis revealed that the OT systems of the invention provide freedom of choice with respect to the stop (selector) codon (Fig. 2C, E). In fact, Opal suppression turned out to be the best performing system, showing an 11-fold selectivity increase. Ochre suppression still showed fivefold selectivity increase with 20% efficiency.

### EXAMPLE 8 - Orthogonal translation of proteins of various cellular compartments

To visualize the power of the OT^{K2::P1} system (the best performing Amber suppression OT system in terms of selectivity and efficiency) beyond "simple" reporters, it was intended to show differential expression of human nucleoporin 153 (Nup153) versus cytoskeletal vimentin. Nup153 locates to the nuclear pore complex and is more than 1500 amino acids long. Hence, its mRNA is approximately six-fold larger than those of the fluorescent protein reporters used above. For this experiment a previously described C-terminal GFP fusion, with an Amber mutation (Nup153::EGFP^{149TAG}) was used that gave rise to a characteristic nuclear envelope stain in confocal images only if Amber suppression was successful (see Nikic et al., Angew Chem 2016, 55:16172-16276). Said Nup153::EGFP^{149TAG} was now tagged at the mRNA level with an MS2-tag (nup153::egfp^{149TAG}::ms2) and co-expressed from the same plasmid with vimentin (a cytoskeletal protein) containing an Amber codon at position 116 fused to mOrange (Vim^{116TAG}::mOrange). Expression in HEK293T cells resulted in production of both proteins in the presence of the cytoplasmic PylRS showing the characteristic nuclear envelope and cytoskeletal staining, respectively. Using the OT^{K2::P1} assembly only Nup153::GFP was visible (selective nuclear rim stain in confocal images of the co-transfected HEK293T cells). Consistent results were also observed in COS-7 cells. Swapping the MS-tag to vimentin inverted the effect, so that only Vim^{116TAG}::mOrange was visible (observed for both COS-7 and HEK293T cell experiments). This showed that the OT^{K2::P1} worked for dramatically different mRNAs.

### EXAMPLE 9 - Orthogonal translation of transmembrane proteins

It was also shown that transmembrane proteins can be selectively expressed using the OT^{K2::P1} assembly. Membrane protein expression represents another layer of translational complexity, as ribosomes need to bind the endoplasmic reticulum during translation, where the proteins are co-translationally inserted into the membrane. In this experiment, a fusion of insulin receptor 1 with an Amber codon at position 676 with mOrange (INSR^{676TAG}::mOrange) was used, which locates to the plasma membrane and gives rise to a characteristic plasma membrane stain in HEK293T cells (see Nikic et al., Angew Chem 2014, 53:2245-2249). This construct was tagged with an MS2-tag in the 3' UTR and cloned with Nup153::EGFP^{149TAG} into one dual-cassette plasmid. Then the construct was expressed in HEK293T cells either in the presence of the cytoplasmic PylRS system or in the presence of the OT^{K2::P1} assembly. In the presence of the OT^{K2::P1} assembly, selective expression of the MS2-tagged protein and the expected plasma membrane localization of INSR^{676TAG}::mOrange were observed (data not shown), indicating the potential of the OT system of the present invention to participate in even more complex membrane-associated translational processes.

### EXAMPLE 10 - Spatial distribution of elements of the OT system in the cell

The spatial distribution of AFPs and particularly PylRS in cells was assessed using immunofluorescence (IF). Additionally, fluorescence *in situ* hybridization (FISH) was used for detecting tRNA^{Pyl}. In contrast to the dual color reporter used in the FFC experiments above, in all IF/FISH experiments a single color NLS-GFP^{39TAG} reporter that was fused to an MS2-tag (nls-gfp^{39TAG}::ms2) was used to identify cells active in Amber suppression (this yields a green nucleus if Amber suppression is successful and helped to optimize distinguishable color channels). IF and FISH stainings showed that in contrast to cytoplasmic PylRS, the **P1** system formed small, intracellular assembler::PylRS droplets (data not shown). This indicated the occurrence of phase separation. The tRNA^{Pyl} co-localized well with highly dispersed assembler::PylRS droplets, indicating that it could nicely partition into the assembler::PylRS phase. Additional stainings showed further co-localization with assembler::MCP (data hot shown). Compared to **P1,** the **P2** system showed larger but still multiple dispersed droplet-like structures (data not shown). With the combination of both assembler strategies **(K1::P1, K2::P1, K1::P2, K2::P2)** the formation of large micron-sized organelle-like structures in the cytoplasm was observed, these structures were in most cases localized to few or even a single position per cell. For the combined assemblers, mRNA::ms2, tRNA^{Pyl}, assembler::PylRS and assembler::MCP all co-localized to organelle-like structures. The combination of the two assembler strategies, that is, phase separation paired with spatial targeting by kinesin truncations, yielded the best confinement as determined by FISH and IF and the highest selectivity increase. This is consistent with the hypothesis that the higher spatial segregation and thus higher local concentration of the tRNA^{Pyl}, PylRS and mRNA correlates with higher selectivity.

Ribosomes were stained to see whether they co-localize to the OT^{K2::P1} assembly. IF staining of the ribosomal protein RPL26L1 revealed strong co-localization with the OT^{K2::P1} organelle (data not shown) demonstrating ribosome recruitment, tentatively due to binding to mRNA::ms2 during translation. High ribosomal mobility can also explain why it was possible to successfully express the membrane protein INSR (construct: INSR^{676TAG}::mOrange::ms2).

Without wishing to be bound by theory, the experimental results strongly suggests that selective orthogonal translation happens within close proximity of, potentially even inside, the OT assemblies, by a set of recruited ribosomes that are near or fully immersed into a concentrated pool of tRNA^{Pyl}. tRNA^{Pyl} itself is recruited to the OT^{K2::P1} assembly due to its affinity for assembler::PyIRS and can readily co-partition into the droplet to be aminoacylated with its cognate ncAA, while assembler::MCP recruits MS2-tagged mRNA. This in turn attracts ribosomes to co-partition into the dense phase formed by the dual assembler system (K2::P1 = KIF16B::FUS::PylRS and KIF16B::EWSR1::MCP), which maintains access to other translation factors for translation to function. Ribosomes elsewhere in the cytoplasm that are not exposed to tRNA^{Pyl} perform their canonical function to terminate translation whenever they encounter a stop codon.

### EXAMPLE 11 - Further OT systems

In addition to the OT systems described in the preceding examples, a variety of other OT systems were tested and found to allow for selective orthogonal translation of the reporter (i.e. the POI). A summary of these experiments is shown in the Table 1 below. Unless noted otherwise, the cytoplasmic NES-PyIRS system as previously described by Nikic et al. (Angew Chem Int Ed Engl 2016, 55(52):16172-16176) but with the corresponding AF, AA or AAAF mutations was used as a nonspecific reference (negative control). All experiments were performed in presence of the codon-specific tRNA^{Pyl} and PylRS mutant corresponding ncAAs.

**Table 1: Tested OT systems**

| **Fusion protein(s) comprising O-RS and RNA-TP segments** | **Reporter (POI)** | **cell lines** |
|---|---|---|
| EWSR1-MCP•FUS-PylRS^{AF} | GFP^{39TAG}-2xMS2•mCherry^{185TAG} | HEK293T/COS-7 |
| | mCherry^{185TAG}-2xMS2•GFP^{39TAG} | |
| FUS-MCP•FUS-PylRS^{AF} | mCherry^{185TAG}-2xMS2•GFP^{39TAG} | HEK293T |
| FUS-MCP-PylRS^{AF} | GFP^{39TAG}-2xMS2•mCherry^{185TAG} | HEK293T/COS-7 |
| | mCherry^{185TAG}-2xMS2•GFP^{39TAG} | |
| MCP-PylRS^{AF} | GFP^{39TAG}-2xMS2•mCherry^{185TAG} | HEK293T/COS-7 |
| | mCherry^{185TAG}-2xMS2•GFP^{39TAG} | |
| SPD5-MCP•SPD5-PylRS^{AF} | GFP^{39TAG}-2xMS2•mCherry^{185TAG} | HEK293T |
| | mCherry^{185TAG}-2xMS2•GFP^{39TAG} | |
| SPD5-MCP-PylRS^{AF} | GFP^{39TAG}-2xMS2•mCherry^{185TAG} | HEK293T |
| | mCherry^{185TAG}-2xMS2•GFP^{39TAG} | |
| KIF16B-PylRS^{AF}•KIF16B-MCP | GFP^{39TAG}-2xMS2•mCherry^{185TAG} | HEK293T/COS-7 |
| | mCherry^{185TAG}-2xMS2•GFP^{39TAG} | |
| KIF16B-MCP-PylRS^{AF} | GFP^{39TAG}-2xMS2•mCherry^{185TAG} | HEK293T/COS-7 |
| | mCherry^{185TAG}-2xMS2•GFP^{39TAG} | |
| KIF16B-FUS-PylRS^{AF}•KIF16B-EWSR1-MCP | GFP^{39TAG}-2xMS2•mCherry^{185TAG} | HEK293T/ COS-7 |
| | mCherry ^{185TAG} -2xMS2•GFP^{39TAG} | |
| | mCherry ^{185TAA}-2xMS2•GFP^{39TAA} | |
| | mCherry^{185TGA}-2xMS2•GFP^{39TGA} | |
| | Nup153-EGFP^{149TAG}-2xMS2•Vimentin ^{116TAG}-mOrange | |
| | Vimentin^{116TAG}-mOrange-2xMS2•Nup153-EGFP^{149TAG} | |
| | I NSR^{676TAG}-EGFP-2xMS2•Nup153-EGFP^{149TAG} | |
| | I NSR^{676TAG}-mOrange-2xMS2•Nup153-EGFP^{149TAG} | |
| | I NSR^{676TAG}-I NSR-2xMS2•Nup1 53-EGFP^{149TAG} | |
| KIF16B-SPD5-PylRS^{AF}•KIF16B-SPD5-MCP | GFP^{39TAG}-2xMS2•mCherry^{185TAG} | HEK293T |
| | mCherry^{185TAG}-2xMS2•GFP^{39TAG} | |
| KIF16B-FUS-PylRS^{AA}•KIF16B-EWSR1-MCP | mCherry^{185TAG}-2xMS2•GFP^{39TAG} | HEK293T |
| KIF16B-FUS-PylRS^{AAAF}•KIF16B-EWSR1-MCP | mCherry^{185TAG}-2xMS2•GFP^{39TAG} | HEK293T |
| KIF13A-PylRS^{AF}•KIF13A-MCP | mCherry^{185TAG}-2xMS2•GFP^{39TAG} | HEK293T |
| KIF13A-FUS-PylRS^{AF}•KIF13A-EWSR1-MCP | mCherry^{185TAG}-2xMS2•GFP^{39TAG} | HEK293T |
| | GFP^{39TAG}-2xMS2•mCherry^{185TAG} | |
| | mCherry ^{185TAG} -4xBoxB•GFP^{39TAG}-2xMS2 | |
| | mCherry ^{185TAA}-2xMS2•GFP^{39TAA} | |
| | mCherry^{185TGA}-2xMS2•GFP^{39TGA} | |
| KIF13A-SPD5-PylRS^{AF}•KIF13A-SPD5-MCP | mCherry^{185TAG}-2xMS2•GFP^{39TAG} | HEK293T |
| KIF13A-FUS-PylRS^{AA}•KIF13A-EWSR1-MCP | mCherry^{185TAG}-2xMS2•GFP^{39TAG} | HEK293T |
| KI F13A-FUS-PylRS^{AAAF} •KIF13A-EWSR1-MCP | mCherry^{185TAG}-2xMS2•GFP^{39TAG} | HEK293T |
| LCK-FUS-PylRS^{AF}•LCK-EWSR1-MCP | GFP^{39TAG}-2xMS2•mCherry^{185TAG} | HEK293T/ COS-7 |
| | mCherry^{185TAG}-2xMS2•GFP^{39TAG} | |
| | mCherry ^{185TAG} -4xBoxB•GFP^{39TAG}-2xMS2 | |
| | mCherry ^{185TAA}-2xMS2•GFP^{39TAA} | |
| | mCherry^{185TGA}-2xMS2•GFP^{39TGA} | |
| | Nup153-EGFP^{149TAG}-2xMS2•Vimentin ^{116TAG}-mOrange | |
| | Vimentin^{116TAG}-mOrange-2xMS2•Nup153-EGFP^{149TAG} | |
| LCK-FUS-MCP-PylRS^{AF} | mCherry^{185TAG}-2xMS2•GFP^{39TAG} | HEK293T |
| | mCherry ^{185TAG} -4xBoxB•GFP^{39TAG}-2xMS2 | |
| LCK-FUS-PylRS^{AAAF}•LCK-EWSR1-MCP | mCherry^{185TAG}-2xMS2•GFP^{39TAG} | HEK293T |
| | mCherry^{185TAG}-4xBoxB•GFP^{39TAG}-2xMS2 | |
| LCK-FUS-PylRS^{AF}•LCK-EWSR1-4xλ_{N22} | mcherry^{185TAG}-4xBoxB•GFP^{39TAG} | HEK293T |
| | mCherry^{185TAG}-4xBoxB•GFP^{39TAG}-2xMS2 | |
| LCK-PylRS^{AF}•LCK-MCP | GFP^{39TAG}-2xMS2•mCherry^{185TAG} | HEK293T/ COS-7 |
| | mCherry^{185TAG}-2xMS2•GFP^{39TAG} | |
| | mCherry ^{185TAG} -4xBoxB•GFP^{39TAG}-2xMS2 | |
| LCK-PyIRS^{AAAF}•LCK-MCP | mCherry^{185TAG}-2xMS2•GFP^{39TAG} | HEK293T |
| | mCherry ^{185TAG} -4xBoxB•GFP^{39TAG}-2xMS2 | |
| LCK-FUS-3xMCP-PyIRS^{AF} | mCherry^{185TAG}-2xMS2•GFP^{39TAG} | HEK293T |
| | mCherry ^{185TAG} -4xBoxB•GFP^{39TAG}-2xMS2 | |
| TOM20-FUS-PylRS^{AF}•TOM20-EWSR1-MCP | mCherry^{185TAG}-2xMS2•GFP^{39TAG} | HEK293T |
| | mCherry^{185TAG}-4xBoxB•GFP^{39TAG}-2xMS2 | |
| TOM20-FUS-PylRS^{AF}•TOM20-EWSR1-4xλ_{N22} | mCherry^{185TAG}-4xBoxB•GFP^{39TAG} | HEK293T/ COS-7 |
| | mCherry^{185TAG}-4xBoxB•GFP^{39TAG}-2xMS2 | |
| TOM20-FUS-PylRS^{AA}•TOM20-EWSR1-MCP | mCherry^{185TAG}-2xMS2•GFP^{39TAG} | HEK293T |
| TOM20-FUS-PylRS^{AA}•TOM20-EWSR1-4xλ_{N22} | mCherry^{185TAG}-4xBoxB•GFP^{39TAG} | HEK293T |
| TOM20-FUS-PylRS^{AAAF}•TOM20-EWSR1-MCP | mCherry^{185TAG}-2xMS2•GFP^{39TAG} | HEK293T |
| TOM20-FUS-PylRS^{AAAF} •TOM20-EWSR1-4xλ_{N22} | mcherry^{185TAG}-4xBoxB•GFP^{39TAG} | HEK293T |
| | mcherry^{185TAG}-4xBoxB•GFP^{39TAG}-2xMS2 | |
| TOM20-3xMCP-PylRS^{AF} | mCherry^{185TAG}-2xMS2•GFP^{39TAG} | HEK293T |
| | mCherry ^{185TAG} -4xBoxB•GFP^{39TAG}-2xMS2 | |
| TOM20-FUS-3xMCP-PylRS^{AF} | mCherry^{185TAG}-2xMS2•GFP^{39TAG} | HEK293T |
| TOM20-FUS-3xMCP-PylRS^{AAAF} | mCherry^{185TAG}-2xMS2•GFP^{39TAG} | HEK293T |
| | mCherry ^{185TAG} -4xBoxB•GFP^{39TAG}-2xMS2 | |
| TOM20-FUS-4xλ_{N22}-PylRS^{AF} | mCherry^{185TAG}-4xBoxB•GFP^{39TAG}-2xMS2 | HEK293T/ COS-7 |
| TOM20-FUS-4xλ_{N22}-PylRS^{AAAF} | mCherry^{185TAG}-4xBoxB•GFP^{39TAG}-2xMS2 | HEK293T |
| KIF16B-FUS-4xλ_{N22}-PylRS^{AF} | mcherry^{185TAG}-4xBoxB•GFP^{39TAG} | HEK293T |
| | mCherry^{185TAG}-4xBoxB•GFP^{39TAG}-2xMS2 | |
| KIF16B-SPD5-4xλ_{N22}-PylRS^{AF} | mcherry^{185TAG}-4xBoxB•GFP^{39TAG} | HEK293T |
| KIF16B-SPD5-MCP-PyIRS^{AF} | mCherry^{185TAG}-2xMS2•GFP^{39TAG} | HEK293T |
| FRB-CD28-FUS-PylRS^{AA}•FRB-CD28-EWSR1-MCP | mCherry^{185TAG}-2xMS2•GFP^{39TAG} | HEK293T |
| FRB-CD28-FUS-PylRS^{AA}•FRB-CD28-EWSR1-4xλ_{N22} | mcherry^{185TAG}-4xBoxB•GFP^{39TAG} | HEK293T |
| FRB-CD28-FUS-PylRS^{AF}•FRB-CD28-EWSR1-MCP | mCherry^{185TAG}-2xMS2•GFP^{39TAG} | HEK293T |
| FRB-CD28-FUS-PylRS^{AF}•FRB-CD28-EWSR1-4xλ_{N22} | mcherry^{185TAG}-4xBoxB•GFP^{39TAG} | HEK293T |
| FUS-CD28-FUS-PylRS^{AA}•FUS-CD28-EWSR1-MCP | mCherry^{185TAG}-2xMS2•GFP^{39TAG} | HEK293T |
| | mCherry^{185TAG}-4xBoxB•GFP^{39TAG}-2xMS2 | |
| FUS-CD28-FUS-PylRS^{AA}•FUS-CD28-EWSR1-4xλ_{N22} | mcherry^{185TAG}-4xBoxB•GFP^{39TAG} | HEK293T |
| | mCherry^{185TAG}-4xBoxB•GFP^{39TAG}-2xMS2 | |
| FUS-CD28-FUS-PylRS^{AF}•FUS-CD28-EWSR1-MCP | mCherry^{185TAG}-2xMS2•GFP^{39TAG} | HEK293T |
| FUS-CD28-FUS-PylRS^{AF}•FUS-CD28-EWSR1-4xλ_{N22} | mcherry^{185TAG}-4xBoxB•GFP^{39TAG} | HEK293T |
| FRB-CD28-FUS-MCP-PylRS^{AA} | mCherry^{185TAG}-2xMS2•GFP^{39TAG} | HEK293T |
| | mCherry ^{185TAG} -4xBoxB•GFP^{39TAG}-2xMS2 | |
| FRB-CD28-FUS-MCP-PylRS^{AF} | mCherry^{185TAG}-2xMS2•GFP^{39TAG} | HEK293T |
| | mCherry ^{185TAG} -4xBoxB•GFP^{39TAG}-2xMS2 | |
| FUS-CD28-FUS-MCP-PylRS^{AF} | mCherry^{185TAG}-2xMS2•GFP^{39TAG} | HEK293T |
| | mCherry ^{185TAG} -4xBoxB•GFP^{39TAG}-2xMS2 | |
| FUS-CD28-FUS-MCP-PylRS^{AA} | mCherry^{185TAG}-2xMS2•GFP^{39TAG} | HEK293T |
| KIF16B-FUS-MCP-PylRS^{AF} | mCherry^{185TAG}-2xMS2•GFP^{39TAG} | HEK293T/COS-7 |
| | mCherry^{185TAG}-4xBoxB•GFP^{39TAG}-2xMS2 | |
| | GFP^{39TAG}-2xMS2•mCherry^{185TAG} | |
| KIF13A-FUS-MCP-PylRS^{AF} | mCherry^{185TAG}-2xMS2•GFP^{39TAG} | HEK293T |
| TOM20-FUS-V5-PylRS^{AF}•TOM20-EWSR1-HA-MCP | mCherry^{185TAG}-2xMS2•GFP^{39TAG} | HEK293T |
| TOM20-FUS-V5-PylRS^{AF}•TOM20-EWSR1-Myc-4xλ_{N22} | mCherry^{185TAG}-4xBoxB•GFP^{39TAG} | HEK293T |
| TOM20-FUS-V5-PylRS^{AA}•TOM20-EWSR1-HA-MCP | mCherry^{185TAG}-2xMS2•GFP^{39TAG} | HEK293T |
| TOM20-FUS-V5-PylRS^{AA}•TOM20-EWSR1-Myc-4xλ_{N22} | mCherry^{185TAG}-4xBoxB•GFP^{39TAG} | HEK293T |
| TOM20-FUS-V5-PylRS^{AAAF}•TOM20-EWSR1-HA-MCP | mCherry^{185TAG}-2xMS2•GFP^{39TAG} | HEK293T |
| TOM20-FUS-V5-PylRS^{AAAF}•TOM20-EWSR1-Myc-4xλ_{N22} | mCherry^{185TAG}-4xBoxB•GFP^{39TAG} | HEK293T |
| KIF16B-VSV-G-FUS-PylRS^{AF}•KIF16B-EWSR1-MCP | mCherry^{185TAG}-2xMS2•GFP^{39TAG} | HEK293T |

### ABBREVIATIONS

- AP: polypeptide segment acting as assembler
- AFP: assembler fusion protein
- BSA: bovine serum albumin
- BoxB: lambda phase RNA stem-loop, specific binding site of λ_{N22}
- CDS: (en-)coding sequence
- EGFP^{149TAG}: enhanced green fluorescent protein, amino acid position 149 encoded by Amber codon (TAG)
- EP: polypeptide segment acting as an effector
- EWSR1: Ewing sarcoma breakpoint region 1 (also termed EWS herein)
- FBS: fetal bovine serum
- FFC: fluorescence flow cytometry
- FISH: fluorescence in situ hybridization
- FRB-CD28: synthetic membrane targeting domain derived from transmembrane proteins CD4, FRB (similar to mTOR) and CD28
- FSC-A: forward scatter area
- FUS: fused-in sarcoma
- FUS-CD28: (synthetic membrane targeting fusion polypeptide derived from CD4, FUS and CD28
- GCE: genetic code expansion
- GFP: green fluorescent protein
- GFP^{39TAA}: green fluorescent protein, amino acid position 39 encoded by Ochre codon (TAA)
- GFP^{39TAG}: green fluorescent protein, amino acid position 39 encoded by Amber codon (TAG)
- GFP^{39TGA}: green fluorescent protein, amino acid position 39 encoded by Opal codon (TGA)
- GFP^{39,149TAG}: green fluorescent protein, each of amino acid positions 39 and 149 encoded by Amber codon (TAG)
- GFP^{39,149,182TAG}: green fluorescent protein, each of amino acid positions 39, 149 and 182 encoded by Amber codon (TAG)
- IC-TP: intracellular targeting polypeptide
- IDP: intrinsically disordered protein
- INSR: insulin receptor
- INSR^{676TAG}: insulin receptor, amino acid position 676 encoded by Amber codon (TAG)
- iRFP: near-infrared fluorescent protein
- KIF13A: kinesin family member 13A - Unless specified otherwise herein, "KIF13A" specifically refers to the fragment covering amino acid residues 1-411 of KIF13A wherein P390 is deleted (KIF13A_{1-411,ΔP390}).
- KIF16B: kinesin family member 16B - Unless specified otherwise herein, "KIF16B" specifically refers to the fragment covering amino acid residues 1-400 of KIF16B (KIF16B₁₋₄₀₀).
- λ_{N22}: 22 amino acid RNA-binding domain of lambda phage antiterminator protein N
- LcK: posttranslational modification site for plasma membrane targeting of lymphocyte-specific protein tyrosine kinase
- mCherry^{185TAG}: mCherry, amino acid position 185 encoded by Amber codon (TAG)
- MCP: MS2 bacteriophage coat protein
- MLC: membrane-less compartment
- MS2: Enterobacteria phage MS2
- MS2-tag: two MS2 RNA stem-loops fused to the 3' untranslated region of the mRNA (or coding sequence therefor)
- ms2: MS2-tag
- ncAA: non-canonical amino acid
- NLS: nuclear localization sequence
- Nup153: nucleoporin 153
- O-RS: orthogonal aminoacyl tRNA synthetase
- OT: assembly spacially enriched components of the GCE machinery in a membrane-less assembly that is able to act as an artificial orthogonally translating (OT) organelle
- PBS: phosphate buffered saline
- PEI: polyethylenimine
- POI: polypeptide of interest (= target polypeptide)
- POI^{TAG}: POI comprising an Amber-(TAG-)encoded amino acid residue (or coding sequence therefor)
- PSP: phase separation polypeptide
- PylRS: pyrrolysyl tRNA synthetase
- PylRS^{AA}: mutant *M. mazei* pyrrolysyl tRNA synthetase comprising amino acid substitutions N346A and C348A
- PylRS^{AF}: mutant *M. mazei* pyrrolysyl tRNA synthetase comprising amino acid substitutions Y306A and Y384F
- PylRS^{AAAF}: mutant *M. mazei* pyrrolysyl tRNA synthetase comprising amino acid substitutions Y306A, N346A, C348A and Y384F
- RNA-TP: RNA targeting polypeptide
- RS: aminoacyl tRNA synthetase
- RT: room temperature
- SCO: cyclooctyne lysine
- SEM: standard error of the mean
- SSC: saline-sodium citrate (buffer)
- SSC-A: side scatter area
- SSC-W: side scatter width
- SPD5: spindle-defective protein 5
- TOMM20: translocase of outer mitochondrial membrane 20
- TOMM20₁₋₇₀: fragment covering amino acid residues 1-70 of TOMM20
- tRNA^{Pyl}: tRNA that is coupled to pyrrolysyl or another non-canonical amino acid residue by a wild-type or modified PylRS and has an anticodon that, for site-specific incorporation of a (non-canonical) amino acid residue into a POI, is preferably the reverse complement of a selector codon. - The tRNA^{Pyl} used in the examples carried the anticodon against the stop codon Amber (tRNA^{Pyl,CUA}), Ochre (tRNA^{Pyl,UUA}) or Opal (tRNA^{Pyl,UCA}), depending on which of these was used as selector codon in the POI-encoding sequence.
- 3' UTR 3': untranslated region
- Vim^{116TAG}: Vimentin, amino acid position 116 encoded by Amber codon (TAG)

### SEQUENCES

The following shows the sequences of the polypeptides and polynucleotides described herein.

### 1. Hybridization probes

Hybridization probe for tRNA^{Pyl} labelled at the 5' terminus with Cy5 CTAACCCGGCTGAACGGATTTAGAGTCCATTCGATC (SEQ ID NO:1)
Hybridization probe for tRNA^{Pyl} labelled at the 5' terminus with digoxigenin CTAACCCGGCTGAACGGATTTAGAGTCCATTCGATC (SEQ ID NO:2)
Hybridization probe for the MS2 RNA stem-loop sequence labelled at the 5' terminus with Alexa Fluor 647 CTGCAGACATGGGTGATCCTCATGTTTTCTA (SEQ ID NO:3)

### 2. tRNAs

DNA sequence for tRNA^{Pyl,CUA} (pyrrolysyl tRNA of *Methanosarcina mazei* for Amber codon; anticodon underlined) 
GGAAACCTGATCATGTAGATCGAATGGACTCTAAATCCGTTCAGCCGGGTTAGATTCCC
GGGGTTTCCG (SEQ ID NO:4)
DNA sequence for tRNA^{Pyl,UCA} (pyrrolysyl tRNA of *Methanosarcina mazei* for Opal codon; anticodon underlined) 
GGAAACCTGATCATGTAGATCGAATGGACTTCAAATCCGTTCAGCCGGGTTAGATTCCC
GGGGTTTCCG (SEQ ID NO:5)
DNA sequence for tRNA^{Pyl,UUA} (pyrrolysyl tRNA of *Methanosarcina mazei* for Ochre codon; anticodon underlined)

GGAAACCTGATCATGTAGATCGAATGGACTTTAAATCCGTTCAGCCGGGTTAGATTCCC
GGGGTTTCCG (SEQ ID NO:6)

### 3. O-RSs

PyIRS^{AF} (*Methanosarcina mazei* pyrrolysyl tRNA synthetase double mutant: Y306A, Y384F; Uniprot: Q8PWY1)
DNA: 
ATGGCGTGCCCGGTGCCGCTGCAGCTGCCGCCGCTGGAACGCCTGACCCTGGATGAT
AAAAAACCGCTGAATACCCTGATCTCTGCTACTGGTCTGTGGATGAGTCGTACCGGAAC
CATTCATAAAATCAAACACCACGAGGTTAGCCGTTCGAAAATCTATATTGAGATGGCGTG
TGGCGATCATCTGGTTGTGAACAATAGCCGCTCTTCTCGTACAGCACGTGCACTGCGTC
ACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCC
TACCCGTACTAAAAAAGCAATGCCGAAATCCGTTGCTCGTGCCCCTAAACCACTGGAAA
ACACTGAAGCAGCACAGGCACAGCCGTCTGGAAGCAAATTCTCTCCGGCCATTCCTGTT
TCTACCCAGGAGTCCGTTTCTGTTCCAGCAAGTGTGAGCACCAGCATTAGCAGTATTAG
CACCGGTGCCACCGCTAGCGCCCTGGTTAAAGGCAATACCAATCCGATTACAAGCATGT
 
CTGCCCCGGTTCAAGCATCAGCTCCAGCACTGACAAAATCCCAAACCGATCGTCTGGA
GGTTCTGCTGAATCCGAAAGACGAAATCAGCCTGAATTCCGGCAAACCGTTTCGTGAAC
TGGAGAGCGAACTGCTGTCACGTCGTAAAAAGACCTGCAACAAATCTATGCCGAAGAA
CGTGAGAACTATCTGGGGAAACTGGAACGTGAAATCACCCGCTTTTTCGTGGATCGTGG
CTTTCTGGAGATCAAATCCCCGATTCTGATTCCTCTGGAGTATATCGAGCGTATGGGCA
TCGACAATGATACCGAACTGAGCAAACAAATTTTCCGTGTGGATAAAAACTTCTGTCTGC
GCCCTATGCTAGCACCAAATCTGGCTAACTATCTGCGCAAACTGGACCGTGCCCTGCCT
GATCCTATCAAAATCTTCGAGATCGGCCCGTGTTATCGTAAAGAGTCCGACGGTAAAGA
ACATCTGGAGGAGTTTACCATGCTGAACTTTTGCCAAATGGGTTCAGGTTGTACTCGTG
AGAACCTGGAAAGCATCATCACCGATTTTCTGAACCACCTGGGCATTGACTTCAAAATTG
TGGGCGACAGCTGTATGGTGTTTGGCGACACCCTGGATGTCATGCACGGCGACCTGGA
ACTGTCTAGTGCCGTTGTGGGCCCAATCCCGCTGGATCGTGAGTGGGGTATCGACAAA
CCTTGGATCGGTGCGGGTTTTGGTCTGGAGCGTCTGCTGAAAGTAAAACACGACTTCAA
GAACATCAAACGTGCTGCACGTTCCGAGTCCTATTACAATGGTATTTCTACTAACCTGTA
A (SEQ ID NO:7)
Protein:

MACPVPLQLPPLERLTLDDKKPLNTLISATGLWMSRTGTIHKIKHHEVSRSKIYIEMACGDHL
VVNNSRSSRTARALRHHKYRKTCKRCRVSDEDLNKFLTKANEDQTSVKVKVVSAPTRTKKA
MPKSVARAPKPLENTEAAQAQPSGSKFSPAIPVSTQESVSVPASVSTSISSISTGATASALVK
GNTNPITSMSAPVQASAPALTKSQTDRLEVLLNPKDEISLNSGKPFRELESELLSRRKKDLQ
QIYAEERENYLGKLEREITRFFVDRGFLEIKSPILIPLEYIERMGIDNDTELSKQIFRVDKNFCL
RPMLAPNLANYLRKLDRALPDPIKIFEIGPCYRKESDGKEHLEEFTMLNFCQMGSGCTRENL
ESIITDFLNHLGIDFKIVGDSCMVFGDTLDVMHGDLELSSAVVGPIPLDREWGIDKPWIGAGF
GLERLLKVKHDFKNIKRAARSESYYNGISTNL (SEQ ID NO:8)

PyIRS^{AA} (*Methanosarcina mazei* pyrrolysyl tRNA synthetase double mutant: N346A, C348A; Uniprot: Q8PWY1)
DNA: 
ATGGCGTGCCCGGTGCCGCTGCAGCTGCCGCCGCTGGAACGCCTGACCCTGGATGAC
AAAAAACCGCTGAATACCCTGATCTCTGCTACTGGTCTGTGGATGAGTCGTACCGGAAC
CATTCATAAAATCAAACACCACGAGGTTAGCCGTTCGAAAATCTATATTGAGATGGCGTG
TGGCGATCATCTGGTTGTGAACAATAGCCGCTCTTCTCGTACAGCACGTGCACTGCGTC
ACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCC
TACCCGTACTAAAAAAGCAATGCCGAAATCCGTTGCTCGTGCCCCTAAACCACTGGAAA
ACACTGAAGCAGCACAGGCACAGCCGTCTGGAAGCAAATTCTCTCCGGCCATTCCTGTT
 
TCTACCCAGGAGTCCGTTTCTGTTCCAGCAAGTGTGAGCACCAGCATTAGCAGTATTAG
CACCGGTGCCACCGCTAGCGCCCTGGTTAAAGGCAATACCAATCCGATTACAAGCATGT
CTGCCCCGGTTCAAGCATCAGCTCCAGCACTGACAAAATCCCAAACCGATCGTCTGGA
GGTTCTGCTGAATCCGAAAGACGAAATCAGCCTGAATTCCGGCAAACCGTTTCGTGAAC
TGGAGAGCGAACTGCTGTCACGTCGTAAAAAGACCTGCAACAAATCTATGCCGAAGAA
CGTGAGAACTATCTGGGGAAACTGGAACGTGAAATCACCCGCTTTTTCGTGGATCGTGG
CTTTCTGGAGATCAAATCCCCGATTCTGATTCCTCTGGAGTATATCGAGCGTATGGGCA
TCGACAATGATACCGAACTGAGCAAACAAATTTTCCGTGTGGATAAAAACTTCTGTCTGC
GCCCTATGCTGGCACCAAATCTGTATAACTATCTGCGCAAACTGGACCGTGCCCTGCCT
GATCCTATCAAAATCTTCGAGATCGGCCCGTGTTATCGTAAAGAGTCCGACGGTAAAGA
ACATCTGGAGGAGTTTACCATGCTGGCCTTTGCCCAAATGGGTTCAGGTTGTACTCGTG
AGAACCTGGAAAGCATCATCACCGATTTTCTGAACCACCTGGGCATTGACTTCAAAATTG
TGGGCGACAGCTGTATGGTGTATGGCGACACCCTGGATGTCATGCACGGCGACCTGGA
ACTGTCTAGTGCCGTTGTTGGACCAATTCCGCTGGACCGTGAGTGGGGTATCGACAAA
CCGTGGATCGGAGCAGGATTCGGTCTGGAACGCCTGCTGAAAGTGAAACACGACTTCA
AAAACATCAAACGTGCCGCCCGTTCTGAATCGTATTATAACGGGATCTCTACGAACCTG
TAA (SEQ ID NO:9)
Protein:

MACPVPLQLPPLERLTLDDKKPLNTLISATGLWMSRTGTIHKIKHHEVSRSKIYIEMACGDHL
VVNNSRSSRTARALRHHKYRKTCKRCRVSDEDLNKFLTKANEDQTSVKVKVVSAPTRTKKA
MPKSVARAPKPLENTEAAQAQPSGSKFSPAIPVSTQESVSVPASVSTSISSISTGATASALVK
GNTNPITSMSAPVQASAPALTKSQTDRLEVLLNPKDEISLNSGKPFRELESELLSRRKKDLQ
QIYAEERENYLGKLEREITRFFVDRGFLEIKSPILIPLEYIERMGIDNDTELSKQIFRVDKNFCL
RPMLAPNLYNYLRKLDRALPDPIKIFEIGPCYRKESDGKEHLEEFTMLAFAQMGSGCTRENL
ESIITDFLNHLGIDFKIVGDSCMVYGDTLDVMHGDLELSSAVVGPIPLDREWGIDKPWIGAGF
GLERLLKVKHDFKNIKRAARSESYYNGISTNL (SEQ ID NO:10)

PyIRS^{AAAF} (*Methanosarcina mazei* pyrrolysyl tRNA synthetase quadruple mutant: Y306A, N346A, C348A, Y384F; Uniprot: Q8PWY1)
DNA: 
GCGTGCCCGGTGCCGCTGCAGCTGCCGCCGCTGGAACGCCTGACCCTGGATGATAAA
AAACCGCTGAATACCCTGATCTCTGCTACTGGTCTGTGGATGAGTCGTACCGGAACCAT
TCATAAAATCAAACACCACGAGGTTAGCCGTTCGAAAATCTATATTGAGATGGCGTGTG
GCGATCATCTGGTTGTGAACAATAGCCGCTCTTCTCGTACAGCACGTGCACTGCGTCAC
CACAAATATCGTAAAACCTGTAAACGTTGCCGTGTGTCCGATGAGGATCTGAACAAATTC
CTGACAAAAGCCAATGAGGACCAAACAAGCGTGAAAGTGAAAGTCGTTAGCGCTCCTAC
 
CCGTACTAAAAAGCAATGCCGAAATCCGTTGCTCGTGCCCCTAAACCACTGGAAAACA
CTGAAGCAGCACAGGCACAGCCGTCTGGAAGCAAATTCTCCGGCCATTCCTGTTTCT
ACCCAGGAGTCCGTTTCTGTTCCAGCAAGTGTGAGCACCAGCATTAGCAGTATTAGCAC
CGGTGCCACCGCTAGCGCCCTGGTTAAAGGCAATACCAATCCGATTACAAGCATGTCTG
CCCCGGTTCAAGCATCAGCTCCAGCACTGACAAAATCCCAAACCGATCGTCTGGAGGTT
CTGCTGAATCCGAAAGACGAAATCAGCCTGAATTCCGGCAAACCGTTTCGTGAACTGGA
GAGCGAACTGCTGTCACGTCGTAAAAAGACCTGCAACAAATCTATGCCGAAGAACGTG
AGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGA
CAATGATACCGAACTGAGCAAACAAATTTTCCGTGTGGATAAAAACTTCTGTCTGCGCCC
TATGCTAGCACCAAATCTGGCTAACTATCTGCGCAAACTGGACCGTGCCCTGCCTGATC
CTATCAAAATCTTCGAGATCGGCCCGTGTTATCGTAAAGAGTCCGACGGTAAAGAACAT
CTGGAGGAGTTTACCATGCTGGCCTTTGCCCAAATGGGTTCAGGTTGTACTCGTGAGAA
CCTGGAAAGCATCATCACCGATTTTCTGAACCACCTGGGCATTGACTTCAAAATTGTGG
GCGACAGCTGTATGGTGTTTGGCGACACCCTGGATGTCATGCACGGCGACCTGGAACT
GTCTAGTGCCGTTGTGGGCCCAATCCCGCTGGATCGTGAGTGGGGTATCGACAAACCT
TGGATCGGTGCGGGTTTTGGTCTGGAGCGTCTGCTGAAAGTAAAACACGACTTCAAGAA
CATCAAACGTGCTGCACGTTCCGAGTCCTATTACAATGGTATTTCTACTAACCTGTAA
(SEQ ID NO:11)
Protein:

ACPVPLQLPPLERLTLDDKKPLNTLISATGLWMSRTGTIHKIKHHEVSRSKIYIEMACGDHLV
VNNSRSSRTARALRHHKYRKTCKRCRVSDEDLNKFLTKANEDQTSVKVKVVSAPTRTKKA
MPKSVARAPKPLENTEAAQAQPSGSKFSPAIPVSTQESVSVPASVSTSISSISTGATASALVK
GNTNPITSMSAPVQASAPALTKSQTDRLEVLLNPKDEISLNSGKPFRELESELLSRRKKDLQ
QIYAEERENYLGKLEREITRFFVDRGFLEIKSPILIPLEYIERMGIDNDTELSKQIFRVDKNFCL
RPMLAPNLANYLRKLDRALPDPIKIFEIGPCYRKESDGKEHLEEFTMLAFAQMGSGCTRENL
ESIITDFLNHLGIDFKIVGDSCMVFGDTLDVMHGDLELSSAVVGPIPLDREWGIDKPWIGAGF
GLERLLKVKHDFKNIKRAARSESYYNGISTNL (SEQ ID NO:12)

### 4. RNA-TPs

MCP (coat protein of Enterobacteria phage MS2)
DNA: 
GCTTCTAACTTTACTCAGTTCGTTCTCGTCGACAATGGCGGAACTGGCGACGTGACTGT
CGCCCCAAGCAACTTCGCTAACGGGATCGCTGAATGGATCAGCTCTAACTCGCGTTCAC
AGGCTTACAAAGTAACCTGTAGCGTTCGTCAGAGCTCTGCGCAGAATCGCAAATACACC
 
ATCAAAGTCGAGGTGCCTAAAGGCGCCTGGCGTTCGTACTTAAATATGGAACTAACCAT
TCCAATTTTCGCCACGAATTCCGACTGCGAGCTTATTGTTAAGGCAATGCAAGGTCTCCT
AAAAGATGGAAACCCGATTCCCTCAGCAATCGCAGCAAACTCCGGCATCTAC (SEQ ID
NO:13)
Protein:

ASNFTQFVLVDNGGTGDVTVAPSNFANGIAEWISSNSRSQAYKVTCSVRQSSAQNRKYTIK
VEVPKGAWRSYLNMELTIPIFATNSDCELIVKAMQGLLKDGNPIPSAIAANSGIY (SEQ ID
NO:14)

λ_{N22} (22 amino acid RNA-binding domain of lambda phage antiterminator protein N)
DNA: 
ATGGACGCACAAACACGACGACGTGAGCGTCGCGCTGAGAAACAAGCTCAATGGAAAG
CTGCAAAC (SEQ ID NO:15)
Protein: MDAQTRRRERRAEKQAQWKAAN (SEQ ID NO:16)

### 5. TNs

DNA sequence of *Enterobacteria phage* MS2 RNA stem-loop ACATGAGGATCACCCATGT (SEQ ID NO:17)
DNA sequence of BoxB (lambda phase RNA stem-loop, specific binding site of λ_{N22}) GCCCTGAAAAAGGGC (SEQ ID NO:18)

### 6. IC-TPs

KIF16B₁₋₄₀₀ (*Homo sapiens* kinesin family member 16B fragment covering amino acid residues 1-400; Uniprot: Q96L93)
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
 
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGGAATCAGATTGCCCTCT
TAGACTCCCCCACA (SEQ ID NO:19)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPT (SEQ ID NO:20)

KIF13A_{1-411,ΔP390} (*Homo sapiens* kinesin family member 13A fragment covering amino acid residues 1-411 wherein P390 is deleted; Uniprot: Q9H1H9)
DNA: 
ATGTCGGATACCAAGGTAAAAGTTGCCGTCCGGGTCCGGCCCATGAACCGACGAGAAC
TGGAACTGAACACCAAGTGCGTGGTGGAGATGGAAGGAATCAAACGGTCCTGCACCC
TCCTCCTTCTAACACCAAACAGGGAGAAAGGAAACCTCCCAAGGTATTTGCCTTTGATTA
TTGCTTTTGGTCCATGGATGAATCTAACACTACAAAATACGCTGGTCAAGAAGTGGTTTT
CAAGTGCCTTGGGGAAGGAATTCTTGAAAAAGCCTTTCAGGGGTATAATGCGTGTATTT
TTGCATATGGACAGACAGGTTCGGGAAAATCCTTTTCCATGATGGGCCATGCTGAGCAG
CTGGGCCTTATTCCAAGGCTCTGCTGTGCTTTATTTAAAAGGATCTCTTTGGAGCAAAAT
GAGTCACAGACCTTTAAAGTTGAAGTGTCCTATATGGAAATTTATAATGAGAAAGTTCGG
GATCTTTTAGACCCCAAAGGGAGTAGACAGTCTCTTAAAGTTCGAGAACATAAAGTTTTG
GGACCATATGTAGATGGTTTATCTCAACTAGCTGTCACTAGTTTTGAGGATATTGAGTCA
 
TTGATGTCTGAGGGAAATAAGTCTCGAACGGTAGCTGCTACCAACATGAACGAAGAAAG
CAGCCGCTCCCATGCTGTGTTCAACATCATAATCACACAGACACTTTATGACCTGCAGT
CTGGGAATTCCGGGGAGAAAGTCAGTAAGGTCAGCTTGGTAGACCTGGCGGGTAGCGA
AAGAGTATCTAAAACAGGAGCTGCAGGAGAGCGACTGAAAGAAGGCAGCAACATTAAC
AAATCGCTTACAACCTTGGGGTTGGTTATATCATCACTGGCTGACCAGGCAGCTGGCAA
GGGTAAAAGCAAATTTGTGCCTTATCGAGATTCAGTCCTCACTTGGCTGCTTAAGGACA
ACTTGGGGGGCAACAGCCAAACCTCTATGATAGCCACAATCAGCCCAGCCGCAGACAA
CTATGAAGAGACCCTCTCCACATTAAGATATGCAGACCGAGCCAAAAGGATTGTGAACC
ATGCTGTTGTGAATGAGGACCCCAACGCAAAAGTGATCCGAGAACTGCGGGAGGAAGT
CGAGAAACTGAGAGAGCAGCTCTCTCAGGCAGAGGCCATGAAGGCCGAACTGAAGGA
GAAGCTCGAAGAGTCTGAAAAGCTGATAAAAGAACTAACAGTGACTTGGGAA (SEQ ID
NO:21)
Protein:

MSDTKVKVAVRVRPMNRRELELNTKCVVEMEGNQTVLHPPPSNTKQGERKPPKVFAFDYC
FWSMDESNTTKYAGQEVVFKCLGEGILEKAFQGYNACIFAYGQTGSGKSFSMMGHAEQLG
LIPRLCCALFKRISLEQNESQTFKVEVSYMEIYNEKVRDLLDPKGSRQSLKVREHKVLGPYV
DGLSQLAVTSFEDIESLMSEGNKSRTVAATNMNEESSRSHAVFNIIITQTLYDLQSGNSGEKV
SKVSLVDLAGSERVSKTGAAGERLKEGSNINKSLTTLGLVISSLADQAAGKGKSKFVPYRDS
VLTWLLKDNLGGNSQTSMIATISPAADNYEETLSTLRYADRAKRIVNHAVVNEDPNAKVIREL
REEVEKLREQLSQAEAMKAELKEKLEESEKLIKELTVTWE (SEQ ID NO:22)

TOMM20₁₋₇₀ (*Homo sapiens* translocase of outer mitochondrial membrane 20 fragment covering amino acid residues 1-70; Uniprot: Q15388)
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAATTCTTC (SEQ ID NO:23)
Protein:

MVGRNSAIAAGVCGALFIGYCIYFDRKRRSDPNFKNRLRERRKKQKLAKERAGLSKLPDLKD
AEAVQKFF (SEQ ID NO:24)

LcK (posttranslational modification site for plasma membrane targeting of *Mus musculus* lymphocyte-specific protein tyrosine kinase; Uniprot: P06240)
DNA: GGCTGCGTGTGCAGCAGCAACCCCGAGGGTACCGAGCTC (SEQ ID NO:25)
Protein: (identical part underlined P06240)
   GCVCSSNPEGTEL (SEQ ID NO:26)

FRB-CD28 (synthetic membrane targeting fusion polypeptide derived from *Mus musculus* CD4 (Uniprot: P06332), FRB (similar to *Homo sapiens* mTOR; Uniprot: P42345) and *Mus musculus* CD28 (Uniprot: P31041)
DNA: 
ATGTGCCGAGCCATCTCTCTTAGGCGCTTGCTGCTGCTGCTGCAGCTGTCACAACT
CCTAGCTGTCACTCAAGGGATGCTCGAGATGTGGCATGAAGGCCTGGAAGAGGCATCT
CGTTTGTACTTTGGGGAAAGGAACGTGAAAGGCATGTTTGAGGTGCTGGAGCCCTTGC
ATGCTATGATGGAACGGGGCCCCCAGACTCTGAAGGAAACATCCTTTAATCAGGCCTAT
GGTCGAGATTTAATGGAGGCCCAAGAGTGGTGCAGGAAGTACATGAAATCAGGGAATG
TCAAGGACCTCCTCCAAGCCTGGGACCTCTATTATCATGTGTTCCGACGAATCTCAAAG
ACTAGAACCGGTAAGCTTTTTTGGGCACTGGTCGTGGTTGCTGGAGTCCTGTTTTGTTA
TGGCTTGCTAGTGACAGTGGCTCTTTGTGTT (SEQ ID NO:27)
Protein:

MCRAISLRRLLLLLLQLSQLLAVTQGMLEMWHEGLEEASRLYFGERNVKGMFEVLEPLHAM
MERGPQTLKETSFNQAYGRDLMEAQEWCRKYMKSGNVKDLLQAWDLYYHVFRRISKTRT
GKLFWALVVVAGVLFCYGLLVTVALCV (SEQ ID NO:28)

FUS-CD28 (synthetic membrane targeting fusion polypeptide derived from *Mus musculus* CD4 (Uniprot: P06332), *Homo sapiens* fused-in sarcoma (Uniprot: P35637) and *Mus musculus* CD28 (Uniprot: P31041)
DNA: 
ATGTGCCGAGCCATCTCTCTTAGGCGCTTGCTGCTGCTGCTGCAGCTGTCACAACT
CCTAGCTGTCACTCAAGGGATGCTCATGGCCTCAAACGATTATACCCAACAAGCAACCC
AAAGCTATGGGGCCTACCCCACCCAGCCCGGGCAGGGCTATTCCCAGCAGAGCAGTCA
GCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTC
AACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCA
CCTCGGGAAGTTACGGTAGCAGTTCTCAGAGCAGCAGCTATGGGCAGCCCCAGAGTGG
GAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTG
GTGGAGGTGGAGGTGGAGGTGGAGGTAACTATGGCCAAGATCAATCCTCCATGAGTAG
TGGTGGTGGCAGTGGTGGCGGTTATGGCAATCAAGACCAGAGTGGTGGAGGTGGCAG
 
CGGTGGCTATGGACAGCAGGACCGTGGAGGCCGCGGCAGGGGTGGCAGTGGTGGCG
GCGGCGGCGGCGGTGGTGGTTACAACCGCAGCAGTGGTGGCTATGAACCCAGAG
GTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAA
TTCAGACAACAACACCATCTTTGTGCAAGGCCTGGGTGAGAATGTTACAATTGAGTCTG
TGGCTGATTACTTCAAGCAGATTGGTATTATTAAGACAAACAAGAAAACGGACAGCCC
ATGATTAATTTGTACACAGACAGGGAAACTGGCAAGCTGAAGGGAGAGGCAACGGTCT
CTTTTGATGACCCACCTTCAGCTAAAGCAGCTATTGACTGGTTTGATGGTAAAGAATTCT
CCGGAAATCCTATCAAGGTCTCATTTGCTACTCGCCGGGCAGACTTTAATCGGGGTGGT
GGCAATGGTCGTGGAGGCCGAGGGCGAGGAGGACCCATGGGCCGTGGAGGCTATGG
AGGTGGTGGCAGTGGTGGTGGTGGCCGAGGAGGATTTCCCAGTGGAGGTGGTGGCGG
TGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGG
AGGGGGACCAGGTGGCTCTCACATGGGGGGTAACTACGGGGATGATCGTCGTGGTGG
CAGAGGAGGCGGCACCGGTAAGCTTTTTGGGCACTGGTCGTGGTTGCTGGAGTCCTG
TTTTGTTATGGCTTGCTAGTGACAGTGGCTCTTTGTGTT (SEQ ID NO:29)
Protein:

MCRAISLRRLLLLLLQLSQLLAVTQGMLMASNDYTQQATQSYGAYPTQPGQGYSQQSSQP
YGQQSYSGYSQSTDTSGYGQSSYSSYGQSQNTGYGTQSTPQGYGSTGGYGSSQSSQSS
YGQQSSYPGYGQQPAPSSTSGSYGSSSQSSSYGQPQSGSYSQQPSYGGQQQSYGQQQ
SYNPPQGYGQQNQYNSSSGGGGGGGGGNYGQDQSSMSSGGGSGGGYGNQDQSGG
GGSGGYGQQDRGGRGRGGSGGGGGGGGGYNRSSGGYEPRGRGGGRGGRGGMGGS
DRGGFNKFGGPRDQGSRHDSEQDNSDNNTIFVQGLGENVTIESVADYFKQIGIIKTNKKTG
QPMINLYTDRETGKLKGEATVSFDDPPSAKAAIDWFDGKEFSGNPIKVSFATRRADFNRGG
GNGRGGRGRGGPMGRGGYGGGGSGGGGRGGFPSGGGGGGGQQRAGDWKCPNPTCE
NMNFSWRNECNQCKAPKPDGPGGGPGGSHMGGNYGDDRRGGRGGGTGKLFWALVVVA
GVLFCYGLLVTVALCV (SEQ ID NO:30)

### 7. PSPs

SPD5 (*Caenorhabditis elegans* spindle-defective protein 5; Uniprot: P91349)
DNA: 
ATGGAGGACAACAGCGTGCTGAACGAGGACAGCAACCTGGAGCACGTGGAGGGCCAG
CCCAGAAGAAGCATGAGCCAGCCCGTGCTGAACGTGGAGGGCGACAAGAGAACCAGC
AGCACCAGCGCCACCCAGCAGCAGGTGCTGAGCGGCGCCTTCAGCAGCGCCGACGTG
AGAAGCATCCCCATCATCCAGACCTGGGAGGAGAACAAGGCCCTGAAGACCAAGATCA
 
CCATCCTGAGAGGCGAGCTGCAGATGTACCAGAGAAGATACAGCGAGGCCAAGGAGG
CCAGCCAGAAGAGAGTGAAGGAGGTGATGGACGACTACGTGGACCTGAAGCTGGGCC
AGGAGAACGTGCAGGAGAAGATGGAGCAGTACAAGCTGATGGAGGAGGACCTGCTGG
CCATGCAGAGCAGAATCGAGACCAGCGAGGACAACTTCGCCAGACAGATGAAGGAGTT
CGAGGCCCAGAAGCACGCCATGGAGGAGAGAATCAAGGAGCTGGAGCTGAGCGCCAC
CGACGCCAACAACACCACCGTGGGCAGCTTCAGAGGCACCCTGGACGACATCCTGAAG
AAGAACGACCCCGACTTCACCCTGACCAGCGGCTACGAGGAGAGAAAGATCAACGACC
TGGAGGCCAAGCTGCTGAGCGAGATCGACAAGGTGGCCGAGCTGGAGGACCACATCC
AGCAGCTGAGACAGGAGCTGGACGACCAGAGCGCCAGACTGGCCGACAGCGAGAACG
TGAGAGCCCAGCTGGAGGCCGCCACCGGCCAGGGCATCCTGGGCGCCGCCGGCAAC
GCCATGGTGCCCAACAGCACCTTCATGATCGGCAACGGCAGAGAGAGCCAGACCAGAG
ACCAGCTGAACTACATCGACGACCTGGAGACCAAGCTGGCCGACGCCAAGAAGGAGAA
CGACAAGGCCAGACAGGCCCTGGTGGAGTACATGAACAAGTGCAGCAAGCTGGAGCA
CGAGATCAGAACCATGGTGAAGAACAGCACCTTCGACAGCAGCAGCATGCTGCTGGGC
GGCCAGACCAGCGACGAGCTGAAGATCCAGATCGGCAAGGTGAACGGCGAGCTGAAC
GTGCTGAGAGCCGAGAACAGAGAGCTGAGAATCAGATGCGACCAGCTGACCGGCGGC
GACGGCAACCTGAGCATCAGCCTGGGCCAGAGCAGACTGATGGCCGGCATCGCCACC
AACGACGTGGACAGCATCGGCCAGGGCAACGAGACCGGCGGCACCAGCATGAGAATC
CTGCCCAGAGAGAGCCAGCTGGACGACCTGGAGGAGAGCAAGCTGCCCCTGATGGAC
ACCAGCAGCGCCGTGAGAAACCAGCAGCAGTTCGCCAGCATGTGGAGGACTTCGAG
AGCGTGAAGGACAGCCTGCAGAACAACCACAACGACACCCTGGAGGGCAGCTTCAACA
GCAGCATGCCCCCCCCCGGCAGAGACGCCACCCAGAGCTTCCTGAGCCAGAAGAGCT
TCAAGAACAGCCCCATCGTGATGCAGAAGCCCAAGAGCCTGCACCTGCACCTGAAGAG
CCACCAGAGCGAGGGCGCCGGCGAGCAGATCCAGAACAACAGCTTCAGCACCAAGAC
CGCCAGCCCCCACGTGAGCCAGAGCCACATCCCCATCCTGCACGACATGCAGCAGATC
CTGGACAGCAGCGCCATGTTCCTGGAGGGCCAGCACGACGTGGCCGTGAACGTGGAG
CAGATGCAGGAGAAGATGAGCCAGATCAGAGAGGCCCTGGCCAGACTGTTCGAGAGAC
TGAAGAGCAGCGCCGCCCTGTTCGAGGAGATCCTGGAGAGAATGGGCAGCAGCGACC
CCAACGCCGACAAGATCAAGAAGATGAAGCTGGCCTTCGAGACCAGCATCAACGACAA
GCTGAACGTGAGCGCCATCCTGGAGGCCGCCGAGAAGGACCTGCACAACATGAGCCT
GAACTTCAGCATCCTGGAGAAGAGCATCGTGAGCCAGGCCGCCGAGGCCAGCAGAAG
ATTCACCATCGCCCCCGACGCCGAGGACGTGGCCAGCAGCAGCCTGCTGAACGCCAG
CTACAGCCCCCTGTTCAAGTTCACCAGCAACAGCGACATCGTGGAGAAGCTGCAGAAC
GAGGTGAGCGAGCTGAAGAACGAGCTGGAGATGGCCAGAACCAGAGACATGAGAAGC
CCCCTGAACGGCAGCAGCGGCAGACTGAGCGACGTGCAGATCAACACCAACAGAATGT
TCGAGGACCTGGAGGTGAGCGAGGCCACCCTGCAGAAGGCCAAGGAGGAGAACAGCA
 
CCCTGAAGAGCCAGTTCGCCGAGCTGGAGGCCAACCTGCACCAGGTGAACAGCAAGCT
GGGCGAGGTGAGATGCGAGCTGAACGAGGCCCTGGCCAGAGTGGACGGCGAGCAGG
AGACCAGAGTGAAGGCCGAGAACGCCCTGGAGGAGGCCAGACAGCTGATCAGCAGCC
TGAAGCACGAGGAGAACGAGCTGAAGAAGACCATCACCGACATGGGCATGAGACTGAA
CGAGGCCAAGAAGAGCGACGAGTTCCTGAAGAGCGAGCTGAGCACCGCCCTGGAGGA
GGAGAAGAAGAGCCAGAACCTGGCCGACGAGCTGAGCGAGGAGCTGAACGGCTGGAG
AATGAGAACCAAGGAGGCCGAGAACAAGGTGGAGCACGCCAGCAGCGAGAAGAGCGA
GATGCTGGAGAGAATCGTGCACCTGGAGACCGAGATGGAGAAGCTGAGCACCAGCGA
GATCGCCGCCGACTACTGCAGCACCAAGATGACCGAGAGAAAGAAGGAGATCGAGCTG
GCCAAGTACAGAGAGGACTTCGAGAACGCCGCCATCGTGGGCCTGGAGAGAATCAGCA
AGGAGATCAGCGAGCTGACCAAGAAGACCCTGAAGGCCAAGATCATCCCCAGCAACAT
CAGCAGCATCCAGCTGGTGTGCGACGAGCTGTGCAGAAGACTGAGCAGAGAGAGAGA
GCAGCAGCACGAGTACGCCAAGGTGATGAGAGACGTGAACGAGAAGATCGAGAAGCT
GCAGCTGGAGAAGGACGCCCTGGAGCACGAGCTGAAGATGATGAGCAGCAACAACGA
GAACGTGCCCCCCGTGGGCACCAGCGTGAGCGGCATGCCCACCAAGACCAGCAACCA
GAAGTGCGCCCAGCCCCACTACACCAGCCCCACCAGACAGCTGCTGCACGAGAGCAC
CATGGCCGTGGACGCCATCGTGCAGAAGCTGAAGAAGACCCACAACATGAGCGGCATG
GGCCCCGAGCTGAAGGAGACCATCGGCAACGTGATCAACGAGAGCAGAGTGCTGAGA
GACTTCCTGCACCAGAAGCTGATCCTGTTCAAGGGCATCGACATGAGCAACTGGAAGAA
CGAGACCGTGGACCAGCTGATCACCGACCTGGGCCAGCTGCACCAGGACAACCTGAT
GCTGGAGGAGCAGATCAAGAAGTACAAGGAGCTGAAGCTGACCAAGAGCGCCATC
CCCACCCTGGGCGTGGAGTTCCAGGACAGAATCAAGACCGAGATCGGCAAGATCGCCA
CCGACATGGGCGGCGCCGTGAAGGAGATCAGAAAGAAG (SEQ ID NO:31)
Protein:

MEDNSVLNEDSNLEHVEGQPRRSMSQPVLNVEGDKRTSSTSATQQQVLSGAFSSADVRSI
PIIQTWEENKALKTKITILRGELQMYQRRYSEAKEASQKRVKEVMDDYVDLKLGQENVQEK
MEQYKLMEEDLLAMQSRIETSEDNFARQMKEFEAQKHAMEERIKELELSATDANNTTVGSF
RGTLDDILKKNDPDFTLTSGYEERKINDLEAKLLSEIDKVAELEDHIQQLRQELDDQSARLAD
SENVRAQLEAATGQGILGAAGNAMVPNSTFMIGNGRESQTRDQLNYIDDLETKLADAKKEN
DKARQALVEYMNKCSKLEHEIRTMVKNSTFDSSSMLLGGQTSDELKIQIGKVNGELNVLRAE
NRELRIRCDQLTGGDGNLSISLGQSRLMAGIATNDVDSIGQGNETGGTSMRILPRESQLDDL
EESKLPLMDTSSAVRNQQQFASMWEDFESVKDSLQNNHNDTLEGSFNSSMPPPGRDATQ
SFLSQKSFKNSPIVMQKPKSLHLHLKSHQSEGAGEQIQNNSFSTKTASPHVSQSHIPILHDM
QQILDSSAMFLEGQHDVAVNVEQMQEKMSQIREALARLFERLKSSAALFEEILERMGSSDP
NADKIKKMKLAFETSINDKLNVSAILEAAEKDLHNMSLNFSILEKSIVSQAAEASRRFTIAPDAE
DVASSSLLNASYSPLFKFTSNSDIVEKLQNEVSELKNELEMARTRDMRSPLNGSSGRLSDV

QINTNRMFEDLEVSEATLQKAKEENSTLKSQFAELEANLHQVNSKLGEVRCELNEALARVD
GEQETRVKAENALEEARQLISSLKHEENELKKTITDMGMRLNEAKKSDEFLKSELSTALEEE
KKSQNLADELSEELNGWRMRTKEAENKVEHASSEKSEMLERIVHLETEMEKLSTSEIAADY
CSTKMTERKKEIELAKYREDFENAAIVGLERISKEISELTKKTLKAKIIPSNISSIQLVCDELCRR
LSREREQQHEYAKVMRDVNEKIEKLQLEKDALEHELKMMSSNNENVPPVGTSVSGMPTKT
SNQKCAQPHYTSPTRQLLHESTMAVDAIVQKLKKTHNMSGMGPELKETIGNVINESRVLRD
FLHQKLILFKGIDMSNWKNETVDQLITDLGQLHQDNLMLEEQIKKYKKELKLTKSAIPTLGVEF
QDRIKTEIGKIATDMGGAVKEIRKK (SEQ ID NO:32)

FUS (*Homo sapiens* fused-in sarcoma; Uniprot: P35637)
DNA: 
ATGGCCTCAAACGATTATACCCAACAAGCAACCCAAAGCTATGGGGCCTACCCCACCCA
GCCCGGGCAGGGCTATTCCCAGCAGAGCAGTCAGCCCTACGGACAGCAGAGTTACAGT
GGTTATAGCCAGTCCACGGACACTTCAGGATATGGCCAGAGCAGCTATTCTTCTTATGG
CCAGAGCCAGAACACAGGCTATGGAACTCAGTCAACTCCCCAGGGATATGGCTCGACT
GGCGGCTATGGCAGTAGCCAGAGCTCCCAATCGTCTTACGGGCAGCAGTCCTCCTACC
CTGGCTATGGCCAGCAGCCAGCTCCCAGCAGCACCTCGGGAAGTTACGGTAGCAGTTC
TCAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATG
GACAGCAGAACCAGTACAACAGCAGCAGTGGTGGTGGAGGTGGAGGTGGAGGTGGAG
GTAACTATGGCCAAGATCAATCCTCCATGAGTAGTGGTGGTGGCAGTGGTGGCGGTTAT
GGCAATCAAGACCAGAGTGGTGGAGGTGGCAGCGGTGGCTATGGACAGCAGGACCGT
GGAGGCCGCGGCAGGGGTGGCAGTGGTGGCGGCGGCGGCGGCGGTGGTGGTT
ACAACCGCAGCAGTGGTGGCTATGAACCCAGAGGTCGTGGAGGTGGCCGTGGAGGCA
GAGGTGGCATGGGCGGAAGTGACCGTGGTGGCTTCAATAAATTTGGTGGCCCTCGGGA
CCAAGGATCACGTCATGACTCCGAACAGGATAATTCAGACAACAACACCATCTTTGTGC
AAGGCCTGGGTGAGAATGTTACAATTGAGTCTGTGGCTGATTACTTCAAGCAGATTGGT
ATTATTAAGACAAACAAGAAAACGGACAGCCCATGATTAATTTGTACACAGACAGGGA
AACTGGCAAGCTGAAGGGAGAGGCAACGGTCTCTTTTGATGACCCACCTTCAGCTAAAG
CAGCTATTGACTGGTTTGATGGTAAAGAATTCTCCGGAAATCCTATCAAGGTCTCATTTG
CTACTCGCCGGGCAGACTTTAATCGGGGTGGTGGCAATGGTCGTGGAGGCCGAGGGC
GAGGAGGACCCATGGGCCGTGGAGGCTATGGAGGTGGTGGCAGTGGTGGTGGTGGC
CGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAA
CCAGTGTAAGGCCCCTAAACCAGATGGCCCAGGAGGGGGACCAGGTGGCTCTCACAT
GGGGGGTAACTACGGGGATGATCGTCGTGGTGGCAGAGGAGGC (SEQ ID NO:33)
Protein:

MASNDYTQQATQSYGAYPTQPGQGYSQQSSQPYGQQSYSGYSQSTDTSGYGQSSYSSY
GQSQNTGYGTQSTPQGYGSTGGYGSSQSSQSSYGQQSSYPGYGQQPAPSSTSGSYGSS
SQSSSYGQPQSGSYSQQPSYGGQQQSYGQQQSYNPPQGYGQQNQYNSSSGGGGGG
GGGNYGQDQSSMSSGGGSGGGYGNQDQSGGGGSGGYGQQDRGGRGRGGSGGGGG
GGGGYNRSSGGYEPRGRGGGRGGRGGMGGSDRGGFNKFGGPRDQGSRHDSEQDNSD
NNTIFVQGLGENVTIESVADYFKQIGIIKTNKKTGQPMINLYTDRETGKLKGEATVSFDDPPSA
KAAIDWFDGKEFSGNPIKVSFATRRADFNRGGGNGRGGRGRGGPMGRGGYGGGGSGGG
GRGGFPSGGGGGGGQQRAGDWKCPNPTCENMNFSWRNECNQCKAPKPDGPGGGPGG
SHMGGNYGDDRRGGRGG (SEQ ID NO:34)

EWSR1 (*Homo sapiens* Ewing sarcoma breakpoint region 1; Uniprot: Q01844)
DNA: 
ATGGCGTCCACGGATTACAGTACCTATAGCCAAGCTGCAGCGCAGCAGGGCTACAGTG
CTTACACCGCCCAGCCCACTCAAGGATATGCACAGACCACCCAGGCATATGGGCAACA
AAGCTATGGAACCTATGGACAGCCCACTGATGTCAGCTATACCCAGGCTCAGACCACTG
CAACCTATGGGCAGACCGCCTATGCAACTTCTTATGGACAGCCTCCCACTGGTTATACT
ACTCCAACTGCCCCCCAGGCATACAGCCAGCCTGTCCAGGGGTATGGCACTGGTGCTT
ATGATACCACCACTGCTACAGTCACCACCACCCAGGCCTCCTATGCAGCTCAGTCTGCA
TATGGCACTCAGCCTGCTTATCCAGCCTATGGGCAGCAGCCAGCAGCCACTGCACCTA
CAAGACCGCAGGATGGAAACAAGCCCACTGAGACTAGTCAACCTCAATCTAGCACAGG
GGGTTACAACCAGCCCAGCCTAGGATATGGACAGAGTAACTACAGTTATCCCCAGGTAC
CTGGGAGCTACCCCATGCAGCCAGTCACTGCACCTCCATCCTACCCTCCTACCAGCTAT
TCCTCTACACAGCCGACTAGTTATGATCAGAGCAGTTACTCTCAGCAGAACACCTATGG
GCAACCGAGCAGCTATGGACAGCAGAGTAGCTATGGTCAACAAAGCAGCTATGGGCAG
CAGCCTCCCACTAGTTACCCACCCCAAACTGGATCCTACAGCCAAGCTCCAAGTCAATA
TAGCCAACAGAGCAGCAGCTACGGGCAGCAGAGTTCATTCCGACAGGACCACCCCAGT
AGCATGGGTGTTTATGGGCAGGAGTCTGGAGGATTTTCCGGACCAGGAGAGAACCGGA
GCATGAGTGGCCCTGATAACCGGGGCAGGGGAAGAGGGGGATTTGATCGTGGAGGCA
TGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCC
CACCTGTAGATCCAGATGAAGACTCTGACAACAGTGCAATTTATGTACAAGGATTAAATG
ACAGTGTGACTCTAGATGATCTGGCAGACTTCTTTAAGCAGTGTGGGGTTGTTAAGATG
AACAAGAGAACTGGGCAACCCATGATCCACATCTACCTGGACAAGGAAACAGGAAAGC
CCAAAGGCGATGCCACAGTGTCCTATGAAGACCCACCCACTGCCAAGGCTGCCGTGGA
ATGGTTTGATGGGAAAGATTTTCAAGGGAGCAAACTTAAAGTCTCCCTTGCTCGGAAGA
 
AGCCTCCAATGAACAGTATGCGGGGTGGTCTGCCACCCCGTGAGGGCAGAGGCATGC
CACCACCACTCCGTGGAGGTCCAGGAGGCCCAGGAGGTCCTGGGGGACCCATGGGTC
GCATGGGAGGCCGTGGAGGAGATAGAGGAGGCTTCCCTCCAAGAGGACCCCGGGGTT
CCCGAGGGAACCCCTCTGGAGGAGGAAACGTCCAGCACCGAGCTGGAGACTGGCAGT
GTCCCAATCCGGGTTGTGGAAACCAGAACTTCGCCTGGAGAACAGAGTGCAACCAGTG
TAAGGCCCCAAAGCCTGAAGGCTTCCTCCCGCCACCCTTTCCGCCCCCGGGTGGTGAT
CGTGGCAGAGGTGGCCCTGGTGGCATGCGGGGAGGAAGAGGTGGCCTCATGGATCGT
GGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGG
GGGGCCCCCTGGACCTTTGATGGAACAG (SEQ ID NO:35)
Protein:

MASTDYSTYSQAAAQQGYSAYTAQPTQGYAQTTQAYGQQSYGTYGQPTDVSYTQAQTTA
TYGQTAYATSYGQPPTGYTTPTAPQAYSQPVQGYGTGAYDTTTATVTTTQASYAAQSAYG
TQPAYPAYGQQPAATAPTRPQDGNKPTETSQPQSSTGGYNQPSLGYGQSNYSYPQVPGS
YPMQPVTAPPSYPPTSYSSTQPTSYDQSSYSQQNTYGQPSSYGQQSSYGQQSSYGQQPP
TSYPPQTGSYSQAPSQYSQQSSSYGQQSSFRQDHPSSMGVYGQESGGFSGPGENRSMS
GPDNRGRGRGGFDRGGMSRGGRGGGRGGMGSAGERGGFNKPGGPMDEGPDLDLGPP
VDPDEDSDNSAIYVQGLNDSVTLDDLADFFKQCGVVKMNKRTGQPMIHIYLDKETGKPKGD
ATVSYEDPPTAKAAVEWFDGKDFQGSKLKVSLARKKPPMNSMRGGLPPREGRGMPPPLR
GGPGGPGGPGGPMGRMGGRGGDRGGFPPRGPRGSRGNPSGGGNVQHRAGDWQCPN
PGCGNQNFAWRTECNQCKAPKPEGFLPPPFPPPGGDRGRGGPGGMRGGRGGLMDRGG
PGGMFRGGRGGDRGGFRGGRGMDRGGFGGGRRGGPGGPPGPLMEQ (SEQ ID NO:36)

### 8. AFPs

EWSR1-MCP
DNA: 
ATGGCGTCCACGGATTACAGTACCTATAGCCAAGCTGCAGCGCAGCAGGGCTACAGTG
CTTACACCGCCCAGCCCACTCAAGGATATGCACAGACCACCCAGGCATATGGGCAACA
AAGCTATGGAACCTATGGACAGCCCACTGATGTCAGCTATACCCAGGCTCAGACCACTG
CAACCTATGGGCAGACCGCCTATGCAACTTCTTATGGACAGCCTCCCACTGGTTATACT
ACTCCAACTGCCCCCCAGGCATACAGCCAGCCTGTCCAGGGGTATGGCACTGGTGCTT
ATGATACCACCACTGCTACAGTCACCACCACCCAGGCCTCCTATGCAGCTCAGTCTGCA
TATGGCACTCAGCCTGCTTATCCAGCCTATGGGCAGCAGCCAGCAGCCACTGCACCTA
CAAGACCGCAGGATGGAAACAAGCCCACTGAGACTAGTCAACCTCAATCTAGCACAGG
GGGTTACAACCAGCCCAGCCTAGGATATGGACAGAGTAACTACAGTTATCCCCAGGTAC
 
CTGGGAGCTACCCCATGCAGCCAGTCACTGCACCTCCATCCTACCCTCCTACCAGCTAT
TCCTCTACACAGCCGACTAGTTATGATCAGAGCAGTTACTCTCAGCAGAACACCTATGG
GCAACCGAGCAGCTATGGACAGCAGAGTAGCTATGGTCAACAAAGCAGCTATGGGCAG
CAGCCTCCCACTAGTTACCCACCCCAAACTGGATCCTACAGCCAAGCTCCAAGTCAATA
TAGCCAACAGAGCAGCAGCTACGGGCAGCAGAGTTCATTCCGACAGGACCACCCCAGT
AGCATGGGTGTTTATGGGCAGGAGTCTGGAGGATTTTCCGGACCAGGAGAGAACCGGA
GCATGAGTGGCCCTGATAACCGGGGCAGGGGAAGAGGGGGATTTGATCGTGGAGGCA
TGAGCAGAGGTGGGCGGGGAGGAGGACGCGGTGGAATGGGCAGCGCTGGAGAGCGA
GGTGGCTTCAATAAGCCTGGTGGACCCATGGATGAAGGACCAGATCTTGATCTAGGCC
CACCTGTAGATCCAGATGAAGACTCTGACAACAGTGCAATTTATGTACAAGGATTAAATG
ACAGTGTGACTCTAGATGATCTGGCAGACTTCTTTAAGCAGTGTGGGGTTGTTAAGATG
AACAAGAGAACTGGGCAACCCATGATCCACATCTACCTGGACAAGGAAACAGGAAAGC
CCAAAGGCGATGCCACAGTGTCCTATGAAGACCCACCCACTGCCAAGGCTGCCGTGGA
ATGGTTTGATGGGAAAGATTTTCAAGGGAGCAAACTTAAAGTCTCCCTTGCTCGGAAGA
AGCCTCCAATGAACAGTATGCGGGGTGGTCTGCCACCCCGTGAGGGCAGAGGCATGC
CACCACCACTCCGTGGAGGTCCAGGAGGCCCAGGAGGTCCTGGGGGACCCATGGGTC
GCATGGGAGGCCGTGGAGGAGATAGAGGAGGCTTCCCTCCAAGAGGACCCCGGGGTT
CCCGAGGGAACCCCTCTGGAGGAGGAAACGTCCAGCACCGAGCTGGAGACTGGCAGT
GTCCCAATCCGGGTTGTGGAAACCAGAACTTCGCCTGGAGAACAGAGTGCAACCAGTG
TAAGGCCCCAAAGCCTGAAGGCTTCCTCCCGCCACCCTTTCCGCCCCCGGGTGGTGAT
CGTGGCAGAGGTGGCCCTGGTGGCATGCGGGGAGGAAGAGGTGGCCTCATGGATCGT
GGTGGTCCCGGTGGAATGTTCAGAGGTGGCCGTGGTGGAGACAGAGGTGGCTTCCGT
GGTGGCCGGGGCATGGACCGAGGTGGCTTTGGTGGAGGAAGACGAGGTGGCCCTGG
GGGGCCCCCTGGACCTTTGATGGAACAGGATTACAAGGATGACGACGATAAGGGTACC
GAGCAGAAGCTGATCTCAGAGGAGGACCTGGGCGCCCCCGGCTCCGCCGGCTCCGCC
GCCGGCTCCGGCGCTTCTAACTTTACTCAGTTCGTTCTCGTCGACAATGGCGGAACTGG
CGACGTGACTGTCGCCCCAAGCAACTTCGCTAACGGGATCGCTGAATGGATCAGCTCT
AACTCGCGTTCACAGGCTTACAAAGTAACCTGTAGCGTTCGTCAGAGCTCTGCGCAGAA
TCGCAAATACACCATCAAAGTCGAGGTGCCTAAAGGCGCCTGGCGTTCGTACTTAAATA
TGGAACTAACCATTCCAATTTTCGCCACGAATTCCGACTGCGAGCTTATTGTTAAGGCAA
TGCAAGGTCTCCTAAAAGATGGAAACCCGATTCCCTCAGCAATCGCAGCAAACTCCGGC
ATCTACTAA (SEQ ID NO:37)
Protein:

MASTDYSTYSQAAAQQGYSAYTAQPTQGYAQTTQAYGQQSYGTYGQPTDVSYTQAQTTA
TYGQTAYATSYGQPPTGYTTPTAPQAYSQPVQGYGTGAYDTTTATVTTTQASYAAQSAYG
TQPAYPAYGQQPAATAPTRPQDGNKPTETSQPQSSTGGYNQPSLGYGQSNYSYPQVPGS

YPMQPVTAPPSYPPTSYSSTQPTSYDQSSYSQQNTYGQPSSYGQQSSYGQQSSYGQQPP
TSYPPQTGSYSQAPSQYSQQSSSYGQQSSFRQDHPSSMGVYGQESGGFSGPGENRSMS
GPDNRGRGRGGFDRGGMSRGGRGGGRGGMGSAGERGGFNKPGGPMDEGPDLDLGPP
VDPDEDSDNSAIYVQGLNDSVTLDDLADFFKQCGVVKMNKRTGQPMIHIYLDKETGKPKGD
ATVSYEDPPTAKAAVEWFDGKDFQGSKLKVSLARKKPPMNSMRGGLPPREGRGMPPPLR
GGPGGPGGPGGPMGRMGGRGGDRGGFPPRGPRGSRGNPSGGGNVQHRAGDWQCPN
PGCGNQNFAWRTECNQCKAPKPEGFLPPPFPPPGGDRGRGGPGGMRGGRGGLMDRGG
PGGMFRGGRGGDRGGFRGGRGMDRGGFGGGRRGGPGGPPGPLMEQDYKDDDDKGTE
QKLISEEDLGAPGSAGSAAGSGASNFTQFVLVDNGGTGDVTVAPSNFANGIAEWISSNSRS
QAYKVTCSVRQSSAQNRKYTIKVEVPKGAWRSYLNMELTIPIFATNSDCELIVKAMQGLLKD
GNPIPSAIAANSGIY (SEQ ID NO:38)

FUS-MCP
DNA: 
ATGGCCTCAAACGATTATACCCAACAAGCAACCCAAAGCTATGGGGCCTACCCCACCCA
GCCCGGGCAGGGCTATTCCCAGCAGAGCAGTCAGCCCTACGGACAGCAGAGTTACAGT
GGTTATAGCCAGTCCACGGACACTTCAGGATATGGCCAGAGCAGCTATTCTTCTTATGG
CCAGAGCCAGAACACAGGCTATGGAACTCAGTCAACTCCCCAGGGATATGGCTCGACT
GGCGGCTATGGCAGTAGCCAGAGCTCCCAATCGTCTTACGGGCAGCAGTCCTCCTACC
CTGGCTATGGCCAGCAGCCAGCTCCCAGCAGCACCTCGGGAAGTTACGGTAGCAGTTC
TCAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATG
GACAGCAGAACCAGTACAACAGCAGCAGTGGTGGTGGAGGTGGAGGTGGAGGTGGAG
GTAACTATGGCCAAGATCAATCCTCCATGAGTAGTGGTGGTGGCAGTGGTGGCGGTTAT
GGCAATCAAGACCAGAGTGGTGGAGGTGGCAGCGGTGGCTATGGACAGCAGGACCGT
GGAGGCCGCGGCAGGGGTGGCAGTGGTGGCGGCGGCGGCGGCGGTGGTGGTT
ACAACCGCAGCAGTGGTGGCTATGAACCCAGAGGTCGTGGAGGTGGCCGTGGAGGCA
GAGGTGGCATGGGCGGAAGTGACCGTGGTGGCTTCAATAAATTTGGTGGCCCTCGGGA
CCAAGGATCACGTCATGACTCCGAACAGGATAATTCAGACAACAACACCATCTTTGTGC
AAGGCCTGGGTGAGAATGTTACAATTGAGTCTGTGGCTGATTACTTCAAGCAGATTGGT
ATTATTAAGACAAACAAGAAAACGGACAGCCCATGATTAATTTGTACACAGACAGGGA
AACTGGCAAGCTGAAGGGAGAGGCAACGGTCTCTTTTGATGACCCACCTTCAGCTAAAG
CAGCTATTGACTGGTTTGATGGTAAAGAATTCTCCGGAAATCCTATCAAGGTCTCATTTG
CTACTCGCCGGGCAGACTTTAATCGGGGTGGTGGCAATGGTCGTGGAGGCCGAGGGC
GAGGAGGACCCATGGGCCGTGGAGGCTATGGAGGTGGTGGCAGTGGTGGTGGTGGC
CGAGGAGGATTTCCCAGTGGAGGTGGTGGCGGTGGAGGACAGCAGCGAGCTGGTGAC
 
TGGAAGTGTCCTAATCCCACCTGTGAGAATATGAACTTCTCTTGGAGGAATGAATGCAA
CCAGTGTAAGGCCCCTAAACCAGATGGCCCAGGAGGGGGACCAGGTGGCTCTCACAT
GGGGGGTAACTACGGGGATGATCGTCGTGGTGGCAGAGGAGGCGATTACAAGGATGA
CGACGATAAGGGTACCGAGCAGAAGCTGATCTCAGAGGAGGACCTGGGCGCCCCCGG
CTCCGCCGGCTCCGCCGCCGGCTCCGGCGCTTCTAACTTTACTCAGTTCGTTCTCGTC
GACAATGGCGGAACTGGCGACGTGACTGTCGCCCCAAGCAACTTCGCTAACGGGATCG
CTGAATGGATCAGCTCTAACTCGCGTTCACAGGCTTACAAAGTAACCTGTAGCGTTCGT
CAGAGCTCTGCGCAGAATCGCAAATACACCATCAAAGTCGAGGTGCCTAAAGGCGCCT
GGCGTTCGTACTTAAATATGGAACTAACCATTCCAATTTTCGCCACGAATTCCGACTGCG
AGCTTATTGTTAAGGCAATGCAAGGTCTCCTAAAAGATGGAAACCCGATTCCCTCAGCA
ATCGCAGCAAACTCCGGCATCTACTAA (SEQ ID NO:39)
Protein:

MASNDYTQQATQSYGAYPTQPGQGYSQQSSQPYGQQSYSGYSQSTDTSGYGQSSYSSY
GQSQNTGYGTQSTPQGYGSTGGYGSSQSSQSSYGQQSSYPGYGQQPAPSSTSGSYGSS
SQSSSYGQPQSGSYSQQPSYGGQQQSYGQQQSYNPPQGYGQQNQYNSSSGGGGGG
GGGNYGQDQSSMSSGGGSGGGYGNQDQSGGGGSGGYGQQDRGGRGRGGSGGGGG
GGGGYNRSSGGYEPRGRGGGRGGRGGMGGSDRGGFNKFGGPRDQGSRHDSEQDNSD
NNTIFVQGLGENVTIESVADYFKQIGIIKTNKKTGQPMINLYTDRETGKLKGEATVSFDDPPSA
KAAIDWFDGKEFSGNPIKVSFATRRADFNRGGGNGRGGRGRGGPMGRGGYGGGGSGGG
GRGGFPSGGGGGGGQQRAGDWKCPNPTCENMNFSWRNECNQCKAPKPDGPGGGPGG
SHMGGNYGDDRRGGRGGDYKDDDDKGTEQKLISEEDLGAPGSAGSAAGSGASNFTQFVL
VDNGGTGDVTVAPSNFANGIAEWISSNSRSQAYKVTCSVRQSSAQNRKYTIKVEVPKGAW
RSYLNMELTIPIFATNSDCELIVKAMQGLLKDGNPIPSAIAANSGIY (SEQ ID NO:40)

FUS-PylRS^{AF}
DNA: 
ATGGCCTCAAACGATTATACCCAACAAGCAACCCAAAGCTATGGGGCCTACCCCACCCA
GCCCGGGCAGGGCTATTCCCAGCAGAGCAGTCAGCCCTACGGACAGCAGAGTTACAGT
GGTTATAGCCAGTCCACGGACACTTCAGGATATGGCCAGAGCAGCTATTCTTCTTATGG
CCAGAGCCAGAACACAGGCTATGGAACTCAGTCAACTCCCCAGGGATATGGCTCGACT
GGCGGCTATGGCAGTAGCCAGAGCTCCCAATCGTCTTACGGGCAGCAGTCCTCCTACC
CTGGCTATGGCCAGCAGCCAGCTCCCAGCAGCACCTCGGGAAGTTACGGTAGCAGTTC
TCAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATG
GACAGCAGAACCAGTACAACAGCAGCAGTGGTGGTGGAGGTGGAGGTGGAGGTGGAG
GTAACTATGGCCAAGATCAATCCTCCATGAGTAGTGGTGGTGGCAGTGGTGGCGGTTAT
 
GGCAATCAAGACCAGAGTGGTGGAGGTGGCAGCGGTGGCTATGGACAGCAGGACCGT
GGAGGCCGCGGCAGGGGTGGCAGTGGTGGCGGCGGCGGCGGCGGTGGTGGTT
ACAACCGCAGCAGTGGTGGCTATGAACCCAGAGGTCGTGGAGGTGGCCGTGGAGGCA
GAGGTGGCATGGGCGGAAGTGACCGTGGTGGCTTCAATAAATTTGGTGGCCCTCGGGA
CCAAGGATCACGTCATGACTCCGAACAGGATAATTCAGACAACAACACCATCTTTGTGC
AAGGCCTGGGTGAGAATGTTACAATTGAGTCTGTGGCTGATTACTTCAAGCAGATTGGT
ATTATTAAGACAAACAAGAAAACGGACAGCCCATGATTAATTTGTACACAGACAGGGA
AACTGGCAAGCTGAAGGGAGAGGCAACGGTCTCTTTTGATGACCCACCTTCAGCTAAAG
CAGCTATTGACTGGTTTGATGGTAAAGAATTCTCCGGAAATCCTATCAAGGTCTCATTTG
CTACTCGCCGGGCAGACTTTAATCGGGGTGGTGGCAATGGTCGTGGAGGCCGAGGGC
GAGGAGGACCCATGGGCCGTGGAGGCTATGGAGGTGGTGGCAGTGGTGGTGGTGGC
CGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAA
CCAGTGTAAGGCCCCTAAACCAGATGGCCCAGGAGGGGGACCAGGTGGCTCTCACAT
GGGGGGTAACTACGGGGATGATCGTCGTGGTGGCAGAGGAGGCGATTACAAGGATGA
CGACGATAAGGGTACCGGCGCCCCCGGCTCCGCCGGCTCCGCCGCCGGCTCCGGCG
CTTCTAACTTTACTCAGTTCGTTCTCGTCGACAATGGCGGAACTGGCGACGTGACTGTC
GCCCCAAGCAACTTCGCTAACGGGATCGCTGAATGGATCAGCTCTAACTCGCGTTCACA
GGCTTACAAAGTAACCTGTAGCGTTCGTCAGAGCTCTGCGCAGAATCGCAAATACACCA
TCAAAGTCGAGGTGCCTAAAGGCGCCTGGCGTTCGTACTTAAATATGGAACTAACCATT
CCAATTTTCGCCACGAATTCCGACTGCGAGCTTATTGTTAAGGCAATGCAAGGTCTCCT
AAAAGATGGAAACCCGATTCCCTCAGCAATCGCAGCAAACTCCGGCATCTACGGTACCG
GCGCCCCCGGCTCCGCCGGCTCCGCCGCCGGCTCCGGCGCGTGCCCGGTGCCGCTG
CAGCTGCCGCCGCTGGAACGCCTGACCCTGGATGATAAAAAACCGCTGAATACCCTGA
TCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAA
CAATAGCCGCTCTTCTCGTACAGCACGTGCACTGCGTCACCACAAATATCGTAAAACCT
GTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAAT
GCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTC
TGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATC
AGCTCCAGCACTGACAAAATCCCAAACCGATCGTCTGGAGGTTCTGCTGAATCCGAAAG
ACGAAATCAGCCTGAATTCCGGCAAACCGTTTCGTGAACTGGAGAGCGAACTGCTGTCA
CGTCGTAAAAAAGACCTGCAACAAATCTATGCCGAAGAACGTGAGAACTATCTGGGGAA
 
ACTGGAACGTGAAATCACCCGCTTTTTCGTGGATCGTGGCTTTCTGGAGATCAAATCCC
CGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGA
GATCGGCCCGTGTTATCGTAAAGAGTCCGACGGTAAAGAACATCTGGAGGAGTTTACCA
TGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGT
GTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTT
TTGGTCTGGAGCGTCTGCTGAAAGTAAAACACGACTTCAAGAACATCAAACGTGCTGCA
CGTTCCGAGTCCTATTACAATGGTATTTCTACTAACCTGTAA (SEQ ID NO:41)
Protein:

MASNDYTQQATQSYGAYPTQPGQGYSQQSSQPYGQQSYSGYSQSTDTSGYGQSSYSSY
GQSQNTGYGTQSTPQGYGSTGGYGSSQSSQSSYGQQSSYPGYGQQPAPSSTSGSYGSS
SQSSSYGQPQSGSYSQQPSYGGQQQSYGQQQSYNPPQGYGQQNQYNSSSGGGGGGG
GGGNYGQDQSSMSSGGGSGGGYGNQDQSGGGGSGGYGQQDRGGRGRGGSGGGGGG
GGGGYNRSSGGYEPRGRGGGRGGRGGMGGSDRGGFNKFGGPRDQGSRHDSEQDNSD
NNTIFVQGLGENVTIESVADYFKQIGIIKTNKKTGQPMINLYTDRETGKLKGEATVSFDDPPSA
KAAIDWFDGKEFSGNPIKVSFATRRADFNRGGGNGRGGRGRGGPMGRGGYGGGGSGGG
GRGGFPSGGGGGGGQQRAGDWKCPNPTCENMNFSWRNECNQCKAPKPDGPGGGPGG
SHMGGNYGDDRRGGRGGDYKDDDDKGTGAPGSAGSAAGSGASNFTQFVLVDNGGTGDV
TVAPSNFANGIAEWISSNSRSQAYKVTCSVRQSSAQNRKYTIKVEVPKGAWRSYLNMELTIP
IFATNSDCELIVKAMQGLLKDGNPIPSAIAANSGIYGTGAPGSAGSAAGSGACPVPLQLPPLE
RLTLDDKKPLNTLISATGLWMSRTGTIHKIKHHEVSRSKIYIEMACGDHLVVNNSRSSRTARA
LRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPV
QASAPALTKSQTDRLEVLLNPKDEISLNSGKPFRELESELLSRRKKDLQQIYAEERENYLGKL
EREITRFFVDRGFLEIKSPILIPLEYIERMGIDNDTELSKQIFRVDKNFCLRPMLAPNLANYLRK
LDRALPDPIKIFEIGPCYRKESDGKEHLEEFTMLNFCQMGSGCTRENLESIITDFLNHLGIDFK
IVGDSCMVFGDTLDVMHGDLELSSAVVGPIPLDREWGIDKPWIGAGFGLERLLKVKHDFKNI
KRAARSESYYNGISTNL (SEQ ID NO:42)

MCP-PylRS^{AF}
DNA: 
ATGGCTTCTAACTTTACTCAGTTCGTTCTCGTCGACAATGGCGGAACTGGCGACGTGAC
TGTCGCCCCAAGCAACTTCGCTAACGGGATCGCTGAATGGATCAGCTCTAACTCGCGTT
 
CACAGGCTTACAAAGTAACCTGTAGCGTTCGTCAGAGCTCTGCGCAGAATCGCAAATAC
ACCATCAAAGTCGAGGTGCCTAAAGGCGCCTGGCGTTCGTACTTAAATATGGAACTAAC
CATTCCAATTTTCGCCACGAATTCCGACTGCGAGCTTATTGTTAAGGCAATGCAAGGTCT
CCTAAAAGATGGAAACCCGATTCCCTCAGCAATCGCAGCAAACTCCGGCATCTACGGTA
CCGGCGCCCCCGGCTCCGCCGGCTCCGCCGCCGGCTCCGGCGCGTGCCCGGTGCCG
CTGCAGCTGCCGCCGCTGGAACGCCTGACCCTGGATGATAAAAAACCGCTGAATACCC
TGATCTCTGCTACTGGTCTGTGGATGAGTCGTACCGGAACCATTCATAAAATCAAACAC
CACGAGGTTAGCCGTTCGAAAATCTATATTGAGATGGCGTGTGGCGATCATCTGGTTGT
GAACAATAGCCGCTCTTCTCGTACAGCACGTGCACTGCGTCACCACAAATATCGTAAAA
CCTGTAAACGTTGCCGTGTGTCCGATGAGGATCTGAACAAATTCCTGACAAAAGCCAAT
GAGGACCAAACAAGCGTGAAAGTGAAAGTCGTTAGCGCTCCTACCCGTACTAAAAAAGC
AATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGT
TTCTGTTCCAGCAAGTGTGAGCACCAGCATTAGCAGTATTAGCACCGGTGCCACCGCTA
GCGCCCTGGTTAAAGGCAATACCAATCCGATTACAAGCATGTCTGCCCCGGTTCAAGCA
TCAGCTCCAGCACTGACAAAATCCCAAACCGATCGTCTGGAGGTTCTGCTGAATCCGAA
AGACGAAATCAGCCTGAATTCCGGCAAACCGTTTCGTGAACTGGAGAGCGAACTGCTGT
CACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATC
CCCGATTCTGATTCCTCTGGAGTATATCGAGCGTATGGGCATCGACAATGATACCGAAC
TGAGCAAACAAATTTTCCGTGTGGATAAAAACTTCTGTCTGCGCCCTATGCTAGCACCAA
ATCTGGCTAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTAC
CATGCTGAACTTTTGCCAAATGGGTTCAGGTTGTACTCGTGAGAACCTGGAAAGCATCA
TCACCGATTTTCTGAACCACCTGGGCATTGACTTCAAAATTGTGGGCGACAGCTGTATG
GTGTTTGGCGACACCCTGGATGTCATGCACGGCGACCTGGAACTGTCTAGTGCCGTTG
TGGGCCCAATCCCGCTGGATCGTGAGTGGGGTATCGACAAACCTTGGATCGGTGCGGG
TTTTGGTCTGGAGCGTCTGCTGAAAGTAAAACACGACTTCAAGAACATCAAACGTGCTG
CACGTTCCGAGTCCTATTACAATGGTATTTCTACTAACCTGTAA (SEQ ID NO:43)
Protein:

MASNFTQFVLVDNGGTGDVTVAPSNFANGIAEWISSNSRSQAYKVTCSVRQSSAQNRKYTI
KVEVPKGAWRSYLNMELTIPIFATNSDCELIVKAMQGLLKDGNPIPSAIAANSGIYGTGAPGS
AGSAAGSGACPVPLQLPPLERLTLDDKKPLNTLISATGLWMSRTGTIHKIKHHEVSRSKIYIE
MACGDHLVVNNSRSSRTARALRHHKYRKTCKRCRVSDEDLNKFLTKANEDQTSVKVKVVS
APTRTKKAMPKSVARAPKPLENTEAAQAQPSGSKFSPAIPVSTQESVSVPASVSTSISSIST
GATASALVKGNTNPITSMSAPVQASAPALTKSQTDRLEVLLNPKDEISLNSGKPFRELESELL

SRRKKDLQQIYAEERENYLGKLEREITRFFVDRGFLEIKSPILIPLEYIERMGIDNDTELSKQIF
RVDKNFCLRPMLAPNLANYLRKLDRALPDPIKIFEIGPCYRKESDGKEHLEEFTMLNFCQMG
SGCTRENLESIITDFLNHLGIDFKIVGDSCMVFGDTLDVMHGDLELSSAVVGPIPLDREWGID
KPWIGAGFGLERLLKVKHDFKNIKRAARSESYYNGISTNL (SEQ ID NO:44)

SPD5-MCP
DNA: 
ATGGAGGACAACAGCGTGCTGAACGAGGACAGCAACCTGGAGCACGTGGAGGGCCAG
CCCAGAAGAAGCATGAGCCAGCCCGTGCTGAACGTGGAGGGCGACAAGAGAACCAGC
AGCACCAGCGCCACCCAGCAGCAGGTGCTGAGCGGCGCCTTCAGCAGCGCCGACGTG
AGAAGCATCCCCATCATCCAGACCTGGGAGGAGAACAAGGCCCTGAAGACCAAGATCA
CCATCCTGAGAGGCGAGCTGCAGATGTACCAGAGAAGATACAGCGAGGCCAAGGAGG
CCAGCCAGAAGAGAGTGAAGGAGGTGATGGACGACTACGTGGACCTGAAGCTGGGCC
AGGAGAACGTGCAGGAGAAGATGGAGCAGTACAAGCTGATGGAGGAGGACCTGCTGG
CCATGCAGAGCAGAATCGAGACCAGCGAGGACAACTTCGCCAGACAGATGAAGGAGTT
CGAGGCCCAGAAGCACGCCATGGAGGAGAGAATCAAGGAGCTGGAGCTGAGCGCCAC
CGACGCCAACAACACCACCGTGGGCAGCTTCAGAGGCACCCTGGACGACATCCTGAAG
AAGAACGACCCCGACTTCACCCTGACCAGCGGCTACGAGGAGAGAAAGATCAACGACC
TGGAGGCCAAGCTGCTGAGCGAGATCGACAAGGTGGCCGAGCTGGAGGACCACATCC
AGCAGCTGAGACAGGAGCTGGACGACCAGAGCGCCAGACTGGCCGACAGCGAGAACG
TGAGAGCCCAGCTGGAGGCCGCCACCGGCCAGGGCATCCTGGGCGCCGCCGGCAAC
GCCATGGTGCCCAACAGCACCTTCATGATCGGCAACGGCAGAGAGAGCCAGACCAGAG
ACCAGCTGAACTACATCGACGACCTGGAGACCAAGCTGGCCGACGCCAAGAAGGAGAA
CGACAAGGCCAGACAGGCCCTGGTGGAGTACATGAACAAGTGCAGCAAGCTGGAGCA
CGAGATCAGAACCATGGTGAAGAACAGCACCTTCGACAGCAGCAGCATGCTGCTGGGC
GGCCAGACCAGCGACGAGCTGAAGATCCAGATCGGCAAGGTGAACGGCGAGCTGAAC
GTGCTGAGAGCCGAGAACAGAGAGCTGAGAATCAGATGCGACCAGCTGACCGGCGGC
GACGGCAACCTGAGCATCAGCCTGGGCCAGAGCAGACTGATGGCCGGCATCGCCACC
AACGACGTGGACAGCATCGGCCAGGGCAACGAGACCGGCGGCACCAGCATGAGAATC
CTGCCCAGAGAGAGCCAGCTGGACGACCTGGAGGAGCAAGCTGCCCCTGATGGAC
ACCAGCAGCGCCGTGAGAAACCAGCAGCAGTTCGCCAGCATGTGGAGGACTTCGAG
AGCGTGAAGGACAGCCTGCAGAACAACCACAACGACACCCTGGAGGGCAGCTTCAACA
GCAGCATGCCCCCCCCCGGCAGAGACGCCACCCAGAGCTTCCTGAGCCAGAAGAGCT
TCAAGAACAGCCCCATCGTGATGCAGAAGCCCAAGAGCCTGCACCTGCACCTGAAGAG
CCACCAGAGCGAGGGCGCCGGCGAGCAGATCCAGAACAACAGCTTCAGCACCAAGAC
CGCCAGCCCCCACGTGAGCCAGAGCCACATCCCCATCCTGCACGACATGCAGCAGATC
 
CTGGACAGCAGCGCCATGTTCCTGGAGGGCCAGCACGACGTGGCCGTGAACGTGGAG
CAGATGCAGGAGAAGATGAGCCAGATCAGAGAGGCCCTGGCCAGACTGTTCGAGAGAC
TGAAGAGCAGCGCCGCCCTGTTCGAGGAGATCCTGGAGAGAATGGGCAGCAGCGACC
CCAACGCCGACAAGATCAAGAAGATGAAGCTGGCCTTCGAGACCAGCATCAACGACAA
GCTGAACGTGAGCGCCATCCTGGAGGCCGCCGAGAAGGACCTGCACAACATGAGCCT
GAACTTCAGCATCCTGGAGAAGAGCATCGTGAGCCAGGCCGCCGAGGCCAGCAGAAG
ATTCACCATCGCCCCCGACGCCGAGGACGTGGCCAGCAGCAGCCTGCTGAACGCCAG
CTACAGCCCCCTGTTCAAGTTCACCAGCAACAGCGACATCGTGGAGAAGCTGCAGAAC
GAGGTGAGCGAGCTGAAGAACGAGCTGGAGATGGCCAGAACCAGAGACATGAAGC
CCCCTGAACGGCAGCAGCGGCAGACTGAGCGACGTGCAGATCAACACCAACAGAATGT
TCGAGGACCTGGAGGTGAGCGAGGCCACCCTGCAGAAGGCCAAGGAGGAGAACAGCA
CCCTGAAGAGCCAGTTCGCCGAGCTGGAGGCCAACCTGCACCAGGTGAACAGCAAGCT
GGGCGAGGTGAGATGCGAGCTGAACGAGGCCCTGGCCAGAGTGGACGGCAGCAGG
AGACCAGAGTGAAGGCCGAGAACGCCCTGGAGGAGGCCAGACAGCTGATCAGCAGCC
TGAAGCACGAGGAGAACGAGCTGAAGAAGACCATCACCGACATGGGCATGAGACTGAA
CGAGGCCAAGAAGAGCGACGAGTTCCTGAAGAGCGAGCTGAGCACCGCCCTGGAGGA
GGAGAAGAAGAGCCAGAACCTGGCCGACGAGCTGAGCGAGGAGCTGAACGGCTGGAG
AATGAGAACCAAGGAGGCCGAGAACAAGGTGGAGCACGCCAGCAGCGAGAAGAGCGA
GATGCTGGAGAGAATCGTGCACCTGGAGACCGAGATGGAGAAGCTGAGCACCAGCGA
GATCGCCGCCGACTACTGCAGCACCAAGATGACCGAGAGAAAGAAGGAGATCGAGCTG
GCCAAGTACAGAGAGGACTTCGAGAACGCCGCCATCGTGGCCTGGAGAGAATCAGCA
AGGAGATCAGCGAGCTGACCAAGAAGACCCTGAAGGCCAAGATCATCCCCAGCAACAT
CAGCAGCATCCAGCTGGTGTGCGACGAGCTGTGCAGAAGACTGAGCAGAGAGAGA
GCAGCAGCACGAGTACGCCAAGGTGATGAGAGACGTGAACGAGAAGATCGAGAAGCT
GCAGCTGGAGAAGGACGCCCTGGAGCACGAGCTGAAGATGATGAGCAGCAACAACGA
GAACGTGCCCCCCGTGGGCACCAGCGTGAGCGGCATGCCCACCAAGACCAGCAACCA
GAAGTGCGCCCAGCCCCACTACACCAGCCCCACCAGACAGCTGCTGCACGAGAGCAC
CATGGCCGTGGACGCCATCGTGCAGAAGCTGAAGAAGACCCACAACATGAGCGGCATG
GGCCCCGAGCTGAAGGAGACCATCGGCAACGTGATCAACGAGAGCAGAGTGCTGAGA
GACTTCCTGCACCAGAAGCTGATCCTGTTCAAGGGCATCGACATGAGCAACTGGAAGAA
CGAGACCGTGGACCAGCTGATCACCGACCTGGGCCAGCTGCACCAGGACAACCTGAT
GCTGGAGGAGCAGATCAAGAAGTACAAGGAGCTGAAGCTGACCAAGAGCGCCATC
CCCACCCTGGGCGTGGAGTTCCAGGACAGAATCAAGACCGAGATCGGCAAGATCGCCA
CCGACATGGGCGGCGCCGTGAAGGAGATCAGAAAGAAGGGTACCGAGCAGAAGCTGA
TCTCAGAGGAGGACCTGGGCGCCCCCGGCTCCGCCGGCTCCGCCGCCGGCTCCGGC
GCTTCTAACTTTACTCAGTTCGTTCTCGTCGACAATGGCGGAACTGGCGACGTGACTGT
 
CGCCCCAAGCAACTTCGCTAACGGGATCGCTGAATGGATCAGCTCTAACTCGCGTTCAC
AGGCTTACAAAGTAACCTGTAGCGTTCGTCAGAGCTCTGCGCAGAATCGCAAATACACC
ATCAAAGTCGAGGTGCCTAAAGGCGCCTGGCGTTCGTACTTAAATATGGAACTAACCAT
TCCAATTTTCGCCACGAATTCCGACTGCGAGCTTATTGTTAAGGCAATGCAAGGTCTCCT
AAAAGATGGAAACCCGATTCCCTCAGCAATCGCAGCAAACTCCGGCATCTACTAA (SEQ
ID NO:45)
Protein:

MEDNSVLNEDSNLEHVEGQPRRSMSQPVLNVEGDKRTSSTSATQQQVLSGAFSSADVRSI
PIIQTWEENKALKTKITILRGELQMYQRRYSEAKEASQKRVKEVMDDYVDLKLGQENVQEK
MEQYKLMEEDLLAMQSRIETSEDNFARQMKEFEAQKHAMEERIKELELSATDANNTTVGSF
RGTLDDILKKNDPDFTLTSGYEERKINDLEAKLLSEIDKVAELEDHIQQLRQELDDQSARLAD
SENVRAQLEAATGQGILGAAGNAMVPNSTFMIGNGRESQTRDQLNYIDDLETKLADAKKEN
DKARQALVEYMNKCSKLEHEIRTMVKNSTFDSSSMLLGGQTSDELKIQIGKVNGELNVLRAE
NRELRIRCDQLTGGDGNLSISLGQSRLMAGIATNDVDSIGQGNETGGTSMRILPRESQLDDL
EESKLPLMDTSSAVRNQQQFASMWEDFESVKDSLQNNHNDTLEGSFNSSMPPPGRDATQ
SFLSQKSFKNSPIVMQKPKSLHLHLKSHQSEGAGEQIQNNSFSTKTASPHVSQSHIPILHDM
QQILDSSAMFLEGQHDVAVNVEQMQEKMSQIREALARLFERLKSSAALFEEILERMGSSDP
NADKIKKMKLAFETSINDKLNVSAILEAAEKDLHNMSLNFSILEKSIVSQAAEASRRFTIAPDAE
DVASSSLLNASYSPLFKFTSNSDIVEKLQNEVSELKNELEMARTRDMRSPLNGSSGRLSDV
QINTNRMFEDLEVSEATLQKAKEENSTLKSQFAELEANLHQVNSKLGEVRCELNEALARVD
GEQETRVKAENALEEARQLISSLKHEENELKKTITDMGMRLNEAKKSDEFLKSELSTALEEE
KKSQNLADELSEELNGWRMRTKEAENKVEHASSEKSEMLERIVHLETEMEKLSTSEIAADY
CSTKMTERKKEIELAKYREDFENAAIVGLERISKEISELTKKTLKAKIIPSNISSIQLVCDELCRR
LSREREQQHEYAKVMRDVNEKIEKLQLEKDALEHELKMMSSNNENVPPVGTSVSGMPTKT
SNQKCAQPHYTSPTRQLLHESTMAVDAIVQKLKKTHNMSGMGPELKETIGNVINESRVLRD
FLHQKLILFKGIDMSNWKNETVDQLITDLGQLHQDNLMLEEQIKKYKKELKLTKSAIPTLGVEF
QDRIKTEIGKIATDMGGAVKEIRKKGTEQKLISEEDLGAPGSAGSAAGSGASNFTQFVLVDN
GGTGDVTVAPSNFANGIAEWISSNSRSQAYKVTCSVRQSSAQNRKYTIKVEVPKGAWRSYL
NMELTIPIFATNSDCELIVKAMQGLLKDGNPIPSAIAANSGIY (SEQ ID NO:46)

SPD5-PylRS^{AF}
DNA: 
ATGGAGGACAACAGCGTGCTGAACGAGGACAGCAACCTGGAGCACGTGGAGGGCCAG
CCCAGAAGAAGCATGAGCCAGCCCGTGCTGAACGTGGAGGGCGACAAGAGAACCAGC
AGCACCAGCGCCACCCAGCAGCAGGTGCTGAGCGGCGCCTTCAGCAGCGCCGACGTG
AGAAGCATCCCCATCATCCAGACCTGGGAGGAGAACAAGGCCCTGAAGACCAAGATCA
 
CCATCCTGAGAGGCGAGCTGCAGATGTACCAGAGAAGATACAGCGAGGCCAAGGAGG
CCAGCCAGAAGAGAGTGAAGGAGGTGATGGACGACTACGTGGACCTGAAGCTGGGCC
AGGAGAACGTGCAGGAGAAGATGGAGCAGTACAAGCTGATGGAGGAGGACCTGCTGG
CCATGCAGAGCAGAATCGAGACCAGCGAGGACAACTTCGCCAGACAGATGAAGGAGTT
CGAGGCCCAGAAGCACGCCATGGAGGAGAGAATCAAGGAGCTGGAGCTGAGCGCCAC
CGACGCCAACAACACCACCGTGGGCAGCTTCAGAGGCACCCTGGACGACATCCTGAAG
AAGAACGACCCCGACTTCACCCTGACCAGCGGCTACGAGGAGAGAAAGATCAACGACC
TGGAGGCCAAGCTGCTGAGCGAGATCGACAAGGTGGCCGAGCTGGAGGACCACATCC
AGCAGCTGAGACAGGAGCTGGACGACCAGAGCGCCAGACTGGCCGACAGCGAGAACG
TGAGAGCCCAGCTGGAGGCCGCCACCGGCCAGGGCATCCTGGGCGCCGCCGGCAAC
GCCATGGTGCCCAACAGCACCTTCATGATCGGCAACGGCAGAGAGAGCCAGACCAGAG
ACCAGCTGAACTACATCGACGACCTGGAGACCAAGCTGGCCGACGCCAAGAAGGAGAA
CGACAAGGCCAGACAGGCCCTGGTGGAGTACATGAACAAGTGCAGCAAGCTGGAGCA
CGAGATCAGAACCATGGTGAAGAACAGCACCTTCGACAGCAGCAGCATGCTGCTGGGC
GGCCAGACCAGCGACGAGCTGAAGATCCAGATCGGCAAGGTGAACGGCGAGCTGAAC
GTGCTGAGAGCCGAGAACAGAGAGCTGAGAATCAGATGCGACCAGCTGACCGGCGGC
GACGGCAACCTGAGCATCAGCCTGGGCCAGAGCAGACTGATGGCCGGCATCGCCACC
AACGACGTGGACAGCATCGGCCAGGGCAACGAGACCGGCGGCACCAGCATGAGAATC
CTGCCCAGAGAGAGCCAGCTGGACGACCTGGAGGAGAGCAAGCTGCCCCTGATGGAC
ACCAGCAGCGCCGTGAGAAACCAGCAGCAGTTCGCCAGCATGTGGAGGACTTCGAG
AGCGTGAAGGACAGCCTGCAGAACAACCACAACGACACCCTGGAGGGCAGCTTCAACA
GCAGCATGCCCCCCCCCGGCAGAGACGCCACCCAGAGCTTCCTGAGCCAGAAGAGCT
TCAAGAACAGCCCCATCGTGATGCAGAAGCCCAAGAGCCTGCACCTGCACCTGAAGAG
CCACCAGAGCGAGGGCGCCGGCGAGCAGATCCAGAACAACAGCTTCAGCACCAAGAC
CGCCAGCCCCCACGTGAGCCAGAGCCACATCCCCATCCTGCACGACATGCAGCAGATC
CTGGACAGCAGCGCCATGTTCCTGGAGGGCCAGCACGACGTGGCCGTGAACGTGGAG
CAGATGCAGGAGAAGATGAGCCAGATCAGAGAGGCCCTGGCCAGACTGTTCGAGAGAC
TGAAGAGCAGCGCCGCCCTGTTCGAGGAGATCCTGGAGAGAATGGGCAGCAGCGACC
CCAACGCCGACAAGATCAAGAAGATGAAGCTGGCCTTCGAGACCAGCATCAACGACAA
GCTGAACGTGAGCGCCATCCTGGAGGCCGCCGAGAAGGACCTGCACAACATGAGCCT
GAACTTCAGCATCCTGGAGAAGAGCATCGTGAGCCAGGCCGCCGAGGCCAGCAGAAG
ATTCACCATCGCCCCCGACGCCGAGGACGTGGCCAGCAGCAGCCTGCTGAACGCCAG
CTACAGCCCCCTGTTCAAGTTCACCAGCAACAGCGACATCGTGGAGAAGCTGCAGAAC
GAGGTGAGCGAGCTGAAGAACGAGCTGGAGATGGCCAGAACCAGAGACATGAGAAGC
CCCCTGAACGGCAGCAGCGGCAGACTGAGCGACGTGCAGATCAACACCAACAGAATGT
TCGAGGACCTGGAGGTGAGCGAGGCCACCCTGCAGAAGGCCAAGGAGGAGAACAGCA
 
CCCTGAAGAGCCAGTTCGCCGAGCTGGAGGCCAACCTGCACCAGGTGAACAGCAAGCT
GGGCGAGGTGAGATGCGAGCTGAACGAGGCCCTGGCCAGAGTGGACGGCGAGCAGG
AGACCAGAGTGAAGGCCGAGAACGCCCTGGAGGAGGCCAGACAGCTGATCAGCAGCC
TGAAGCACGAGGAGAACGAGCTGAAGAAGACCATCACCGACATGGGCATGAGACTGAA
CGAGGCCAAGAAGAGCGACGAGTTCCTGAAGAGCGAGCTGAGCACCGCCCTGGAGGA
GGAGAAGAAGAGCCAGAACCTGGCCGACGAGCTGAGCGAGGAGCTGAACGGCTGGAG
AATGAGAACCAAGGAGGCCGAGAACAAGGTGGAGCACGCCAGCAGCGAGAAGAGCGA
GATGCTGGAGAGAATCGTGCACCTGGAGACCGAGATGGAGAAGCTGAGCACCAGCGA
GATCGCCGCCGACTACTGCAGCACCAAGATGACCGAGAGAAAGAAGGAGATCGAGCTG
GCCAAGTACAGAGAGGACTTCGAGAACGCCGCCATCGTGGCCTGGAGAGAATCAGCA
AGGAGATCAGCGAGCTGACCAAGAAGACCCTGAAGGCCAAGATCATCCCCAGCAACAT
CAGCAGCATCCAGCTGGTGTGCGACGAGCTGTGCAGAAGACTGAGCAGAGAGAGA
GCAGCAGCACGAGTACGCCAAGGTGATGAGAGACGTGAACGAGAAGATCGAGAAGCT
GCAGCTGGAGAAGGACGCCCTGGAGCACGAGCTGAAGATGATGAGCAGCAACAACGA
GAACGTGCCCCCCGTGGGCACCAGCGTGAGCGGCATGCCCACCAAGACCAGCAACCA
GAAGTGCGCCCAGCCCCACTACACCAGCCCCACCAGACAGCTGCTGCACGAGAGCAC
CATGGCCGTGGACGCCATCGTGCAGAAGCTGAAGAAGACCCACAACATGAGCGGCATG
GGCCCCGAGCTGAAGGAGACCATCGGCAACGTGATCAACGAGAGCAGAGTGCTGAGA
GACTTCCTGCACCAGAAGCTGATCCTGTTCAAGGGCATCGACATGAGCAACTGGAAGAA
CGAGACCGTGGACCAGCTGATCACCGACCTGGGCCAGCTGCACCAGGACAACCTGAT
GCTGGAGGAGCAGATCAAGAAGTACAAGGAGCTGAAGCTGACCAAGAGCGCCATC
CCCACCCTGGGCGTGGAGTTCCAGGACAGAATCAAGACCGAGATCGGCAAGATCGCCA
CCGACATGGGCGGCGCCGTGAAGGAGATCAGAAAGAAGGTACCGGCGCCCCCGGCT
CCGCCGGCTCCGCCGCCGGCTCCGGCGCGTGCCCGGTGCCGCTGCAGCTGCCGCCG
CTGGAACGCCTGACCCTGGATGATAAAAAACCGCTGAATACCCTGATCTCTGCTACTGG
TCTGTGGATGAGTCGTACCGGAACCATTCATAAAATCAAACACCACGAGGTTAGCCGTT
CGAAAATCTATATTGAGATGGCGTGTGGCGATCATCTGGTTGTGAACAATAGCCGCTCT
TCTCGTACAGCACGTGCACTGCGTCACCACAAATATCGTAAAACCTGTAAACGTTGCCG
TGTGTCCGATGAGGATCTGAACAAATTCCTGACAAAAGCCAATGAGGACCAAACAAGCG
TGAAAGTGAAAGTCGTTAGCGCTCCTACCCGTACTAAAAAAGCAATGCCGAAATCCGTT
GCTCGTGCCCCTAAACCACTGGAAAACACTGAAGCAGCACAGGCACAGCCGTCTGGAA
GCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAG
GCAATACCAATCCGATTACAAGCATGTCTGCCCCGGTTCAAGCATCAGCTCCAGCACTG
ACAAAATCCCAAACCGATCGTCTGGAGGTTCTGCTGAATCCGAAAGACGAAATCAGCCT
GAATTCCGGCAAACCGTTTCGTGAACTGGAGAGCGAACTGCTGTCACGTCGTAAAAAAG
 
ACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTT
CCGTGTGGATAAAAACTTCTGTCTGCGCCCTATGCTAGCACCAAATCTGGCTAACTATCT
GCGCAAACTGGACCGTGCCCTGCCTGATCCTATCAAAATCTTCGAGATCGGCCCGTGTT
ATCGTAAAGAGTCCGACGGTAAAGAACATCTGGAGGAGTTTACCATGCTGAACTTTTGC
CAAATGGGTTCAGGTTGTACTCGTGAGAACCTGGAAAGCATCATCACCGATTTTCTGAA
CCACCTGGGCATTGACTTCAAAATTGTGGGCGACAGCTGTATGGTGTTTGGCGACACCC
TGGATGTCATGCACGGCGACCTGGAACTGTCTAGTGCCGTTGTGGGCCCAATCCCGCT
GGATCGTGAGTGGGGTATCGACAAACCTTGGATCGGTGCGGGTTTTGGTCTGGAGCGT
CTGCTGAAAGTAAAACACGACTTCAAGAACATCAAACGTGCTGCACGTTCCGAGTCCTA
TTACAATGGTATTTCTACTAACCTGTAA (SEQ ID NO:47)
Protein:

MEDNSVLNEDSNLEHVEGQPRRSMSQPVLNVEGDKRTSSTSATQQQVLSGAFSSADVRSI
PIIQTWEENKALKTKITILRGELQMYQRRYSEAKEASQKRVKEVMDDYVDLKLGQENVQEK
MEQYKLMEEDLLAMQSRIETSEDNFARQMKEFEAQKHAMEERIKELELSATDANNTTVGSF
RGTLDDILKKNDPDFTLTSGYEERKINDLEAKLLSEIDKVAELEDHIQQLRQELDDQSARLAD
SENVRAQLEAATGQGILGAAGNAMVPNSTFMIGNGRESQTRDQLNYIDDLETKLADAKKEN
DKARQALVEYMNKCSKLEHEIRTMVKNSTFDSSSMLLGGQTSDELKIQIGKVNGELNVLRAE
NRELRIRCDQLTGGDGNLSISLGQSRLMAGIATNDVDSIGQGNETGGTSMRILPRESQLDDL
EESKLPLMDTSSAVRNQQQFASMWEDFESVKDSLQNNHNDTLEGSFNSSMPPPGRDATQ
SFLSQKSFKNSPIVMQKPKSLHLHLKSHQSEGAGEQIQNNSFSTKTASPHVSQSHIPILHDM
QQILDSSAMFLEGQHDVAVNVEQMQEKMSQIREALARLFERLKSSAALFEEILERMGSSDP
NADKIKKMKLAFETSINDKLNVSAILEAAEKDLHNMSLNFSILEKSIVSQAAEASRRFTIAPDAE
DVASSSLLNASYSPLFKFTSNSDIVEKLQNEVSELKNELEMARTRDMRSPLNGSSGRLSDV
QINTNRMFEDLEVSEATLQKAKEENSTLKSQFAELEANLHQVNSKLGEVRCELNEALARVD
GEQETRVKAENALEEARQLISSLKHEENELKKTITDMGMRLNEAKKSDEFLKSELSTALEEE
KKSQNLADELSEELNGWRMRTKEAENKVEHASSEKSEMLERIVHLETEMEKLSTSEIAADY
CSTKMTERKKEIELAKYREDFENAAIVGLERISKEISELTKKTLKAKIIPSNISSIQLVCDELCRR
LSREREQQHEYAKVMRDVNEKIEKLQLEKDALEHELKMMSSNNENVPPVGTSVSGMPTKT
SNQKCAQPHYTSPTRQLLHESTMAVDAIVQKLKKTHNMSGMGPELKETIGNVINESRVLRD
FLHQKLILFKGIDMSNWKNETVDQLITDLGQLHQDNLMLEEQIKKYKKELKLTKSAIPTLGVEF
QDRIKTEIGKIATDMGGAVKEIRKKGTGAPGSAGSAAGSGACPVPLQLPPLERLTLDDKKPL
NTLISATGLWMSRTGTIHKIKHHEVSRSKIYIEMACGDHLVVNNSRSSRTARALRHHKYRKT
CKRCRVSDEDLNKFLTKANEDQTSVKVKVVSAPTRTKKAMPKSVARAPKPLENTEAAQAQP
SGSKFSPAIPVSTQESVSVPASVSTSISSISTGATASALVKGNTNPITSMSAPVQASAPALTK

SQTDRLEVLLNPKDEISLNSGKPFRELESELLSRRKKDLQQIYAEERENYLGKLEREITRFFV
DRGFLEIKSPILIPLEYIERMGIDNDTELSKQIFRVDKNFCLRPMLAPNLANYLRKLDRALPDPI
KIFEIGPCYRKESDGKEHLEEFTMLNFCQMGSGCTRENLESIITDFLNHLGIDFKIVGDSCMV
FGDTLDVMHGDLELSSAVVGPIPLDREWGIDKPWIGAGFGLERLLKVKHDFKNIKRAARSES
YYNGISTNL (SEQ ID NO:48)

KIF16B-FUS-PylRS^{AF}
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGGAATCAGATTGCCCTCT
TAGACTCCCCCACATATACAGATATTGAAATGAACAGATTGGGAAAGGCGCCCCCGGC
TCCGCCGGCTCCGCCGCCGGCTCCGGCATGGCCTCAAACGATTATACCCAACAAGCAA
CCCAAAGCTATGGGGCCTACCCCACCCAGCCCGGGCAGGGCTATTCCCAGCAGAGCA
GTCAGCCCTACGGACAGCAGAGTTACAGTGGTTATAGCCAGTCCACGGACACTTCAGG
ATATGGCCAGAGCAGCTATTCTTCTTATGGCCAGAGCCAGAACACAGGCTATGGAACTC
AGTCAACTCCCCAGGGATATGGCTCGACTGGCGGCTATGGCAGTAGCCAGAGCTCCCA
ATCGTCTTACGGGCAGCAGTCCTCCTACCCTGGCTATGGCCAGCAGCCAGCTCCCAGC
AGCACCTCGGGAAGTTACGGTAGCAGTTCTCAGAGCAGCAGCTATGGGCAGCCCCAGA
 
GTGGGAGCTACAGCCAGCAGCCTAGCTATGGTGGACAGCAGCAAAGCTATGGACAGCA
GCAAAGCTATAATCCCCCTCAGGGCTATGGACAGCAGAACCAGTACAACAGCAGCAGT
GGTGGTGGAGGTGGAGGTGGAGGTGGAGGTAACTATGGCCAAGATCAATCCTCCATGA
GTAGTGGTGGTGGCAGTGGTGGCGGTTATGGCAATCAAGACCAGAGTGGTGGAGGTG
GCAGCGGTGGCTATGGACAGCAGGACCGTGGAGGCCGCGGCAGGGGTGGCAGTGGT
GGCGGCGGCGGCGGCGGCGGTGGTGGTTACAACCGCAGCAGTGGTGGCTATGAACC
CAGAGGTCGTGGAGGTGGCCGTGGAGGCAGAGGTGGCATGGGCGGAAGTGACCGTG
GTGGCTTCAATAAATTTGGTGGCCCTCGGGACCAAGGATCACGTCATGACTCCGAACAG
GATAATTCAGACAACAACACCATCTTTGTGCAAGGCCTGGGTGAGAATGTTACAATTGA
GTCTGTGGCTGATTACTTCAAGCAGATTGGTATTATTAAGACAAACAAGAAAACGGACA
GCCCATGATTAATTTGTACACAGACAGGGAAACTGGCAAGCTGAAGGGAGAGGCAACG
GTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGG
TGGTGGCAATGGTCGTGGAGGCCGAGGGCGAGGAGGACCCATGGGCCGTGGAGGCT
ATGGAGGTGGTGGCAGTGGTGGTGGTGGCCGAGGAGGATTTCCCAGTGGAGGTGGTG
GCGGTGGAGGACAGCAGCGAGCTGGTGACTGGAAGTGTCCTAATCCCACCTGTGAGAA
TATGAACTTCTCTTGGAGGAATGAATGCAACCAGTGTAAGGCCCCTAAACCAGATGGCC
CAGGAGGGGGACCAGGTGGCTCTCACATGGGGGGTAACTACGGGGATGATCGTCGTG
GTGGCAGAGGAGGCGATTACAAGGATGACGACGATAAGGGTACCGGCGCCCCCGGCT
CCGCCGGCTCCGCCGCCGGCTCCGGCGCGTGCCCGGTGCCGCTGCAGCTGCCGCCG
CTGGAACGCCTGACCCTGGATGATAAAAAACCGCTGAATACCCTGATCTCTGCTACTGG
TCTGTGGATGAGTCGTACCGGAACCATTCATAAAATCAAACACCACGAGGTTAGCCGTT
CGAAAATCTATATTGAGATGGCGTGTGGCGATCATCTGGTTGTGAACAATAGCCGCTCT
TCTCGTACAGCACGTGCACTGCGTCACCACAAATATCGTAAAACCTGTAAACGTTGCCG
TGTGTCCGATGAGGATCTGAACAAATTCCTGACAAAAGCCAATGAGGACCAAACAAGCG
TGAAAGTGAAAGTCGTTAGCGCTCCTACCCGTACTAAAAAAGCAATGCCGAAATCCGTT
GCTCGTGCCCCTAAACCACTGGAAAACACTGAAGCAGCACAGGCACAGCCGTCTGGAA
GCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAG
GCAATACCAATCCGATTACAAGCATGTCTGCCCCGGTTCAAGCATCAGCTCCAGCACTG
ACAAAATCCCAAACCGATCGTCTGGAGGTTCTGCTGAATCCGAAAGACGAAATCAGCCT
GAATTCCGGCAAACCGTTTCGTGAACTGGAGAGCGAACTGCTGTCACGTCGTAAAAAAG
ACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTT
CCGTGTGGATAAAAACTTCTGTCTGCGCCCTATGCTAGCACCAAATCTGGCTAACTATCT
 
GCGCAAACTGGACCGTGCCCTGCCTGATCCTATCAAAATCTTCGAGATCGGCCCGTGTT
ATCGTAAAGAGTCCGACGGTAAAGAACATCTGGAGGAGTTTACCATGCTGAACTTTTGC
CAAATGGGTTCAGGTTGTACTCGTGAGAACCTGGAAAGCATCATCACCGATTTTCTGAA
CCACCTGGGCATTGACTTCAAAATTGTGGGCGACAGCTGTATGGTGTTTGGCGACACCC
TGGATGTCATGCACGGCGACCTGGAACTGTCTAGTGCCGTTGTGGGCCCAATCCCGCT
GGATCGTGAGTGGGGTATCGACAAACCTTGGATCGGTGCGGGTTTTGGTCTGGAGCGT
CTGCTGAAAGTAAAACACGACTTCAAGAACATCAAACGTGCTGCACGTTCCGAGTCCTA
TTACAATGGTATTTCTACTAACCTGTAA (SEQ ID NO:49)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTYTDIEMNRLGKGAPGSAGSAAGSGMASNDYTQQAT
QSYGAYPTQPGQGYSQQSSQPYGQQSYSGYSQSTDTSGYGQSSYSSYGQSQNTGYGTQ
STPQGYGSTGGYGSSQSSQSSYGQQSSYPGYGQQPAPSSTSGSYGSSSQSSSYGQPQS
GSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGGYNRSSGG
YEPRGRGGGRGGRGGMGGSDRGGFNKFGGPRDQGSRHDSEQDNSDNNTIFVQGLGEN
VTIESVADYFKQIGIIKTNKKTGQPMINLYTDRETGKLKGEATVSFDDPPSAKAAIDWFDGKE
FSGNPIKVSFATRRADFNRGGGNGRGGRGRGGPMGRGGYGGGGSGGGGRGGFPSGGG
GGGGQQRAGDWKCPNPTCENMNFSWRNECNQCKAPKPDGPGGGPGGSHMGGNYGDD
RRGGRGGDYKDDDDKGTGAPGSAGSAAGSGACPVPLQLPPLERLTLDDKKPLNTLISATG
LWMSRTGTIHKIKHHEVSRSKIYIEMACGDHLVVNNSRSSRTARALRHHKYRKTCKRCRVS
DEDLNKFLTKANEDQTSVKVKVVSAPTRTKKAMPKSVARAPKPLENTEAAQAQPSGSKFSP
AIPVSTQESVSVPASVSTSISSISTGATASALVKGNTNPITSMSAPVQASAPALTKSQTDRLE
VLLNPKDEISLNSGKPFRELESELLSRRKKDLQQIYAEERENYLGKLEREITRFFVDRGFLEIK
SPILIPLEYIERMGIDNDTELSKQIFRVDKNFCLRPMLAPNLANYLRKLDRALPDPIKIFEIGPC
YRKESDGKEHLEEFTMLNFCQMGSGCTRENLESIITDFLNHLGIDFKIVGDSCMVFGDTLDV
MHGDLELSSAVVGPIPLDREWGIDKPWIGAGFGLERLLKVKHDFKNIKRAARSESYYNGIST
NL (SEQ ID NO:50)

KIF16B-VSV-G-FUS-PylRS^{AF}
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGAATCAGATTGCCCTCT
TAGACTCCCCCACATATACAGATATTGAAATGAACAGATTGGGAAAGGCGCCCCCGGC
TCCGCCGGCTCCGCCGCCGGCTCCGGCATGGCCTCAAACGATTATACCCAACAAGCAA
CCCAAAGCTATGGGGCCTACCCCACCCAGCCCGGGCAGGGCTATTCCCAGCAGAGCA
GTCAGCCCTACGGACAGCAGAGTTACAGTGGTTATAGCCAGTCCACGGACACTTCAGG
ATATGGCCAGAGCAGCTATTCTTCTTATGGCCAGAGCCAGAACACAGGCTATGGAACTC
AGTCAACTCCCCAGGGATATGGCTCGACTGGCGGCTATGGCAGTAGCCAGAGCTCCCA
ATCGTCTTACGGGCAGCAGTCCTCCTACCCTGGCTATGGCCAGCAGCCAGCTCCCAGC
AGCACCTCGGGAAGTTACGGTAGCAGTTCTCAGAGCAGCAGCTATGGGCAGCCCCAGA
GTGGGAGCTACAGCCAGCAGCCTAGCTATGGTGGACAGCAGCAAAGCTATGGACAGCA
GCAAAGCTATAATCCCCCTCAGGGCTATGGACAGCAGAACCAGTACAACAGCAGCAGT
GGTGGTGGAGGTGGAGGTGGAGGTGGAGGTAACTATGGCCAAGATCAATCCTCCATGA
GTAGTGGTGGTGGCAGTGGTGGCGGTTATGGCAATCAAGACCAGAGTGGTGGAGGTG
GCAGCGGTGGCTATGGACAGCAGGACCGTGGAGGCCGCGGCAGGGGTGGCAGTGGT
GGCGGCGGCGGCGGCGGCGGTGGTGGTTACAACCGCAGCAGTGGTGGCTATGAACC
CAGAGGTCGTGGAGGTGGCCGTGGAGGCAGAGGTGGCATGGGCGGAAGTGACCGTG
GTGGCTTCAATAAATTTGGTGGCCCTCGGGACCAAGGATCACGTCATGACTCCGAACAG
 
GATAATTCAGACAACAACACCATCTTTGTGCAAGGCCTGGGTGAGAATGTTACAATTGA
GTCTGTGGCTGATTACTTCAAGCAGATTGGTATTATTAAGACAAACAAGAAAACGGGACA
GCCCATGATTAATTTGTACACAGACAGGGAAACTGGCAAGCTGAAGGGAGAGGCAACG
GTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGG
TGGTGGCAATGGTCGTGGAGGCCGAGGGCGAGGAGGACCCATGGGCCGTGGAGGCT
ATGGAGGTGGTGGCAGTGGTGGTGGTGGCCGAGGAGGATTTCCCAGTGGAGGTGGTG
GCGGTGGAGGACAGCAGCGAGCTGGTGACTGGAAGTGTCCTAATCCCACCTGTGAGAA
TATGAACTTCTCTTGGAGGAATGAATGCAACCAGTGTAAGGCCCCTAAACCAGATGGCC
CAGGAGGGGGACCAGGTGGCTCTCACATGGGGGGTAACTACGGGGATGATCGTCGTG
GTGGCAGAGGTGGTGCGATCGCAGGAGCACCAGGAAGTGCTGGTTCTGCTGCTGGTA
GTGGAGCGTGCCCGGTGCCGCTGCAGCTGCCGCCGCTGGAACGCCTGACCCTGGATG
ATAAAAAACCGCTGAATACCCTGATCTCTGCTACTGGTCTGTGGATGAGTCGTACCGGA
ACCATTCATAAAATCAAACACCACGAGGTTAGCCGTTCGAAAATCTATATTGAGATGGCG
TGTGGCGATCATCTGGTTGTGAACAATAGCCGCTCTTCTCGTACAGCACGTGCACTGCG
TCACCACAAATATCGTAAAACCTGTAAACGTTGCCGTGTGTCCGATGAGGATCTGAACA
AATTCCTGACAAAAGCCAATGAGGACCAAACAAGCGTGAAAGTGAAAGTCGTTAGCGCT
CCTACCCGTACTAAAAAAGCAATGCCGAAATCCGTTGCTCGTGCCCCTAAACCACTGGA
AAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTAT
TAGCACCGGTGCCACCGCTAGCGCCCTGGTTAAAGGCAATACCAATCCGATTACAAGC
ATGTCTGCCCCGGTTCAAGCATCAGCTCCAGCACTGACAAAATCCCAAACCGATCGTCT
GGAGGTTCTGCTGAATCCGAAAGACGAAATCAGCCTGAATTCCGGCAAACCGTTTCGTG
AACTGGAGAGCGAACTGCTGTCACGTCGTAAAAAAGACCTGCAACAAATCTATGCCGAA
GAACGTGAGAACTATCTGGGGAAACTGGAACGTGAAATCACCCGCTTTTTCGTGGATCG
TGGCTTTCTGGAGATCAAATCCCCGATTCTGATTCCTCTGGAGTATATCGAGCGTATGG
GCATCGACAATGATACCGAACTGAGCAAACAAATTTTCCGTGTGGATAAAAACTTCTGTC
TGCGCCCTATGCTAGCACCAAATCTGGCTAACTATCTGCGCAAACTGGACCGTGCCCTG
CCTGATCCTATCAAAATCTTCGAGATCGGCCCGTGTTATCGTAAAGAGTCCGACGGTAA
AGAACATCTGGAGGAGTTTACCATGCTGAACTTTTGCCAAATGGGTTCAGGTTGTACTC
GTGAGAACCTGGAAAGCATCATCACCGATTTTCTGAACCACCTGGGCATTGACTTCAAA
ATTGTGGGCGACAGCTGTATGGTGTTTGGCGACACCCTGGATGTCATGCACGGCGACC
TGGAACTGTCTAGTGCCGTTGTGGGCCCAATCCCGCTGGATCGTGAGTGGGGTATCGA
CAAACCTTGGATCGGTGCGGGTTTTGGTCTGGAGCGTCTGCTGAAAGTAAAACACGACT
TCAAGAACATCAAACGTGCTGCACGTTCCGAGTCCTATTACAATGGTATTTCTACTAACC
TGTAA (SEQ ID NO:51)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTYTDIEMNRLGKGAPGSAGSAAGSGMASNDYTQQAT
QSYGAYPTQPGQGYSQQSSQPYGQQSYSGYSQSTDTSGYGQSSYSSYGQSQNTGYGTQ
STPQGYGSTGGYGSSQSSQSSYGQQSSYPGYGQQPAPSSTSGSYGSSSQSSSYGQPQS
GSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGYNRSSGG
YEPRGRGGGRGGRGGMGGSDRGGFNKFGGPRDQGSRHDSEQDNSDNNTIFVQGLGEN
VTIESVADYFKQIGIIKTNKKTGQPMINLYTDRETGKLKGEATVSFDDPPSAKAAIDWFDGKE
FSGNPIKVSFATRRADFNRGGGNGRGGRGRGGPMGRGGYGGGGSGGGGRGGFPSGGG
GGGGQQRAGDWKCPNPTCENMNFSWRNECNQCKAPKPDGPGGGPGGSHMGGNYGDD
RRGGRGGAIAGAPGSAGSAAGSGACPVPLQLPPLERLTLDDKKPLNTLISATGLWMSRTGT
IHKIKHHEVSRSKIYIEMACGDHLVVNNSRSSRTARALRHHKYRKTCKRCRVSDEDLNKFLT
KANEDQTSVKVKVVSAPTRTKKAMPKSVARAPKPLENTEAAQAQPSGSKFSPAIPVSTQES
VSVPASVSTSISSISTGATASALVKGNTNPITSMSAPVQASAPALTKSQTDRLEVLLNPKDEIS
LNSGKPFRELESELLSRRKKDLQQIYAEERENYLGKLEREITRFFVDRGFLEIKSPILIPLEYIE
RMGIDNDTELSKQIFRVDKNFCLRPMLAPNLANYLRKLDRALPDPIKIFEIGPCYRKESDGKE
HLEEFTMLNFCQMGSGCTRENLESIITDFLNHLGIDFKIVGDSCMVFGDTLDVMHGDLELSS
AVVGPIPLDREWGIDKPWIGAGFGLERLLKVKHDFKNIKRAARSESYYNGISTNL (SEQ ID
NO:52)

KIF16B-FUS-PylRS^{AA}
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
 
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGAATCAGATTGCCCTCT
TAGACTCCCCCACATATACAGATATTGAAATGAACAGATTGGGAAAGGCGCCCCCGGC
TCCGCCGGCTCCGCCGCCGGCTCCGGCATGGCCTCAAACGATTATACCCAACAAGCAA
CCCAAAGCTATGGGGCCTACCCCACCCAGCCCGGGCAGGGCTATTCCCAGCAGAGCA
GTCAGCCCTACGGACAGCAGAGTTACAGTGGTTATAGCCAGTCCACGGACACTTCAGG
ATATGGCCAGAGCAGCTATTCTTCTTATGGCCAGAGCCAGAACACAGGCTATGGAACTC
AGTCAACTCCCCAGGGATATGGCTCGACTGGCGGCTATGGCAGTAGCCAGAGCTCCCA
ATCGTCTTACGGGCAGCAGTCCTCCTACCCTGGCTATGGCCAGCAGCCAGCTCCCAGC
AGCACCTCGGGAAGTTACGGTAGCAGTTCTCAGAGCAGCAGCTATGGGCAGCCCCAGA
GTGGGAGCTACAGCCAGCAGCCTAGCTATGGTGGACAGCAGCAAAGCTATGGACAGCA
GCAAAGCTATAATCCCCCTCAGGGCTATGGACAGCAGAACCAGTACAACAGCAGCAGT
GGTGGTGGAGGTGGAGGTGGAGGTGGAGGTAACTATGGCCAAGATCAATCCTCCATGA
GTAGTGGTGGTGGCAGTGGTGGCGGTTATGGCAATCAAGACCAGAGTGGTGGAGGTG
GCAGCGGTGGCTATGGACAGCAGGACCGTGGAGGCCGCGGCAGGGGTGGCAGTGGT
GGCGGCGGCGGCGGCGGCGGTGGTGGTTACAACCGCAGCAGTGGTGGCTATGAACC
CAGAGGTCGTGGAGGTGGCCGTGGAGGCAGAGGTGGCATGGGCGGAAGTGACCGTG
GTGGCTTCAATAAATTTGGTGGCCCTCGGGACCAAGGATCACGTCATGACTCCGAACAG
GATAATTCAGACAACAACACCATCTTTGTGCAAGGCCTGGGTGAGAATGTTACAATTGA
GTCTGTGGCTGATTACTTCAAGCAGATTGGTATTATTAAGACAAACAAGAAAACGGACA
GCCCATGATTAATTTGTACACAGACAGGGAAACTGGCAAGCTGAAGGAGAGGCAACG
GTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGG
TGGTGGCAATGGTCGTGGAGGCCGAGGGCGAGGAGGACCCATGGGCCGTGGAGGCT
ATGGAGGTGGTGGCAGTGGTGGTGGTGGCCGAGGAGGATTTCCCAGTGGAGGTGGTG
GCGGTGGAGGACAGCAGCGAGCTGGTGACTGGAAGTGTCCTAATCCCACCTGTGAGAA
 
TATGAACTTCTCTTGGAGGAATGAATGCAACCAGTGTAAGGCCCCTAAACCAGATGGCC
CAGGAGGGGGACCAGGTGGCTCTCACATGGGGGGTAACTACGGGGATGATCGTCGTG
GTGGCAGAGGAGGCGGCGCCCCCGGCTCCGCCGGCTCCGCCGCCGGCTCCGGCATG
GCGTGCCCGGTGCCGCTGCAGCTGCCGCCGCTGGAACGCCTGACCCTGGATGACAAA
AAACCGCTGAATACCCTGATCTCTGCTACTGGTCTGTGGATGAGTCGTACCGGAACCAT
TCATAAAATCAAACACCACGAGGTTAGCCGTTCGAAAATCTATATTGAGATGGCGTGTG
GCGATCATCTGGTTGTGAACAATAGCCGCTCTTCTCGTACAGCACGTGCACTGCGTCAC
CACAAATATCGTAAAACCTGTAAACGTTGCCGTGTGTCCGATGAGGATCTGAACAAATTC
CTGACAAAAGCCAATGAGGACCAAACAAGCGTGAAAGTGAAAGTCGTTAGCGCTCCTAC
CCGTACTAAAAAAGCAATGCCGAAATCCGTTGCTCGTGCCCCTAAACCACTGGAAAACA
CTGAAGCAGCACAGGCACAGCCGTCTGGAAGCAAATTCTCTCCGGCCATTCCTGTTTCT
ACCCAGGAGTCCGTTTCTGTTCCAGCAAGTGTGAGCACCAGCATTAGCAGTATTAGCAC
CGGTGCCACCGCTAGCGCCCTGGTTAAAGGCAATACCAATCCGATTACAAGCATGTCTG
CCCCGGTTCAAGCATCAGCTCCAGCACTGACAAAATCCCAAACCGATCGTCTGGAGGTT
CTGCTGAATCCGAAAGACGAAATCAGCCTGAATTCCGGCAAACCGTTTCGTGAACTGGA
GAGCGAACTGCTGTCACGTCGTAAAAAGACCTGCAACAAATCTATGCCGAAGAACGTG
AGAACTATCTGGGGAAACTGGAACGTGAAATCACCCGCTTTTTCGTGGATCGTGGCTTT
CTGGAGATCAAATCCCCGATTCTGATTCCTCTGGAGTATATCGAGCGTATGGGCATCGA
CAATGATACCGAACTGAGCAAACAAATTTTCCGTGTGGATAAAAACTTCTGTCTGCGCCC
TATGCTGGCACCAAATCTGTATAACTATCTGCGCAAACTGGACCGTGCCCTGCCTGATC
CTATCAAAATCTTCGAGATCGGCCCGTGTTATCGTAAAGAGTCCGACGGTAAAGAACAT
CTGGAGGAGTTTACCATGCTGGCCTTTGCCCAAATGGGTTCAGGTTGTACTCGTGAGAA
CCTGGAAAGCATCATCACCGATTTTCTGAACCACCTGGGCATTGACTTCAAAATTGTGG
GCGACAGCTGTATGGTGTATGGCGACACCCTGGATGTCATGCACGGCGACCTGGAACT
GTCTAGTGCCGTTGTTGGACCAATTCCGCTGGACCGTGAGTGGGGTATCGACAAACCG
TGGATCGGAGCAGGATTCGGTCTGGAACGCCTGCTGAAAGTGAAACACGACTTCAAAA
ACATCAAACGTGCCGCCCGTTCTGAATCGTATTATAACGGGATCTCTACGAACCTGTAA
(SEQ ID NO:53)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTYTDIEMNRLGKGAPGSAGSAAGSGMASNDYTQQAT

QSYGAYPTQPGQGYSQQSSQPYGQQSYSGYSQSTDTSGYGQSSYSSYGQSQNTGYGTQ
STPQGYGSTGGYGSSQSSQSSYGQQSSYPGYGQQPAPSSTSGSYGSSSQSSSYGQPQS
GSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGYNRSSGG
YEPRGRGGGRGGRGGMGGSDRGGFNKFGGPRDQGSRHDSEQDNSDNNTIFVQGLGEN
VTIESVADYFKQIGIIKTNKKTGQPMINLYTDRETGKLKGEATVSFDDPPSAKAAIDWFDGKE
FSGNPIKVSFATRRADFNRGGGNGRGGRGRGGPMGRGGYGGGGSGGGGRGGFPSGGG
GGGGQQRAGDWKCPNPTCENMNFSWRNECNQCKAPKPDGPGGGPGGSHMGGNYGDD
RRGGRGGGAPGSAGSAAGSGMACPVPLQLPPLERLTLDDKKPLNTLISATGLWMSRTGTIH
KIKHHEVSRSKIYIEMACGDHLVVNNSRSSRTARALRHHKYRKTCKRCRVSDEDLNKFLTKA
NEDQTSVKVKVVSAPTRTKKAMPKSVARAPKPLENTEAAQAQPSGSKFSPAIPVSTQESVS
VPASVSTSISSISTGATASALVKGNTNPITSMSAPVQASAPALTKSQTDRLEVLLNPKDEISLN
SGKPFRELESELLSRRKKDLQQIYAEERENYLGKLEREITRFFVDRGFLEIKSPILIPLEYIERM
GIDNDTELSKQIFRVDKNFCLRPMLAPNLYNYLRKLDRALPDPIKIFEIGPCYRKESDGKEHL
EEFTMLAFAQMGSGCTRENLESIITDFLNHLGIDFKIVGDSCMVYGDTLDVMHGDLELSSAV
VGPIPLDREWGIDKPWIGAGFGLERLLKVKHDFKNIKRAARSESYYNGISTNL (SEQ ID
NO:54)

KIF16B-FUS-PylRS^{AAAF}
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
 
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGGAATCAGATTGCCCTCT
TAGACTCCCCCACATATACAGATATTGAAATGAACAGATTGGGAAAGGGCGCCCCCGGC
TCCGCCGGCTCCGCCGCCGGCTCCGGCATGGCCTCAAACGATTATACCCAACAAGCAA
CCCAAAGCTATGGGGCCTACCCCACCCAGCCCGGGCAGGGCTATTCCCAGCAGAGCA
GTCAGCCCTACGGACAGCAGAGTTACAGTGGTTATAGCCAGTCCACGGACACTTCAGG
ATATGGCCAGAGCAGCTATTCTTCTTATGGCCAGAGCCAGAACACAGGCTATGGAACTC
AGTCAACTCCCCAGGGATATGGCTCGACTGGCGGCTATGGCAGTAGCCAGAGCTCCCA
ATCGTCTTACGGGCAGCAGTCCTCCTACCCTGGCTATGGCCAGCAGCCAGCTCCCAGC
AGCACCTCGGGAAGTTACGGTAGCAGTTCTCAGAGCAGCAGCTATGGGCAGCCCCAGA
GTGGGAGCTACAGCCAGCAGCCTAGCTATGGTGGACAGCAGCAAAGCTATGGACAGCA
GCAAAGCTATAATCCCCCTCAGGGCTATGGACAGCAGAACCAGTACAACAGCAGCAGT
GGTGGTGGAGGTGGAGGTGGAGGTGGAGGTAACTATGGCCAAGATCAATCCTCCATGA
GTAGTGGTGGTGGCAGTGGTGGCGGTTATGGCAATCAAGACCAGAGTGGTGGAGGTG
GCAGCGGTGGCTATGGACAGCAGGACCGTGGAGGCCGCGGCAGGGGTGGCAGTGGT
GGCGGCGGCGGCGGCGGTGGTGGTTACAACCGCAGCAGTGGTGGCTATGAACC
CAGAGGTCGTGGAGGTGGCCGTGGAGGCAGAGGTGGCATGGGCGGAAGTGACCGTG
GTGGCTTCAATAAATTTGGTGGCCCTCGGGACCAAGGATCACGTCATGACTCCGAACAG
GATAATTCAGACAACAACACCATCTTTGTGCAAGGCCTGGGTGAGAATGTTACAATTGA
GTCTGTGGCTGATTACTTCAAGCAGATTGGTATTATTAAGACAAACAAGAAAACGGGACA
GCCCATGATTAATTTGTACACAGACAGGGAAACTGGCAAGCTGAAGGGAGAGGCAACG
GTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGG
TGGTGGCAATGGTCGTGGAGGCCGAGGGCGAGGAGGACCCATGGGCCGTGGAGGCT
ATGGAGGTGGTGGCAGTGGTGGTGGTGGCCGAGGAGGATTTCCCAGTGGAGGTGGTG
GCGGTGGAGGACAGCAGCGAGCTGGTGACTGGAAGTGTCCTAATCCCACCTGTGAGAA
TATGAACTTCTCTTGGAGGAATGAATGCAACCAGTGTAAGGCCCCTAAACCAGATGGCC
CAGGAGGGGGACCAGGTGGCTCTCACATGGGGGGTAACTACGGGGATGATCGTCGTG
GTGGCAGAGGAGGCGATTACAAGGATGACGACGATAAGGGTACCGGCGCCCCCGGCT
CCGCCGGCTCCGCCGCCGGCTCCGGCGCGTGCCCGGTGCCGCTGCAGCTGCCGCCG
CTGGAACGCCTGACCCTGGATGATAAAAAACCGCTGAATACCCTGATCTCTGCTACTGG
TCTGTGGATGAGTCGTACCGGAACCATTCATAAAATCAAACACCACGAGGTTAGCCGTT
CGAAAATCTATATTGAGATGGCGTGTGGCGATCATCTGGTTGTGAACAATAGCCGCTCT
TCTCGTACAGCACGTGCACTGCGTCACCACAAATATCGTAAAACCTGTAAACGTTGCCG
 
TGTGTCCGATGAGGATCTGAACAAATTCCTGACAAAAGCCAATGAGGACCAAACAAGCG
TGAAAGTGAAAGTCGTTAGCGCTCCTACCCGTACTAAAAAGCAATGCCGAAATCCGTT
GCTCGTGCCCCTAAACCACTGGAAAACACTGAAGCAGCACAGGCACAGCCGTCTGGAA
GCAAATTCTCTCCGGCCATTCCTGTTTCTACCCAGGAGTCCGTTTCTGTTCCAGCAAGT
GTGAGCACCAGCATTAGCAGTATTAGCACCGGTGCCACCGCTAGCGCCCTGGTTAAAG
GCAATACCAATCCGATTACAAGCATGTCTGCCCCGGTTCAAGCATCAGCTCCAGCACTG
ACAAAATCCCAAACCGATCGTCTGGAGGTTCTGCTGAATCCGAAAGACGAAATCAGCCT
GAATTCCGGCAAACCGTTTCGTGAACTGGAGAGCGAACTGCTGTCACGTCGTAAAAAG
ACCTGCAACAAATCTATGCCGAAGAACGTGAGAACTATCTGGGGAAACTGGAACGTGAA
ATCACCCGCTTTTTCGTGGATCGTGGCTTTCTGGAGATCAAATCCCCGATTCTGATTCCT
CTGGAGTATATCGAGCGTATGGGCATCGACAATGATACCGAACTGAGCAAACAAATTTT
CCGTGTGGATAAAAACTTCTGTCTGCGCCCTATGCTAGCACCAAATCTGGCTAACTATCT
GCGCAAACTGGACCGTGCCCTGCCTGATCCTATCAAAATCTTCGAGATCGGCCCGTGTT
ATCGTAAAGAGTCCGACGGTAAAGAACATCTGGAGGAGTTTACCATGCTGGCCTTTGCC
CAAATGGGTTCAGGTTGTACTCGTGAGAACCTGGAAAGCATCATCACCGATTTTCTGAA
CCACCTGGGCATTGACTTCAAAATTGTGGGCGACAGCTGTATGGTGTTTGGCGACACCC
TGGATGTCATGCACGGCGACCTGGAACTGTCTAGTGCCGTTGTGGGCCCAATCCCGCT
GGATCGTGAGTGGGGTATCGACAAACCTTGGATCGGTGCGGGTTTTGGTCTGGAGCGT
CTGCTGAAAGTAAAACACGACTTCAAGAACATCAAACGTGCTGCACGTTCCGAGTCCTA
TTACAATGGTATTTCTACTAACCTGTAA (SEQ ID NO:55)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTYTDIEMNRLGKGAPGSAGSAAGSGMASNDYTQQAT
QSYGAYPTQPGQGYSQQSSQPYGQQSYSGYSQSTDTSGYGQSSYSSYGQSQNTGYGTQ
STPQGYGSTGGYGSSQSSQSSYGQQSSYPGYGQQPAPSSTSGSYGSSSQSSSYGQPQS
GSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGYNRSSGG
YEPRGRGGGRGGRGGMGGSDRGGFNKFGGPRDQGSRHDSEQDNSDNNTIFVQGLGEN
VTIESVADYFKQIGIIKTNKKTGQPMINLYTDRETGKLKGEATVSFDDPPSAKAAIDWFDGKE
FSGNPIKVSFATRRADFNRGGGNGRGGRGRGGPMGRGGYGGGGSGGGGRGGFPSGGG
GGGGQQRAGDWKCPNPTCENMNFSWRNECNQCKAPKPDGPGGGPGGSHMGGNYGDD

RRGGRGGDYKDDDDKGTGAPGSAGSAAGSGACPVPLQLPPLERLTLDDKKPLNTLISATG
LWMSRTGTIHKIKHHEVSRSKIYIEMACGDHLVVNNSRSSRTARALRHHKYRKTCKRCRVS
DEDLNKFLTKANEDQTSVKVKVVSAPTRTKKAMPKSVARAPKPLENTEAAQAQPSGSKFSP
AIPVSTQESVSVPASVSTSISSISTGATASALVKGNTNPITSMSAPVQASAPALTKSQTDRLE
VLLNPKDEISLNSGKPFRELESELLSRRKKDLQQIYAEERENYLGKLEREITRFFVDRGFLEIK
SPILIPLEYIERMGIDNDTELSKQIFRVDKNFCLRPMLAPNLANYLRKLDRALPDPIKIFEIGPC
YRKESDGKEHLEEFTMLAFAQMGSGCTRENLESIITDFLNHLGIDFKIVGDSCMVFGDTLDV
MHGDLELSSAVVGPIPLDREWGIDKPWIGAGFGLERLLKVKHDFKNIKRAARSESYYNGIST
NL (SEQ ID NO:56)

KIF16B-EWSR1-MCP
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGGAATCAGATTGCCCTCT
TAGACTCCCCCACAATGGCGTCCACGGATTACAGTACCTATAGCCAAGCTGCAGCGCA
GCAGGGCTACAGTGCTTACACCGCCCAGCCCACTCAAGGATATGCACAGACCACCCAG
GCATATGGGCAACAAAGCTATGGAACCTATGGACAGCCCACTGATGTCAGCTATACCCA
GGCTCAGACCACTGCAACCTATGGGCAGACCGCCTATGCAACTTCTTATGGACAGCCTC
 
CCACTGGTTATACTACTCCAACTGCCCCCAGGCATACAGCCAGCCTGTCCAGGGGTAT
GGCACTGGTGCTTATGATACCACCACTGCTACAGTCACCACCACCCAGGCCTCCTATGC
AGCTCAGTCTGCATATGGCACTCAGCCTGCTTATCCAGCCTATGGGCAGCAGCCAGCA
GCCACTGCACCTACAAGACCGCAGGATGGAAACAAGCCCACTGAGACTAGTCAACCTC
AATCTAGCACAGGGGGTTACAACCAGCCCAGCCTAGGATATGGACAGAGTAACTACAGT
TATCCCCAGGTACCTGGGAGCTACCCCATGCAGCCAGTCACTGCACCTCCATCCTACC
CTCCTACCAGCTATTCCTCTACACAGCCGACTAGTTATGATCAGAGCAGTTACTCTCAGC
AGAACACCTATGGGCAACCGAGCAGCTATGGACAGCAGAGTAGCTATGGTCAACAAAG
CAGCTATGGGCAGCAGCCTCCCACTAGTTACCCACCCCAAACTGGATCCTACAGCCAA
GCTCCAAGTCAATATAGCCAACAGAGCAGCAGCTACGGGCAGCAGAGTTCATTCCGAC
AGGACCACCCCAGTAGCATGGGTGTTTATGGGCAGGAGTCTGGAGGATTTTCCGGACC
AGGAGAGAACCGGAGCATGAGTGGCCCTGATAACCGGGGCAGGGGAAGAGGGGGATT
TGATCGTGGAGGCATGAGCAGAGGTGGGCGGGGAGGAGGACGCGGTGGAATGGGCA
GCGCTGGAGAGCGAGGTGGCTTCAATAAGCCTGGTGGACCCATGGATGAAGGACCAG
ATCTTGATCTAGGCCCACCTGTAGATCCAGATGAAGACTCTGACAACAGTGCAATTTATG
TACAAGGATTAAATGACAGTGTGACTCTAGATGATCTGGCAGACTTCTTTAAGCAGTGTG
GGGTTGTTAAGATGAACAAGAGAACTGGGCAACCCATGATCCACATCTACCTGGACAAG
GAAACAGGAAAGCCCAAAGGCGATGCCACAGTGTCCTATGAAGACCCACCTACTGCCA
AGGCTGCCGTGGAATGGTTTGATGGGAAAGATTTTCAAGGGAGCAAACTTAAAGTCTCC
CTTGCTCGGAAGAAGCCTCCAATGAACAGTATGCGGGGTGGTCTGCCACCCCGTGAGG
GCAGAGGCATGCCACCACCACTCCGTGGAGGTCCAGGAGGCCCAGGAGGTCCTGGGG
GACCCATGGGTCGCATGGGAGGCCGTGGAGGAGATAGAGGAGGCTTCCCTCCAAGAG
GACCCCGGGGTTCCCGAGGGAACCCCTCTGGAGGAGGAAACGTCCAGCACCGAGCTG
GAGACTGGCAGTGTCCCAATCCGGGTTGTGGAAACCAGAACTTCGCCTGGAGAACAGA
GTGCAACCAGTGTAAGGCCCCAAAGCCTGAAGGCTTCCTCCCGCCACCCTTTCCGCCC
CCGGGTGGTGATCGTGGCAGAGGTGGCCCTGGTGGCATGCGGGGAGGAAGAGGTGG
CCTCATGGATCGTGGTGGTCCCGGTGGAATGTTCAGAGGTGGCCGTGGTGGAGACAGA
GGTGGCTTCCGTGGTGGCCGGGGCATGGACCGAGGTGGCTTTGGTGGAGGAAGACGA
GGTGGCCCTGGGGGGCCCCCTGGACCTTTGATGGAACAGGATTACAAGGATGACGAC
GATAAGGGTACCGAGCAGAAGCTGATCTCAGAGGAGGACCTGGGCGCCCCCGGCTCC
GCCGGCTCCGCCGCCGGCTCCGGCGCTTCTAACTTTACTCAGTTCGTTCTCGTCGACA
ATGGCGGAACTGGCGACGTGACTGTCGCCCCAAGCAACTTCGCTAACGGGATCGCTGA
ATGGATCAGCTCTAACTCGCGTTCACAGGCTTACAAAGTAACCTGTAGCGTTCGTCAGA
GCTCTGCGCAGAATCGCAAATACACCATCAAAGTCGAGGTGCCTAAAGGCGCCTGGCG
TTCGTACTTAAATATGGAACTAACCATTCCAATTTTCGCCACGAATTCCGACTGCGAGCT
 
TATTGTTAAGGCAATGCAAGGTCTCCTAAAAGATGGAAACCCGATTCCCTCAGCAATCG
CAGCAAACTCCGGCATCTACTAA (SEQ ID NO:57)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTMASTDYSTYSQAAAQQGYSAYTAQPTQGYAQTTQA
YGQQSYGTYGQPTDVSYTQAQTTATYGQTAYATSYGQPPTGYTTPTAPQAYSQPVQGYG
TGAYDTTTATVTTTQASYAAQSAYGTQPAYPAYGQQPAATAPTRPQDGNKPTETSQPQSS
TGGYNQPSLGYGQSNYSYPQVPGSYPMQPVTAPPSYPPTSYSSTQPTSYDQSSYSQQNT
YGQPSSYGQQSSYGQQSSYGQQPPTSYPPQTGSYSQAPSQYSQQSSSYGQQSSFRQDH
PSSMGVYGQESGGFSGPGENRSMSGPDNRGRGRGGFDRGGMSRGGRGGGRGGMGSA
GERGGFNKPGGPMDEGPDLDLGPPVDPDEDSDNSAIYVQGLNDSVTLDDLADFFKQCGVV
KMNKRTGQPMIHIYLDKETGKPKGDATVSYEDPPTAKAAVEWFDGKDFQGSKLKVSLARKK
PPMNSMRGGLPPREGRGMPPPLRGGPGGPGGPGGPMGRMGGRGGDRGGFPPRGPRG
SRGNPSGGGNVQHRAGDWQCPNPGCGNQNFAWRTECNQCKAPKPEGFLPPPFPPPGG
DRGRGGPGGMRGGRGGLMDRGGPGGMFRGGRGGDRGGFRGGRGMDRGGFGGGRRG
GPGGPPGPLMEQDYKDDDDKGTEQKLISEEDLGAPGSAGSAAGSGASNFTQFVLVDNGGT
GDVTVAPSNFANGIAEWISSNSRSQAYKVTCSVRQSSAQNRKYTIKVEVPKGAWRSYLNME
LTIPIFATNSDCELIVKAMQGLLKDGNPIPSAIAANSGIY (SEQ ID NO:58)

KIF16B-FUS-4xλ_{N22}-PylRS^{AF}
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
 
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGAATCAGATTGCCCTCT
TAGACTCCCCCACATATACAGATATTGAAATGAACAGATTGGGAAAGGCGCCCCCGGC
TCCGCCGGCTCCGCCGCCGGCTCCGGCATGGCCTCAAACGATTATACCCAACAAGCAA
CCCAAAGCTATGGGGCCTACCCCACCCAGCCCGGGCAGGGCTATTCCCAGCAGAGCA
GTCAGCCCTACGGACAGCAGAGTTACAGTGGTTATAGCCAGTCCACGGACACTTCAGG
ATATGGCCAGAGCAGCTATTCTTCTTATGGCCAGAGCCAGAACACAGGCTATGGAACTC
AGTCAACTCCCCAGGGATATGGCTCGACTGGCGGCTATGGCAGTAGCCAGAGCTCCCA
ATCGTCTTACGGGCAGCAGTCCTCCTACCCTGGCTATGGCCAGCAGCCAGCTCCCAGC
AGCACCTCGGGAAGTTACGGTAGCAGTTCTCAGAGCAGCAGCTATGGGCAGCCCAGA
GTGGGAGCTACAGCCAGCAGCCTAGCTATGGTGGACAGCAGCAAAGCTATGGACAGCA
GCAAAGCTATAATCCCCCTCAGGGCTATGGACAGCAGAACCAGTACAACAGCAGCAGT
GGTGGTGGAGGTGGAGGTGGAGGTGGAGGTAACTATGGCCAAGATCAATCCTCCATGA
GTAGTGGTGGTGGCAGTGGTGGCGGTTATGGCAATCAAGACCAGAGTGGTGGAGGTG
GCAGCGGTGGCTATGGACAGCAGGACCGTGGAGGCCGCGGCAGGGGTGGCAGTGGT
GGCGGCGGCGGCGGCGGTGGTGGTTACAACCGCAGCAGTGGTGGCTATGAACC
CAGAGGTCGTGGAGGTGGCCGTGGAGGCAGAGGTGGCATGGGCGGAAGTGACCGTG
GTGGCTTCAATAAATTTGGTGGCCCTCGGGACCAAGGATCACGTCATGACTCCGAACAG
GATAATTCAGACAACAACACCATCTTTGTGCAAGGCCTGGGTGAGAATGTTACAATTGA
GTCTGTGGCTGATTACTTCAAGCAGATTGGTATTATTAAGACAAACAAGAAAACGGACA
GCCCATGATTAATTTGTACACAGACAGGGAAACTGGCAAGCTGAAGGGAGAGGCAACG
GTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGG
TGGTGGCAATGGTCGTGGAGGCCGAGGGCGAGGAGGACCCATGGGCCGTGGAGGCT
ATGGAGGTGGTGGCAGTGGTGGTGGTGGCCGAGGAGGATTTCCCAGTGGAGGTGGTG
GCGGTGGAGGACAGCAGCGAGCTGGTGACTGGAAGTGTCCTAATCCCACCTGTGAGAA
TATGAACTTCTCTTGGAGGAATGAATGCAACCAGTGTAAGGCCCCTAAACCAGATGGCC
CAGGAGGGGGACCAGGTGGCTCTCACATGGGGGGTAACTACGGGGATGATCGTCGTG
 
GTGGCAGAGGAGGCGCCACCATGGACGCACAAACACGACGACGTGAGCGTCGCGCTG
AGAAACAAGCTCAATGGAAAGCTGCAAACCCACCGCTCGACGGAGCCGGAGCTGGCG
CTGGAGCTGGAGCCGGAGCTGGCGGTCTAGCCACCATGGACGCACAAACACGACGAC
GTGAGCGTCGCGCTGAGAAACAAGCTCAATGGAAAGCTGCAAACCCACCGCTCGACGG
AGCCGGAGCTGGCGCTGGAGCTGGAGCCGGAGCTGGCGGTCTAGCCACCATGGACGC
ACAAACACGACGACGTGAGCGTCGCGCTGAGAAACAAGCTCAATGGAAAGCTGCAAAC
CCACCGCTCGACGGAGCCGGAGCTGGCGCTGGAGCTGGAGCCGGAGCTGGCGGTCT
AGCCACCATGGACGCACAAACACGACGACGTGAGCGTCGCGCTGAGAAACAAGCTCAA
TGGAAAGCTGCAAACCCACCGCTCGATTACAAGGATGACGACGATAAGGGTACCGGCG
CCCCCGGCTCCGCCGGCTCCGCCGCCGGCTCCGGCGCGTGCCCGGTGCCGCTGCAG
CTGCCGCCGCTGGAACGCCTGACCCTGGATGATAAAAACCGCTGAATACCCTGATCT
CTGCTACTGGTCTGTGGATGAGTCGTACCGGAACCATTCATAAAATCAAACACCACGAG
GTTAGCCGTTCGAAAATCTATATTGAGATGGCGTGTGGCGATCATCTGGTTGTGAACAA
TAGCCGCTCTTCTCGTACAGCACGTGCACTGCGTCACCACAAATATCGTAAAACCTGTA
AACGTTGCCGTGTGTCCGATGAGGATCTGAACAAATTCCTGACAAAAGCCAATGAGGAC
CAAACAAGCGTGAAAGTGAAAGTCGTTAGCGCTCCTACCCGTACTAAAAAAGCAATGCC
GAAATCCGTTGCTCGTGCCCCTAAACCACTGGAAAACACTGAAGCAGCACAGGCACAG
CCGTCTGGAAGCAAATTCTCTCCGGCCATTCCTGTTTCTACCCAGGAGTCCGTTTCTGT
TCCAGCAAGTGTGAGCACCAGCATTAGCAGTATTAGCACCGGTGCCACCGCTAGCGCC
CTGGTTAAAGGCAATACCAATCCGATTACAAGCATGTCTGCCCCGGTTCAAGCATCAGC
TCCAGCACTGACAAAATCCCAAACCGATCGTCTGGAGGTTCTGCTGAATCCGAAAGACG
AAATCAGCCTGAATTCCGGCAAACCGTTTCGTGAACTGGAGAGCGAACTGCTGTCACGT
CGTAAAAAAGACCTGCAACAAATCTATGCCGAAGAACGTGAGAACTATCTGGGGAAACT
GGAACGTGAAATCACCCGCTTTTTCGTGGATCGTGGCTTTCTGGAGATCAAATCCCCGA
TTCTGATTCCTCTGGAGTATATCGAGCGTATGGGCATCGACAATGATACCGAACTGAGC
AAACAAATTTTCCGTGTGGATAAAAACTTCTGTCTGCGCCCTATGCTAGCACCAAATCTG
GCTAACTATCTGCGCAAACTGGACCGTGCCCTGCCTGATCCTATCAAAATCTTCGAGAT
CGGCCCGTGTTATCGTAAAGAGTCCGACGGTAAAGAACATCTGGAGGAGTTTACCATGC
TGAACTTTTGCCAAATGGGTTCAGGTTGTACTCGTGAGAACCTGGAAAGCATCATCACC
GATTTTCTGAACCACCTGGGCATTGACTTCAAAATTGTGGGCGACAGCTGTATGGTGTT
TGGCGACACCCTGGATGTCATGCACGGCGACCTGGAACTGTCTAGTGCCGTTGTGGGC
CCAATCCCGCTGGATCGTGAGTGGGGTATCGACAAACCTTGGATCGGTGCGGGTTTTG
GTCTGGAGCGTCTGCTGAAAGTAAAACACGACTTCAAGAACATCAAACGTGCTGCACGT
TCCGAGTCCTATTACAATGGTATTTCTACTAACCTGTAA (SEQ ID NO:59)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTYTDIEMNRLGKGAPGSAGSAAGSGMASNDYTQQAT
QSYGAYPTQPGQGYSQQSSQPYGQQSYSGYSQSTDTSGYGQSSYSSYGQSQNTGYGTQ
STPQGYGSTGGYGSSQSSQSSYGQQSSYPGYGQQPAPSSTSGSYGSSSQSSSYGQPQS
GSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGYNRSSGG
YEPRGRGGGRGGRGGMGGSDRGGFNKFGGPRDQGSRHDSEQDNSDNNTIFVQGLGEN
VTIESVADYFKQIGIIKTNKKTGQPMINLYTDRETGKLKGEATVSFDDPPSAKAAIDWFDGKE
FSGNPIKVSFATRRADFNRGGGNGRGGRGRGGPMGRGGYGGGGSGGGGRGGFPSGGG
GGGGQQRAGDWKCPNPTCENMNFSWRNECNQCKAPKPDGPGGGPGGSHMGGNYGDD
RRGGRGGATMDAQTRRRERRAEKQAQWKAANPPLDGAGAGAGAGAGAGGLATMDAQT
RRRERRAEKQAQWKAANPPLDGAGAGAGAGAGAGGLATMDAQTRRRERRAEKQAQWKA
ANPPLDGAGAGAGAGAGAGGLATMDAQTRRRERRAEKQAQWKAANPPLDYKDDDDKGT
GAPGSAGSAAGSGACPVPLQLPPLERLTLDDKKPLNTLISATGLWMSRTGTIHKIKHHEVSR
SKIYIEMACGDHLVVNNSRSSRTARALRHHKYRKTCKRCRVSDEDLNKFLTKANEDQTSVK
VKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFREL
ESELLSRRKKDLQQIYAEERENYLGKLEREITRFFVDRGFLEIKSPILIPLEYIERMGIDNDTEL
SKQIFRVDKNFCLRPMLAPNLANYLRKLDRALPDPIKIFEIGPCYRKESDGKEHLEEFTMLNF
CQMGSGCTRENLESIITDFLNHLGIDFKIVGDSCMVFGDTLDVMHGDLELSSAVVGPIPLDRE
WGIDKPWIGAGFGLERLLKVKHDFKNIKRAARSESYYNGISTNL (SEQ ID NO:60)

KIF16B-FUS-MCP-PylRS^{AF}
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
 
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGAATCAGATTGCCCTCT
TAGACTCCCCCACATATACAGATATTGAAATGAACAGATTGGGAAAGGCGCCCCCGGC
TCCGCCGGCTCCGCCGCCGGCTCCGGCATGGCCTCAAACGATTATACCCAACAAGCAA
CCCAAAGCTATGGGGCCTACCCCACCCAGCCCGGGCAGGGCTATTCCCAGCAGAGCA
GTCAGCCCTACGGACAGCAGAGTTACAGTGGTTATAGCCAGTCCACGGACACTTCAGG
ATATGGCCAGAGCAGCTATTCTTCTTATGGCCAGAGCCAGAACACAGGCTATGGAACTC
AGTCAACTCCCCAGGGATATGGCTCGACTGGCGGCTATGGCAGTAGCCAGAGCTCCCA
ATCGTCTTACGGGCAGCAGTCCTCCTACCCTGGCTATGGCCAGCAGCCAGCTCCCAGC
AGCACCTCGGGAAGTTACGGTAGCAGTTCTCAGAGCAGCAGCTATGGGCAGCCCCAGA
GTGGGAGCTACAGCCAGCAGCCTAGCTATGGTGGACAGCAGCAAAGCTATGGACAGCA
GCAAAGCTATAATCCCCCTCAGGGCTATGGACAGCAGAACCAGTACAACAGCAGCAGT
GGTGGTGGAGGTGGAGGTGGAGGTGGAGGTAACTATGGCCAAGATCAATCCTCCATGA
GTAGTGGTGGTGGCAGTGGTGGCGGTTATGGCAATCAAGACCAGAGTGGTGGAGGTG
GCAGCGGTGGCTATGGACAGCAGGACCGTGGAGGCCGCGGCAGGGGTGGCAGTGGT
GGCGGCGGCGGCGGCGGTGGTGGTTACAACCGCAGCAGTGGTGGCTATGAACC
CAGAGGTCGTGGAGGTGGCCGTGGAGGCAGAGGTGGCATGGGCGGAAGTGACCGTG
GTGGCTTCAATAAATTTGGTGGCCCTCGGGACCAAGGATCACGTCATGACTCCGAACAG
GATAATTCAGACAACAACACCATCTTTGTGCAAGGCCTGGGTGAGAATGTTACAATTGA
GTCTGTGGCTGATTACTTCAAGCAGATTGGTATTATTAAGACAAACAAGAAAACGGACA
GCCCATGATTAATTTGTACACAGACAGGGAAACTGGCAAGCTGAAGGGAGAGGCAACG
GTCTCTTTTGATGACCCACCTTCAGCTAAAGCAGCTATTGACTGGTTTGATGGTAAAGAA
TTCTCCGGAAATCCTATCAAGGTCTCATTTGCTACTCGCCGGGCAGACTTTAATCGGGG
TGGTGGCAATGGTCGTGGAGGCCGAGGGCGAGGAGGACCCATGGGCCGTGGAGGCT
ATGGAGGTGGTGGCAGTGGTGGTGGTGGCCGAGGAGGATTTCCCAGTGGAGGTGGTG
 
GCGGTGGAGGACAGCAGCGAGCTGGTGACTGGAAGTGTCCTAATCCCACCTGTGAGAA
TATGAACTTCTCTTGGAGGAATGAATGCAACCAGTGTAAGGCCCCTAAACCAGATGGCC
CAGGAGGGGGACCAGGTGGCTCTCACATGGGGGGTAACTACGGGGATGATCGTCGTG
GTGGCAGAGGAGGCGATTACAAGGATGACGACGATAAGGGTACCGGCGCCCCCGGCT
CCGCCGGCTCCGCCGCCGGCTCCGGCGCTTCTAACTTTACTCAGTTCGTTCTCGTCGA
CAATGGCGGAACTGGCGACGTGACTGTCGCCCCAAGCAACTTCGCTAACGGGATCGCT
GAATGGATCAGCTCTAACTCGCGTTCACAGGCTTACAAAGTAACCTGTAGCGTTCGTCA
GAGCTCTGCGCAGAATCGCAAATACACCATCAAAGTCGAGGTGCCTAAAGGCGCCTGG
CGTTCGTACTTAAATATGGAACTAACCATTCCAATTTTCGCCACGAATTCCGACTGCGAG
CTTATTGTTAAGGCAATGCAAGGTCTCCTAAAAGATGGAAACCCGATTCCCTCAGCAATC
GCAGCAAACTCCGGCATCTACGGTACCGGCGCCCCCGGCTCCGCCGGCTCCGCCGCC
GGCTCCGGCGCGTGCCCGGTGCCGCTGCAGCTGCCGCCGCTGGAACGCCTGACCCTG
GATGATAAAAAACCGCTGAATACCCTGATCTCTGCTACTGGTCTGTGGATGAGTCGTAC
CGGAACCATTCATAAAATCAAACACCACGAGGTTAGCCGTTCGAAAATCTATATTGAGAT
GGCGTGTGGCGATCATCTGGTTGTGAACAATAGCCGCTCTTCTCGTACAGCACGTGCA
CTGCGTCACCACAAATATCGTAAAACCTGTAAACGTTGCCGTGTGTCCGATGAGGATCT
GAACAAATTCCTGACAAAAGCCAATGAGGACCAAACAAGCGTGAAAGTGAAAGTCGTTA
GCGCTCCTACCCGTACTAAAAAAGCAATGCCGAAATCCGTTGCTCGTGCCCCTAAACCA
CTGGAAAACACTGAAGCAGCACAGGCACAGCCGTCTGGAAGCAAATTCTCTCCGGCCA
TTCCTGTTTCTACCCAGGAGTCCGTTTCTGTTCCAGCAAGTGTGAGCACCAGCATTAGC
AGTATTAGCACCGGTGCCACCGCTAGCGCCCTGGTTAAAGGCAATACCAATCCGATTAC
AAGCATGTCTGCCCCGGTTCAAGCATCAGCTCCAGCACTGACAAAATCCCAAACCGATC
GTCTGGAGGTTCTGCTGAATCCGAAAGACGAAATCAGCCTGAATTCCGGCAAACCGTTT
CGTGAACTGGAGAGCGAACTGCTGTCACGTCGTAAAAAAGACCTGCAACAAATCTATGC
CGAAGAACGTGAGAACTATCTGGGGAAACTGGAACGTGAAATCACCCGCTTTTTCGTGG
ATCGTGGCTTTCTGGAGATCAAATCCCCGATTCTGATTCCTCTGGAGTATATCGAGCGT
ATGGGCATCGACAATGATACCGAACTGAGCAAACAAATTTTCCGTGTGGATAAAAACTTC
TGTCTGCGCCCTATGCTAGCACCAAATCTGGCTAACTATCTGCGCAAACTGGACCGTGC
CCTGCCTGATCCTATCAAAATCTTCGAGATCGGCCCGTGTTATCGTAAAGAGTCCGACG
GTAAAGAACATCTGGAGGAGTTTACCATGCTGAACTTTTGCCAAATGGGTTCAGGTTGT
ACTCGTGAGAACCTGGAAAGCATCATCACCGATTTTCTGAACCACCTGGGCATTGACTT
CAAAATTGTGGGCGACAGCTGTATGGTGTTTGGCGACACCCTGGATGTCATGCACGGC
GACCTGGAACTGTCTAGTGCCGTTGTGGGCCCAATCCCGCTGGATCGTGAGTGGGGTA
TCGACAAACCTTGGATCGGTGCGGGTTTTGGTCTGGAGCGTCTGCTGAAAGTAAAACAC
GACTTCAAGAACATCAAACGTGCTGCACGTTCCGAGTCCTATTACAATGGTATTTCTACT
AACCTGTAA (SEQ ID NO:61)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTYTDIEMNRLGKGAPGSAGSAAGSGMASNDYTQQAT
QSYGAYPTQPGQGYSQQSSQPYGQQSYSGYSQSTDTSGYGQSSYSSYGQSQNTGYGTQ
STPQGYGSTGGYGSSQSSQSSYGQQSSYPGYGQQPAPSSTSGSYGSSSQSSSYGQPQS
GSYSQQPSYGGQQQSYGQQQSYNPPQGYGQQNQYNSSSGGGGGGGGGNYGQDQSS
MSSGGGSGGGYGNQDQSGGGGSGGYGQQDRGGRGRGGSGGGGGGGGGYNRSSGG
YEPRGRGGGRGGRGGMGGSDRGGFNKFGGPRDQGSRHDSEQDNSDNNTIFVQGLGEN
VTIESVADYFKQIGIIKTNKKTGQPMINLYTDRETGKLKGEATVSFDDPPSAKAAIDWFDGKE
FSGNPIKVSFATRRADFNRGGGNGRGGRGRGGPMGRGGYGGGGSGGGGRGGFPSGGG
GGGGQQRAGDWKCPNPTCENMNFSWRNECNQCKAPKPDGPGGGPGGSHMGGNYGDD
RRGGRGGDYKDDDDKGTGAPGSAGSAAGSGASNFTQFVLVDNGGTGDVTVAPSNFANGI
AEWISSNSRSQAYKVTCSVRQSSAQNRKYTIKVEVPKGAWRSYLNMELTIPIFATNSDCELIV
KAMQGLLKDGNPIPSAIAANSGIYGTGAPGSAGSAAGSGACPVPLQLPPLERLTLDDKKPLN
TLISATGLWMSRTGTIHKIKHHEVSRSKIYIEMACGDHLVVNNSRSSRTARALRHHKYRKTCK
RCRVSDEDLNKFLTKANEDQTSVKVKVVSAPTRTKKAMPKSVARAPKPLENTEAAQAQPS
GSKFSPAIPVSTQESVSVPASVSTSISSISTGATASALVKGNTNPITSMSAPVQASAPALTKS
QTDRLEVLLNPKDEISLNSGKPFRELESELLSRRKKDLQQIYAEERENYLGKLEREITRFFVD
RGFLEIKSPILIPLEYIERMGIDNDTELSKQIFRVDKNFCLRPMLAPNLANYLRKLDRALPDPIKI
FEIGPCYRKESDGKEHLEEFTMLNFCQMGSGCTRENLESIITDFLNHLGIDFKIVGDSCMVFG
DTLDVMHGDLELSSAVVGPIPLDREWGIDKPWIGAGFGLERLLKVKHDFKNIKRAARSESYY
NGISTNL (SEQ ID NO:62)

KIF16B-PyIRS^{AF}
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
 
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGAATCAGATTGCCCTCT
TAGACTCCCCCACATATACAGATATTGAAATGAACAGATTGGGAAAGGGCGCCCCCGGC
TCCGCCGGCTCCGCCGGCTCCGCCGCCGGCTCCGGCATGGCGTGCCCGGTGCCGCT
GCAGCTGCCGCCGCTGGAACGCCTGACCCTGGATGATAAAAAACCGCTGAATACCCTG
ATCTCTGCTACTGGTCTGTGGATGAGTCGTACCGGAACCATTCATAAAATCAAACACCA
CGAGGTTAGCCGTTCGAAAATCTATATTGAGATGGCGTGTGGCGATCATCTGGTTGTGA
ACAATAGCCGCTCTTCTCGTACAGCACGTGCACTGCGTCACCACAAATATCGTAAAACC
TGTAAACGTTGCCGTGTGTCCGATGAGGATCTGAACAAATTCCTGACAAAAGCCAATGA
GGACCAAACAAGCGTGAAAGTGAAAGTCGTTAGCGCTCCTACCCGTACTAAAAAAGCAA
TGCCGAAATCCGTTGCTCGTGCCCCTAAACCACTGGAAAACACTGAAGCAGCACAGGC
ACAGCCGTCTGGAAGCAAATTCTCTCCGGCCATTCCTGTTTCTACCCAGGAGTCCGTTT
CTGTTCCAGCAAGTGTGAGCACCAGCATTAGCAGTATTAGCACCGGTGCCACCGCTAG
CGCCCTGGTTAAAGGCAATACCAATCCGATTACAAGCATGTCTGCCCCGGTTCAAGCAT
CAGCTCCAGCACTGACAAAATCCCAAACCGATCGTCTGGAGGTTCTGCTGAATCCGAAA
GACGAAATCAGCCTGAATTCCGGCAAACCGTTTCGTGAACTGGAGAGCGAACTGCTGT
CACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATC
CCCGATTCTGATTCCTCTGGAGTATATCGAGCGTATGGGCATCGACAATGATACCGAAC
TGAGCAAACAAATTTTCCGTGTGGATAAAAACTTCTGTCTGCGCCCTATGCTAGCACCAA
ATCTGGCTAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTAC
CATGCTGAACTTTTGCCAAATGGGTTCAGGTTGTACTCGTGAGAACCTGGAAAGCATCA
TCACCGATTTTCTGAACCACCTGGGCATTGACTTCAAAATTGTGGGCGACAGCTGTATG
 
GTGTTTGGCGACACCCTGGATGTCATGCACGGCGACCTGGAACTGTCTAGTGCCGTTG
TGGGCCCAATCCCGCTGGATCGTGAGTGGGGTATCGACAAACCTTGGATCGGTGCGGG
TTTTGGTCTGGAGCGTCTGCTGAAAGTAAAACACGACTTCAAGAACATCAAACGTGCTG
CACGTTCCGAGTCCTATTACAATGGTATTTCTACTAACCTGTAA (SEQ ID NO:63)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTYTDIEMNRLGKGAPGSAGSAGSAAGSGMACPVPLQL
PPLERLTLDDKKPLNTLISATGLWMSRTGTIHKIKHHEVSRSKIYIEMACGDHLVVNNSRSSR
TARALRHHKYRKTCKRCRVSDEDLNKFLTKANEDQTSVKVKVVSAPTRTKKAMPKSVARAP
KPLENTEAAQAQPSGSKFSPAIPVSTQESVSVPASVSTSISSISTGATASALVKGNTNPITSM
SAPVQASAPALTKSQTDRLEVLLNPKDEISLNSGKPFRELESELLSRRKKDLQQIYAEEREN
YLGKLEREITRFFVDRGFLEIKSPILIPLEYIERMGIDNDTELSKQIFRVDKNFCLRPMLAPNLA
NYLRKLDRALPDPIKIFEIGPCYRKESDGKEHLEEFTMLNFCQMGSGCTRENLESIITDFLNH
LGIDFKIVGDSCMVFGDTLDVMHGDLELSSAVVGPIPLDREWGIDKPWIGAGFGLERLLKVK
HDFKNIKRAARSESYYNGISTNL (SEQ ID NO:64)

KIF16B-MCP
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
 
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGGAATCAGATTGCCCTCT
TAGACTCCCCCACATATACAGATATTGAAATGAACAGATTGGGAAAGGCGCCCCCGGC
TCCGCCGGCTCCGCCGCCGGCTCCGGCATGGCTTCTAACTTTACTCAGTTCGTTCTCGT
CGACAATGGCGGAACTGGCGACGTGACTGTCGCCCCAAGCAACTTCGCTAACGGGATC
GCTGAATGGATCAGCTCTAACTCGCGTTCACAGGCTTACAAAGTAACCTGTAGCGTTCG
TCAGAGCTCTGCGCAGAATCGCAAATACACCATCAAAGTCGAGGTGCCTAAAGGCGCC
TGGCGTTCGTACTTAAATATGGAACTAACCATTCCAATTTTCGCCACGAATTCCGACTGC
GAGCTTATTGTTAAGGCAATGCAAGGTCTCCTAAAAGATGGAAACCCGATTCCCTCAGC
AATCGCAGCAAACTCCGGCATCTACTAA (SEQ ID NO:65)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTYTDIEMNRLGKGAPGSAGSAAGSGMASNFTQFVLVD
NGGTGDVTVAPSNFANGIAEWISSNSRSQAYKVTCSVRQSSAQNRKYTIKVEVPKGAWRS
YLNMELTIPIFATNSDCELIVKAMQGLLKDGNPIPSAIAANSGIY (SEQ ID NO:66)

KIF16B-MCP-PylRS^{AF}
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
 
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGAATCAGATTGCCCTCT
TAGACTCCCCCACATATACAGATATTGAAATGAACAGATTGGGAAAGGCGCCCCCGGC
TCCGCCGGCTCCGCCGCCGGCTCCGGCATGGCTTCTAACTTTACTCAGTTCGTTCTCGT
CGACAATGGCGGAACTGGCGACGTGACTGTCGCCCCAAGCAACTTCGCTAACGGGATC
GCTGAATGGATCAGCTCTAACTCGCGTTCACAGGCTTACAAAGTAACCTGTAGCGTTCG
TCAGAGCTCTGCGCAGAATCGCAAATACACCATCAAAGTCGAGGTGCCTAAAGGCGCC
TGGCGTTCGTACTTAAATATGGAACTAACCATTCCAATTTTCGCCACGAATTCCGACTGC
GAGCTTATTGTTAAGGCAATGCAAGGTCTCCTAAAAGATGGAAACCCGATTCCCTCAGC
AATCGCAGCAAACTCCGGCATCTACGGTACCGGCGCCCCCGGCTCCGCCGGCTCCGC
CGCCGGCTCCGGCGCGTGCCCGGTGCCGCTGCAGCTGCCGCCGCTGGAACGCCTGA
CCCTGGATGATAAAAAACCGCTGAATACCCTGATCTCTGCTACTGGTCTGTGGATGAGT
CGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCAC
GTGCACTGCGTCACCACAAATATCGTAAAACCTGTAAACGTTGCCGTGTGTCCGATGAG
GATCTGAACAAATTCCTGACAAAAGCCAATGAGGACCAAACAAGCGTGAAAGTGAAAGT
CGTTAGCGCTCCTACCCGTACTAAAAAAGCAATGCCGAAATCCGTTGCTCGTGCCCCTA
AACCACTGGAAAACACTGAAGCAGCACAGGCACAGCCGTCTGGAAGCAAATTCTCTCC
GGCCATTCCTGTTTCTACCCAGGAGTCCGTTTCTGTTCCAGCAAGTGTGAGCACCAGCA
TTAGCAGTATTAGCACCGGTGCCACCGCTAGCGCCCTGGTTAAAGGCAATACCAATCCG
ATTACAAGCATGTCTGCCCCGGTTCAAGCATCAGCTCCAGCACTGACAAAATCCCAAAC
CGATCGTCTGGAGGTTCTGCTGAATCCGAAAGACGAAATCAGCCTGAATTCCGGCAAAC
CGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTT
CGTGGATCGTGGCTTTCTGGAGATCAAATCCCCGATTCTGATTCCTCTGGAGTATATCG
AGCGTATGGGCATCGACAATGATACCGAACTGAGCAAACAAATTTTCCGTGTGGATAAA
 
AACTTCTGTCTGCGCCCTATGCTAGCACCAAATCTGGCTAACTATCTGCGCAAACTGGA
CCGTGCCCTGCCTGATCCTATCAAAATCTTCGAGATCGGCCCGTGTTATCGTAAAGAGT
CCGACGGTAAAGAACATCTGGAGGAGTTTACCATGCTGAACTTTTGCCAAATGGGTTCA
GGTTGTACTCGTGAGAACCTGGAAAGCATCATCACCGATTTTCTGAACCACCTGGGCAT
TGACTTCAAAATTGTGGGCGACAGCTGTATGGTGTTTGGCGACACCCTGGATGTCATGC
ACGGCGACCTGGAACTGTCTAGTGCCGTTGTGGGCCCAATCCCGCTGGATCGTGAGTG
GGGTATCGACAAACCTTGGATCGGTGCGGGTTTTGGTCTGGAGCGTCTGCTGAAAGTA
AAACACGACTTCAAGAACATCAAACGTGCTGCACGTTCCGAGTCCTATTACAATGGTATT
TCTACTAACCTGTAA (SEQ ID NO:67)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTYTDIEMNRLGKGAPGSAGSAAGSGMASNFTQFVLVD
NGGTGDVTVAPSNFANGIAEWISSNSRSQAYKVTCSVRQSSAQNRKYTIKVEVPKGAWRS
YLNMELTIPIFATNSDCELIVKAMQGLLKDGNPIPSAIAANSGIYGTGAPGSAGSAAGSGACP
VPLQLPPLERLTLDDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKS
VARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAE
ERENYLGKLEREITRFFVDRGFLEIKSPILIPLEYIERMGIDNDTELSKQIFRVDKNFCLRPMLA
PNLANYLRKLDRALPDPIKIFEIGPCYRKESDGKEHLEEFTMLNFCQMGSGCTRENLESIITD
FLNHLGIDFKIVGDSCMVFGDTLDVMHGDLELSSAVVGPIPLDREWGIDKPWIGAGFGLERL
LKVKHDFKNIKRAARSESYYNGISTNL (SEQ ID NO:68)

KIF16B-SPD5-PyIRS^{AF}
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
 
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTcGTGACTCTGGGGAACGTCATTTCTGCCTTAGCTGATTTATCTCAGGATGCT
GCAAATACTCTTGCAAAGAAGAAGCAAGTTTTCGTGCCTTACAGGGATTCTGTGTTGACT
TGGTTGTTAAAAGATAGCCTTGGAGGAAACTCTAAAACTATCATGATTGCCACCATTTCA
CCTGCTGATGTCAATTATGGAGAAACCCTAAGTACTCTTCGCTATGCAAATAGAGCCAAA
AACATCATCAACAAGCCTACCATTAATGAGGATGCCAACGTCAAACTTATCCGTGAGCT
GCGAGCTGAAATAGCCAGACTGAAAACGCTGCTTGCTCAAGGAATCAGATTGCCCTCT
TAGACTCCCCCACAATGGAGGACAACAGCGTGCTGAACGAGGACAGCAACCTGGAGCA
CGTGGAGGGCCAGCCCAGAAGAAGCATGAGCCAGCCCGTGCTGAACGTGGAGGGCGA
CAAGAGAACCAGCAGCACCAGCGCCACCCAGCAGCAGGTGCTGAGCGGCGCCTTCAG
CAGCGCCGACGTGAGAAGCATCCCCATCATCCAGACCTGGGAGGAGAACAAGGCCCT
GAAGACCAAGATCACCATCCTGAGAGGCGAGCTGCAGATGTACCAGAGAAGATACAGC
GAGGCCAAGGAGGCCAGCCAGAAGAGAGTGAAGGAGGTGATGGACGACTACGTGGAC
CTGAAGCTGGGCCAGGAGAACGTGCAGGAGAAGATGGAGCAGTACAAGCTGATGGAG
GAGGACCTGCTGGCCATGCAGAGCAGAATCGAGACCAGCGAGGACAACTTCGCCAGA
CAGATGAAGGAGTTCGAGGCCCAGAAGCACGCCATGGAGGAGAGAATCAAGGAGCTG
GAGCTGAGCGCCACCGACGCCAACAACACCACCGTGGGCAGCTTCAGAGGCACCCTG
GACGACATCCTGAAGAAGAACGACCCCGACTTCACCCTGACCAGCGGCTACGAGGAGA
GAAAGATCAACGACCTGGAGGCCAAGCTGCTGAGCGAGATCGACAAGGTGGCCGAGC
TGGAGGACCACATCCAGCAGCTGAGACAGGAGCTGGACGACCAGAGCGCCAGACTGG
CCGACAGCGAGAACGTGAGAGCCCAGCTGGAGGCCGCCACCGGCCAGGGCATCCTGG
GCGCCGCCGGCAACGCCATGGTGCCCAACAGCACCTTCATGATCGGCAACGGCAGAG
AGAGCCAGACCAGAGACCAGCTGAACTACATCGACGACCTGGAGACCAAGCTGGCCGA
CGCCAAGAAGGAGAACGACAAGGCCAGACAGGCCCTGGTGGAGTACATGAACAAGTG
CAGCAAGCTGGAGCACGAGATCAGAACCATGGTGAAGAACAGCACCTTCGACAGCAGC
AGCATGCTGCTGGGCGGCCAGACCAGCGACGAGCTGAAGATCCAGATCGGCAAGGTG
AACGGCGAGCTGAACGTGCTGAGAGCCGAGAACAGAGAGCTGAGAATCAGATGCGAC
CAGCTGACCGGCGGCGACGGCAACCTGAGCATCAGCCTGGGCCAGAGCAGACTGATG
GCCGGCATCGCCACCAACGACGTGGACAGCATCGGCCAGGGCAACGAGACCGGCGGC
 
ACCAGCATGAGAATCCTGCCCAGAGAGAGCCAGCTGGACGACCTGGAGGAGAGCAAG
CTGCCCCTGATGGACACCAGCAGCGCCGTGAGAAACCAGCAGCAGTTCGCCAGCATGT
GGGAGGACTTCGAGAGCGTGAAGGACAGCCTGCAGAACAACCACAACGACACCCTGG
AGGGCAGCTTCAACAGCAGCATGCCCCCCCCGGCAGAGACGCCACCCAGAGCTTCC
TGAGCCAGAAGAGCTTCAAGAACAGCCCCATCGTGATGCAGAAGCCCAAGAGCCTGCA
CCTGCACCTGAAGAGCCACCAGAGCGAGGGCGCCGGCGAGCAGATCCAGAACAACAG
CTTCAGCACCAAGACCGCCAGCCCCCACGTGAGCCAGAGCCACATCCCCATCCTGCAC
GACATGCAGCAGATCCTGGACAGCAGCGCCATGTTCCTGGAGGGCCAGCACGACGTG
GCCGTGAACGTGGAGCAGATGCAGGAGAAGATGAGCCAGATCAGAGAGGCCCTGGCC
AGACTGTTCGAGAGACTGAAGAGCAGCGCCGCCCTGTTCGAGGAGATCCTGGAGAGAA
TGGGCAGCAGCGACCCCAACGCCGACAAGATCAAGAAGATGAAGCTGGCCTTCGAGAC
CAGCATCAACGACAAGCTGAACGTGAGCGCCATCCTGGAGGCCGCCGAGAAGGACCT
GCACAACATGAGCCTGAACTTCAGCATCCTGGAGAAGAGCATCGTGAGCCAGGCCGCC
GAGGCCAGCAGAAGATTCACCATCGCCCCCGACGCCGAGGACGTGGCCAGCAGCAGC
CTGCTGAACGCCAGCTACAGCCCCCTGTTCAAGTTCACCAGCAACAGCGACATCGTGG
AGAAGCTGCAGAACGAGGTGAGCGAGCTGAAGAACGAGCTGGAGATGGCCAGAACCA
GAGACATGAGAAGCCCCCTGAACGGCAGCAGCGGCAGACTGAGCGACGTGCAGATCA
ACACCAACAGAATGTTCGAGGACCTGGAGGTGAGCGAGGCCACCCTGCAGAAGGCCAA
GGAGGAGAACAGCACCCTGAAGAGCCAGTTCGCCGAGCTGGAGGCCAACCTGCACCA
GGTGAACAGCAAGCTGGGCGAGGTGAGATGCGAGCTGAACGAGGCCCTGGCCAGAGT
GGACGGCGAGCAGGAGACCAGAGTGAAGGCCGAGAACGCCCTGGAGGAGGCCAGAC
AGCTGATCAGCAGCCTGAAGCACGAGGAGAACGAGCTGAAGAAGACCATCACCGACAT
GGGCATGAGACTGAACGAGGCCAAGAAGAGCGACGAGTTCCTGAAGAGCGAGCTGAG
CACCGCCCTGGAGGAGGAGAAGAAGAGCCAGAACCTGGCCGACGAGCTGAGCGAGGA
GCTGAACGGCTGGAGAATGAGAACCAAGGAGGCCGAGAACAAGGTGGAGCACGCCAG
CAGCGAGAAGAGCGAGATGCTGGAGAGAATCGTGCACCTGGAGACCGAGATGGAGAA
GCTGAGCACCAGCGAGATCGCCGCCGACTACTGCAGCACCAAGATGACCGAGAGAAA
GAAGGAGATCGAGCTGGCCAAGTACAGAGAGGACTTCGAGAACGCCGCCATCGTGGG
CCTGGAGAGAATCAGCAAGGAGATCAGCGAGCTGACCAAGAAGACCCTGAAGGCCAAG
ATCATCCCCAGCAACATCAGCAGCATCCAGCTGGTGTGCGACGAGCTGTGCAGAAGAC
TGAGCAGAGAGAGAGAGCAGCAGCACGAGTACGCCAAGGTGATGAGAGACGTGAACG
AGAAGATCGAGAAGCTGCAGCTGGAGAAGGACGCCCTGGAGCACGAGCTGAAGATGAT
GAGCAGCAACAACGAGAACGTGCCCCCCGTGGGCACCAGCGTGAGCGGCATGCCCAC
CAAGACCAGCAACCAGAAGTGCGCCCAGCCCCACTACACCAGCCCCACCAGACAGCTG
CTGCACGAGAGCACCATGGCCGTGGACGCCATCGTGCAGAAGCTGAAGAAGACCCACA
ACATGAGCGGCATGGGCCCCGAGCTGAAGGAGACCATCGGCAACGTGATCAACGAGA
 
GCAGAGTGCTGAGAGACTTCCTGCACCAGAAGCTGATCCTGTTCAAGGGCATCGACAT
GAGCAACTGGAAGAACGAGACCGTGGACCAGCTGATCACCGACCTGGGCCAGCTGCA
CCAGGACAACCTGATGCTGGAGGAGCAGATCAAGAAGTACAAGGAGCTGAAGCTG
ACCAAGAGCGCCATCCCCACCCTGGGCGTGGAGTTCCAGGACAGAATCAAGACCGAGA
TCGGCAAGATCGCCACCGACATGGGCGGCGCCGTGAAGGAGATCAGAAAGAAGGGTA
CCGGCGCCCCCGGCTCCGCCGGCTCCGCCGCCGGCTCCGGCGCGTGCCCGGTGCCG
CTGCAGCTGCCGCCGCTGGAACGCCTGACCCTGGATGATAAAAACCGCTGAATACCC
TGATCTCTGCTACTGGTCTGTGGATGAGTCGTACCGGAACCATTCATAAAATCAAACAC
CACGAGGTTAGCCGTTCGAAAATCTATATTGAGATGGCGTGTGGCGATCATCTGGTTGT
GAACAATAGCCGCTCTTCTCGTACAGCACGTGCACTGCGTCACCACAAATATCGTAAAA
CCTGTAAACGTTGCCGTGTGTCCGATGAGGATCTGAACAAATTCCTGACAAAAGCCAAT
GAGGACCAAACAAGCGTGAAAGTGAAAGTCGTTAGCGCTCCTACCCGTACTAAAAAAGC
AATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGT
TTCTGTTCCAGCAAGTGTGAGCACCAGCATTAGCAGTATTAGCACCGGTGCCACCGCTA
GCGCCCTGGTTAAAGGCAATACCAATCCGATTACAAGCATGTCTGCCCCGGTTCAAGCA
TCAGCTCCAGCACTGACAAAATCCCAAACCGATCGTCTGGAGGTTCTGCTGAATCCGAA
AGACGAAATCAGCCTGAATTCCGGCAAACCGTTTCGTGAACTGGAGAGCGAACTGCTGT
CACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATC
CCCGATTCTGATTCCTCTGGAGTATATCGAGCGTATGGGCATCGACAATGATACCGAAC
TGAGCAAACAAATTTTCCGTGTGGATAAAAACTTCTGTCTGCGCCCTATGCTAGCACCAA
ATCTGGCTAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTAC
CATGCTGAACTTTTGCCAAATGGGTTCAGGTTGTACTCGTGAGAACCTGGAAAGCATCA
TCACCGATTTTCTGAACCACCTGGGCATTGACTTCAAAATTGTGGGCGACAGCTGTATG
GTGTTTGGCGACACCCTGGATGTCATGCACGGCGACCTGGAACTGTCTAGTGCCGTTG
TGGGCCCAATCCCGCTGGATCGTGAGTGGGGTATCGACAAACCTTGGATCGGTGCGGG
TTTTGGTCTGGAGCGTCTGCTGAAAGTAAAACACGACTTCAAGAACATCAAACGTGCTG
CACGTTCCGAGTCCTATTACAATGGTATTTCTACTAACCTGTAA (SEQ ID NO:69)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP

YRDSVLTWLLKDSLGGNSKTIMIATISPADVNYGETLSTLRYANRAKNIINKPTINEDANVKLIR
ELRAEIARLKTLLAQGNQIALLDSPTMEDNSVLNEDSNLEHVEGQPRRSMSQPVLNVEGDK
RTSSTSATQQQVLSGAFSSADVRSIPIIQTWEENKALKTKITILRGELQMYQRRYSEAKEASQ
KRVKEVMDDYVDLKLGQENVQEKMEQYKLMEEDLLAMQSRIETSEDNFARQMKEFEAQKH
AMEERIKELELSATDANNTTVGSFRGTLDDILKKNDPDFTLTSGYEERKINDLEAKLLSEIDKV
AELEDHIQQLRQELDDQSARLADSENVRAQLEAATGQGILGAAGNAMVPNSTFMIGNGRES
QTRDQLNYIDDLETKLADAKKENDKARQALVEYMNKCSKLEHEIRTMVKNSTFDSSSMLLG
GQTSDELKIQIGKVNGELNVLRAENRELRIRCDQLTGGDGNLSISLGQSRLMAGIATNDVDSI
GQGNETGGTSMRILPRESQLDDLEESKLPLMDTSSAVRNQQQFASMWEDFESVKDSLQNN
HNDTLEGSFNSSMPPPGRDATQSFLSQKSFKNSPIVMQKPKSLHLHLKSHQSEGAGEQIQN
NSFSTKTASPHVSQSHIPILHDMQQILDSSAMFLEGQHDVAVNVEQMQEKMSQIREALARLF
ERLKSSAALFEEILERMGSSDPNADKIKKMKLAFETSINDKLNVSAILEAAEKDLHNMSLNFSI
LEKSIVSQAAEASRRFTIAPDAEDVASSSLLNASYSPLFKFTSNSDIVEKLQNEVSELKNELE
MARTRDMRSPLNGSSGRLSDVQINTNRMFEDLEVSEATLQKAKEENSTLKSQFAELEANLH
QVNSKLGEVRCELNEALARVDGEQETRVKAENALEEARQLISSLKHEENELKKTITDMGMR
LNEAKKSDEFLKSELSTALEEEKKSQNLADELSEELNGWRMRTKEAENKVEHASSEKSEML
ERIVHLETEMEKLSTSEIAADYCSTKMTERKKEIELAKYREDFENAAIVGLERISKEISELTKKT
LKAKIIPSNISSIQLVCDELCRRLSREREQQHEYAKVMRDVNEKIEKLQLEKDALEHELKMMS
SNNENVPPVGTSVSGMPTKTSNQKCAQPHYTSPTRQLLHESTMAVDAIVQKLKKTHNMSG
MGPELKETIGNVINESRVLRDFLHQKLILFKGIDMSNWKNETVDQLITDLGQLHQDNLMLEEQ
IKKYKKELKLTKSAIPTLGVEFQDRIKTEIGKIATDMGGAVKEIRKKGTGAPGSAGSAAGSGA
CPVPLQLPPLERLTLDDKKPLNTLISATGLWMSRTGTIHKIKHHEVSRSKIYIEMACGDHLVV
NNSRSSRTARALRHHKYRKTCKRCRVSDEDLNKFLTKANEDQTSVKVKVVSAPTRTKKAM
PKSVARAPKPLENTEAAQAQPSGSKFSPAIPVSTQESVSVPASVSTSISSISTGATASALVKG
NTNPITSMSAPVQASAPALTKSQTDRLEVLLNPKDEISLNSGKPFRELESELLSRRKKDLQQI
YAEERENYLGKLEREITRFFVDRGFLEIKSPILIPLEYIERMGIDNDTELSKQIFRVDKNFCLRP
MLAPNLANYLRKLDRALPDPIKIFEIGPCYRKESDGKEHLEEFTMLNFCQMGSGCTRENLESI
ITDFLNHLGIDFKIVGDSCMVFGDTLDVMHGDLELSSAVVGPIPLDREWGIDKPWIGAGFGLE
RLLKVKHDFKNIKRAARSESYYNGISTNL (SEQ ID NO:70)

KIF16B-SPD5-MCP
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
 
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGGAATCAGATTGCCCTCT
TAGACTCCCCCACAATGGAGGACAACAGCGTGCTGAACGAGGACAGCAACCTGGAGCA
CGTGGAGGGCCAGCCCAGAAGAAGCATGAGCCAGCCCGTGCTGAACGTGGAGGGCGA
CAAGAGAACCAGCAGCACCAGCGCCACCCAGCAGCAGGTGCTGAGCGGCGCCTTCAG
CAGCGCCGACGTGAGAAGCATCCCCATCATCCAGACCTGGGAGGAGAACAAGGCCCT
GAAGACCAAGATCACCATCCTGAGAGGCGAGCTGCAGATGTACCAGAGAAGATACAGC
GAGGCCAAGGAGGCCAGCCAGAAGAGAGTGAAGGAGGTGATGGACGACTACGTGGAC
CTGAAGCTGGGCCAGGAGAACGTGCAGGAGAAGATGGAGCAGTACAAGCTGATGGAG
GAGGACCTGCTGGCCATGCAGAGCAGAATCGAGACCAGCGAGGACAACTTCGCCAGA
CAGATGAAGGAGTTCGAGGCCCAGAAGCACGCCATGGAGGAGAATCAAGGAGCTG
GAGCTGAGCGCCACCGACGCCAACAACACCACCGTGGGCAGCTTCAGAGGCACCCTG
GACGACATCCTGAAGAAGAACGACCCCGACTTCACCCTGACCAGCGGCTACGAGGAGA
GAAAGATCAACGACCTGGAGGCCAAGCTGCTGAGCGAGATCGACAAGGTGGCCGAGC
TGGAGGACCACATCCAGCAGCTGAGACAGGAGCTGGACGACCAGAGCGCCAGACTGG
CCGACAGCGAGAACGTGAGAGCCCAGCTGGAGGCCGCCACCGGCCAGGGCATCCTGG
GCGCCGCCGGCAACGCCATGGTGCCCAACAGCACCTTCATGATCGGCAACGGCAGAG
AGAGCCAGACCAGAGACCAGCTGAACTACATCGACGACCTGGAGACCAAGCTGGCCGA
CGCCAAGAAGGAGAACGACAAGGCCAGACAGGCCCTGGTGGAGTACATGAACAAGTG
CAGCAAGCTGGAGCACGAGATCAGAACCATGGTGAAGAACAGCACCTTCGACAGCAGC
AGCATGCTGCTGGGCGGCCAGACCAGCGACGAGCTGAAGATCCAGATCGGCAAGGTG
AACGGCGAGCTGAACGTGCTGAGAGCCGAGAACAGAGAGCTGAGAATCAGATGCGAC
 
CAGCTGACCGGCGGCGACGGCAACCTGAGCATCAGCCTGGGCCAGAGCAGACTGATG
GCCGGCATCGCCACCAACGACGTGGACAGCATCGGCCAGGGCAACGAGACCGGCGGC
ACCAGCATGAGAATCCTGCCCAGAGAGAGCCAGCTGGACGACCTGGAGGAGAGCAAG
CTGCCCCTGATGGACACCAGCAGCGCCGTGAGAAACCAGCAGCAGTTCGCCAGCATGT
GGGAGGACTTCGAGAGCGTGAAGGACAGCCTGCAGAACAACCACAACGACACCCTGG
AGGGCAGCTTCAACAGCAGCATGCCCCCCCCGGCAGAGACGCCACCCAGAGCTTCC
TGAGCCAGAAGAGCTTCAAGAACAGCCCCATCGTGATGCAGAAGCCCAAGAGCCTGCA
CCTGCACCTGAAGAGCCACCAGAGCGAGGGCGCCGGCGAGCAGATCCAGAACAACAG
CTTCAGCACCAAGACCGCCAGCCCCCACGTGAGCCAGAGCCACATCCCCATCCTGCAC
GACATGCAGCAGATCCTGGACAGCAGCGCCATGTTCCTGGAGGGCCAGCACGACGTG
GCCGTGAACGTGGAGCAGATGCAGGAGAAGATGAGCCAGATCAGAGAGGCCCTGGCC
AGACTGTTCGAGAGACTGAAGAGCAGCGCCGCCCTGTTCGAGGAGATCCTGGAGAGAA
TGGGCAGCAGCGACCCCAACGCCGACAAGATCAAGAAGATGAAGCTGGCCTTCGAGAC
CAGCATCAACGACAAGCTGAACGTGAGCGCCATCCTGGAGGCCGCCGAGAAGGACCT
GCACAACATGAGCCTGAACTTCAGCATCCTGGAGAAGAGCATCGTGAGCCAGGCCGCC
GAGGCCAGCAGAAGATTCACCATCGCCCCCGACGCCGAGGACGTGGCCAGCAGCAGC
CTGCTGAACGCCAGCTACAGCCCCCTGTTCAAGTTCACCAGCAACAGCGACATCGTGG
AGAAGCTGCAGAACGAGGTGAGCGAGCTGAAGAACGAGCTGGAGATGGCCAGAACCA
GAGACATGAGAAGCCCCCTGAACGGCAGCAGCGGCAGACTGAGCGACGTGCAGATCA
ACACCAACAGAATGTTCGAGGACCTGGAGGTGAGCGAGGCCACCCTGCAGAAGGCCAA
GGAGGAGAACAGCACCCTGAAGAGCCAGTTCGCCGAGCTGGAGGCCAACCTGCACCA
GGTGAACAGCAAGCTGGGCGAGGTGAGATGCGAGCTGAACGAGGCCCTGGCCAGAGT
GGACGGCGAGCAGGAGACCAGAGTGAAGGCCGAGAACGCCCTGGAGGAGGCCAGAC
AGCTGATCAGCAGCCTGAAGCACGAGGAGAACGAGCTGAAGAAGACCATCACCGACAT
GGGCATGAGACTGAACGAGGCCAAGAAGAGCGACGAGTTCCTGAAGAGCGAGCTGAG
CACCGCCCTGGAGGAGGAGAAGAAGAGCCAGAACCTGGCCGACGAGCTGAGCGAGGA
GCTGAACGGCTGGAGAATGAGAACCAAGGAGGCCGAGAACAAGGTGGAGCACGCCAG
CAGCGAGAAGAGCGAGATGCTGGAGAGAATCGTGCACCTGGAGACCGAGATGGAGAA
GCTGAGCACCAGCGAGATCGCCGCCGACTACTGCAGCACCAAGATGACCGAGAGAAA
GAAGGAGATCGAGCTGGCCAAGTACAGAGAGGACTTCGAGAACGCCGCCATCGTGGG
CCTGGAGAGAATCAGCAAGGAGATCAGCGAGCTGACCAAGAAGACCCTGAAGGCCAAG
ATCATCCCCAGCAACATCAGCAGCATCCAGCTGGTGTGCGACGAGCTGTGCAGAAGAC
TGAGCAGAGAGAGAGAGCAGCAGCACGAGTACGCCAAGGTGATGAGAGACGTGAACG
AGAAGATCGAGAAGCTGCAGCTGGAGAAGGACGCCCTGGAGCACGAGCTGAAGATGAT
GAGCAGCAACAACGAGAACGTGCCCCCCGTGGGCACCAGCGTGAGCGGCATGCCCAC
CAAGACCAGCAACCAGAAGTGCGCCCAGCCCCACTACACCAGCCCCACCAGACAGCTG
 
CTGCACGAGAGCACCATGGCCGTGGACGCCATCGTGCAGAAGCTGAAGAAGACCCACA
ACATGAGCGGCATGGGCCCCGAGCTGAAGGAGACCATCGGCAACGTGATCAACGAGA
GCAGAGTGCTGAGAGACTTCCTGCACCAGAAGCTGATCCTGTTCAAGGGCATCGACAT
GAGCAACTGGAAGAACGAGACCGTGGACCAGCTGATCACCGACCTGGGCCAGCTGCA
CCAGGACAACCTGATGCTGGAGGAGCAGATCAAGAAGTACAAGAAGGAGCTGAAGCTG
ACCAAGAGCGCCATCCCCACCCTGGGCGTGGAGTTCCAGGACAGAATCAAGACCGAGA
TCGGCAAGATCGCCACCGACATGGGCGGCGCCGTGAAGGAGATCAGAAAGAAGGGTA
CCGAGCAGAAGCTGATCTCAGAGGAGGACCTGGGCGCCCCCGGCTCCGCCGGCTCCG
CCGCCGGCTCCGGCGCTTCTAACTTTACTCAGTTCGTTCTCGTCGACAATGGCGGAACT
GGCGACGTGACTGTCGCCCCAAGCAACTTCGCTAACGGGATCGCTGAATGGATCAGCT
CTAACTCGCGTTCACAGGCTTACAAAGTAACCTGTAGCGTTCGTCAGAGCTCTGCGCAG
AATCGCAAATACACCATCAAAGTCGAGGTGCCTAAAGGCGCCTGGCGTTCGTACTTAAA
TATGGAACTAACCATTCCAATTTTCGCCACGAATTCCGACTGCGAGCTTATTGTTAAGGC
AATGCAAGGTCTCCTAAAAGATGGAAACCCGATTCCCTCAGCAATCGCAGCAAACTCCG
GCATCTACTAA (SEQ ID NO:71)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTMEDNSVLNEDSNLEHVEGQPRRSMSQPVLNVEGDK
RTSSTSATQQQVLSGAFSSADVRSIPIIQTWEENKALKTKITILRGELQMYQRRYSEAKEASQ
KRVKEVMDDYVDLKLGQENVQEKMEQYKLMEEDLLAMQSRIETSEDNFARQMKEFEAQKH
AMEERIKELELSATDANNTTVGSFRGTLDDILKKNDPDFTLTSGYEERKINDLEAKLLSEIDKV
AELEDHIQQLRQELDDQSARLADSENVRAQLEAATGQGILGAAGNAMVPNSTFMIGNGRES
QTRDQLNYIDDLETKLADAKKENDKARQALVEYMNKCSKLEHEIRTMVKNSTFDSSSMLLG
GQTSDELKIQIGKVNGELNVLRAENRELRIRCDQLTGGDGNLSISLGQSRLMAGIATNDVDSI
GQGNETGGTSMRILPRESQLDDLEESKLPLMDTSSAVRNQQQFASMWEDFESVKDSLQNN
HNDTLEGSFNSSMPPPGRDATQSFLSQKSFKNSPIVMQKPKSLHLHLKSHQSEGAGEQIQN
NSFSTKTASPHVSQSHIPILHDMQQILDSSAMFLEGQHDVAVNVEQMQEKMSQIREALARLF
ERLKSSAALFEEILERMGSSDPNADKIKKMKLAFETSINDKLNVSAILEAAEKDLHNMSLNFSI
LEKSIVSQAAEASRRFTIAPDAEDVASSSLLNASYSPLFKFTSNSDIVEKLQNEVSELKNELE
MARTRDMRSPLNGSSGRLSDVQINTNRMFEDLEVSEATLQKAKEENSTLKSQFAELEANLH
QVNSKLGEVRCELNEALARVDGEQETRVKAENALEEARQLISSLKHEENELKKTITDMGMR

LNEAKKSDEFLKSELSTALEEEKKSQNLADELSEELNGWRMRTKEAENKVEHASSEKSEML
ERIVHLETEMEKLSTSEIAADYCSTKMTERKKEIELAKYREDFENAAIVGLERISKEISELTKKT
LKAKIIPSNISSIQLVCDELCRRLSREREQQHEYAKVMRDVNEKIEKLQLEKDALEHELKMMS
SNNENVPPVGTSVSGMPTKTSNQKCAQPHYTSPTRQLLHESTMAVDAIVQKLKKTHNMSG
MGPELKETIGNVINESRVLRDFLHQKLILFKGIDMSNWKNETVDQLITDLGQLHQDNLMLEEQ
IKKYKKELKLTKSAIPTLGVEFQDRIKTEIGKIATDMGGAVKEIRKKGTEQKLISEEDLGAPGSA
GSAAGSGASNFTQFVLVDNGGTGDVTVAPSNFANGIAEWISSNSRSQAYKVTCSVRQSSA
QNRKYTIKVEVPKGAWRSYLNMELTIPIFATNSDCELIVKAMQGLLKDGNPIPSAIAANSGIY
(SEQ ID NO:72)

KIF16B-SPD5-MCP-PyIRS^{AF}
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
TCTGAAATGCCATGTGAAACCGTCAGTAAGATCCACTTGGTTGATCTTGCCGGAAGTGA
GCGTGCAGATGCCACCGGAGCCACCGGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGGAATCAGATTGCCCTCT
TAGACTCCCCCACAATGGAGGACAACAGCGTGCTGAACGAGGACAGCAACCTGGAGCA
CGTGGAGGGCCAGCCCAGAAGAAGCATGAGCCAGCCCGTGCTGAACGTGGAGGGCGA
CAAGAGAACCAGCAGCACCAGCGCCACCCAGCAGCAGGTGCTGAGCGGCGCCTTCAG
CAGCGCCGACGTGAGAAGCATCCCCATCATCCAGACCTGGGAGGAGAACAAGGCCCT
 
GAAGACCAAGATCACCATCCTGAGAGGCGAGCTGCAGATGTACCAGAGAAGATACAGC
GAGGCCAAGGAGGCCAGCCAGAAGAGAGTGAAGGAGGTGATGGACGACTACGTGGAC
CTGAAGCTGGGCCAGGAGAACGTGCAGGAGAAGATGGAGCAGTACAAGCTGATGGAG
GAGGACCTGCTGGCCATGCAGAGCAGAATCGAGACCAGCGAGGACAACTTCGCCAGA
CAGATGAAGGAGTTCGAGGCCCAGAAGCACGCCATGGAGGAGAGAATCAAGGAGCTG
GAGCTGAGCGCCACCGACGCCAACAACACCACCGTGGGCAGCTTCAGAGGCACCCTG
GACGACATCCTGAAGAAGAACGACCCCGACTTCACCCTGACCAGCGGCTACGAGGAGA
GAAAGATCAACGACCTGGAGGCCAAGCTGCTGAGCGAGATCGACAAGGTGGCCGAGC
TGGAGGACCACATCCAGCAGCTGAGACAGGAGCTGGACGACCAGAGCGCCAGACTGG
CCGACAGCGAGAACGTGAGAGCCCAGCTGGAGGCCGCCACCGGCCAGGGCATCCTGG
GCGCCGCCGGCAACGCCATGGTGCCCAACAGCACCTTCATGATCGGCAACGGCAGAG
AGAGCCAGACCAGAGACCAGCTGAACTACATCGACGACCTGGAGACCAAGCTGGCCGA
CGCCAAGAAGGAGAACGACAAGGCCAGACAGGCCCTGGTGGAGTACATGAACAAGTG
CAGCAAGCTGGAGCACGAGATCAGAACCATGGTGAAGAACAGCACCTTCGACAGCAGC
AGCATGCTGCTGGGCGGCCAGACCAGCGACGAGCTGAAGATCCAGATCGGCAAGGTG
AACGGCGAGCTGAACGTGCTGAGAGCCGAGAACAGAGAGCTGAGAATCAGATGCGAC
CAGCTGACCGGCGGCGACGGCAACCTGAGCATCAGCCTGGGCCAGAGCAGACTGATG
GCCGGCATCGCCACCAACGACGTGGACAGCATCGGCCAGGGCAACGAGACCGGCGGC
ACCAGCATGAGAATCCTGCCCAGAGAGAGCCAGCTGGACGACCTGGAGGAGAGCAAG
CTGCCCCTGATGGACACCAGCAGCGCCGTGAGAAACCAGCAGCAGTTCGCCAGCATGT
GGGAGGACTTCGAGAGCGTGAAGGACAGCCTGCAGAACAACCACAACGACACCCTGG
AGGGCAGCTTCAACAGCAGCATGCCCCCCCCGGCAGAGACGCCACCCAGAGCTTCC
TGAGCCAGAAGAGCTTCAAGAACAGCCCCATCGTGATGCAGAAGCCCAAGAGCCTGCA
CCTGCACCTGAAGAGCCACCAGAGCGAGGGCGCCGGCGAGCAGATCCAGAACAACAG
CTTCAGCACCAAGACCGCCAGCCCCCACGTGAGCCAGAGCCACATCCCCATCCTGCAC
GACATGCAGCAGATCCTGGACAGCAGCGCCATGTTCCTGGAGGGCCAGCACGACGTG
GCCGTGAACGTGGAGCAGATGCAGGAGAAGATGAGCCAGATCAGAGAGGCCCTGGCC
AGACTGTTCGAGAGACTGAAGAGCAGCGCCGCCCTGTTCGAGGAGATCCTGGAGAGAA
TGGGCAGCAGCGACCCCAACGCCGACAAGATCAAGAAGATGAAGCTGGCCTTCGAGAC
CAGCATCAACGACAAGCTGAACGTGAGCGCCATCCTGGAGGCCGCCGAGAAGGACCT
GCACAACATGAGCCTGAACTTCAGCATCCTGGAGAAGAGCATCGTGAGCCAGGCCGCC
GAGGCCAGCAGAAGATTCACCATCGCCCCCGACGCCGAGGACGTGGCCAGCAGCAGC
CTGCTGAACGCCAGCTACAGCCCCCTGTTCAAGTTCACCAGCAACAGCGACATCGTGG
AGAAGCTGCAGAACGAGGTGAGCGAGCTGAAGAACGAGCTGGAGATGGCCAGAACCA
GAGACATGAGAAGCCCCCTGAACGGCAGCAGCGGCAGACTGAGCGACGTGCAGATCA
ACACCAACAGAATGTTCGAGGACCTGGAGGTGAGCGAGGCCACCCTGCAGAAGGCCAA
 
GGAGGAGAACAGCACCCTGAAGAGCCAGTTCGCCGAGCTGGAGGCCAACCTGCACCA
GGTGAACAGCAAGCTGGGCGAGGTGAGATGCGAGCTGAACGAGGCCCTGGCCAGAGT
GGACGGCGAGCAGGAGACCAGAGTGAAGGCCGAGAACGCCCTGGAGGAGGCCAGAC
AGCTGATCAGCAGCCTGAAGCACGAGGAGAACGAGCTGAAGAAGACCATCACCGACAT
GGGCATGAGACTGAACGAGGCCAAGAAGAGCGACGAGTTCCTGAAGAGCGAGCTGAG
CACCGCCCTGGAGGAGGAGAAGAAGAGCCAGAACCTGGCCGACGAGCTGAGCGAGGA
GCTGAACGGCTGGAGAATGAGAACCAAGGAGGCCGAGAACAAGGTGGAGCACGCCAG
CAGCGAGAAGAGCGAGATGCTGGAGAGAATCGTGCACCTGGAGACCGAGATGGAGAA
GCTGAGCACCAGCGAGATCGCCGCCGACTACTGCAGCACCAAGATGACCGAGAGAAA
GAAGGAGATCGAGCTGGCCAAGTACAGAGAGGACTTCGAGAACGCCGCCATCGTGGG
CCTGGAGAGAATCAGCAAGGAGATCAGCGAGCTGACCAAGAAGACCCTGAAGGCCAAG
ATCATCCCCAGCAACATCAGCAGCATCCAGCTGGTGTGCGACGAGCTGTGCAGAAGAC
TGAGCAGAGAGAGAGAGCAGCAGCACGAGTACGCCAAGGTGATGAGAGACGTGAACG
AGAAGATCGAGAAGCTGCAGCTGGAGAAGGACGCCCTGGAGCACGAGCTGAAGATGAT
GAGCAGCAACAACGAGAACGTGCCCCCCGTGGGCACCAGCGTGAGCGGCATGCCCAC
CAAGACCAGCAACCAGAAGTGCGCCCAGCCCCACTACACCAGCCCCACCAGACAGCTG
CTGCACGAGAGCACCATGGCCGTGGACGCCATCGTGCAGAAGCTGAAGAAGACCCACA
ACATGAGCGGCATGGCCCCGAGCTGAAGGAGACCATCGGCAACGTGATCAACGAGA
GCAGAGTGCTGAGAGACTTCCTGCACCAGAAGCTGATCCTGTTCAAGGGCATCGACAT
GAGCAACTGGAAGAACGAGACCGTGGACCAGCTGATCACCGACCTGGGCCAGCTGCA
CCAGGACAACCTGATGCTGGAGGAGCAGATCAAGAAGTACAAGGAGCTGAAGCTG
ACCAAGAGCGCCATCCCCACCCTGGGCGTGGAGTTCCAGGACAGAATCAAGACCGAGA
TCGGCAAGATCGCCACCGACATGGGCGGCGCCGTGAAGGAGATCAGAAAGAAGGGTA
CCGAGCAGAAGCTGATCTCAGAGGAGGACCTGGGCGCCCCCGGCTCCGCCGGCTCCG
CCGCCGGCTCCGGCGCTTCTAACTTTACTCAGTTCGTTCTCGTCGACAATGGCGGAACT
GGCGACGTGACTGTCGCCCCAAGCAACTTCGCTAACGGGATCGCTGAATGGATCAGCT
CTAACTCGCGTTCACAGGCTTACAAAGTAACCTGTAGCGTTCGTCAGAGCTCTGCGCAG
AATCGCAAATACACCATCAAAGTCGAGGTGCCTAAAGGCGCCTGGCGTTCGTACTTAAA
TATGGAACTAACCATTCCAATTTTCGCCACGAATTCCGACTGCGAGCTTATTGTTAAGGC
AATGCAAGGTCTCCTAAAAGATGGAAACCCGATTCCCTCAGCAATCGCAGCAAACTCCG
GCATCTACGGTACCGGCGCCCCCGGCTCCGCCGGCTCCGCCGCCGGCTCCGGCGCG
TGCCCGGTGCCGCTGCAGCTGCCGCCGCTGGAACGCCTGACCCTGGATGATAAAAAC
CGCTGAATACCCTGATCTCTGCTACTGGTCTGTGGATGAGTCGTACCGGAACCATTCAT
AAAATCAAACACCACGAGGTTAGCCGTTCGAAAATCTATATTGAGATGGCGTGTGGCGA
TCATCTGGTTGTGAACAATAGCCGCTCTTCTCGTACAGCACGTGCACTGCGTCACCACA
AATATCGTAAAACCTGTAAACGTTGCCGTGTGTCCGATGAGGATCTGAACAAATTCCTGA
 
CAAAAGCCAATGAGGACCAAACAAGCGTGAAAGTGAAAGTCGTTAGCGCTCCTACCCGT
ACTAAAAAAGCAATGCCGAAATCCGTTGCTCGTGCCCCTAAACCACTGGAAAACACTGA
AGCAGCACAGGCACAGCCGTCTGGAAGCAAATTCTCTCCGGCCATTCCTGTTTCTACCC
AGGAGTCCGTTTCTGTTCCAGCAAGTGTGAGCACCAGCATTAGCAGTATTAGCACCGGT
GCCACCGCTAGCGCCCTGGTTAAAGGCAATACCAATCCGATTACAAGCATGTCTGCCCC
GGTTCAAGCATCAGCTCCAGCACTGACAAAATCCCAAACCGATCGTCTGGAGGTTCTGC
TGAATCCGAAAGACGAAATCAGCCTGAATTCCGGCAAACCGTTTCGTGAACTGGAGAGC
GAACTGCTGTCACGTCGTAAAAAAGACCTGCAACAAATCTATGCCGAAGAACGTGAGAA
CTATCTGGGGAAACTGGAACGTGAAATCACCCGCTTTTTCGTGGATCGTGGCTTTCTGG
AGATCAAATCCCCGATTCTGATTCCTCTGGAGTATATCGAGCGTATGGGCATCGACAAT
GATACCGAACTGAGCAAACAAATTTTCCGTGTGGATAAAAACTTCTGTCTGCGCCCTATG
CTAGCACCAAATCTGGCTAACTATCTGCGCAAACTGGACCGTGCCCTGCCTGATCCTAT
CAAAATCTTCGAGATCGGCCCGTGTTATCGTAAAGAGTCCGACGGTAAAGAACATCTGG
AGGAGTTTACCATGCTGAACTTTTGCCAAATGGGTTCAGGTTGTACTCGTGAGAACCTG
GAAAGCATCATCACCGATTTTCTGAACCACCTGGGCATTGACTTCAAAATTGTGGGCGA
CAGCTGTATGGTGTTTGGCGACACCCTGGATGTCATGCACGGCGACCTGGAACTGTCT
AGTGCCGTTGTGGGCCCAATCCCGCTGGATCGTGAGTGGGGTATCGACAAACCTTGGA
TCGGTGCGGGTTTTGGTCTGGAGCGTCTGCTGAAAGTAAAACACGACTTCAAGAACATC
AAACGTGCTGCACGTTCCGAGTCCTATTACAATGGTATTTCTACTAACCTGTAA (SEQ ID
NO:73)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI
PRICEGLFSRINETTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTMEDNSVLNEDSNLEHVEGQPRRSMSQPVLNVEGDK
RTSSTSATQQQVLSGAFSSADVRSIPIIQTWEENKALKTKITILRGELQMYQRRYSEAKEASQ
KRVKEVMDDYVDLKLGQENVQEKMEQYKLMEEDLLAMQSRIETSEDNFARQMKEFEAQKH
AMEERIKELELSATDANNTTVGSFRGTLDDILKKNDPDFTLTSGYEERKINDLEAKLLSEIDKV
AELEDHIQQLRQELDDQSARLADSENVRAQLEAATGQGILGAAGNAMVPNSTFMIGNGRES
QTRDQLNYIDDLETKLADAKKENDKARQALVEYMNKCSKLEHEIRTMVKNSTFDSSSMLLG
GQTSDELKIQIGKVNGELNVLRAENRELRIRCDQLTGGDGNLSISLGQSRLMAGIATNDVDSI
GQGNETGGTSMRILPRESQLDDLEESKLPLMDTSSAVRNQQQFASMWEDFESVKDSLQNN
HNDTLEGSFNSSMPPPGRDATQSFLSQKSFKNSPIVMQKPKSLHLHLKSHQSEGAGEQIQN

NSFSTKTASPHVSQSHIPILHDMQQILDSSAMFLEGQHDVAVNVEQMQEKMSQIREALARLF
ERLKSSAALFEEILERMGSSDPNADKIKKMKLAFETSINDKLNVSAILEAAEKDLHNMSLNFSI
LEKSIVSQAAEASRRFTIAPDAEDVASSSLLNASYSPLFKFTSNSDIVEKLQNEVSELKNELE
MARTRDMRSPLNGSSGRLSDVQINTNRMFEDLEVSEATLQKAKEENSTLKSQFAELEANLH
QVNSKLGEVRCELNEALARVDGEQETRVKAENALEEARQLISSLKHEENELKKTITDMGMR
LNEAKKSDEFLKSELSTALEEEKKSQNLADELSEELNGWRMRTKEAENKVEHASSEKSEML
ERIVHLETEMEKLSTSEIAADYCSTKMTERKKEIELAKYREDFENAAIVGLERISKEISELTKKT
LKAKIIPSNISSIQLVCDELCRRLSREREQQHEYAKVMRDVNEKIEKLQLEKDALEHELKMMS
SNNENVPPVGTSVSGMPTKTSNQKCAQPHYTSPTRQLLHESTMAVDAIVQKLKKTHNMSG
MGPELKETIGNVINESRVLRDFLHQKLILFKGIDMSNWKNETVDQLITDLGQLHQDNLMLEEQ
IKKYKKELKLTKSAIPTLGVEFQDRIKTEIGKIATDMGGAVKEIRKKGTEQKLISEEDLGAPGSA
GSAAGSGASNFTQFVLVDNGGTGDVTVAPSNFANGIAEWISSNSRSQAYKVTCSVRQSSA
QNRKYTIKVEVPKGAWRSYLNMELTIPIFATNSDCELIVKAMQGLLKDGNPIPSAIAANSGIYG
TGAPGSAGSAAGSGACPVPLQLPPLERLTLDDKKPLNTLISATGLWMSRTGTIHKIKHHEVS
RSKIYIEMACGDHLVVNNSRSSRTARALRHHKYRKTCKRCRVSDEDLNKFLTKANEDQTSV
KVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFR
ELESELLSRRKKDLQQIYAEERENYLGKLEREITRFFVDRGFLEIKSPILIPLEYIERMGIDNDT
ELSKQIFRVDKNFCLRPMLAPNLANYLRKLDRALPDPIKIFEIGPCYRKESDGKEHLEEFTML
NFCQMGSGCTRENLESIITDFLNHLGIDFKIVGDSCMVFGDTLDVMHGDLELSSAVVGPIPLD
REWGIDKPWIGAGFGLERLLKVKHDFKNIKRAARSESYYNGISTNL (SEQ ID NO:74)

KIF16B-SPD5-4xλ_{N22}-PyIRS^{AF}
DNA: 
ATGGCATCGGTCAAGGTGGCCGTGAGGGTCCGGCCCATGAATCGCAGGGAAAAGGAC
TTGGAGGCCAAGTTCATTATTCAGATGGAGAAAGCAAAACGACAATCACAAACTTAAAG
ATACCAGAAGGAGGCACTGGGGACTCAGGAAGAGAACGGACCAAGACCTTCACCTATG
ACTTTTCTTTTTATTCTGCTGATACAAAAAGCCCAGATTACGTTTCACAAGAAATGGTTTT
CAAAACCCTCGGCACAGATGTCGTGAAGTCTGCATTTGAAGGTTATAATGCTTGTGTCTT
TGCATATGGGCAAACTGGATCTGGAAAGTCATACACTATGATGGGAAATTCTGGAGATT
CTGGCTTAATACCTCGGATCTGTGAAGGACTCTTCAGTCGGATAAATGAAACCACCAGA
TGGGATGAAGCTTCTTTTCGAACTGAAGTCAGCTACTTAGAAATTTATAACGAACGTGTG
AGAGATCTACTTCGGCGGAAGTCATCTAAAACCTTCAATTTGAGAGTCCGTGAGCATCC
CAAAGAAGGCCCTTATGTTGAGGATTTATCCAAACATTTAGTACAGAATTATGGTGACGT
AGAAGAACTTATGGATGCGGGCAATATCAACCGGACCACCGCAGCGACTGGGATGAAC
GACGTCAGTAGCAGGTCTCATGCCATCTTCACCATCAAGTTCACTCAGGCTAAATTTGAT
 
TCTGAAATGCCATGTGAAACCGTCAGTAAGATCCACTTGGTTGATCTTGCCGGAAGTGA
GCGTGCAGATGCCACCGGAGCCACCGGGTTAGGCTAAAGGAAGGGGGAAATATTAAC
AAGTCCCTCGTGACTCTGGGGAACGTCATTTCTGCCTTAGCTGATTTATCTCAGGATGC
TGCAAATACTCTTGCAAAGAAGAAGCAAGTTTTCGTGCCTTACAGGGATTCTGTGTTGAC
TTGGTTGTTAAAAGATAGCCTTGGAGGAAACTCTAAAACTATCATGATTGCCACCATTTC
ACCTGCTGATGTCAATTATGGAGAAACCCTAAGTACTCTTCGCTATGCAAATAGAGCCAA
AAACATCATCAACAAGCCTACCATTAATGAGGATGCCAACGTCAAACTTATCCGTGAGCT
GCGAGCTGAAATAGCCAGACTGAAAACGCTGCTTGCTCAAGGGAATCAGATTGCCCTCT
TAGACTCCCCCACAATGGAGGACAACAGCGTGCTGAACGAGGACAGCAACCTGGAGCA
CGTGGAGGGCCAGCCCAGAAGAAGCATGAGCCAGCCCGTGCTGAACGTGGAGGGCGA
CAAGAGAACCAGCAGCACCAGCGCCACCCAGCAGCAGGTGCTGAGCGGCGCCTTCAG
CAGCGCCGACGTGAGAAGCATCCCCATCATCCAGACCTGGGAGGAGAACAAGGCCCT
GAAGACCAAGATCACCATCCTGAGAGGCGAGCTGCAGATGTACCAGAGAAGATACAGC
GAGGCCAAGGAGGCCAGCCAGAAGAGAGTGAAGGAGGTGATGGACGACTACGTGGAC
CTGAAGCTGGGCCAGGAGAACGTGCAGGAGAAGATGGAGCAGTACAAGCTGATGGAG
GAGGACCTGCTGGCCATGCAGAGCAGAATCGAGACCAGCGAGGACAACTTCGCCAGA
CAGATGAAGGAGTTCGAGGCCCAGAAGCACGCCATGGAGGAGAGAATCAAGGAGCTG
GAGCTGAGCGCCACCGACGCCAACAACACCACCGTGGGCAGCTTCAGAGGCACCCTG
GACGACATCCTGAAGAAGAACGACCCCGACTTCACCCTGACCAGCGGCTACGAGGAGA
GAAAGATCAACGACCTGGAGGCCAAGCTGCTGAGCGAGATCGACAAGGTGGCCGAGC
TGGAGGACCACATCCAGCAGCTGAGACAGGAGCTGGACGACCAGAGCGCCAGACTGG
CCGACAGCGAGAACGTGAGAGCCCAGCTGGAGGCCGCCACCGGCCAGGGCATCCTGG
GCGCCGCCGGCAACGCCATGGTGCCCAACAGCACCTTCATGATCGGCAACGGCAGAG
AGAGCCAGACCAGAGACCAGCTGAACTACATCGACGACCTGGAGACCAAGCTGGCCGA
CGCCAAGAAGGAGAACGACAAGGCCAGACAGGCCCTGGTGGAGTACATGAACAAGTG
CAGCAAGCTGGAGCACGAGATCAGAACCATGGTGAAGAACAGCACCTTCGACAGCAGC
AGCATGCTGCTGGGCGGCCAGACCAGCGACGAGCTGAAGATCCAGATCGGCAAGGTG
AACGGCGAGCTGAACGTGCTGAGAGCCGAGAACAGAGAGCTGAGAATCAGATGCGAC
CAGCTGACCGGCGGCGACGGCAACCTGAGCATCAGCCTGGGCCAGAGCAGACTGATG
GCCGGCATCGCCACCAACGACGTGGACAGCATCGGCCAGGGCAACGAGACCGGCGGC
ACCAGCATGAGAATCCTGCCCAGAGAGAGCCAGCTGGACGACCTGGAGGAGAGCAAG
CTGCCCCTGATGGACACCAGCAGCGCCGTGAGAAACCAGCAGCAGTTCGCCAGCATGT
GGGAGGACTTCGAGAGCGTGAAGGACAGCCTGCAGAACAACCACAACGACACCCTGG
AGGGCAGCTTCAACAGCAGCATGCCCCCCCCCGGCAGAGACGCCACCCAGAGCTTCC
TGAGCCAGAAGAGCTTCAAGAACAGCCCCATCGTGATGCAGAAGCCCAAGAGCCTGCA
CCTGCACCTGAAGAGCCACCAGAGCGAGGGCGCCGGCGAGCAGATCCAGAACAACAG
 
CTTCAGCACCAAGACCGCCAGCCCCCACGTGAGCCAGAGCCACATCCCCATCCTGCAC
GACATGCAGCAGATCCTGGACAGCAGCGCCATGTTCCTGGAGGGCCAGCACGACGTG
GCCGTGAACGTGGAGCAGATGCAGGAGAAGATGAGCCAGATCAGAGAGGCCCTGGCC
AGACTGTTCGAGAGACTGAAGAGCAGCGCCGCCCTGTTCGAGGAGATCCTGGAGAGAA
TGGGCAGCAGCGACCCCAACGCCGACAAGATCAAGAAGATGAAGCTGGCCTTCGAGAC
CAGCATCAACGACAAGCTGAACGTGAGCGCCATCCTGGAGGCCGCCGAGAAGGACCT
GCACAACATGAGCCTGAACTTCAGCATCCTGGAGAAGAGCATCGTGAGCCAGGCCGCC
GAGGCCAGCAGAAGATTCACCATCGCCCCCGACGCCGAGGACGTGGCCAGCAGCAGC
CTGCTGAACGCCAGCTACAGCCCCCTGTTCAAGTTCACCAGCAACAGCGACATCGTGG
AGAAGCTGCAGAACGAGGTGAGCGAGCTGAAGAACGAGCTGGAGATGGCCAGAACCA
GAGACATGAGAAGCCCCCTGAACGGCAGCAGCGGCAGACTGAGCGACGTGCAGATCA
ACACCAACAGAATGTTCGAGGACCTGGAGGTGAGCGAGGCCACCCTGCAGAAGGCCAA
GGAGGAGAACAGCACCCTGAAGAGCCAGTTCGCCGAGCTGGAGGCCAACCTGCACCA
GGTGAACAGCAAGCTGGGCGAGGTGAGATGCGAGCTGAACGAGGCCCTGGCCAGAGT
GGACGGCGAGCAGGAGACCAGAGTGAAGGCCGAGAACGCCCTGGAGGAGGCCAGAC
AGCTGATCAGCAGCCTGAAGCACGAGGAGAACGAGCTGAAGAAGACCATCACCGACAT
GGGCATGAGACTGAACGAGGCCAAGAAGAGCGACGAGTTCCTGAAGAGCGAGCTGAG
CACCGCCCTGGAGGAGGAGAAGAAGAGCCAGAACCTGGCCGACGAGCTGAGCGAGGA
GCTGAACGGCTGGAGAATGAGAACCAAGGAGGCCGAGAACAAGGTGGAGCACGCCAG
CAGCGAGAAGAGCGAGATGCTGGAGAGAATCGTGCACCTGGAGACCGAGATGGAGAA
GCTGAGCACCAGCGAGATCGCCGCCGACTACTGCAGCACCAAGATGACCGAGAGAAA
GAAGGAGATCGAGCTGGCCAAGTACAGAGAGGACTTCGAGAACGCCGCCATCGTGGG
CCTGGAGAGAATCAGCAAGGAGATCAGCGAGCTGACCAAGAAGACCCTGAAGGCCAAG
ATCATCCCCAGCAACATCAGCAGCATCCAGCTGGTGTGCGACGAGCTGTGCAGAAGAC
TGAGCAGAGAGAGAGAGCAGCAGCACGAGTACGCCAAGGTGATGAGAGACGTGAACG
AGAAGATCGAGAAGCTGCAGCTGGAGAAGGACGCCCTGGAGCACGAGCTGAAGATGAT
GAGCAGCAACAACGAGAACGTGCCCCCCGTGGGCACCAGCGTGAGCGGCATGCCCAC
CAAGACCAGCAACCAGAAGTGCGCCCAGCCCCACTACACCAGCCCCACCAGACAGCTG
CTGCACGAGAGCACCATGGCCGTGGACGCCATCGTGCAGAAGCTGAAGAAGACCCACA
ACATGAGCGGCATGGGCCCCGAGCTGAAGGAGACCATCGGCAACGTGATCAACGAGA
GCAGAGTGCTGAGAGACTTCCTGCACCAGAAGCTGATCCTGTTCAAGGGCATCGACAT
GAGCAACTGGAAGAACGAGACCGTGGACCAGCTGATCACCGACCTGGGCCAGCTGCA
CCAGGACAACCTGATGCTGGAGGAGCAGATCAAGAAGTACAAGGAGCTGAAGCTG
ACCAAGAGCGCCATCCCCACCCTGGGCGTGGAGTTCCAGGACAGAATCAAGACCGAGA
TCGGCAAGATCGCCACCGACATGGGCGGCGCCGTGAAGGAGATCAGAAAGAAGTCCG
GATATCCCTATGATGTGCCGGATTATGCTTCCACCATGGACGCACAAACACGACGACGT
 
GAGCGTCGCGCTGAGAAACAAGCTCAATGGAAAGCTGCAAACCCACCGCTCGACGGAG
CCGGAGCTGGCGCTGGAGCTGGAGCCGGAGCTGGCGGTCTAGCCACCATGGACGCAC
AAACACGACGACGTGAGCGTCGCGCTGAGAAACAAGCTCAATGGAAAGCTGCAAACCC
ACCGCTCGACGGAGCCGGAGCTGGCGCTGGAGCTGGAGCCGGAGCTGGCGGTCTAG
CCACCATGGACGCACAAACACGACGACGTGAGCGTCGCGCTGAGAAACAAGCTCAATG
GAAAGCTGCAAACCCACCGCTCGACGGAGCCGGAGCTGGCGCTGGAGCTGGAGCCGG
AGCTGGCGGTCTAGCCACCATGGACGCACAAACACGACGACGTGAGCGTCGCGCTGA
GAAACAAGCTCAATGGAAAGCTGCAAACCCACCGCTCGGTACCGGCGCCCCCGGCTCC
GCCGGCTCCGCCGCCGGCTCCGGCGCGTGCCCGGTGCCGCTGCAGCTGCCGCCGCT
GGAACGCCTGACCCTGGATGATAAAAACCGCTGAATACCCTGATCTCTGCTACTGGTC
TGTGGATGAGTCGTACCGGAACCATTCATAAAATCAAACACCACGAGGTTAGCCGTTCG
AAAATCTATATTGAGATGGCGTGTGGCGATCATCTGGTTGTGAACAATAGCCGCTCTTCT
CGTACAGCACGTGCACTGCGTCACCACAAATATCGTAAAACCTGTAAACGTTGCCGTGT
GTCCGATGAGGATCTGAACAAATTCCTGACAAAAGCCAATGAGGACCAAACAAGCGTGA
AAGTGAAAGTCGTTAGCGCTCCTACCCGTACTAAAAAAGCAATGCCGAAATCCGTTGCT
CGTGCCCCTAAACCACTGGAAAACACTGAAGCAGCACAGGCACAGCCGTCTGGAAGCA
AATTCTCTCCGGCCATTCCTGTTTCTACCCAGGAGTCCGTTTCTGTTCCAGCAAGTGTGA
GCACCAGCATTAGCAGTATTAGCACCGGTGCCACCGCTAGCGCCCTGGTTAAAGGCAA
TACCAATCCGATTACAAGCATGTCTGCCCCGGTTCAAGCATCAGCTCCAGCACTGACAA
AATCCCAAACCGATCGTCTGGAGGTTCTGCTGAATCCGAAAGACGAAATCAGCCTGAAT
TCCGGCAAACCGTTTCGTGAACTGGAGAGCGAACTGCTGTCACGTCGTAAAAAAGACCT
GCAACAAATCTATGCCGAAGAACGTGAGAACTATCTGGGGAAACTGGAACGTGAAATCA
CCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCG
TGTGGATAAAAACTTCTGTCTGCGCCCTATGCTAGCACCAAATCTGGCTAACTATCTGC
GCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGAACTTTTGCCA
AATGGGTTCAGGTTGTACTCGTGAGAACCTGGAAAGCATCATCACCGATTTTCTGAACC
ACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGG
ATCGTGAGTGGGGTATCGACAAACCTTGGATCGGTGCGGGTTTTGGTCTGGAGCGTCT
GCTGAAAGTAAAACACGACTTCAAGAACATCAAACGTGCTGCACGTTCCGAGTCCTATT
ACAATGGTATTTCTACTAACCTGTAA (SEQ ID NO:75)
Protein:

MASVKVAVRVRPMNRREKDLEAKFIIQMEKSKTTITNLKIPEGGTGDSGRERTKTFTYDFSF
YSADTKSPDYVSQEMVFKTLGTDVVKSAFEGYNACVFAYGQTGSGKSYTMMGNSGDSGLI

PRICEGLFSRINETTRWDEASFRTEVSYLEIYNERVRDLLRRKSSKTFNLRVREHPKEGPYV
EDLSKHLVQNYGDVEELMDAGNINRTTAATGMNDVSSRSHAIFTIKFTQAKFDSEMPCETVS
KIHLVDLAGSERADATGATGVRLKEGGNINKSLVTLGNVISALADLSQDAANTLAKKKQVFVP
YRDSVLTWLLKDSLGGNSKTIMIATISPADVNYGETLSTLRYANRAKNIINKPTINEDANVKLIR
ELRAEIARLKTLLAQGNQIALLDSPTMEDNSVLNEDSNLEHVEGQPRRSMSQPVLNVEGDK
RTSSTSATQQQVLSGAFSSADVRSIPIIQTWEENKALKTKITILRGELQMYQRRYSEAKEASQ
KRVKEVMDDYVDLKLGQENVQEKMEQYKLMEEDLLAMQSRIETSEDNFARQMKEFEAQKH
AMEERIKELELSATDANNTTVGSFRGTLDDILKKNDPDFTLTSGYEERKINDLEAKLLSEIDKV
AELEDHIQQLRQELDDQSARLADSENVRAQLEAATGQGILGAAGNAMVPNSTFMIGNGRES
QTRDQLNYIDDLETKLADAKKENDKARQALVEYMNKCSKLEHEIRTMVKNSTFDSSSMLLG
GQTSDELKIQIGKVNGELNVLRAENRELRIRCDQLTGGDGNLSISLGQSRLMAGIATNDVDSI
GQGNETGGTSMRILPRESQLDDLEESKLPLMDTSSAVRNQQQFASMWEDFESVKDSLQNN
HNDTLEGSFNSSMPPPGRDATQSFLSQKSFKNSPIVMQKPKSLHLHLKSHQSEGAGEQIQN
NSFSTKTASPHVSQSHIPILHDMQQILDSSAMFLEGQHDVAVNVEQMQEKMSQIREALARLF
ERLKSSAALFEEILERMGSSDPNADKIKKMKLAFETSINDKLNVSAILEAAEKDLHNMSLNFSI
LEKSIVSQAAEASRRFTIAPDAEDVASSSLLNASYSPLFKFTSNSDIVEKLQNEVSELKNELE
MARTRDMRSPLNGSSGRLSDVQINTNRMFEDLEVSEATLQKAKEENSTLKSQFAELEANLH
QVNSKLGEVRCELNEALARVDGEQETRVKAENALEEARQLISSLKHEENELKKTITDMGMR
LNEAKKSDEFLKSELSTALEEEKKSQNLADELSEELNGWRMRTKEAENKVEHASSEKSEML
ERIVHLETEMEKLSTSEIAADYCSTKMTERKKEIELAKYREDFENAAIVGLERISKEISELTKKT
LKAKIIPSNISSIQLVCDELCRRLSREREQQHEYAKVMRDVNEKIEKLQLEKDALEHELKMMS
SNNENVPPVGTSVSGMPTKTSNQKCAQPHYTSPTRQLLHESTMAVDAIVQKLKKTHNMSG
MGPELKETIGNVINESRVLRDFLHQKLILFKGIDMSNWKNETVDQLITDLGQLHQDNLMLEEQ
IKKYKKELKLTKSAIPTLGVEFQDRIKTEIGKIATDMGGAVKEIRKKSGYPYDVPDYASTMDAQ
TRRRERRAEKQAQWKAANPPLDGAGAGAGAGAGAGGLATMDAQTRRRERRAEKQAQWK
AANPPLDGAGAGAGAGAGAGGLATMDAQTRRRERRAEKQAQWKAANPPLDGAGAGAGA
GAGAGGLATMDAQTRRRERRAEKQAQWKAANPPLGTGAPGSAGSAAGSGACPVPLQLPP
LERLTLDDKKPLNTLISATGLWMSRTGTIHKIKHHEVSRSKIYIEMACGDHLVVNNSRSSRTA
RALRHHKYRKTCKRCRVSDEDLNKFLTKANEDQTSVKVKVVSAPTRTKKAMPKSVARAPKP
LENTEAAQAQPSGSKFSPAIPVSTQESVSVPASVSTSISSISTGATASALVKGNTNPITSMSA
PVQASAPALTKSQTDRLEVLLNPKDEISLNSGKPFRELESELLSRRKKDLQQIYAEERENYL
GKLEREITRFFVDRGFLEIKSPILIPLEYIERMGIDNDTELSKQIFRVDKNFCLRPMLAPNLANY
LRKLDRALPDPIKIFEIGPCYRKESDGKEHLEEFTMLNFCQMGSGCTRENLESIITDFLNHLGI
DFKIVGDSCMVFGDTLDVMHGDLELSSAVVGPIPLDREWGIDKPWIGAGFGLERLLKVKHDF
KNIKRAARSESYYNGISTNL (SEQ ID NO:76)

KIF13A-FUS-PyIRS^{AF}
DNA: 
ATGTCGGATACCAAGGTAAAAGTTGCCGTCCGGGTCCGGCCCATGAACCGACGAGAAC
TGGAACTGAACACCAAGTGCGTGGTGGAGATGGAAGGGAATCAAACGGTCCTGCACCC
TCCTCCTTCTAACACCAAACAGGGAGAAAGGAAACCTCCCAAGGTATTTGCCTTTGATTA
TTGCTTTTGGTCCATGGATGAATCTAACACTACAAAATACGCTGGTCAAGAAGTGGTTTT
CAAGTGCCTTGGGGAAGGAATTCTTGAAAAAGCCTTTCAGGGGTATAATGCGTGTATTT
TTGCATATGGACAGACAGGTTCGGGAAAATCCTTTTCCATGATGGGCCATGCTGAGCAG
CTGGGCCTTATTCCAAGGCTCTGCTGTGCTTTATTTAAAAGGATCTCTTTGGAGCAAAAT
GAGTCACAGACCTTTAAAGTTGAAGTGTCCTATATGGAAATTTATAATGAGAAAGTTCGG
GATCTTTTAGACCCCAAAGGGAGTAGACAGTCTCTTAAAGTTCGAGAACATAAAGTTTTG
GGACCATATGTAGATGGTTTATCTCAACTAGCTGTCACTAGTTTTGAGGATATTGAGTCA
TTGATGTCTGAGGGAAATAAGTCTCGAACGGTAGCTGCTACCAACATGAACGAAGAAAG
CAGCCGCTCCCATGCTGTGTTCAACATCATAATCACACAGACACTTTATGACCTGCAGT
CTGGGAATTCCGGGGAGAAAGTCAGTAAGGTCAGCTTGGTAGACCTGGCGGGTAGCGA
AAGAGTATCTAAAACAGGAGCTGCAGGAGAGCGACTGAAAGAAGGCAGCAACATTAAC
AAATCGCTTACAACCTTGGGGTTGGTTATATCATCACTGGCTGACCAGGCAGCTGGCAA
GGGTAAAAGCAAATTTGTGCCTTATCGAGATTCAGTCCTCACTTGGCTGCTTAAGGACA
ACTTGGGGGGCAACAGCCAAACCTCTATGATAGCCACAATCAGCCCAGCCGCAGACAA
CTATGAAGAGACCCTCTCCACATTAAGATATGCAGACCGAGCCAAAAGGATTGTGAACC
ATGCTGTTGTGAATGAGGACCCCAACGCAAAAGTGATCCGAGAACTGCGGGAGGAAGT
CGAGAAACTGAGAGAGCAGCTCTCTCAGGCAGAGGCCATGAAGGCCGAACTGAAGGA
GAAGCTCGAAGAGTCTGAAAAGCTGATAAAAGAACTAACAGTGACTTGGGAATATACAG
ATATTGAAATGAACAGATTGGGAAAGGGCGCCCCCGGCTCCGCCGGCTCCGCCGCCG
GCTCCGGCATGGCCTCAAACGATTATACCCAACAAGCAACCCAAAGCTATGGGGCCTA
CCCCACCCAGCCCGGGCAGGGCTATTCCCAGCAGAGCAGTCAGCCCTACGGACAGCA
GAGTTACAGTGGTTATAGCCAGTCCACGGACACTTCAGGATATGGCCAGAGCAGCTATT
CTTCTTATGGCCAGAGCCAGAACACAGGCTATGGAACTCAGTCAACTCCCCAGGATAT
GGCTCGACTGGCGGCTATGGCAGTAGCCAGAGCTCCCAATCGTCTTACGGGCAGCAGT
CCTCCTACCCTGGCTATGGCCAGCAGCCAGCTCCCAGCAGCACCTCGGGAAGTTACGG
TAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCA
GGGCTATGGACAGCAGAACCAGTACAACAGCAGCAGTGGTGGTGGAGGTGGAGGTGG
AGGTGGAGGTAACTATGGCCAAGATCAATCCTCCATGAGTAGTGGTGGTGGCAGTGGT
GGCGGTTATGGCAATCAAGACCAGAGTGGTGGAGGTGGCAGCGGTGGCTATGGACAG
CAGGACCGTGGAGGCCGCGGCAGGGGTGGCAGTGGTGGCGGCGGCGGCGGCG
 
GTGGTGGTTACAACCGCAGCAGTGGTGGCTATGAACCCAGAGGTCGTGGAGGTGGCC
GTGGAGGCAGAGGTGGCATGGGCGGAAGTGACCGTGGTGGCTTCAATAAATTTGGTGG
CCCTCGGGACCAAGGATCACGTCATGACTCCGAACAGGATAATTCAGACAACAACACCA
TCTTTGTGCAAGGCCTGGGTGAGAATGTTACAATTGAGTCTGTGGCTGATTACTTCAAG
CAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTT
CAGCTAAAGCAGCTATTGACTGGTTTGATGGTAAAGAATTCTCCGGAAATCCTATCAAG
GTCTCATTTGCTACTCGCCGGGCAGACTTTAATCGGGGTGGTGGCAATGGTCGTGGAG
GCCGAGGGCGAGGAGGACCCATGGGCCGTGGAGGCTATGGAGGTGGTGGCAGTGGT
GGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAA
TGAATGCAACCAGTGTAAGGCCCCTAAACCAGATGGCCCAGGAGGGGGACCAGGTGG
CTCTCACATGGGGGGTAACTACGGGGATGATCGTCGTGGTGGCAGAGGAGGCGATTAC
AAGGATGACGACGATAAGGGTACCGGCGCCCCGGCTCCGCCGGCTCCGCCGCCGGC
TCCGGCGCGTGCCCGGTGCCGCTGCAGCTGCCGCCGCTGGAACGCCTGACCCTGGAT
GATAAAAAACCGCTGAATACCCTGATCTCTGCTACTGGTCTGTGGATGAGTCGTACCGG
AACCATTCATAAAATCAAACACCACGAGGTTAGCCGTTCGAAAATCTATATTGAGATGGC
GTGTGGCGATCATCTGGTTGTGAACAATAGCCGCTCTTCTCGTACAGCACGTGCACTGC
GTCACCACAAATATCGTAAAACCTGTAAACGTTGCCGTGTGTCCGATGAGGATCTGAAC
AAATTCCTGACAAAAGCCAATGAGGACCAAACAAGCGTGAAAGTGAAAGTCGTTAGCGC
TCCTACCCGTACTAAAAAAGCAATGCCGAAATCCGTTGCTCGTGCCCCTAAACCACTGG
AAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTAT
TAGCACCGGTGCCACCGCTAGCGCCCTGGTTAAAGGCAATACCAATCCGATTACAAGC
ATGTCTGCCCCGGTTCAAGCATCAGCTCCAGCACTGACAAAATCCCAAACCGATCGTCT
GGAGGTTCTGCTGAATCCGAAAGACGAAATCAGCCTGAATTCCGGCAAACCGTTTCGTG
AACTGGAGAGCGAACTGCTGTCACGTCGTAAAAAAGACCTGCAACAAATCTATGCCGAA
GAACGTGAGAACTATCTGGGGAAACTGGAACGTGAAATCACCCGCTTTTTCGTGGATCG
TGGCTTTCTGGAGATCAAATCCCCGATTCTGATTCCTCTGGAGTATATCGAGCGTATGG
GCATCGACAATGATACCGAACTGAGCAAACAAATTTTCCGTGTGGATAAAAACTTCTGTC
TGCGCCCTATGCTAGCACCAAATCTGGCTAACTATCTGCGCAAACTGGACCGTGCCCTG
CCTGATCCTATCAAAATCTTCGAGATCGGCCCGTGTTATCGTAAAGAGTCCGACGGTAA
AGAACATCTGGAGGAGTTTACCATGCTGAACTTTTGCCAAATGGGTTCAGGTTGTACTC
GTGAGAACCTGGAAAGCATCATCACCGATTTTCTGAACCACCTGGGCATTGACTTCAAA
ATTGTGGGCGACAGCTGTATGGTGTTTGGCGACACCCTGGATGTCATGCACGGCGACC
TGGAACTGTCTAGTGCCGTTGTGGGCCCAATCCCGCTGGATCGTGAGTGGGGTATCGA
 
CAAACCTTGGATCGGTGCGGGTTTTGGTCTGGAGCGTCTGCTGAAAGTAAAACACGACT
TCAAGAACATCAAACGTGCTGCACGTTCCGAGTCCTATTACAATGGTATTTCTACTAACC
TGTAA (SEQ ID NO:77)
Protein:

MSDTKVKVAVRVRPMNRRELELNTKCVVEMEGNQTVLHPPPSNTKQGERKPPKVFAFDYC
FWSMDESNTTKYAGQEVVFKCLGEGILEKAFQGYNACIFAYGQTGSGKSFSMMGHAEQLG
LIPRLCCALFKRISLEQNESQTFKVEVSYMEIYNEKVRDLLDPKGSRQSLKVREHKVLGPYV
DGLSQLAVTSFEDIESLMSEGNKSRTVAATNMNEESSRSHAVFNIIITQTLYDLQSGNSGEKV
SKVSLVDLAGSERVSKTGAAGERLKEGSNINKSLTTLGLVISSLADQAAGKGKSKFVPYRDS
VLTWLLKDNLGGNSQTSMIATISPAADNYEETLSTLRYADRAKRIVNHAVVNEDPNAKVIREL
REEVEKLREQLSQAEAMKAELKEKLEESEKLIKELTVTWEYTDIEMNRLGKGAPGSAGSAA
GSGMASNDYTQQATQSYGAYPTQPGQGYSQQSSQPYGQQSYSGYSQSTDTSGYGQSSY
SSYGQSQNTGYGTQSTPQGYGSTGGYGSSQSSQSSYGQQSSYPGYGQQPAPSSTSGSY
GSSSQSSSYGQPQSGSYSQQPSYGGQQQSYGQQQSYNPPQGYGQQNQYNSSSGGGG
GGGGGGNYGQDQSSMSSGGGSGGGYGNQDQSGGGGSGGYGQQDRGGRGRGGSGGG
GGGGGGGYNRSSGGYEPRGRGGGRGGRGGMGGSDRGGFNKFGGPRDQGSRHDSEQD
NSDNNTIFVQGLGENVTIESVADYFKQIGIIKTNKKTGQPMINLYTDRETGKLKGEATVSFDD
PPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGG
GPGGSHMGGNYGDDRRGGRGGDYKDDDDKGTGAPGSAGSAAGSGACPVPLQLPPLERL
TLDDKKPLNTLISATGLWMSRTGTIHKIKHHEVSRSKIYIEMACGDHLVVNNSRSSRTARALR
HHKYRKTCKRCRVSDEDLNKFLTKANEDQTSVKVKVVSAPTRTKKAMPKSVARAPKPLENT
EAAQAQPSGSKFSPAIPVSTQESVSVPASVSTSISSISTGATASALVKGNTNPITSMSAPVQA
SAPALTKSQTDRLEVLLNPKDEISLNSGKPFRELESELLSRRKKDLQQIYAEERENYLGKLER
EITRFFVDRGFLEIKSPILIPLEYIERMGIDNDTELSKQIFRVDKNFCLRPMLAPNLANYLRKLD
RALPDPIKIFEIGPCYRKESDGKEHLEEFTMLNFCQMGSGCTRENLESIITDFLNHLGIDFKIV
GDSCMVFGDTLDVMHGDLELSSAVVGPIPLDREWGIDKPWIGAGFGLERLLKVKHDFKNIK
RAARSESYYNGISTNL (SEQ ID NO:78)

KIF13A-FUS-PyIRS^{AA}
DNA: 
ATGTCGGATACCAAGGTAAAAGTTGCCGTCCGGGTCCGGCCCATGAACCGACGAGAAC
TGGAACTGAACACCAAGTGCGTGGTGGAGATGGAAGGAATCAAACGGTCCTGCACCC
TCCTCCTTCTAACACCAAACAGGGAGAAAGGAAACCTCCCAAGGTATTTGCCTTTGATTA
TTGCTTTTGGTCCATGGATGAATCTAACACTACAAAATACGCTGGTCAAGAAGTGGTTTT
CAAGTGCCTTGGGGAAGGAATTCTTGAAAAAGCCTTTCAGGGGTATAATGCGTGTATTT
 
TTGCATATGGACAGACAGGTTCGGGAAAATCCTTTTCCATGATGGCCATGCTGAGCAG
CTGGGCCTTATTCCAAGGCTCTGCTGTGCTTTATTTAAAAGGATCTCTTTGGAGCAAAAT
GAGTCACAGACCTTTAAAGTTGAAGTGTCCTATATGGAAATTTATAATGAGAAAGTTCGG
GATCTTTTAGACCCCAAAGGGAGTAGACAGTCTCTTAAAGTTCGAGAACATAAAGTTTTG
GGACCATATGTAGATGGTTTATCTCAACTAGCTGTCACTAGTTTTGAGGATATTGAGTCA
TTGATGTCTGAGGGAAATAAGTCTCGAACGGTAGCTGCTACCAACATGAACGAAGAAAG
CAGCCGCTCCCATGCTGTGTTCAACATCATAATCACACAGACACTTTATGACCTGCAGT
CTGGGAATTCCGGGGAGAAAGTCAGTAAGGTCAGCTTGGTAGACCTGGCGGGTAGCGA
AAGAGTATCTAAAACAGGAGCTGCAGGAGAGCGACTGAAAGAAGGCAGCAACATTAAC
AAATCGCTTACAACCTTGGGGTTGGTTATATCATCACTGGCTGACCAGGCAGCTGGCAA
GGGTAAAAGCAAATTTGTGCCTTATCGAGATTCAGTCCTCACTTGGCTGCTTAAGGACA
ACTTGGGGGGCAACAGCCAAACCTCTATGATAGCCACAATCAGCCCAGCCGCAGACAA
CTATGAAGAGACCCTCTCCACATTAAGATATGCAGACCGAGCCAAAAGGATTGTGAACC
ATGCTGTTGTGAATGAGGACCCCAACGCAAAAGTGATCCGAGAACTGCGGGAGGAAGT
CGAGAAACTGAGAGAGCAGCTCTCTCAGGCAGAGGCCATGAAGGCCGAACTGAAGGA
GAAGCTCGAAGAGTCTGAAAAGCTGATAAAAGAACTAACAGTGACTTGGGAATATACAG
ATATTGAAATGAACAGATTGGGAAAGGCGCCCCCGGCTCCGCCGGCTCCGCCGCCG
GCTCCGGCATGGCCTCAAACGATTATACCCAACAAGCAACCCAAAGCTATGGGCCTA
CCCCACCCAGCCCGGGCAGGGCTATTCCCAGCAGAGCAGTCAGCCCTACGGACAGCA
GAGTTACAGTGGTTATAGCCAGTCCACGGACACTTCAGGATATGGCCAGAGCAGCTATT
CTTCTTATGGCCAGAGCCAGAACACAGGCTATGGAACTCAGTCAACTCCCCAGGGATAT
GGCTCGACTGGCGGCTATGGCAGTAGCCAGAGCTCCCAATCGTCTTACGGGCAGCAGT
CCTCCTACCCTGGCTATGGCCAGCAGCCAGCTCCCAGCAGCACCTCGGGAAGTTACGG
TAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCA
GGGCTATGGACAGCAGAACCAGTACAACAGCAGCAGTGGTGGTGGAGGTGGAGGTGG
AGGTGGAGGTAACTATGGCCAAGATCAATCCTCCATGAGTAGTGGTGGTGGCAGTGGT
GGCGGTTATGGCAATCAAGACCAGAGTGGTGGAGGTGGCAGCGGTGGCTATGGACAG
CAGGACCGTGGAGGCCGCGGCAGGGGTGGCAGTGGTGGCGGCGGCGGCGGCGGCG
GTGGTGGTTACAACCGCAGCAGTGGTGGCTATGAACCCAGAGGTCGTGGAGGTGGCC
GTGGAGGCAGAGGTGGCATGGGCGGAAGTGACCGTGGTGGCTTCAATAAATTTGGTGG
CCCTCGGGACCAAGGATCACGTCATGACTCCGAACAGGATAATTCAGACAACAACACCA
TCTTTGTGCAAGGCCTGGGTGAGAATGTTACAATTGAGTCTGTGGCTGATTACTTCAAG
CAGATTGGTATTATTAAGACAAACAAGAAAACGGACAGCCCATGATTAATTTGTACACA
GACAGGGAAACTGGCAAGCTGAAGGGAGAGGCAACGGTCTCTTTTGATGACCCACCTT
CAGCTAAAGCAGCTATTGACTGGTTTGATGGTAAAGAATTCTCCGGAAATCCTATCAAG
 
GTCTCATTTGCTACTCGCCGGGCAGACTTTAATCGGGGTGGTGGCAATGGTCGTGGAG
GCCGAGGGCGAGGAGGACCCATGGGCCGTGGAGGCTATGGAGGTGGTGGCAGTGGT
GGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAA
TGAATGCAACCAGTGTAAGGCCCCTAAACCAGATGGCCCAGGAGGGGGACCAGGTGG
CTCTCACATGGGGGGTAACTACGGGGATGATCGTCGTGGTGGCAGAGGAGGCGGCGC
CCCCGGCTCCGCCGGCTCCGCCGCCGGCTCCGGCATGGCGTGCCCGGTGCCGCTGC
AGCTGCCGCCGCTGGAACGCCTGACCCTGGATGACAAAAACCGCTGAATACCCTGAT
CTCTGCTACTGGTCTGTGGATGAGTCGTACCGGAACCATTCATAAAATCAAACACCACG
AGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTG
TAAACGTTGCCGTGTGTCCGATGAGGATCTGAACAAATTCCTGACAAAAGCCAATGAGG
ACCAAACAAGCGTGAAAGTGAAAGTCGTTAGCGCTCCTACCCGTACTAAAAAAGCAATG
CCGAAATCCGTTGCTCGTGCCCCTAAACCACTGGAAAACACTGAAGCAGCACAGGCAC
AGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCG
CCCTGGTTAAAGGCAATACCAATCCGATTACAAGCATGTCTGCCCCGGTTCAAGCATCA
GCTCCAGCACTGACAAAATCCCAAACCGATCGTCTGGAGGTTCTGCTGAATCCGAAAGA
CGAAATCAGCCTGAATTCCGGCAAACCGTTTCGTGAACTGGAGAGCGAACTGCTGTCAC
GTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCC
GATTCTGATTCCTCTGGAGTATATCGAGCGTATGGGCATCGACAATGATACCGAACTGA
GCAAACAAATTTTCCGTGTGGATAAAAACTTCTGTCTGCGCCCTATGCTGGCACCAAATC
TGTATAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCAT
GCTGGCCTTTGCCCAAATGGGTTCAGGTTGTACTCGTGAGAACCTGGAAAGCATCATCA
CCGATTTTCTGAACCACCTGGGCATTGACTTCAAAATTGTGGGCGACAGCTGTATGGTG
TATGGCGACACCCTGGATGTCATGCACGGCGACCTGGAACTGTCTAGTGCCGTTGTTG
GACCAATTCCGCTGGACCGTGAGTGGGGTATCGACAAACCGTGGATCGGAGCAGGATT
CGGTCTGGAACGCCTGCTGAAAGTGAAACACGACTTCAAAAACATCAAACGTGCCGCC
CGTTCTGAATCGTATTATAACGGGATCTCTACGAACCTGTAA (SEQ ID NO:79)
Protein:

MSDTKVKVAVRVRPMNRRELELNTKCVVEMEGNQTVLHPPPSNTKQGERKPPKVFAFDYC
FWSMDESNTTKYAGQEVVFKCLGEGILEKAFQGYNACIFAYGQTGSGKSFSMMGHAEQLG
LIPRLCCALFKRISLEQNESQTFKVEVSYMEIYNEKVRDLLDPKGSRQSLKVREHKVLGPYV
DGLSQLAVTSFEDIESLMSEGNKSRTVAATNMNEESSRSHAVFNIIITQTLYDLQSGNSGEKV

SKVSLVDLAGSERVSKTGAAGERLKEGSNINKSLTTLGLVISSLADQAAGKGKSKFVPYRDS
VLTWLLKDNLGGNSQTSMIATISPAADNYEETLSTLRYADRAKRIVNHAVVNEDPNAKVIREL
REEVEKLREQLSQAEAMKAELKEKLEESEKLIKELTVTWEYTDIEMNRLGKGAPGSAGSAA
GSGMASNDYTQQATQSYGAYPTQPGQGYSQQSSQPYGQQSYSGYSQSTDTSGYGQSSY
SSYGQSQNTGYGTQSTPQGYGSTGGYGSSQSSQSSYGQQSSYPGYGQQPAPSSTSGSY
GSSSQSSSYGQPQSGSYSQQPSYGGQQQSYGQQQSYNPPQGYGQQNQYNSSSGGGG
GGGGGGNYGQDQSSMSSGGGSGGGYGNQDQSGGGGSGGYGQQDRGGRGRGGSGGG
GGGGGGGYNRSSGGYEPRGRGGGRGGRGGMGGSDRGGFNKFGGPRDQGSRHDSEQD
NSDNNTIFVQGLGENVTIESVADYFKQIGIIKTNKKTGQPMINLYTDRETGKLKGEATVSFDD
PPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGG
GPGGSHMGGNYGDDRRGGRGGGAPGSAGSAAGSGMACPVPLQLPPLERLTLDDKKPLN
TLISATGLWMSRTGTIHKIKHHEVSRSKIYIEMACGDHLVVNNSRSSRTARALRHHKYRKTCK
RCRVSDEDLNKFLTKANEDQTSVKVKVVSAPTRTKKAMPKSVARAPKPLENTEAAQAQPS
GSKFSPAIPVSTQESVSVPASVSTSISSISTGATASALVKGNTNPITSMSAPVQASAPALTKS
QTDRLEVLLNPKDEISLNSGKPFRELESELLSRRKKDLQQIYAEERENYLGKLEREITRFFVD
RGFLEIKSPILIPLEYIERMGIDNDTELSKQIFRVDKNFCLRPMLAPNLYNYLRKLDRALPDPIKI
FEIGPCYRKESDGKEHLEEFTMLAFAQMGSGCTRENLESIITDFLNHLGIDFKIVGDSCMVYG
DTLDVMHGDLELSSAVVGPIPLDREWGIDKPWIGAGFGLERLLKVKHDFKNIKRAARSESYY
NGISTNL (SEQ ID NO:80)

KIF13A-FUS-PyIRS^{AAAF}
DNA: 
ATGTCGGATACCAAGGTAAAAGTTGCCGTCCGGGTCCGGCCCATGAACCGACGAGAAC
TGGAACTGAACACCAAGTGCGTGGTGGAGATGGAAGGGAATCAAACGGTCCTGCACCC
TCCTCCTTCTAACACCAAACAGGGAGAAAGGAAACCTCCCAAGGTATTTGCCTTTGATTA
TTGCTTTTGGTCCATGGATGAATCTAACACTACAAAATACGCTGGTCAAGAAGTGGTTTT
CAAGTGCCTTGGGGAAGGAATTCTTGAAAAAGCCTTTCAGGGGTATAATGCGTGTATTT
TTGCATATGGACAGACAGGTTCGGGAAAATCCTTTTCCATGATGGGCCATGCTGAGCAG
CTGGGCCTTATTCCAAGGCTCTGCTGTGCTTTATTTAAAAGGATCTCTTTGGAGCAAAAT
GAGTCACAGACCTTTAAAGTTGAAGTGTCCTATATGGAAATTTATAATGAGAAAGTTCGG
GATCTTTTAGACCCCAAAGGGAGTAGACAGTCTCTTAAAGTTCGAGAACATAAAGTTTTG
GGACCATATGTAGATGGTTTATCTCAACTAGCTGTCACTAGTTTTGAGGATATTGAGTCA
TTGATGTCTGAGGGAAATAAGTCTCGAACGGTAGCTGCTACCAACATGAACGAAGAAAG
CAGCCGCTCCCATGCTGTGTTCAACATCATAATCACACAGACACTTTATGACCTGCAGT
CTGGGAATTCCGGGGAGAAAGTCAGTAAGGTCAGCTTGGTAGACCTGGCGGGTAGCGA
 
AAGAGTATCTAAAACAGGAGCTGCAGGAGCGACTGAAAGAAGGCAGCAACATTAAC
AAATCGCTTACAACCTTGGGGTTGGTTATATCATCACTGGCTGACCAGGCAGCTGGCAA
GGGTAAAAGCAAATTTGTGCCTTATCGAGATTCAGTCCTCACTTGGCTGCTTAAGGACA
ACTTGGGGGGCAACAGCCAAACCTCTATGATAGCCACAATCAGCCCAGCCGCAGACAA
CTATGAAGAGACCCTCTCCACATTAAGATATGCAGACCGAGCCAAAAGGATTGTGAACC
ATGCTGTTGTGAATGAGGACCCCAACGCAAAAGTGATCCGAGAACTGCGGGAGGAAGT
CGAGAAACTGAGAGAGCAGCTCTCTCAGGCAGAGGCCATGAAGGCCGAACTGAAGGA
GAAGCTCGAAGAGTCTGAAAAGCTGATAAAAGAACTAACAGTGACTTGGGAATATACAG
ATATTGAAATGAACAGATTGGGAAAGGGCGCCCCCGGCTCCGCCGGCTCCGCCGCCG
GCTCCGGCATGGCCTCAAACGATTATACCCAACAAGCAACCCAAAGCTATGGGGCCTA
CCCCACCCAGCCCGGGCAGGGCTATTCCCAGCAGAGCAGTCAGCCCTACGGACAGCA
GAGTTACAGTGGTTATAGCCAGTCCACGGACACTTCAGGATATGGCCAGAGCAGCTATT
CTTCTTATGGCCAGAGCCAGAACACAGGCTATGGAACTCAGTCAACTCCCCAGGGATAT
GGCTCGACTGGCGGCTATGGCAGTAGCCAGAGCTCCCAATCGTCTTACGGCAGCAGT
CCTCCTACCCTGGCTATGGCCAGCAGCCAGCTCCCAGCAGCACCTCGGGAAGTTACGG
TAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCA
GGGCTATGGACAGCAGAACCAGTACAACAGCAGCAGTGGTGGTGGAGGTGGAGGTGG
AGGTGGAGGTAACTATGGCCAAGATCAATCCTCCATGAGTAGTGGTGGTGGCAGTGGT
GGCGGTTATGGCAATCAAGACCAGAGTGGTGGAGGTGGCAGCGGTGGCTATGGACAG
CAGGACCGTGGAGGCCGCGGCAGGGGTGGCAGTGGTGGCGGCGGCGGCGGCG
GTGGTGGTTACAACCGCAGCAGTGGTGGCTATGAACCCAGAGGTCGTGGAGGTGGCC
GTGGAGGCAGAGGTGGCATGGGCGGAAGTGACCGTGGTGGCTTCAATAAATTTGGTGG
CCCTCGGGACCAAGGATCACGTCATGACTCCGAACAGGATAATTCAGACAACAACACCA
TCTTTGTGCAAGGCCTGGGTGAGAATGTTACAATTGAGTCTGTGGCTGATTACTTCAAG
CAGATTGGTATTATTAAGACAAACAAGAAAACGGGACAGCCCATGATTAATTTGTACACA
GACAGGGAAACTGGCAAGCTGAAGGGAGAGGCAACGGTCTCTTTTGATGACCCACCTT
CAGCTAAAGCAGCTATTGACTGGTTTGATGGTAAAGAATTCTCCGGAAATCCTATCAAG
GTCTCATTTGCTACTCGCCGGGCAGACTTTAATCGGGGTGGTGGCAATGGTCGTGGAG
GCCGAGGGCGAGGAGGACCCATGGGCCGTGGAGGCTATGGAGGTGGTGGCAGTGGT
GGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAA
TGAATGCAACCAGTGTAAGGCCCCTAAACCAGATGGCCCAGGAGGGGGACCAGGTGG
CTCTCACATGGGGGGTAACTACGGGGATGATCGTCGTGGTGGCAGAGGAGGCGATTAC
AAGGATGACGACGATAAGGGTACCGGCGCCCCCGGCTCCGCCGGCTCCGCCGCCGGC
TCCGGCGCGTGCCCGGTGCCGCTGCAGCTGCCGCCGCTGGAACGCCTGACCCTGGAT
 
GATAAAAAACCGCTGAATACCCTGATCTCTGCTACTGGTCTGTGGATGAGTCGTACCGG
AACCATTCATAAAATCAAACACCACGAGGTTAGCCGTTCGAAAATCTATATTGAGATGGC
GTGTGGCGATCATCTGGTTGTGAACAATAGCCGCTCTTCTCGTACAGCACGTGCACTGC
GTCACCACAAATATCGTAAAACCTGTAAACGTTGCCGTGTGTCCGATGAGGATCTGAAC
AAATTCCTGACAAAAGCCAATGAGGACCAAACAAGCGTGAAAGTGAAAGTCGTTAGCGC
TCCTACCCGTACTAAAAAAGCAATGCCGAAATCCGTTGCTCGTGCCCCTAAACCACTGG
AAAACACTGAAGCAGCACAGGCACAGCCGTCTGGAAGCAAATTCTCTCCGGCCATTCCT
GTTTCTACCCAGGAGTCCGTTTCTGTTCCAGCAAGTGTGAGCACCAGCATTAGCAGTAT
TAGCACCGGTGCCACCGCTAGCGCCCTGGTTAAAGGCAATACCAATCCGATTACAAGC
ATGTCTGCCCCGGTTCAAGCATCAGCTCCAGCACTGACAAAATCCCAAACCGATCGTCT
GGAGGTTCTGCTGAATCCGAAAGACGAAATCAGCCTGAATTCCGGCAAACCGTTTCGTG
AACTGGAGAGCGAACTGCTGTCACGTCGTAAAAAAGACCTGCAACAAATCTATGCCGAA
GAACGTGAGAACTATCTGGGGAAACTGGAACGTGAAATCACCCGCTTTTTCGTGGATCG
TGGCTTTCTGGAGATCAAATCCCCGATTCTGATTCCTCTGGAGTATATCGAGCGTATGG
GCATCGACAATGATACCGAACTGAGCAAACAAATTTTCCGTGTGGATAAAAACTTCTGTC
TGCGCCCTATGCTAGCACCAAATCTGGCTAACTATCTGCGCAAACTGGACCGTGCCCTG
CCTGATCCTATCAAAATCTTCGAGATCGGCCCGTGTTATCGTAAAGAGTCCGACGGTAA
AGAACATCTGGAGGAGTTTACCATGCTGGCCTTTGCCCAAATGGGTTCAGGTTGTACTC
GTGAGAACCTGGAAAGCATCATCACCGATTTTCTGAACCACCTGGGCATTGACTTCAAA
ATTGTGGGCGACAGCTGTATGGTGTTTGGCGACACCCTGGATGTCATGCACGGCGACC
TGGAACTGTCTAGTGCCGTTGTGGGCCCAATCCCGCTGGATCGTGAGTGGGGTATCGA
CAAACCTTGGATCGGTGCGGGTTTTGGTCTGGAGCGTCTGCTGAAAGTAAAACACGACT
TCAAGAACATCAAACGTGCTGCACGTTCCGAGTCCTATTACAATGGTATTTCTACTAACC
TGTAA (SEQ ID NO:81)
Protein:

MSDTKVKVAVRVRPMNRRELELNTKCVVEMEGNQTVLHPPPSNTKQGERKPPKVFAFDYC
FWSMDESNTTKYAGQEVVFKCLGEGILEKAFQGYNACIFAYGQTGSGKSFSMMGHAEQLG
LIPRLCCALFKRISLEQNESQTFKVEVSYMEIYNEKVRDLLDPKGSRQSLKVREHKVLGPYV
DGLSQLAVTSFEDIESLMSEGNKSRTVAATNMNEESSRSHAVFNIIITQTLYDLQSGNSGEKV
SKVSLVDLAGSERVSKTGAAGERLKEGSNINKSLTTLGLVISSLADQAAGKGKSKFVPYRDS
VLTWLLKDNLGGNSQTSMIATISPAADNYEETLSTLRYADRAKRIVNHAVVNEDPNAKVIREL
REEVEKLREQLSQAEAMKAELKEKLEESEKLIKELTVTWEYTDIEMNRLGKGAPGSAGSAA
GSGMASNDYTQQATQSYGAYPTQPGQGYSQQSSQPYGQQSYSGYSQSTDTSGYGQSSY
SSYGQSQNTGYGTQSTPQGYGSTGGYGSSQSSQSSYGQQSSYPGYGQQPAPSSTSGSY
GSSSQSSSYGQPQSGSYSQQPSYGGQQQSYGQQQSYNPPQGYGQQNQYNSSSGGGG
GGGGGGNYGQDQSSMSSGGGSGGGYGNQDQSGGGGSGGYGQQDRGGRGRGGSGGG

GGGGGGGYNRSSGGYEPRGRGGGRGGRGGMGGSDRGGFNKFGGPRDQGSRHDSEQD
NSDNNTIFVQGLGENVTIESVADYFKQIGIIKTNKKTGQPMINLYTDRETGKLKGEATVSFDD
PPSAKAAIDWFDGKEFSGNPIKVSFATRRADFNRGGGNGRGGRGRGGPMGRGGYGGGG
SGGGGRGGFPSGGGGGGGQQRAGDWKCPNPTCENMNFSWRNECNQCKAPKPDGPGG
GPGGSHMGGNYGDDRRGGRGGDYKDDDDKGTGAPGSAGSAAGSGACPVPLQLPPLERL
TLDDKKPLNTLISATGLWMSRTGTIHKIKHHEVSRSKIYIEMACGDHLVVNNSRSSRTARALR
HHKYRKTCKRCRVSDEDLNKFLTKANEDQTSVKVKVVSAPTRTKKAMPKSVARAPKPLENT
EAAQAQPSGSKFSPAIPVSTQESVSVPASVSTSISSISTGATASALVKGNTNPITSMSAPVQA
SAPALTKSQTDRLEVLLNPKDEISLNSGKPFRELESELLSRRKKDLQQIYAEERENYLGKLER
EITRFFVDRGFLEIKSPILIPLEYIERMGIDNDTELSKQIFRVDKNFCLRPMLAPNLANYLRKLD
RALPDPIKIFEIGPCYRKESDGKEHLEEFTMLAFAQMGSGCTRENLESIITDFLNHLGIDFKIV
GDSCMVFGDTLDVMHGDLELSSAVVGPIPLDREWGIDKPWIGAGFGLERLLKVKHDFKNIK
RAARSESYYNGISTNL (SEQ ID NO:82)

KIF13A-EWSR1-MCP
DNA: 
ATGTCGGATACCAAGGTAAAAGTTGCCGTCCGGGTCCGGCCCATGAACCGACGAGAAC
TGGAACTGAACACCAAGTGCGTGGTGGAGATGGAAGGAATCAAACGGTCCTGCACCC
TCCTCCTTCTAACACCAAACAGGGAGAAAGGAAACCTCCCAAGGTATTTGCCTTTGATTA
TTGCTTTTGGTCCATGGATGAATCTAACACTACAAAATACGCTGGTCAAGAAGTGGTTTT
CAAGTGCCTTGGGGAAGGAATTCTTGAAAAAGCCTTTCAGGGGTATAATGCGTGTATTT
TTGCATATGGACAGACAGGTTCGGGAAAATCCTTTTCCATGATGGGCCATGCTGAGCAG
CTGGGCCTTATTCCAAGGCTCTGCTGTGCTTTATTTAAAAGGATCTCTTTGGAGCAAAAT
GAGTCACAGACCTTTAAAGTTGAAGTGTCCTATATGGAAATTTATAATGAGAAAGTTCGG
GATCTTTTAGACCCCAAAGGGAGTAGACAGTCTCTTAAAGTTCGAGAACATAAAGTTTTG
GGACCATATGTAGATGGTTTATCTCAACTAGCTGTCACTAGTTTTGAGGATATTGAGTCA
TTGATGTCTGAGGGAAATAAGTCTCGAACGGTAGCTGCTACCAACATGAACGAAGAAAG
CAGCCGCTCCCATGCTGTGTTCAACATCATAATCACACAGACACTTTATGACCTGCAGT
CTGGGAATTCCGGGGAGAAAGTCAGTAAGGTCAGCTTGGTAGACCTGGCGGGTAGCGA
AAGAGTATCTAAAACAGGAGCTGCAGGAGAGCGACTGAAAGAAGGCAGCAACATTAAC
AAATCGCTTACAACCTTGGGGTTGGTTATATCATCACTGGCTGACCAGGCAGCTGGCAA
GGGTAAAAGCAAATTTGTGCCTTATCGAGATTCAGTCCTCACTTGGCTGCTTAAGGACA
ACTTGGGGGGCAACAGCCAAACCTCTATGATAGCCACAATCAGCCCAGCCGCAGACAA
CTATGAAGAGACCCTCTCCACATTAAGATATGCAGACCGAGCCAAAAGGATTGTGAACC
ATGCTGTTGTGAATGAGGACCCCAACGCAAAAGTGATCCGAGAACTGCGGGAGGAAGT
CGAGAAACTGAGAGAGCAGCTCTCTCAGGCAGAGGCCATGAAGGCCGAACTGAAGGA
 
GAAGCTCGAAGAGTCTGAAAAGCTGATAAAAGAACTAACAGTGACTTGGGAAATGGCGT
CCACGGATTACAGTACCTATAGCCAAGCTGCAGCGCAGCAGGGCTACAGTGCTTACAC
CGCCCAGCCCACTCAAGGATATGCACAGACCACCCAGGCATATGGGCAACAAAGCTAT
GGAACCTATGGACAGCCCACTGATGTCAGCTATACCCAGGCTCAGACCACTGCAACCTA
TGGGCAGACCGCCTATGCAACTTCTTATGGACAGCCTCCCACTGGTTATACTACTCCAA
CTGCCCCCCAGGCATACAGCCAGCCTGTCCAGGGGTATGGCACTGGTGCTTATGATAC
CACCACTGCTACAGTCACCACCACCCAGGCCTCCTATGCAGCTCAGTCTGCATATGGCA
CTCAGCCTGCTTATCCAGCCTATGGGCAGCAGCCAGCAGCCACTGCACCTACAAGACC
GCAGGATGGAAACAAGCCCACTGAGACTAGTCAACCTCAATCTAGCACAGGGGGTTAC
AACCAGCCCAGCCTAGGATATGGACAGAGTAACTACAGTTATCCCCAGGTACCTGGGA
GCTACCCCATGCAGCCAGTCACTGCACCTCCATCCTACCCTCCTACCAGCTATTCCTCT
ACACAGCCGACTAGTTATGATCAGAGCAGTTACTCTCAGCAGAACACCTATGGGCAACC
GAGCAGCTATGGACAGCAGAGTAGCTATGGTCAACAAAGCAGCTATGGGCAGCAGCCT
CCCACTAGTTACCCACCCCAAACTGGATCCTACAGCCAAGCTCCAAGTCAATATAGCCA
ACAGAGCAGCAGCTACGGGCAGCAGAGTTCATTCCGACAGGACCACCCCAGTAGCATG
GGTGTTTATGGGCAGGAGTCTGGAGGATTTTCCGGACCAGGAGAGAACCGGAGCATGA
GTGGCCCTGATAACCGGGGCAGGGGAAGAGGGGGATTTGATCGTGGAGGCATGAGCA
GAGGTGGGCGGGGAGGAGGACGCGGTGGAATGGGCAGCGCTGGAGAGCGAGGTGGC
TTCAATAAGCCTGGTGGACCCATGGATGAAGGACCAGATCTTGATCTAGGCCCACCTGT
AGATCCAGATGAAGACTCTGACAACAGTGCAATTTATGTACAAGGATTAAATGACAGTGT
GACTCTAGATGATCTGGCAGACTTCTTTAAGCAGTGTGGGGTTGTTAAGATGAACAAGA
GAACTGGGCAACCCATGATCCACATCTACCTGGACAAGGAAACAGGAAAGCCCAAAGG
CGATGCCACAGTGTCCTATGAAGACCCACCTACTGCCAAGGCTGCCGTGGAATGGTTT
GATGGGAAAGATTTTCAAGGGAGCAAACTTAAAGTCTCCCTTGCTCGGAAGAAGCCTCC
AATGAACAGTATGCGGGGTGGTCTGCCACCCCGTGAGGGCAGAGGCATGCCACCACC
ACTCCGTGGAGGTCCAGGAGGCCCAGGAGGTCCTGGGGGACCCATGGGTCGCATGGG
AGGCCGTGGAGGAGATAGAGGAGGCTTCCCTCCAAGAGGACCCCGGGGTTCCCGAGG
GAACCCCTCTGGAGGAGGAAACGTCCAGCACCGAGCTGGAGACTGGCAGTGTCCCAAT
CCGGGTTGTGGAAACCAGAACTTCGCCTGGAGAACAGAGTGCAACCAGTGTAAGGCCC
CAAAGCCTGAAGGCTTCCTCCCGCCACCCTTTCCGCCCCCGGGTGGTGATCGTGGCAG
AGGTGGCCCTGGTGGCATGCGGGGAGGAAGAGGTGGCCTCATGGATCGTGGTGGTCC
CGGTGGAATGTTCAGAGGTGGCCGTGGTGGAGACAGAGGTGGCTTCCGTGGTGGCCG
GGGCATGGACCGAGGTGGCTTTGGTGGAGGAAGACGAGGTGGCCCTGGGGGGCCCC
CTGGACCTTTGATGGAACAGGATTACAAGGATGACGACGATAAGGGTACCGAGCAGAA
GCTGATCTCAGAGGAGGACCTGGGCGCCCCCGGCTCCGCCGGCTCCGCCGCCGGCTC
CGGCGCTTCTAACTTTACTCAGTTCGTTCTCGTCGACAATGGCGGAACTGGCGACGTGA
 
CTGTCGCCCCAAGCAACTTCGCTAACGGGATCGCTGAATGGATCAGCTCTAACTCGCGT
TCACAGGCTTACAAAGTAACCTGTAGCGTTCGTCAGAGCTCTGCGCAGAATCGCAAATA
CACCATCAAAGTCGAGGTGCCTAAAGGCGCCTGGCGTTCGTACTTAAATATGGAACTAA
CCATTCCAATTTTCGCCACGAATTCCGACTGCGAGCTTATTGTTAAGGCAATGCAAGGT
CTCCTAAAAGATGGAAACCCGATTCCCTCAGCAATCGCAGCAAACTCCGGCATCTACTA
A (SEQ ID NO:83)
Protein:

MSDTKVKVAVRVRPMNRRELELNTKCVVEMEGNQTVLHPPPSNTKQGERKPPKVFAFDYC
FWSMDESNTTKYAGQEVVFKCLGEGILEKAFQGYNACIFAYGQTGSGKSFSMMGHAEQLG
LIPRLCCALFKRISLEQNESQTFKVEVSYMEIYNEKVRDLLDPKGSRQSLKVREHKVLGPYV
DGLSQLAVTSFEDIESLMSEGNKSRTVAATNMNEESSRSHAVFNIIITQTLYDLQSGNSGEKV
SKVSLVDLAGSERVSKTGAAGERLKEGSNINKSLTTLGLVISSLADQAAGKGKSKFVPYRDS
VLTWLLKDNLGGNSQTSMIATISPAADNYEETLSTLRYADRAKRIVNHAVVNEDPNAKVIREL
REEVEKLREQLSQAEAMKAELKEKLEESEKLIKELTVTWEMASTDYSTYSQAAAQQGYSAY
TAQPTQGYAQTTQAYGQQSYGTYGQPTDVSYTQAQTTATYGQTAYATSYGQPPTGYTTPT
APQAYSQPVQGYGTGAYDTTTATVTTTQASYAAQSAYGTQPAYPAYGQQPAATAPTRPQD
GNKPTETSQPQSSTGGYNQPSLGYGQSNYSYPQVPGSYPMQPVTAPPSYPPTSYSSTQP
TSYDQSSYSQQNTYGQPSSYGQQSSYGQQSSYGQQPPTSYPPQTGSYSQAPSQYSQQS
SSYGQQSSFRQDHPSSMGVYGQESGGFSGPGENRSMSGPDNRGRGRGGFDRGGMSRG
GRGGGRGGMGSAGERGGFNKPGGPMDEGPDLDLGPPVDPDEDSDNSAIYVQGLNDSVT
LDDLADFFKQCGVVKMNKRTGQPMIHIYLDKETGKPKGDATVSYEDPPTAKAAVEWFDGKD
FQGSKLKVSLARKKPPMNSMRGGLPPREGRGMPPPLRGGPGGPGGPGGPMGRMGGRG
GDRGGFPPRGPRGSRGNPSGGGNVQHRAGDWQCPNPGCGNQNFAWRTECNQCKAPKP
EGFLPPPFPPPGGDRGRGGPGGMRGGRGGLMDRGGPGGMFRGGRGGDRGGFRGGRG
MDRGGFGGGRRGGPGGPPGPLMEQDYKDDDDKGTEQKLISEEDLGAPGSAGSAAGSGA
SNFTQFVLVDNGGTGDVTVAPSNFANGIAEWISSNSRSQAYKVTCSVRQSSAQNRKYTIKV
EVPKGAWRSYLNMELTIPIFATNSDCELIVKAMQGLLKDGNPIPSAIAANSGIY (SEQ ID
NO:84)

KIF13A-SPD5-PyIRS^{AF}
DNA: 
ATGTCGGATACCAAGGTAAAAGTTGCCGTCCGGGTCCGGCCCATGAACCGACGAGAAC
TGGAACTGAACACCAAGTGCGTGGTGGAGATGGAAGGAATCAAACGGTCCTGCACCC
TCCTCCTTCTAACACCAAACAGGGAGAAAGGAAACCTCCCAAGGTATTTGCCTTTGATTA
TTGCTTTTGGTCCATGGATGAATCTAACACTACAAAATACGCTGGTCAAGAAGTGGTTTT
CAAGTGCCTTGGGGAAGGAATTCTTGAAAAAGCCTTTCAGGGGTATAATGCGTGTATTT
 
TTGCATATGGACAGACAGGTTCGGGAAAATCCTTTTCCATGATGGGCCATGCTGAGCAG
CTGGGCCTTATTCCAAGGCTCTGCTGTGCTTTATTTAAAAGGATCTCTTTGGAGCAAAAT
GAGTCACAGACCTTTAAAGTTGAAGTGTCCTATATGGAAATTTATAATGAGAAAGTTCGG
GATCTTTTAGACCCCAAAGGGAGTAGACAGTCTCTTAAAGTTCGAGAACATAAAGTTTTG
GGACCATATGTAGATGGTTTATCTCAACTAGCTGTCACTAGTTTTGAGGATATTGAGTCA
TTGATGTCTGAGGGAAATAAGTCTCGAACGGTAGCTGCTACCAACATGAACGAAGAAAG
CAGCCGCTCCCATGCTGTGTTCAACATCATAATCACACAGACACTTTATGACCTGCAGT
CTGGGAATTCCGGGGAGAAAGTCAGTAAGGTCAGCTTGGTAGACCTGGCGGGTAGCGA
AAGAGTATCTAAAACAGGAGCTGCAGGAGAGCGACTGAAAGAAGGCAGCAACATTAAC
AAATCGCTTACAACCTTGGGGTTGGTTATATCATCACTGGCTGACCAGGCAGCTGGCAA
GGGTAAAAGCAAATTTGTGCCTTATCGAGATTCAGTCCTCACTTGGCTGCTTAAGGACA
ACTTGGGGGGCAACAGCCAAACCTCTATGATAGCCACAATCAGCCCAGCCGCAGACAA
CTATGAAGAGACCCTCTCCACATTAAGATATGCAGACCGAGCCAAAAGGATTGTGAACC
ATGCTGTTGTGAATGAGGACCCCAACGCAAAAGTGATCCGAGAACTGCGGGAGGAAGT
CGAGAAACTGAGAGAGCAGCTCTCTCAGGCAGAGGCCATGAAGGCCGAACTGAAGGA
GAAGCTCGAAGAGTCTGAAAAGCTGATAAAAGAACTAACAGTGACTTGGGAAATGGAGG
ACAACAGCGTGCTGAACGAGGACAGCAACCTGGAGCACGTGGAGGGCCAGCCCAGAA
GAAGCATGAGCCAGCCCGTGCTGAACGTGGAGGGCGACAAGAGAACCAGCAGCACCA
GCGCCACCCAGCAGCAGGTGCTGAGCGGCGCCTTCAGCAGCGCCGACGTGAGAAGCA
TCCCCATCATCCAGACCTGGGAGGAGAACAAGGCCCTGAAGACCAAGATCACCATCCT
GAGAGGCGAGCTGCAGATGTACCAGAGAAGATACAGCGAGGCCAAGGAGGCCAGCCA
GAAGAGAGTGAAGGAGGTGATGGACGACTACGTGGACCTGAAGCTGGGCCAGGAGAA
CGTGCAGGAGAAGATGGAGCAGTACAAGCTGATGGAGGAGGACCTGCTGGCCATGCA
GAGCAGAATCGAGACCAGCGAGGACAACTTCGCCAGACAGATGAAGGAGTTCGAGGCC
CAGAAGCACGCCATGGAGGAGAGAATCAAGGAGCTGGAGCTGAGCGCCACCGACGCC
AACAACACCACCGTGGGCAGCTTCAGAGGCACCCTGGACGACATCCTGAAGAAGAACG
ACCCCGACTTCACCCTGACCAGCGGCTACGAGGAGAGAAAGATCAACGACCTGGAGGC
CAAGCTGCTGAGCGAGATCGACAAGGTGGCCGAGCTGGAGGACCACATCCAGCAGCT
GAGACAGGAGCTGGACGACCAGAGCGCCAGACTGGCCGACAGCGAGAACGTGAGAGC
CCAGCTGGAGGCCGCCACCGGCCAGGGCATCCTGGGCGCCGCCGGCAACGCCATGG
TGCCCAACAGCACCTTCATGATCGGCAACGGCAGAGAGAGCCAGACCAGAGACCAGCT
GAACTACATCGACGACCTGGAGACCAAGCTGGCCGACGCCAAGAAGGAGAACGACAAG
GCCAGACAGGCCCTGGTGGAGTACATGAACAAGTGCAGCAAGCTGGAGCACGAGATCA
GAACCATGGTGAAGAACAGCACCTTCGACAGCAGCAGCATGCTGCTGGGCGGCCAGAC
CAGCGACGAGCTGAAGATCCAGATCGGCAAGGTGAACGGCGAGCTGAACGTGCTGAG
AGCCGAGAACAGAGAGCTGAGAATCAGATGCGACCAGCTGACCGGCGGCGACGGCAA
 
CCTGAGCATCAGCCTGGGCCAGAGCAGACTGATGGCCGGCATCGCCACCAACGACGT
GGACAGCATCGGCCAGGGCAACGAGACCGGCGGCACCAGCATGAGAATCCTGCCCAG
AGAGAGCCAGCTGGACGACCTGGAGGAGAGCAAGCTGCCCCTGATGGACACCAGCAG
CGCCGTGAGAAACCAGCAGCAGTTCGCCAGCATGTGGAGGACTTCGAGAGCGTGAA
GGACAGCCTGCAGAACAACCACAACGACACCCTGGAGGGCAGCTTCAACAGCAGCATG
CCCCCCCCCGGCAGAGACGCCACCCAGAGCTTCCTGAGCCAGAAGAGCTTCAAGAACA
GCCCCATCGTGATGCAGAAGCCCAAGAGCCTGCACCTGCACCTGAAGAGCCACCAGAG
CGAGGGCGCCGGCGAGCAGATCCAGAACAACAGCTTCAGCACCAAGACCGCCAGCCC
CCACGTGAGCCAGAGCCACATCCCCATCCTGCACGACATGCAGCAGATCCTGGACAGC
AGCGCCATGTTCCTGGAGGGCCAGCACGACGTGGCCGTGAACGTGGAGCAGATGCAG
GAGAAGATGAGCCAGATCAGAGAGGCCCTGGCCAGACTGTTCGAGAGACTGAAGAGCA
GCGCCGCCCTGTTCGAGGAGATCCTGGAGAGAATGGGCAGCAGCGACCCCAACGCCG
ACAAGATCAAGAAGATGAAGCTGGCCTTCGAGACCAGCATCAACGACAAGCTGAACGT
GAGCGCCATCCTGGAGGCCGCCGAGAAGGACCTGCACAACATGAGCCTGAACTTCAGC
ATCCTGGAGAAGAGCATCGTGAGCCAGGCCGCCGAGGCCAGCAGAAGATTCACCATCG
CCCCCGACGCCGAGGACGTGGCCAGCAGCAGCCTGCTGAACGCCAGCTACAGCCCCC
TGTTCAAGTTCACCAGCAACAGCGACATCGTGGAGAAGCTGCAGAACGAGGTGAGCGA
GCTGAAGAACGAGCTGGAGATGGCCAGAACCAGAGACATGAGAAGCCCCCTGAACGG
CAGCAGCGGCAGACTGAGCGACGTGCAGATCAACACCAACAGAATGTTCGAGGACCTG
GAGGTGAGCGAGGCCACCCTGCAGAAGGCCAAGGAGGAGAACAGCACCCTGAAGAGC
CAGTTCGCCGAGCTGGAGGCCAACCTGCACCAGGTGAACAGCAAGCTGGGCGAGGTG
AGATGCGAGCTGAACGAGGCCCTGGCCAGAGTGGACGGCGAGCAGGAGACCAGAGTG
AAGGCCGAGAACGCCCTGGAGGAGGCCAGACAGCTGATCAGCAGCCTGAAGCACGAG
GAGAACGAGCTGAAGAAGACCATCACCGACATGGGCATGAGACTGAACGAGGCCAAGA
AGAGCGACGAGTTCCTGAAGAGCGAGCTGAGCACCGCCCTGGAGGAGGAGAAGAAGA
GCCAGAACCTGGCCGACGAGCTGAGCGAGGAGCTGAACGGCTGGAGAATGAGAACCA
AGGAGGCCGAGAACAAGGTGGAGCACGCCAGCAGCGAGAAGAGCGAGATGCTGGAGA
GAATCGTGCACCTGGAGACCGAGATGGAGAAGCTGAGCACCAGCGAGATCGCCGCCG
ACTACTGCAGCACCAAGATGACCGAGAGAAAGAAGGAGATCGAGCTGGCCAAGTACAG
AGAGGACTTCGAGAACGCCGCCATCGTGGGCCTGGAGAGAATCAGCAAGGAGATCAG
CGAGCTGACCAAGAAGACCCTGAAGGCCAAGATCATCCCCAGCAACATCAGCAGCATC
CAGCTGGTGTGCGACGAGCTGTGCAGAAGACTGAGCAGAGAGAGAGAGCAGCAGCAC
GAGTACGCCAAGGTGATGAGAGACGTGAACGAGAAGATCGAGAAGCTGCAGCTGGAGA
AGGACGCCCTGGAGCACGAGCTGAAGATGATGAGCAGCAACAACGAGAACGTGCCCC
CCGTGGGCACCAGCGTGAGCGGCATGCCCACCAAGACCAGCAACCAGAAGTGCGCCC
AGCCCCACTACACCAGCCCCACCAGACAGCTGCTGCACGAGAGCACCATGGCCGTGG
 
ACGCCATCGTGCAGAAGCTGAAGAAGACCCACAACATGAGCGGCATGGGCCCGAGCT
GAAGGAGACCATCGGCAACGTGATCAACGAGAGCAGAGTGCTGAGAGACTTCCTGCAC
CAGAAGCTGATCCTGTTCAAGGGCATCGACATGAGCAACTGGAAGAACGAGACCGTGG
ACCAGCTGATCACCGACCTGGGCCAGCTGCACCAGGACAACCTGATGCTGGAGGAGCA
GATCAAGAAGTACAAGAAGGAGCTGAAGCTGACCAAGAGCGCCATCCCCACCCTGGGC
GTGGAGTTCCAGGACAGAATCAAGACCGAGATCGGCAAGATCGCCACCGACATGGGCG
GCGCCGTGAAGGAGATCAGAAAGAAGGGTACCGGCGCCCCCGGCTCCGCCGGCTCCG
CCGCCGGCTCCGGCGCGTGCCCGGTGCCGCTGCAGCTGCCGCCGCTGGAACGCCTG
ACCCTGGATGATAAAAAACCGCTGAATACCCTGATCTCTGCTACTGGTCTGTGGATGAG
TCGTACCGGAACCATTCATAAAATCAAACACCACGAGGTTAGCCGTTCGAAAATCTATAT
TGAGATGGCGTGTGGCGATCATCTGGTTGTGAACAATAGCCGCTCTTCTCGTACAGCAC
GTGCACTGCGTCACCACAAATATCGTAAAACCTGTAAACGTTGCCGTGTGTCCGATGAG
GATCTGAACAAATTCCTGACAAAAGCCAATGAGGACCAAACAAGCGTGAAAGTGAAAGT
CGTTAGCGCTCCTACCCGTACTAAAAAGCAATGCCGAAATCCGTTGCTCGTGCCCCTA
AACCACTGGAAAACACTGAAGCAGCACAGGCACAGCCGTCTGGAAGCAAATTCTCTCC
GGCCATTCCTGTTTCTACCCAGGAGTCCGTTTCTGTTCCAGCAAGTGTGAGCACCAGCA
TTAGCAGTATTAGCACCGGTGCCACCGCTAGCGCCCTGGTTAAAGGCAATACCAATCCG
ATTACAAGCATGTCTGCCCCGGTTCAAGCATCAGCTCCAGCACTGACAAAATCCCAAAC
CGATCGTCTGGAGGTTCTGCTGAATCCGAAAGACGAAATCAGCCTGAATTCCGGCAAAC
CGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTT
CGTGGATCGTGGCTTTCTGGAGATCAAATCCCCGATTCTGATTCCTCTGGAGTATATCG
AGCGTATGGGCATCGACAATGATACCGAACTGAGCAAACAAATTTTCCGTGTGGATAAA
AACTTCTGTCTGCGCCCTATGCTAGCACCAAATCTGGCTAACTATCTGCGCAAACTGGA
CCGTGCCCTGCCTGATCCTATCAAAATCTTCGAGATCGGCCCGTGTTATCGTAAAGAGT
CCGACGGTAAAGAACATCTGGAGGAGTTTACCATGCTGAACTTTTGCCAAATGGGTTCA
GGTTGTACTCGTGAGAACCTGGAAAGCATCATCACCGATTTTCTGAACCACCTGGGCAT
TGACTTCAAAATTGTGGGCGACAGCTGTATGGTGTTTGGCGACACCCTGGATGTCATGC
ACGGCGACCTGGAACTGTCTAGTGCCGTTGTGGGCCCAATCCCGCTGGATCGTGAGTG
GGGTATCGACAAACCTTGGATCGGTGCGGGTTTTGGTCTGGAGCGTCTGCTGAAAGTA
AAACACGACTTCAAGAACATCAAACGTGCTGCACGTTCCGAGTCCTATTACAATGGTATT
TCTACTAACCTGTAA (SEQ ID NO:85)
Protein:

MSDTKVKVAVRVRPMNRRELELNTKCVVEMEGNQTVLHPPPSNTKQGERKPPKVFAFDYC
FWSMDESNTTKYAGQEVVFKCLGEGILEKAFQGYNACIFAYGQTGSGKSFSMMGHAEQLG
LIPRLCCALFKRISLEQNESQTFKVEVSYMEIYNEKVRDLLDPKGSRQSLKVREHKVLGPYV

DGLSQLAVTSFEDIESLMSEGNKSRTVAATNMNEESSRSHAVFNIIITQTLYDLQSGNSGEKV
SKVSLVDLAGSERVSKTGAAGERLKEGSNINKSLTTLGLVISSLADQAAGKGKSKFVPYRDS
VLTWLLKDNLGGNSQTSMIATISPAADNYEETLSTLRYADRAKRIVNHAVVNEDPNAKVIREL
REEVEKLREQLSQAEAMKAELKEKLEESEKLIKELTVTWEMEDNSVLNEDSNLEHVEGQPR
RSMSQPVLNVEGDKRTSSTSATQQQVLSGAFSSADVRSIPIIQTWEENKALKTKITILRGELQ
MYQRRYSEAKEASQKRVKEVMDDYVDLKLGQENVQEKMEQYKLMEEDLLAMQSRIETSED
NFARQMKEFEAQKHAMEERIKELELSATDANNTTVGSFRGTLDDILKKNDPDFTLTSGYEER
KINDLEAKLLSEIDKVAELEDHIQQLRQELDDQSARLADSENVRAQLEAATGQGILGAAGNA
MVPNSTFMIGNGRESQTRDQLNYIDDLETKLADAKKENDKARQALVEYMNKCSKLEHEIRT
MVKNSTFDSSSMLLGGQTSDELKIQIGKVNGELNVLRAENRELRIRCDQLTGGDGNLSISLG
QSRLMAGIATNDVDSIGQGNETGGTSMRILPRESQLDDLEESKLPLMDTSSAVRNQQQFAS
MWEDFESVKDSLQNNHNDTLEGSFNSSMPPPGRDATQSFLSQKSFKNSPIVMQKPKSLHL
HLKSHQSEGAGEQIQNNSFSTKTASPHVSQSHIPILHDMQQILDSSAMFLEGQHDVAVNVE
QMQEKMSQIREALARLFERLKSSAALFEEILERMGSSDPNADKIKKMKLAFETSINDKLNVSA
ILEAAEKDLHNMSLNFSILEKSIVSQAAEASRRFTIAPDAEDVASSSLLNASYSPLFKFTSNSDI
VEKLQNEVSELKNELEMARTRDMRSPLNGSSGRLSDVQINTNRMFEDLEVSEATLQKAKEE
NSTLKSQFAELEANLHQVNSKLGEVRCELNEALARVDGEQETRVKAENALEEARQLISSLKH
EENELKKTITDMGMRLNEAKKSDEFLKSELSTALEEEKKSQNLADELSEELNGWRMRTKEA
ENKVEHASSEKSEMLERIVHLETEMEKLSTSEIAADYCSTKMTERKKEIELAKYREDFENAAI
VGLERISKEISELTKKTLKAKIIPSNISSIQLVCDELCRRLSREREQQHEYAKVMRDVNEKIEKL
QLEKDALEHELKMMSSNNENVPPVGTSVSGMPTKTSNQKCAQPHYTSPTRQLLHESTMAV
DAIVQKLKKTHNMSGMGPELKETIGNVINESRVLRDFLHQKLILFKGIDMSNWKNETVDQLIT
DLGQLHQDNLMLEEQIKKYKKELKLTKSAIPTLGVEFQDRIKTEIGKIATDMGGAVKEIRKKGT
GAPGSAGSAAGSGACPVPLQLPPLERLTLDDKKPLNTLISATGLWMSRTGTIHKIKHHEVSR
SKIYIEMACGDHLVVNNSRSSRTARALRHHKYRKTCKRCRVSDEDLNKFLTKANEDQTSVK
VKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFREL
ESELLSRRKKDLQQIYAEERENYLGKLEREITRFFVDRGFLEIKSPILIPLEYIERMGIDNDTEL
SKQIFRVDKNFCLRPMLAPNLANYLRKLDRALPDPIKIFEIGPCYRKESDGKEHLEEFTMLNF
CQMGSGCTRENLESIITDFLNHLGIDFKIVGDSCMVFGDTLDVMHGDLELSSAVVGPIPLDRE
WGIDKPWIGAGFGLERLLKVKHDFKNIKRAARSESYYNGISTNL (SEQ ID NO:86)

KIF13A-SPD5-MCP
DNA: 
ATGTCGGATACCAAGGTAAAAGTTGCCGTCCGGGTCCGGCCCATGAACCGACGAGAAC
TGGAACTGAACACCAAGTGCGTGGTGGAGATGGAAGGGAATCAAACGGTCCTGCACCC
 
TCCTCCTTCTAACACCAAACAGGGAGAAAGGAAACCTCCCAAGGTATTTGCCTTTGATTA
TTGCTTTTGGTCCATGGATGAATCTAACACTACAAAATACGCTGGTCAAGAAGTGGTTTT
CAAGTGCCTTGGGGAAGGAATTCTTGAAAAAGCCTTTCAGGGGTATAATGCGTGTATTT
TTGCATATGGACAGACAGGTTCGGGAAAATCCTTTTCCATGATGGGCCATGCTGAGCAG
CTGGGCCTTATTCCAAGGCTCTGCTGTGCTTTATTTAAAAGGATCTCTTTGGAGCAAAAT
GAGTCACAGACCTTTAAAGTTGAAGTGTCCTATATGGAAATTTATAATGAGAAAGTTCGG
GATCTTTTAGACCCCAAAGGGAGTAGACAGTCTCTTAAAGTTCGAGAACATAAAGTTTTG
GGACCATATGTAGATGGTTTATCTCAACTAGCTGTCACTAGTTTTGAGGATATTGAGTCA
TTGATGTCTGAGGGAAATAAGTCTCGAACGGTAGCTGCTACCAACATGAACGAAGAAAG
CAGCCGCTCCCATGCTGTGTTCAACATCATAATCACACAGACACTTTATGACCTGCAGT
CTGGGAATTCCGGGGAGAAAGTCAGTAAGGTCAGCTTGGTAGACCTGGCGGGTAGCGA
AAGAGTATCTAAAACAGGAGCTGCAGGAGAGCGACTGAAAGAAGGCAGCAACATTAAC
AAATCGCTTACAACCTTGGGGTTGGTTATATCATCACTGGCTGACCAGGCAGCTGGCAA
GGGTAAAAGCAAATTTGTGCCTTATCGAGATTCAGTCCTCACTTGGCTGCTTAAGGACA
ACTTGGGGGGCAACAGCCAAACCTCTATGATAGCCACAATCAGCCCAGCCGCAGACAA
CTATGAAGAGACCCTCTCCACATTAAGATATGCAGACCGAGCCAAAAGGATTGTGAACC
ATGCTGTTGTGAATGAGGACCCCAACGCAAAAGTGATCCGAGAACTGCGGGAGGAAGT
CGAGAAACTGAGAGAGCAGCTCTCTCAGGCAGAGGCCATGAAGGCCGAACTGAAGGA
GAAGCTCGAAGAGTCTGAAAAGCTGATAAAAGAACTAACAGTGACTTGGGAAATGGAGG
ACAACAGCGTGCTGAACGAGGACAGCAACCTGGAGCACGTGGAGGGCCAGCCCAGAA
GAAGCATGAGCCAGCCCGTGCTGAACGTGGAGGGCGACAAGAGAACCAGCAGCACCA
GCGCCACCCAGCAGCAGGTGCTGAGCGGCGCCTTCAGCAGCGCCGACGTGAGAAGCA
TCCCCATCATCCAGACCTGGGAGGAGAACAAGGCCCTGAAGACCAAGATCACCATCCT
GAGAGGCGAGCTGCAGATGTACCAGAGAAGATACAGCGAGGCCAAGGAGGCCAGCCA
GAAGAGAGTGAAGGAGGTGATGGACGACTACGTGGACCTGAAGCTGGGCCAGGAGAA
CGTGCAGGAGAAGATGGAGCAGTACAAGCTGATGGAGGAGGACCTGCTGGCCATGCA
GAGCAGAATCGAGACCAGCGAGGACAACTTCGCCAGACAGATGAAGGAGTTCGAGGCC
CAGAAGCACGCCATGGAGGAGAGAATCAAGGAGCTGGAGCTGAGCGCCACCGACGCC
AACAACACCACCGTGGGCAGCTTCAGAGGCACCCTGGACGACATCCTGAAGAAGAACG
ACCCCGACTTCACCCTGACCAGCGGCTACGAGGAGAGAAAGATCAACGACCTGGAGGC
CAAGCTGCTGAGCGAGATCGACAAGGTGGCCGAGCTGGAGGACCACATCCAGCAGCT
GAGACAGGAGCTGGACGACCAGAGCGCCAGACTGGCCGACAGCGAGAACGTGAGAGC
CCAGCTGGAGGCCGCCACCGGCCAGGGCATCCTGGGCGCCGCCGGCAACGCCATGG
TGCCCAACAGCACCTTCATGATCGGCAACGGCAGAGAGAGCCAGACCAGAGACCAGCT
GAACTACATCGACGACCTGGAGACCAAGCTGGCCGACGCCAAGAAGGAGAACGACAAG
GCCAGACAGGCCCTGGTGGAGTACATGAACAAGTGCAGCAAGCTGGAGCACGAGATCA
 
GAACCATGGTGAAGAACAGCACCTTCGACAGCAGCAGCATGCTGCTGGGCGGCCAGAC
CAGCGACGAGCTGAAGATCCAGATCGGCAAGGTGAACGGCGAGCTGAACGTGCTGAG
AGCCGAGAACAGAGAGCTGAGAATCAGATGCGACCAGCTGACCGGCGGCGACGGCAA
CCTGAGCATCAGCCTGGGCCAGAGCAGACTGATGGCCGGCATCGCCACCAACGACGT
GGACAGCATCGGCCAGGGCAACGAGACCGGCGGCACCAGCATGAGAATCCTGCCCAG
AGAGAGCCAGCTGGACGACCTGGAGGAGCAAGCTGCCCCTGATGGACACCAGCAG
CGCCGTGAGAAACCAGCAGCAGTTCGCCAGCATGTGGAGGACTTCGAGAGCGTGAA
GGACAGCCTGCAGAACAACCACAACGACACCCTGGAGGGCAGCTTCAACAGCAGCATG
CCCCCCCCCGGCAGAGACGCCACCCAGAGCTTCCTGAGCCAGAAGAGCTTCAAGAACA
GCCCCATCGTGATGCAGAAGCCCAAGAGCCTGCACCTGCACCTGAAGAGCCACCAGAG
CGAGGGCGCCGGCGAGCAGATCCAGAACAACAGCTTCAGCACCAAGACCGCCAGCCC
CCACGTGAGCCAGAGCCACATCCCCATCCTGCACGACATGCAGCAGATCCTGGACAGC
AGCGCCATGTTCCTGGAGGGCCAGCACGACGTGGCCGTGAACGTGGAGCAGATGCAG
GAGAAGATGAGCCAGATCAGAGAGGCCCTGGCCAGACTGTTCGAGAGACTGAAGAGCA
GCGCCGCCCTGTTCGAGGAGATCCTGGAGAGAATGGGCAGCAGCGACCCCAACGCCG
ACAAGATCAAGAAGATGAAGCTGGCCTTCGAGACCAGCATCAACGACAAGCTGAACGT
GAGCGCCATCCTGGAGGCCGCCGAGAAGGACCTGCACAACATGAGCCTGAACTTCAGC
ATCCTGGAGAAGAGCATCGTGAGCCAGGCCGCCGAGGCCAGCAGAAGATTCACCATCG
CCCCCGACGCCGAGGACGTGGCCAGCAGCAGCCTGCTGAACGCCAGCTACAGCCCCC
TGTTCAAGTTCACCAGCAACAGCGACATCGTGGAGAAGCTGCAGAACGAGGTGAGCGA
GCTGAAGAACGAGCTGGAGATGGCCAGAACCAGAGACATGAGAAGCCCCTGAACGG
CAGCAGCGGCAGACTGAGCGACGTGCAGATCAACACCAACAGAATGTTCGAGGACCTG
GAGGTGAGCGAGGCCACCCTGCAGAAGGCCAAGGAGGAGAACAGCACCCTGAAGAGC
CAGTTCGCCGAGCTGGAGGCCAACCTGCACCAGGTGAACAGCAAGCTGGGCGAGGTG
AGATGCGAGCTGAACGAGGCCCTGGCCAGAGTGGACGGCGAGCAGGAGACCAGAGTG
AAGGCCGAGAACGCCCTGGAGGAGGCCAGACAGCTGATCAGCAGCCTGAAGCACGAG
GAGAACGAGCTGAAGAAGACCATCACCGACATGGGCATGAGACTGAACGAGGCCAAGA
AGAGCGACGAGTTCCTGAAGAGCGAGCTGAGCACCGCCCTGGAGGAGGAGAAGAAGA
GCCAGAACCTGGCCGACGAGCTGAGCGAGGAGCTGAACGGCTGGAGAATGAGAACCA
AGGAGGCCGAGAACAAGGTGGAGCACGCCAGCAGCGAGAAGAGCGAGATGCTGGAGA
GAATCGTGCACCTGGAGACCGAGATGGAGAAGCTGAGCACCAGCGAGATCGCCGCCG
ACTACTGCAGCACCAAGATGACCGAGAGAAAGAAGGAGATCGAGCTGGCCAAGTACAG
AGAGGACTTCGAGAACGCCGCCATCGTGGGCCTGGAGAGAATCAGCAAGGAGATCAG
CGAGCTGACCAAGAAGACCCTGAAGGCCAAGATCATCCCCAGCAACATCAGCAGCATC
CAGCTGGTGTGCGACGAGCTGTGCAGAAGACTGAGCAGAGAGAGAGAGCAGCAGCAC
GAGTACGCCAAGGTGATGAGAGACGTGAACGAGAAGATCGAGAAGCTGCAGCTGGAGA
 
AGGACGCCCTGGAGCACGAGCTGAAGATGATGAGCAGCAACAACGAGAACGTGCCCC
CCGTGGGCACCAGCGTGAGCGGCATGCCCACCAAGACCAGCAACCAGAAGTGCGCCC
AGCCCCACTACACCAGCCCCACCAGACAGCTGCTGCACGAGAGCACCATGGCCGTGG
ACGCCATCGTGCAGAAGCTGAAGAAGACCCACAACATGAGCGGCATGGCCCCGAGCT
GAAGGAGACCATCGGCAACGTGATCAACGAGAGCAGAGTGCTGAGAGACTTCCTGCAC
CAGAAGCTGATCCTGTTCAAGGGCATCGACATGAGCAACTGGAAGAACGAGACCGTGG
ACCAGCTGATCACCGACCTGGGCCAGCTGCACCAGGACAACCTGATGCTGGAGGAGCA
GATCAAGAAGTACAAGAAGGAGCTGAAGCTGACCAAGAGCGCCATCCCCACCCTGGGC
GTGGAGTTCCAGGACAGAATCAAGACCGAGATCGGCAAGATCGCCACCGACATGGGCG
GCGCCGTGAAGGAGATCAGAAAGAAGGGTACCGAGCAGAAGCTGATCTCAGAGGAGG
ACCTGGGCGCCCCCGGCTCCGCCGGCTCCGCCGCCGGCTCCGGCGCTTCTAACTTTA
CTCAGTTCGTTCTCGTCGACAATGGCGGAACTGGCGACGTGACTGTCGCCCCAAGCAA
CTTCGCTAACGGGATCGCTGAATGGATCAGCTCTAACTCGCGTTCACAGGCTTACAAAG
TAACCTGTAGCGTTCGTCAGAGCTCTGCGCAGAATCGCAAATACACCATCAAAGTCGAG
GTGCCTAAAGGCGCCTGGCGTTCGTACTTAAATATGGAACTAACCATTCCAATTTTCGC
CACGAATTCCGACTGCGAGCTTATTGTTAAGGCAATGCAAGGTCTCCTAAAAGATGGAA
ACCCGATTCCCTCAGCAATCGCAGCAAACTCCGGCATCTACTAA (SEQ ID NO:87)
Protein:

MSDTKVKVAVRVRPMNRRELELNTKCVVEMEGNQTVLHPPPSNTKQGERKPPKVFAFDYC
FWSMDESNTTKYAGQEVVFKCLGEGILEKAFQGYNACIFAYGQTGSGKSFSMMGHAEQLG
LIPRLCCALFKRISLEQNESQTFKVEVSYMEIYNEKVRDLLDPKGSRQSLKVREHKVLGPYV
DGLSQLAVTSFEDIESLMSEGNKSRTVAATNMNEESSRSHAVFNIIITQTLYDLQSGNSGEKV
SKVSLVDLAGSERVSKTGAAGERLKEGSNINKSLTTLGLVISSLADQAAGKGKSKFVPYRDS
VLTWLLKDNLGGNSQTSMIATISPAADNYEETLSTLRYADRAKRIVNHAVVNEDPNAKVIREL
REEVEKLREQLSQAEAMKAELKEKLEESEKLIKELTVTWEMEDNSVLNEDSNLEHVEGQPR
RSMSQPVLNVEGDKRTSSTSATQQQVLSGAFSSADVRSIPIIQTWEENKALKTKITILRGELQ
MYQRRYSEAKEASQKRVKEVMDDYVDLKLGQENVQEKMEQYKLMEEDLLAMQSRIETSED
NFARQMKEFEAQKHAMEERIKELELSATDANNTTVGSFRGTLDDILKKNDPDFTLTSGYEER
KINDLEAKLLSEIDKVAELEDHIQQLRQELDDQSARLADSENVRAQLEAATGQGILGAAGNA
MVPNSTFMIGNGRESQTRDQLNYIDDLETKLADAKKENDKARQALVEYMNKCSKLEHEIRT
MVKNSTFDSSSMLLGGQTSDELKIQIGKVNGELNVLRAENRELRIRCDQLTGGDGNLSISLG
QSRLMAGIATNDVDSIGQGNETGGTSMRILPRESQLDDLEESKLPLMDTSSAVRNQQQFAS
MWEDFESVKDSLQNNHNDTLEGSFNSSMPPPGRDATQSFLSQKSFKNSPIVMQKPKSLHL
HLKSHQSEGAGEQIQNNSFSTKTASPHVSQSHIPILHDMQQILDSSAMFLEGQHDVAVNVE
QMQEKMSQIREALARLFERLKSSAALFEEILERMGSSDPNADKIKKMKLAFETSINDKLNVSA
ILEAAEKDLHNMSLNFSILEKSIVSQAAEASRRFTIAPDAEDVASSSLLNASYSPLFKFTSNSDI

VEKLQNEVSELKNELEMARTRDMRSPLNGSSGRLSDVQINTNRMFEDLEVSEATLQKAKEE
NSTLKSQFAELEANLHQVNSKLGEVRCELNEALARVDGEQETRVKAENALEEARQLISSLKH
EENELKKTITDMGMRLNEAKKSDEFLKSELSTALEEEKKSQNLADELSEELNGWRMRTKEA
ENKVEHASSEKSEMLERIVHLETEMEKLSTSEIAADYCSTKMTERKKEIELAKYREDFENAAI
VGLERISKEISELTKKTLKAKIIPSNISSIQLVCDELCRRLSREREQQHEYAKVMRDVNEKIEKL
QLEKDALEHELKMMSSNNENVPPVGTSVSGMPTKTSNQKCAQPHYTSPTRQLLHESTMAV
DAIVQKLKKTHNMSGMGPELKETIGNVINESRVLRDFLHQKLILFKGIDMSNWKNETVDQLIT
DLGQLHQDNLMLEEQIKKYKKELKLTKSAIPTLGVEFQDRIKTEIGKIATDMGGAVKEIRKKGT
EQKLISEEDLGAPGSAGSAAGSGASNFTQFVLVDNGGTGDVTVAPSNFANGIAEWISSNSR
SQAYKVTCSVRQSSAQNRKYTIKVEVPKGAWRSYLNMELTIPIFATNSDCELIVKAMQGLLK
DGNPIPSAIAANSGIY (SEQ ID NO:88)

KIF13A-FUS-MCP-PyIRS^{AF}
DNA: 
ATGTCGGATACCAAGGTAAAAGTTGCCGTCCGGGTCCGGCCCATGAACCGACGAGAAC
TGGAACTGAACACCAAGTGCGTGGTGGAGATGGAAGGAATCAAACGGTCCTGCACCC
TCCTCCTTCTAACACCAAACAGGGAGAAAGGAAACCTCCCAAGGTATTTGCCTTTGATTA
TTGCTTTTGGTCCATGGATGAATCTAACACTACAAAATACGCTGGTCAAGAAGTGGTTTT
CAAGTGCCTTGGGGAAGGAATTCTTGAAAAAGCCTTTCAGGGGTATAATGCGTGTATTT
TTGCATATGGACAGACAGGTTCGGGAAAATCCTTTTCCATGATGGGCCATGCTGAGCAG
CTGGGCCTTATTCCAAGGCTCTGCTGTGCTTTATTTAAAAGGATCTCTTTGGAGCAAAAT
GAGTCACAGACCTTTAAAGTTGAAGTGTCCTATATGGAAATTTATAATGAGAAAGTTCGG
GATCTTTTAGACCCCAAAGGGAGTAGACAGTCTCTTAAAGTTCGAGAACATAAAGTTTTG
GGACCATATGTAGATGGTTTATCTCAACTAGCTGTCACTAGTTTTGAGGATATTGAGTCA
TTGATGTCTGAGGGAAATAAGTCTCGAACGGTAGCTGCTACCAACATGAACGAAGAAAG
CAGCCGCTCCCATGCTGTGTTCAACATCATAATCACACAGACACTTTATGACCTGCAGT
CTGGGAATTCCGGGGAGAAAGTCAGTAAGGTCAGCTTGGTAGACCTGGCGGGTAGCGA
AAGAGTATCTAAAACAGGAGCTGCAGGAGAGCGACTGAAAGAAGGCAGCAACATTAAC
AAATCGCTTACAACCTTGGGGTTGGTTATATCATCACTGGCTGACCAGGCAGCTGGCAA
GGGTAAAAGCAAATTTGTGCCTTATCGAGATTCAGTCCTCACTTGGCTGCTTAAGGACA
ACTTGGGGGGCAACAGCCAAACCTCTATGATAGCCACAATCAGCCCAGCCGCAGACAA
CTATGAAGAGACCCTCTCCACATTAAGATATGCAGACCGAGCCAAAAGGATTGTGAACC
ATGCTGTTGTGAATGAGGACCCCAACGCAAAAGTGATCCGAGAACTGCGGGAGGAAGT
CGAGAAACTGAGAGAGCAGCTCTCTCAGGCAGAGGCCATGAAGGCCGAACTGAAGGA
GAAGCTCGAAGAGTCTGAAAAGCTGATAAAAGAACTAACAGTGACTTGGGAATATACAG
ATATTGAAATGAACAGATTGGGAAAGGGCGCCCCCGGCTCCGCCGGCTCCGCCGCCG
 
GCTCCGGCATGGCCTCAAACGATTATACCCAACAAGCAACCCAAAGCTATGGGCCTA
CCCCACCCAGCCCGGGCAGGGCTATTCCCAGCAGAGCAGTCAGCCCTACGGACAGCA
GAGTTACAGTGGTTATAGCCAGTCCACGGACACTTCAGGATATGGCCAGAGCAGCTATT
CTTCTTATGGCCAGAGCCAGAACACAGGCTATGGAACTCAGTCAACTCCCCAGGGATAT
GGCTCGACTGGCGGCTATGGCAGTAGCCAGAGCTCCCAATCGTCTTACGGGCAGCAGT
CCTCCTACCCTGGCTATGGCCAGCAGCCAGCTCCCAGCAGCACCTCGGGAAGTTACGG
TAGCAGTTCTCAGAGCAGCAGCTATGGGCAGCCCCAGAGTGGGAGCTACAGCCAGCAG
CCTAGCTATGGTGGACAGCAGCAAAGCTATGGACAGCAGCAAAGCTATAATCCCCCTCA
GGGCTATGGACAGCAGAACCAGTACAACAGCAGCAGTGGTGGTGGAGGTGGAGGTGG
AGGTGGAGGTAACTATGGCCAAGATCAATCCTCCATGAGTAGTGGTGGTGGCAGTGGT
GGCGGTTATGGCAATCAAGACCAGAGTGGTGGAGGTGGCAGCGGTGGCTATGGACAG
CAGGACCGTGGAGGCCGCGGCAGGGGTGGCAGTGGTGGCGGCGGCGGCGGCGGCG
GTGGTGGTTACAACCGCAGCAGTGGTGGCTATGAACCCAGAGGTCGTGGAGGTGGCC
GTGGAGGCAGAGGTGGCATGGGCGGAAGTGACCGTGGTGGCTTCAATAAATTTGGTGG
CCCTCGGGACCAAGGATCACGTCATGACTCCGAACAGGATAATTCAGACAACAACACCA
TCTTTGTGCAAGGCCTGGGTGAGAATGTTACAATTGAGTCTGTGGCTGATTACTTCAAG
CAGATTGGTATTATTAAGACAAACAAGAAAACGGACAGCCCATGATTAATTTGTACACA
GACAGGGAAACTGGCAAGCTGAAGGGAGAGGCAACGGTCTCTTTTGATGACCCACCTT
CAGCTAAAGCAGCTATTGACTGGTTTGATGGTAAAGAATTCTCCGGAAATCCTATCAAG
GTCTCATTTGCTACTCGCCGGGCAGACTTTAATCGGGGTGGTGGCAATGGTCGTGGAG
GCCGAGGGCGAGGAGGACCCATGGGCCGTGGAGGCTATGGAGGTGGTGGCAGTGGT
GGTGGTGGCCGAGGAGGATTTCCCAGTGGAGGTGGTGGCGGTGGAGGACAGCAGCGA
GCTGGTGACTGGAAGTGTCCTAATCCCACCTGTGAGAATATGAACTTCTCTTGGAGGAA
TGAATGCAACCAGTGTAAGGCCCCTAAACCAGATGGCCCAGGAGGGGGACCAGGTGG
CTCTCACATGGGGGGTAACTACGGGGATGATCGTCGTGGTGGCAGAGGAGGCGATTAC
AAGGATGACGACGATAAGGGTACCGGCGCCCCCGGCTCCGCCGGCTCCGCCGCCGGC
TCCGGCGCTTCTAACTTTACTCAGTTCGTTCTCGTCGACAATGGCGGAACTGGCGACGT
GACTGTCGCCCCAAGCAACTTCGCTAACGGGATCGCTGAATGGATCAGCTCTAACTCG
CGTTCACAGGCTTACAAAGTAACCTGTAGCGTTCGTCAGAGCTCTGCGCAGAATCGCAA
ATACACCATCAAAGTCGAGGTGCCTAAAGGCGCCTGGCGTTCGTACTTAAATATGGAAC
TAACCATTCCAATTTTCGCCACGAATTCCGACTGCGAGCTTATTGTTAAGGCAATGCAAG
GTCTCCTAAAAGATGGAAACCCGATTCCCTCAGCAATCGCAGCAAACTCCGGCATCTAC
GGTACCGGCGCCCCCGGCTCCGCCGGCTCCGCCGCCGGCTCCGGCGCGTGCCCGGT
GCCGCTGCAGCTGCCGCCGCTGGAACGCCTGACCCTGGATGATAAAAACCGCTGAAT
ACCCTGATCTCTGCTACTGGTCTGTGGATGAGTCGTACCGGAACCATTCATAAAATCAA
ACACCACGAGGTTAGCCGTTCGAAAATCTATATTGAGATGGCGTGTGGCGATCATCTGG
 
TTGTGAACAATAGCCGCTCTTCTCGTACAGCACGTGCACTGCGTCACCACAAATATCGT
AAAACCTGTAAACGTTGCCGTGTGTCCGATGAGGATCTGAACAAATTCCTGACAAAAGC
CAATGAGGACCAAACAAGCGTGAAAGTGAAAGTCGTTAGCGCTCCTACCCGTACTAAAA
AAGCAATGCCGAAATCCGTTGCTCGTGCCCCTAAACCACTGGAAAACACTGAAGCAGCA
CAGGCACAGCCGTCTGGAAGCAAATTCTCTCCGGCCATTCCTGTTTCTACCCAGGAGTC
CGTTTCTGTTCCAGCAAGTGTGAGCACCAGCATTAGCAGTATTAGCACCGGTGCCACCG
CTAGCGCCCTGGTTAAAGGCAATACCAATCCGATTACAAGCATGTCTGCCCCGGTTCAA
GCATCAGCTCCAGCACTGACAAAATCCCAAACCGATCGTCTGGAGGTTCTGCTGAATCC
GAAAGACGAAATCAGCCTGAATTCCGGCAAACCGTTTCGTGAACTGGAGAGCGAACTG
CTGTCACGTCGTAAAAAGACCTGCAACAAATCTATGCCGAAGAACGTGAGAACTATCT
GGGGAAACTGGAACGTGAAATCACCCGCTTTTTCGTGGATCGTGGCTTTCTGGAGATCA
AATCCCCGATTCTGATTCCTCTGGAGTATATCGAGCGTATGGGCATCGACAATGATACC
GAACTGAGCAAACAAATTTTCCGTGTGGATAAAAACTTCTGTCTGCGCCCTATGCTAGCA
CCAAATCTGGCTAACTATCTGCGCAAACTGGACCGTGCCCTGCCTGATCCTATCAAAAT
CTTCGAGATCGGCCCGTGTTATCGTAAAGAGTCCGACGGTAAAGAACATCTGGAGGAG
TTTACCATGCTGAACTTTTGCCAAATGGGTTCAGGTTGTACTCGTGAGAACCTGGAAAG
CATCATCACCGATTTTCTGAACCACCTGGGCATTGACTTCAAAATTGTGGGCGACAGCT
GTATGGTGTTTGGCGACACCCTGGATGTCATGCACGGCGACCTGGAACTGTCTAGTGC
CGTTGTGGGCCCAATCCCGCTGGATCGTGAGTGGGGTATCGACAAACCTTGGATCGGT
GCGGGTTTTGGTCTGGAGCGTCTGCTGAAAGTAAAACACGACTTCAAGAACATCAAACG
TGCTGCACGTTCCGAGTCCTATTACAATGGTATTTCTACTAACCTGTAA (SEQ ID NO:89)
Protein:

MSDTKVKVAVRVRPMNRRELELNTKCVVEMEGNQTVLHPPPSNTKQGERKPPKVFAFDYC
FWSMDESNTTKYAGQEVVFKCLGEGILEKAFQGYNACIFAYGQTGSGKSFSMMGHAEQLG
LIPRLCCALFKRISLEQNESQTFKVEVSYMEIYNEKVRDLLDPKGSRQSLKVREHKVLGPYV
DGLSQLAVTSFEDIESLMSEGNKSRTVAATNMNEESSRSHAVFNIIITQTLYDLQSGNSGEKV
SKVSLVDLAGSERVSKTGAAGERLKEGSNINKSLTTLGLVISSLADQAAGKGKSKFVPYRDS
VLTWLLKDNLGGNSQTSMIATISPAADNYEETLSTLRYADRAKRIVNHAVVNEDPNAKVIREL
REEVEKLREQLSQAEAMKAELKEKLEESEKLIKELTVTWEYTDIEMNRLGKGAPGSAGSAA
GSGMASNDYTQQATQSYGAYPTQPGQGYSQQSSQPYGQQSYSGYSQSTDTSGYGQSSY
SSYGQSQNTGYGTQSTPQGYGSTGGYGSSQSSQSSYGQQSSYPGYGQQPAPSSTSGSY
GSSSQSSSYGQPQSGSYSQQPSYGGQQQSYGQQQSYNPPQGYGQQNQYNSSSGGGG
GGGGGGNYGQDQSSMSSGGGSGGGYGNQDQSGGGGSGGYGQQDRGGRGRGGSGGG
GGGGGGGYNRSSGGYEPRGRGGGRGGRGGMGGSDRGGFNKFGGPRDQGSRHDSEQD
NSDNNTIFVQGLGENVTIESVADYFKQIGIIKTNKKTGQPMINLYTDRETGKLKGEATVSFDD
PPSAKAAIDWFDGKEFSGNPIKVSFATRRADFNRGGGNGRGGRGRGGPMGRGGYGGGG

SGGGGRGGFPSGGGGGGGQQRAGDWKCPNPTCENMNFSWRNECNQCKAPKPDGPGG
GPGGSHMGGNYGDDRRGGRGGDYKDDDDKGTGAPGSAGSAAGSGASNFTQFVLVDNG
GTGDVTVAPSNFANGIAEWISSNSRSQAYKVTCSVRQSSAQNRKYTIKVEVPKGAWRSYLN
MELTIPIFATNSDCELIVKAMQGLLKDGNPIPSAIAANSGIYGTGAPGSAGSAAGSGACPVPL
QLPPLERLTLDDKKPLNTLISATGLWMSRTGTIHKIKHHEVSRSKIYIEMACGDHLVVNNSRS
SRTARALRHHKYRKTCKRCRVSDEDLNKFLTKANEDQTSVKVKVVSAPTRTKKAMPKSVAR
APKPLENTEAAQAQPSGSKFSPAIPVSTQESVSVPASVSTSISSISTGATASALVKGNTNPIT
SMSAPVQASAPALTKSQTDRLEVLLNPKDEISLNSGKPFRELESELLSRRKKDLQQIYAEER
ENYLGKLEREITRFFVDRGFLEIKSPILIPLEYIERMGIDNDTELSKQIFRVDKNFCLRPMLAPN
LANYLRKLDRALPDPIKIFEIGPCYRKESDGKEHLEEFTMLNFCQMGSGCTRENLESIITDFLN
HLGIDFKIVGDSCMVFGDTLDVMHGDLELSSAVVGPIPLDREWGIDKPWIGAGFGLERLLKV
KHDFKNIKRAARSESYYNGISTNL (SEQ ID NO:90)

KIF13A-PyIRS^{AF}
DNA: 
ATGTCGGATACCAAGGTAAAAGTTGCCGTCCGGGTCCGGCCCATGAACCGACGAGAAC
TGGAACTGAACACCAAGTGCGTGGTGGAGATGGAAGGAATCAAACGGTCCTGCACCC
TCCTCCTTCTAACACCAAACAGGGAGAAAGGAAACCTCCCAAGGTATTTGCCTTTGATTA
TTGCTTTTGGTCCATGGATGAATCTAACACTACAAAATACGCTGGTCAAGAAGTGGTTTT
CAAGTGCCTTGGGGAAGGAATTCTTGAAAAAGCCTTTCAGGGGTATAATGCGTGTATTT
TTGCATATGGACAGACAGGTTCGGGAAAATCCTTTTCCATGATGGGCCATGCTGAGCAG
CTGGGCCTTATTCCAAGGCTCTGCTGTGCTTTATTTAAAAGGATCTCTTTGGAGCAAAAT
GAGTCACAGACCTTTAAAGTTGAAGTGTCCTATATGGAAATTTATAATGAGAAAGTTCGG
GATCTTTTAGACCCCAAAGGGAGTAGACAGTCTCTTAAAGTTCGAGAACATAAAGTTTTG
GGACCATATGTAGATGGTTTATCTCAACTAGCTGTCACTAGTTTTGAGGATATTGAGTCA
TTGATGTCTGAGGGAAATAAGTCTCGAACGGTAGCTGCTACCAACATGAACGAAGAAAG
CAGCCGCTCCCATGCTGTGTTCAACATCATAATCACACAGACACTTTATGACCTGCAGT
CTGGGAATTCCGGGGAGAAAGTCAGTAAGGTCAGCTTGGTAGACCTGGCGGGTAGCGA
AAGAGTATCTAAAACAGGAGCTGCAGGAGAGCGACTGAAAGAAGGCAGCAACATTAAC
AAATCGCTTACAACCTTGGGGTTGGTTATATCATCACTGGCTGACCAGGCAGCTGGCAA
GGGTAAAAGCAAATTTGTGCCTTATCGAGATTCAGTCCTCACTTGGCTGCTTAAGGACA
ACTTGGGGGGCAACAGCCAAACCTCTATGATAGCCACAATCAGCCCAGCCGCAGACAA
CTATGAAGAGACCCTCTCCACATTAAGATATGCAGACCGAGCCAAAAGGATTGTGAACC
ATGCTGTTGTGAATGAGGACCCCAACGCAAAAGTGATCCGAGAACTGCGGGAGGAAGT
CGAGAAACTGAGAGAGCAGCTCTCTCAGGCAGAGGCCATGAAGGCCGAACTGAAGGA
GAAGCTCGAAGAGTCTGAAAAGCTGATAAAAGAACTAACAGTGACTTGGGAATATACAG
 
ATATTGAAATGAACAGATTGGGAAAGGGCGCCCCCGGCTCCGCCGGCTCCGCCGCCG
GCTCCGGCATGGCGTGCCCGGTGCCGCTGCAGCTGCCGCCGCTGGAACGCCTGACCC
TGGATGATAAAAAACCGCTGAATACCCTGATCTCTGCTACTGGTCTGTGGATGAGTCGT
ACCGGAACCATTCATAAAATCAAACACCACGAGGTTAGCCGTTCGAAAATCTATATTGAG
ATGGCGTGTGGCGATCATCTGGTTGTGAACAATAGCCGCTCTTCTCGTACAGCACGTGC
ACTGCGTCACCACAAATATCGTAAAACCTGTAAACGTTGCCGTGTGTCCGATGAGGATC
TGAACAAATTCCTGACAAAAGCCAATGAGGACCAAACAAGCGTGAAAGTGAAAGTCGTT
AGCGCTCCTACCCGTACTAAAAAAGCAATGCCGAAATCCGTTGCTCGTGCCCCTAAACC
ACTGGAAAACACTGAAGCAGCACAGGCACAGCCGTCTGGAAGCAAATTCTCTCCGGCC
ATTCCTGTTTCTACCCAGGAGTCCGTTTCTGTTCCAGCAAGTGTGAGCACCAGCATTAG
CAGTATTAGCACCGGTGCCACCGCTAGCGCCCTGGTTAAAGGCAATACCAATCCGATTA
CAAGCATGTCTGCCCCGGTTCAAGCATCAGCTCCAGCACTGACAAAATCCCAAACCGAT
CGTCTGGAGGTTCTGCTGAATCCGAAAGACGAAATCAGCCTGAATTCCGGCAAACCGTT
TCGTGAACTGGAGAGCGAACTGCTGTCACGTCGTAAAAAAGACCTGCAACAAATCTATG
CCGAAGAACGTGAGAACTATCTGGGGAAACTGGAACGTGAAATCACCCGCTTTTTCGTG
GATCGTGGCTTTCTGGAGATCAAATCCCCGATTCTGATTCCTCTGGAGTATATCGAGCG
TATGGGCATCGACAATGATACCGAACTGAGCAAACAAATTTTCCGTGTGGATAAAAACTT
CTGTCTGCGCCCTATGCTAGCACCAAATCTGGCTAACTATCTGCGCAAACTGGACCGTG
CCCTGCCTGATCCTATCAAAATCTTCGAGATCGGCCCGTGTTATCGTAAAGAGTCCGAC
GGTAAAGAACATCTGGAGGAGTTTACCATGCTGAACTTTTGCCAAATGGGTTCAGGTTG
TACTCGTGAGAACCTGGAAAGCATCATCACCGATTTTCTGAACCACCTGGGCATTGACT
TCAAAATTGTGGGCGACAGCTGTATGGTGTTTGGCGACACCCTGGATGTCATGCACGG
CGACCTGGAACTGTCTAGTGCCGTTGTGGGCCCAATCCCGCTGGATCGTGAGTGGGGT
ATCGACAAACCTTGGATCGGTGCGGGTTTTGGTCTGGAGCGTCTGCTGAAAGTAAAACA
CGACTTCAAGAACATCAAACGTGCTGCACGTTCCGAGTCCTATTACAATGGTATTTCTAC
TAACCTGTAA (SEQ ID NO:91)
Protein:

MSDTKVKVAVRVRPMNRRELELNTKCVVEMEGNQTVLHPPPSNTKQGERKPPKVFAFDYC
FWSMDESNTTKYAGQEVVFKCLGEGILEKAFQGYNACIFAYGQTGSGKSFSMMGHAEQLG
LIPRLCCALFKRISLEQNESQTFKVEVSYMEIYNEKVRDLLDPKGSRQSLKVREHKVLGPYV
DGLSQLAVTSFEDIESLMSEGNKSRTVAATNMNEESSRSHAVFNIIITQTLYDLQSGNSGEKV
SKVSLVDLAGSERVSKTGAAGERLKEGSNINKSLTTLGLVISSLADQAAGKGKSKFVPYRDS
VLTWLLKDNLGGNSQTSMIATISPAADNYEETLSTLRYADRAKRIVNHAVVNEDPNAKVIREL
REEVEKLREQLSQAEAMKAELKEKLEESEKLIKELTVTWEYTDIEMNRLGKGAPGSAGSAA
GSGMACPVPLQLPPLERLTLDDKKPLNTLISATGLWMSRTGTIHKIKHHEVSRSKIYIEMACG
DHLVVNNSRSSRTARALRHHKYRKTCKRCRVSDEDLNKFLTKANEDQTSVKVKVVSAPTRT

KKAMPKSVARAPKPLENTEAAQAQPSGSKFSPAIPVSTQESVSVPASVSTSISSISTGATAS
ALVKGNTNPITSMSAPVQASAPALTKSQTDRLEVLLNPKDEISLNSGKPFRELESELLSRRKK
DLQQIYAEERENYLGKLEREITRFFVDRGFLEIKSPILIPLEYIERMGIDNDTELSKQIFRVDKN
FCLRPMLAPNLANYLRKLDRALPDPIKIFEIGPCYRKESDGKEHLEEFTMLNFCQMGSGCTR
ENLESIITDFLNHLGIDFKIVGDSCMVFGDTLDVMHGDLELSSAVVGPIPLDREWGIDKPWIG
AGFGLERLLKVKHDFKNIKRAARSESYYNGISTNL (SEQ ID NO:92)

KIF13A-MCP
DNA: 
ATGTCGGATACCAAGGTAAAAGTTGCCGTCCGGGTCCGGCCCATGAACCGACGAGAAC
TGGAACTGAACACCAAGTGCGTGGTGGAGATGGAAGGAATCAAACGGTCCTGCACCC
TCCTCCTTCTAACACCAAACAGGGAGAAAGGAAACCTCCCAAGGTATTTGCCTTTGATTA
TTGCTTTTGGTCCATGGATGAATCTAACACTACAAAATACGCTGGTCAAGAAGTGGTTTT
CAAGTGCCTTGGGGAAGGAATTCTTGAAAAAGCCTTTCAGGGGTATAATGCGTGTATTT
TTGCATATGGACAGACAGGTTCGGGAAAATCCTTTTCCATGATGGGCCATGCTGAGCAG
CTGGGCCTTATTCCAAGGCTCTGCTGTGCTTTATTTAAAGGATCTCTTTGGAGCAAAAT
GAGTCACAGACCTTTAAAGTTGAAGTGTCCTATATGGAAATTTATAATGAGAAAGTTCGG
GATCTTTTAGACCCCAAAGGGAGTAGACAGTCTCTTAAAGTTCGAGAACATAAAGTTTTG
GGACCATATGTAGATGGTTTATCTCAACTAGCTGTCACTAGTTTTGAGGATATTGAGTCA
TTGATGTCTGAGGGAAATAAGTCTCGAACGGTAGCTGCTACCAACATGAACGAAGAAAG
CAGCCGCTCCCATGCTGTGTTCAACATCATAATCACACAGACACTTTATGACCTGCAGT
CTGGGAATTCCGGGGAGAAAGTCAGTAAGGTCAGCTTGGTAGACCTGGCGGGTAGCGA
AAGAGTATCTAAAACAGGAGCTGCAGGAGAGCGACTGAAAGAAGGCAGCAACATTAAC
AAATCGCTTACAACCTTGGGGTTGGTTATATCATCACTGGCTGACCAGGCAGCTGGCAA
GGGTAAAAGCAAATTTGTGCCTTATCGAGATTCAGTCCTCACTTGGCTGCTTAAGGACA
ACTTGGGGGGCAACAGCCAAACCTCTATGATAGCCACAATCAGCCCAGCCGCAGACAA
CTATGAAGAGACCCTCTCCACATTAAGATATGCAGACCGAGCCAAAAGGATTGTGAACC
ATGCTGTTGTGAATGAGGACCCCAACGCAAAAGTGATCCGAGAACTGCGGGAGGAAGT
CGAGAAACTGAGAGAGCAGCTCTCTCAGGCAGAGGCCATGAAGGCCGAACTGAAGGA
GAAGCTCGAAGAGTCTGAAAAGCTGATAAAAGAACTAACAGTGACTTGGGAATATACAG
ATATTGAAATGAACAGATTGGGAAAGGGCGCCCCCGGCTCCGCCGGCTCCGCCGCCG
GCTCCGGCATGGCTTCTAACTTTACTCAGTTCGTTCTCGTCGACAATGGCGGAACTGGC
GACGTGACTGTCGCCCCAAGCAACTTCGCTAACGGGATCGCTGAATGGATCAGCTCTA
ACTCGCGTTCACAGGCTTACAAAGTAACCTGTAGCGTTCGTCAGAGCTCTGCGCAGAAT
CGCAAATACACCATCAAAGTCGAGGTGCCTAAAGGCGCCTGGCGTTCGTACTTAAATAT
GGAACTAACCATTCCAATTTTCGCCACGAATTCCGACTGCGAGCTTATTGTTAAGGCAAT
 
GCAAGGTCTCCTAAAAGATGGAAACCCGATTCCCTCAGCAATCGCAGCAAACTCCGGCA
TCTACTAA (SEQ ID NO:93)
Protein:

MSDTKVKVAVRVRPMNRRELELNTKCVVEMEGNQTVLHPPPSNTKQGERKPPKVFAFDYC
FWSMDESNTTKYAGQEVVFKCLGEGILEKAFQGYNACIFAYGQTGSGKSFSMMGHAEQLG
LIPRLCCALFKRISLEQNESQTFKVEVSYMEIYNEKVRDLLDPKGSRQSLKVREHKVLGPYV
DGLSQLAVTSFEDIESLMSEGNKSRTVAATNMNEESSRSHAVFNIIITQTLYDLQSGNSGEKV
SKVSLVDLAGSERVSKTGAAGERLKEGSNINKSLTTLGLVISSLADQAAGKGKSKFVPYRDS
VLTWLLKDNLGGNSQTSMIATISPAADNYEETLSTLRYADRAKRIVNHAVVNEDPNAKVIREL
REEVEKLREQLSQAEAMKAELKEKLEESEKLIKELTVTWEYTDIEMNRLGKGAPGSAGSAA
GSGMASNFTQFVLVDNGGTGDVTVAPSNFANGIAEWISSNSRSQAYKVTCSVRQSSAQNR
KYTIKVEVPKGAWRSYLNMELTIPIFATNSDCELIVKAMQGLLKDGNPIPSAIAANSGIY (SEQ
ID NO:94)

TOMM20-EWSR1-MCP
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAGTTCTTCATGGCGTCCACGGATTACAGTACC
TATAGCCAAGCTGCAGCGCAGCAGGGCTACAGTGCTTACACCGCCCAGCCCACTCAAG
GATATGCACAGACCACCCAGGCATATGGGCAACAAAGCTATGGAACCTATGGACAGCC
CACTGATGTCAGCTATACCCAGGCTCAGACCACTGCAACCTATGGGCAGACCGCCTAT
GCAACTTCTTATGGACAGCCTCCCACTGGTTATACTACTCCAACTGCCCCCCAGGCATA
CAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCC
AGCCTATGGGCAGCAGCCAGCAGCCACTGCACCTACAAGACCGCAGGATGGAAACAAG
CCCACTGAGACTAGTCAACCTCAATCTAGCACAGGGGGTTACAACCAGCCCAGCCTAG
GATATGGACAGAGTAACTACAGTTATCCCCAGGTACCTGGGAGCTACCCCATGCAGCCA
GTCACTGCACCTCCATCCTACCCTCCTACCAGCTATTCCTCTACACAGCCGACTAGTTAT
GATCAGAGCAGTTACTCTCAGCAGAACACCTATGGGCAACCGAGCAGCTATGGACAGC
AGAGTAGCTATGGTCAACAAAGCAGCTATGGGCAGCAGCCTCCCACTAGTTACCCACC
CCAAACTGGATCCTACAGCCAAGCTCCAAGTCAATATAGCCAACAGAGCAGCAGCTACG
GGCAGCAGAGTTCATTCCGACAGGACCACCCCAGTAGCATGGGTGTTTATGGGCAGGA
GTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGG
 
AGGACGCGGTGGAATGGGCAGCGCTGGAGAGCGAGGTGGCTTCAATAAGCCTGGTGG
ACCCATGGATGAAGGACCAGATCTTGATCTAGGCCCACCTGTAGATCCAGATGAAGACT
CTGACAACAGTGCAATTTATGTACAAGGATTAAATGACAGTGTGACTCTAGATGATCTGG
CAGACTTCTTTAAGCAGTGTGGGGTTGTTAAGATGAACAAGAGAACTGGGCAACCCATG
ATCCACATCTACCTGGACAAGGAAACAGGAAAGCCCAAAGGCGATGCCACAGTGTCCT
ATGAAGACCCACCTACTGCCAAGGCTGCCGTGGAATGGTTTGATGGGAAAGATTTTCAA
GGGAGCAAACTTAAAGTCTCCCTTGCTCGGAAGAAGCCTCCAATGAACAGTATGCGGG
GTGGTCTGCCACCCCGTGAGGGCAGAGGCATGCCACCACCACTCCGTGGAGGTCCAG
GAGGCCCAGGAGGTCCTGGGGGACCCATGGGTCGCATGGGAGGCCGTGGAGGAGAT
AGAGGAGGCTTCCCTCCAAGAGGACCCCGGGGTTCCCGAGGGAACCCCTCTGGAGGA
GGAAACGTCCAGCACCGAGCTGGAGACTGGCAGTGTCCCAATCCGGGTTGTGGAAACC
AGAACTTCGCCTGGAGAACAGAGTGCAACCAGTGTAAGGCCCCAAAGCCTGAAGGCTT
CCTCCCGCCACCCTTTCCGCCCCCGGGTGGTGATCGTGGCAGAGGTGGCCCTGGTGG
CATGCGGGGAGGAAGAGGTGGCCTCATGGATCGTGGTGGTCCCGGTGGAATGTTCAG
AGGTGGCCGTGGTGGAGACAGAGGTGGCTTCCGTGGTGGCCGGGGCATGGACCGAG
GTGGCTTTGGTGGAGGAAGACGAGGTGGCCCTGGGGGGCCCCCTGGACCTTTGATGG
AACAGGATTACAAGGATGACGACGATAAGGGTACCGAGCAGAAGCTGATCTCAGAGGA
GGACCTGGGCGCCCCCGGCTCCGCCGGCTCCGCCGCCGGCTCCGGCGCTTCTAACTT
TACTCAGTTCGTTCTCGTCGACAATGGCGGAACTGGCGACGTGACTGTCGCCCCAAGC
AACTTCGCTAACGGGATCGCTGAATGGATCAGCTCTAACTCGCGTTCACAGGCTTACAA
AGTAACCTGTAGCGTTCGTCAGAGCTCTGCGCAGAATCGCAAATACACCATCAAAGTCG
AGGTGCCTAAAGGCGCCTGGCGTTCGTACTTAAATATGGAACTAACCATTCCAATTTTC
GCCACGAATTCCGACTGCGAGCTTATTGTTAAGGCAATGCAAGGTCTCCTAAAAGATGG
AAACCCGATTCCCTCAGCAATCGCAGCAAACTCCGGCATCTACTAA (SEQ ID NO:95)
Protein:

MVGRNSAIAAGVCGALFIGYCIYFDRKRRSDPNFKNRLRERRKKQKLAKERAGLSKLPDLKD
AEAVQKFFMASTDYSTYSQAAAQQGYSAYTAQPTQGYAQTTQAYGQQSYGTYGQPTDVS
YTQAQTTATYGQTAYATSYGQPPTGYTTPTAPQAYSQPVQGYGTGAYDTTTATVTTTQAS
YAAQSAYGTQPAYPAYGQQPAATAPTRPQDGNKPTETSQPQSSTGGYNQPSLGYGQSNY
SYPQVPGSYPMQPVTAPPSYPPTSYSSTQPTSYDQSSYSQQNTYGQPSSYGQQSSYGQQ
SSYGQQPPTSYPPQTGSYSQAPSQYSQQSSSYGQQSSFRQDHPSSMGVYGQESGGFSG
PGENRSMSGPDNRGRGRGGFDRGGMSRGGRGGGRGGMGSAGERGGFNKPGGPMDEG
PDLDLGPPVDPDEDSDNSAIYVQGLNDSVTLDDLADFFKQCGVVKMNKRTGQPMIHIYLDK
ETGKPKGDATVSYEDPPTAKAAVEWFDGKDFQGSKLKVSLARKKPPMNSMRGGLPPREG
RGMPPPLRGGPGGPGGPGGPMGRMGGRGGDRGGFPPRGPRGSRGNPSGGGNVQHRA
GDWQCPNPGCGNQNFAWRTECNQCKAPKPEGFLPPPFPPPGGDRGRGGPGGMRGGRG

GLMDRGGPGGMFRGGRGGDRGGFRGGRGMDRGGFGGGRRGGPGGPPGPLMEQDYKD
DDDKGTEQKLISEEDLGAPGSAGSAAGSGASNFTQFVLVDNGGTGDVTVAPSNFANGIAE
WISSNSRSQAYKVTCSVRQSSAQNRKYTIKVEVPKGAWRSYLNMELTIPIFATNSDCELIVKA
MQGLLKDGNPIPSAIAANSGIY (SEQ ID NO:96)

TOMM20-EWSR1-HA-MCP
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAGTTCTTCATGGCGTCCACGGATTACAGTACC
TATAGCCAAGCTGCAGCGCAGCAGGGCTACAGTGCTTACACCGCCCAGCCCACTCAAG
GATATGCACAGACCACCCAGGCATATGGGCAACAAAGCTATGGAACCTATGGACAGCC
CACTGATGTCAGCTATACCCAGGCTCAGACCACTGCAACCTATGGGCAGACCGCCTAT
GCAACTTCTTATGGACAGCCTCCCACTGGTTATACTACTCCAACTGCCCCCCAGGCATA
CAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCC
AGCCTATGGGCAGCAGCCAGCAGCCACTGCACCTACAAGACCGCAGGATGGAAACAAG
CCCACTGAGACTAGTCAACCTCAATCTAGCACAGGGGGTTACAACCAGCCCAGCCTAG
GATATGGACAGAGTAACTACAGTTATCCCCAGGTACCTGGGAGCTACCCCATGCAGCCA
GTCACTGCACCTCCATCCTACCCTCCTACCAGCTATTCCTCTACACAGCCGACTAGTTAT
GATCAGAGCAGTTACTCTCAGCAGAACACCTATGGGCAACCGAGCAGCTATGGACAGC
AGAGTAGCTATGGTCAACAAAGCAGCTATGGGCAGCAGCCTCCCACTAGTTACCCACC
CCAAACTGGATCCTACAGCCAAGCTCCAAGTCAATATAGCCAACAGAGCAGCAGCTACG
GGCAGCAGAGTTCATTCCGACAGGACCACCCCAGTAGCATGGGTGTTTATGGGCAGGA
GTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGGCGGGGAGG
AGGACGCGGTGGAATGGGCAGCGCTGGAGAGCGAGGTGGCTTCAATAAGCCTGGTGG
ACCCATGGATGAAGGACCAGATCTTGATCTAGGCCCACCTGTAGATCCAGATGAAGACT
CTGACAACAGTGCAATTTATGTACAAGGATTAAATGACAGTGTGACTCTAGATGATCTGG
CAGACTTCTTTAAGCAGTGTGGGGTTGTTAAGATGAACAAGAGAACTGGGCAACCCATG
ATCCACATCTACCTGGACAAGGAAACAGGAAAGCCCAAAGGCGATGCCACAGTGTCCT
ATGAAGACCCACCTACTGCCAAGGCTGCCGTGGAATGGTTTGATGGGAAAGATTTTCAA
GGGAGCAAACTTAAAGTCTCCCTTGCTCGGAAGAAGCCTCCAATGAACAGTATGCGGG
GTGGTCTGCCACCCCGTGAGGGCAGAGGCATGCCACCACCACTCCGTGGAGGTCCAG
GAGGCCCAGGAGGTCCTGGGGGACCCATGGGTCGCATGGGAGGCCGTGGAGGAGAT
 
AGAGGAGGCTTCCCTCCAAGAGGACCCCGGGGTTCCCGAGGGAACCCCTCTGGAGGA
GGAAACGTCCAGCACCGAGCTGGAGACTGGCAGTGTCCCAATCCGGGTTGTGGAAACC
AGAACTTCGCCTGGAGAACAGAGTGCAACCAGTGTAAGGCCCCAAAGCCTGAAGGCTT
CCTCCCGCCACCCTTTCCGCCCCCGGGTGGTGATCGTGGCAGAGGTGGCCCTGGTGG
CATGCGGGGAGGAAGAGGTGGCCTCATGGATCGTGGTGGTCCCGGTGGAATGTTCAG
AGGTGGCCGTGGTGGAGACAGAGGTGGCTTCCGTGGTGGCCGGGGCATGGACCGAG
GTGGCTTTGGTGGAGGAAGACGAGGTGGCCCTGGGGGGCCCCCTGGACCTTTGATGG
AACAGGCGATCGCATATCCCTATGATGTGCCGGATTATGCTGGAGCACCAGGAAGTGC
TGGTTCTGCTGCTGGTAGTGGAGCTTCTAACTTTACTCAGTTCGTTCTCGTCGACAATG
GCGGAACTGGCGACGTGACTGTCGCCCCAAGCAACTTCGCTAACGGGATCGCTGAATG
GATCAGCTCTAACTCGCGTTCACAGGCTTACAAAGTAACCTGTAGCGTTCGTCAGAGCT
CTGCGCAGAATCGCAAATACACCATCAAAGTCGAGGTGCCTAAAGGCGCCTGGCGTTC
GTACTTAAATATGGAACTAACCATTCCAATTTTCGCCACGAATTCCGACTGCGAGCTTAT
TGTTAAGGCAATGCAAGGTCTCCTAAAAGATGGAAACCCGATTCCCTCAGCAATCGCAG
CAAACTCCGGCATCTACTAA (SEQ ID NO:97)
Protein:

MVGRNSAIAAGVCGALFIGYCIYFDRKRRSDPNFKNRLRERRKKQKLAKERAGLSKLPDLKD
AEAVQKFFMASTDYSTYSQAAAQQGYSAYTAQPTQGYAQTTQAYGQQSYGTYGQPTDVS
YTQAQTTATYGQTAYATSYGQPPTGYTTPTAPQAYSQPVQGYGTGAYDTTTATVTTTQAS
YAAQSAYGTQPAYPAYGQQPAATAPTRPQDGNKPTETSQPQSSTGGYNQPSLGYGQSNY
SYPQVPGSYPMQPVTAPPSYPPTSYSSTQPTSYDQSSYSQQNTYGQPSSYGQQSSYGQQ
SSYGQQPPTSYPPQTGSYSQAPSQYSQQSSSYGQQSSFRQDHPSSMGVYGQESGGFSG
PGENRSMSGPDNRGRGRGGFDRGGMSRGGRGGGRGGMGSAGERGGFNKPGGPMDEG
PDLDLGPPVDPDEDSDNSAIYVQGLNDSVTLDDLADFFKQCGVVKMNKRTGQPMIHIYLDK
ETGKPKGDATVSYEDPPTAKAAVEWFDGKDFQGSKLKVSLARKKPPMNSMRGGLPPREG
RGMPPPLRGGPGGPGGPGGPMGRMGGRGGDRGGFPPRGPRGSRGNPSGGGNVQHRA
GDWQCPNPGCGNQNFAWRTECNQCKAPKPEGFLPPPFPPPGGDRGRGGPGGMRGGRG
GLMDRGGPGGMFRGGRGGDRGGFRGGRGMDRGGFGGGRRGGPGGPPGPLMEQAIAY
PYDVPDYAGAPGSAGSAAGSGASNFTQFVLVDNGGTGDVTVAPSNFANGIAEWISSNSRS
QAYKVTCSVRQSSAQNRKYTIKVEVPKGAWRSYLNMELTIPIFATNSDCELIVKAMQGLLKD
GNPIPSAIAANSGIY (SEQ ID NO:98)

TOMM20-FUS-PyIRS^{AF}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
 
GAACGAAGAAAGAAACAGAAGCTTGCCAAGGAGAGAGCTGGGCTTTCCAAGTTACCTG
ACCTTAAAGATGCTGAAGCTGTTCAGAAATTCTTCATGGCCTCAAACGATTATACCCAAC
AAGCAACCCAAAGCTATGGGGCCTACCCCACCCAGCCCGGGCAGGGCTATTCCCAGCA
GAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGG
AACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTC
CCAGCAACACCTCGGGAAGTTACGGTAGCAGTTCTCAGAGCAGCAGCTATGGGCAGCC
CCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCA
GCAGTGGTGGTGGAGGTGGAGGTGGAGGTGGAGGTAACTATGGCCAAGATCAATCCTC
CATGAGTAGTGGTGGTGGCAGTGGTGGCGGTTATGGCAATCAAGACCAGAGTGGTGGA
GGTGGCAGCGGTGGCTATGGACAGCAGGACCGTGGAGGCCGCGGCAGGGGTGGCAG
TGGTGGCGGCGGCGGCGGCGGTGGTGGTTACAACCGCAGCAGTGGTGGCTATGA
ACCCAGAGGTCGTGGAGGTGGCCGTGGAGGCAGAGGTGGCATGGGCGGAAGTGACC
GTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAAT
TGAGTCTGTGGCTGATTACTTCAAGCAGATTGGTATTATTAAGACAAACAAGAAAACGGG
ACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAA
GAATTCTCCGGAAATCCTATCAAGGTCTCATTTGCTACTCGCCGGGCAGACTTTAATCG
GGGTGGTGGCAATGGTCGTGGAGGCCGAGGGCGAGGAGGACCCATGGGCCGTGGAG
GCTATGGAGGTGGTGGCAGTGGTGGTGGTGGCCGAGGAGGATTTCCCAGTGGAGGTG
GTGGCGGTGGAGGACAGCAGCGAGCTGGTGACTGGAAGTGTCCTAATCCCACCTGTGA
GAATATGAACTTCTCTTGGAGGAATGAATGCAACCAGTGTAAGGCCCCTAAACCAGATG
GCCCAGGAGGGGGACCAGGTGGCTCTCACATGGGGGGTAACTACGGGGATGATCGTC
GTGGTGGCAGAGGAGGCGATTACAAGGATGACGACGATAAGGGTACCGGCGCCCCCG
GCTCCGCCGGCTCCGCCGCCGGCTCCGGCGCGTGCCCGGTGCCGCTGCAGCTGCCG
CCGCTGGAACGCCTGACCCTGGATGATAAAAAACCGCTGAATACCCTGATCTCTGCTAC
TGGTCTGTGGATGAGTCGTACCGGAACCATTCATAAAATCAAACACCACGAGGTTAGCC
GTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTG
CCGTGTGTCCGATGAGGATCTGAACAAATTCCTGACAAAAGCCAATGAGGACCAAACAA
GCGTGAAAGTGAAAGTCGTTAGCGCTCCTACCCGTACTAAAAAGCAATGCCGAAATCC
GTTGCTCGTGCCCCTAAACCACTGGAAAACACTGAAGCAGCACAGGCACAGCCGTCTG
GAAGCAAATTCTCTCCGGCCATTCCTGTTTCTACCCAGGAGTCCGTTTCTGTTCCAGCA
 
AGTGTGAGCACCAGCATTAGCAGTATTAGCACCGGTGCCACCGCTAGCGCCCTGGTTA
AAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAG
CCTGAATTCCGGCAAACCGTTTCGTGAACTGGAGAGCGAACTGCTGTCACGTCGTAAAA
AAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATT
CCTCTGGAGTATATCGAGCGTATGGGCATCGACAATGATACCGAACTGAGCAAACAAAT
TTTCCGTGTGGATAAAAACTTCTGTCTGCGCCCTATGCTAGCACCAAATCTGGCTAACTA
TCTGCGCAAACTGGACCGTGCCCTGCCTGATCCTATCAAAATCTTCGAGATCGGCCCGT
GTTATCGTAAAGAGTCCGACGGTAAAGAACATCTGGAGGAGTTTACCATGCTGAACTTT
TGCCAAATGGGTTCAGGTTGTACTCGTGAGAACCTGGAAAGCATCATCACCGATTTTCT
GAACCACCTGGGCATTGACTTCAAAATTGTGGGCGACAGCTGTATGGTGTTTGGCGACA
CCCTGGATGTCATGCACGGCGACCTGGAACTGTCTAGTGCCGTTGTGGGCCCAATCCC
GCTGGATCGTGAGTGGGGTATCGACAAACCTTGGATCGGTGCGGGTTTTGGTCTGGAG
CGTCTGCTGAAAGTAAAACACGACTTCAAGAACATCAAACGTGCTGCACGTTCCGAGTC
CTATTACAATGGTATTTCTACTAACCTGTAA (SEQ ID NO:99)
Protein:

MVGRNSAIAAGVCGALFIGYCIYFDRKRRSDPNFKNRLRERRKKQKLAKERAGLSKLPDLKD
AEAVQKFFMASNDYTQQATQSYGAYPTQPGQGYSQQSSQPYGQQSYSGYSQSTDTSGY
GQSSYSSYGQSQNTGYGTQSTPQGYGSTGGYGSSQSSQSSYGQQSSYPGYGQQPAPSN
TSGSYGSSSQSSSYGQPQSGSYSQQPSYGGQQQSYGQQQSYNPPQGYGQQNQYNSSS
GGGGGGGGGGNYGQDQSSMSSGGGSGGGYGNQDQSGGGGSGGYGQQDRGGRGRG
GSGGGGGGGGGYNRSSGGYEPRGRGGGRGGRGGMGGSDRGGFNKFGGPRDQGSR
HDSEQDNSDNNTIFVQGLGENVTIESVADYFKQIGIIKTNKKTGQPMINLYTDRETGKLKGEA
TVSFDDPPSAKAAIDWFDGKEFSGNPIKVSFATRRADFNRGGGNGRGGRGRGGPMGRGG
YGGGGSGGGGRGGFPSGGGGGGGQQRAGDWKCPNPTCENMNFSWRNECNQCKAPKP
DGPGGGPGGSHMGGNYGDDRRGGRGGDYKDDDDKGTGAPGSAGSAAGSGACPVPLQL
PPLERLTLDDKKPLNTLISATGLWMSRTGTIHKIKHHEVSRSKIYIEMACGDHLVVNNSRSSR
TARALRHHKYRKTCKRCRVSDEDLNKFLTKANEDQTSVKVKVVSAPTRTKKAMPKSVARAP
KPLENTEAAQAQPSGSKFSPAIPVSTQESVSVPASVSTSISSISTGATASALVKGNTNPITSM
SAPVQASAPALTKSQTDRLEVLLNPKDEISLNSGKPFRELESELLSRRKKDLQQIYAEEREN
YLGKLEREITRFFVDRGFLEIKSPILIPLEYIERMGIDNDTELSKQIFRVDKNFCLRPMLAPNLA
NYLRKLDRALPDPIKIFEIGPCYRKESDGKEHLEEFTMLNFCQMGSGCTRENLESIITDFLNH
LGIDFKIVGDSCMVFGDTLDVMHGDLELSSAVVGPIPLDREWGIDKPWIGAGFGLERLLKVK
HDFKNIKRAARSESYYNGISTNL (SEQ ID NO:100)

TOMM20-FUS-V5-PyIRS^{AF}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAATTCTTCATGGCCTCAAACGATTATACCCAAC
AAGCAACCCAAAGCTATGGGGCCTACCCCACCCAGCCCGGGCAGGGCTATTCCCAGCA
GAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGG
AACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTC
CCAGCAACACCTCGGGAAGTTACGGTAGCAGTTCTCAGAGCAGCAGCTATGGGCAGCC
CCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCA
GCAGTGGTGGTGGAGGTGGAGGTGGAGGTGGAGGTAACTATGGCCAAGATCAATCCTC
CATGAGTAGTGGTGGTGGCAGTGGTGGCGGTTATGGCAATCAAGACCAGAGTGGTGGA
GGTGGCAGCGGTGGCTATGGACAGCAGGACCGTGGAGGCCGCGGCAGGGGTGGCAG
TGGTGGCGGCGGCGGCGGCGGTGGTGGTTACAACCGCAGCAGTGGTGGCTATGA
ACCCAGAGGTCGTGGAGGTGGCCGTGGAGGCAGAGGTGGCATGGGCGGAAGTGACC
GTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAAT
TGAGTCTGTGGCTGATTACTTCAAGCAGATTGGTATTATTAAGACAAACAAGAAAACGGG
ACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAA
GAATTCTCCGGAAATCCTATCAAGGTCTCATTTGCTACTCGCCGGGCAGACTTTAATCG
GGGTGGTGGCAATGGTCGTGGAGGCCGAGGGCGAGGAGGACCCATGGGCCGTGGAG
GCTATGGAGGTGGTGGCAGTGGTGGTGGTGGCCGAGGAGGATTTCCCAGTGGAGGTG
GTGGCGGTGGAGGACAGCAGCGAGCTGGTGACTGGAAGTGTCCTAATCCCACCTGTGA
GAATATGAACTTCTCTTGGAGGAATGAATGCAACCAGTGTAAGGCCCCTAAACCAGATG
GCCCAGGAGGGGGACCAGGTGGCTCTCACATGGGGGGTAACTACGGGGATGATCGTC
GTGGTGGCAGAGGAGGCGCGATCGCAGGCAAGCCTATTCCCAACCCCCTGCTGGGCC
TGGATAGCACCGGAGCACCAGGAAGTGCTGGTTCTGCTGCTGGTAGTGGAGCGTGCCC
GGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCT
GGTTGTGAACAATAGCCGCTCTTCTCGTACAGCACGTGCACTGCGTCACCACAAATATC
 
GTAAAACCTGTAAACGTTGCCGTGTGTCCGATGAGGATCTGAACAAATTCCTGACAAAA
GCCAATGAGGACCAAACAAGCGTGAAAGTGAAAGTCGTTAGCGCTCCTACCCGTACTAA
AAAAGCAATGCCGAAATCCGTTGCTCGTGCCCCTAAACCACTGGAAAACACTGAAGCAG
CACAGGCACAGCCGTCTGGAAGCAAATTCTCTCCGGCCATTCCTGTTTCTACCCAGGAG
TCCGTTTCTGTTCCAGCAAGTGTGAGCACCAGCATTAGCAGTATTAGCACCGGTGCCAC
CGCTAGCGCCCTGGTTAAAGGCAATACCAATCCGATTACAAGCATGTCTGCCCCGGTTC
AAGCATCAGCTCCAGCACTGACAAAATCCCAAACCGATCGTCTGGAGGTTCTGCTGAAT
CCGAAAGACGAAATCAGCCTGAATTCCGGCAAACCGTTTCGTGAACTGGAGAGCGAAC
TGCTGTCACGTCGTAAAAAAGACCTGCAACAAATCTATGCCGAAGAACGTGAGAACTAT
CTGGGGAAACTGGAACGTGAAATCACCCGCTTTTTCGTGGATCGTGGCTTTCTGGAGAT
CAAATCCCCGATTCTGATTCCTCTGGAGTATATCGAGCGTATGGGCATCGACAATGATA
CCGAACTGAGCAAACAAATTTTCCGTGTGGATAAAAACTTCTGTCTGCGCCCTATGCTA
GCACCAAATCTGGCTAACTATCTGCGCAAACTGGACCGTGCCCTGCCTGATCCTATCAA
AATCTTCGAGATCGGCCCGTGTTATCGTAAAGAGTCCGACGGTAAAGAACATCTGGAGG
AGTTTACCATGCTGAACTTTTGCCAAATGGGTTCAGGTTGTACTCGTGAGAACCTGGAA
AGCATCATCACCGATTTTCTGAACCACCTGGGCATTGACTTCAAAATTGTGGGCGACAG
CTGTATGGTGTTTGGCGACACCCTGGATGTCATGCACGGCGACCTGGAACTGTCTAGT
GCCGTTGTGGGCCCAATCCCGCTGGATCGTGAGTGGGGTATCGACAAACCTTGGATCG
GTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID
NO:101)
Protein:

MVGRNSAIAAGVCGALFIGYCIYFDRKRRSDPNFKNRLRERRKKQKLAKERAGLSKLPDLKD
AEAVQKFFMASNDYTQQATQSYGAYPTQPGQGYSQQSSQPYGQQSYSGYSQSTDTSGY
GQSSYSSYGQSQNTGYGTQSTPQGYGSTGGYGSSQSSQSSYGQQSSYPGYGQQPAPSN
TSGSYGSSSQSSSYGQPQSGSYSQQPSYGGQQQSYGQQQSYNPPQGYGQQNQYNSSS
GGGGGGGGGGNYGQDQSSMSSGGGSGGGYGNQDQSGGGGSGGYGQQDRGGRGRG
GSGGGGGGGGGGYNRSSGGYEPRGRGGGRGGRGGMGGSDRGGFNKFGGPRDQGSR
HDSEQDNSDNNTIFVQGLGENVTIESVADYFKQIGIIKTNKKTGQPMINLYTDRETGKLKGEA
TVSFDDPPSAKAAIDWFDGKEFSGNPIKVSFATRRADFNRGGGNGRGGRGRGGPMGRGG
YGGGGSGGGGRGGFPSGGGGGGGQQRAGDWKCPNPTCENMNFSWRNECNQCKAPKP
DGPGGGPGGSHMGGNYGDDRRGGRGGAIAGKPIPNPLLGLDSTGAPGSAGSAAGSGACP
VPLQLPPLERLTLDDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKS
VARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAE

ERENYLGKLEREITRFFVDRGFLEIKSPILIPLEYIERMGIDNDTELSKQIFRVDKNFCLRPMLA
PNLANYLRKLDRALPDPIKIFEIGPCYRKESDGKEHLEEFTMLNFCQMGSGCTRENLESIITD
FLNHLGIDFKIVGDSCMVFGDTLDVMHGDLELSSAVVGPIPLDREWGIDKPWIGAGFGLERL
LKVKHDFKNIKRAARSESYYNGISTNL (SEQ ID NO:102)

TOMM20-FUS-PyIRS^{AA}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAGTTCTTCATGGCCTCAAACGATTATACCCAAC
AAGCAACCCAAAGCTATGGGGCCTACCCCACCCAGCCCGGGCAGGGCTATTCCCAGCA
GAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGG
AACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTC
CCAGCAGCACCTCGGGAAGTTACGGTAGCAGTTCTCAGAGCAGCAGCTATGGGCAGCC
CCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCA
GCAGTGGTGGTGGAGGTGGAGGTGGAGGTGGAGGTAACTATGGCCAAGATCAATCCTC
CATGAGTAGTGGTGGTGGCAGTGGTGGCGGTTATGGCAATCAAGACCAGAGTGGTGGA
GGTGGCAGCGGTGGCTATGGACAGCAGGACCGTGGAGGCCGCGGCAGGGGTGGCAG
TGGTGGCGGCGGCGGCGGCGGTGGTGGTTACAACCGCAGCAGTGGTGGCTATGA
ACCCAGAGGTCGTGGAGGTGGCCGTGGAGGCAGAGGTGGCATGGGCGGAAGTGACC
GTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAAT
TGAGTCTGTGGCTGATTACTTCAAGCAGATTGGTATTATTAAGACAAACAAGAAAACGGG
ACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAA
GAATTCTCCGGAAATCCTATCAAGGTCTCATTTGCTACTCGCCGGGCAGACTTTAATCG
GGGTGGTGGCAATGGTCGTGGAGGCCGAGGGCGAGGAGGACCCATGGGCCGTGGAG
GCTATGGAGGTGGTGGCAGTGGTGGTGGTGGCCGAGGAGGATTTCCCAGTGGAGGTG
GTGGCGGTGGAGGACAGCAGCGAGCTGGTGACTGGAAGTGTCCTAATCCCACCTGTGA
GAATATGAACTTCTCTTGGAGGAATGAATGCAACCAGTGTAAGGCCCCTAAACCAGATG
GCCCAGGAGGGGGACCAGGTGGCTCTCACATGGGGGGTAACTACGGGGATGATCGTC
GTGGTGGCAGAGGAGGCGGCGCCCCCGGCTCCGCCGGCTCCGCCGCCGGCTCCGGC
 
ATGGCGTGCCCGGTGCCGCTGCAGCTGCCGCCGCTGGAACGCCTGACCCTGGATGAC
AAAAAACCGCTGAATACCCTGATCTCTGCTACTGGTCTGTGGATGAGTCGTACCGGAAC
CATTCATAAAATCAAACACCACGAGGTTAGCCGTTCGAAAATCTATATTGAGATGGCGTG
TGGCGATCATCTGGTTGTGAACAATAGCCGCTCTTCTCGTACAGCACGTGCACTGCGTC
ACCACAAATATCGTAAAACCTGTAAACGTTGCCGTGTGTCCGATGAGGATCTGAACAAA
TTCCTGACAAAAGCCAATGAGGACCAAACAAGCGTGAAAGTGAAAGTCGTTAGCGCTCC
TACCCGTACTAAAAAAGCAATGCCGAAATCCGTTGCTCGTGCCCCTAAACCACTGGAAA
ACACTGAAGCAGCACAGGCACAGCCGTCTGGAAGCAAATTCTCTCCGGCCATTCCTGTT
TCTACCCAGGAGTCCGTTTCTGTTCCAGCAAGTGTGAGCACCAGCATTAGCAGTATTAG
CACCGGTGCCACCGCTAGCGCCCTGGTTAAAGGCAATACCAATCCGATTACAAGCATGT
CTGCCCCGGTTCAAGCATCAGCTCCAGCACTGACAAAATCCCAAACCGATCGTCTGGA
GGTTCTGCTGAATCCGAAAGACGAAATCAGCCTGAATTCCGGCAAACCGTTTCGTGAAC
TGGAGAGCGAACTGCTGTCACGTCGTAAAAAAGACCTGCAACAAATCTATGCCGAAGAA
CGTGAGAACTATCTGGGGAAACTGGAACGTGAAATCACCCGCTTTTTCGTGGATCGTGG
CTTTCTGGAGATCAAATCCCCGATTCTGATTCCTCTGGAGTATATCGAGCGTATGGGCA
TCGACAATGATACCGAACTGAGCAAACAAATTTTCCGTGTGGATAAAAACTTCTGTCTGC
GCCCTATGCTGGCACCAAATCTGTATAACTATCTGCGCAAACTGGACCGTGCCCTGCCT
GATCCTATCAAAATCTTCGAGATCGGCCCGTGTTATCGTAAAGAGTCCGACGGTAAAGA
ACATCTGGAGGAGTTTACCATGCTGGCCTTTGCCCAAATGGGTTCAGGTTGTACTCGTG
AGAACCTGGAAAGCATCATCACCGATTTTCTGAACCACCTGGGCATTGACTTCAAAATTG
TGGGCGACAGCTGTATGGTGTATGGCGACACCCTGGATGTCATGCACGGCGACCTGGA
ACTGTCTAGTGCCGTTGTTGGACCAATTCCGCTGGACCGTGAGTGGGGTATCGACAAA
CCGTGGATCGGAGCAGGATTCGGTCTGGAACGCCTGCTGAAAGTGAAACACGACTTCA
AAAACATCAAACGTGCCGCCCGTTCTGAATCGTATTATAACGGGATCTCTACGAACCTG
TAA (SEQ ID NO:103)
Protein:

MVGRNSAIAAGVCGALFIGYCIYFDRKRRSDPNFKNRLRERRKKQKLAKERAGLSKLPDLKD
AEAVQKFFMASNDYTQQATQSYGAYPTQPGQGYSQQSSQPYGQQSYSGYSQSTDTSGY
GQSSYSSYGQSQNTGYGTQSTPQGYGSTGGYGSSQSSQSSYGQQSSYPGYGQQPAPSS
TSGSYGSSSQSSSYGQPQSGSYSQQPSYGGQQQSYGQQQSYNPPQGYGQQNQYNSSS
GGGGGGGGGGNYGQDQSSMSSGGGSGGGYGNQDQSGGGGSGGYGQQDRGGRGRG
GSGGGGGGGGGYNRSSGGYEPRGRGGGRGGRGGMGGSDRGGFNKFGGPRDQGSR
HDSEQDNSDNNTIFVQGLGENVTIESVADYFKQIGIIKTNKKTGQPMINLYTDRETGKLKGEA
TVSFDDPPSAKAAIDWFDGKEFSGNPIKVSFATRRADFNRGGGNGRGGRGRGGPMGRGG
YGGGGSGGGGRGGFPSGGGGGGGQQRAGDWKCPNPTCENMNFSWRNECNQCKAPKP
DGPGGGPGGSHMGGNYGDDRRGGRGGGAPGSAGSAAGSGMACPVPLQLPPLERLTLDD

KKPLNTLISATGLWMSRTGTIHKIKHHEVSRSKIYIEMACGDHLVVNNSRSSRTARALRHHKY
RKTCKRCRVSDEDLNKFLTKANEDQTSVKVKVVSAPTRTKKAMPKSVARAPKPLENTEAAQ
AQPSGSKFSPAIPVSTQESVSVPASVSTSISSISTGATASALVKGNTNPITSMSAPVQASAPA
LTKSQTDRLEVLLNPKDEISLNSGKPFRELESELLSRRKKDLQQIYAEERENYLGKLEREITR
FFVDRGFLEIKSPILIPLEYIERMGIDNDTELSKQIFRVDKNFCLRPMLAPNLYNYLRKLDRALP
DPIKIFEIGPCYRKESDGKEHLEEFTMLAFAQMGSGCTRENLESIITDFLNHLGIDFKIVGDSC
MVYGDTLDVMHGDLELSSAVVGPIPLDREWGIDKPWIGAGFGLERLLKVKHDFKNIKRAARS
ESYYNGISTNL (SEQ ID NO:104)

TOMM20-FUS-V5-PyIRS^{AA}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAATTCTTCATGGCCTCAAACGATTATACCCAAC
AAGCAACCCAAAGCTATGGGGCCTACCCCACCCAGCCCGGGCAGGGCTATTCCCAGCA
GAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGG
AACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTC
CCAGCAACACCTCGGGAAGTTACGGTAGCAGTTCTCAGAGCAGCAGCTATGGGCAGCC
CCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCA
GCAGTGGTGGTGGAGGTGGAGGTGGAGGTGGAGGTAACTATGGCCAAGATCAATCCTC
CATGAGTAGTGGTGGTGGCAGTGGTGGCGGTTATGGCAATCAAGACCAGAGTGGTGGA
GGTGGCAGCGGTGGCTATGGACAGCAGGACCGTGGAGGCCGCGGCAGGGGTGGCAG
TGGTGGCGGCGGCGGCGGCGGCGGTGGTGGTTACAACCGCAGCAGTGGTGGCTATGA
ACCCAGAGGTCGTGGAGGTGGCCGTGGAGGCAGAGGTGGCATGGGCGGAAGTGACC
GTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAAT
TGAGTCTGTGGCTGATTACTTCAAGCAGATTGGTATTATTAAGACAAACAAGAAAACGGG
ACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAA
GAATTCTCCGGAAATCCTATCAAGGTCTCATTTGCTACTCGCCGGGCAGACTTTAATCG
GGGTGGTGGCAATGGTCGTGGAGGCCGAGGGCGAGGAGGACCCATGGGCCGTGGAG
GCTATGGAGGTGGTGGCAGTGGTGGTGGTGGCCGAGGAGGATTTCCCAGTGGAGGTG
 
GTGGCGGTGGAGGACAGCAGCGAGCTGGTGACTGGAAGTGTCCTAATCCCACCTGTGA
GAATATGAACTTCTCTTGGAGGAATGAATGCAACCAGTGTAAGGCCCCTAAACCAGATG
GCCCAGGAGGGGGACCAGGTGGCTCTCACATGGGGGGTAACTACGGGATGATCGTC
GTGGTGGCAGAGGAGGCGCGATCGCAGGCAAGCCTATTCCCAACCCCCTGCTGGGCC
TGGATAGCACCGGAGCACCAGGAAGTGCTGGTTCTGCTGCTGGTAGTGGAGCGTGCCC
GGTGCCGCTGCAGCTGCCGCCGCTGGAACGCCTGACCCTGGATGACAAAAACCGCT
GAATACCCTGATCTCTGCTACTGGTCTGTGGATGAGTCGTACCGGAACCATTCATAAAA
TCAAACACCACGAGGTTAGCCGTTCGAAAATCTATATTGAGATGGCGTGTGGCGATCAT
CTGGTTGTGAACAATAGCCGCTCTTCTCGTACAGCACGTGCACTGCGTCACCACAAATA
TCGTAAAACCTGTAAACGTTGCCGTGTGTCCGATGAGGATCTGAACAAATTCCTGACAA
AAGCCAATGAGGACCAAACAAGCGTGAAAGTGAAAGTCGTTAGCGCTCCTACCCGTACT
AAAAAAGCAATGCCGAAATCCGTTGCTCGTGCCCCTAAACCACTGGAAAACACTGAAGC
AGCACAGGCACAGCCGTCTGGAAGCAAATTCTCTCCGGCCATTCCTGTTTCTACCCAGG
AGTCCGTTTCTGTTCCAGCAAGTGTGAGCACCAGCATTAGCAGTATTAGCACCGGTGCC
ACCGCTAGCGCCCTGGTTAAAGGCAATACCAATCCGATTACAAGCATGTCTGCCCCGGT
TCAAGCATCAGCTCCAGCACTGACAAAATCCCAAACCGATCGTCTGGAGGTTCTGCTGA
ATCCGAAAGACGAAATCAGCCTGAATTCCGGCAAACCGTTTCGTGAACTGGAGAGCGAA
CTGCTGTCACGTCGTAAAAAAGACCTGCAACAAATCTATGCCGAAGAACGTGAGAACTA
TCTGGGGAAACTGGAACGTGAAATCACCCGCTTTTTCGTGGATCGTGGCTTTCTGGAGA
TCAAATCCCCGATTCTGATTCCTCTGGAGTATATCGAGCGTATGGGCATCGACAATGAT
ACCGAACTGAGCAAACAAATTTTCCGTGTGGATAAAAACTTCTGTCTGCGCCCTATGCT
GGCACCAAATCTGTATAACTATCTGCGCAAACTGGACCGTGCCCTGCCTGATCCTATCA
AAATCTTCGAGATCGGCCCGTGTTATCGTAAAGAGTCCGACGGTAAAGAACATCTGGAG
GAGTTTACCATGCTGGCCTTTGCCCAAATGGGTTCAGGTTGTACTCGTGAGAACCTGGA
AAGCATCATCACCGATTTTCTGAACCACCTGGGCATTGACTTCAAAATTGTGGGCGACA
GCTGTATGGTGTATGGCGACACCCTGGATGTCATGCACGGCGACCTGGAACTGTCTAG
TGCCGTTGTTGGACCAATTCCGCTGGACCGTGAGTGGGGTATCGACAAACCGTGGATC
GGAGCAGGATTCGGTCTGGAACGCCTGCTGAAAGTGAAACACGACTTCAAAAACATCAA
ACGTGCCGCCCGTTCTGAATCGTATTATAACGGGATCTCTACGAACCTGTAA (SEQ ID
NO:105)
Protein:

MVGRNSAIAAGVCGALFIGYCIYFDRKRRSDPNFKNRLRERRKKQKLAKERAGLSKLPDLKD
AEAVQKFFMASNDYTQQATQSYGAYPTQPGQGYSQQSSQPYGQQSYSGYSQSTDTSGY
GQSSYSSYGQSQNTGYGTQSTPQGYGSTGGYGSSQSSQSSYGQQSSYPGYGQQPAPSN
TSGSYGSSSQSSSYGQPQSGSYSQQPSYGGQQQSYGQQQSYNPPQGYGQQNQYNSSS
GGGGGGGGGNYGQDQSSMSSGGGSGGGYGNQDQSGGGGSGGYGQQDRGGRGRG

GSGGGGGGGGGGYNRSSGGYEPRGRGGGRGGRGGMGGSDRGGFNKFGGPRDQGSR
HDSEQDNSDNNTIFVQGLGENVTIESVADYFKQIGIIKTNKKTGQPMINLYTDRETGKLKGEA
TVSFDDPPSAKAAIDWFDGKEFSGNPIKVSFATRRADFNRGGGNGRGGRGRGGPMGRGG
YGGGGSGGGGRGGFPSGGGGGGGQQRAGDWKCPNPTCENMNFSWRNECNQCKAPKP
DGPGGGPGGSHMGGNYGDDRRGGRGGAIAGKPIPNPLLGLDSTGAPGSAGSAAGSGACP
VPLQLPPLERLTLDDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKS
VARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAE
ERENYLGKLEREITRFFVDRGFLEIKSPILIPLEYIERMGIDNDTELSKQIFRVDKNFCLRPMLA
PNLYNYLRKLDRALPDPIKIFEIGPCYRKESDGKEHLEEFTMLAFAQMGSGCTRENLESIITDF
LNHLGIDFKIVGDSCMVYGDTLDVMHGDLELSSAVVGPIPLDREWGIDKPWIGAGFGLERLL
KVKHDFKNIKRAARSESYYNGISTNL (SEQ ID NO:106)

TOMM20-FUS-PyIRS^{AAAF}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAATTCTTCATGGCCTCAAACGATTATACCCAAC
AAGCAACCCAAAGCTATGGGGCCTACCCCACCCAGCCCGGGCAGGGCTATTCCCAGCA
GAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGG
AACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTC
CCAGCAACACCTCGGGAAGTTACGGTAGCAGTTCTCAGAGCAGCAGCTATGGGCAGCC
CCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCA
GCAGTGGTGGTGGAGGTGGAGGTGGAGGTGGAGGTAACTATGGCCAAGATCAATCCTC
CATGAGTAGTGGTGGTGGCAGTGGTGGCGGTTATGGCAATCAAGACCAGAGTGGTGGA
GGTGGCAGCGGTGGCTATGGACAGCAGGACCGTGGAGGCCGCGGCAGGGGTGGCAG
TGGTGGCGGCGGCGGCGGCGGTGGTGGTTACAACCGCAGCAGTGGTGGCTATGA
ACCCAGAGGTCGTGGAGGTGGCCGTGGAGGCAGAGGTGGCATGGGCGGAAGTGACC
GTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAAT
TGAGTCTGTGGCTGATTACTTCAAGCAGATTGGTATTATTAAGACAAACAAGAAAACGGG
 
ACAGCCCATGATTAATTTGTACACAGACAGGGAAACTGGCAAGCTGAAGGAGAGGCA
ACGGTCTCTTTTGATGACCCACCTTCAGCTAAAGCAGCTATTGACTGGTTTGATGGTAAA
GAATTCTCCGGAAATCCTATCAAGGTCTCATTTGCTACTCGCCGGGCAGACTTTAATCG
GGGTGGTGGCAATGGTCGTGGAGGCCGAGGGCGAGGAGGACCCATGGGCCGTGGAG
GCTATGGAGGTGGTGGCAGTGGTGGTGGTGGCCGAGGAGGATTTCCCAGTGGAGGTG
GTGGCGGTGGAGGACAGCAGCGAGCTGGTGACTGGAAGTGTCCTAATCCCACCTGTGA
GAATATGAACTTCTCTTGGAGGAATGAATGCAACCAGTGTAAGGCCCCTAAACCAGATG
GCCCAGGAGGGGGACCAGGTGGCTCTCACATGGGGGGTAACTACGGGGATGATCGTC
GTGGTGGCAGAGGAGGCGATTACAAGGATGACGACGATAAGGGTACCGGCGCCCCCG
GCTCCGCCGGCTCCGCCGCCGGCTCCGGCGCGTGCCCGGTGCCGCTGCAGCTGCCG
CCGCTGGAACGCCTGACCCTGGATGATAAAAACCGCTGAATACCCTGATCTCTGCTAC
TGGTCTGTGGATGAGTCGTACCGGAACCATTCATAAAATCAAACACCACGAGGTTAGCC
GTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTG
CCGTGTGTCCGATGAGGATCTGAACAAATTCCTGACAAAAGCCAATGAGGACCAAACAA
GCGTGAAAGTGAAAGTCGTTAGCGCTCCTACCCGTACTAAAAAGCAATGCCGAAATCC
GTTGCTCGTGCCCCTAAACCACTGGAAAACACTGAAGCAGCACAGGCACAGCCGTCTG
GAAGCAAATTCTCTCCGGCCATTCCTGTTTCTACCCAGGAGTCCGTTTCTGTTCCAGCA
AGTGTGAGCACCAGCATTAGCAGTATTAGCACCGGTGCCACCGCTAGCGCCCTGGTTA
AAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAG
CCTGAATTCCGGCAAACCGTTTCGTGAACTGGAGAGCGAACTGCTGTCACGTCGTAAAA
AAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATT
CCTCTGGAGTATATCGAGCGTATGGGCATCGACAATGATACCGAACTGAGCAAACAAAT
TTTCCGTGTGGATAAAAACTTCTGTCTGCGCCCTATGCTAGCACCAAATCTGGCTAACTA
TCTGCGCAAACTGGACCGTGCCCTGCCTGATCCTATCAAAATCTTCGAGATCGGCCCGT
GTTATCGTAAAGAGTCCGACGGTAAAGAACATCTGGAGGAGTTTACCATGCTGGCCTTT
GCCCAAATGGGTTCAGGTTGTACTCGTGAGAACCTGGAAAGCATCATCACCGATTTTCT
GAACCACCTGGGCATTGACTTCAAAATTGTGGGCGACAGCTGTATGGTGTTTGGCGACA
CCCTGGATGTCATGCACGGCGACCTGGAACTGTCTAGTGCCGTTGTGGGCCCAATCCC
GCTGGATCGTGAGTGGGGTATCGACAAACCTTGGATCGGTGCGGGTTTTGGTCTGGAG
CGTCTGCTGAAAGTAAAACACGACTTCAAGAACATCAAACGTGCTGCACGTTCCGAGTC
CTATTACAATGGTATTTCTACTAACCTGTAA (SEQ ID NO:107)
Protein:

MVGRNSAIAAGVCGALFIGYCIYFDRKRRSDPNFKNRLRERRKKQKLAKERAGLSKLPDLKD
AEAVQKFFMASNDYTQQATQSYGAYPTQPGQGYSQQSSQPYGQQSYSGYSQSTDTSGY
GQSSYSSYGQSQNTGYGTQSTPQGYGSTGGYGSSQSSQSSYGQQSSYPGYGQQPAPSN
TSGSYGSSSQSSSYGQPQSGSYSQQPSYGGQQQSYGQQQSYNPPQGYGQQNQYNSSS
GGGGGGGGGGNYGQDQSSMSSGGGSGGGYGNQDQSGGGGSGGYGQQDRGGRGRG
GSGGGGGGGGGYNRSSGGYEPRGRGGGRGGRGGMGGSDRGGFNKFGGPRDQGSR
HDSEQDNSDNNTIFVQGLGENVTIESVADYFKQIGIIKTNKKTGQPMINLYTDRETGKLKGEA
TVSFDDPPSAKAAIDWFDGKEFSGNPIKVSFATRRADFNRGGGNGRGGRGRGGPMGRGG
YGGGGSGGGGRGGFPSGGGGGGGQQRAGDWKCPNPTCENMNFSWRNECNQCKAPKP
DGPGGGPGGSHMGGNYGDDRRGGRGGDYKDDDDKGTGAPGSAGSAAGSGACPVPLQL
PPLERLTLDDKKPLNTLISATGLWMSRTGTIHKIKHHEVSRSKIYIEMACGDHLVVNNSRSSR
TARALRHHKYRKTCKRCRVSDEDLNKFLTKANEDQTSVKVKVVSAPTRTKKAMPKSVARAP
KPLENTEAAQAQPSGSKFSPAIPVSTQESVSVPASVSTSISSISTGATASALVKGNTNPITSM
SAPVQASAPALTKSQTDRLEVLLNPKDEISLNSGKPFRELESELLSRRKKDLQQIYAEEREN
YLGKLEREITRFFVDRGFLEIKSPILIPLEYIERMGIDNDTELSKQIFRVDKNFCLRPMLAPNLA
NYLRKLDRALPDPIKIFEIGPCYRKESDGKEHLEEFTMLAFAQMGSGCTRENLESIITDFLNHL
GIDFKIVGDSCMVFGDTLDVMHGDLELSSAVVGPIPLDREWGIDKPWIGAGFGLERLLKVKH
DFKNIKRAARSESYYNGISTNL (SEQ ID NO:108)

TOMM20-FUS-V5-PyIRS^{AAAF}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAATTCTTCATGGCCTCAAACGATTATACCCAAC
AAGCAACCCAAAGCTATGGGGCCTACCCCACCCAGCCCGGGCAGGGCTATTCCCAGCA
GAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGG
AACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTC
CCAGCAACACCTCGGGAAGTTACGGTAGCAGTTCTCAGAGCAGCAGCTATGGGCAGCC
CCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCA
GCAGTGGTGGTGGAGGTGGAGGTGGAGGTGGAGGTAACTATGGCCAAGATCAATCCTC
CATGAGTAGTGGTGGTGGCAGTGGTGGCGGTTATGGCAATCAAGACCAGAGTGGTGGA
GGTGGCAGCGGTGGCTATGGACAGCAGGACCGTGGAGGCCGCGGCAGGGGTGGCAG
 
TGGTGGCGGCGGCGGCGGCGGTGGTGGTTACAACCGCAGCAGTGGTGGCTATGA
ACCCAGAGGTCGTGGAGGTGGCCGTGGAGGCAGAGGTGGCATGGGCGGAAGTGACC
GTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAAT
TGAGTCTGTGGCTGATTACTTCAAGCAGATTGGTATTATTAAGACAAACAAGAAAACGGG
ACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAA
GAATTCTCCGGAAATCCTATCAAGGTCTCATTTGCTACTCGCCGGGCAGACTTTAATCG
GGGTGGTGGCAATGGTCGTGGAGGCCGAGGGCGAGGAGGACCCATGGGCCGTGGAG
GCTATGGAGGTGGTGGCAGTGGTGGTGGTGGCCGAGGAGGATTTCCCAGTGGAGGTG
GTGGCGGTGGAGGACAGCAGCGAGCTGGTGACTGGAAGTGTCCTAATCCCACCTGTGA
GAATATGAACTTCTCTTGGAGGAATGAATGCAACCAGTGTAAGGCCCCTAAACCAGATG
GCCCAGGAGGGGGACCAGGTGGCTCTCACATGGGGGGTAACTACGGGGATGATCGTC
GTGGTGGCAGAGGAGGCGCGATCGCAGGCAAGCCTATTCCCAACCCCCTGCTGGGCC
TGGATAGCACCGGAGCACCAGGAAGTGCTGGTTCTGCTGCTGGTAGTGGAGCGTGCCC
GGTGCCGCTGCAGCTGCCGCCGCTGGAACGCCTGACCCTGGATGATAAAAAACCGCTG
AATACCCTGATCTCTGCTACTGGTCTGTGGATGAGTCGTACCGGAACCATTCATAAAATC
AAACACCACGAGGTTAGCCGTTCGAAAATCTATATTGAGATGGCGTGTGGCGATCATCT
GGTTGTGAACAATAGCCGCTCTTCTCGTACAGCACGTGCACTGCGTCACCACAAATATC
GTAAAACCTGTAAACGTTGCCGTGTGTCCGATGAGGATCTGAACAAATTCCTGACAAAA
GCCAATGAGGACCAAACAAGCGTGAAAGTGAAAGTCGTTAGCGCTCCTACCCGTACTAA
AAAAGCAATGCCGAAATCCGTTGCTCGTGCCCCTAAACCACTGGAAAACACTGAAGCAG
CACAGGCACAGCCGTCTGGAAGCAAATTCTCTCCGGCCATTCCTGTTTCTACCCAGGAG
TCCGTTTCTGTTCCAGCAAGTGTGAGCACCAGCATTAGCAGTATTAGCACCGGTGCCAC
CGCTAGCGCCCTGGTTAAAGGCAATACCAATCCGATTACAAGCATGTCTGCCCCGGTTC
AAGCATCAGCTCCAGCACTGACAAAATCCCAAACCGATCGTCTGGAGGTTCTGCTGAAT
CCGAAAGACGAAATCAGCCTGAATTCCGGCAAACCGTTTCGTGAACTGGAGAGCGAAC
TGCTGTCACGTCGTAAAAAAGACCTGCAACAAATCTATGCCGAAGAACGTGAGAACTAT
CTGGGGAAACTGGAACGTGAAATCACCCGCTTTTTCGTGGATCGTGGCTTTCTGGAGAT
CAAATCCCCGATTCTGATTCCTCTGGAGTATATCGAGCGTATGGGCATCGACAATGATA
CCGAACTGAGCAAACAAATTTTCCGTGTGGATAAAAACTTCTGTCTGCGCCCTATGCTA
GCACCAAATCTGGCTAACTATCTGCGCAAACTGGACCGTGCCCTGCCTGATCCTATCAA
AATCTTCGAGATCGGCCCGTGTTATCGTAAAGAGTCCGACGGTAAAGAACATCTGGAGG
AGTTTACCATGCTGGCCTTTGCCCAAATGGGTTCAGGTTGTACTCGTGAGAACCTGGAA
AGCATCATCACCGATTTTCTGAACCACCTGGGCATTGACTTCAAAATTGTGGGCGACAG
CTGTATGGTGTTTGGCGACACCCTGGATGTCATGCACGGCGACCTGGAACTGTCTAGT
 
GCCGTTGTGGGCCCAATCCCGCTGGATCGTGAGTGGGGTATCGACAAACCTTGGATCG
GTGCGGGTTTTGGTCTGGAGCGTCTGCTGAAAGTAAAACACGACTTCAAGAACATCAAA
CGTGCTGCACGTTCCGAGTCCTATTACAATGGTATTTCTACTAACCTGTAA (SEQ ID
NO:109)
Protein:

MVGRNSAIAAGVCGALFIGYCIYFDRKRRSDPNFKNRLRERRKKQKLAKERAGLSKLPDLKD
AEAVQKFFMASNDYTQQATQSYGAYPTQPGQGYSQQSSQPYGQQSYSGYSQSTDTSGY
GQSSYSSYGQSQNTGYGTQSTPQGYGSTGGYGSSQSSQSSYGQQSSYPGYGQQPAPSN
TSGSYGSSSQSSSYGQPQSGSYSQQPSYGGQQQSYGQQQSYNPPQGYGQQNQYNSSS
GGGGGGGGGGNYGQDQSSMSSGGGSGGGYGNQDQSGGGGSGGYGQQDRGGRGRG
GSGGGGGGGGGYNRSSGGYEPRGRGGGRGGRGGMGGSDRGGFNKFGGPRDQGSR
HDSEQDNSDNNTIFVQGLGENVTIESVADYFKQIGIIKTNKKTGQPMINLYTDRETGKLKGEA
TVSFDDPPSAKAAIDWFDGKEFSGNPIKVSFATRRADFNRGGGNGRGGRGRGGPMGRGG
YGGGGSGGGGRGGFPSGGGGGGGQQRAGDWKCPNPTCENMNFSWRNECNQCKAPKP
DGPGGGPGGSHMGGNYGDDRRGGRGGAIAGKPIPNPLLGLDSTGAPGSAGSAAGSGACP
VPLQLPPLERLTLDDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKS
VARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAE
ERENYLGKLEREITRFFVDRGFLEIKSPILIPLEYIERMGIDNDTELSKQIFRVDKNFCLRPMLA
PNLANYLRKLDRALPDPIKIFEIGPCYRKESDGKEHLEEFTMLAFAQMGSGCTRENLESIITDF
LNHLGIDFKIVGDSCMVFGDTLDVMHGDLELSSAVVGPIPLDREWGIDKPWIGAGFGLERLL
KVKHDFKNIKRAARSESYYNGISTNL (SEQ ID NO:110)

TOMM20-EWSR1-λ_{N22}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAGTTCTTCATGGCGTCCACGGATTACAGTACC
TATAGCCAAGCTGCAGCGCAGCAGGGCTACAGTGCTTACACCGCCCAGCCCACTCAAG
GATATGCACAGACCACCCAGGCATATGGGCAACAAAGCTATGGAACCTATGGACAGCC
CACTGATGTCAGCTATACCCAGGCTCAGACCACTGCAACCTATGGGCAGACCGCCTAT
GCAACTTCTTATGGACAGCCTCCCACTGGTTATACTACTCCAACTGCCCCCCAGGCATA
CAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCC
 
AGCCTATGGGCAGCAGCCAGCAGCCACTGCACCTACAAGACCGCAGGATGGAAACAAG
CCCACTGAGACTAGTCAACCTCAATCTAGCACAGGGGGTTACAACCAGCCCAGCCTAG
GATATGGACAGAGTAACTACAGTTATCCCCAGGTACCTGGGAGCTACCCCATGCAGCCA
GTCACTGCACCTCCATCCTACCCTCCTACCAGCTATTCCTCTACACAGCCGACTAGTTAT
GATCAGAGCAGTTACTCTCAGCAGAACACCTATGGGCAACCGAGCAGCTATGGACAGC
AGAGTAGCTATGGTCAACAAAGCAGCTATGGGCAGCAGCCTCCCACTAGTTACCCACC
CCAAACTGGATCCTACAGCCAAGCTCCAAGTCAATATAGCCAACAGAGCAGCAGCTACG
GGCAGCAGAGTTCATTCCGACAGGACCACCCCAGTAGCATGGGTGTTTATGGGCAGGA
GTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGCGGGGAGG
AGGACGCGGTGGAATGGGCAGCGCTGGAGAGCGAGGTGGCTTCAATAAGCCTGGTGG
ACCCATGGATGAAGGACCAGATCTTGATCTAGGCCCACCTGTAGATCCAGATGAAGACT
CTGACAACAGTGCAATTTATGTACAAGGATTAAATGACAGTGTGACTCTAGATGATCTGG
CAGACTTCTTTAAGCAGTGTGGGGTTGTTAAGATGAACAAGAGAACTGGGCAACCCATG
ATCCACATCTACCTGGACAAGGAAACAGGAAAGCCCAAAGGCGATGCCACAGTGTCCT
ATGAAGACCCACCTACTGCCAAGGCTGCCGTGGAATGGTTTGATGGGAAAGATTTTCAA
GGGAGCAAACTTAAAGTCTCCCTTGCTCGGAAGAAGCCTCCAATGAACAGTATGCGGG
GTGGTCTGCCACCCCGTGAGGGCAGAGGCATGCCACCACCACTCCGTGGAGGTCCAG
GAGGCCCAGGAGGTCCTGGGGGACCCATGGGTCGCATGGGAGGCCGTGGAGGAGAT
AGAGGAGGCTTCCCTCCAAGAGGACCCCGGGGTTCCCGAGGGAACCCCTCTGGAGGA
GGAAACGTCCAGCACCGAGCTGGAGACTGGCAGTGTCCCAATCCGGGTTGTGGAAACC
AGAACTTCGCCTGGAGAACAGAGTGCAACCAGTGTAAGGCCCCAAAGCCTGAAGGCTT
CCTCCCGCCACCCTTTCCGCCCCCGGGTGGTGATCGTGGCAGAGGTGGCCCTGGTGG
CATGCGGGGAGGAAGAGGTGGCCTCATGGATCGTGGTGGTCCCGGTGGAATGTTCAG
AGGTGGCCGTGGTGGAGACAGAGGTGGCTTCCGTGGTGGCCGGGGCATGGACCGAG
GTGGCTTTGGTGGAGGAAGACGAGGTGGCCCTGGGGGGCCCCCTGGACCTTTGATGG
AACAGGCGATCGCAGGAGCACCAGGAAGTGCTGGTTCTGCTGCTGGTAGTGGAGAGCA
GAAGCTGATCTCAGAGGAGGACCTGCTAGCCACCATGGACGCACAAACACGACGACGT
GAGCGTCGCGCTGAGAAACAAGCTCAATGGAAAGCTGCAAACCCACCGCTCGACGGAG
CCGGAGCTGGCGCTGGAGCTGGAGCCGGAGCTGGCGGTCTAGCCACCATGGACGCAC
AAACACGACGACGTGAGCGTCGCGCTGAGAAACAAGCTCAATGGAAAGCTGCAAACCC
ACCGCTCGACGGAGCCGGAGCTGGCGCTGGAGCTGGAGCCGGAGCTGGCGGTCTAG
CCACCATGGACGCACAAACACGACGACGTGAGCGTCGCGCTGAGAAACAAGCTCAATG
GAAAGCTGCAAACCCACCGCTCGACGGAGCCGGAGCTGGCGCTGGAGCTGGAGCCGG
AGCTGGCGGTCTAGCCACCATGGACGCACAAACACGACGACGTGAGCGTCGCGCTGA
 
GAAACAAGCTCAATGGAAAGCTGCAAACCCACCGCTCGAGTCTAGAGGGCCCGTTTAA
(SEQ ID NO:111)
Protein:

MVGRNSAIAAGVCGALFIGYCIYFDRKRRSDPNFKNRLRERRKKQKLAKERAGLSKLPDLKD
AEAVQKFFMASTDYSTYSQAAAQQGYSAYTAQPTQGYAQTTQAYGQQSYGTYGQPTDVS
YTQAQTTATYGQTAYATSYGQPPTGYTTPTAPQAYSQPVQGYGTGAYDTTTATVTTTQAS
YAAQSAYGTQPAYPAYGQQPAATAPTRPQDGNKPTETSQPQSSTGGYNQPSLGYGQSNY
SYPQVPGSYPMQPVTAPPSYPPTSYSSTQPTSYDQSSYSQQNTYGQPSSYGQQSSYGQQ
SSYGQQPPTSYPPQTGSYSQAPSQYSQQSSSYGQQSSFRQDHPSSMGVYGQESGGFSG
PGENRSMSGPDNRGRGRGGFDRGGMSRGGRGGGRGGMGSAGERGGFNKPGGPMDEG
PDLDLGPPVDPDEDSDNSAIYVQGLNDSVTLDDLADFFKQCGVVKMNKRTGQPMIHIYLDK
ETGKPKGDATVSYEDPPTAKAAVEWFDGKDFQGSKLKVSLARKKPPMNSMRGGLPPREG
RGMPPPLRGGPGGPGGPGGPMGRMGGRGGDRGGFPPRGPRGSRGNPSGGGNVQHRA
GDWQCPNPGCGNQNFAWRTECNQCKAPKPEGFLPPPFPPPGGDRGRGGPGGMRGGRG
GLMDRGGPGGMFRGGRGGDRGGFRGGRGMDRGGFGGGRRGGPGGPPGPLMEQAIAG
APGSAGSAAGSGEQKLISEEDLLATMDAQTRRRERRAEKQAQWKAANPPLDGAGAGAGA
GAGAGGLATMDAQTRRRERRAEKQAQWKAANPPLDGAGAGAGAGAGAGGLATMDAQTR
RRERRAEKQAQWKAANPPLDGAGAGAGAGAGAGGLATMDAQTRRRERRAEKQAQWKAA
NPPLESRGPV (SEQ ID NO:112)

TOMM20-EWSR1-Myc-λ_{N22}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAGTTCTTCATGGCGTCCACGGATTACAGTACC
TATAGCCAAGCTGCAGCGCAGCAGGGCTACAGTGCTTACACCGCCCAGCCCACTCAAG
GATATGCACAGACCACCCAGGCATATGGGCAACAAAGCTATGGAACCTATGGACAGCC
CACTGATGTCAGCTATACCCAGGCTCAGACCACTGCAACCTATGGGCAGACCGCCTAT
GCAACTTCTTATGGACAGCCTCCCACTGGTTATACTACTCCAACTGCCCCCCAGGCATA
CAGCCAGCCTGTCCAGGGGTATGGCACTGGTGCTTATGATACCACCACTGCTACAGTC
ACCACCACCCAGGCCTCCTATGCAGCTCAGTCTGCATATGGCACTCAGCCTGCTTATCC
AGCCTATGGGCAGCAGCCAGCAGCCACTGCACCTACAAGACCGCAGGATGGAAACAAG
CCCACTGAGACTAGTCAACCTCAATCTAGCACAGGGGGTTACAACCAGCCCAGCCTAG
GATATGGACAGAGTAACTACAGTTATCCCCAGGTACCTGGGAGCTACCCCATGCAGCCA
GTCACTGCACCTCCATCCTACCCTCCTACCAGCTATTCCTCTACACAGCCGACTAGTTAT
 
GATCAGAGCAGTTACTCTCAGCAGAACACCTATGGGCAACCGAGCAGCTATGGACAGC
AGAGTAGCTATGGTCAACAAAGCAGCTATGGGCAGCAGCCTCCCACTAGTTACCCACC
CCAAACTGGATCCTACAGCCAAGCTCCAAGTCAATATAGCCAACAGAGCAGCAGCTACG
GGCAGCAGAGTTCATTCCGACAGGACCACCCCAGTAGCATGGGTGTTTATGGGCAGGA
GTCTGGAGGATTTTCCGGACCAGGAGAGAACCGGAGCATGAGTGGCCCTGATAACCGG
GGCAGGGGAAGAGGGGGATTTGATCGTGGAGGCATGAGCAGAGGTGGCGGGGAGG
AGGACGCGGTGGAATGGGCAGCGCTGGAGAGCGAGGTGGCTTCAATAAGCCTGGTGG
ACCCATGGATGAAGGACCAGATCTTGATCTAGGCCCACCTGTAGATCCAGATGAAGACT
CTGACAACAGTGCAATTTATGTACAAGGATTAAATGACAGTGTGACTCTAGATGATCTGG
CAGACTTCTTTAAGCAGTGTGGGGTTGTTAAGATGAACAAGAGAACTGGGCAACCCATG
ATCCACATCTACCTGGACAAGGAAACAGGAAAGCCCAAAGGCGATGCCACAGTGTCCT
ATGAAGACCCACCTACTGCCAAGGCTGCCGTGGAATGGTTTGATGGGAAAGATTTTCAA
GGGAGCAAACTTAAAGTCTCCCTTGCTCGGAAGAAGCCTCCAATGAACAGTATGCGGG
GTGGTCTGCCACCCCGTGAGGGCAGAGGCATGCCACCACCACTCCGTGGAGGTCCAG
GAGGCCCAGGAGGTCCTGGGGGACCCATGGGTCGCATGGGAGGCCGTGGAGGAGAT
AGAGGAGGCTTCCCTCCAAGAGGACCCCGGGGTTCCCGAGGGAACCCCTCTGGAGGA
GGAAACGTCCAGCACCGAGCTGGAGACTGGCAGTGTCCCAATCCGGGTTGTGGAAACC
AGAACTTCGCCTGGAGAACAGAGTGCAACCAGTGTAAGGCCCCAAAGCCTGAAGGCTT
CCTCCCGCCACCCTTTCCGCCCCCGGGTGGTGATCGTGGCAGAGGTGGCCCTGGTGG
CATGCGGGGAGGAAGAGGTGGCCTCATGGATCGTGGTGGTCCCGGTGGAATGTTCAG
AGGTGGCCGTGGTGGAGACAGAGGTGGCTTCCGTGGTGGCCGGGGCATGGACCGAG
GTGGCTTTGGTGGAGGAAGACGAGGTGGCCCTGGGGGGCCCCCTGGACCTTTGATGG
AACAGGCGATCGCAGGAGCACCAGGAAGTGCTGGTTCTGCTGCTGGTAGTGGAGAGCA
GAAGCTGATCTCAGAGGAGGACCTGCTAGCCACCATGGACGCACAAACACGACGACGT
GAGCGTCGCGCTGAGAAACAAGCTCAATGGAAAGCTGCAAACCCACCGCTCGACGGAG
CCGGAGCTGGCGCTGGAGCTGGAGCCGGAGCTGGCGGTCTAGCCACCATGGACGCAC
AAACACGACGACGTGAGCGTCGCGCTGAGAAACAAGCTCAATGGAAAGCTGCAAACCC
ACCGCTCGACGGAGCCGGAGCTGGCGCTGGAGCTGGAGCCGGAGCTGGCGGTCTAG
CCACCATGGACGCACAAACACGACGACGTGAGCGTCGCGCTGAGAAACAAGCTCAATG
GAAAGCTGCAAACCCACCGCTCGACGGAGCCGGAGCTGGCGCTGGAGCTGGAGCCGG
AGCTGGCGGTCTAGCCACCATGGACGCACAAACACGACGACGTGAGCGTCGCGCTGA
GAAACAAGCTCAATGGAAAGCTGCAAACCCACCGCTCGAGTCTAGAGGGCCCGTTTAA
(SEQ ID NO:113)
Protein:

MVGRNSAIAAGVCGALFIGYCIYFDRKRRSDPNFKNRLRERRKKQKLAKERAGLSKLPDLKD
AEAVQKFFMASTDYSTYSQAAAQQGYSAYTAQPTQGYAQTTQAYGQQSYGTYGQPTDVS

YTQAQTTATYGQTAYATSYGQPPTGYTTPTAPQAYSQPVQGYGTGAYDTTTATVTTTQAS
YAAQSAYGTQPAYPAYGQQPAATAPTRPQDGNKPTETSQPQSSTGGYNQPSLGYGQSNY
SYPQVPGSYPMQPVTAPPSYPPTSYSSTQPTSYDQSSYSQQNTYGQPSSYGQQSSYGQQ
SSYGQQPPTSYPPQTGSYSQAPSQYSQQSSSYGQQSSFRQDHPSSMGVYGQESGGFSG
PGENRSMSGPDNRGRGRGGFDRGGMSRGGRGGGRGGMGSAGERGGFNKPGGPMDEG
PDLDLGPPVDPDEDSDNSAIYVQGLNDSVTLDDLADFFKQCGVVKMNKRTGQPMIHIYLDK
ETGKPKGDATVSYEDPPTAKAAVEWFDGKDFQGSKLKVSLARKKPPMNSMRGGLPPREG
RGMPPPLRGGPGGPGGPGGPMGRMGGRGGDRGGFPPRGPRGSRGNPSGGGNVQHRA
GDWQCPNPGCGNQNFAWRTECNQCKAPKPEGFLPPPFPPPGGDRGRGGPGGMRGGRG
GLMDRGGPGGMFRGGRGGDRGGFRGGRGMDRGGFGGGRRGGPGGPPGPLMEQAIAG
APGSAGSAAGSGEQKLISEEDLLATMDAQTRRRERRAEKQAQWKAANPPLDGAGAGAGA
GAGAGGLATMDAQTRRRERRAEKQAQWKAANPPLDGAGAGAGAGAGAGGLATMDAQTR
RRERRAEKQAQWKAANPPLDGAGAGAGAGAGAGGLATMDAQTRRRERRAEKQAQWKAA
NPPLESRGPV (SEQ ID NO:114)

TOMM20-3xMCP-PyIRS^{AF}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAGTTCTTCTATACAGATATTGAAATGAACAGAT
TGGGAAAGGAGCAGAAGCTGATCTCAGAGGAGGACCTGGGCGCCCCCGGCTCCGCCG
GCTCCGCCGCCGGCTCCGGCGCTTCTAACTTTACTCAGTTCGTTCTCGTCGACAATGGC
GGAACTGGCGACGTGACTGTCGCCCCAAGCAACTTCGCTAACGGGATCGCTGAATGGA
TCAGCTCTAACTCGCGTTCACAGGCTTACAAAGTAACCTGTAGCGTTCGTCAGAGCTCT
GCGCAGAATCGCAAATACACCATCAAAGTCGAGGTGCCTAAAGGCGCCTGGCGTTCGT
ACTTAAATATGGAACTAACCATTCCAATTTTCGCCACGAATTCCGACTGCGAGCTTATTG
TTAAGGCAATGCAAGGTCTCCTAAAAGATGGAAACCCGATTCCCTCAGCAATCGCAGCA
AACTCCGGCATCTACGGTGCCCCAGGCTCCGCAGGAAGCGCAGCGGGGTCCGGAGCT
TCTAACTTTACTCAGTTCGTTCTCGTCGACAATGGCGGAACTGGCGACGTGACTGTCGC
CCCAAGCAACTTCGCTAACGGGATCGCTGAATGGATCAGCTCTAACTCGCGTTCACAGG
CTTACAAAGTAACCTGTAGCGTTCGTCAGAGCTCTGCGCAGAATCGCAAATACACCATC
AAAGTCGAGGTGCCTAAAGGCGCCTGGCGTTCGTACTTAAATATGGAACTAACCATTCC
AATTTTCGCCACGAATTCCGACTGCGAGCTTATTGTTAAGGCAATGCAAGGTCTCCTAAA
AGATGGAAACCCGATTCCCTCAGCAATCGCAGCAAACTCCGGCATCTACGGCGCACCT
GGTAGTGCTGGTTCTGCTGCTGGATCAGGTGCTTCTAACTTTACTCAGTTCGTTCTCGT
 
CGACAATGGCGGAACTGGCGACGTGACTGTCGCCCCAAGCAACTTCGCTAACGGATC
GCTGAATGGATCAGCTCTAACTCGCGTTCACAGGCTTACAAAGTAACCTGTAGCGTTCG
TCAGAGCTCTGCGCAGAATCGCAAATACACCATCAAAGTCGAGGTGCCTAAAGGCGCC
TGGCGTTCGTACTTAAATATGGAACTAACCATTCCAATTTTCGCCACGAATTCCGACTGC
GAGCTTATTGTTAAGGCAATGCAAGGTCTCCTAAAAGATGGAAACCCGATTCCCTCAGC
AATCGCAGCAAACTCCGGCATCTACGGTACCGGCGCCCCCGGCTCCGCCGGCTCCGC
CGCCGGCTCCGGCGCGTGCCCGGTGCCGCTGCAGCTGCCGCCGCTGGAACGCCTGA
CCCTGGATGATAAAAAACCGCTGAATACCCTGATCTCTGCTACTGGTCTGTGGATGAGT
CGTACCGGAACCATTCATAAAATCAAACACCACGAGGTTAGCCGTTCGAAAATCTATATT
GAGATGGCGTGTGGCGATCATCTGGTTGTGAACAATAGCCGCTCTTCTCGTACAGCAC
GTGCACTGCGTCACCACAAATATCGTAAAACCTGTAAACGTTGCCGTGTGTCCGATGAG
GATCTGAACAAATTCCTGACAAAAGCCAATGAGGACCAAACAAGCGTGAAAGTGAAAGT
CGTTAGCGCTCCTACCCGTACTAAAAAAGCAATGCCGAAATCCGTTGCTCGTGCCCCTA
AACCACTGGAAAACACTGAAGCAGCACAGGCACAGCCGTCTGGAAGCAAATTCTCTCC
GGCCATTCCTGTTTCTACCCAGGAGTCCGTTTCTGTTCCAGCAAGTGTGAGCACCAGCA
TTAGCAGTATTAGCACCGGTGCCACCGCTAGCGCCCTGGTTAAAGGCAATACCAATCCG
ATTACAAGCATGTCTGCCCCGGTTCAAGCATCAGCTCCAGCACTGACAAAATCCCAAAC
CGATCGTCTGGAGGTTCTGCTGAATCCGAAAGACGAAATCAGCCTGAATTCCGGCAAAC
CGTTTCGTGAACTGGAGAGCGAACTGCTGTCACGTCGTAAAAAAGACCTGCAACAAATC
TATGCCGAAGAACGTGAGAACTATCTGGGGAAACTGGAACGTGAAATCACCCGCTTTTT
CGTGGATCGTGGCTTTCTGGAGATCAAATCCCCGATTCTGATTCCTCTGGAGTATATCG
AGCGTATGGGCATCGACAATGATACCGAACTGAGCAAACAAATTTTCCGTGTGGATAAA
AACTTCTGTCTGCGCCCTATGCTAGCACCAAATCTGGCTAACTATCTGCGCAAACTGGA
CCGTGCCCTGCCTGATCCTATCAAAATCTTCGAGATCGGCCCGTGTTATCGTAAAGAGT
CCGACGGTAAAGAACATCTGGAGGAGTTTACCATGCTGAACTTTTGCCAAATGGGTTCA
GGTTGTACTCGTGAGAACCTGGAAAGCATCATCACCGATTTTCTGAACCACCTGGGCAT
TGACTTCAAAATTGTGGGCGACAGCTGTATGGTGTTTGGCGACACCCTGGATGTCATGC
ACGGCGACCTGGAACTGTCTAGTGCCGTTGTGGGCCCAATCCCGCTGGATCGTGAGTG
GGGTATCGACAAACCTTGGATCGGTGCGGGTTTTGGTCTGGAGCGTCTGCTGAAAGTA
AAACACGACTTCAAGAACATCAAACGTGCTGCACGTTCCGAGTCCTATTACAATGGTATT
TCTACTAACCTGTAA (SEQ ID NO:115)
Protein:

MVGRNSAIAAGVCGALFIGYCIYFDRKRRSDPNFKNRLRERRKKQKLAKERAGLSKLPDLKD
AEAVQKFFYTDIEMNRLGKEQKLISEEDLGAPGSAGSAAGSGASNFTQFVLVDNGGTGDVT
VAPSNFANGIAEWISSNSRSQAYKVTCSVRQSSAQNRKYTIKVEVPKGAWRSYLNMELTIPI
FATNSDCELIVKAMQGLLKDGNPIPSAIAANSGIYGAPGSAGSAAGSGASNFTQFVLVDNGG

TGDVTVAPSNFANGIAEWISSNSRSQAYKVTCSVRQSSAQNRKYTIKVEVPKGAWRSYLNM
ELTIPIFATNSDCELIVKAMQGLLKDGNPIPSAIAANSGIYGAPGSAGSAAGSGASNFTQFVLV
DNGGTGDVTVAPSNFANGIAEWISSNSRSQAYKVTCSVRQSSAQNRKYTIKVEVPKGAWR
SYLNMELTIPIFATNSDCELIVKAMQGLLKDGNPIPSAIAANSGIYGTGAPGSAGSAAGSGAC
PVPLQLPPLERLTLDDKKPLNTLISATGLWMSRTGTIHKIKHHEVSRSKIYIEMACGDHLVVN
NSRSSRTARALRHHKYRKTCKRCRVSDEDLNKFLTKANEDQTSVKVKVVSAPTRTKKAMPK
SVARAPKPLENTEAAQAQPSGSKFSPAIPVSTQESVSVPASVSTSISSISTGATASALVKGNT
NPITSMSAPVQASAPALTKSQTDRLEVLLNPKDEISLNSGKPFRELESELLSRRKKDLQQIYA
EERENYLGKLEREITRFFVDRGFLEIKSPILIPLEYIERMGIDNDTELSKQIFRVDKNFCLRPML
APNLANYLRKLDRALPDPIKIFEIGPCYRKESDGKEHLEEFTMLNFCQMGSGCTRENLESIIT
DFLNHLGIDFKIVGDSCMVFGDTLDVMHGDLELSSAVVGPIPLDREWGIDKPWIGAGFGLER
LLKVKHDFKNIKRAARSESYYNGISTNL (SEQ ID NO:116)

TOMM20-FUS-3xMCP-PyIRS^{AF}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAATTCTTCATGGCCTCAAACGATTATACCCAAC
AAGCAACCCAAAGCTATGGGGCCTACCCCACCCAGCCCGGGCAGGGCTATTCCCAGCA
GAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGG
AACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTC
CCAGCAACACCTCGGGAAGTTACGGTAGCAGTTCTCAGAGCAGCAGCTATGGGCAGCC
CCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCA
GCAGTGGTGGTGGAGGTGGAGGTGGAGGTGGAGGTAACTATGGCCAAGATCAATCCTC
CATGAGTAGTGGTGGTGGCAGTGGTGGCGGTTATGGCAATCAAGACCAGAGTGGTGGA
GGTGGCAGCGGTGGCTATGGACAGCAGGACCGTGGAGGCCGCGGCAGGGGTGGCAG
TGGTGGCGGCGGCGGCGGCGGTGGTGGTTACAACCGCAGCAGTGGTGGCTATGA
ACCCAGAGGTCGTGGAGGTGGCCGTGGAGGCAGAGGTGGCATGGGCGGAAGTGACC
GTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAAT
TGAGTCTGTGGCTGATTACTTCAAGCAGATTGGTATTATTAAGACAAACAAGAAAACGGG
ACAGCCCATGATTAATTTGTACACAGACAGGGAAACTGGCAAGCTGAAGGGAGAGGCA
 
ACGGTCTCTTTTGATGACCCACCTTCAGCTAAAGCAGCTATTGACTGGTTTGATGGTAAA
GAATTCTCCGGAAATCCTATCAAGGTCTCATTTGCTACTCGCCGGGCAGACTTTAATCG
GGGTGGTGGCAATGGTCGTGGAGGCCGAGGGCGAGGAGGACCCATGGGCCGTGGAG
GCTATGGAGGTGGTGGCAGTGGTGGTGGTGGCCGAGGAGGATTTCCCAGTGGAGGTG
GTGGCGGTGGAGGACAGCAGCGAGCTGGTGACTGGAAGTGTCCTAATCCCACCTGTGA
GAATATGAACTTCTCTTGGAGGAATGAATGCAACCAGTGTAAGGCCCCTAAACCAGATG
GCCCAGGAGGGGGACCAGGTGGCTCTCACATGGGGGGTAACTACGGGGATGATCGTC
GTGGTGGCAGAGGAGGCGAGCAGAAGCTGATCTCAGAGGAGGACCTGGCGCCCCCG
GCTCCGCCGGCTCCGCCGCCGGCTCCGGCGCTTCTAACTTTACTCAGTTCGTTCTCGT
CGACAATGGCGGAACTGGCGACGTGACTGTCGCCCCAAGCAACTTCGCTAACGGGATC
GCTGAATGGATCAGCTCTAACTCGCGTTCACAGGCTTACAAAGTAACCTGTAGCGTTCG
TCAGAGCTCTGCGCAGAATCGCAAATACACCATCAAAGTCGAGGTGCCTAAAGGCGCC
TGGCGTTCGTACTTAAATATGGAACTAACCATTCCAATTTTCGCCACGAATTCCGACTGC
GAGCTTATTGTTAAGGCAATGCAAGGTCTCCTAAAAGATGGAAACCCGATTCCCTCAGC
AATCGCAGCAAACTCCGGCATCTACGGTGCCCCAGGCTCCGCAGGAAGCGCAGCGGG
GTCCGGAGCTTCTAACTTTACTCAGTTCGTTCTCGTCGACAATGGCGGAACTGGCGACG
TGACTGTCGCCCCAAGCAACTTCGCTAACGGGATCGCTGAATGGATCAGCTCTAACTCG
CGTTCACAGGCTTACAAAGTAACCTGTAGCGTTCGTCAGAGCTCTGCGCAGAATCGCAA
ATACACCATCAAAGTCGAGGTGCCTAAAGGCGCCTGGCGTTCGTACTTAAATATGGAAC
TAACCATTCCAATTTTCGCCACGAATTCCGACTGCGAGCTTATTGTTAAGGCAATGCAAG
GTCTCCTAAAAGATGGAAACCCGATTCCCTCAGCAATCGCAGCAAACTCCGGCATCTAC
GGCGCACCTGGTAGTGCTGGTTCTGCTGCTGGATCAGGTGCTTCTAACTTTACTCAGTT
CGTTCTCGTCGACAATGGCGGAACTGGCGACGTGACTGTCGCCCCAAGCAACTTCGCT
AACGGGATCGCTGAATGGATCAGCTCTAACTCGCGTTCACAGGCTTACAAAGTAACCTG
TAGCGTTCGTCAGAGCTCTGCGCAGAATCGCAAATACACCATCAAAGTCGAGGTGCCTA
AAGGCGCCTGGCGTTCGTACTTAAATATGGAACTAACCATTCCAATTTTCGCCACGAATT
CCGACTGCGAGCTTATTGTTAAGGCAATGCAAGGTCTCCTAAAAGATGGAAACCCGATT
CCCTCAGCAATCGCAGCAAACTCCGGCATCTACGGTACCGGCGCCCCCGGCTCCGCC
GGCTCCGCCGCCGGCTCCGGCGCGTGCCCGGTGCCGCTGCAGCTGCCGCCGCTGGA
ACGCCTGACCCTGGATGATAAAAAACCGCTGAATACCCTGATCTCTGCTACTGGTCTGT
GGATGAGTCGTACCGGAACCATTCATAAAATCAAACACCACGAGGTTAGCCGTTCGAAA
ATCTATATTGAGATGGCGTGTGGCGATCATCTGGTTGTGAACAATAGCCGCTCTTCTCG
TACAGCACGTGCACTGCGTCACCACAAATATCGTAAAACCTGTAAACGTTGCCGTGTGT
CCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCG
TGCCCCTAAACCACTGGAAAACACTGAAGCAGCACAGGCACAGCCGTCTGGAAGCAAA
 
TTCTCTCCGGCCATTCCTGTTTCTACCCAGGAGTCCGTTTCTGTTCCAGCAAGTGTGAG
CACCAGCATTAGCAGTATTAGCACCGGTGCCACCGCTAGCGCCCTGGTTAAAGGCAAT
ACCAATCCGATTACAAGCATGTCTGCCCCGGTTCAAGCATCAGCTCCAGCACTGACAAA
ATCCCAAACCGATCGTCTGGAGGTTCTGCTGAATCCGAAAGACGAAATCAGCCTGAATT
CCGGCAAACCGTTTCGTGAACTGGAGAGCGAACTGCTGTCACGTCGTAAAAAGACCT
GCAACAAATCTATGCCGAAGAACGTGAGAACTATCTGGGGAAACTGGAACGTGAAATCA
CCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCG
TGTGGATAAAAACTTCTGTCTGCGCCCTATGCTAGCACCAAATCTGGCTAACTATCTGC
GCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGAACTTTTGCCA
AATGGGTTCAGGTTGTACTCGTGAGAACCTGGAAAGCATCATCACCGATTTTCTGAACC
ACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGG
ATCGTGAGTGGGGTATCGACAAACCTTGGATCGGTGCGGGTTTTGGTCTGGAGCGTCT
GCTGAAAGTAAAACACGACTTCAAGAACATCAAACGTGCTGCACGTTCCGAGTCCTATT
ACAATGGTATTTCTACTAACCTGTAA (SEQ ID NO:117)
Protein:

MVGRNSAIAAGVCGALFIGYCIYFDRKRRSDPNFKNRLRERRKKQKLAKERAGLSKLPDLKD
AEAVQKFFMASNDYTQQATQSYGAYPTQPGQGYSQQSSQPYGQQSYSGYSQSTDTSGY
GQSSYSSYGQSQNTGYGTQSTPQGYGSTGGYGSSQSSQSSYGQQSSYPGYGQQPAPSN
TSGSYGSSSQSSSYGQPQSGSYSQQPSYGGQQQSYGQQQSYNPPQGYGQQNQYNSSS
GGGGGGGGGGNYGQDQSSMSSGGGSGGGYGNQDQSGGGGSGGYGQQDRGGRGRG
GSGGGGGGGGGYNRSSGGYEPRGRGGGRGGRGGMGGSDRGGFNKFGGPRDQGSR
HDSEQDNSDNNTIFVQGLGENVTIESVADYFKQIGIIKTNKKTGQPMINLYTDRETGKLKGEA
TVSFDDPPSAKAAIDWFDGKEFSGNPIKVSFATRRADFNRGGGNGRGGRGRGGPMGRGG
YGGGGSGGGGRGGFPSGGGGGGGQQRAGDWKCPNPTCENMNFSWRNECNQCKAPKP
DGPGGGPGGSHMGGNYGDDRRGGRGGEQKLISEEDLGAPGSAGSAAGSGASNFTQFVL
VDNGGTGDVTVAPSNFANGIAEWISSNSRSQAYKVTCSVRQSSAQNRKYTIKVEVPKGAW
RSYLNMELTIPIFATNSDCELIVKAMQGLLKDGNPIPSAIAANSGIYGAPGSAGSAAGSGASN
FTQFVLVDNGGTGDVTVAPSNFANGIAEWISSNSRSQAYKVTCSVRQSSAQNRKYTIKVEV
PKGAWRSYLNMELTIPIFATNSDCELIVKAMQGLLKDGNPIPSAIAANSGIYGAPGSAGSAAG
SGASNFTQFVLVDNGGTGDVTVAPSNFANGIAEWISSNSRSQAYKVTCSVRQSSAQNRKY
TIKVEVPKGAWRSYLNMELTIPIFATNSDCELIVKAMQGLLKDGNPIPSAIAANSGIYGTGAPG
SAGSAAGSGACPVPLQLPPLERLTLDDKKPLNTLISATGLWMSRTGTIHKIKHHEVSRSKIYI
EMACGDHLVVNNSRSSRTARALRHHKYRKTCKRCRVSDEDLNKFLTKANEDQTSVKVKVV

SAPTRTKKAMPKSVARAPKPLENTEAAQAQPSGSKFSPAIPVSTQESVSVPASVSTSISSIST
GATASALVKGNTNPITSMSAPVQASAPALTKSQTDRLEVLLNPKDEISLNSGKPFRELESELL
SRRKKDLQQIYAEERENYLGKLEREITRFFVDRGFLEIKSPILIPLEYIERMGIDNDTELSKQIF
RVDKNFCLRPMLAPNLANYLRKLDRALPDPIKIFEIGPCYRKESDGKEHLEEFTMLNFCQMG
SGCTRENLESIITDFLNHLGIDFKIVGDSCMVFGDTLDVMHGDLELSSAVVGPIPLDREWGID
KPWIGAGFGLERLLKVKHDFKNIKRAARSESYYNGISTNL (SEQ ID NO:118)

TOMM20-FUS-3xMCP-PyIRS^{AAAF}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAATTCTTCATGGCCTCAAACGATTATACCCAAC
AAGCAACCCAAAGCTATGGGGCCTACCCCACCCAGCCCGGGCAGGGCTATTCCCAGCA
GAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGG
AACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTC
CCAGCAACACCTCGGGAAGTTACGGTAGCAGTTCTCAGAGCAGCAGCTATGGGCAGCC
CCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCA
GCAGTGGTGGTGGAGGTGGAGGTGGAGGTGGAGGTAACTATGGCCAAGATCAATCCTC
CATGAGTAGTGGTGGTGGCAGTGGTGGCGGTTATGGCAATCAAGACCAGAGTGGTGGA
GGTGGCAGCGGTGGCTATGGACAGCAGGACCGTGGAGGCCGCGGCAGGGGTGGCAG
TGGTGGCGGCGGCGGCGGCGGTGGTGGTTACAACCGCAGCAGTGGTGGCTATGA
ACCCAGAGGTCGTGGAGGTGGCCGTGGAGGCAGAGGTGGCATGGGCGGAAGTGACC
GTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAAT
TGAGTCTGTGGCTGATTACTTCAAGCAGATTGGTATTATTAAGACAAACAAGAAAACGGG
ACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAA
GAATTCTCCGGAAATCCTATCAAGGTCTCATTTGCTACTCGCCGGGCAGACTTTAATCG
GGGTGGTGGCAATGGTCGTGGAGGCCGAGGGCGAGGAGGACCCATGGGCCGTGGAG
GCTATGGAGGTGGTGGCAGTGGTGGTGGTGGCCGAGGAGGATTTCCCAGTGGAGGTG
GTGGCGGTGGAGGACAGCAGCGAGCTGGTGACTGGAAGTGTCCTAATCCCACCTGTGA
GAATATGAACTTCTCTTGGAGGAATGAATGCAACCAGTGTAAGGCCCCTAAACCAGATG
 
GCCCAGGAGGGGACCAGGTGGCTCTCACATGGGGGTAACTACGGGATGATCGTC
GTGGTGGCAGAGGAGGCGAGCAGAAGCTGATCTCAGAGGAGGACCTGGGCGCCCCCG
GCTCCGCCGGCTCCGCCGCCGGCTCCGGCGCTTCTAACTTTACTCAGTTCGTTCTCGT
CGACAATGGCGGAACTGGCGACGTGACTGTCGCCCCAAGCAACTTCGCTAACGGATC
GCTGAATGGATCAGCTCTAACTCGCGTTCACAGGCTTACAAAGTAACCTGTAGCGTTCG
TCAGAGCTCTGCGCAGAATCGCAAATACACCATCAAAGTCGAGGTGCCTAAAGGCGCC
TGGCGTTCGTACTTAAATATGGAACTAACCATTCCAATTTTCGCCACGAATTCCGACTGC
GAGCTTATTGTTAAGGCAATGCAAGGTCTCCTAAAAGATGGAAACCCGATTCCCTCAGC
AATCGCAGCAAACTCCGGCATCTACGGTGCCCCAGGCTCCGCAGGAAGCGCAGCGGG
GTCCGGAGCTTCTAACTTTACTCAGTTCGTTCTCGTCGACAATGGCGGAACTGGCGACG
TGACTGTCGCCCCAAGCAACTTCGCTAACGGATCGCTGAATGGATCAGCTCTAACTCG
CGTTCACAGGCTTACAAAGTAACCTGTAGCGTTCGTCAGAGCTCTGCGCAGAATCGCAA
ATACACCATCAAAGTCGAGGTGCCTAAAGGCGCCTGGCGTTCGTACTTAAATATGGAAC
TAACCATTCCAATTTTCGCCACGAATTCCGACTGCGAGCTTATTGTTAAGGCAATGCAAG
GTCTCCTAAAAGATGGAAACCCGATTCCCTCAGCAATCGCAGCAAACTCCGGCATCTAC
GGCGCACCTGGTAGTGCTGGTTCTGCTGCTGGATCAGGTGCTTCTAACTTTACTCAGTT
CGTTCTCGTCGACAATGGCGGAACTGGCGACGTGACTGTCGCCCCAAGCAACTTCGCT
AACGGATCGCTGAATGGATCAGCTCTAACTCGCGTTCACAGGCTTACAAAGTAACCTG
TAGCGTTCGTCAGAGCTCTGCGCAGAATCGCAAATACACCATCAAAGTCGAGGTGCCTA
AAGGCGCCTGGCGTTCGTACTTAAATATGGAACTAACCATTCCAATTTTCGCCACGAATT
CCGACTGCGAGCTTATTGTTAAGGCAATGCAAGGTCTCCTAAAAGATGGAAACCCGATT
CCCTCAGCAATCGCAGCAAACTCCGGCATCTACGGTACCGGCGCCCCCGGCTCCGCC
GGCTCCGCCGCCGGCTCCGGCGCGTGCCCGGTGCCGCTGCAGCTGCCGCCGCTGGA
ACGCCTGACCCTGGATGATAAAAAACCGCTGAATACCCTGATCTCTGCTACTGGTCTGT
GGATGAGTCGTACCGGAACCATTCATAAAATCAAACACCACGAGGTTAGCCGTTCGAAA
ATCTATATTGAGATGGCGTGTGGCGATCATCTGGTTGTGAACAATAGCCGCTCTTCTCG
TACAGCACGTGCACTGCGTCACCACAAATATCGTAAAACCTGTAAACGTTGCCGTGTGT
CCGATGAGGATCTGAACAAATTCCTGACAAAAGCCAATGAGGACCAAACAAGCGTGAAA
GTGAAAGTCGTTAGCGCTCCTACCCGTACTAAAAAAGCAATGCCGAAATCCGTTGCTCG
TGCCCCTAAACCACTGGAAAACACTGAAGCAGCACAGGCACAGCCGTCTGGAAGCAAA
TTCTCTCCGGCCATTCCTGTTTCTACCCAGGAGTCCGTTTCTGTTCCAGCAAGTGTGAG
CACCAGCATTAGCAGTATTAGCACCGGTGCCACCGCTAGCGCCCTGGTTAAAGGCAAT
ACCAATCCGATTACAAGCATGTCTGCCCCGGTTCAAGCATCAGCTCCAGCACTGACAAA
ATCCCAAACCGATCGTCTGGAGGTTCTGCTGAATCCGAAAGACGAAATCAGCCTGAATT
CCGGCAAACCGTTTCGTGAACTGGAGAGCGAACTGCTGTCACGTCGTAAAAAAGACCT
GCAACAAATCTATGCCGAAGAACGTGAGAACTATCTGGGGAAACTGGAACGTGAAATCA
 
CCCGCTTTTTCGTGGATCGTGGCTTTCTGGAGATCAAATCCCCGATTCTGATTCCTCTG
GAGTATATCGAGCGTATGGGCATCGACAATGATACCGAACTGAGCAAACAAATTTTCCG
TGTGGATAAAAACTTCTGTCTGCGCCCTATGCTAGCACCAAATCTGGCTAACTATCTGC
GCAAACTGGACCGTGCCCTGCCTGATCCTATCAAAATCTTCGAGATCGGCCCGTGTTAT
CGTAAAGAGTCCGACGGTAAAGAACATCTGGAGGAGTTTACCATGCTGGCCTTTGCCCA
AATGGGTTCAGGTTGTACTCGTGAGAACCTGGAAAGCATCATCACCGATTTTCTGAACC
ACCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGG
ATCGTGAGTGGGGTATCGACAAACCTTGGATCGGTGCGGGTTTTGGTCTGGAGCGTCT
GCTGAAAGTAAAACACGACTTCAAGAACATCAAACGTGCTGCACGTTCCGAGTCCTATT
ACAATGGTATTTCTACTAACCTGTAA (SEQ ID NO:119)
Protein:

MVGRNSAIAAGVCGALFIGYCIYFDRKRRSDPNFKNRLRERRKKQKLAKERAGLSKLPDLKD
AEAVQKFFMASNDYTQQATQSYGAYPTQPGQGYSQQSSQPYGQQSYSGYSQSTDTSGY
GQSSYSSYGQSQNTGYGTQSTPQGYGSTGGYGSSQSSQSSYGQQSSYPGYGQQPAPSN
TSGSYGSSSQSSSYGQPQSGSYSQQPSYGGQQQSYGQQQSYNPPQGYGQQNQYNSSS
GGGGGGGGGGNYGQDQSSMSSGGGSGGGYGNQDQSGGGGSGGYGQQDRGGRGRG
GSGGGGGGGGGGYNRSSGGYEPRGRGGGRGGRGGMGGSDRGGFNKFGGPRDQGSR
HDSEQDNSDNNTIFVQGLGENVTIESVADYFKQIGIIKTNKKTGQPMINLYTDRETGKLKGEA
TVSFDDPPSAKAAIDWFDGKEFSGNPIKVSFATRRADFNRGGGNGRGGRGRGGPMGRGG
YGGGGSGGGGRGGFPSGGGGGGGQQRAGDWKCPNPTCENMNFSWRNECNQCKAPKP
DGPGGGPGGSHMGGNYGDDRRGGRGGEQKLISEEDLGAPGSAGSAAGSGASNFTQFVL
VDNGGTGDVTVAPSNFANGIAEWISSNSRSQAYKVTCSVRQSSAQNRKYTIKVEVPKGAW
RSYLNMELTIPIFATNSDCELIVKAMQGLLKDGNPIPSAIAANSGIYGAPGSAGSAAGSGASN
FTQFVLVDNGGTGDVTVAPSNFANGIAEWISSNSRSQAYKVTCSVRQSSAQNRKYTIKVEV
PKGAWRSYLNMELTIPIFATNSDCELIVKAMQGLLKDGNPIPSAIAANSGIYGAPGSAGSAAG
SGASNFTQFVLVDNGGTGDVTVAPSNFANGIAEWISSNSRSQAYKVTCSVRQSSAQNRKY
TIKVEVPKGAWRSYLNMELTIPIFATNSDCELIVKAMQGLLKDGNPIPSAIAANSGIYGTGAPG
SAGSAAGSGACPVPLQLPPLERLTLDDKKPLNTLISATGLWMSRTGTIHKIKHHEVSRSKIYI
EMACGDHLVVNNSRSSRTARALRHHKYRKTCKRCRVSDEDLNKFLTKANEDQTSVKVKVV
SAPTRTKKAMPKSVARAPKPLENTEAAQAQPSGSKFSPAIPVSTQESVSVPASVSTSISSIST
GATASALVKGNTNPITSMSAPVQASAPALTKSQTDRLEVLLNPKDEISLNSGKPFRELESELL
SRRKKDLQQIYAEERENYLGKLEREITRFFVDRGFLEIKSPILIPLEYIERMGIDNDTELSKQIF
RVDKNFCLRPMLAPNLANYLRKLDRALPDPIKIFEIGPCYRKESDGKEHLEEFTMLAFAQMG
SGCTRENLESIITDFLNHLGIDFKIVGDSCMVFGDTLDVMHGDLELSSAVVGPIPLDREWGID
KPWIGAGFGLERLLKVKHDFKNIKRAARSESYYNGISTNL (SEQ ID NO:120)

TOMM20-FUS-4xλ_{N22}-PyIRS^{AF}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAATTCTTCATGGCCTCAAACGATTATACCCAAC
AAGCAACCCAAAGCTATGGGGCCTACCCCACCCAGCCCGGGCAGGGCTATTCCCAGCA
GAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGG
AACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTC
CCAGCAACACCTCGGGAAGTTACGGTAGCAGTTCTCAGAGCAGCAGCTATGGCAGCC
CCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCA
GCAGTGGTGGTGGAGGTGGAGGTGGAGGTGGAGGTAACTATGGCCAAGATCAATCCTC
CATGAGTAGTGGTGGTGGCAGTGGTGGCGGTTATGGCAATCAAGACCAGAGTGGTGGA
GGTGGCAGCGGTGGCTATGGACAGCAGGACCGTGGAGGCCGCGGCAGGGGTGGCAG
TGGTGGCGGCGGCGGCGGCGGTGGTGGTTACAACCGCAGCAGTGGTGGCTATGA
ACCCAGAGGTCGTGGAGGTGGCCGTGGAGGCAGAGGTGGCATGGGCGGAAGTGACC
GTGGTGGCTTCAATAAATTTGGTGGCCCTCGGGACCAAGGATCACGTCATGACTCCGAA
CAGGATAATTCAGACAACAACACCATCTTTGTGCAAGGCCTGGGTGAGAATGTTACAAT
TGAGTCTGTGGCTGATTACTTCAAGCAGATTGGTATTATTAAGACAAACAAGAAAACGGG
ACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAA
GAATTCTCCGGAAATCCTATCAAGGTCTCATTTGCTACTCGCCGGGCAGACTTTAATCG
GGGTGGTGGCAATGGTCGTGGAGGCCGAGGGCGAGGAGGACCCATGGCCGTGGAG
GCTATGGAGGTGGTGGCAGTGGTGGTGGTGGCCGAGGAGGATTTCCCAGTGGAGGTG
GTGGCGGTGGAGGACAGCAGCGAGCTGGTGACTGGAAGTGTCCTAATCCCACCTGTGA
GAATATGAACTTCTCTTGGAGGAATGAATGCAACCAGTGTAAGGCCCCTAAACCAGATG
GCCCAGGAGGGGGACCAGGTGGCTCTCACATGGGGGGTAACTACGGGGATGATCGTC
GTGGTGGCAGAGGAGGCGCCACCATGGACGCACAAACACGACGACGTGAGCGTCGCG
CTGAGAAACAAGCTCAATGGAAAGCTGCAAACCCACCGCTCGACGGAGCCGGAGCTGG
CGCTGGAGCTGGAGCCGGAGCTGGCGGTCTAGCCACCATGGACGCACAAACACGACG
ACGTGAGCGTCGCGCTGAGAAACAAGCTCAATGGAAAGCTGCAAACCCACCGCTCGAC
GGAGCCGGAGCTGGCGCTGGAGCTGGAGCCGGAGCTGGCGGTCTAGCCACCATGGA
 
CGCACAAACACGACGACGTGAGCGTCGCGCTGAGAAACAAGCTCAATGGAAAGCTGCA
AACCCACCGCTCGACGGAGCCGGAGCTGGCGCTGGAGCTGGAGCCGGAGCTGGCGG
TCTAGCCACCATGGACGCACAAACACGACGACGTGAGCGTCGCGCTGAGAAACAAGCT
CAATGGAAAGCTGCAAACCCACCGCTCGATTACAAGGATGACGACGATAAGGGTACCG
GCGCCCCCGGCTCCGCCGGCTCCGCCGCCGGCTCCGGCGCGTGCCCGGTGCCGCTG
CAGCTGCCGCCGCTGGAACGCCTGACCCTGGATGATAAAAACCGCTGAATACCCTGA
TCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAA
CAATAGCCGCTCTTCTCGTACAGCACGTGCACTGCGTCACCACAAATATCGTAAAACCT
GTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAAT
GCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTC
TGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATC
AGCTCCAGCACTGACAAAATCCCAAACCGATCGTCTGGAGGTTCTGCTGAATCCGAAAG
ACGAAATCAGCCTGAATTCCGGCAAACCGTTTCGTGAACTGGAGAGCGAACTGCTGTCA
CGTCGTAAAAAAGACCTGCAACAAATCTATGCCGAAGAACGTGAGAACTATCTGGGGAA
ACTGGAACGTGAAATCACCCGCTTTTTCGTGGATCGTGGCTTTCTGGAGATCAAATCCC
CGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGA
GATCGGCCCGTGTTATCGTAAAGAGTCCGACGGTAAAGAACATCTGGAGGAGTTTACCA
TGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGT
GTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTT
TTGGTCTGGAGCGTCTGCTGAAAGTAAAACACGACTTCAAGAACATCAAACGTGCTGCA
CGTTCCGAGTCCTATTACAATGGTATTTCTACTAACCTGTAA (SEQ ID NO:121)
Protein:

MVGRNSAIAAGVCGALFIGYCIYFDRKRRSDPNFKNRLERRKKQKLAKERAGLSKLPDLKD
AEAVQKFFMASNDYTQQATQSYGAYPTQPGQGYSQQSSQPYGQQSYSGYSQSTDTSGY
GQSSYSSYGQSQNTGYGTQSTPQGYGSTGGYGSSQSSQSSYGQQSSYPGYGQQPAPSN
TSGSYGSSSQSSSYGQPQSGSYSQQPSYGGQQQSYGQQQSYNPPQGYGQQNQYNSSS
GGGGGGGGGNYGQDQSSMSSGGGSGGGYGNQDQSGGGGSGGYGQQDRGGRGRG
GSGGGGGGGGGYNRSSGGYEPRGRGGGRGGRGGMGGSDRGGFNKFGGPRDQGSR

HDSEQDNSDNNTIFVQGLGENVTIESVADYFKQIGIIKTNKKTGQPMINLYTDRETGKLKGEA
TVSFDDPPSAKAAIDWFDGKEFSGNPIKVSFATRRADFNRGGGNGRGGRGRGGPMGRGG
YGGGGSGGGGRGGFPSGGGGGGGQQRAGDWKCPNPTCENMNFSWRNECNQCKAPKP
DGPGGGPGGSHMGGNYGDDRRGGRGGATMDAQTRRRERRAEKQAQWKAANPPLDGA
GAGAGAGAGAGGLATMDAQTRRRERRAEKQAQWKAANPPLDGAGAGAGAGAGAGGLAT
MDAQTRRRERRAEKQAQWKAANPPLDGAGAGAGAGAGAGGLATMDAQTRRRERRAEKQ
AQWKAANPPLDYKDDDDKGTGAPGSAGSAAGSGACPVPLQLPPLERLTLDDKKPLNTLISA
TGLWMSRTGTIHKIKHHEVSRSKIYIEMACGDHLVVNNSRSSRTARALRHHKYRKTCKRCR
VSDEDLNKFLTKANEDQTSVKVKVVSAPTRTKKAMPKSVARAPKPLENTEAAQAQPSGSKF
SPAIPVSTQESVSVPASVSTSISSISTGATASALVKGNTNPITSMSAPVQASAPALTKSQTDR
LEVLLNPKDEISLNSGKPFRELESELLSRRKKDLQQIYAEERENYLGKLEREITRFFVDRGFL
EIKSPILIPLEYIERMGIDNDTELSKQIFRVDKNFCLRPMLAPNLANYLRKLDRALPDPIKIFEIG
PCYRKESDGKEHLEEFTMLNFCQMGSGCTRENLESIITDFLNHLGIDFKIVGDSCMVFGDTL
DVMHGDLELSSAVVGPIPLDREWGIDKPWIGAGFGLERLLKVKHDFKNIKRAARSESYYNGI
STNL (SEQ ID NO:122)

TOMM20-FUS-4xλ_{N22}-PyIRS^{AAAF}
DNA: 
ATGGTGGGTCGGAACAGCGCCATCGCCGCCGGTGTATGCGGGGCCCTTTTCATTGGGT
ACTGCATCTACTTCGACCGCAAAAGACGAAGTGACCCCAACTTCAAGAACAGGCTTCGA
GAACGAAGAAAGAAACAGAAGCTTGCCAAGGAGAGAGCTGGGCTTTCCAAGTTACCTG
ACCTTAAAGATGCTGAAGCTGTTCAGAAATTCTTCATGGCCTCAAACGATTATACCCAAC
AAGCAACCCAAAGCTATGGGGCCTACCCCACCCAGCCCGGGCAGGGCTATTCCCAGCA
GAGCAGTCAGCCCTACGGACAGCAGAGTTACAGTGGTTATAGCCAGTCCACGGACACT
TCAGGATATGGCCAGAGCAGCTATTCTTCTTATGGCCAGAGCCAGAACACAGGCTATGG
AACTCAGTCAACTCCCCAGGGATATGGCTCGACTGGCGGCTATGGCAGTAGCCAGAGC
TCCCAATCGTCTTACGGGCAGCAGTCCTCCTACCCTGGCTATGGCCAGCAGCCAGCTC
CCAGCAACACCTCGGGAAGTTACGGTAGCAGTTCTCAGAGCAGCAGCTATGGGCAGCC
CCAGAGTGGGAGCTACAGCCAGCAGCCTAGCTATGGTGGACAGCAGCAAAGCTATGGA
CAGCAGCAAAGCTATAATCCCCCTCAGGGCTATGGACAGCAGAACCAGTACAACAGCA
GCAGTGGTGGTGGAGGTGGAGGTGGAGGTGGAGGTAACTATGGCCAAGATCAATCCTC
CATGAGTAGTGGTGGTGGCAGTGGTGGCGGTTATGGCAATCAAGACCAGAGTGGTGGA
GGTGGCAGCGGTGGCTATGGACAGCAGGACCGTGGAGGCCGCGGCAGGGGTGGCAG
TGGTGGCGGCGGCGGCGGCGGTGGTGGTTACAACCGCAGCAGTGGTGGCTATGA
ACCCAGAGGTCGTGGAGGTGGCCGTGGAGGCAGAGGTGGCATGGGCGGAAGTGACC
GTGGTGGCTTCAATAAATTTGGTGGCCCTCGGGACCAAGGATCACGTCATGACTCCGAA
 
CAGGATAATTCAGACAACAACACCATCTTTGTGCAAGGCCTGGGTGAGAATGTTACAAT
TGAGTCTGTGGCTGATTACTTCAAGCAGATTGGTATTATTAAGACAAACAAGAAAACGGG
ACAGCCCATGATTAATTTGTACACAGACAGGGAAACTGGCAAGCTGAAGGGAGAGGCA
ACGGTCTCTTTTGATGACCCACCTTCAGCTAAAGCAGCTATTGACTGGTTTGATGGTAAA
GAATTCTCCGGAAATCCTATCAAGGTCTCATTTGCTACTCGCCGGGCAGACTTTAATCG
GGGTGGTGGCAATGGTCGTGGAGGCCGAGGGCGAGGAGGACCCATGGGCCGTGGAG
GCTATGGAGGTGGTGGCAGTGGTGGTGGTGGCCGAGGAGGATTTCCCAGTGGAGGTG
GTGGCGGTGGAGGACAGCAGCGAGCTGGTGACTGGAAGTGTCCTAATCCCACCTGTGA
GAATATGAACTTCTCTTGGAGGAATGAATGCAACCAGTGTAAGGCCCCTAAACCAGATG
GCCCAGGAGGGGGACCAGGTGGCTCTCACATGGGGGGTAACTACGGGGATGATCGTC
GTGGTGGCAGAGGAGGCGCCACCATGGACGCACAAACACGACGACGTGAGCGTCGCG
CTGAGAAACAAGCTCAATGGAAAGCTGCAAACCCACCGCTCGACGGAGCCGGAGCTGG
CGCTGGAGCTGGAGCCGGAGCTGGCGGTCTAGCCACCATGGACGCACAAACACGACG
ACGTGAGCGTCGCGCTGAGAAACAAGCTCAATGGAAAGCTGCAAACCCACCGCTCGAC
GGAGCCGGAGCTGGCGCTGGAGCTGGAGCCGGAGCTGGCGGTCTAGCCACCATGGA
CGCACAAACACGACGACGTGAGCGTCGCGCTGAGAAACAAGCTCAATGGAAAGCTGCA
AACCCACCGCTCGACGGAGCCGGAGCTGGCGCTGGAGCTGGAGCCGGAGCTGGCGG
TCTAGCCACCATGGACGCACAAACACGACGACGTGAGCGTCGCGCTGAGAAACAAGCT
CAATGGAAAGCTGCAAACCCACCGCTCGATTACAAGGATGACGACGATAAGGGTACCG
GCGCCCCCGGCTCCGCCGGCTCCGCCGCCGGCTCCGGCGCGTGCCCGGTGCCGCTG
CAGCTGCCGCCGCTGGAACGCCTGACCCTGGATGATAAAAAACCGCTGAATACCCTGA
TCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAA
CAATAGCCGCTCTTCTCGTACAGCACGTGCACTGCGTCACCACAAATATCGTAAAACCT
GTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAAT
GCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTC
TGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATC
AGCTCCAGCACTGACAAAATCCCAAACCGATCGTCTGGAGGTTCTGCTGAATCCGAAAG
ACGAAATCAGCCTGAATTCCGGCAAACCGTTTCGTGAACTGGAGAGCGAACTGCTGTCA
CGTCGTAAAAAAGACCTGCAACAAATCTATGCCGAAGAACGTGAGAACTATCTGGGGAA
ACTGGAACGTGAAATCACCCGCTTTTTCGTGGATCGTGGCTTTCTGGAGATCAAATCCC
CGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAGCACCAAAT
 
CTGGCTAACTATCTGCGCAAACTGGACCGTGCCCTGCCTGATCCTATCAAAATCTTCGA
GATCGGCCCGTGTTATCGTAAAGAGTCCGACGGTAAAGAACATCTGGAGGAGTTTACCA
TGCTGGCCTTTGCCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGT
GTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTT
TTGGTCTGGAGCGTCTGCTGAAAGTAAAACACGACTTCAAGAACATCAAACGTGCTGCA
CGTTCCGAGTCCTATTACAATGGTATTTCTACTAACCTGTAA (SEQ ID NO:123)
Protein:

MVGRNSAIAAGVCGALFIGYCIYFDRKRRSDPNFKNRLRERRKKQKLAKERAGLSKLPDLKD
AEAVQKFFMASNDYTQQATQSYGAYPTQPGQGYSQQSSQPYGQQSYSGYSQSTDTSGY
GQSSYSSYGQSQNTGYGTQSTPQGYGSTGGYGSSQSSQSSYGQQSSYPGYGQQPAPSN
TSGSYGSSSQSSSYGQPQSGSYSQQPSYGGQQQSYGQQQSYNPPQGYGQQNQYNSSS
GGGGGGGGGGNYGQDQSSMSSGGGSGGGYGNQDQSGGGGSGGYGQQDRGGRGRG
GSGGGGGGGGGYNRSSGGYEPRGRGGGRGGRGGMGGSDRGGFNKFGGPRDQGSR
HDSEQDNSDNNTIFVQGLGENVTIESVADYFKQIGIIKTNKKTGQPMINLYTDRETGKLKGEA
TVSFDDPPSAKAAIDWFDGKEFSGNPIKVSFATRRADFNRGGGNGRGGRGRGGPMGRGG
YGGGGSGGGGRGGFPSGGGGGGGQQRAGDWKCPNPTCENMNFSWRNECNQCKAPKP
DGPGGGPGGSHMGGNYGDDRRGGRGGATMDAQTRRRERRAEKQAQWKAANPPLDGA
GAGAGAGAGAGGLATMDAQTRRRERRAEKQAQWKAANPPLDGAGAGAGAGAGAGGLAT
MDAQTRRRERRAEKQAQWKAANPPLDGAGAGAGAGAGAGGLATMDAQTRRRERRAEKQ
AQWKAANPPLDYKDDDDKGTGAPGSAGSAAGSGACPVPLQLPPLERLTLDDKKPLNTLISA
TGLWMSRTGTIHKIKHHEVSRSKIYIEMACGDHLVVNNSRSSRTARALRHHKYRKTCKRCR
VSDEDLNKFLTKANEDQTSVKVKVVSAPTRTKKAMPKSVARAPKPLENTEAAQAQPSGSKF
SPAIPVSTQESVSVPASVSTSISSISTGATASALVKGNTNPITSMSAPVQASAPALTKSQTDR
LEVLLNPKDEISLNSGKPFRELESELLSRRKKDLQQIYAEERENYLGKLEREITRFFVDRGFL
EIKSPILIPLEYIERMGIDNDTELSKQIFRVDKNFCLRPMLAPNLANYLRKLDRALPDPIKIFEIG
PCYRKESDGKEHLEEFTMLAFAQMGSGCTRENLESIITDFLNHLGIDFKIVGDSCMVFGDTL
DVMHGDLELSSAVVGPIPLDREWGIDKPWIGAGFGLERLLKVKHDFKNIKRAARSESYYNGI
STNL (SEQ ID NO:124)

LcK-EWSR1-MCP
DNA: 
ATGGGCTGCGTGTGCAGCAGCAACCCCGAGGGTACCGAGCTCGCGTCCACGGATTAC
AGTACCTATAGCCAAGCTGCAGCGCAGCAGGGCTACAGTGCTTACACCGCCCAGCCCA
CTCAAGGATATGCACAGACCACCCAGGCATATGGGCAACAAAGCTATGGAACCTATGGA
 
CAGCCCACTGATGTCAGCTATACCCAGGCTCAGACCACTGCAACCTATGGGCAGACCG
CCTATGCAACTTCTTATGGACAGCCTCCCACTGGTTATACTACTCCAACTGCCCCCAG
GCATACAGCCAGCCTGTCCAGGGGTATGGCACTGGTGCTTATGATACCACCACTGCTA
CAGTCACCACCACCCAGGCCTCCTATGCAGCTCAGTCTGCATATGGCACTCAGCCTGCT
TATCCAGCCTATGGGCAGCAGCCAGCAGCCACTGCACCTACAAGACCGCAGGATGGAA
ACAAGCCCACTGAGACTAGTCAACCTCAATCTAGCACAGGGGGTTACAACCAGCCCAG
CCTAGGATATGGACAGAGTAACTACAGTTATCCCCAGGTACCTGGGAGCTACCCCATGC
AGCCAGTCACTGCACCTCCATCCTACCCTCCTACCAGCTATTCCTCTACACAGCCGACT
AGTTATGATCAGAGCAGTTACTCTCAGCAGAACACCTATGGGCAACCGAGCAGCTATGG
ACAGCAGAGTAGCTATGGTCAACAAAGCAGCTATGGGCAGCAGCCTCCCACTAGTTAC
CCACCCCAAACTGGATCCTACAGCCAAGCTCCAAGTCAATATAGCCAACAGAGCAGCAG
CTACGGGCAGCAGAGTTCATTCCGACAGGACCACCCCAGTAGCATGGGTGTTTATGGG
CAGGAGTCTGGAGGATTTTCCGGACCAGGAGAGAACCGGAGCATGAGTGGCCCTGATA
ACCGGGGCAGGGGAAGAGGGGGATTTGATCGTGGAGGCATGAGCAGAGGTGGGCGG
GGAGGAGGACGCGGTGGAATGGGCAGCGCTGGAGAGCGAGGTGGCTTCAATAAGCCT
GGTGGACCCATGGATGAAGGACCAGATCTTGATCTAGGCCCACCTGTAGATCCAGATG
AAGACTCTGACAACAGTGCAATTTATGTACAAGGATTAAATGACAGTGTGACTCTAGATG
ATCTGGCAGACTTCTTTAAGCAGTGTGGGGTTGTTAAGATGAACAAGAGAACTGGCAA
CCCATGATCCACATCTACCTGGACAAGGAAACAGGAAAGCCCAAAGGCGATGCCACAG
TGTCCTATGAAGACCCACCCACTGCCAAGGCTGCCGTGGAATGGTTTGATGGGAAAGA
TTTTCAAGGGAGCAAACTTAAAGTCTCCCTTGCTCGGAAGAAGCCTCCAATGAACAGTA
TGCGGGGTGGTCTGCCACCCCGTGAGGGCAGAGGCATGCCACCACCACTCCGTGGAG
GTCCAGGAGGCCCAGGAGGTCCTGGGGGACCCATGGGTCGCATGGGAGGCCGTGGA
GGAGATAGAGGAGGCTTCCCTCCAAGAGGACCCCGGGGTTCCCGAGGGAACCCCTCT
GGAGGAGGAAACGTCCAGCACCGAGCTGGAGACTGGCAGTGTCCCAATCCGGGTTGT
GGAAACCAGAACTTCGCCTGGAGAACAGAGTGCAACCAGTGTAAGGCCCCAAAGCCTG
AAGGCTTCCTCCCGCCACCCTTTCCGCCCCCGGGTGGTGATCGTGGCAGAGGTGGCC
CTGGTGGCATGCGGGGAGGAAGAGGTGGCCTCATGGATCGTGGTGGTCCCGGTGGAA
TGTTCAGAGGTGGCCGTGGTGGAGACAGAGGTGGCTTCCGTGGTGGCCGGGGCATGG
ACCGAGGTGGCTTTGGTGGAGGAAGACGAGGTGGCCCTGGGGGGCCCCCTGGACCTT
TGATGGAACAGGATTACAAGGATGACGACGATAAGGGTACCGAGCAGAAGCTGATCTC
AGAGGAGGACCTGGGCGCCCCCGGCTCCGCCGGCTCCGCCGCCGGCTCCGGCGCTT
CTAACTTTACTCAGTTCGTTCTCGTCGACAATGGCGGAACTGGCGACGTGACTGTCGCC
CCAAGCAACTTCGCTAACGGGATCGCTGAATGGATCAGCTCTAACTCGCGTTCACAGGC
TTACAAAGTAACCTGTAGCGTTCGTCAGAGCTCTGCGCAGAATCGCAAATACACCATCA
AAGTCGAGGTGCCTAAAGGCGCCTGGCGTTCGTACTTAAATATGGAACTAACCATTCCA
 
ATTTTCGCCACGAATTCCGACTGCGAGCTTATTGTTAAGGCAATGCAAGGTCTCCTAAAA
GATGGAAACCCGATTCCCTCAGCAATCGCAGCAAACTCCGGCATCTACTAA (SEQ ID
NO:125)
Protein:

MGCVCSSNPEGTELASTDYSTYSQAAAQQGYSAYTAQPTQGYAQTTQAYGQQSYGTYGQ
PTDVSYTQAQTTATYGQTAYATSYGQPPTGYTTPTAPQAYSQPVQGYGTGAYDTTTATVTT
TQASYAAQSAYGTQPAYPAYGQQPAATAPTRPQDGNKPTETSQPQSSTGGYNQPSLGYG
QSNYSYPQVPGSYPMQPVTAPPSYPPTSYSSTQPTSYDQSSYSQQNTYGQPSSYGQQSS
YGQQSSYGQQPPTSYPPQTGSYSQAPSQYSQQSSSYGQQSSFRQDHPSSMGVYGQESG
GFSGPGENRSMSGPDNRGRGRGGFDRGGMSRGGRGGGRGGMGSAGERGGFNKPGGP
MDEGPDLDLGPPVDPDEDSDNSAIYVQGLNDSVTLDDLADFFKQCGVVKMNKRTGQPMIHI
YLDKETGKPKGDATVSYEDPPTAKAAVEWFDGKDFQGSKLKVSLARKKPPMNSMRGGLPP
REGRGMPPPLRGGPGGPGGPGGPMGRMGGRGGDRGGFPPRGPRGSRGNPSGGGNVQ
HRAGDWQCPNPGCGNQNFAWRTECNQCKAPKPEGFLPPPFPPPGGDRGRGGPGGMRG
GRGGLMDRGGPGGMFRGGRGGDRGGFRGGRGMDRGGFGGGRRGGPGGPPGPLMEQ
DYKDDDDKGTEQKLISEEDLGAPGSAGSAAGSGASNFTQFVLVDNGGTGDVTVAPSNFAN
GIAEWISSNSRSQAYKVTCSVRQSSAQNRKYTIKVEVPKGAWRSYLNMELTIPIFATNSDCE
LIVKAMQGLLKDGNPIPSAIAANSGIY (SEQ ID NO:126)

LcK-EWSR1-4xλ_{N22}
DNA: 
ATGGGCTGCGTGTGCAGCAGCAACCCCGAGGGTACCGAGCTCGCGTCCACGGATTAC
AGTACCTATAGCCAAGCTGCAGCGCAGCAGGGCTACAGTGCTTACACCGCCCAGCCCA
CTCAAGGATATGCACAGACCACCCAGGCATATGGGCAACAAAGCTATGGAACCTATGGA
CAGCCCACTGATGTCAGCTATACCCAGGCTCAGACCACTGCAACCTATGGGCAGACCG
CCTATGCAACTTCTTATGGACAGCCTCCCACTGGTTATACTACTCCAACTGCCCCCCAG
GCATACAGCCAGCCTGTCCAGGGGTATGGCACTGGTGCTTATGATACCACCACTGCTA
CAGTCACCACCACCCAGGCCTCCTATGCAGCTCAGTCTGCATATGGCACTCAGCCTGCT
TATCCAGCCTATGGGCAGCAGCCAGCAGCCACTGCACCTACAAGACCGCAGGATGGAA
ACAAGCCCACTGAGACTAGTCAACCTCAATCTAGCACAGGGGGTTACAACCAGCCCAG
CCTAGGATATGGACAGAGTAACTACAGTTATCCCCAGGTACCTGGGAGCTACCCCATGC
AGCCAGTCACTGCACCTCCATCCTACCCTCCTACCAGCTATTCCTCTACACAGCCGACT
AGTTATGATCAGAGCAGTTACTCTCAGCAGAACACCTATGGGCAACCGAGCAGCTATGG
ACAGCAGAGTAGCTATGGTCAACAAAGCAGCTATGGGCAGCAGCCTCCCACTAGTTAC
CCACCCCAAACTGGATCCTACAGCCAAGCTCCAAGTCAATATAGCCAACAGAGCAGCAG
CTACGGGCAGCAGAGTTCATTCCGACAGGACCACCCCAGTAGCATGGGTGTTTATGGG
 
CAGGAGTCTGGAGGATTTTCCGGACCAGGAGAGAACCGGAGCATGAGTGGCCCTGATA
ACCGGGGCAGGGGAAGAGGGGGATTTGATCGTGGAGGCATGAGCAGAGGTGGCGG
GGAGGAGGACGCGGTGGAATGGGCAGCGCTGGAGAGCGAGGTGGCTTCAATAAGCCT
GGTGGACCCATGGATGAAGGACCAGATCTTGATCTAGGCCCACCTGTAGATCCAGATG
AAGACTCTGACAACAGTGCAATTTATGTACAAGGATTAAATGACAGTGTGACTCTAGATG
ATCTGGCAGACTTCTTTAAGCAGTGTGGGGTTGTTAAGATGAACAAGAGAACTGGGCAA
CCCATGATCCACATCTACCTGGACAAGGAAACAGGAAAGCCCAAAGGCGATGCCACAG
TGTCCTATGAAGACCCACCTACTGCCAAGGCTGCCGTGGAATGGTTTGATGGAAAGAT
TTTCAAGGGAGCAAACTTAAAGTCTCCCTTGCTCGGAAGAAGCCTCCAATGAACAGTAT
GCGGGGTGGTCTGCCACCCCGTGAGGGCAGAGGCATGCCACCACTCCGTGGAGG
TCCAGGAGGCCCAGGAGGTCCTGGGGGACCCATGGGTCGCATGGGAGGCCGTGGAG
GAGATAGAGGAGGCTTCCCTCCAAGAGGACCCCGGGGTTCCCGAGGAACCCCTCTG
GAGGAGGAAACGTCCAGCACCGAGCTGGAGACTGGCAGTGTCCCAATCCGGGTTGTG
GAAACCAGAACTTCGCCTGGAGAACAGAGTGCAACCAGTGTAAGGCCCCAAAGCCTGA
AGGCTTCCTCCCGCCACCCTTTCCGCCCCCGGGTGGTGATCGTGGCAGAGGTGGCCC
TGGTGGCATGCGGGGAGGAAGAGGTGGCCTCATGGATCGTGGTGGTCCCGGTGGAAT
GTTCAGAGGTGGCCGTGGTGGAGACAGAGGTGGCTTCCGTGGTGGCCGGGGCATGGA
CCGAGGTGGCTTTGGTGGAGGAAGACGAGGTGGCCCTGGGGGCCCCCTGGACCTTT
GATGGAACAGGATTACAAGGATGACGACGATAAGGGTACCGAGCAGAAGCTGATCTCA
GAGGAGGACCTGCTAGCCACCATGGACGCACAAACACGACGACGTGAGCGTCGCGCT
GAGAAACAAGCTCAATGGAAAGCTGCAAACCCACCGCTCGACGGAGCCGGAGCTGGC
GCTGGAGCTGGAGCCGGAGCTGGCGGTCTAGCCACCATGGACGCACAAACACGACGA
CGTGAGCGTCGCGCTGAGAAACAAGCTCAATGGAAAGCTGCAAACCCACCGCTCGACG
GAGCCGGAGCTGGCGCTGGAGCTGGAGCCGGAGCTGGCGGTCTAGCCACCATGGAC
GCACAAACACGACGACGTGAGCGTCGCGCTGAGAAACAAGCTCAATGGAAAGCTGCAA
ACCCACCGCTCGACGGAGCCGGAGCTGGCGCTGGAGCTGGAGCCGGAGCTGGCGGT
CTAGCCACCATGGACGCACAAACACGACGACGTGAGCGTCGCGCTGAGAAACAAGCTC
AATGGAAAGCTGCAAACCCACCGCTCGAGTCTAGAGGGCCCGTTAAACCCGCTGATCA
GCCTCGACTGTGCCTTCTAGTTGCCAGCCATCTGTTGTTTGCCCCTCCCCCGTGCCTTC
CTTGA (SEQ ID NO:127)
Protein:

MGCVCSSNPEGTELASTDYSTYSQAAAQQGYSAYTAQPTQGYAQTTQAYGQQSYGTYGQ
PTDVSYTQAQTTATYGQTAYATSYGQPPTGYTTPTAPQAYSQPVQGYGTGAYDTTTATVTT
TQASYAAQSAYGTQPAYPAYGQQPAATAPTRPQDGNKPTETSQPQSSTGGYNQPSLGYG
QSNYSYPQVPGSYPMQPVTAPPSYPPTSYSSTQPTSYDQSSYSQQNTYGQPSSYGQQSS
YGQQSSYGQQPPTSYPPQTGSYSQAPSQYSQQSSSYGQQSSFRQDHPSSMGVYGQESG

GFSGPGENRSMSGPDNRGRGRGGFDRGGMSRGGRGGGRGGMGSAGERGGFNKPGGP
MDEGPDLDLGPPVDPDEDSDNSAIYVQGLNDSVTLDDLADFFKQCGVVKMNKRTGQPMIHI
YLDKETGKPKGDATVSYEDPPTAKAAVEWFDGKDFQGSKLKVSLARKKPPMNSMRGGLPP
REGRGMPPPLRGGPGGPGGPGGPMGRMGGRGGDRGGFPPRGPRGSRGNPSGGGNVQ
HRAGDWQCPNPGCGNQNFAWRTECNQCKAPKPEGFLPPPFPPPGGDRGRGGPGGMRG
GRGGLMDRGGPGGMFRGGRGGDRGGFRGGRGMDRGGFGGGRRGGPGGPPGPLMEQ
DYKDDDDKGTEQKLISEEDLLATMDAQTRRRERRAEKQAQWKAANPPLDGAGAGAGAGA
GAGGLATMDAQTRRRERRAEKQAQWKAANPPLDGAGAGAGAGAGAGGLATMDAQTRRR
ERRAEKQAQWKAANPPLDGAGAGAGAGAGAGGLATMDAQTRRRERRAEKQAQWKAANP
PLESRGPVKPADQPRLCLLVASHLLFAPPPCLP (SEQ ID NO:128)

LcK-FUS-PyIRS^{AF}
DNA: 
ATGGGCTGCGTGTGCAGCAGCAACCCCGAGGGTACCGAGCTCGCCTCAAACGATTATA
CCCAACAAGCAACCCAAAGCTATGGGGCCTACCCCACCCAGCCCGGGCAGGGCTATTC
CCAGCAGAGCAGTCAGCCCTACGGACAGCAGAGTTACAGTGGTTATAGCCAGTCCACG
GACACTTCAGGATATGGCCAGAGCAGCTATTCTTCTTATGGCCAGAGCCAGAACACAGG
CTATGGAACTCAGTCAACTCCCCAGGGATATGGCTCGACTGGCGGCTATGGCAGTAGC
CAGAGCTCCCAATCGTCTTACGGGCAGCAGTCCTCCTACCCTGGCTATGGCCAGCAGC
CAGCTCCCAGCAACACCTCGGGAAGTTACGGTAGCAGTTCTCAGAGCAGCAGCTATGG
GCAGCCCCAGAGTGGGAGCTACAGCCAGCAGCCTAGCTATGGTGGACAGCAGCAAAG
CTATGGACAGCAGCAAAGCTATAATCCCCCTCAGGGCTATGGACAGCAGAACCAGTACA
ACAGCAGCAGTGGTGGTGGAGGTGGAGGTGGAGGTGGAGGTAACTATGGCCAAGATC
AATCCTCCATGAGTAGTGGTGGTGGCAGTGGTGGCGGTTATGGCAATCAAGACCAGAG
TGGTGGAGGTGGCAGCGGTGGCTATGGACAGCAGGACCGTGGAGGCCGCGGCAGGG
GTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGA
AGTGACCGTGGTGGCTTCAATAAATTTGGTGGCCCTCGGGACCAAGGATCACGTCATG
ACTCCGAACAGGATAATTCAGACAACAACACCATCTTTGTGCAAGGCCTGGGTGAGAAT
GTTACAATTGAGTCTGTGGCTGATTACTTCAAGCAGATTGGTATTATTAAGACAAACAAG
AAAACGGGACAGCCCATGATTAATTTGTACACAGACAGGGAAACTGGCAAGCTGAAGG
GAGAGGCAACGGTCTCTTTTGATGACCCACCTTCAGCTAAAGCAGCTATTGACTGGTTT
GATGGTAAAGAATTCTCCGGAAATCCTATCAAGGTCTCATTTGCTACTCGCCGGGCAGA
CTTTAATCGGGGTGGTGGCAATGGTCGTGGAGGCCGAGGGCGAGGAGGACCCATGGG
CCGTGGAGGCTATGGAGGTGGTGGCAGTGGTGGTGGTGGCCGAGGAGGATTTCCCAG
TGGAGGTGGTGGCGGTGGAGGACAGCAGCGAGCTGGTGACTGGAAGTGTCCTAATCC
 
CACCTGTGAGAATATGAACTTCTCTTGGAGGAATGAATGCAACCAGTGTAAGGCCCCTA
AACCAGATGGCCCAGGAGGGGACCAGGTGGCTCTCACATGGGGGTAACTACGGGG
ATGATCGTCGTGGTGGCAGAGGAGGCGATTACAAGGATGACGACGATAAGGGTACCGG
CGCCCCCGGCTCCGCCGGCTCCGCCGCCGGCTCCGGCGCGTGCCCGGTGCCGCTGC
AGCTGCCGCCGCTGGAACGCCTGACCCTGGATGATAAAAACCGCTGAATACCCTGAT
CTCTGCTACTGGTCTGTGGATGAGTCGTACCGGAACCATTCATAAAATCAAACACCACG
AGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTG
TAAACGTTGCCGTGTGTCCGATGAGGATCTGAACAAATTCCTGACAAAAGCCAATGAGG
ACCAAACAAGCGTGAAAGTGAAAGTCGTTAGCGCTCCTACCCGTACTAAAAAGCAATG
CCGAAATCCGTTGCTCGTGCCCCTAAACCACTGGAAAACACTGAAGCAGCACAGGCAC
AGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCG
CCCTGGTTAAAGGCAATACCAATCCGATTACAAGCATGTCTGCCCCGGTTCAAGCATCA
GCTCCAGCACTGACAAAATCCCAAACCGATCGTCTGGAGGTTCTGCTGAATCCGAAAGA
CGAAATCAGCCTGAATTCCGGCAAACCGTTTCGTGAACTGGAGAGCGAACTGCTGTCAC
GTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCC
GATTCTGATTCCTCTGGAGTATATCGAGCGTATGGGCATCGACAATGATACCGAACTGA
GCAAACAAATTTTCCGTGTGGATAAAAACTTCTGTCTGCGCCCTATGCTAGCACCAAATC
TGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCAT
GCTGAACTTTTGCCAAATGGGTTCAGGTTGTACTCGTGAGAACCTGGAAAGCATCATCA
CCGATTTTCTGAACCACCTGGGCATTGACTTCAAAATTGTGGGCGACAGCTGTATGGTG
TTTGGCGACACCCTGGATGTCATGCACGGCGACCTGGAACTGTCTAGTGCCGTTGTGG
GCCCAATCCCGCTGGATCGTGAGTGGGGTATCGACAAACCTTGGATCGGTGCGGGTTT
TGGTCTGGAGCGTCTGCTGAAAGTAAAACACGACTTCAAGAACATCAAACGTGCTGCAC
GTTCCGAGTCCTATTACAATGGTATTTCTACTAACCTGTAA (SEQ ID NO:129)
Protein:

MGCVCSSNPEGTELASNDYTQQATQSYGAYPTQPGQGYSQQSSQPYGQQSYSGYSQST
DTSGYGQSSYSSYGQSQNTGYGTQSTPQGYGSTGGYGSSQSSQSSYGQQSSYPGYGQQ
PAPSNTSGSYGSSSQSSSYGQPQSGSYSQQPSYGGQQQSYGQQQSYNPPQGYGQQNQ
YNSSSGGGGGGGGGNYGQDQSSMSSGGGSGGGYGNQDQSGGGGSGGYGQQDRGG
RGRGGSGGGGGGGGGYNRSSGGYEPRGRGGGRGGRGGMGGSDRGGFNKFGGPRD
QGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPM

GRGGYGGGGSGGGGRGGFPSGGGGGGGQQRAGDWKCPNPTCENMNFSWRNECNQC
KAPKPDGPGGGPGGSHMGGNYGDDRRGGRGGDYKDDDDKGTGAPGSAGSAAGSGACP
VPLQLPPLERLTLDDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKS
VARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAE
ERENYLGKLEREITRFFVDRGFLEIKSPILIPLEYIERMGIDNDTELSKQIFRVDKNFCLRPMLA
PNLANYLRKLDRALPDPIKIFEIGPCYRKESDGKEHLEEFTMLNFCQMGSGCTRENLESIITD
FLNHLGIDFKIVGDSCMVFGDTLDVMHGDLELSSAVVGPIPLDREWGIDKPWIGAGFGLERL
LKVKHDFKNIKRAARSESYYNGISTNL (SEQ ID NO:130)

LcK-FUS-MCP-PyIRS^{AF}
DNA: 
ATGGGCTGCGTGTGCAGCAGCAACCCCGAGGGTACCGAGCTCGCCTCAAACGATTATA
CCCAACAAGCAACCCAAAGCTATGGGGCCTACCCCACCCAGCCCGGGCAGGGCTATTC
CCAGCAGAGCAGTCAGCCCTACGGACAGCAGAGTTACAGTGGTTATAGCCAGTCCACG
GACACTTCAGGATATGGCCAGAGCAGCTATTCTTCTTATGGCCAGAGCCAGAACACAGG
CTATGGAACTCAGTCAACTCCCCAGGGATATGGCTCGACTGGCGGCTATGGCAGTAGC
CAGAGCTCCCAATCGTCTTACGGGCAGCAGTCCTCCTACCCTGGCTATGGCCAGCAGC
CAGCTCCCAGCAGCACCTCGGGAAGTTACGGTAGCAGTTCTCAGAGCAGCAGCTATGG
GCAGCCCCAGAGTGGGAGCTACAGCCAGCAGCCTAGCTATGGTGGACAGCAGCAAAG
CTATGGACAGCAGCAAAGCTATAATCCCCCTCAGGGCTATGGACAGCAGAACCAGTACA
ACAGCAGCAGTGGTGGTGGAGGTGGAGGTGGAGGTGGAGGTAACTATGGCCAAGATC
AATCCTCCATGAGTAGTGGTGGTGGCAGTGGTGGCGGTTATGGCAATCAAGACCAGAG
TGGTGGAGGTGGCAGCGGTGGCTATGGACAGCAGGACCGTGGAGGCCGCGGCAGGG
GTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGA
AGTGACCGTGGTGGCTTCAATAAATTTGGTGGCCCTCGGGACCAAGGATCACGTCATG
ACTCCGAACAGGATAATTCAGACAACAACACCATCTTTGTGCAAGGCCTGGGTGAGAAT
GTTACAATTGAGTCTGTGGCTGATTACTTCAAGCAGATTGGTATTATTAAGACAAACAAG
AAAACGGGACAGCCCATGATTAATTTGTACACAGACAGGGAAACTGGCAAGCTGAAGG
GAGAGGCAACGGTCTCTTTTGATGACCCACCTTCAGCTAAAGCAGCTATTGACTGGTTT
GATGGTAAAGAATTCTCCGGAAATCCTATCAAGGTCTCATTTGCTACTCGCCGGGCAGA
CTTTAATCGGGGTGGTGGCAATGGTCGTGGAGGCCGAGGGCGAGGAGGACCCATGGG
CCGTGGAGGCTATGGAGGTGGTGGCAGTGGTGGTGGTGGCCGAGGAGGATTTCCCAG
TGGAGGTGGTGGCGGTGGAGGACAGCAGCGAGCTGGTGACTGGAAGTGTCCTAATCC
 
CACCTGTGAGAATATGAACTTCTCTTGGAGGAATGAATGCAACCAGTGTAAGGCCCCTA
AACCAGATGGCCCAGGAGGGGGACCAGGTGGCTCTCACATGGGGGGTAACTACGGGG
ATGATCGTCGTGGTGGCAGAGGAGGCGATTACAAGGATGACGACGATAAGGGTACCGG
CGCCCCCGGCTCCGCCGGCTCCGCCGCCGGCTCCGGCGCTTCTAACTTTACTCAGTTC
GTTCTCGTCGACAATGGCGGAACTGGCGACGTGACTGTCGCCCCAAGCAACTTCGCTA
ACGGGATCGCTGAATGGATCAGCTCTAACTCGCGTTCACAGGCTTACAAAGTAACCTGT
AGCGTTCGTCAGAGCTCTGCGCAGAATCGCAAATACACCATCAAAGTCGAGGTGCCTAA
AGGCGCCTGGCGTTCGTACTTAAATATGGAACTAACCATTCCAATTTTCGCCACGAATTC
CGACTGCGAGCTTATTGTTAAGGCAATGCAAGGTCTCCTAAAAGATGGAAACCCGATTC
CCTCAGCAATCGCAGCAAACTCCGGCATCTACGGTACCGGCGCCCCCGGCTCCGCCG
GCTCCGCCGCCGGCTCCGGCGCGTGCCCGGTGCCGCTGCAGCTGCCGCCGCTGGAA
CGCCTGACCCTGGATGATAAAAACCGCTGAATACCCTGATCTCTGCTACTGGTCTGTG
GATGAGTCGTACCGGAACCATTCATAAAATCAAACACCACGAGGTTAGCCGTTCGAAAA
TCTATATTGAGATGGCGTGTGGCGATCATCTGGTTGTGAACAATAGCCGCTCTTCTCGT
ACAGCACGTGCACTGCGTCACCACAAATATCGTAAAACCTGTAAACGTTGCCGTGTGTC
CGATGAGGATCTGAACAAATTCCTGACAAAAGCCAATGAGGACCAAACAAGCGTGAAAG
TGAAAGTCGTTAGCGCTCCTACCCGTACTAAAAAAGCAATGCCGAAATCCGTTGCTCGT
GCCCCTAAACCACTGGAAAACACTGAAGCAGCACAGGCACAGCCGTCTGGAAGCAAAT
TCTCTCCGGCCATTCCTGTTTCTACCCAGGAGTCCGTTTCTGTTCCAGCAAGTGTGAGC
ACCAGCATTAGCAGTATTAGCACCGGTGCCACCGCTAGCGCCCTGGTTAAAGGCAATA
CCAATCCGATTACAAGCATGTCTGCCCCGGTTCAAGCATCAGCTCCAGCACTGACAAAA
TCCCAAACCGATCGTCTGGAGGTTCTGCTGAATCCGAAAGACGAAATCAGCCTGAATTC
CGGCAAACCGTTTCGTGAACTGGAGAGCGAACTGCTGTCACGTCGTAAAAAAGACCTG
CAACAAATCTATGCCGAAGAACGTGAGAACTATCTGGGGAAACTGGAACGTGAAATCAC
CCGCTTTTTCGTGGATCGTGGCTTTCTGGAGATCAAATCCCCGATTCTGATTCCTCTGG
AGTATATCGAGCGTATGGGCATCGACAATGATACCGAACTGAGCAAACAAATTTTCCGT
GTGGATAAAAACTTCTGTCTGCGCCCTATGCTAGCACCAAATCTGGCTAACTATCTGCG
CAAACTGGACCGTGCCCTGCCTGATCCTATCAAAATCTTCGAGATCGGCCCGTGTTATC
GTAAAGAGTCCGACGGTAAAGAACATCTGGAGGAGTTTACCATGCTGAACTTTTGCCAA
ATGGGTTCAGGTTGTACTCGTGAGAACCTGGAAAGCATCATCACCGATTTTCTGAACCA
CCTGGGCATTGACTTCAAAATTGTGGGCGACAGCTGTATGGTGTTTGGCGACACCCTG
GATGTCATGCACGGCGACCTGGAACTGTCTAGTGCCGTTGTGGGCCCAATCCCGCTGG
ATCGTGAGTGGGGTATCGACAAACCTTGGATCGGTGCGGGTTTTGGTCTGGAGCGTCT
GCTGAAAGTAAAACACGACTTCAAGAACATCAAACGTGCTGCACGTTCCGAGTCCTATT
ACAATGGTATTTCTACTAACCTGTAA (SEQ ID NO:131)
Protein:

MGCVCSSNPEGTELASNDYTQQATQSYGAYPTQPGQGYSQQSSQPYGQQSYSGYSQST
DTSGYGQSSYSSYGQSQNTGYGTQSTPQGYGSTGGYGSSQSSQSSYGQQSSYPGYGQQ
PAPSSTSGSYGSSSQSSSYGQPQSGSYSQQPSYGGQQQSYGQQQSYNPPQGYGQQNQ
YNSSSGGGGGGGGGNYGQDQSSMSSGGGSGGGYGNQDQSGGGGSGGYGQQDRGG
RGRGGSGGGGGGGGGYNRSSGGYEPRGRGGGRGGRGGMGGSDRGGFNKFGGPRD
QGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPM
GRGGYGGGGSGGGGRGGFPSGGGGGGGQQRAGDWKCPNPTCENMNFSWRNECNQC
KAPKPDGPGGGPGGSHMGGNYGDDRRGGRGGDYKDDDDKGTGAPGSAGSAAGSGASN
FTQFVLVDNGGTGDVTVAPSNFANGIAEWISSNSRSQAYKVTCSVRQSSAQNRKYTIKVEV
PKGAWRSYLNMELTIPIFATNSDCELIVKAMQGLLKDGNPIPSAIAANSGIYGTGAPGSAGSA
AGSGACPVPLQLPPLERLTLDDKKPLNTLISATGLWMSRTGTIHKIKHHEVSRSKIYIEMACG
DHLVVNNSRSSRTARALRHHKYRKTCKRCRVSDEDLNKFLTKANEDQTSVKVKVVSAPTRT
KKAMPKSVARAPKPLENTEAAQAQPSGSKFSPAIPVSTQESVSVPASVSTSISSISTGATAS
ALVKGNTNPITSMSAPVQASAPALTKSQTDRLEVLLNPKDEISLNSGKPFRELESELLSRRKK
DLQQIYAEERENYLGKLEREITRFFVDRGFLEIKSPILIPLEYIERMGIDNDTELSKQIFRVDKN
FCLRPMLAPNLANYLRKLDRALPDPIKIFEIGPCYRKESDGKEHLEEFTMLNFCQMGSGCTR
ENLESIITDFLNHLGIDFKIVGDSCMVFGDTLDVMHGDLELSSAVVGPIPLDREWGIDKPWIG
AGFGLERLLKVKHDFKNIKRAARSESYYNGISTNL (SEQ ID NO:132)

LCK-FUS-3xMCP-PyIRS^{AF}
DNA: 
ATGGGCTGCGTGTGCAGCAGCAACCCCGAGGGTACCGAGCTCGCCTCAAACGATTATA
CCCAACAAGCAACCCAAAGCTATGGGGCCTACCCCACCCAGCCCGGGCAGGGCTATTC
CCAGCAGAGCAGTCAGCCCTACGGACAGCAGAGTTACAGTGGTTATAGCCAGTCCACG
GACACTTCAGGATATGGCCAGAGCAGCTATTCTTCTTATGGCCAGAGCCAGAACACAGG
CTATGGAACTCAGTCAACTCCCCAGGGATATGGCTCGACTGGCGGCTATGGCAGTAGC
CAGAGCTCCCAATCGTCTTACGGGCAGCAGTCCTCCTACCCTGGCTATGGCCAGCAGC
CAGCTCCCAGCAACACCTCGGGAAGTTACGGTAGCAGTTCTCAGAGCAGCAGCTATGG
GCAGCCCCAGAGTGGGAGCTACAGCCAGCAGCCTAGCTATGGTGGACAGCAGCAAAG
CTATGGACAGCAGCAAAGCTATAATCCCCCTCAGGGCTATGGACAGCAGAACCAGTACA
ACAGCAGCAGTGGTGGTGGAGGTGGAGGTGGAGGTGGAGGTAACTATGGCCAAGATC
AATCCTCCATGAGTAGTGGTGGTGGCAGTGGTGGCGGTTATGGCAATCAAGACCAGAG
TGGTGGAGGTGGCAGCGGTGGCTATGGACAGCAGGACCGTGGAGGCCGCGGCAGGG
GTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGA
 
AGTGACCGTGGTGGCTTCAATAAATTTGGTGGCCCTCGGGACCAAGGATCACGTCATG
ACTCCGAACAGGATAATTCAGACAACAACACCATCTTTGTGCAAGGCCTGGGTGAGAAT
GTTACAATTGAGTCTGTGGCTGATTACTTCAAGCAGATTGGTATTATTAAGACAAACAAG
AAAACGGACAGCCCATGATTAATTTGTACACAGACAGGGAAACTGGCAAGCTGAAGG
GAGAGGCAACGGTCTCTTTTGATGACCCACCTTCAGCTAAAGCAGCTATTGACTGGTTT
GATGGTAAAGAATTCTCCGGAAATCCTATCAAGGTCTCATTTGCTACTCGCCGGGCAGA
CTTTAATCGGGGTGGTGGCAATGGTCGTGGAGGCCGAGGGCGAGGAGGACCCATGGG
CCGTGGAGGCTATGGAGGTGGTGGCAGTGGTGGTGGTGGCCGAGGAGGATTTCCCAG
TGGAGGTGGTGGCGGTGGAGGACAGCAGCGAGCTGGTGACTGGAAGTGTCCTAATCC
CACCTGTGAGAATATGAACTTCTCTTGGAGGAATGAATGCAACCAGTGTAAGGCCCCTA
AACCAGATGGCCCAGGAGGGGGACCAGGTGGCTCTCACATGGGGGGTAACTACGGGG
ATGATCGTCGTGGTGGCAGAGGAGGCGAGCAGAAGCTGATCTCAGAGGAGGACCTGG
GCGCCCCCGGCTCCGCCGGCTCCGCCGCCGGCTCCGGCGCTTCTAACTTTACTCAGTT
CGTTCTCGTCGACAATGGCGGAACTGGCGACGTGACTGTCGCCCCAAGCAACTTCGCT
AACGGGATCGCTGAATGGATCAGCTCTAACTCGCGTTCACAGGCTTACAAAGTAACCTG
TAGCGTTCGTCAGAGCTCTGCGCAGAATCGCAAATACACCATCAAAGTCGAGGTGCCTA
AAGGCGCCTGGCGTTCGTACTTAAATATGGAACTAACCATTCCAATTTTCGCCACGAATT
CCGACTGCGAGCTTATTGTTAAGGCAATGCAAGGTCTCCTAAAAGATGGAAACCCGATT
CCCTCAGCAATCGCAGCAAACTCCGGCATCTACGGTGCCCCAGGCTCCGCAGGAAGCG
CAGCGGGGTCCGGAGCTTCTAACTTTACTCAGTTCGTTCTCGTCGACAATGGCGGAACT
GGCGACGTGACTGTCGCCCCAAGCAACTTCGCTAACGGGATCGCTGAATGGATCAGCT
CTAACTCGCGTTCACAGGCTTACAAAGTAACCTGTAGCGTTCGTCAGAGCTCTGCGCAG
AATCGCAAATACACCATCAAAGTCGAGGTGCCTAAAGGCGCCTGGCGTTCGTACTTAAA
TATGGAACTAACCATTCCAATTTTCGCCACGAATTCCGACTGCGAGCTTATTGTTAAGGC
AATGCAAGGTCTCCTAAAAGATGGAAACCCGATTCCCTCAGCAATCGCAGCAAACTCCG
GCATCTACGGCGCACCTGGTAGTGCTGGTTCTGCTGCTGGATCAGGTGCTTCTAACTTT
ACTCAGTTCGTTCTCGTCGACAATGGCGGAACTGGCGACGTGACTGTCGCCCCAAGCA
ACTTCGCTAACGGGATCGCTGAATGGATCAGCTCTAACTCGCGTTCACAGGCTTACAAA
GTAACCTGTAGCGTTCGTCAGAGCTCTGCGCAGAATCGCAAATACACCATCAAAGTCGA
GGTGCCTAAAGGCGCCTGGCGTTCGTACTTAAATATGGAACTAACCATTCCAATTTTCG
CCACGAATTCCGACTGCGAGCTTATTGTTAAGGCAATGCAAGGTCTCCTAAAAGATGGA
AACCCGATTCCCTCAGCAATCGCAGCAAACTCCGGCATCTACGGTACCGGCGCCCCCG
GCTCCGCCGGCTCCGCCGCCGGCTCCGGCGCGTGCCCGGTGCCGCTGCAGCTGCCG
CCGCTGGAACGCCTGACCCTGGATGATAAAAACCGCTGAATACCCTGATCTCTGCTAC
TGGTCTGTGGATGAGTCGTACCGGAACCATTCATAAAATCAAACACCACGAGGTTAGCC
GTTCGAAAATCTATATTGAGATGGCGTGTGGCGATCATCTGGTTGTGAACAATAGCCGC
 
TCTTCTCGTACAGCACGTGCACTGCGTCACCACAAATATCGTAAAACCTGTAAACGTTG
CCGTGTGTCCGATGAGGATCTGAACAAATTCCTGACAAAAGCCAATGAGGACCAAACAA
GCGTGAAAGTGAAAGTCGTTAGCGCTCCTACCCGTACTAAAAAGCAATGCCGAAATCC
GTTGCTCGTGCCCCTAAACCACTGGAAAACACTGAAGCAGCACAGGCACAGCCGTCTG
GAAGCAAATTCTCTCCGGCCATTCCTGTTTCTACCCAGGAGTCCGTTTCTGTTCCAGCA
AGTGTGAGCACCAGCATTAGCAGTATTAGCACCGGTGCCACCGCTAGCGCCCTGGTTA
AAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAG
CCTGAATTCCGGCAAACCGTTTCGTGAACTGGAGAGCGAACTGCTGTCACGTCGTAAAA
AAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATT
CCTCTGGAGTATATCGAGCGTATGGGCATCGACAATGATACCGAACTGAGCAAACAAAT
TTTCCGTGTGGATAAAAACTTCTGTCTGCGCCCTATGCTAGCACCAAATCTGGCTAACTA
TCTGCGCAAACTGGACCGTGCCCTGCCTGATCCTATCAAAATCTTCGAGATCGGCCCGT
GTTATCGTAAAGAGTCCGACGGTAAAGAACATCTGGAGGAGTTTACCATGCTGAACTTT
TGCCAAATGGGTTCAGGTTGTACTCGTGAGAACCTGGAAAGCATCATCACCGATTTTCT
GAACCACCTGGGCATTGACTTCAAAATTGTGGGCGACAGCTGTATGGTGTTTGGCGACA
CCCTGGATGTCATGCACGGCGACCTGGAACTGTCTAGTGCCGTTGTGGGCCCAATCCC
GCTGGATCGTGAGTGGGGTATCGACAAACCTTGGATCGGTGCGGGTTTTGGTCTGGAG
CGTCTGCTGAAAGTAAAACACGACTTCAAGAACATCAAACGTGCTGCACGTTCCGAGTC
CTATTACAATGGTATTTCTACTAACCTGTAA (SEQ ID NO:133)
Protein:

MGCVCSSNPEGTELASNDYTQQATQSYGAYPTQPGQGYSQQSSQPYGQQSYSGYSQST
DTSGYGQSSYSSYGQSQNTGYGTQSTPQGYGSTGGYGSSQSSQSSYGQQSSYPGYGQQ
PAPSNTSGSYGSSSQSSSYGQPQSGSYSQQPSYGGQQQSYGQQQSYNPPQGYGQQNQ
YNSSSGGGGGGGGGNYGQDQSSMSSGGGSGGGYGNQDQSGGGGSGGYGQQDRGG
RGRGGSGGGGGGGGGYNRSSGGYEPRGRGGGRGGRGGMGGSDRGGFNKFGGPRD
QGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPM
GRGGYGGGGSGGGGRGGFPSGGGGGGGQQRAGDWKCPNPTCENMNFSWRNECNQC
KAPKPDGPGGGPGGSHMGGNYGDDRRGGRGGEQKLISEEDLGAPGSAGSAAGSGASNF
TQFVLVDNGGTGDVTVAPSNFANGIAEWISSNSRSQAYKVTCSVRQSSAQNRKYTIKVEVP
KGAWRSYLNMELTIPIFATNSDCELIVKAMQGLLKDGNPIPSAIAANSGIYGAPGSAGSAAGS
GASNFTQFVLVDNGGTGDVTVAPSNFANGIAEWISSNSRSQAYKVTCSVRQSSAQNRKYTI
KVEVPKGAWRSYLNMELTIPIFATNSDCELIVKAMQGLLKDGNPIPSAIAANSGIYGAPGSAG
SAAGSGASNFTQFVLVDNGGTGDVTVAPSNFANGIAEWISSNSRSQAYKVTCSVRQSSAQ

NRKYTIKVEVPKGAWRSYLNMELTIPIFATNSDCELIVKAMQGLLKDGNPIPSAIAANSGIYGT
GAPGSAGSAAGSGACPVPLQLPPLERLTLDDKKPLNTLISATGLWMSRTGTIHKIKHHEVSR
SKIYIEMACGDHLVVNNSRSSRTARALRHHKYRKTCKRCRVSDEDLNKFLTKANEDQTSVK
VKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFREL
ESELLSRRKKDLQQIYAEERENYLGKLEREITRFFVDRGFLEIKSPILIPLEYIERMGIDNDTEL
SKQIFRVDKNFCLRPMLAPNLANYLRKLDRALPDPIKIFEIGPCYRKESDGKEHLEEFTMLNF
CQMGSGCTRENLESIITDFLNHLGIDFKIVGDSCMVFGDTLDVMHGDLELSSAVVGPIPLDRE
WGIDKPWIGAGFGLERLLKVKHDFKNIKRAARSESYYNGISTNL (SEQ ID NO:134)

LcK-FUS-PyIRS^{AAAF}
DNA: 
ATGGGCTGCGTGTGCAGCAGCAACCCCGAGGGTACCGAGCTCGCCTCAAACGATTATA
CCCAACAAGCAACCCAAAGCTATGGGGCCTACCCCACCCAGCCCGGGCAGGGCTATTC
CCAGCAGAGCAGTCAGCCCTACGGACAGCAGAGTTACAGTGGTTATAGCCAGTCCACG
GACACTTCAGGATATGGCCAGAGCAGCTATTCTTCTTATGGCCAGAGCCAGAACACAGG
CTATGGAACTCAGTCAACTCCCCAGGGATATGGCTCGACTGGCGGCTATGGCAGTAGC
CAGAGCTCCCAATCGTCTTACGGGCAGCAGTCCTCCTACCCTGGCTATGGCCAGCAGC
CAGCTCCCAGCAACACCTCGGGAAGTTACGGTAGCAGTTCTCAGAGCAGCAGCTATGG
GCAGCCCCAGAGTGGGAGCTACAGCCAGCAGCCTAGCTATGGTGGACAGCAGCAAAG
CTATGGACAGCAGCAAAGCTATAATCCCCCTCAGGGCTATGGACAGCAGAACCAGTACA
ACAGCAGCAGTGGTGGTGGAGGTGGAGGTGGAGGTGGAGGTAACTATGGCCAAGATC
AATCCTCCATGAGTAGTGGTGGTGGCAGTGGTGGCGGTTATGGCAATCAAGACCAGAG
TGGTGGAGGTGGCAGCGGTGGCTATGGACAGCAGGACCGTGGAGGCCGCGGCAGGG
GTGGCAGTGGTGGCGGCGGCGGCGGCGGTGGTGGTTACAACCGCAGCAGTGGT
GGCTATGAACCCAGAGGTCGTGGAGGTGGCCGTGGAGGCAGAGGTGGCATGGGCGGA
AGTGACCGTGGTGGCTTCAATAAATTTGGTGGCCCTCGGGACCAAGGATCACGTCATG
ACTCCGAACAGGATAATTCAGACAACAACACCATCTTTGTGCAAGGCCTGGGTGAGAAT
GTTACAATTGAGTCTGTGGCTGATTACTTCAAGCAGATTGGTATTATTAAGACAAACAAG
AAAACGGGACAGCCCATGATTAATTTGTACACAGACAGGGAAACTGGCAAGCTGAAGG
GAGAGGCAACGGTCTCTTTTGATGACCCACCTTCAGCTAAAGCAGCTATTGACTGGTTT
GATGGTAAAGAATTCTCCGGAAATCCTATCAAGGTCTCATTTGCTACTCGCCGGGCAGA
CTTTAATCGGGGTGGTGGCAATGGTCGTGGAGGCCGAGGGCGAGGAGGACCCATGGG
CCGTGGAGGCTATGGAGGTGGTGGCAGTGGTGGTGGTGGCCGAGGAGGATTTCCCAG
TGGAGGTGGTGGCGGTGGAGGACAGCAGCGAGCTGGTGACTGGAAGTGTCCTAATCC
CACCTGTGAGAATATGAACTTCTCTTGGAGGAATGAATGCAACCAGTGTAAGGCCCCTA
 
AACCAGATGGCCCAGGAGGGGGACCAGGTGGCTCTCACATGGGGGGTAACTACGGGG
ATGATCGTCGTGGTGGCAGAGGAGGCGATTACAAGGATGACGACGATAAGGGTACCGG
CGCCCCCGGCTCCGCCGGCTCCGCCGCCGGCTCCGGCGCGTGCCCGGTGCCGCTGC
AGCTGCCGCCGCTGGAACGCCTGACCCTGGATGATAAAAACCGCTGAATACCCTGAT
CTCTGCTACTGGTCTGTGGATGAGTCGTACCGGAACCATTCATAAAATCAAACACCACG
AGGTTAGCCGTTCGAAAATCTATATTGAGATGGCGTGTGGCGATCATCTGGTTGTGAAC
AATAGCCGCTCTTCTCGTACAGCACGTGCACTGCGTCACCACAAATATCGTAAAACCTG
TAAACGTTGCCGTGTGTCCGATGAGGATCTGAACAAATTCCTGACAAAAGCCAATGAGG
ACCAAACAAGCGTGAAAGTGAAAGTCGTTAGCGCTCCTACCCGTACTAAAAAGCAATG
CCGAAATCCGTTGCTCGTGCCCCTAAACCACTGGAAAACACTGAAGCAGCACAGGCAC
AGCCGTCTGGAAGCAAATTCTCTCCGGCCATTCCTGTTTCTACCCAGGAGTCCGTTTCT
GTTCCAGCAAGTGTGAGCACCAGCATTAGCAGTATTAGCACCGGTGCCACCGCTAGCG
CCCTGGTTAAAGGCAATACCAATCCGATTACAAGCATGTCTGCCCCGGTTCAAGCATCA
GCTCCAGCACTGACAAAATCCCAAACCGATCGTCTGGAGGTTCTGCTGAATCCGAAAGA
CGAAATCAGCCTGAATTCCGGCAAACCGTTTCGTGAACTGGAGAGCGAACTGCTGTCAC
GTCGTAAAAAAGACCTGCAACAAATCTATGCCGAAGAACGTGAGAACTATCTGGGGAAA
CTGGAACGTGAAATCACCCGCTTTTTCGTGGATCGTGGCTTTCTGGAGATCAAATCCCC
GATTCTGATTCCTCTGGAGTATATCGAGCGTATGGGCATCGACAATGATACCGAACTGA
GCAAACAAATTTTCCGTGTGGATAAAAACTTCTGTCTGCGCCCTATGCTAGCACCAAATC
TGGCTAACTATCTGCGCAAACTGGACCGTGCCCTGCCTGATCCTATCAAAATCTTCGAG
ATCGGCCCGTGTTATCGTAAAGAGTCCGACGGTAAAGAACATCTGGAGGAGTTTACCAT
GCTGGCCTTTGCCCAAATGGGTTCAGGTTGTACTCGTGAGAACCTGGAAAGCATCATCA
CCGATTTTCTGAACCACCTGGGCATTGACTTCAAAATTGTGGGCGACAGCTGTATGGTG
TTTGGCGACACCCTGGATGTCATGCACGGCGACCTGGAACTGTCTAGTGCCGTTGTGG
GCCCAATCCCGCTGGATCGTGAGTGGGGTATCGACAAACCTTGGATCGGTGCGGGTTT
TGGTCTGGAGCGTCTGCTGAAAGTAAAACACGACTTCAAGAACATCAAACGTGCTGCAC
GTTCCGAGTCCTATTACAATGGTATTTCTACTAACCTGTAA (SEQ ID NO:135)
Protein:

MGCVCSSNPEGTELASNDYTQQATQSYGAYPTQPGQGYSQQSSQPYGQQSYSGYSQST
DTSGYGQSSYSSYGQSQNTGYGTQSTPQGYGSTGGYGSSQSSQSSYGQQSSYPGYGQQ
PAPSNTSGSYGSSSQSSSYGQPQSGSYSQQPSYGGQQQSYGQQQSYNPPQGYGQQNQ
YNSSSGGGGGGGGGGNYGQDQSSMSSGGGSGGGYGNQDQSGGGGSGGYGQQDRGG
RGRGGSGGGGGGGGGGYNRSSGGYEPRGRGGGRGGRGGMGGSDRGGFNKFGGPRD
QGSRHDSEQDNSDNNTIFVQGLGENVTIESVADYFKQIGIIKTNKKTGQPMINLYTDRETGKL
KGEATVSFDDPPSAKAAIDWFDGKEFSGNPIKVSFATRRADFNRGGGNGRGGRGRGGPM
GRGGYGGGGSGGGGRGGFPSGGGGGGGQQRAGDWKCPNPTCENMNFSWRNECNQC

KAPKPDGPGGGPGGSHMGGNYGDDRRGGRGGDYKDDDDKGTGAPGSAGSAAGSGACP
VPLQLPPLERLTLDDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKS
VARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAE
ERENYLGKLEREITRFFVDRGFLEIKSPILIPLEYIERMGIDNDTELSKQIFRVDKNFCLRPMLA
PNLANYLRKLDRALPDPIKIFEIGPCYRKESDGKEHLEEFTMLAFAQMGSGCTRENLESIITDF
LNHLGIDFKIVGDSCMVFGDTLDVMHGDLELSSAVVGPIPLDREWGIDKPWIGAGFGLERLL
KVKHDFKNIKRAARSESYYNGISTNL (SEQ ID NO:136)

LcK-PyIRS^{AF}
DNA: 
ATGGGCTGCGTGTGCAGCAGCAACCCCGAGGGTACCGAGCTCGCGTGCCCGGTGCCG
CTGCAGCTGCCGCCGCTGGAACGCCTGACCCTGGATGATAAAAACCGCTGAATACCC
TGATCTCTGCTACTGGTCTGTGGATGAGTCGTACCGGAACCATTCATAAAATCAAACAC
CACGAGGTTAGCCGTTCGAAAATCTATATTGAGATGGCGTGTGGCGATCATCTGGTTGT
GAACAATAGCCGCTCTTCTCGTACAGCACGTGCACTGCGTCACCACAAATATCGTAAAA
CCTGTAAACGTTGCCGTGTGTCCGATGAGGATCTGAACAAATTCCTGACAAAAGCCAAT
GAGGACCAAACAAGCGTGAAAGTGAAAGTCGTTAGCGCTCCTACCCGTACTAAAAAGC
AATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGT
TTCTGTTCCAGCAAGTGTGAGCACCAGCATTAGCAGTATTAGCACCGGTGCCACCGCTA
GCGCCCTGGTTAAAGGCAATACCAATCCGATTACAAGCATGTCTGCCCCGGTTCAAGCA
TCAGCTCCAGCACTGACAAAATCCCAAACCGATCGTCTGGAGGTTCTGCTGAATCCGAA
AGACGAAATCAGCCTGAATTCCGGCAAACCGTTTCGTGAACTGGAGAGCGAACTGCTGT
CACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATC
CCCGATTCTGATTCCTCTGGAGTATATCGAGCGTATGGGCATCGACAATGATACCGAAC
TGAGCAAACAAATTTTCCGTGTGGATAAAAACTTCTGTCTGCGCCCTATGCTAGCACCAA
ATCTGGCTAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTAC
CATGCTGAACTTTTGCCAAATGGGTTCAGGTTGTACTCGTGAGAACCTGGAAAGCATCA
TCACCGATTTTCTGAACCACCTGGGCATTGACTTCAAAATTGTGGGCGACAGCTGTATG
GTGTTTGGCGACACCCTGGATGTCATGCACGGCGACCTGGAACTGTCTAGTGCCGTTG
TGGGCCCAATCCCGCTGGATCGTGAGTGGGGTATCGACAAACCTTGGATCGGTGCGGG
 
TTTTGGTCTGGAGCGTCTGCTGAAAGTAAAACACGACTTCAAGAACATCAAACGTGCTG
CACGTTCCGAGTCCTATTACAATGGTATTTCTACTAACCTGTAA (SEQ ID NO:137)
Protein:

MGCVCSSNPEGTELACPVPLQLPPLERLTLDDKKPLNTLISATGLWMSRTGTIHKIKHHEVS
RSKIYIEMACGDHLVVNNSRSSRTARALRHHKYRKTCKRCRVSDEDLNKFLTKANEDQTSV
KVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFR
ELESELLSRRKKDLQQIYAEERENYLGKLEREITRFFVDRGFLEIKSPILIPLEYIERMGIDNDT
ELSKQIFRVDKNFCLRPMLAPNLANYLRKLDRALPDPIKIFEIGPCYRKESDGKEHLEEFTML
NFCQMGSGCTRENLESIITDFLNHLGIDFKIVGDSCMVFGDTLDVMHGDLELSSAVVGPIPLD
REWGIDKPWIGAGFGLERLLKVKHDFKNIKRAARSESYYNGISTNL (SEQ ID NO:138)

LcK-PyIRS^{AAAF}
DNA: 
ATGGGCTGCGTGTGCAGCAGCAACCCCGAGGGTACCGAGCTCGCGTGCCCGGTGCCG
CTGCAGCTGCCGCCGCTGGAACGCCTGACCCTGGATGATAAAAACCGCTGAATACCC
TGATCTCTGCTACTGGTCTGTGGATGAGTCGTACCGGAACCATTCATAAAATCAAACAC
CACGAGGTTAGCCGTTCGAAAATCTATATTGAGATGGCGTGTGGCGATCATCTGGTTGT
GAACAATAGCCGCTCTTCTCGTACAGCACGTGCACTGCGTCACCACAAATATCGTAAAA
CCTGTAAACGTTGCCGTGTGTCCGATGAGGATCTGAACAAATTCCTGACAAAAGCCAAT
GAGGACCAAACAAGCGTGAAAGTGAAAGTCGTTAGCGCTCCTACCCGTACTAAAAAGC
AATGCCGAAATCCGTTGCTCGTGCCCCTAAACCACTGGAAAACACTGAAGCAGCACAG
GCACAGCCGTCTGGAAGCAAATTCTCTCCGGCCATTCCTGTTTCTACCCAGGAGTCCGT
TTCTGTTCCAGCAAGTGTGAGCACCAGCATTAGCAGTATTAGCACCGGTGCCACCGCTA
GCGCCCTGGTTAAAGGCAATACCAATCCGATTACAAGCATGTCTGCCCCGGTTCAAGCA
TCAGCTCCAGCACTGACAAAATCCCAAACCGATCGTCTGGAGGTTCTGCTGAATCCGAA
AGACGAAATCAGCCTGAATTCCGGCAAACCGTTTCGTGAACTGGAGAGCGAACTGCTGT
CACGTCGTAAAAAAGACCTGCAACAAATCTATGCCGAAGAACGTGAGAACTATCTGGGG
AAACTGGAACGTGAAATCACCCGCTTTTTCGTGGATCGTGGCTTTCTGGAGATCAAATC
CCCGATTCTGATTCCTCTGGAGTATATCGAGCGTATGGGCATCGACAATGATACCGAAC
TGAGCAAACAAATTTTCCGTGTGGATAAAAACTTCTGTCTGCGCCCTATGCTAGCACCAA
ATCTGGCTAACTATCTGCGCAAACTGGACCGTGCCCTGCCTGATCCTATCAAAATCTTC
GAGATCGGCCCGTGTTATCGTAAAGAGTCCGACGGTAAAGAACATCTGGAGGAGTTTAC
CATGCTGGCCTTTGCCCAAATGGGTTCAGGTTGTACTCGTGAGAACCTGGAAAGCATCA
TCACCGATTTTCTGAACCACCTGGGCATTGACTTCAAAATTGTGGGCGACAGCTGTATG
GTGTTTGGCGACACCCTGGATGTCATGCACGGCGACCTGGAACTGTCTAGTGCCGTTG
 
TGGGCCCAATCCCGCTGGATCGTGAGTGGGGTATCGACAAACCTTGGATCGGTGCGGG
TTTTGGTCTGGAGCGTCTGCTGAAAGTAAAACACGACTTCAAGAACATCAAACGTGCTG
CACGTTCCGAGTCCTATTACAATGGTATTTCTACTAACCTGTAA (SEQ ID NO:139)
Protein:

MGCVCSSNPEGTELACPVPLQLPPLERLTLDDKKPLNTLISATGLWMSRTGTIHKIKHHEVS
RSKIYIEMACGDHLVVNNSRSSRTARALRHHKYRKTCKRCRVSDEDLNKFLTKANEDQTSV
KVKVVSAPTRTKKAMPKSVARAPKPLENTEAAQAQPSGSKFSPAIPVSTQESVSVPASVST
SISSISTGATASALVKGNTNPITSMSAPVQASAPALTKSQTDRLEVLLNPKDEISLNSGKPFR
ELESELLSRRKKDLQQIYAEERENYLGKLEREITRFFVDRGFLEIKSPILIPLEYIERMGIDNDT
ELSKQIFRVDKNFCLRPMLAPNLANYLRKLDRALPDPIKIFEIGPCYRKESDGKEHLEEFTML
AFAQMGSGCTRENLESIITDFLNHLGIDFKIVGDSCMVFGDTLDVMHGDLELSSAVVGPIPLD
REWGIDKPWIGAGFGLERLLKVKHDFKNIKRAARSESYYNGISTNL (SEQ ID NO:140)

LcK-MCP
DNA: 
ATGGGCTGCGTGTGCAGCAGCAACCCCGAGGGTACCGAGCTCGAGCAGAAGCTGATC
TCAGAGGAGGACCTGGGCGCCCCGGCTCCGCCGGCTCCGCCGCCGGCTCCGGCGC
TTCTAACTTTACTCAGTTCGTTCTCGTCGACAATGGCGGAACTGGCGACGTGACTGTCG
CCCCAAGCAACTTCGCTAACGGGATCGCTGAATGGATCAGCTCTAACTCGCGTTCACAG
GCTTACAAAGTAACCTGTAGCGTTCGTCAGAGCTCTGCGCAGAATCGCAAATACACCAT
CAAAGTCGAGGTGCCTAAAGGCGCCTGGCGTTCGTACTTAAATATGGAACTAACCATTC
CAATTTTCGCCACGAATTCCGACTGCGAGCTTATTGTTAAGGCAATGCAAGGTCTCCTAA
AAGATGGAAACCCGATTCCCTCAGCAATCGCAGCAAACTCCGGCATCTACTAA (SEQ ID
NO:141)
Protein:

MGCVCSSNPEGTELEQKLISEEDLGAPGSAGSAAGSGASNFTQFVLVDNGGTGDVTVAPS
NFANGIAEWISSNSRSQAYKVTCSVRQSSAQNRKYTIKVEVPKGAWRSYLNMELTIPIFATN
SDCELIVKAMQGLLKDGNPIPSAIAANSGIY (SEQ ID NO:142)

FRB-CD28-FUS-PyIRS^{AF}
DNA: 
ATGTGCCGAGCCATCTCTCTTAGGCGCTTGCTGCTGCTGCTGCAGCTGTCACAACT
CCTAGCTGTCACTCAAGGGATGCTCGAGATGTGGCATGAAGGCCTGGAAGAGGCATCT
CGTTTGTACTTTGGGGAAAGGAACGTGAAAGGCATGTTTGAGGTGCTGGAGCCCTTGC
ATGCTATGATGGAACGGGGCCCCCAGACTCTGAAGGAAACATCCTTTAATCAGGCCTAT
GGTCGAGATTTAATGGAGGCCCAAGAGTGGTGCAGGAAGTACATGAAATCAGGGAATG
 
TCAAGGACCTCCTCCAAGCCTGGACCTCTATTATCATGTGTTCCGACGAATCTCAAAG
ACTAGAACCGGTAAGCTTTTTTGGGCACTGGTCGTGGTTGCTGGAGTCCTGTTTTGTTA
TGGCTTGCTAGTGACAGTGGCTCTTTGTGTTATCTGGGTAAGATCTGGTATGGCCTCAA
ACGATTATACCCAACAAGCAACCCAAAGCTATGGGGCCTACCCCACCCAGCCCGGGCA
GGGCTATTCCCAGCAGAGCAGTCAGCCCTACGGACAGCAGAGTTACAGTGGTTATAGC
CAGTCCACGGACACTTCAGGATATGGCCAGAGCAGCTATTCTTCTTATGGCCAGAGCCA
GAACACAGGCTATGGAACTCAGTCAACTCCCCAGGGATATGGCTCGACTGGCGGCTAT
GGCAGTAGCCAGAGCTCCCAATCGTCTTACGGGCAGCAGTCCTCCTACCCTGGCTATG
GCCAGCAGCCAGCTCCCAGCAGCACCTCGGGAAGTTACGGTAGCAGTTCTCAGAGCAG
CAGCTATGGGCAGCCCCAGAGTGGGAGCTACAGCCAGCAGCCTAGCTATGGTGGACA
GCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTAT
GGCCAAGATCAATCCTCCATGAGTAGTGGTGGTGGCAGTGGTGGCGGTTATGGCAATC
AAGACCAGAGTGGTGGAGGTGGCAGCGGTGGCTATGGACAGCAGGACCGTGGAGGCC
GCGGCAGGGGTGGCAGTGGTGGCGGCGGCGGCGGCGGTGGTGGTTACAACCGC
AGCAGTGGTGGCTATGAACCCAGAGGTCGTGGAGGTGGCCGTGGAGGCAGAGGTGGC
ATGGGCGGAAGTGACCGTGGTGGCTTCAATAAATTTGGTGGCCCTCGGGACCAAGGAT
CACGTCATGACTCCGAACAGGATAATTCAGACAACAACACCATCTTTGTGCAAGGCCTG
GGTGAGAATGTTACAATTGAGTCTGTGGCTGATTACTTCAAGCAGATTGGTATTATTAAG
ACAAACAAGAAAACGGACAGCCCATGATTAATTTGTACACAGACAGGGAAACTGGCAA
GCTGAAGGGAGAGGCAACGGTCTCTTTTGATGACCCACCTTCAGCTAAAGCAGCTATTG
ACTGGTTTGATGGTAAAGAATTCTCCGGAAATCCTATCAAGGTCTCATTTGCTACTCGCC
GGGCAGACTTTAATCGGGGTGGTGGCAATGGTCGTGGAGGCCGAGGGCGAGGAGGAC
CCATGGGCCGTGGAGGCTATGGAGGTGGTGGCAGTGGTGGTGGTGGCCGAGGAGGAT
TTCCCAGTGGAGGTGGTGGCGGTGGAGGACAGCAGCGAGCTGGTGACTGGAAGTGTC
CTAATCCCACCTGTGAGAATATGAACTTCTCTTGGAGGAATGAATGCAACCAGTGTAAG
GCCCCTAAACCAGATGGCCCAGGAGGGGGACCAGGTGGCTCTCACATGGGGGGTAAC
TACGGGGATGATCGTCGTGGTGGCAGAGGAGGCGGCGCCCCCGGCTCCGCCGGCTC
CGCCGCCGGCTCCGGCATGGCGTGCCCGGTGCCGCTGCAGCTGCCGCCGCTGGAAC
GCCTGACCCTGGATGATAAAAAACCGCTGAATACCCTGATCTCTGCTACTGGTCTGTGG
ATGAGTCGTACCGGAACCATTCATAAAATCAAACACCACGAGGTTAGCCGTTCGAAAAT
CTATATTGAGATGGCGTGTGGCGATCATCTGGTTGTGAACAATAGCCGCTCTTCTCGTA
CAGCACGTGCACTGCGTCACCACAAATATCGTAAAACCTGTAAACGTTGCCGTGTGTCC
GATGAGGATCTGAACAAATTCCTGACAAAAGCCAATGAGGACCAAACAAGCGTGAAAGT
GAAAGTCGTTAGCGCTCCTACCCGTACTAAAAAAGCAATGCCGAAATCCGTTGCTCGTG
CCCCTAAACCACTGGAAAACACTGAAGCAGCACAGGCACAGCCGTCTGGAAGCAAATT
 
CTCTCCGGCCATTCCTGTTTCTACCCAGGAGTCCGTTTCTGTTCCAGCAAGTGTGAGCA
CCAGCATTAGCAGTATTAGCACCGGTGCCACCGCTAGCGCCCTGGTTAAAGGCAATAC
CAATCCGATTACAAGCATGTCTGCCCCGGTTCAAGCATCAGCTCCAGCACTGACAAAAT
CCCAAACCGATCGTCTGGAGGTTCTGCTGAATCCGAAAGACGAAATCAGCCTGAATTCC
GGCAAACCGTTTCGTGAACTGGAGAGCGAACTGCTGTCACGTCGTAAAAAAGACCTGC
AACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGA
GTATATCGAGCGTATGGGCATCGACAATGATACCGAACTGAGCAAACAAATTTTCCGTG
TGGATAAAAACTTCTGTCTGCGCCCTATGCTAGCACCAAATCTGGCTAACTATCTGCGC
AAACTGGACCGTGCCCTGCCTGATCCTATCAAAATCTTCGAGATCGGCCCGTGTTATCG
TAAAGAGTCCGACGGTAAAGAACATCTGGAGGAGTTTACCATGCTGAACTTTTGCCAAA
TGGGTTCAGGTTGTACTCGTGAGAACCTGGAAAGCATCATCACCGATTTTCTGAACCAC
CTGGGCATTGACTTCAAAATTGTGGGCGACAGCTGTATGGTGTTTGGCGACACCCTGGA
TGTCATGCACGGCGACCTGGAACTGTCTAGTGCCGTTGTGGGCCCAATCCCGCTGGAT
CGTGAGTGGGGTATCGACAAACCTTGGATCGGTGCGGGTTTTGGTCTGGAGCGTCTGC
TGAAAGTAAAACACGACTTCAAGAACATCAAACGTGCTGCACGTTCCGAGTCCTATTACA
ATGGTATTTCTACTAACCTGTAA (SEQ ID NO:143)
Protein:

MCRAISLRRLLLLLLQLSQLLAVTQGMLEMWHEGLEEASRLYFGERNVKGMFEVLEPLHAM
MERGPQTLKETSFNQAYGRDLMEAQEWCRKYMKSGNVKDLLQAWDLYYHVFRRISKTRT
GKLFWALVVVAGVLFCYGLLVTVALCVIWVRSGMASNDYTQQATQSYGAYPTQPGQGYSQ
QSSQPYGQQSYSGYSQSTDTSGYGQSSYSSYGQSQNTGYGTQSTPQGYGSTGGYGSSQ
SSQSSYGQQSSYPGYGQQPAPSSTSGSYGSSSQSSSYGQPQSGSYSQQPSYGGQQQSY
GQQQSYNPPQGYGQQNQYNSSSGGGGGGGGGNYGQDQSSMSSGGGSGGGYGNQD
QSGGGGSGGYGQQDRGGRGRGGSGGGGGGGGGYNRSSGGYEPRGRGGGRGGRGG
MGGSDRGGFNKFGGPRDQGSRHDSEQDNSDNNTIFVQGLGENVTIESVADYFKQIGIIKTN
KKTGQPMINLYTDRETGKLKGEATVSFDDPPSAKAAIDWFDGKEFSGNPIKVSFATRRADFN
RGGGNGRGGRGRGGPMGRGGYGGGGSGGGGRGGFPSGGGGGGGQQRAGDWKCPNP
TCENMNFSWRNECNQCKAPKPDGPGGGPGGSHMGGNYGDDRRGGRGGGAPGSAGSA
AGSGMACPVPLQLPPLERLTLDDKKPLNTLISATGLWMSRTGTIHKIKHHEVSRSKIYIEMAC
GDHLVVNNSRSSRTARALRHHKYRKTCKRCRVSDEDLNKFLTKANEDQTSVKVKVVSAPT
RTKKAMPKSVARAPKPLENTEAAQAQPSGSKFSPAIPVSTQESVSVPASVSTSISSISTGAT
ASALVKGNTNPITSMSAPVQASAPALTKSQTDRLEVLLNPKDEISLNSGKPFRELESELLSRR
KKDLQQIYAEERENYLGKLEREITRFFVDRGFLEIKSPILIPLEYIERMGIDNDTELSKQIFRVD
KNFCLRPMLAPNLANYLRKLDRALPDPIKIFEIGPCYRKESDGKEHLEEFTMLNFCQMGSGC

TRENLESIITDFLNHLGIDFKIVGDSCMVFGDTLDVMHGDLELSSAVVGPIPLDREWGIDKPWI
GAGFGLERLLKVKHDFKNIKRAARSESYYNGISTNL (SEQ ID NO:144)

FRB-CD28-FUS-PyIRS^{AA}
DNA: 
ATGTGCCGAGCCATCTCTCTTAGGCGCTTGCTGCTGCTGCTGCAGCTGTCACAACT
CCTAGCTGTCACTCAAGGGATGCTCGAGATGTGGCATGAAGGCCTGGAAGAGGCATCT
CGTTTGTACTTTGGGGAAAGGAACGTGAAAGGCATGTTTGAGGTGCTGGAGCCCTTGC
ATGCTATGATGGAACGGGGCCCCCAGACTCTGAAGGAAACATCCTTTAATCAGGCCTAT
GGTCGAGATTTAATGGAGGCCCAAGAGTGGTGCAGGAAGTACATGAAATCAGGGAATG
TCAAGGACCTCCTCCAAGCCTGGGACCTCTATTATCATGTGTTCCGACGAATCTCAAAG
ACTAGAACCGGTAAGCTTTTTGGGCACTGGTCGTGGTTGCTGGAGTCCTGTTTTGTTA
TGGCTTGCTAGTGACAGTGGCTCTTTGTGTTATCTGGGTAAGATCTGGTATGGCCTCAA
ACGATTATACCCAACAAGCAACCCAAAGCTATGGGGCCTACCCCACCCAGCCCGGGCA
GGGCTATTCCCAGCAGAGCAGTCAGCCCTACGGACAGCAGAGTTACAGTGGTTATAGC
CAGTCCACGGACACTTCAGGATATGGCCAGAGCAGCTATTCTTCTTATGGCCAGAGCCA
GAACACAGGCTATGGAACTCAGTCAACTCCCCAGGGATATGGCTCGACTGGCGGCTAT
GGCAGTAGCCAGAGCTCCCAATCGTCTTACGGGCAGCAGTCCTCCTACCCTGGCTATG
GCCAGCAGCCAGCTCCCAGCAGCACCTCGGGAAGTTACGGTAGCAGTTCTCAGAGCAG
CAGCTATGGGCAGCCCCAGAGTGGGAGCTACAGCCAGCAGCCTAGCTATGGTGGACA
GCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTAT
GGCCAAGATCAATCCTCCATGAGTAGTGGTGGTGGCAGTGGTGGCGGTTATGGCAATC
AAGACCAGAGTGGTGGAGGTGGCAGCGGTGGCTATGGACAGCAGGACCGTGGAGGCC
GCGGCAGGGGTGGCAGTGGTGGCGGCGGCGGCGGCGGTGGTGGTTACAACCGC
AGCAGTGGTGGCTATGAACCCAGAGGTCGTGGAGGTGGCCGTGGAGGCAGAGGTGGC
ATGGGCGGAAGTGACCGTGGTGGCTTCAATAAATTTGGTGGCCCTCGGGACCAAGGAT
CACGTCATGACTCCGAACAGGATAATTCAGACAACAACACCATCTTTGTGCAAGGCCTG
GGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAA
GCTGAAGGGAGAGGCAACGGTCTCTTTTGATGACCCACCTTCAGCTAAAGCAGCTATTG
ACTGGTTTGATGGTAAAGAATTCTCCGGAAATCCTATCAAGGTCTCATTTGCTACTCGCC
GGGCAGACTTTAATCGGGGTGGTGGCAATGGTCGTGGAGGCCGAGGGCGAGGAGGAC
CCATGGGCCGTGGAGGCTATGGAGGTGGTGGCAGTGGTGGTGGTGGCCGAGGAGGAT
TTCCCAGTGGAGGTGGTGGCGGTGGAGGACAGCAGCGAGCTGGTGACTGGAAGTGTC
CTAATCCCACCTGTGAGAATATGAACTTCTCTTGGAGGAATGAATGCAACCAGTGTAAG
 
GCCCCTAAACCAGATGGCCCAGGAGGGGACCAGGTGGCTCTCACATGGGGGGTAAC
TACGGGGATGATCGTCGTGGTGGCAGAGGAGGCGGCGCCCCCGGCTCCGCCGGCTC
CGCCGCCGGCTCCGGCATGGCGTGCCCGGTGCCGCTGCAGCTGCCGCCGCTGGAAC
GCCTGACCCTGGATGACAAAAAACCGCTGAATACCCTGATCTCTGCTACTGGTCTGTGG
ATGAGTCGTACCGGAACCATTCATAAAATCAAACACCACGAGGTTAGCCGTTCGAAAAT
CTATATTGAGATGGCGTGTGGCGATCATCTGGTTGTGAACAATAGCCGCTCTTCTCGTA
CAGCACGTGCACTGCGTCACCACAAATATCGTAAAACCTGTAAACGTTGCCGTGTGTCC
GATGAGGATCTGAACAAATTCCTGACAAAAGCCAATGAGGACCAAACAAGCGTGAAAGT
GAAAGTCGTTAGCGCTCCTACCCGTACTAAAAAAGCAATGCCGAAATCCGTTGCTCGTG
CCCCTAAACCACTGGAAAACACTGAAGCAGCACAGGCACAGCCGTCTGGAAGCAAATT
CTCTCCGGCCATTCCTGTTTCTACCCAGGAGTCCGTTTCTGTTCCAGCAAGTGTGAGCA
CCAGCATTAGCAGTATTAGCACCGGTGCCACCGCTAGCGCCCTGGTTAAAGGCAATAC
CAATCCGATTACAAGCATGTCTGCCCCGGTTCAAGCATCAGCTCCAGCACTGACAAAAT
CCCAAACCGATCGTCTGGAGGTTCTGCTGAATCCGAAAGACGAAATCAGCCTGAATTCC
GGCAAACCGTTTCGTGAACTGGAGAGCGAACTGCTGTCACGTCGTAAAAAAGACCTGC
AACAAATCTATGCCGAAGAACGTGAGAACTATCTGGGGAAACTGGAACGTGAAATCACC
CGCTTTTTCGTGGATCGTGGCTTTCTGGAGATCAAATCCCCGATTCTGATTCCTCTGGA
GTATATCGAGCGTATGGGCATCGACAATGATACCGAACTGAGCAAACAAATTTTCCGTG
TGGATAAAAACTTCTGTCTTCGCCCTATGCTGGCACCAAATCTGTATAACTATCTGCGCA
AACTGGACCGTGCCCTGCCTGATCCTATCAAAATCTTCGAGATCGGCCCGTGTTATCGT
AAAGAGTCCGACGGTAAAGAACATCTGGAGGAGTTTACCATGCTGGCCTTTGCCCAAAT
GGGTTCAGGTTGTACTCGTGAGAACCTGGAAAGCATCATCACCGATTTTCTGAACCACC
TGGGCATTGACTTCAAAATTGTGGGCGACAGCTGTATGGTGTATGGCGACACCCTGGAT
GTCATGCACGGCGACCTGGAACTGTCTAGTGCCGTTGTTGGACCAATTCCGCTGGACC
GTGAGTGGGGTATCGACAAACCGTGGATCGGAGCAGGATTCGGTCTGGAACGCCTGCT
GAAAGTGAAACACGACTTCAAAAACATCAAACGTGCCGCCCGTTCTGAATCGTATTATAA
CGGGATCTCTACGAACCTGTAA (SEQ ID NO:145)
Protein:

MCRAISLRRLLLLLLQLSQLLAVTQGMLEMWHEGLEEASRLYFGERNVKGMFEVLEPLHAM
MERGPQTLKETSFNQAYGRDLMEAQEWCRKYMKSGNVKDLLQAWDLYYHVFRRISKTRT
GKLFWALVVVAGVLFCYGLLVTVALCVIWVRSGMASNDYTQQATQSYGAYPTQPGQGYSQ
QSSQPYGQQSYSGYSQSTDTSGYGQSSYSSYGQSQNTGYGTQSTPQGYGSTGGYGSSQ
SSQSSYGQQSSYPGYGQQPAPSSTSGSYGSSSQSSSYGQPQSGSYSQQPSYGGQQQSY
GQQQSYNPPQGYGQQNQYNSSSGGGGGGGGGNYGQDQSSMSSGGGSGGGYGNQD
QSGGGGSGGYGQQDRGGRGRGGSGGGGGGGGGYNRSSGGYEPRGRGGGRGGRGG
MGGSDRGGFNKFGGPRDQGSRHDSEQDNSDNNTIFVQGLGENVTIESVADYFKQIGIIKTN

KKTGQPMINLYTDRETGKLKGEATVSFDDPPSAKAAIDWFDGKEFSGNPIKVSFATRRADFN
RGGGNGRGGRGRGGPMGRGGYGGGGSGGGGRGGFPSGGGGGGGQQRAGDWKCPNP
TCENMNFSWRNECNQCKAPKPDGPGGGPGGSHMGGNYGDDRRGGRGGGAPGSAGSA
AGSGMACPVPLQLPPLERLTLDDKKPLNTLISATGLWMSRTGTIHKIKHHEVSRSKIYIEMAC
GDHLVVNNSRSSRTARALRHHKYRKTCKRCRVSDEDLNKFLTKANEDQTSVKVKVVSAPT
RTKKAMPKSVARAPKPLENTEAAQAQPSGSKFSPAIPVSTQESVSVPASVSTSISSISTGAT
ASALVKGNTNPITSMSAPVQASAPALTKSQTDRLEVLLNPKDEISLNSGKPFRELESELLSRR
KKDLQQIYAEERENYLGKLEREITRFFVDRGFLEIKSPILIPLEYIERMGIDNDTELSKQIFRVD
KNFCLRPMLAPNLYNYLRKLDRALPDPIKIFEIGPCYRKESDGKEHLEEFTMLAFAQMGSGC
TRENLESIITDFLNHLGIDFKIVGDSCMVYGDTLDVMHGDLELSSAVVGPIPLDREWGIDKPW
IGAGFGLERLLKVKHDFKNIKRAARSESYYNGISTNL (SEQ ID NO:146)

FRB-CD28-EWSR1-MCP
DNA: 
ATGTGCCGAGCCATCTCTCTTAGGCGCTTGCTGCTGCTGCTGCAGCTGTCACAACT
CCTAGCTGTCACTCAAGGGATGCTCGAGATGTGGCATGAAGGCCTGGAAGAGGCATCT
CGTTTGTACTTTGGGGAAAGGAACGTGAAAGGCATGTTTGAGGTGCTGGAGCCCTTGC
ATGCTATGATGGAACGGGGCCCCCAGACTCTGAAGGAAACATCCTTTAATCAGGCCTAT
GGTCGAGATTTAATGGAGGCCCAAGAGTGGTGCAGGAAGTACATGAAATCAGGGAATG
TCAAGGACCTCCTCCAAGCCTGGGACCTCTATTATCATGTGTTCCGACGAATCTCAAAG
ACTAGAACCGGTAAGCTTTTTTGGGCACTGGTCGTGGTTGCTGGAGTCCTGTTTTGTTA
TGGCTTGCTAGTGACAGTGGCTCTTTGTGTTATCTGGGTAAGATCTATGGCGTCCACGG
ATTACAGTACCTATAGCCAAGCTGCAGCGCAGCAGGGCTACAGTGCTTACACCGCCCA
GCCCACTCAAGGATATGCACAGACCACCCAGGCATATGGGCAACAAAGCTATGGAACC
TATGGACAGCCCACTGATGTCAGCTATACCCAGGCTCAGACCACTGCAACCTATGGGCA
GACCGCCTATGCAACTTCTTATGGACAGCCTCCCACTGGTTATACTACTCCAACTGCCC
CCCAGGCATACAGCCAGCCTGTCCAGGGGTATGGCACTGGTGCTTATGATACCACCAC
TGCTACAGTCACCACCACCCAGGCCTCCTATGCAGCTCAGTCTGCATATGGCACTCAGC
CTGCTTATCCAGCCTATGGGCAGCAGCCAGCAGCCACTGCACCTACAAGACCGCAGGA
TGGAAACAAGCCCACTGAGACTAGTCAACCTCAATCTAGCACAGGGGGTTACAACCAGC
CCAGCCTAGGATATGGACAGAGTAACTACAGTTATCCCCAGGTACCTGGGAGCTACCC
CATGCAGCCAGTCACTGCACCTCCATCCTACCCTCCTACCAGCTATTCCTCTACACAGC
CGACTAGTTATGATCAGAGCAGTTACTCTCAGCAGAACACCTATGGGCAACCGAGCAGC
TATGGACAGCAGAGTAGCTATGGTCAACAAAGCAGCTATGGGCAGCAGCCTCCCACTA
GTTACCCACCCCAAACTGGATCCTACAGCCAAGCTCCAAGTCAATATAGCCAACAGAGC
AGCAGCTACGGGCAGCAGAGTTCATTCCGACAGGACCACCCCAGTAGCATGGGTGTTT
 
ATGGGCAGGAGTCTGGAGGATTTTCCGGACCAGGAGAGAACCGGAGCATGAGTGGCC
CTGATAACCGGGGCAGGGGAAGAGGGGGATTTGATCGTGGAGGCATGAGCAGAGGTG
GGCGGGGAGGAGGACGCGGTGGAATGGGCAGCGCTGGAGAGCGAGGTGGCTTCAAT
AAGCCTGGTGGACCCATGGATGAAGGACCAGATCTTGATCTAGGCCCACCTGTAGATC
CAGATGAAGACTCTGACAACAGTGCAATTTATGTACAAGGATTAAATGACAGTGTGACTC
TAGATGATCTGGCAGACTTCTTTAAGCAGTGTGGGGTTGTTAAGATGAACAAGAGAACT
GGGCAACCCATGATCCACATCTACCTGGACAAGGAAACAGGAAAGCCCAAAGGCGATG
CCACAGTGTCCTATGAAGACCCACCTACTGCCAAGGCTGCCGTGGAATGGTTTGATGG
GAAAGATTTTCAAGGGAGCAAACTTAAAGTCTCCCTTGCTCGGAAGAAGCCTCCAATGA
ACAGTATGCGGGGTGGTCTGCCACCCCGTGAGGGCAGAGGCATGCCACCACCACTCC
GTGGAGGTCCAGGAGGCCCAGGAGGTCCTGGGGGACCCATGGGTCGCATGGGAGGC
CGTGGAGGAGATAGAGGAGGCTTCCCTCCAAGAGGACCCCGGGGTTCCCGAGGGAAC
CCCTCTGGAGGAGGAAACGTCCAGCACCGAGCTGGAGACTGGCAGTGTCCCAATCCG
GGTTGTGGAAACCAGAACTTCGCCTGGAGAACAGAGTGCAACCAGTGTAAGGCCCCAA
AGCCTGAAGGCTTCCTCCCGCCACCCTTTCCGCCCCCGGGTGGTGATCGTGGCAGAG
GTGGCCCTGGTGGCATGCGGGGAGGAAGAGGTGGCCTCATGGATCGTGGTGGTCCCG
GTGGAATGTTCAGAGGTGGCCGTGGTGGAGACAGAGGTGGCTTCCGTGGTGGCCGGG
GCATGGACCGAGGTGGCTTTGGTGGAGGAAGACGAGGTGGCCCTGGGGGGCCCCCTG
GACCTTTGATGGAACAGGATTACAAGGATGACGACGATAAGGGTACCGAGCAGAAGCT
GATCTCAGAGGAGGACCTGGGCGCCCCCGGCTCCGCCGGCTCCGCCGCCGGCTCCG
GCGCTTCTAACTTTACTCAGTTCGTTCTCGTCGACAATGGCGGAACTGGCGACGTGACT
GTCGCCCCAAGCAACTTCGCTAACGGGATCGCTGAATGGATCAGCTCTAACTCGCGTTC
ACAGGCTTACAAAGTAACCTGTAGCGTTCGTCAGAGCTCTGCGCAGAATCGCAAATACA
CCATCAAAGTCGAGGTGCCTAAAGGCGCCTGGCGTTCGTACTTAAATATGGAACTAACC
ATTCCAATTTTCGCCACGAATTCCGACTGCGAGCTTATTGTTAAGGCAATGCAAGGTCTC
CTAAAAGATGGAAACCCGATTCCCTCAGCAATCGCAGCAAACTCCGGCATCTACTAA
(SEQ ID NO:147)
Protein:

MCRAISLRRLLLLLLQLSQLLAVTQGMLEMWHEGLEEASRLYFGERNVKGMFEVLEPLHAM
MERGPQTLKETSFNQAYGRDLMEAQEWCRKYMKSGNVKDLLQAWDLYYHVFRRISKTRT
GKLFWALVVVAGVLFCYGLLVTVALCVIWVRSMASTDYSTYSQAAAQQGYSAYTAQPTQG
YAQTTQAYGQQSYGTYGQPTDVSYTQAQTTATYGQTAYATSYGQPPTGYTTPTAPQAYSQ
PVQGYGTGAYDTTTATVTTTQASYAAQSAYGTQPAYPAYGQQPAATAPTRPQDGNKPTET
SQPQSSTGGYNQPSLGYGQSNYSYPQVPGSYPMQPVTAPPSYPPTSYSSTQPTSYDQSS
YSQQNTYGQPSSYGQQSSYGQQSSYGQQPPTSYPPQTGSYSQAPSQYSQQSSSYGQQS
SFRQDHPSSMGVYGQESGGFSGPGENRSMSGPDNRGRGRGGFDRGGMSRGGRGGGR

GGMGSAGERGGFNKPGGPMDEGPDLDLGPPVDPDEDSDNSAIYVQGLNDSVTLDDLADF
FKQCGVVKMNKRTGQPMIHIYLDKETGKPKGDATVSYEDPPTAKAAVEWFDGKDFQGSKL
KVSLARKKPPMNSMRGGLPPREGRGMPPPLRGGPGGPGGPMGRMGGRGGDRGGF
PPRGPRGSRGNPSGGGNVQHRAGDWQCPNPGCGNQNFAWRTECNQCKAPKPEGFLPP
PFPPPGGDRGRGGPGGMRGGRGGLMDRGGPGGMFRGGRGGDRGGFRGGRGMDRGG
FGGGRRGGPGGPPGPLMEQDYKDDDDKGTEQKLISEEDLGAPGSAGSAAGSGASNFTQF
VLVDNGGTGDVTVAPSNFANGIAEWISSNSRSQAYKVTCSVRQSSAQNRKYTIKVEVPKGA
WRSYLNMELTIPIFATNSDCELIVKAMQGLLKDGNPIPSAIAANSGIY (SEQ ID NO:148)

FRB-CD28-EWSR1-4xλ_{N22}
DNA: 
ATGTGCCGAGCCATCTCTCTTAGGCGCTTGCTGCTGCTGCTGCAGCTGTCACAACT
CCTAGCTGTCACTCAAGGGATGCTCGAGATGTGGCATGAAGGCCTGGAAGAGGCATCT
CGTTTGTACTTTGGGGAAAGGAACGTGAAAGGCATGTTTGAGGTGCTGGAGCCCTTGC
ATGCTATGATGGAACGGGGCCCCCAGACTCTGAAGGAAACATCCTTTAATCAGGCCTAT
GGTCGAGATTTAATGGAGGCCCAAGAGTGGTGCAGGAAGTACATGAAATCAGGGAATG
TCAAGGACCTCCTCCAAGCCTGGGACCTCTATTATCATGTGTTCCGACGAATCTCAAAG
ACTAGAACCGGTAAGCTTTTTGGGCACTGGTCGTGGTTGCTGGAGTCCTGTTTTGTTA
TGGCTTGCTAGTGACAGTGGCTCTTTGTGTTATCTGGGTAAGATCTATGGCGTCCACGG
ATTACAGTACCTATAGCCAAGCTGCAGCGCAGCAGGGCTACAGTGCTTACACCGCCCA
GCCCACTCAAGGATATGCACAGACCACCCAGGCATATGGGCAACAAAGCTATGGAACC
TATGGACAGCCCACTGATGTCAGCTATACCCAGGCTCAGACCACTGCAACCTATGGGCA
GACCGCCTATGCAACTTCTTATGGACAGCCTCCCACTGGTTATACTACTCCAACTGCCC
CCCAGGCATACAGCCAGCCTGTCCAGGGGTATGGCACTGGTGCTTATGATACCACCAC
TGCTACAGTCACCACCACCCAGGCCTCCTATGCAGCTCAGTCTGCATATGGCACTCAGC
CTGCTTATCCAGCCTATGGGCAGCAGCCAGCAGCCACTGCACCTACAAGACCGCAGGA
TGGAAACAAGCCCACTGAGACTAGTCAACCTCAATCTAGCACAGGGGGTTACAACCAGC
CCAGCCTAGGATATGGACAGAGTAACTACAGTTATCCCCAGGTACCTGGGAGCTACCC
CATGCAGCCAGTCACTGCACCTCCATCCTACCCTCCTACCAGCTATTCCTCTACACAGC
CGACTAGTTATGATCAGAGCAGTTACTCTCAGCAGAACACCTATGGCAACCGAGCAGC
TATGGACAGCAGAGTAGCTATGGTCAACAAAGCAGCTATGGGCAGCAGCCTCCCACTA
GTTACCCACCCCAAACTGGATCCTACAGCCAAGCTCCAAGTCAATATAGCCAACAGAGC
AGCAGCTACGGGCAGCAGAGTTCATTCCGACAGGACCACCCCAGTAGCATGGGTGTTT
ATGGGCAGGAGTCTGGAGGATTTTCCGGACCAGGAGAGAACCGGAGCATGAGTGGCC
CTGATAACCGGGGCAGGGGAAGAGGGGGATTTGATCGTGGAGGCATGAGCAGAGGTG
GGCGGGGAGGAGGACGCGGTGGAATGGGCAGCGCTGGAGAGCGAGGTGGCTTCAAT
 
AAGCCTGGTGGACCCATGGATGAAGGACCAGATCTTGATCTAGGCCCACCTGTAGATC
CAGATGAAGACTCTGACAACAGTGCAATTTATGTACAAGGATTAAATGACAGTGTGACTC
TAGATGATCTGGCAGACTTCTTTAAGCAGTGTGGGGTTGTTAAGATGAACAAGAGAACT
GGGCAACCCATGATCCACATCTACCTGGACAAGGAAACAGGAAAGCCCAAAGGCGATG
CCACAGTGTCCTATGAAGACCCACCTACTGCCAAGGCTGCCGTGGAATGGTTTGATGG
GAAAGATTTTCAAGGGAGCAAACTTAAAGTCTCCCTTGCTCGGAAGAAGCCTCCAATGA
ACAGTATGCGGGGTGGTCTGCCACCCCGTGAGGGCAGAGGCATGCCACCACCACTCC
GTGGAGGTCCAGGAGGCCCAGGAGGTCCTGGGGGACCCATGGGTCGCATGGGAGGC
CGTGGAGGAGATAGAGGAGGCTTCCCTCCAAGAGGACCCCGGGGTTCCCGAGGGAAC
CCCTCTGGAGGAGGAAACGTCCAGCACCGAGCTGGAGACTGGCAGTGTCCCAATCCG
GGTTGTGGAAACCAGAACTTCGCCTGGAGAACAGAGTGCAACCAGTGTAAGGCCCCAA
AGCCTGAAGGCTTCCTCCCGCCACCCTTTCCGCCCCCGGGTGGTGATCGTGGCAGAG
GTGGCCCTGGTGGCATGCGGGGAGGAAGAGGTGGCCTCATGGATCGTGGTGGTCCCG
GTGGAATGTTCAGAGGTGGCCGTGGTGGAGACAGAGGTGGCTTCCGTGGTGGCCGGG
GCATGGACCGAGGTGGCTTTGGTGGAGGAAGACGAGGTGGCCCTGGGGGGCCCCCTG
GACCTTTGATGGAACAGGATTACAAGGATGACGACGATAAGGGTACCGAGCAGAAGCT
GATCTCAGAGGAGGACCTGCTAGCCACCATGGACGCACAAACACGACGACGTGAGCGT
CGCGCTGAGAAACAAGCTCAATGGAAAGCTGCAAACCCACCGCTCGACGGAGCCGGA
GCTGGCGCTGGAGCTGGAGCCGGAGCTGGCGGTCTAGCCACCATGGACGCACAAACA
CGACGACGTGAGCGTCGCGCTGAGAAACAAGCTCAATGGAAAGCTGCAAACCCACCGC
TCGACGGAGCCGGAGCTGGCGCTGGAGCTGGAGCCGGAGCTGGCGGTCTAGCCACCA
TGGACGCACAAACACGACGACGTGAGCGTCGCGCTGAGAAACAAGCTCAATGGAAAGC
TGCAAACCCACCGCTCGACGGAGCCGGAGCTGGCGCTGGAGCTGGAGCCGGAGCTGG
CGGTCTAGCCACCATGGACGCACAAACACGACGACGTGAGCGTCGCGCTGAGAAACAA
GCTCAATGGAAAGCTGCAAACCCACCGCTCGAGTCTAGAGGGCCCGTTTAA (SEQ ID
NO:149)
Protein:

MCRAISLRRLLLLLLQLSQLLAVTQGMLEMWHEGLEEASRLYFGERNVKGMFEVLEPLHAM
MERGPQTLKETSFNQAYGRDLMEAQEWCRKYMKSGNVKDLLQAWDLYYHVFRRISKTRT
GKLFWALVVVAGVLFCYGLLVTVALCVIWVRSMASTDYSTYSQAAAQQGYSAYTAQPTQG
YAQTTQAYGQQSYGTYGQPTDVSYTQAQTTATYGQTAYATSYGQPPTGYTTPTAPQAYSQ
PVQGYGTGAYDTTTATVTTTQASYAAQSAYGTQPAYPAYGQQPAATAPTRPQDGNKPTET
SQPQSSTGGYNQPSLGYGQSNYSYPQVPGSYPMQPVTAPPSYPPTSYSSTQPTSYDQSS
YSQQNTYGQPSSYGQQSSYGQQSSYGQQPPTSYPPQTGSYSQAPSQYSQQSSSYGQQS
SFRQDHPSSMGVYGQESGGFSGPGENRSMSGPDNRGRGRGGFDRGGMSRGGRGGGR
GGMGSAGERGGFNKPGGPMDEGPDLDLGPPVDPDEDSDNSAIYVQGLNDSVTLDDLADF

FKQCGVVKMNKRTGQPMIHIYLDKETGKPKGDATVSYEDPPTAKAAVEWFDGKDFQGSKL
KVSLARKKPPMNSMRGGLPPREGRGMPPPLRGGPGGPGGPMGRMGGRGGDRGGF
PPRGPRGSRGNPSGGGNVQHRAGDWQCPNPGCGNQNFAWRTECNQCKAPKPEGFLPP
PFPPPGGDRGRGGPGGMRGGRGGLMDRGGPGGMFRGGRGGDRGGFRGGRGMDRGG
FGGGRRGGPGGPPGPLMEQDYKDDDDKGTEQKLISEEDLLATMDAQTRRRERRAEKQAQ
WKAANPPLDGAGAGAGAGAGAGGLATMDAQTRRRERRAEKQAQWKAANPPLDGAGAGA
GAGAGAGGLATMDAQTRRRERRAEKQAQWKAANPPLDGAGAGAGAGAGAGGLATMDAQ
TRRRERRAEKQAQWKAANPPLESRGPV (SEQ ID NO:150)

FUS-CD28-FUS-PyIRS^{AF}
DNA: 
ATGTGCCGAGCCATCTCTCTTAGGCGCTTGCTGCTGCTGCTGCAGCTGTCACAACT
CCTAGCTGTCACTCAAGGGATGCTCATGGCCTCAAACGATTATACCCAACAAGCAACCC
AAAGCTATGGGGCCTACCCCACCCAGCCCGGGCAGGGCTATTCCCAGCAGAGCAGTCA
GCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTC
AACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCA
CCTCGGGAAGTTACGGTAGCAGTTCTCAGAGCAGCAGCTATGGGCAGCCCCAGAGTGG
GAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTG
GTGGAGGTGGAGGTGGAGGTGGAGGTAACTATGGCCAAGATCAATCCTCCATGAGTAG
TGGTGGTGGCAGTGGTGGCGGTTATGGCAATCAAGACCAGAGTGGTGGAGGTGGCAG
CGGTGGCTATGGACAGCAGGACCGTGGAGGCCGCGGCAGGGGTGGCAGTGGTGGCG
GCGGCGGCGGCGGTGGTGGTTACAACCGCAGCAGTGGTGGCTATGAACCCAGAG
GTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAA
TTCAGACAACAACACCATCTTTGTGCAAGGCCTGGGTGAGAATGTTACAATTGAGTCTG
TGGCTGATTACTTCAAGCAGATTGGTATTATTAAGACAAACAAGAAAACGGACAGCCC
ATGATTAATTTGTACACAGACAGGGAAACTGGCAAGCTGAAGGGAGAGGCAACGGTCT
CTTTTGATGACCCACCTTCAGCTAAAGCAGCTATTGACTGGTTTGATGGTAAAGAATTCT
CCGGAAATCCTATCAAGGTCTCATTTGCTACTCGCCGGGCAGACTTTAATCGGGGTGGT
GGCAATGGTCGTGGAGGCCGAGGGCGAGGAGGACCCATGGGCCGTGGAGGCTATGG
AGGTGGTGGCAGTGGTGGTGGTGGCCGAGGAGGATTTCCCAGTGGAGGTGGTGGCGG
TGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGG
 
AGGGGGACCAGGTGGCTCTCACATGGGGGGTAACTACGGGGATGATCGTCGTGGTGG
CAGAGGAGGCGGCACCGGTAAGCTTTTTTGGGCACTGGTCGTGGTTGCTGGAGTCCTG
TTTTGTTATGGCTTGCTAGTGACAGTGGCTCTTTGTGTTATCTGGGTAAGATCTGGTATG
GCCTCAAACGATTATACCCAACAAGCAACCCAAAGCTATGGGCCTACCCCACCCAGC
CCGGGCAGGGCTATTCCCAGCAGAGCAGTCAGCCCTACGGACAGCAGAGTTACAGTG
GTTATAGCCAGTCCACGGACACTTCAGGATATGGCCAGAGCAGCTATTCTTCTTATGGC
CAGAGCCAGAACACAGGCTATGGAACTCAGTCAACTCCCCAGGGATATGGCTCGACTG
GCGGCTATGGCAGTAGCCAGAGCTCCCAATCGTCTTACGGGCAGCAGTCCTCCTACCC
TGGCTATGGCCAGCAGCCAGCTCCCAGCAGCACCTCGGGAAGTTACGGTAGCAGTTCT
CAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATG
GACAGCAGAACCAGTACAACAGCAGCAGTGGTGGTGGAGGTGGAGGTGGAGGTGGAG
GTAACTATGGCCAAGATCAATCCTCCATGAGTAGTGGTGGTGGCAGTGGTGGCGGTTAT
GGCAATCAAGACCAGAGTGGTGGAGGTGGCAGCGGTGGCTATGGACAGCAGGACCGT
GGAGGCCGCGGCAGGGGTGGCAGTGGTGGCGGCGGCGGCGGCGGTGGTGGTT
ACAACCGCAGCAGTGGTGGCTATGAACCCAGAGGTCGTGGAGGTGGCCGTGGAGGCA
GAGGTGGCATGGGCGGAAGTGACCGTGGTGGCTTCAATAAATTTGGTGGCCCTCGGGA
CCAAGGATCACGTCATGACTCCGAACAGGATAATTCAGACAACAACACCATCTTTGTGC
AAGGCCTGGGTGAGAATGTTACAATTGAGTCTGTGGCTGATTACTTCAAGCAGATTGGT
ATTATTAAGACAAACAAGAAAACGGGACAGCCCATGATTAATTTGTACACAGACAGGGA
AACTGGCAAGCTGAAGGGAGAGGCAACGGTCTCTTTTGATGACCCACCTTCAGCTAAAG
CAGCTATTGACTGGTTTGATGGTAAAGAATTCTCCGGAAATCCTATCAAGGTCTCATTTG
CTACTCGCCGGGCAGACTTTAATCGGGGTGGTGGCAATGGTCGTGGAGGCCGAGGGC
GAGGAGGACCCATGGGCCGTGGAGGCTATGGAGGTGGTGGCAGTGGTGGTGGTGGC
CGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAA
CCAGTGTAAGGCCCCTAAACCAGATGGCCCAGGAGGGGGACCAGGTGGCTCTCACAT
GGGGGGTAACTACGGGGATGATCGTCGTGGTGGCAGAGGAGGCGGCGCCCCGGCT
CCGCCGGCTCCGCCGCCGGCTCCGGCATGGCGTGCCCGGTGCCGCTGCAGCTGCCG
CCGCTGGAACGCCTGACCCTGGATGATAAAAAACCGCTGAATACCCTGATCTCTGCTAC
TGGTCTGTGGATGAGTCGTACCGGAACCATTCATAAAATCAAACACCACGAGGTTAGCC
GTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTG
CCGTGTGTCCGATGAGGATCTGAACAAATTCCTGACAAAAGCCAATGAGGACCAAACAA
GCGTGAAAGTGAAAGTCGTTAGCGCTCCTACCCGTACTAAAAAGCAATGCCGAAATCC
GTTGCTCGTGCCCCTAAACCACTGGAAAACACTGAAGCAGCACAGGCACAGCCGTCTG
 
GAAGCAAATTCTCTCCGGCCATTCCTGTTTCTACCCAGGAGTCCGTTTCTGTTCCAGCA
AGTGTGAGCACCAGCATTAGCAGTATTAGCACCGGTGCCACCGCTAGCGCCCTGGTTA
AAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAG
CCTGAATTCCGGCAAACCGTTTCGTGAACTGGAGAGCGAACTGCTGTCACGTCGTAAAA
AAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATT
CCTCTGGAGTATATCGAGCGTATGGGCATCGACAATGATACCGAACTGAGCAAACAAAT
TTTCCGTGTGGATAAAAACTTCTGTCTGCGCCCTATGCTAGCACCAAATCTGGCTAACTA
TCTGCGCAAACTGGACCGTGCCCTGCCTGATCCTATCAAAATCTTCGAGATCGGCCCGT
GTTATCGTAAAGAGTCCGACGGTAAAGAACATCTGGAGGAGTTTACCATGCTGAACTTT
TGCCAAATGGGTTCAGGTTGTACTCGTGAGAACCTGGAAAGCATCATCACCGATTTTCT
GAACCACCTGGGCATTGACTTCAAAATTGTGGGCGACAGCTGTATGGTGTTTGGCGACA
CCCTGGATGTCATGCACGGCGACCTGGAACTGTCTAGTGCCGTTGTGGGCCCAATCCC
GCTGGATCGTGAGTGGGGTATCGACAAACCTTGGATCGGTGCGGGTTTTGGTCTGGAG
CGTCTGCTGAAAGTAAAACACGACTTCAAGAACATCAAACGTGCTGCACGTTCCGAGTC
CTATTACAATGGTATTTCTACTAACCTGTAA (SEQ ID NO:151)
Protein:

MCRAISLRRLLLLLLQLSQLLAVTQGMLMASNDYTQQATQSYGAYPTQPGQGYSQQSSQP
YGQQSYSGYSQSTDTSGYGQSSYSSYGQSQNTGYGTQSTPQGYGSTGGYGSSQSSQSS
YGQQSSYPGYGQQPAPSSTSGSYGSSSQSSSYGQPQSGSYSQQPSYGGQQQSYGQQQ
SYNPPQGYGQQNQYNSSSGGGGGGGGGNYGQDQSSMSSGGGSGGGYGNQDQSGG
GGSGGYGQQDRGGRGRGGSGGGGGGGGGYNRSSGGYEPRGRGGGRGGRGGMGGS
DRGGFNKFGGPRDQGSRHDSEQDNSDNNTIFVQGLGENVTIESVADYFKQIGIIKTNKKTG
QPMINLYTDRETGKLKGEATVSFDDPPSAKAAIDWFDGKEFSGNPIKVSFATRRADFNRGG
GNGRGGRGRGGPMGRGGYGGGGSGGGGRGGFPSGGGGGGGQQRAGDWKCPNPTCE
NMNFSWRNECNQCKAPKPDGPGGGPGGSHMGGNYGDDRRGGRGGGTGKLFWALVVVA
GVLFCYGLLVTVALCVIWVRSGMASNDYTQQATQSYGAYPTQPGQGYSQQSSQPYGQQS
YSGYSQSTDTSGYGQSSYSSYGQSQNTGYGTQSTPQGYGSTGGYGSSQSSQSSYGQQS
SYPGYGQQPAPSSTSGSYGSSSQSSSYGQPQSGSYSQQPSYGGQQQSYGQQQSYNPP
QGYGQQNQYNSSSGGGGGGGGGNYGQDQSSMSSGGGSGGGYGNQDQSGGGGSGG
YGQQDRGGRGRGGSGGGGGGGGGYNRSSGGYEPRGRGGGRGGRGGMGGSDRGGF
NKFGGPRDQGSRHDSEQDNSDNNTIFVQGLGENVTIESVADYFKQIGIIKTNKKTGQPMINL
YTDRETGKLKGEATVSFDDPPSAKAAIDWFDGKEFSGNPIKVSFATRRADFNRGGGNGRG
GRGRGGPMGRGGYGGGGSGGGGRGGFPSGGGGGGGQQRAGDWKCPNPTCENMNFS
WRNECNQCKAPKPDGPGGGPGGSHMGGNYGDDRRGGRGGGAPGSAGSAAGSGMACP

VPLQLPPLERLTLDDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKS
VARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAE
ERENYLGKLEREITRFFVDRGFLEIKSPILIPLEYIERMGIDNDTELSKQIFRVDKNFCLRPMLA
PNLANYLRKLDRALPDPIKIFEIGPCYRKESDGKEHLEEFTMLNFCQMGSGCTRENLESIITD
FLNHLGIDFKIVGDSCMVFGDTLDVMHGDLELSSAVVGPIPLDREWGIDKPWIGAGFGLERL
LKVKHDFKNIKRAARSESYYNGISTNL (SEQ ID NO:152)

FUS-CD28-FUS-PyIRS^{AA}
DNA: 
ATGTGCCGAGCCATCTCTCTTAGGCGCTTGCTGCTGCTGCTGCAGCTGTCACAACT
CCTAGCTGTCACTCAAGGGATGCTCATGGCCTCAAACGATTATACCCAACAAGCAACCC
AAAGCTATGGGGCCTACCCCACCCAGCCCGGGCAGGGCTATTCCCAGCAGAGCAGTCA
GCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTC
AACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCA
CCTCGGGAAGTTACGGTAGCAGTTCTCAGAGCAGCAGCTATGGGCAGCCCCAGAGTGG
GAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTG
GTGGAGGTGGAGGTGGAGGTGGAGGTAACTATGGCCAAGATCAATCCTCCATGAGTAG
TGGTGGTGGCAGTGGTGGCGGTTATGGCAATCAAGACCAGAGTGGTGGAGGTGGCAG
CGGTGGCTATGGACAGCAGGACCGTGGAGGCCGCGGCAGGGGTGGCAGTGGTGGCG
GCGGCGGCGGCGGTGGTGGTTACAACCGCAGCAGTGGTGGCTATGAACCCAGAG
GTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAA
TTCAGACAACAACACCATCTTTGTGCAAGGCCTGGGTGAGAATGTTACAATTGAGTCTG
TGGCTGATTACTTCAAGCAGATTGGTATTATTAAGACAAACAAGAAAACGGACAGCCC
ATGATTAATTTGTACACAGACAGGGAAACTGGCAAGCTGAAGGGAGAGGCAACGGTCT
CTTTTGATGACCCACCTTCAGCTAAAGCAGCTATTGACTGGTTTGATGGTAAAGAATTCT
CCGGAAATCCTATCAAGGTCTCATTTGCTACTCGCCGGGCAGACTTTAATCGGGGTGGT
GGCAATGGTCGTGGAGGCCGAGGGCGAGGAGGACCCATGGGCCGTGGAGGCTATGG
AGGTGGTGGCAGTGGTGGTGGTGGCCGAGGAGGATTTCCCAGTGGAGGTGGTGGCGG
TGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGG
 
AGGGGGACCAGGTGGCTCTCACATGGGGGGTAACTACGGGGATGATCGTCGTGGTGG
CAGAGGAGGCGGCACCGGTAAGCTTTTTTGGGCACTGGTCGTGGTTGCTGGAGTCCTG
TTTTGTTATGGCTTGCTAGTGACAGTGGCTCTTTGTGTTATCTGGGTAAGATCTGGTATG
GCCTCAAACGATTATACCCAACAAGCAACCCAAAGCTATGGGCCTACCCCACCCAGC
CCGGGCAGGGCTATTCCCAGCAGAGCAGTCAGCCCTACGGACAGCAGAGTTACAGTG
GTTATAGCCAGTCCACGGACACTTCAGGATATGGCCAGAGCAGCTATTCTTCTTATGGC
CAGAGCCAGAACACAGGCTATGGAACTCAGTCAACTCCCCAGGGATATGGCTCGACTG
GCGGCTATGGCAGTAGCCAGAGCTCCCAATCGTCTTACGGGCAGCAGTCCTCCTACCC
TGGCTATGGCCAGCAGCCAGCTCCCAGCAGCACCTCGGGAAGTTACGGTAGCAGTTCT
CAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATG
GACAGCAGAACCAGTACAACAGCAGCAGTGGTGGTGGAGGTGGAGGTGGAGGTGGAG
GTAACTATGGCCAAGATCAATCCTCCATGAGTAGTGGTGGTGGCAGTGGTGGCGGTTAT
GGCAATCAAGACCAGAGTGGTGGAGGTGGCAGCGGTGGCTATGGACAGCAGGACCGT
GGAGGCCGCGGCAGGGGTGGCAGTGGTGGCGGCGGCGGCGGCGGTGGTGGTT
ACAACCGCAGCAGTGGTGGCTATGAACCCAGAGGTCGTGGAGGTGGCCGTGGAGGCA
GAGGTGGCATGGGCGGAAGTGACCGTGGTGGCTTCAATAAATTTGGTGGCCCTCGGGA
CCAAGGATCACGTCATGACTCCGAACAGGATAATTCAGACAACAACACCATCTTTGTGC
AAGGCCTGGGTGAGAATGTTACAATTGAGTCTGTGGCTGATTACTTCAAGCAGATTGGT
ATTATTAAGACAAACAAGAAAACGGGACAGCCCATGATTAATTTGTACACAGACAGGGA
AACTGGCAAGCTGAAGGGAGAGGCAACGGTCTCTTTTGATGACCCACCTTCAGCTAAAG
CAGCTATTGACTGGTTTGATGGTAAAGAATTCTCCGGAAATCCTATCAAGGTCTCATTTG
CTACTCGCCGGGCAGACTTTAATCGGGGTGGTGGCAATGGTCGTGGAGGCCGAGGGC
GAGGAGGACCCATGGGCCGTGGAGGCTATGGAGGTGGTGGCAGTGGTGGTGGTGGC
CGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAA
CCAGTGTAAGGCCCCTAAACCAGATGGCCCAGGAGGGGGACCAGGTGGCTCTCACAT
GGGGGGTAACTACGGGGATGATCGTCGTGGTGGCAGAGGAGGCGGCGCCCCGGCT
CCGCCGGCTCCGCCGCCGGCTCCGGCATGGCGTGCCCGGTGCCGCTGCAGCTGCCG
CCGCTGGAACGCCTGACCCTGGATGACAAAAACCGCTGAATACCCTGATCTCTGCTAC
TGGTCTGTGGATGAGTCGTACCGGAACCATTCATAAAATCAAACACCACGAGGTTAGCC
GTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTG
CCGTGTGTCCGATGAGGATCTGAACAAATTCCTGACAAAAGCCAATGAGGACCAAACAA
GCGTGAAAGTGAAAGTCGTTAGCGCTCCTACCCGTACTAAAAAAGCAATGCCGAAATCC
GTTGCTCGTGCCCCTAAACCACTGGAAAACACTGAAGCAGCACAGGCACAGCCGTCTG
 
GAAGCAAATTCTCTCCGGCCATTCCTGTTTCTACCCAGGAGTCCGTTTCTGTTCCAGCA
AGTGTGAGCACCAGCATTAGCAGTATTAGCACCGGTGCCACCGCTAGCGCCCTGGTTA
AAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAG
CCTGAATTCCGGCAAACCGTTTCGTGAACTGGAGAGCGAACTGCTGTCACGTCGTAAAA
AAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATT
CCTCTGGAGTATATCGAGCGTATGGGCATCGACAATGATACCGAACTGAGCAAACAAAT
TTTCCGTGTGGATAAAAACTTCTGTCTGCGCCCTATGCTGGCACCAAATCTGTATAACTA
TCTGCGCAAACTGGACCGTGCCCTGCCTGATCCTATCAAAATCTTCGAGATCGGCCCGT
GTTATCGTAAAGAGTCCGACGGTAAAGAACATCTGGAGGAGTTTACCATGCTGGCCTTT
GCCCAAATGGGTTCAGGTTGTACTCGTGAGAACCTGGAAAGCATCATCACCGATTTTCT
GAACCACCTGGGCATTGACTTCAAAATTGTGGGCGACAGCTGTATGGTGTATGGCGACA
CCCTGGATGTCATGCACGGCGACCTGGAACTGTCTAGTGCCGTTGTTGGACCAATTCC
GCTGGACCGTGAGTGGGGTATCGACAAACCGTGGATCGGAGCAGGATTCGGTCTGGAA
CGCCTGCTGAAAGTGAAACACGACTTCAAAAACATCAAACGTGCCGCCCGTTCTGAATC
GTATTATAACGGGATCTCTACGAACCTGTAA (SEQ ID NO:153)
Protein:

MCRAISLRRLLLLLLQLSQLLAVTQGMLMASNDYTQQATQSYGAYPTQPGQGYSQQSSQP
YGQQSYSGYSQSTDTSGYGQSSYSSYGQSQNTGYGTQSTPQGYGSTGGYGSSQSSQSS
YGQQSSYPGYGQQPAPSSTSGSYGSSSQSSSYGQPQSGSYSQQPSYGGQQQSYGQQQ
SYNPPQGYGQQNQYNSSSGGGGGGGGGNYGQDQSSMSSGGGSGGGYGNQDQSGG
GGSGGYGQQDRGGRGRGGSGGGGGGGGGYNRSSGGYEPRGRGGGRGGRGGMGGS
DRGGFNKFGGPRDQGSRHDSEQDNSDNNTIFVQGLGENVTIESVADYFKQIGIIKTNKKTG
QPMINLYTDRETGKLKGEATVSFDDPPSAKAAIDWFDGKEFSGNPIKVSFATRRADFNRGG
GNGRGGRGRGGPMGRGGYGGGGSGGGGRGGFPSGGGGGGGQQRAGDWKCPNPTCE
NMNFSWRNECNQCKAPKPDGPGGGPGGSHMGGNYGDDRRGGRGGGTGKLFWALVVVA
GVLFCYGLLVTVALCVIWVRSGMASNDYTQQATQSYGAYPTQPGQGYSQQSSQPYGQQS
YSGYSQSTDTSGYGQSSYSSYGQSQNTGYGTQSTPQGYGSTGGYGSSQSSQSSYGQQS
SYPGYGQQPAPSSTSGSYGSSSQSSSYGQPQSGSYSQQPSYGGQQQSYGQQQSYNPP
QGYGQQNQYNSSSGGGGGGGGGNYGQDQSSMSSGGGSGGGYGNQDQSGGGGSGG
YGQQDRGGRGRGGSGGGGGGGGGYNRSSGGYEPRGRGGGRGGRGGMGGSDRGGF
NKFGGPRDQGSRHDSEQDNSDNNTIFVQGLGENVTIESVADYFKQIGIIKTNKKTGQPMINL
YTDRETGKLKGEATVSFDDPPSAKAAIDWFDGKEFSGNPIKVSFATRRADFNRGGGNGRG
GRGRGGPMGRGGYGGGGSGGGGRGGFPSGGGGGGGQQRAGDWKCPNPTCENMNFS
WRNECNQCKAPKPDGPGGGPGGSHMGGNYGDDRRGGRGGGAPGSAGSAAGSGMACP

VPLQLPPLERLTLDDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKS
VARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAE
ERENYLGKLEREITRFFVDRGFLEIKSPILIPLEYIERMGIDNDTELSKQIFRVDKNFCLRPMLA
PNLYNYLRKLDRALPDPIKIFEIGPCYRKESDGKEHLEEFTMLAFAQMGSGCTRENLESIITDF
LNHLGIDFKIVGDSCMVYGDTLDVMHGDLELSSAVVGPIPLDREWGIDKPWIGAGFGLERLL
KVKHDFKNIKRAARSESYYNGISTNL (SEQ ID NO:154)

FUS-CD28-FUS-MCP-PyIRS^{AF}
DNA: 
ATGTGCCGAGCCATCTCTCTTAGGCGCTTGCTGCTGCTGCTGCAGCTGTCACAACT
CCTAGCTGTCACTCAAGGGATGCTCATGGCCTCAAACGATTATACCCAACAAGCAACCC
AAAGCTATGGGGCCTACCCCACCCAGCCCGGGCAGGGCTATTCCCAGCAGAGCAGTCA
GCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTC
AACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCA
CCTCGGGAAGTTACGGTAGCAGTTCTCAGAGCAGCAGCTATGGGCAGCCCCAGAGTGG
GAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTG
GTGGAGGTGGAGGTGGAGGTGGAGGTAACTATGGCCAAGATCAATCCTCCATGAGTAG
TGGTGGTGGCAGTGGTGGCGGTTATGGCAATCAAGACCAGAGTGGTGGAGGTGGCAG
CGGTGGCTATGGACAGCAGGACCGTGGAGGCCGCGGCAGGGGTGGCAGTGGTGGCG
GCGGCGGCGGCGGTGGTGGTTACAACCGCAGCAGTGGTGGCTATGAACCCAGAG
GTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAA
TTCAGACAACAACACCATCTTTGTGCAAGGCCTGGGTGAGAATGTTACAATTGAGTCTG
TGGCTGATTACTTCAAGCAGATTGGTATTATTAAGACAAACAAGAAAACGGGACAGCCC
ATGATTAATTTGTACACAGACAGGGAAACTGGCAAGCTGAAGGGAGAGGCAACGGTCT
CTTTTGATGACCCACCTTCAGCTAAAGCAGCTATTGACTGGTTTGATGGTAAAGAATTCT
CCGGAAATCCTATCAAGGTCTCATTTGCTACTCGCCGGGCAGACTTTAATCGGGGTGGT
GGCAATGGTCGTGGAGGCCGAGGGCGAGGAGGACCCATGGGCCGTGGAGGCTATGG
AGGTGGTGGCAGTGGTGGTGGTGGCCGAGGAGGATTTCCCAGTGGAGGTGGTGGCGG
TGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGG
 
AGGGGGACCAGGTGGCTCTCACATGGGGGGTAACTACGGGGATGATCGTCGTGGTGG
CAGAGGAGGCGGCACCGGTAAGCTTTTTTGGGCACTGGTCGTGGTTGCTGGAGTCCTG
TTTTGTTATGGCTTGCTAGTGACAGTGGCTCTTTGTGTTATCTGGGTAAGATCTGGTATG
GCCTCAAACGATTATACCCAACAAGCAACCCAAAGCTATGGGCCTACCCCACCCAGC
CCGGGCAGGGCTATTCCCAGCAGAGCAGTCAGCCCTACGGACAGCAGAGTTACAGTG
GTTATAGCCAGTCCACGGACACTTCAGGATATGGCCAGAGCAGCTATTCTTCTTATGGC
CAGAGCCAGAACACAGGCTATGGAACTCAGTCAACTCCCCAGGGATATGGCTCGACTG
GCGGCTATGGCAGTAGCCAGAGCTCCCAATCGTCTTACGGGCAGCAGTCCTCCTACCC
TGGCTATGGCCAGCAGCCAGCTCCCAGCAGCACCTCGGGAAGTTACGGTAGCAGTTCT
CAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATG
GACAGCAGAACCAGTACAACAGCAGCAGTGGTGGTGGAGGTGGAGGTGGAGGTGGAG
GTAACTATGGCCAAGATCAATCCTCCATGAGTAGTGGTGGTGGCAGTGGTGGCGGTTAT
GGCAATCAAGACCAGAGTGGTGGAGGTGGCAGCGGTGGCTATGGACAGCAGGACCGT
GGAGGCCGCGGCAGGGGTGGCAGTGGTGGCGGCGGCGGCGGCGGTGGTGGTT
ACAACCGCAGCAGTGGTGGCTATGAACCCAGAGGTCGTGGAGGTGGCCGTGGAGGCA
GAGGTGGCATGGGCGGAAGTGACCGTGGTGGCTTCAATAAATTTGGTGGCCCTCGGGA
CCAAGGATCACGTCATGACTCCGAACAGGATAATTCAGACAACAACACCATCTTTGTGC
AAGGCCTGGGTGAGAATGTTACAATTGAGTCTGTGGCTGATTACTTCAAGCAGATTGGT
ATTATTAAGACAAACAAGAAAACGGGACAGCCCATGATTAATTTGTACACAGACAGGGA
AACTGGCAAGCTGAAGGGAGAGGCAACGGTCTCTTTTGATGACCCACCTTCAGCTAAAG
CAGCTATTGACTGGTTTGATGGTAAAGAATTCTCCGGAAATCCTATCAAGGTCTCATTTG
CTACTCGCCGGGCAGACTTTAATCGGGGTGGTGGCAATGGTCGTGGAGGCCGAGGGC
GAGGAGGACCCATGGGCCGTGGAGGCTATGGAGGTGGTGGCAGTGGTGGTGGTGGC
CGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAA
CCAGTGTAAGGCCCCTAAACCAGATGGCCCAGGAGGGGGACCAGGTGGCTCTCACAT
GGGGGGTAACTACGGGGATGATCGTCGTGGTGGCAGAGGAGGCGATTACAAGGATGA
CGACGATAAGGGTACCGGCGCCCCCGGCTCCGCCGGCTCCGCCGCCGGCTCCGGCG
CTTCTAACTTTACTCAGTTCGTTCTCGTCGACAATGGCGGAACTGGCGACGTGACTGTC
GCCCCAAGCAACTTCGCTAACGGGATCGCTGAATGGATCAGCTCTAACTCGCGTTCACA
GGCTTACAAAGTAACCTGTAGCGTTCGTCAGAGCTCTGCGCAGAATCGCAAATACACCA
TCAAAGTCGAGGTGCCTAAAGGCGCCTGGCGTTCGTACTTAAATATGGAACTAACCATT
CCAATTTTCGCCACGAATTCCGACTGCGAGCTTATTGTTAAGGCAATGCAAGGTCTCCT
AAAAGATGGAAACCCGATTCCCTCAGCAATCGCAGCAAACTCCGGCATCTACGGTACCG
GCGCCCCCGGCTCCGCCGGCTCCGCCGCCGGCTCCGGCGCGTGCCCGGTGCCGCTG
 
CAGCTGCCGCCGCTGGAACGCCTGACCCTGGATGATAAAAAACCGCTGAATACCCTGA
TCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAA
CAATAGCCGCTCTTCTCGTACAGCACGTGCACTGCGTCACCACAAATATCGTAAAACCT
GTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAAT
GCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTC
TGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATC
AGCTCCAGCACTGACAAAATCCCAAACCGATCGTCTGGAGGTTCTGCTGAATCCGAAAG
ACGAAATCAGCCTGAATTCCGGCAAACCGTTTCGTGAACTGGAGAGCGAACTGCTGTCA
CGTCGTAAAAAAGACCTGCAACAAATCTATGCCGAAGAACGTGAGAACTATCTGGGGAA
ACTGGAACGTGAAATCACCCGCTTTTTCGTGGATCGTGGCTTTCTGGAGATCAAATCCC
CGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGA
GATCGGCCCGTGTTATCGTAAAGAGTCCGACGGTAAAGAACATCTGGAGGAGTTTACCA
TGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGT
GTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTT
TTGGTCTGGAGCGTCTGCTGAAAGTAAAACACGACTTCAAGAACATCAAACGTGCTGCA
CGTTCCGAGTCCTATTACAATGGTATTTCTACTAACCTGTAA (SEQ ID NO:155)
Protein:

MCRAISLRRLLLLLLQLSQLLAVTQGMLMASNDYTQQATQSYGAYPTQPGQGYSQQSSQP
YGQQSYSGYSQSTDTSGYGQSSYSSYGQSQNTGYGTQSTPQGYGSTGGYGSSQSSQSS
YGQQSSYPGYGQQPAPSSTSGSYGSSSQSSSYGQPQSGSYSQQPSYGGQQQSYGQQQ
SYNPPQGYGQQNQYNSSSGGGGGGGGGGNYGQDQSSMSSGGGSGGGYGNQDQSGG
GGSGGYGQQDRGGRGRGGSGGGGGGGGGYNRSSGGYEPRGRGGGRGGRGGMGGS
DRGGFNKFGGPRDQGSRHDSEQDNSDNNTIFVQGLGENVTIESVADYFKQIGIIKTNKKTG
QPMINLYTDRETGKLKGEATVSFDDPPSAKAAIDWFDGKEFSGNPIKVSFATRRADFNRGG
GNGRGGRGRGGPMGRGGYGGGGSGGGGRGGFPSGGGGGGGQQRAGDWKCPNPTCE
NMNFSWRNECNQCKAPKPDGPGGGPGGSHMGGNYGDDRRGGRGGGTGKLFWALVVVA
GVLFCYGLLVTVALCVIWVRSGMASNDYTQQATQSYGAYPTQPGQGYSQQSSQPYGQQS
YSGYSQSTDTSGYGQSSYSSYGQSQNTGYGTQSTPQGYGSTGGYGSSQSSQSSYGQQS

SYPGYGQQPAPSSTSGSYGSSSQSSSYGQPQSGSYSQQPSYGGQQQSYGQQQSYNPP
QGYGQQNQYNSSSGGGGGGGGGNYGQDQSSMSSGGGSGGGYGNQDQSGGGGSGG
YGQQDRGGRGRGGSGGGGGGGGGYNRSSGGYEPRGRGGGRGGRGGMGGSDRGGF
NKFGGPRDQGSRHDSEQDNSDNNTIFVQGLGENVTIESVADYFKQIGIIKTNKKTGQPMINL
YTDRETGKLKGEATVSFDDPPSAKAAIDWFDGKEFSGNPIKVSFATRRADFNRGGGNGRG
GRGRGGPMGRGGYGGGGSGGGGRGGFPSGGGGGGGQQRAGDWKCPNPTCENMNFS
WRNECNQCKAPKPDGPGGGPGGSHMGGNYGDDRRGGRGGDYKDDDDKGTGAPGSAG
SAAGSGASNFTQFVLVDNGGTGDVTVAPSNFANGIAEWISSNSRSQAYKVTCSVRQSSAQ
NRKYTIKVEVPKGAWRSYLNMELTIPIFATNSDCELIVKAMQGLLKDGNPIPSAIAANSGIYGT
GAPGSAGSAAGSGACPVPLQLPPLERLTLDDKKPLNTLISATGLWMSRTGTIHKIKHHEVSR
SKIYIEMACGDHLVVNNSRSSRTARALRHHKYRKTCKRCRVSDEDLNKFLTKANEDQTSVK
VKVVSAPTRTKKAMPKSVARAPKPLENTEAAQAQPSGSKFSPAIPVSTQESVSVPASVSTSI
SSISTGATASALVKGNTNPITSMSAPVQASAPALTKSQTDRLEVLLNPKDEISLNSGKPFREL
ESELLSRRKKDLQQIYAEERENYLGKLEREITRFFVDRGFLEIKSPILIPLEYIERMGIDNDTEL
SKQIFRVDKNFCLRPMLAPNLANYLRKLDRALPDPIKIFEIGPCYRKESDGKEHLEEFTMLNF
CQMGSGCTRENLESIITDFLNHLGIDFKIVGDSCMVFGDTLDVMHGDLELSSAVVGPIPLDRE
WGIDKPWIGAGFGLERLLKVKHDFKNIKRAARSESYYNGISTNL (SEQ ID NO:156)

FUS-CD28-FUS-MCP-PyIRS^{AA}
DNA: 
ATGTGCCGAGCCATCTCTCTTAGGCGCTTGCTGCTGCTGCTGCAGCTGTCACAACT
CCTAGCTGTCACTCAAGGGATGCTCATGGCCTCAAACGATTATACCCAACAAGCAACCC
AAAGCTATGGGGCCTACCCCACCCAGCCCGGGCAGGGCTATTCCCAGCAGAGCAGTCA
GCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTC
AACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCA
CCTCGGGAAGTTACGGTAGCAGTTCTCAGAGCAGCAGCTATGGGCAGCCCCAGAGTGG
GAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTG
GTGGAGGTGGAGGTGGAGGTGGAGGTAACTATGGCCAAGATCAATCCTCCATGAGTAG
TGGTGGTGGCAGTGGTGGCGGTTATGGCAATCAAGACCAGAGTGGTGGAGGTGGCAG
CGGTGGCTATGGACAGCAGGACCGTGGAGGCCGCGGCAGGGGTGGCAGTGGTGGCG
GCGGCGGCGGCGGCGGTGGTGGTTACAACCGCAGCAGTGGTGGCTATGAACCCAGAG
GTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAA
 
TTCAGACAACAACACCATCTTTGTGCAAGGCCTGGGTGAGAATGTTACAATTGAGTCTG
TGGCTGATTACTTCAAGCAGATTGGTATTATTAAGACAAACAAGAAAACGGACAGCCC
ATGATTAATTTGTACACAGACAGGGAAACTGGCAAGCTGAAGGGAGAGGCAACGGTCT
CTTTTGATGACCCACCTTCAGCTAAAGCAGCTATTGACTGGTTTGATGGTAAAGAATTCT
CCGGAAATCCTATCAAGGTCTCATTTGCTACTCGCCGGGCAGACTTTAATCGGGGTGGT
GGCAATGGTCGTGGAGGCCGAGGGCGAGGAGGACCCATGGGCCGTGGAGGCTATGG
AGGTGGTGGCAGTGGTGGTGGTGGCCGAGGAGGATTTCCCAGTGGAGGTGGTGGCGG
TGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGG
AGGGGGACCAGGTGGCTCTCACATGGGGGGTAACTACGGGGATGATCGTCGTGGTGG
CAGAGGAGGCGGCACCGGTAAGCTTTTTGGGCACTGGTCGTGGTTGCTGGAGTCCTG
TTTTGTTATGGCTTGCTAGTGACAGTGGCTCTTTGTGTTATCTGGGTAAGATCTGGTATG
GCCTCAAACGATTATACCCAACAAGCAACCCAAAGCTATGGGGCCTACCCCACCCAGC
CCGGGCAGGGCTATTCCCAGCAGAGCAGTCAGCCCTACGGACAGCAGAGTTACAGTG
GTTATAGCCAGTCCACGGACACTTCAGGATATGGCCAGAGCAGCTATTCTTCTTATGGC
CAGAGCCAGAACACAGGCTATGGAACTCAGTCAACTCCCCAGGGATATGGCTCGACTG
GCGGCTATGGCAGTAGCCAGAGCTCCCAATCGTCTTACGGGCAGCAGTCCTCCTACCC
TGGCTATGGCCAGCAGCCAGCTCCCAGCAGCACCTCGGGAAGTTACGGTAGCAGTTCT
CAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATG
GACAGCAGAACCAGTACAACAGCAGCAGTGGTGGTGGAGGTGGAGGTGGAGGTGGAG
GTAACTATGGCCAAGATCAATCCTCCATGAGTAGTGGTGGTGGCAGTGGTGGCGGTTAT
GGCAATCAAGACCAGAGTGGTGGAGGTGGCAGCGGTGGCTATGGACAGCAGGACCGT
GGAGGCCGCGGCAGGGGTGGCAGTGGTGGCGGCGGCGGCGGCGGTGGTGGTT
ACAACCGCAGCAGTGGTGGCTATGAACCCAGAGGTCGTGGAGGTGGCCGTGGAGGCA
GAGGTGGCATGGGCGGAAGTGACCGTGGTGGCTTCAATAAATTTGGTGGCCCTCGGGA
CCAAGGATCACGTCATGACTCCGAACAGGATAATTCAGACAACAACACCATCTTTGTGC
AAGGCCTGGGTGAGAATGTTACAATTGAGTCTGTGGCTGATTACTTCAAGCAGATTGGT
ATTATTAAGACAAACAAGAAAACGGACAGCCCATGATTAATTTGTACACAGACAGGGA
AACTGGCAAGCTGAAGGGAGAGGCAACGGTCTCTTTTGATGACCCACCTTCAGCTAAAG
CAGCTATTGACTGGTTTGATGGTAAAGAATTCTCCGGAAATCCTATCAAGGTCTCATTTG
CTACTCGCCGGGCAGACTTTAATCGGGGTGGTGGCAATGGTCGTGGAGGCCGAGGGC
GAGGAGGACCCATGGGCCGTGGAGGCTATGGAGGTGGTGGCAGTGGTGGTGGTGGC
CGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAA
CCAGTGTAAGGCCCCTAAACCAGATGGCCCAGGAGGGGGACCAGGTGGCTCTCACAT
 
GGGGGGTAACTACGGGGATGATCGTCGTGGTGGCAGAGGAGGCGGCGCCCCCGGCT
CCGCCGGCTCCGCCGCCGGCTCCGGCATGGCGTGCCCGGTGCCGCTGCAGCTGCCG
CCGCTGGAACGCCTGACCCTGGATGACAAAAACCGCTGAATACCCTGATCTCTGCTAC
TGGTCTGTGGATGAGTCGTACCGGAACCATTCATAAAATCAAACACCACGAGGTTAGCC
GTTCGAAAATCTATATTGAGATGGCGTGTGGCGATCATCTGGTTGTGAACAATAGCCGC
TCTTCTCGTACAGCACGTGCACTGCGTCACCACAAATATCGTAAAACCTGTAAACGTTG
CCGTGTGTCCGATGAGGATCTGAACAAATTCCTGACAAAAGCCAATGAGGACCAAACAA
GCGTGAAAGTGAAAGTCGTTAGCGCTCCTACCCGTACTAAAAAGCAATGCCGAAATCC
GTTGCTCGTGCCCCTAAACCACTGGAAAACACTGAAGCAGCACAGGCACAGCCGTCTG
GAAGCAAATTCTCTCCGGCCATTCCTGTTTCTACCCAGGAGTCCGTTTCTGTTCCAGCA
AGTGTGAGCACCAGCATTAGCAGTATTAGCACCGGTGCCACCGCTAGCGCCCTGGTTA
AAGGCAATACCAATCCGATTACAAGCATGTCTGCCCCGGTTCAAGCATCAGCTCCAGCA
CTGACAAAATCCCAAACCGATCGTCTGGAGGTTCTGCTGAATCCGAAAGACGAAATCAG
CCTGAATTCCGGCAAACCGTTTCGTGAACTGGAGAGCGAACTGCTGTCACGTCGTAAAA
AAGACCTGCAACAAATCTATGCCGAAGAACGTGAGAACTATCTGGGGAAACTGGAACGT
GAAATCACCCGCTTTTTCGTGGATCGTGGCTTTCTGGAGATCAAATCCCCGATTCTGATT
CCTCTGGAGTATATCGAGCGTATGGGCATCGACAATGATACCGAACTGAGCAAACAAAT
TTTCCGTGTGGATAAAAACTTCTGTCTGCGCCCTATGCTGGCACCAAATCTGTATAACTA
TCTGCGCAAACTGGACCGTGCCCTGCCTGATCCTATCAAAATCTTCGAGATCGGCCCGT
GTTATCGTAAAGAGTCCGACGGTAAAGAACATCTGGAGGAGTTTACCATGCTGGCCTTT
GCCCAAATGGGTTCAGGTTGTACTCGTGAGAACCTGGAAAGCATCATCACCGATTTTCT
GAACCACCTGGGCATTGACTTCAAAATTGTGGGCGACAGCTGTATGGTGTATGGCGACA
CCCTGGATGTCATGCACGGCGACCTGGAACTGTCTAGTGCCGTTGTTGGACCAATTCC
GCTGGACCGTGAGTGGGGTATCGACAAACCGTGGATCGGAGCAGGATTCGGTCTGGAA
CGCCTGCTGAAAGTGAAACACGACTTCAAAAACATCAAACGTGCCGCCCGTTCTGAATC
GTATTATAACGGGATCTCTACGAACCTGTAA (SEQ ID NO:157)
Protein:

MCRAISLRRLLLLLLQLSQLLAVTQGMLMASNDYTQQATQSYGAYPTQPGQGYSQQSSQP
YGQQSYSGYSQSTDTSGYGQSSYSSYGQSQNTGYGTQSTPQGYGSTGGYGSSQSSQSS
YGQQSSYPGYGQQPAPSSTSGSYGSSSQSSSYGQPQSGSYSQQPSYGGQQQSYGQQQ
SYNPPQGYGQQNQYNSSSGGGGGGGGGNYGQDQSSMSSGGGSGGGYGNQDQSGG
GGSGGYGQQDRGGRGRGGSGGGGGGGGGYNRSSGGYEPRGRGGGRGGRGGMGGS
DRGGFNKFGGPRDQGSRHDSEQDNSDNNTIFVQGLGENVTIESVADYFKQIGIIKTNKKTG
QPMINLYTDRETGKLKGEATVSFDDPPSAKAAIDWFDGKEFSGNPIKVSFATRRADFNRGG
GNGRGGRGRGGPMGRGGYGGGGSGGGGRGGFPSGGGGGGGQQRAGDWKCPNPTCE
NMNFSWRNECNQCKAPKPDGPGGGPGGSHMGGNYGDDRRGGRGGGTGKLFWALVVVA

GVLFCYGLLVTVALCVIWVRSGMASNDYTQQATQSYGAYPTQPGQGYSQQSSQPYGQQS
YSGYSQSTDTSGYGQSSYSSYGQSQNTGYGTQSTPQGYGSTGGYGSSQSSQSSYGQQS
SYPGYGQQPAPSSTSGSYGSSSQSSSYGQPQSGSYSQQPSYGGQQQSYGQQQSYNPP
QGYGQQNQYNSSSGGGGGGGGGNYGQDQSSMSSGGGSGGGYGNQDQSGGGGSGG
YGQQDRGGRGRGGSGGGGGGGGGYNRSSGGYEPRGRGGGRGGRGGMGGSDRGGF
NKFGGPRDQGSRHDSEQDNSDNNTIFVQGLGENVTIESVADYFKQIGIIKTNKKTGQPMINL
YTDRETGKLKGEATVSFDDPPSAKAAIDWFDGKEFSGNPIKVSFATRRADFNRGGGNGRG
GRGRGGPMGRGGYGGGGSGGGGRGGFPSGGGGGGGQQRAGDWKCPNPTCENMNFS
WRNECNQCKAPKPDGPGGGPGGSHMGGNYGDDRRGGRGGGAPGSAGSAAGSGMACP
VPLQLPPLERLTLDDKKPLNTLISATGLWMSRTGTIHKIKHHEVSRSKIYIEMACGDHLVVNN
SRSSRTARALRHHKYRKTCKRCRVSDEDLNKFLTKANEDQTSVKVKVVSAPTRTKKAMPKS
VARAPKPLENTEAAQAQPSGSKFSPAIPVSTQESVSVPASVSTSISSISTGATASALVKGNTN
PITSMSAPVQASAPALTKSQTDRLEVLLNPKDEISLNSGKPFRELESELLSRRKKDLQQIYAE
ERENYLGKLEREITRFFVDRGFLEIKSPILIPLEYIERMGIDNDTELSKQIFRVDKNFCLRPMLA
PNLYNYLRKLDRALPDPIKIFEIGPCYRKESDGKEHLEEFTMLAFAQMGSGCTRENLESIITDF
LNHLGIDFKIVGDSCMVYGDTLDVMHGDLELSSAVVGPIPLDREWGIDKPWIGAGFGLERLL
KVKHDFKNIKRAARSESYYNGISTNL (SEQ ID NO:158)

FUS-CD28-EWSR1-MCP
DNA: 
ATGTGCCGAGCCATCTCTCTTAGGCGCTTGCTGCTGCTGCTGCAGCTGTCACAACT
CCTAGCTGTCACTCAAGGGATGCTCATGGCCTCAAACGATTATACCCAACAAGCAACCC
AAAGCTATGGGGCCTACCCCACCCAGCCCGGGCAGGGCTATTCCCAGCAGAGCAGTCA
GCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTC
AACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCA
CCTCGGGAAGTTACGGTAGCAGTTCTCAGAGCAGCAGCTATGGGCAGCCCCAGAGTGG
GAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTG
GTGGAGGTGGAGGTGGAGGTGGAGGTAACTATGGCCAAGATCAATCCTCCATGAGTAG
TGGTGGTGGCAGTGGTGGCGGTTATGGCAATCAAGACCAGAGTGGTGGAGGTGGCAG
CGGTGGCTATGGACAGCAGGACCGTGGAGGCCGCGGCAGGGGTGGCAGTGGTGGCG
GCGGCGGCGGCGGTGGTGGTTACAACCGCAGCAGTGGTGGCTATGAACCCAGAG
GTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAA
 
TTCAGACAACAACACCATCTTTGTGCAAGGCCTGGGTGAGAATGTTACAATTGAGTCTG
TGGCTGATTACTTCAAGCAGATTGGTATTATTAAGACAAACAAGAAAACGGACAGCCC
ATGATTAATTTGTACACAGACAGGGAAACTGGCAAGCTGAAGGGAGAGGCAACGGTCT
CTTTTGATGACCCACCTTCAGCTAAAGCAGCTATTGACTGGTTTGATGGTAAAGAATTCT
CCGGAAATCCTATCAAGGTCTCATTTGCTACTCGCCGGGCAGACTTTAATCGGGGTGGT
GGCAATGGTCGTGGAGGCCGAGGGCGAGGAGGACCCATGGGCCGTGGAGGCTATGG
AGGTGGTGGCAGTGGTGGTGGTGGCCGAGGAGGATTTCCCAGTGGAGGTGGTGGCGG
TGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGG
AGGGGGACCAGGTGGCTCTCACATGGGGGGTAACTACGGGGATGATCGTCGTGGTGG
CAGAGGAGGCGGCACCGGTAAGCTTTTTGGGCACTGGTCGTGGTTGCTGGAGTCCTG
TTTTGTTATGGCTTGCTAGTGACAGTGGCTCTTTGTGTTATCTGGGTAAGATCTATGGCG
TCCACGGATTACAGTACCTATAGCCAAGCTGCAGCGCAGCAGGGCTACAGTGCTTACA
CCGCCCAGCCCACTCAAGGATATGCACAGACCACCCAGGCATATGGGCAACAAAGCTA
TGGAACCTATGGACAGCCCACTGATGTCAGCTATACCCAGGCTCAGACCACTGCAACCT
ATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATA
CCACCACTGCTACAGTCACCACCACCCAGGCCTCCTATGCAGCTCAGTCTGCATATGGC
ACTCAGCCTGCTTATCCAGCCTATGGGCAGCAGCCAGCAGCCACTGCACCTACAAGAC
CGCAGGATGGAAACAAGCCCACTGAGACTAGTCAACCTCAATCTAGCACAGGGGGTTA
CAACCAGCCCAGCCTAGGATATGGACAGAGTAACTACAGTTATCCCCAGGTACCTGGG
AGCTACCCCATGCAGCCAGTCACTGCACCTCCATCCTACCCTCCTACCAGCTATTCCTC
TACACAGCCGACTAGTTATGATCAGAGCAGTTACTCTCAGCAGAACACCTATGGGCAAC
CGAGCAGCTATGGACAGCAGAGTAGCTATGGTCAACAAAGCAGCTATGGGCAGCAGCC
TCCCACTAGTTACCCACCCCAAACTGGATCCTACAGCCAAGCTCCAAGTCAATATAGCC
AACAGAGCAGCAGCTACGGGCAGCAGAGTTCATTCCGACAGGACCACCCCAGTAGCAT
GGGTGTTTATGGGCAGGAGTCTGGAGGATTTTCCGGACCAGGAGAGAACCGGAGCATG
AGTGGCCCTGATAACCGGGGCAGGGGAAGAGGGGGATTTGATCGTGGAGGCATGAGC
AGAGGTGGGCGGGGAGGAGGACGCGGTGGAATGGGCAGCGCTGGAGAGCGAGGTGG
CTTCAATAAGCCTGGTGGACCCATGGATGAAGGACCAGATCTTGATCTAGGCCCACCTG
TAGATCCAGATGAAGACTCTGACAACAGTGCAATTTATGTACAAGGATTAAATGACAGTG
TGACTCTAGATGATCTGGCAGACTTCTTTAAGCAGTGTGGGGTTGTTAAGATGAACAAG
AGAACTGGGCAACCCATGATCCACATCTACCTGGACAAGGAAACAGGAAAGCCCAAAG
GCGATGCCACAGTGTCCTATGAAGACCCACCCACTGCCAAGGCTGCCGTGGAATGGTT
TGATGGGAAAGATTTTCAAGGGAGCAAACTTAAAGTCTCCCTTGCTCGGAAGAAGCCTC
CAATGAACAGTATGCGGGGTGGTCTGCCACCCCGTGAGGGCAGAGGCATGCCACCAC
 
CACTCCGTGGAGGTCCAGGAGGCCCAGGAGGTCCTGGGGGACCCATGGGTCGCATGG
GAGGCCGTGGAGGAGATAGAGGAGGCTTCCCTCCAAGAGGACCCCGGGGTTCCCGAG
GGAACCCCTCTGGAGGAGGAAACGTCCAGCACCGAGCTGGAGACTGGCAGTGTCCCA
ATCCGGGTTGTGGAAACCAGAACTTCGCCTGGAGAACAGAGTGCAACCAGTGTAAGGC
CCCAAAGCCTGAAGGCTTCCTCCCGCCACCCTTTCCGCCCCCGGGTGGTGATCGTGGC
AGAGGTGGCCCTGGTGGCATGCGGGGAGGAAGAGGTGGCCTCATGGATCGTGGTGGT
CCCGGTGGAATGTTCAGAGGTGGCCGTGGTGGAGACAGAGGTGGCTTCCGTGGTGGC
CGGGGCATGGACCGAGGTGGCTTTGGTGGAGGAAGACGAGGTGGCCCTGGGGGGCC
CCCTGGACCTTTGATGGAACAGGATTACAAGGATGACGACGATAAGGGTACCGAGCAG
AAGCTGATCTCAGAGGAGGACCTGGGCGCCCCCGGCTCCGCCGGCTCCGCCGCCGGC
TCCGGCGCTTCTAACTTTACTCAGTTCGTTCTCGTCGACAATGGCGGAACTGGCGACGT
GACTGTCGCCCCAAGCAACTTCGCTAACGGGATCGCTGAATGGATCAGCTCTAACTCG
CGTTCACAGGCTTACAAAGTAACCTGTAGCGTTCGTCAGAGCTCTGCGCAGAATCGCAA
ATACACCATCAAAGTCGAGGTGCCTAAAGGCGCCTGGCGTTCGTACTTAAATATGGAAC
TAACCATTCCAATTTTCGCCACGAATTCCGACTGCGAGCTTATTGTTAAGGCAATGCAAG
GTCTCCTAAAAGATGGAAACCCGATTCCCTCAGCAATCGCAGCAAACTCCGGCATCTAC
TAA (SEQ ID NO:159)
Protein:

MCRAISLRRLLLLLLQLSQLLAVTQGMLMASNDYTQQATQSYGAYPTQPGQGYSQQSSQP
YGQQSYSGYSQSTDTSGYGQSSYSSYGQSQNTGYGTQSTPQGYGSTGGYGSSQSSQSS
YGQQSSYPGYGQQPAPSSTSGSYGSSSQSSSYGQPQSGSYSQQPSYGGQQQSYGQQQ
SYNPPQGYGQQNQYNSSSGGGGGGGGGNYGQDQSSMSSGGGSGGGYGNQDQSGG
GGSGGYGQQDRGGRGRGGSGGGGGGGGGYNRSSGGYEPRGRGGGRGGRGGMGGS
DRGGFNKFGGPRDQGSRHDSEQDNSDNNTIFVQGLGENVTIESVADYFKQIGIIKTNKKTG
QPMINLYTDRETGKLKGEATVSFDDPPSAKAAIDWFDGKEFSGNPIKVSFATRRADFNRGG
GNGRGGRGRGGPMGRGGYGGGGSGGGGRGGFPSGGGGGGGQQRAGDWKCPNPTCE
NMNFSWRNECNQCKAPKPDGPGGGPGGSHMGGNYGDDRRGGRGGGTGKLFWALVVVA
GVLFCYGLLVTVALCVIWVRSMASTDYSTYSQAAAQQGYSAYTAQPTQGYAQTTQAYGQQ
SYGTYGQPTDVSYTQAQTTATYGQTAYATSYGQPPTGYTTPTAPQAYSQPVQGYGTGAYD
TTTATVTTTQASYAAQSAYGTQPAYPAYGQQPAATAPTRPQDGNKPTETSQPQSSTGGYN
QPSLGYGQSNYSYPQVPGSYPMQPVTAPPSYPPTSYSSTQPTSYDQSSYSQQNTYGQPS
SYGQQSSYGQQSSYGQQPPTSYPPQTGSYSQAPSQYSQQSSSYGQQSSFRQDHPSSMG
VYGQESGGFSGPGENRSMSGPDNRGRGRGGFDRGGMSRGGRGGGRGGMGSAGERGG
FNKPGGPMDEGPDLDLGPPVDPDEDSDNSAIYVQGLNDSVTLDDLADFFKQCGVVKMNKR
TGQPMIHIYLDKETGKPKGDATVSYEDPPTAKAAVEWFDGKDFQGSKLKVSLARKKPPMNS
MRGGLPPREGRGMPPPLRGGPGGPGGPGGPMGRMGGRGGDRGGFPPRGPRGSRGNP

SGGGNVQHRAGDWQCPNPGCGNQNFAWRTECNQCKAPKPEGFLPPPFPPPGGDRGRG
GPGGMRGGRGGLMDRGGPGGMFRGGRGGDRGGFRGGRGMDRGGFGGGRRGGPGGP
PGPLMEQDYKDDDDKGTEQKLISEEDLGAPGSAGSAAGSGASNFTQFVLVDNGGTGDVTV
APSNFANGIAEWISSNSRSQAYKVTCSVRQSSAQNRKYTIKVEVPKGAWRSYLNMELTIPIF
ATNSDCELIVKAMQGLLKDGNPIPSAIAANSGIY (SEQ ID NO:160)

FUS-CD28-EWSR1-4xλ_{N22}
DNA: 
ATGTGCCGAGCCATCTCTCTTAGGCGCTTGCTGCTGCTGCTGCAGCTGTCACAACT
CCTAGCTGTCACTCAAGGGATGCTCATGGCCTCAAACGATTATACCCAACAAGCAACCC
AAAGCTATGGGGCCTACCCCACCCAGCCCGGGCAGGGCTATTCCCAGCAGAGCAGTCA
GCCCTACGGACAGCAGAGTTACAGTGGTTATAGCCAGTCCACGGACACTTCAGGATAT
GGCCAGAGCAGCTATTCTTCTTATGGCCAGAGCCAGAACACAGGCTATGGAACTCAGTC
AACTCCCCAGGGATATGGCTCGACTGGCGGCTATGGCAGTAGCCAGAGCTCCCAATCG
TCTTACGGGCAGCAGTCCTCCTACCCTGGCTATGGCCAGCAGCCAGCTCCCAGCAGCA
CCTCGGGAAGTTACGGTAGCAGTTCTCAGAGCAGCAGCTATGGGCAGCCCCAGAGTGG
GAGCTACAGCCAGCAGCCTAGCTATGGTGGACAGCAGCAAAGCTATGGACAGCAGCAA
AGCTATAATCCCCCTCAGGGCTATGGACAGCAGAACCAGTACAACAGCAGCAGTGGTG
GTGGAGGTGGAGGTGGAGGTGGAGGTAACTATGGCCAAGATCAATCCTCCATGAGTAG
TGGTGGTGGCAGTGGTGGCGGTTATGGCAATCAAGACCAGAGTGGTGGAGGTGGCAG
CGGTGGCTATGGACAGCAGGACCGTGGAGGCCGCGGCAGGGGTGGCAGTGGTGGCG
GCGGCGGCGGCGGTGGTGGTTACAACCGCAGCAGTGGTGGCTATGAACCCAGAG
GTCGTGGAGGTGGCCGTGGAGGCAGAGGTGGCATGGGCGGAAGTGACCGTGGTGGC
TTCAATAAATTTGGTGGCCCTCGGGACCAAGGATCACGTCATGACTCCGAACAGGATAA
TTCAGACAACAACACCATCTTTGTGCAAGGCCTGGGTGAGAATGTTACAATTGAGTCTG
TGGCTGATTACTTCAAGCAGATTGGTATTATTAAGACAAACAAGAAAACGGACAGCCC
ATGATTAATTTGTACACAGACAGGGAAACTGGCAAGCTGAAGGAGAGGCAACGGTCT
CTTTTGATGACCCACCTTCAGCTAAAGCAGCTATTGACTGGTTTGATGGTAAAGAATTCT
CCGGAAATCCTATCAAGGTCTCATTTGCTACTCGCCGGGCAGACTTTAATCGGGGTGGT
GGCAATGGTCGTGGAGGCCGAGGGCGAGGAGGACCCATGGGCCGTGGAGGCTATGG
AGGTGGTGGCAGTGGTGGTGGTGGCCGAGGAGGATTTCCCAGTGGAGGTGGTGGCGG
TGGAGGACAGCAGCGAGCTGGTGACTGGAAGTGTCCTAATCCCACCTGTGAGAATATG
AACTTCTCTTGGAGGAATGAATGCAACCAGTGTAAGGCCCCTAAACCAGATGGCCCAGG
AGGGGGACCAGGTGGCTCTCACATGGGGGGTAACTACGGGGATGATCGTCGTGGTGG
CAGAGGAGGCGGCACCGGTAAGCTTTTTGGGCACTGGTCGTGGTTGCTGGAGTCCTG
TTTTGTTATGGCTTGCTAGTGACAGTGGCTCTTTGTGTTATCTGGGTAAGATCTATGGCG
 
TCCACGGATTACAGTACCTATAGCCAAGCTGCAGCGCAGCAGGGCTACAGTGCTTACA
CCGCCCAGCCCACTCAAGGATATGCACAGACCACCCAGGCATATGGGCAACAAAGCTA
TGGAACCTATGGACAGCCCACTGATGTCAGCTATACCCAGGCTCAGACCACTGCAACCT
ATGGGCAGACCGCCTATGCAACTTCTTATGGACAGCCTCCCACTGGTTATACTACTCCA
ACTGCCCCCCAGGCATACAGCCAGCCTGTCCAGGGGTATGGCACTGGTGCTTATGATA
CCACCACTGCTACAGTCACCACCACCCAGGCCTCCTATGCAGCTCAGTCTGCATATGGC
ACTCAGCCTGCTTATCCAGCCTATGGGCAGCAGCCAGCAGCCACTGCACCTACAAGAC
CGCAGGATGGAAACAAGCCCACTGAGACTAGTCAACCTCAATCTAGCACAGGGGGTTA
CAACCAGCCCAGCCTAGGATATGGACAGAGTAACTACAGTTATCCCCAGGTACCTGGG
AGCTACCCCATGCAGCCAGTCACTGCACCTCCATCCTACCCTCCTACCAGCTATTCCTC
TACACAGCCGACTAGTTATGATCAGAGCAGTTACTCTCAGCAGAACACCTATGGGCAAC
CGAGCAGCTATGGACAGCAGAGTAGCTATGGTCAACAAAGCAGCTATGGGCAGCAGCC
TCCCACTAGTTACCCACCCCAAACTGGATCCTACAGCCAAGCTCCAAGTCAATATAGCC
AACAGAGCAGCAGCTACGGGCAGCAGAGTTCATTCCGACAGGACCACCCCAGTAGCAT
GGGTGTTTATGGGCAGGAGTCTGGAGGATTTTCCGGACCAGGAGAGAACCGGAGCATG
AGTGGCCCTGATAACCGGGGCAGGGGAAGAGGGGGATTTGATCGTGGAGGCATGAGC
AGAGGTGGGCGGGGAGGAGGACGCGGTGGAATGGGCAGCGCTGGAGAGCGAGGTGG
CTTCAATAAGCCTGGTGGACCCATGGATGAAGGACCAGATCTTGATCTAGGCCCACCTG
TAGATCCAGATGAAGACTCTGACAACAGTGCAATTTATGTACAAGGATTAAATGACAGTG
TGACTCTAGATGATCTGGCAGACTTCTTTAAGCAGTGTGGGGTTGTTAAGATGAACAAG
AGAACTGGGCAACCCATGATCCACATCTACCTGGACAAGGAAACAGGAAAGCCCAAAG
GCGATGCCACAGTGTCCTATGAAGACCCACCTACTGCCAAGGCTGCCGTGGAATGGTT
TGATGGGAAAGATTTTCAAGGGAGCAAACTTAAAGTCTCCCTTGCTCGGAAGAAGCCTC
CAATGAACAGTATGCGGGGTGGTCTGCCACCCCGTGAGGGCAGAGGCATGCCACCAC
CACTCCGTGGAGGTCCAGGAGGCCCAGGAGGTCCTGGGGGACCCATGGGTCGCATGG
GAGGCCGTGGAGGAGATAGAGGAGGCTTCCCTCCAAGAGGACCCCGGGGTTCCCGAG
GGAACCCCTCTGGAGGAGGAAACGTCCAGCACCGAGCTGGAGACTGGCAGTGTCCCA
ATCCGGGTTGTGGAAACCAGAACTTCGCCTGGAGAACAGAGTGCAACCAGTGTAAGGC
CCCAAAGCCTGAAGGCTTCCTCCCGCCACCCTTTCCGCCCCCGGGTGGTGATCGTGGC
AGAGGTGGCCCTGGTGGCATGCGGGGAGGAAGAGGTGGCCTCATGGATCGTGGTGGT
CCCGGTGGAATGTTCAGAGGTGGCCGTGGTGGAGACAGAGGTGGCTTCCGTGGTGGC
CGGGGCATGGACCGAGGTGGCTTTGGTGGAGGAAGACGAGGTGGCCCTGGGGGGCC
CCCTGGACCTTTGATGGAACAGGATTACAAGGATGACGACGATAAGGGTACCGAGCAG
AAGCTGATCTCAGAGGAGGACCTGCTAGCCACCATGGACGCACAAACACGACGACGTG
AGCGTCGCGCTGAGAAACAAGCTCAATGGAAAGCTGCAAACCCACCGCTCGACGGAGC
CGGAGCTGGCGCTGGAGCTGGAGCCGGAGCTGGCGGTCTAGCCACCATGGACGCACA
 
AACACGACGACGTGAGCGTCGCGCTGAGAAACAAGCTCAATGGAAAGCTGCAAACCCA
CCGCTCGACGGAGCCGGAGCTGGCGCTGGAGCTGGAGCCGGAGCTGGCGGTCTAGC
CACCATGGACGCACAAACACGACGACGTGAGCGTCGCGCTGAGAAACAAGCTCAATGG
AAAGCTGCAAACCCACCGCTCGACGGAGCCGGAGCTGGCGCTGGAGCTGGAGCCGGA
GCTGGCGGTCTAGCCACCATGGACGCACAAACACGACGACGTGAGCGTCGCGCTGAG
AAACAAGCTCAATGGAAAGCTGCAAACCCACCGCTCGAGTCTAGAGGGCCCGTTTAA
(SEQ ID NO:161)
Protein:

MCRAISLRRLLLLLLQLSQLLAVTQGMLMASNDYTQQATQSYGAYPTQPGQGYSQQSSQP
YGQQSYSGYSQSTDTSGYGQSSYSSYGQSQNTGYGTQSTPQGYGSTGGYGSSQSSQSS
YGQQSSYPGYGQQPAPSSTSGSYGSSSQSSSYGQPQSGSYSQQPSYGGQQQSYGQQQ
SYNPPQGYGQQNQYNSSSGGGGGGGGGNYGQDQSSMSSGGGSGGGYGNQDQSGG
GGSGGYGQQDRGGRGRGGSGGGGGGGGGYNRSSGGYEPRGRGGGRGGRGGMGGS
DRGGFNKFGGPRDQGSRHDSEQDNSDNNTIFVQGLGENVTIESVADYFKQIGIIKTNKKTG
QPMINLYTDRETGKLKGEATVSFDDPPSAKAAIDWFDGKEFSGNPIKVSFATRRADFNRGG
GNGRGGRGRGGPMGRGGYGGGGSGGGGRGGFPSGGGGGGGQQRAGDWKCPNPTCE
NMNFSWRNECNQCKAPKPDGPGGGPGGSHMGGNYGDDRRGGRGGGTGKLFWALVVVA
GVLFCYGLLVTVALCVIWVRSMASTDYSTYSQAAAQQGYSAYTAQPTQGYAQTTQAYGQQ
SYGTYGQPTDVSYTQAQTTATYGQTAYATSYGQPPTGYTTPTAPQAYSQPVQGYGTGAYD
TTTATVTTTQASYAAQSAYGTQPAYPAYGQQPAATAPTRPQDGNKPTETSQPQSSTGGYN
QPSLGYGQSNYSYPQVPGSYPMQPVTAPPSYPPTSYSSTQPTSYDQSSYSQQNTYGQPS
SYGQQSSYGQQSSYGQQPPTSYPPQTGSYSQAPSQYSQQSSSYGQQSSFRQDHPSSMG
VYGQESGGFSGPGENRSMSGPDNRGRGRGGFDRGGMSRGGRGGGRGGMGSAGERGG
FNKPGGPMDEGPDLDLGPPVDPDEDSDNSAIYVQGLNDSVTLDDLADFFKQCGVVKMNKR
TGQPMIHIYLDKETGKPKGDATVSYEDPPTAKAAVEWFDGKDFQGSKLKVSLARKKPPMNS
MRGGLPPREGRGMPPPLRGGPGGPGGPGGPMGRMGGRGGDRGGFPPRGPRGSRGNP
SGGGNVQHRAGDWQCPNPGCGNQNFAWRTECNQCKAPKPEGFLPPPFPPPGGDRGRG
GPGGMRGGRGGLMDRGGPGGMFRGGRGGDRGGFRGGRGMDRGGFGGGRRGGPGGP
PGPLMEQDYKDDDDKGTEQKLISEEDLLATMDAQTRRRERRAEKQAQWKAANPPLDGAG
AGAGAGAGAGGLATMDAQTRRRERRAEKQAQWKAANPPLDGAGAGAGAGAGAGGLATM
DAQTRRRERRAEKQAQWKAANPPLDGAGAGAGAGAGAGGLATMDAQTRRRERRAEKQA
QWKAANPPLESRGPV (SEQ ID NO:162)

FRB-CD28-FUS-MCP-PyIRS^{AA}
DNA: 
ATGTGCCGAGCCATCTCTCTTAGGCGCTTGCTGCTGCTGCTGCAGCTGTCACAACT
CCTAGCTGTCACTCAAGGGATGCTCGAGATGTGGCATGAAGGCCTGGAAGAGGCATCT
CGTTTGTACTTTGGGGAAAGGAACGTGAAAGGCATGTTTGAGGTGCTGGAGCCCTTGC
ATGCTATGATGGAACGGGGCCCCCAGACTCTGAAGGAAACATCCTTTAATCAGGCCTAT
GGTCGAGATTTAATGGAGGCCCAAGAGTGGTGCAGGAAGTACATGAAATCAGGGAATG
TCAAGGACCTCCTCCAAGCCTGGGACCTCTATTATCATGTGTTCCGACGAATCTCAAAG
ACTAGAACCGGTAAGCTTTTTTGGGCACTGGTCGTGGTTGCTGGAGTCCTGTTTTGTTA
TGGCTTGCTAGTGACAGTGGCTCTTTGTGTTATCTGGGTAAGATCTGGTATGGCCTCAA
ACGATTATACCCAACAAGCAACCCAAAGCTATGGGGCCTACCCCACCCAGCCCGGGCA
GGGCTATTCCCAGCAGAGCAGTCAGCCCTACGGACAGCAGAGTTACAGTGGTTATAGC
CAGTCCACGGACACTTCAGGATATGGCCAGAGCAGCTATTCTTCTTATGGCCAGAGCCA
GAACACAGGCTATGGAACTCAGTCAACTCCCCAGGGATATGGCTCGACTGGCGGCTAT
GGCAGTAGCCAGAGCTCCCAATCGTCTTACGGGCAGCAGTCCTCCTACCCTGGCTATG
GCCAGCAGCCAGCTCCCAGCAGCACCTCGGGAAGTTACGGTAGCAGTTCTCAGAGCAG
CAGCTATGGGCAGCCCCAGAGTGGGAGCTACAGCCAGCAGCCTAGCTATGGTGGACA
GCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTAT
GGCCAAGATCAATCCTCCATGAGTAGTGGTGGTGGCAGTGGTGGCGGTTATGGCAATC
AAGACCAGAGTGGTGGAGGTGGCAGCGGTGGCTATGGACAGCAGGACCGTGGAGGCC
GCGGCAGGGGTGGCAGTGGTGGCGGCGGCGGCGGCGGTGGTGGTTACAACCGC
AGCAGTGGTGGCTATGAACCCAGAGGTCGTGGAGGTGGCCGTGGAGGCAGAGGTGGC
ATGGGCGGAAGTGACCGTGGTGGCTTCAATAAATTTGGTGGCCCTCGGGACCAAGGAT
CACGTCATGACTCCGAACAGGATAATTCAGACAACAACACCATCTTTGTGCAAGGCCTG
GGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAA
GCTGAAGGGAGAGGCAACGGTCTCTTTTGATGACCCACCTTCAGCTAAAGCAGCTATTG
ACTGGTTTGATGGTAAAGAATTCTCCGGAAATCCTATCAAGGTCTCATTTGCTACTCGCC
GGGCAGACTTTAATCGGGGTGGTGGCAATGGTCGTGGAGGCCGAGGGCGAGGAGGAC
CCATGGGCCGTGGAGGCTATGGAGGTGGTGGCAGTGGTGGTGGTGGCCGAGGAGGAT
TTCCCAGTGGAGGTGGTGGCGGTGGAGGACAGCAGCGAGCTGGTGACTGGAAGTGTC
CTAATCCCACCTGTGAGAATATGAACTTCTCTTGGAGGAATGAATGCAACCAGTGTAAG
GCCCCTAAACCAGATGGCCCAGGAGGGGGACCAGGTGGCTCTCACATGGGGGGTAAC
TACGGGGATGATCGTCGTGGTGGCAGAGGAGGCGATTACAAGGATGACGACGATAAGG
GTACCGGCGCCCCCGGCTCCGCCGGCTCCGCCGCCGGCTCCGGCGCTTCTAACTTTA
CTCAGTTCGTTCTCGTCGACAATGGCGGAACTGGCGACGTGACTGTCGCCCCAAGCAA
CTTCGCTAACGGGATCGCTGAATGGATCAGCTCTAACTCGCGTTCACAGGCTTACAAAG
 
TAACCTGTAGCGTTCGTCAGAGCTCTGCGCAGAATCGCAAATACACCATCAAAGTCGAG
GTGCCTAAAGGCGCCTGGCGTTCGTACTTAAATATGGAACTAACCATTCCAATTTTCGC
CACGAATTCCGACTGCGAGCTTATTGTTAAGGCAATGCAAGGTCTCCTAAAAGATGGAA
ACCCGATTCCCTCAGCAATCGCAGCAAACTCCGGCATCTACGGTACCGGCGCCCCCGG
CTCCGCCGGCTCCGCCGCCGGCTCCGGCGCGTGCCCGGTGCCGCTGCAGCTGCCGC
CGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCG
TTCGAAAATCTATATTGAGATGGCGTGTGGCGATCATCTGGTTGTGAACAATAGCCGCT
CTTCTCGTACAGCACGTGCACTGCGTCACCACAAATATCGTAAAACCTGTAAACGTTGC
CGTGTGTCCGATGAGGATCTGAACAAATTCCTGACAAAAGCCAATGAGGACCAAACAAG
CGTGAAAGTGAAAGTCGTTAGCGCTCCTACCCGTACTAAAAAAGCAATGCCGAAATCCG
TTGCTCGTGCCCCTAAACCACTGGAAAACACTGAAGCAGCACAGGCACAGCCGTCTGG
AAGCAAATTCTCTCCGGCCATTCCTGTTTCTACCCAGGAGTCCGTTTCTGTTCCAGCAA
GTGTGAGCACCAGCATTAGCAGTATTAGCACCGGTGCCACCGCTAGCGCCCTGGTTAA
AGGCAATACCAATCCGATTACAAGCATGTCTGCCCCGGTTCAAGCATCAGCTCCAGCAC
TGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAA
AGACCTGCAACAAATCTATGCCGAAGAACGTGAGAACTATCTGGGGAAACTGGAACGTG
AAATCACCCGCTTTTTCGTGGATCGTGGCTTTCTGGAGATCAAATCCCCGATTCTGATTC
CTCTGGAGTATATCGAGCGTATGGGCATCGACAATGATACCGAACTGAGCAAACAAATT
TTCCGTGTGGATAAAAACTTCTGTCTGCGCCCTATGCTGGCACCAAATCTGTATAACTAT
CTGCGCAAACTGGACCGTGCCCTGCCTGATCCTATCAAAATCTTCGAGATCGGCCCGT
GTTATCGTAAAGAGTCCGACGGTAAAGAACATCTGGAGGAGTTTACCATGCTGGCCTTT
GCCCAAATGGGTTCAGGTTGTACTCGTGAGAACCTGGAAAGCATCATCACCGATTTTCT
GAACCACCTGGGCATTGACTTCAAAATTGTGGGCGACAGCTGTATGGTGTATGGCGACA
CCCTGGATGTCATGCACGGCGACCTGGAACTGTCTAGTGCCGTTGTTGGACCAATTCC
GCTGGACCGTGAGTGGGGTATCGACAAACCGTGGATCGGAGCAGGATTCGGTCTGGAA
CGCCTGCTGAAAGTGAAACACGACTTCAAAAACATCAAACGTGCCGCCCGTTCTGAATC
GTATTATAACGGGATCTCTACGAACCTGTAA (SEQ ID NO:163)
Protein:

MCRAISLRRLLLLLLQLSQLLAVTQGMLEMWHEGLEEASRLYFGERNVKGMFEVLEPLHAM
MERGPQTLKETSFNQAYGRDLMEAQEWCRKYMKSGNVKDLLQAWDLYYHVFRRISKTRT
GKLFWALVVVAGVLFCYGLLVTVALCVIWVRSGMASNDYTQQATQSYGAYPTQPGQGYSQ
QSSQPYGQQSYSGYSQSTDTSGYGQSSYSSYGQSQNTGYGTQSTPQGYGSTGGYGSSQ
SSQSSYGQQSSYPGYGQQPAPSSTSGSYGSSSQSSSYGQPQSGSYSQQPSYGGQQQSY
GQQQSYNPPQGYGQQNQYNSSSGGGGGGGGGGNYGQDQSSMSSGGGSGGGYGNQD

QSGGGGSGGYGQQDRGGRGRGGSGGGGGGGGGYNRSSGGYEPRGRGGGRGGRGG
MGGSDRGGFNKFGGPRDQGSRHDSEQDNSDNNTIFVQGLGENVTIESVADYFKQIGIIKTN
KKTGQPMINLYTDRETGKLKGEATVSFDDPPSAKAAIDWFDGKEFSGNPIKVSFATRRADFN
RGGGNGRGGRGRGGPMGRGGYGGGGSGGGGRGGFPSGGGGGGGQQRAGDWKCPNP
TCENMNFSWRNECNQCKAPKPDGPGGGPGGSHMGGNYGDDRRGGRGGDYKDDDDKG
TGAPGSAGSAAGSGASNFTQFVLVDNGGTGDVTVAPSNFANGIAEWISSNSRSQAYKVTC
SVRQSSAQNRKYTIKVEVPKGAWRSYLNMELTIPIFATNSDCELIVKAMQGLLKDGNPIPSAI
AANSGIYGTGAPGSAGSAAGSGACPVPLQLPPLERLTLDDKKPLNTLISATGLWMSRTGTIH
KIKHHEVSRSKIYIEMACGDHLVVNNSRSSRTARALRHHKYRKTCKRCRVSDEDLNKFLTKA
NEDQTSVKVKVVSAPTRTKKAMPKSVARAPKPLENTEAAQAQPSGSKFSPAIPVSTQESVS
VPASVSTSISSISTGATASALVKGNTNPITSMSAPVQASAPALTKSQTDRLEVLLNPKDEISLN
SGKPFRELESELLSRRKKDLQQIYAEERENYLGKLEREITRFFVDRGFLEIKSPILIPLEYIERM
GIDNDTELSKQIFRVDKNFCLRPMLAPNLYNYLRKLDRALPDPIKIFEIGPCYRKESDGKEHL
EEFTMLAFAQMGSGCTRENLESIITDFLNHLGIDFKIVGDSCMVYGDTLDVMHGDLELSSAV
VGPIPLDREWGIDKPWIGAGFGLERLLKVKHDFKNIKRAARSESYYNGISTNL (SEQ ID
NO:164)

FRB-CD28-FUS-MCP-PyIRS^{AF}
DNA: 
ATGTGCCGAGCCATCTCTCTTAGGCGCTTGCTGCTGCTGCTGCAGCTGTCACAACT
CCTAGCTGTCACTCAAGGGATGCTCGAGATGTGGCATGAAGGCCTGGAAGAGGCATCT
CGTTTGTACTTTGGGGAAAGGAACGTGAAAGGCATGTTTGAGGTGCTGGAGCCCTTGC
ATGCTATGATGGAACGGGGCCCCCAGACTCTGAAGGAAACATCCTTTAATCAGGCCTAT
GGTCGAGATTTAATGGAGGCCCAAGAGTGGTGCAGGAAGTACATGAAATCAGGGAATG
TCAAGGACCTCCTCCAAGCCTGGGACCTCTATTATCATGTGTTCCGACGAATCTCAAAG
ACTAGAACCGGTAAGCTTTTTTGGGCACTGGTCGTGGTTGCTGGAGTCCTGTTTTGTTA
TGGCTTGCTAGTGACAGTGGCTCTTTGTGTTATCTGGGTAAGATCTGGTATGGCCTCAA
ACGATTATACCCAACAAGCAACCCAAAGCTATGGGGCCTACCCCACCCAGCCCGGGCA
GGGCTATTCCCAGCAGAGCAGTCAGCCCTACGGACAGCAGAGTTACAGTGGTTATAGC
CAGTCCACGGACACTTCAGGATATGGCCAGAGCAGCTATTCTTCTTATGGCCAGAGCCA
GAACACAGGCTATGGAACTCAGTCAACTCCCCAGGGATATGGCTCGACTGGCGGCTAT
GGCAGTAGCCAGAGCTCCCAATCGTCTTACGGGCAGCAGTCCTCCTACCCTGGCTATG
GCCAGCAGCCAGCTCCCAGCAGCACCTCGGGAAGTTACGGTAGCAGTTCTCAGAGCAG
CAGCTATGGGCAGCCCCAGAGTGGGAGCTACAGCCAGCAGCCTAGCTATGGTGGACA
GCAGCAAAGCTATGGACAGCAGCAAAGCTATAATCCCCCTCAGGGCTATGGACAGCAG
AACCAGTACAACAGCAGCAGTGGTGGTGGAGGTGGAGGTGGAGGTGGAGGTAACTAT
 
GGCCAAGATCAATCCTCCATGAGTAGTGGTGGCAGTGGTGGCGGTTATGGCAATC
AAGACCAGAGTGGTGGAGGTGGCAGCGGTGGCTATGGACAGCAGGACCGTGGAGGCC
GCGGCAGGGGTGGCAGTGGTGGCGGCGGCGGCGGCGGTGGTGGTTACAACCGC
AGCAGTGGTGGCTATGAACCCAGAGGTCGTGGAGGTGGCCGTGGAGGCAGAGGTGGC
ATGGGCGGAAGTGACCGTGGTGGCTTCAATAAATTTGGTGGCCCTCGGGACCAAGGAT
CACGTCATGACTCCGAACAGGATAATTCAGACAACAACACCATCTTTGTGCAAGGCCTG
GGTGAGAATGTTACAATTGAGTCTGTGGCTGATTACTTCAAGCAGATTGGTATTATTAAG
ACAAACAAGAAAACGGGACAGCCCATGATTAATTTGTACACAGACAGGGAAACTGGCAA
GCTGAAGGGAGAGGCAACGGTCTCTTTTGATGACCCACCTTCAGCTAAAGCAGCTATTG
ACTGGTTTGATGGTAAAGAATTCTCCGGAAATCCTATCAAGGTCTCATTTGCTACTCGCC
GGGCAGACTTTAATCGGGGTGGTGGCAATGGTCGTGGAGGCCGAGGGCGAGGAGGAC
CCATGGGCCGTGGAGGCTATGGAGGTGGTGGCAGTGGTGGTGGTGGCCGAGGAGGAT
TTCCCAGTGGAGGTGGTGGCGGTGGAGGACAGCAGCGAGCTGGTGACTGGAAGTGTC
CTAATCCCACCTGTGAGAATATGAACTTCTCTTGGAGGAATGAATGCAACCAGTGTAAG
GCCCCTAAACCAGATGGCCCAGGAGGGGGACCAGGTGGCTCTCACATGGGGGGTAAC
TACGGGGATGATCGTCGTGGTGGCAGAGGAGGCGATTACAAGGATGACGACGATAAGG
GTACCGGCGCCCCCGGCTCCGCCGGCTCCGCCGCCGGCTCCGGCGCTTCTAACTTTA
CTCAGTTCGTTCTCGTCGACAATGGCGGAACTGGCGACGTGACTGTCGCCCCAAGCAA
CTTCGCTAACGGGATCGCTGAATGGATCAGCTCTAACTCGCGTTCACAGGCTTACAAAG
TAACCTGTAGCGTTCGTCAGAGCTCTGCGCAGAATCGCAAATACACCATCAAAGTCGAG
GTGCCTAAAGGCGCCTGGCGTTCGTACTTAAATATGGAACTAACCATTCCAATTTTCGC
CACGAATTCCGACTGCGAGCTTATTGTTAAGGCAATGCAAGGTCTCCTAAAAGATGGAA
ACCCGATTCCCTCAGCAATCGCAGCAAACTCCGGCATCTACGGTACCGGCGCCCCCGG
CTCCGCCGGCTCCGCCGCCGGCTCCGGCGCGTGCCCGGTGCCGCTGCAGCTGCCGC
CGCTGGAACGCCTGACCCTGGATGATAAAAAACCGCTGAATACCCTGATCTCTGCTACT
GGTCTGTGGATGAGTCGTACCGGAACCATTCATAAAATCAAACACCACGAGGTTAGCCG
TTCGAAAATCTATATTGAGATGGCGTGTGGCGATCATCTGGTTGTGAACAATAGCCGCT
CTTCTCGTACAGCACGTGCACTGCGTCACCACAAATATCGTAAAACCTGTAAACGTTGC
CGTGTGTCCGATGAGGATCTGAACAAATTCCTGACAAAAGCCAATGAGGACCAAACAAG
CGTGAAAGTGAAAGTCGTTAGCGCTCCTACCCGTACTAAAAAAGCAATGCCGAAATCCG
TTGCTCGTGCCCCTAAACCACTGGAAAACACTGAAGCAGCACAGGCACAGCCGTCTGG
AAGCAAATTCTCTCCGGCCATTCCTGTTTCTACCCAGGAGTCCGTTTCTGTTCCAGCAA
GTGTGAGCACCAGCATTAGCAGTATTAGCACCGGTGCCACCGCTAGCGCCCTGGTTAA
AGGCAATACCAATCCGATTACAAGCATGTCTGCCCCGGTTCAAGCATCAGCTCCAGCAC
TGACAAAATCCCAAACCGATCGTCTGGAGGTTCTGCTGAATCCGAAAGACGAAATCAGC
CTGAATTCCGGCAAACCGTTTCGTGAACTGGAGAGCGAACTGCTGTCACGTCGTAAAAA
 
AGACCTGCAACAAATCTATGCCGAAGAACGTGAGAACTATCTGGGGAAACTGGAACGTG
AAATCACCCGCTTTTTCGTGGATCGTGGCTTTCTGGAGATCAAATCCCCGATTCTGATTC
CTCTGGAGTATATCGAGCGTATGGGCATCGACAATGATACCGAACTGAGCAAACAAATT
TTCCGTGTGGATAAAAACTTCTGTCTGCGCCCTATGCTAGCACCAAATCTGGCTAACTAT
CTGCGCAAACTGGACCGTGCCCTGCCTGATCCTATCAAAATCTTCGAGATCGGCCCGT
GTTATCGTAAAGAGTCCGACGGTAAAGAACATCTGGAGGAGTTTACCATGCTGAACTTT
TGCCAAATGGGTTCAGGTTGTACTCGTGAGAACCTGGAAAGCATCATCACCGATTTTCT
GAACCACCTGGGCATTGACTTCAAAATTGTGGGCGACAGCTGTATGGTGTTTGGCGACA
CCCTGGATGTCATGCACGGCGACCTGGAACTGTCTAGTGCCGTTGTGGGCCCAATCCC
GCTGGATCGTGAGTGGGGTATCGACAAACCTTGGATCGGTGCGGGTTTTGGTCTGGAG
CGTCTGCTGAAAGTAAAACACGACTTCAAGAACATCAAACGTGCTGCACGTTCCGAGTC
CTATTACAATGGTATTTCTACTAACCTGTAA (SEQ ID NO:165)
Protein:

MCRAISLRRLLLLLLQLSQLLAVTQGMLEMWHEGLEEASRLYFGERNVKGMFEVLEPLHAM
MERGPQTLKETSFNQAYGRDLMEAQEWCRKYMKSGNVKDLLQAWDLYYHVFRRISKTRT
GKLFWALVVVAGVLFCYGLLVTVALCVIWVRSGMASNDYTQQATQSYGAYPTQPGQGYSQ
QSSQPYGQQSYSGYSQSTDTSGYGQSSYSSYGQSQNTGYGTQSTPQGYGSTGGYGSSQ
SSQSSYGQQSSYPGYGQQPAPSSTSGSYGSSSQSSSYGQPQSGSYSQQPSYGGQQQSY
GQQQSYNPPQGYGQQNQYNSSSGGGGGGGGGNYGQDQSSMSSGGGSGGGYGNQD
QSGGGGSGGYGQQDRGGRGRGGSGGGGGGGGGYNRSSGGYEPRGRGGGRGGRGG
MGGSDRGGFNKFGGPRDQGSRHDSEQDNSDNNTIFVQGLGENVTIESVADYFKQIGIIKTN
KKTGQPMINLYTDRETGKLKGEATVSFDDPPSAKAAIDWFDGKEFSGNPIKVSFATRRADFN
RGGGNGRGGRGRGGPMGRGGYGGGGSGGGGRGGFPSGGGGGGGQQRAGDWKCPNP
TCENMNFSWRNECNQCKAPKPDGPGGGPGGSHMGGNYGDDRRGGRGGDYKDDDDKG
TGAPGSAGSAAGSGASNFTQFVLVDNGGTGDVTVAPSNFANGIAEWISSNSRSQAYKVTC
SVRQSSAQNRKYTIKVEVPKGAWRSYLNMELTIPIFATNSDCELIVKAMQGLLKDGNPIPSAI
AANSGIYGTGAPGSAGSAAGSGACPVPLQLPPLERLTLDDKKPLNTLISATGLWMSRTGTIH
KIKHHEVSRSKIYIEMACGDHLVVNNSRSSRTARALRHHKYRKTCKRCRVSDEDLNKFLTKA
NEDQTSVKVKVVSAPTRTKKAMPKSVARAPKPLENTEAAQAQPSGSKFSPAIPVSTQESVS
VPASVSTSISSISTGATASALVKGNTNPITSMSAPVQASAPALTKSQTDRLEVLLNPKDEISLN
SGKPFRELESELLSRRKKDLQQIYAEERENYLGKLEREITRFFVDRGFLEIKSPILIPLEYIERM
GIDNDTELSKQIFRVDKNFCLRPMLAPNLANYLRKLDRALPDPIKIFEIGPCYRKESDGKEHL
EEFTMLNFCQMGSGCTRENLESIITDFLNHLGIDFKIVGDSCMVFGDTLDVMHGDLELSSAV
VGPIPLDREWGIDKPWIGAGFGLERLLKVKHDFKNIKRAARSESYYNGISTNL (SEQ ID
NO:166)

FUS-MCP-PyIRS^{AF}
DNA: 
ATGGCCTCAAACGATTATACCCAACAAGCAACCCAAAGCTATGGGGCCTACCCCACCCA
GCCCGGGCAGGGCTATTCCCAGCAGAGCAGTCAGCCCTACGGACAGCAGAGTTACAGT
GGTTATAGCCAGTCCACGGACACTTCAGGATATGGCCAGAGCAGCTATTCTTCTTATGG
CCAGAGCCAGAACACAGGCTATGGAACTCAGTCAACTCCCCAGGGATATGGCTCGACT
GGCGGCTATGGCAGTAGCCAGAGCTCCCAATCGTCTTACGGGCAGCAGTCCTCCTACC
CTGGCTATGGCCAGCAGCCAGCTCCCAGCAGCACCTCGGGAAGTTACGGTAGCAGTTC
TCAGAGCAGCAGCTATGGGCAGCCCCAGAGTGGGAGCTACAGCCAGCAGCCTAGCTAT
GGTGGACAGCAGCAAAGCTATGGACAGCAGCAAAGCTATAATCCCCCTCAGGGCTATG
GACAGCAGAACCAGTACAACAGCAGCAGTGGTGGTGGAGGTGGAGGTGGAGGTGGAG
GTAACTATGGCCAAGATCAATCCTCCATGAGTAGTGGTGGTGGCAGTGGTGGCGGTTAT
GGCAATCAAGACCAGAGTGGTGGAGGTGGCAGCGGTGGCTATGGACAGCAGGACCGT
GGAGGCCGCGGCAGGGGTGGCAGTGGTGGCGGCGGCGGCGGCGGTGGTGGTT
ACAACCGCAGCAGTGGTGGCTATGAACCCAGAGGTCGTGGAGGTGGCCGTGGAGGCA
GAGGTGGCATGGGCGGAAGTGACCGTGGTGGCTTCAATAAATTTGGTGGCCCTCGGGA
CCAAGGATCACGTCATGACTCCGAACAGGATAATTCAGACAACAACACCATCTTTGTGC
AAGGCCTGGGTGAGAATGTTACAATTGAGTCTGTGGCTGATTACTTCAAGCAGATTGGT
ATTATTAAGACAAACAAGAAAACGGGACAGCCCATGATTAATTTGTACACAGACAGGGA
AACTGGCAAGCTGAAGGGAGAGGCAACGGTCTCTTTTGATGACCCACCTTCAGCTAAAG
CAGCTATTGACTGGTTTGATGGTAAAGAATTCTCCGGAAATCCTATCAAGGTCTCATTTG
CTACTCGCCGGGCAGACTTTAATCGGGGTGGTGGCAATGGTCGTGGAGGCCGAGGGC
GAGGAGGACCCATGGGCCGTGGAGGCTATGGAGGTGGTGGCAGTGGTGGTGGTGGC
CGAGGAGGATTTCCCAGTGGAGGTGGTGGCGGTGGAGGACAGCAGCGAGCTGGTGAC
TGGAAGTGTCCTAATCCCACCTGTGAGAATATGAACTTCTCTTGGAGGAATGAATGCAA
CCAGTGTAAGGCCCCTAAACCAGATGGCCCAGGAGGGGGACCAGGTGGCTCTCACAT
GGGGGGTAACTACGGGGATGATCGTCGTGGTGGCAGAGGAGGCGATTACAAGGATGA
CGACGATAAGGGTACCGGCGCCCCCGGCTCCGCCGGCTCCGCCGCCGGCTCCGGCG
CTTCTAACTTTACTCAGTTCGTTCTCGTCGACAATGGCGGAACTGGCGACGTGACTGTC
GCCCCAAGCAACTTCGCTAACGGGATCGCTGAATGGATCAGCTCTAACTCGCGTTCACA
GGCTTACAAAGTAACCTGTAGCGTTCGTCAGAGCTCTGCGCAGAATCGCAAATACACCA
TCAAAGTCGAGGTGCCTAAAGGCGCCTGGCGTTCGTACTTAAATATGGAACTAACCATT
CCAATTTTCGCCACGAATTCCGACTGCGAGCTTATTGTTAAGGCAATGCAAGGTCTCCT
AAAAGATGGAAACCCGATTCCCTCAGCAATCGCAGCAAACTCCGGCATCTACGGTACCG
GCGCCCCCGGCTCCGCCGGCTCCGCCGCCGGCTCCGGCGCGTGCCCGGTGCCGCTG
CAGCTGCCGCCGCTGGAACGCCTGACCCTGGATGATAAAAAACCGCTGAATACCCTGA
 
TCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAA
CAATAGCCGCTCTTCTCGTACAGCACGTGCACTGCGTCACCACAAATATCGTAAAACCT
GTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAAT
GCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTC
TGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATC
AGCTCCAGCACTGACAAAATCCCAAACCGATCGTCTGGAGGTTCTGCTGAATCCGAAAG
ACGAAATCAGCCTGAATTCCGGCAAACCGTTTCGTGAACTGGAGAGCGAACTGCTGTCA
CGTCGTAAAAAAGACCTGCAACAAATCTATGCCGAAGAACGTGAGAACTATCTGGGGAA
ACTGGAACGTGAAATCACCCGCTTTTTCGTGGATCGTGGCTTTCTGGAGATCAAATCCC
CGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGA
GATCGGCCCGTGTTATCGTAAAGAGTCCGACGGTAAAGAACATCTGGAGGAGTTTACCA
TGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGT
GTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTT
TTGGTCTGGAGCGTCTGCTGAAAGTAAAACACGACTTCAAGAACATCAAACGTGCTGCA
CGTTCCGAGTCCTATTACAATGGTATTTCTACTAACCTGTAA (SEQ ID NO:167)
Protein:

MASNDYTQQATQSYGAYPTQPGQGYSQQSSQPYGQQSYSGYSQSTDTSGYGQSSYSSY
GQSQNTGYGTQSTPQGYGSTGGYGSSQSSQSSYGQQSSYPGYGQQPAPSSTSGSYGSS
SQSSSYGQPQSGSYSQQPSYGGQQQSYGQQQSYNPPQGYGQQNQYNSSSGGGGGG
GGGNYGQDQSSMSSGGGSGGGYGNQDQSGGGGSGGYGQQDRGGRGRGGSGGGGG
GGGGYNRSSGGYEPRGRGGGRGGRGGMGGSDRGGFNKFGGPRDQGSRHDSEQDNSD
NNTIFVQGLGENVTIESVADYFKQIGIIKTNKKTGQPMINLYTDRETGKLKGEATVSFDDPPSA
KAAIDWFDGKEFSGNPIKVSFATRRADFNRGGGNGRGGRGRGGPMGRGGYGGGGSGGG
GRGGFPSGGGGGGGQQRAGDWKCPNPTCENMNFSWRNECNQCKAPKPDGPGGGPGG
SHMGGNYGDDRRGGRGGDYKDDDDKGTGAPGSAGSAAGSGASNFTQFVLVDNGGTGDV
TVAPSNFANGIAEWISSNSRSQAYKVTCSVRQSSAQNRKYTIKVEVPKGAWRSYLNMELTIP
IFATNSDCELIVKAMQGLLKDGNPIPSAIAANSGIYGTGAPGSAGSAAGSGACPVPLQLPPLE
RLTLDDKKPLNTLISATGLWMSRTGTIHKIKHHEVSRSKIYIEMACGDHLVVNNSRSSRTARA

LRHHKYRKTCKRCRVSDEDLNKFLTKANEDQTSVKVKVVSAPTRTKKAMPKSVARAPKPLE
NTEAAQAQPSGSKFSPAIPVSTQESVSVPASVSTSISSISTGATASALVKGNTNPITSMSAPV
QASAPALTKSQTDRLEVLLNPKDEISLNSGKPFRELESELLSRRKKDLQQIYAEERENYLGKL
EREITRFFVDRGFLEIKSPILIPLEYIERMGIDNDTELSKQIFRVDKNFCLRPMLAPNLANYLRK
LDRALPDPIKIFEIGPCYRKESDGKEHLEEFTMLNFCQMGSGCTRENLESIITDFLNHLGIDFK
IVGDSCMVFGDTLDVMHGDLELSSAVVGPIPLDREWGIDKPWIGAGFGLERLLKVKHDFKNI
KRAARSESYYNGISTNL (SEQ ID NO:168)

SPD5-MCP-PyIRS^{AF}
DNA: 
ATGGAGGACAACAGCGTGCTGAACGAGGACAGCAACCTGGAGCACGTGGAGGGCCAG
CCCAGAAGAAGCATGAGCCAGCCCGTGCTGAACGTGGAGGGCGACAAGAGAACCAGC
AGCACCAGCGCCACCCAGCAGCAGGTGCTGAGCGGCGCCTTCAGCAGCGCCGACGTG
AGAAGCATCCCCATCATCCAGACCTGGGAGGAGAACAAGGCCCTGAAGACCAAGATCA
CCATCCTGAGAGGCGAGCTGCAGATGTACCAGAGAAGATACAGCGAGGCCAAGGAGG
CCAGCCAGAAGAGAGTGAAGGAGGTGATGGACGACTACGTGGACCTGAAGCTGGGCC
AGGAGAACGTGCAGGAGAAGATGGAGCAGTACAAGCTGATGGAGGAGGACCTGCTGG
CCATGCAGAGCAGAATCGAGACCAGCGAGGACAACTTCGCCAGACAGATGAAGGAGTT
CGAGGCCCAGAAGCACGCCATGGAGGAGAGAATCAAGGAGCTGGAGCTGAGCGCCAC
CGACGCCAACAACACCACCGTGGGCAGCTTCAGAGGCACCCTGGACGACATCCTGAAG
AAGAACGACCCCGACTTCACCCTGACCAGCGGCTACGAGGAGAGAAAGATCAACGACC
TGGAGGCCAAGCTGCTGAGCGAGATCGACAAGGTGGCCGAGCTGGAGGACCACATCC
AGCAGCTGAGACAGGAGCTGGACGACCAGAGCGCCAGACTGGCCGACAGCGAGAACG
TGAGAGCCCAGCTGGAGGCCGCCACCGGCCAGGGCATCCTGGGCGCCGCCGGCAAC
GCCATGGTGCCCAACAGCACCTTCATGATCGGCAACGGCAGAGAGAGCCAGACCAGAG
ACCAGCTGAACTACATCGACGACCTGGAGACCAAGCTGGCCGACGCCAAGAAGGAGAA
CGACAAGGCCAGACAGGCCCTGGTGGAGTACATGAACAAGTGCAGCAAGCTGGAGCA
CGAGATCAGAACCATGGTGAAGAACAGCACCTTCGACAGCAGCAGCATGCTGCTGGGC
GGCCAGACCAGCGACGAGCTGAAGATCCAGATCGGCAAGGTGAACGGCGAGCTGAAC
GTGCTGAGAGCCGAGAACAGAGAGCTGAGAATCAGATGCGACCAGCTGACCGGCGGC
GACGGCAACCTGAGCATCAGCCTGGGCCAGAGCAGACTGATGGCCGGCATCGCCACC
AACGACGTGGACAGCATCGGCCAGGGCAACGAGACCGGCGGCACCAGCATGAGAATC
CTGCCCAGAGAGAGCCAGCTGGACGACCTGGAGGAGAGCAAGCTGCCCCTGATGGAC
ACCAGCAGCGCCGTGAGAAACCAGCAGCAGTTCGCCAGCATGTGGGAGGACTTCGAG
AGCGTGAAGGACAGCCTGCAGAACAACCACAACGACACCCTGGAGGGCAGCTTCAACA
GCAGCATGCCCCCCCCCGGCAGAGACGCCACCCAGAGCTTCCTGAGCCAGAAGAGCT
 
TCAAGAACAGCCCCATCGTGATGCAGAAGCCCAAGAGCCTGCACCTGCACCTGAAGAG
CCACCAGAGCGAGGGCGCCGGCGAGCAGATCCAGAACAACAGCTTCAGCACCAAGAC
CGCCAGCCCCCACGTGAGCCAGAGCCACATCCCCATCCTGCACGACATGCAGCAGATC
CTGGACAGCAGCGCCATGTTCCTGGAGGGCCAGCACGACGTGGCCGTGAACGTGGAG
CAGATGCAGGAGAAGATGAGCCAGATCAGAGAGGCCCTGGCCAGACTGTTCGAGAGAC
TGAAGAGCAGCGCCGCCCTGTTCGAGGAGATCCTGGAGAGAATGGGCAGCAGCGACC
CCAACGCCGACAAGATCAAGAAGATGAAGCTGGCCTTCGAGACCAGCATCAACGACAA
GCTGAACGTGAGCGCCATCCTGGAGGCCGCCGAGAAGGACCTGCACAACATGAGCCT
GAACTTCAGCATCCTGGAGAAGAGCATCGTGAGCCAGGCCGCCGAGGCCAGCAGAAG
ATTCACCATCGCCCCCGACGCCGAGGACGTGGCCAGCAGCAGCCTGCTGAACGCCAG
CTACAGCCCCCTGTTCAAGTTCACCAGCAACAGCGACATCGTGGAGAAGCTGCAGAAC
GAGGTGAGCGAGCTGAAGAACGAGCTGGAGATGGCCAGAACCAGAGACATGAAGC
CCCCTGAACGGCAGCAGCGGCAGACTGAGCGACGTGCAGATCAACACCAACAGAATGT
TCGAGGACCTGGAGGTGAGCGAGGCCACCCTGCAGAAGGCCAAGGAGGAGAACAGCA
CCCTGAAGAGCCAGTTCGCCGAGCTGGAGGCCAACCTGCACCAGGTGAACAGCAAGCT
GGGCGAGGTGAGATGCGAGCTGAACGAGGCCCTGGCCAGAGTGGACGGCGAGCAGG
AGACCAGAGTGAAGGCCGAGAACGCCCTGGAGGAGGCCAGACAGCTGATCAGCAGCC
TGAAGCACGAGGAGAACGAGCTGAAGAAGACCATCACCGACATGGGCATGAGACTGAA
CGAGGCCAAGAAGAGCGACGAGTTCCTGAAGAGCGAGCTGAGCACCGCCCTGGAGGA
GGAGAAGAAGAGCCAGAACCTGGCCGACGAGCTGAGCGAGGAGCTGAACGGCTGGAG
AATGAGAACCAAGGAGGCCGAGAACAAGGTGGAGCACGCCAGCAGCGAGAAGAGCGA
GATGCTGGAGAGAATCGTGCACCTGGAGACCGAGATGGAGAAGCTGAGCACCAGCGA
GATCGCCGCCGACTACTGCAGCACCAAGATGACCGAGAGAAAGAAGGAGATCGAGCTG
GCCAAGTACAGAGAGGACTTCGAGAACGCCGCCATCGTGGGCCTGGAGAGAATCAGCA
AGGAGATCAGCGAGCTGACCAAGAAGACCCTGAAGGCCAAGATCATCCCCAGCAACAT
CAGCAGCATCCAGCTGGTGTGCGACGAGCTGTGCAGAAGACTGAGCAGAGAGAGAGA
GCAGCAGCACGAGTACGCCAAGGTGATGAGAGACGTGAACGAGAAGATCGAGAAGCT
GCAGCTGGAGAAGGACGCCCTGGAGCACGAGCTGAAGATGATGAGCAGCAACAACGA
GAACGTGCCCCCCGTGGGCACCAGCGTGAGCGGCATGCCCACCAAGACCAGCAACCA
GAAGTGCGCCCAGCCCCACTACACCAGCCCCACCAGACAGCTGCTGCACGAGAGCAC
CATGGCCGTGGACGCCATCGTGCAGAAGCTGAAGAAGACCCACAACATGAGCGGCATG
GGCCCCGAGCTGAAGGAGACCATCGGCAACGTGATCAACGAGAGCAGAGTGCTGAGA
GACTTCCTGCACCAGAAGCTGATCCTGTTCAAGGGCATCGACATGAGCAACTGGAAGAA
CGAGACCGTGGACCAGCTGATCACCGACCTGGGCCAGCTGCACCAGGACAACCTGAT
GCTGGAGGAGCAGATCAAGAAGTACAAGGAGCTGAAGCTGACCAAGAGCGCCATC
CCCACCCTGGGCGTGGAGTTCCAGGACAGAATCAAGACCGAGATCGGCAAGATCGCCA
 
CCGACATGGGCGGCGCCGTGAAGGAGATCAGAAAGAAGGGTACCGAGCAGAAGCTGA
TCTCAGAGGAGGACCTGGGCGCCCCCGGCTCCGCCGGCTCCGCCGCCGGCTCCGGC
GCTTCTAACTTTACTCAGTTCGTTCTCGTCGACAATGGCGGAACTGGCGACGTGACTGT
CGCCCCAAGCAACTTCGCTAACGGGATCGCTGAATGGATCAGCTCTAACTCGCGTTCAC
AGGCTTACAAAGTAACCTGTAGCGTTCGTCAGAGCTCTGCGCAGAATCGCAAATACACC
ATCAAAGTCGAGGTGCCTAAAGGCGCCTGGCGTTCGTACTTAAATATGGAACTAACCAT
TCCAATTTTCGCCACGAATTCCGACTGCGAGCTTATTGTTAAGGCAATGCAAGGTCTCCT
AAAAGATGGAAACCCGATTCCCTCAGCAATCGCAGCAAACTCCGGCATCTACGGTACCG
GCGCCCCCGGCTCCGCCGGCTCCGCCGCCGGCTCCGGCGCGTGCCCGGTGCCGCTG
CAGCTGCCGCCGCTGGAACGCCTGACCCTGGATGATAAAAACCGCTGAATACCCTGA
TCTCTGCTACTGGTCTGTGGATGAGTCGTACCGGAACCATTCATAAAATCAAACACCAC
GAGGTTAGCCGTTCGAAAATCTATATTGAGATGGCGTGTGGCGATCATCTGGTTGTGAA
CAATAGCCGCTCTTCTCGTACAGCACGTGCACTGCGTCACCACAAATATCGTAAAACCT
GTAAACGTTGCCGTGTGTCCGATGAGGATCTGAACAAATTCCTGACAAAAGCCAATGAG
GACCAAACAAGCGTGAAAGTGAAAGTCGTTAGCGCTCCTACCCGTACTAAAAAAGCAAT
GCCGAAATCCGTTGCTCGTGCCCCTAAACCACTGGAAAACACTGAAGCAGCACAGGCA
CAGCCGTCTGGAAGCAAATTCTCTCCGGCCATTCCTGTTTCTACCCAGGAGTCCGTTTC
TGTTCCAGCAAGTGTGAGCACCAGCATTAGCAGTATTAGCACCGGTGCCACCGCTAGC
GCCCTGGTTAAAGGCAATACCAATCCGATTACAAGCATGTCTGCCCCGGTTCAAGCATC
AGCTCCAGCACTGACAAAATCCCAAACCGATCGTCTGGAGGTTCTGCTGAATCCGAAAG
ACGAAATCAGCCTGAATTCCGGCAAACCGTTTCGTGAACTGGAGAGCGAACTGCTGTCA
CGTCGTAAAAAAGACCTGCAACAAATCTATGCCGAAGAACGTGAGAACTATCTGGGGAA
ACTGGAACGTGAAATCACCCGCTTTTTCGTGGATCGTGGCTTTCTGGAGATCAAATCCC
CGATTCTGATTCCTCTGGAGTATATCGAGCGTATGGGCATCGACAATGATACCGAACTG
AGCAAACAAATTTTCCGTGTGGATAAAAACTTCTGTCTGCGCCCTATGCTAGCACCAAAT
CTGGCTAACTATCTGCGCAAACTGGACCGTGCCCTGCCTGATCCTATCAAAATCTTCGA
GATCGGCCCGTGTTATCGTAAAGAGTCCGACGGTAAAGAACATCTGGAGGAGTTTACCA
TGCTGAACTTTTGCCAAATGGGTTCAGGTTGTACTCGTGAGAACCTGGAAAGCATCATC
ACCGATTTTCTGAACCACCTGGGCATTGACTTCAAAATTGTGGGCGACAGCTGTATGGT
GTTTGGCGACACCCTGGATGTCATGCACGGCGACCTGGAACTGTCTAGTGCCGTTGTG
GGCCCAATCCCGCTGGATCGTGAGTGGGGTATCGACAAACCTTGGATCGGTGCGGGTT
TTGGTCTGGAGCGTCTGCTGAAAGTAAAACACGACTTCAAGAACATCAAACGTGCTGCA
CGTTCCGAGTCCTATTACAATGGTATTTCTACTAACCTGTAA (SEQ ID NO:169)
Protein:

MEDNSVLNEDSNLEHVEGQPRRSMSQPVLNVEGDKRTSSTSATQQQVLSGAFSSADVRSI
PIIQTWEENKALKTKITILRGELQMYQRRYSEAKEASQKRVKEVMDDYVDLKLGQENVQEK

MEQYKLMEEDLLAMQSRIETSEDNFARQMKEFEAQKHAMEERIKELELSATDANNTTVGSF
RGTLDDILKKNDPDFTLTSGYEERKINDLEAKLLSEIDKVAELEDHIQQLRQELDDQSARLAD
SENVRAQLEAATGQGILGAAGNAMVPNSTFMIGNGRESQTRDQLNYIDDLETKLADAKKEN
DKARQALVEYMNKCSKLEHEIRTMVKNSTFDSSSMLLGGQTSDELKIQIGKVNGELNVLRAE
NRELRIRCDQLTGGDGNLSISLGQSRLMAGIATNDVDSIGQGNETGGTSMRILPRESQLDDL
EESKLPLMDTSSAVRNQQQFASMWEDFESVKDSLQNNHNDTLEGSFNSSMPPPGRDATQ
SFLSQKSFKNSPIVMQKPKSLHLHLKSHQSEGAGEQIQNNSFSTKTASPHVSQSHIPILHDM
QQILDSSAMFLEGQHDVAVNVEQMQEKMSQIREALARLFERLKSSAALFEEILERMGSSDP
NADKIKKMKLAFETSINDKLNVSAILEAAEKDLHNMSLNFSILEKSIVSQAAEASRRFTIAPDAE
DVASSSLLNASYSPLFKFTSNSDIVEKLQNEVSELKNELEMARTRDMRSPLNGSSGRLSDV
QINTNRMFEDLEVSEATLQKAKEENSTLKSQFAELEANLHQVNSKLGEVRCELNEALARVD
GEQETRVKAENALEEARQLISSLKHEENELKKTITDMGMRLNEAKKSDEFLKSELSTALEEE
KKSQNLADELSEELNGWRMRTKEAENKVEHASSEKSEMLERIVHLETEMEKLSTSEIAADY
CSTKMTERKKEIELAKYREDFENAAIVGLERISKEISELTKKTLKAKIIPSNISSIQLVCDELCRR
LSREREQQHEYAKVMRDVNEKIEKLQLEKDALEHELKMMSSNNENVPPVGTSVSGMPTKT
SNQKCAQPHYTSPTRQLLHESTMAVDAIVQKLKKTHNMSGMGPELKETIGNVINESRVLRD
FLHQKLILFKGIDMSNWKNETVDQLITDLGQLHQDNLMLEEQIKKYKKELKLTKSAIPTLGVEF
QDRIKTEIGKIATDMGGAVKEIRKKGTEQKLISEEDLGAPGSAGSAAGSGASNFTQFVLVDN
GGTGDVTVAPSNFANGIAEWISSNSRSQAYKVTCSVRQSSAQNRKYTIKVEVPKGAWRSYL
NMELTIPIFATNSDCELIVKAMQGLLKDGNPIPSAIAANSGIYGTGAPGSAGSAAGSGACPVP
LQLPPLERLTLDDKKPLNTLISATGLWMSRTGTIHKIKHHEVSRSKIYIEMACGDHLVVNNSR
SSRTARALRHHKYRKTCKRCRVSDEDLNKFLTKANEDQTSVKVKVVSAPTRTKKAMPKSVA
RAPKPLENTEAAQAQPSGSKFSPAIPVSTQESVSVPASVSTSISSISTGATASALVKGNTNPI
TSMSAPVQASAPALTKSQTDRLEVLLNPKDEISLNSGKPFRELESELLSRRKKDLQQIYAEE
RENYLGKLEREITRFFVDRGFLEIKSPILIPLEYIERMGIDNDTELSKQIFRVDKNFCLRPMLAP
NLANYLRKLDRALPDPIKIFEIGPCYRKESDGKEHLEEFTMLNFCQMGSGCTRENLESIITDFL
NHLGIDFKIVGDSCMVFGDTLDVMHGDLELSSAVVGPIPLDREWGIDKPWIGAGFGLERLLK
VKHDFKNIKRAARSESYYNGISTNL (SEQ ID NO:170)

### 9. POIs (reporters) and controls

GFP^{39TAG} (GFP with Amber-encoded amino acid position 39)
DNA: (Amber codon underlined) 
ATGGGCCGCCTGGAAAGCACCCCGCCGAAAAAAAAACGCAAAGTGGAAGATAGCGCGA
GCGATTACAAAGATGATGATGATAAAGTGAGCAAGGGCGAGGAGCTGTTCACCGGGGT
GGTGCCCATCCTGGTCGAGCTGGACGGCGACGTAAACGGCCACAAGTTCAGCGTGTCC
 
GGCGAGGGCGAGGGCGATGCCACCTAGGGCAAGCTGACCCTGAAGTTCATCTGCACC
ACCGGCAAGCTGCCCGTGCCCTGGCCCACCCTCGTGACCACCCTGACCTACGGCGTG
CAGTGCTTCAGCCGCTACCCCGACCACATGAAGCAGCACGACTTCTTCAAGTCCGCCAT
GCCCGAAGGCTACGTCCAGGAGCGCACCATCTTCTTCAAGGACGACGGCAACTACAAG
ACCCGCGCCGAGGTGAAGTTCGAGGGCGACACCCTGGTGAACCGCATCGAGCTGAAG
GGCATCGACTTCAAGGAGGACGGCAACATCCTGGGGCACAAGCTGGAGTACAACTACA
ACAGCCACAACGTCTATATCATGGCCGACAAGCAGAAGAACGGCATCAAGGCCAACTTC
AAGATCCGCCACAACATCGAGGACGGCAGCGTGCAGCTCGCCGACCACTACCAGCAGA
ACACCCCCATCGGCGACGGCCCCGTGCTGCTGCCCGACAACCACTACCTGAGCACCCA
GTCCGCCCTGAGCAAAGACCCCAACGAGAAGCGCGATCACATGGTCCTGCTGGAGTTC
GTGACCGCCGCCGGGATCACTCTCGGCATGGACGAGCTGTACAAGCATCACCATCACC
ATCACTAA (SEQ ID NO:171)
Protein: (X indicates non-canonical amino acid)

MGRLESTPPKKKRKVEDSASDYKDDDDKVSKGEELFTGVVPILVELDGDVNGHKFSVSGE
GEGDATXGKLTLKFICTTGKLPVPWPTLVTTLTYGVQCFSRYPDHMKQHDFFKSAMPEGYV
QERTIFFKDDGNYKTRAEVKFEGDTLVNRIELKGIDFKEDGNILGHKLEYNYNSHNVYIMADK
QKNGIKANFKIRHNIEDGSVQLADHYQQNTPIGDGPVLLPDNHYLSTQSALSKDPNEKRDHM
VLLEFVTAAGITLGMDELYKHHHHHH (SEQ ID NO:172)

GFP^{39TAG}-2xMS2 (GFP^{39TAG} with 2 MS2 stem-loops)
DNA: (MS2 stem-loops and Amber codon underlined) 
ATGGGCCGCCTGGAAAGCACCCCGCCGAAAAAAAAACGCAAAGTGGAAGATAGCGCGA
GCGATTACAAAGATGATGATGATAAAGTGAGCAAGGGCGAGGAGCTGTTCACCGGGGT
GGTGCCCATCCTGGTCGAGCTGGACGGCGACGTAAACGGCCACAAGTTCAGCGTGTCC
GGCGAGGGCGAGGGCGATGCCACCTAGGGCAAGCTGACCCTGAAGTTCATCTGCACC
ACCGGCAAGCTGCCCGTGCCCTGGCCCACCCTCGTGACCACCCTGACCTACGGCGTG
CAGTGCTTCAGCCGCTACCCCGACCACATGAAGCAGCACGACTTCTTCAAGTCCGCCAT
GCCCGAAGGCTACGTCCAGGAGCGCACCATCTTCTTCAAGGACGACGGCAACTACAAG
ACCCGCGCCGAGGTGAAGTTCGAGGGCGACACCCTGGTGAACCGCATCGAGCTGAAG
GGCATCGACTTCAAGGAGGACGGCAACATCCTGGGGCACAAGCTGGAGTACAACTACA
ACAGCCACAACGTCTATATCATGGCCGACAAGCAGAAGAACGGCATCAAGGCCAACTTC
AAGATCCGCCACAACATCGAGGACGGCAGCGTGCAGCTCGCCGACCACTACCAGCAGA
ACACCCCCATCGGCGACGGCCCCGTGCTGCTGCCCGACAACCACTACCTGAGCACCCA
GTCCGCCCTGAGCAAAGACCCCAACGAGAAGCGCGATCACATGGTCCTGCTGGAGTTC
GTGACCGCCGCCGGGATCACTCTCGGCATGGACGAGCTGTACAAGCATCACCATCACC
 
ATCACTAAGGATCCTAAGGTACCTAATTGCCTAGAAAACATGAGGATCACCCATGTCTG
CAGGTCGACTCTAGAAAACATGAGGATCACCCATGT (SEQ ID NO:173)
Protein: (X indicates non-canonical amino acid)

MGRLESTPPKKKRKVEDSASDYKDDDDKVSKGEELFTGVVPILVELDGDVNGHKFSVSGE
GEGDATXGKLTLKFICTTGKLPVPWPTLVTTLTYGVQCFSRYPDHMKQHDFFKSAMPEGYV
QERTIFFKDDGNYKTRAEVKFEGDTLVNRIELKGIDFKEDGNILGHKLEYNYNSHNVYIMADK
QKNGIKANFKIRHNIEDGSVQLADHYQQNTPIGDGPVLLPDNHYLSTQSALSKDPNEKRDHM
VLLEFVTAAGITLGMDELYKHHHHHH (SEQ ID NO:174)

mOrange
DNA: 
ATGGTGAGCAAGGGCGAGGAGAATAATATGGCCATCATCAAGGAGTTCATGCGCTTCAA
GGTGCGCATGGAGGGCACCGTGAACGGCCACGAGTTCGAGATCGAGGGCGAGGGCG
AGGGCCGCCCCTACGAGGGCTTTCAGACCGCTAAGCTGAAGGTGACCAAGGGCGGCC
CCCTGCCCTTCGCCTGGGACATCCTGTCCCCTCTCTTCACCTACGGCTCCAAGGCCTAC
GTGAAGCACCCCGCCGACATCCCCGACTACTTCAAGCTGTCCTTCCCCGAGGGCTTCA
AGTGGGAGCGCGTGATGAACTACGAGGACGGCGGCGTGGTGACCGTGACCCAGGACT
CCTCACTGCAGGACGGCGAGTTCATCTACAAGGTGAAGATGCGCGGCACCAACTTCCC
CTCCGACGGCCCCGTGATGCAGAAGAAGACCATGGGCTGGGAGGCCTCCTCCGAGCG
GATGTACCCCGAGGACGGCGCCCTGAAGGGCGAGATCAGGATGAGGCTGAAGCTGAA
GGACGGCGGCCACTACACCTCCGAGGTCAAGACCACCTACAAGGCCAAGAAGTCCGTG
CAGCTGCCCGGCGCCTACATCGTCGGCATCAAGCTGGACATCACCTCCCACAACGAGG
ACTACACCATCGTGGAACAGTACGAACGCGCCGAGGGCCGCCACTCCACCGGCGGCA
TGGACGAGCTGTACAAGTAA (SEQ ID NO:175)
Protein:

MVSKGEENNMAIIKEFMRFKVRMEGTVNGHEFEIEGEGEGRPYEGFQTAKLKVTKGGPLPF
AWDILSPLFTYGSKAYVKHPADIPDYFKLSFPEGFKWERVMNYEDGGVVTVTQDSSLQDGE
FIYKVKMRGTNFPSDGPVMQKKTMGWEASSERMYPEDGALKGEIRMRLKLKDGGHYTSEV
KTTYKAKKSVQLPGAYIVGIKLDITSHNEDYTIVEQYERAEGRHSTGGMDELYK (SEQ ID
NO:176)

iRFP (near-infrared fluorescent protein)
DNA: 
GAAGGATCCGTCGCCAGGCAGCCTGACCTCTTGACCTGCGACGATGAGCCGATCCATA
TCCCCGGTGCCATCCAACCGCATGGACTGCTGCTCGCCCTCGCCGCCGACATGACGAT
CGTTGCCGGCAGCGACAACCTTCCCGAACTCACCGGACTGGCGATCGGCGCCCTGATC
 
GGCCGCTCTGCGGCCGATGTCTTCGACTCGGAGACGCACAACCGTCTGACGATCGCCT
TGGCCGAGCCCGGGGCGGCCGTCGGAGCACCGATCACTGTCGGCTTCACGATGCGAA
AGGACGCAGGCTTCATCGGCTCCTGGCATCGCCATGATCAGCTCATCTTCCTCGAGCT
CGAGCCTCCCCAGCGGGACGTCGCCGAGCCGCAGGCGTTCTTCCGCCGCACCAACAG
CGCCATCCGCCGCCTGCAGGCCGCCGAAACCTTGGAAAGCGCCTGCGCCGCCGCGGC
GCAAGAGGTGCGGAAGATTACCGGCTTCGATCGGGTGATGATCTATCGCTTCGCCTCC
GACTTCAGCGGCGAAGTGATCGCAGAGGATCGGTGCGCCGAGGTCGAGTCAAAACTAG
GCCTGCACTATCCTGCCTCAACCGTGCCGGCGCAGGCCCGTCGGCTCTATACCATCAA
CCCGGTACGGATCATTCCCGATATCAATTATCGGCCGGTGCCGGTCACCCCAGACCTC
AATCCGGTCACCGGGCGGCCGATTGATCTTAGCTTCGCCATCCTGCGCAGCGTCTCGC
CCGTCCATCTGGAATTCATGCGCAACATAGGCATGCACGGCACGATGTCGATCTCGATT
TTGCGCGGCGAGCGACTGTGGGGATTGATCGTTTGCCATCACCGAACGCCGTACTACG
TCGATCTCGATGGCCGCCAAGCCTGCGAGCTAGTCGCCCAGGTTCTGGCCTGGCAGAT
CGGCGTGATGGAAGAG (SEQ ID NO:177)
Protein:

EGSVARQPDLLTCDDEPIHIPGAIQPHGLLLALAADMTIVAGSDNLPELTGLAIGALIGRSAAD
VFDSETHNRLTIALAEPGAAVGAPITVGFTMRKDAGFIGSWHRHDQLIFLELEPPQRDVAEP
QAFFRRTNSAIRRLQAAETLESACAAAAQEVRKITGFDRVMIYRFASDFSGEVIAEDRCAEV
ESKLGLHYPASTVPAQARRLYTINPVRIIPDINYRPVPVTPDLNPVTGRPIDLSFAILRSVSPVH
LEFMRNIGMHGTMSISILRGERLWGLIVCHHRTPYYVDLDGRQACELVAQVLAWQIGVMEE
(SEQ ID NO:178)

mCherry^{185TAG} (mCherry with Amber-encoded amino acid position 185)
DNA: (Amber codon underlined) 
ATGGGCCGCCTGGAAAGCACCCCGCCGAAAAAAAAACGCAAAGTGGAAGATAGCGCGA
GCGTGAGCAAGGGCGAGGAGGATAACATGGCCATCATCAAGGAGTTCATGCGCTTCAA
GGTGCACATGGAGGGCTCCGTGAACGGCCACGAGTTCGAGATCGAGGGCGAGGGCGA
GGGCCGCCCCTACGAGGGCACCCAGACCGCCAAGCTGAAGGTGACCAAGGGTGGCCC
CCTGCCCTTCGCCTGGGACATCCTGTCCCCTCAGTTCATGTACGGCTCCAAGGCCTAC
GTGAAGCACCCCGCCGACATCCCCGACTACTTGAAGCTGTCCTTCCCCGAGGGCTTCA
AGTGGGAGCGCGTGATGAACTTCGAGGACGGCGGCGTGGTGACCGTGACCCAGGACT
CCTCCCTGCAGGACGGCGAGTTCATCTACAAGGTGAAGCTGCGCGGCACCAACTTCCC
CTCCGACGGCCCCGTAATGCAGAAGAAGACGATGGGCTGGGAGGCCTCCTCCGAGCG
GATGTACCCCGAGGACGGCGCCCTGAAGGGCGAGATCAAGCAGAGGCTGAAGCTGAA
GGACGGCGGCCACTACGACGCTGAGGTCAAGACCACCTACAAGGCCAAGTAGCCCGT
GCAGCTGCCCGGCGCCTACAACGTCAACATCAAGTTGGACATCACCTCCCACAACGAG
 
GACTACACCATCGTGGAACAGTACGAACGCGCCGAGGGCCGCCACTCCACCGGCGGC
ATGGACGAGCTGTACAAGCATCATCATCATCATCATTAA (SEQ ID NO:179)
Protein: (X non-canonical amino acid)

MGRLESTPPKKKRKVEDSASVSKGEEDNMAIIKEFMRFKVHMEGSVNGHEFEIEGEGEGRP
YEGTQTAKLKVTKGGPLPFAWDILSPQFMYGSKAYVKHPADIPDYLKLSFPEGFKWERVMN
FEDGGVVTVTQDSSLQDGEFIYKVKLRGTNFPSDGPVMQKKTMGWEASSERMYPEDGALK
GEIKQRLKLKDGGHYDAEVKTTYKAKXPVQLPGAYNVNIKLDITSHNEDYTIVEQYERAEGR
HSTGGMDELYKHHHHHH (SEQ ID NO:180)

mCherry^{185TAG}-2xMS2(mCherry^{185TAG} with 2 MS2 RNA stem-loops)
DNA: (MS2 stem-loops and Amber codon underlined) 
ATGGGCCGCCTGGAAAGCACCCCGCCGAAAAAAAAACGCAAAGTGGAAGATAGCGCGA
GCGTGAGCAAGGGCGAGGAGGATAACATGGCCATCATCAAGGAGTTCATGCGCTTCAA
GGTGCACATGGAGGGCTCCGTGAACGGCCACGAGTTCGAGATCGAGGGCGAGGGCGA
GGGCCGCCCCTACGAGGGCACCCAGACCGCCAAGCTGAAGGTGACCAAGGGTGGCCC
CCTGCCCTTCGCCTGGGACATCCTGTCCCCTCAGTTCATGTACGGCTCCAAGGCCTAC
GTGAAGCACCCCGCCGACATCCCCGACTACTTGAAGCTGTCCTTCCCCGAGGGCTTCA
AGTGGGAGCGCGTGATGAACTTCGAGGACGGCGGCGTGGTGACCGTGACCCAGGACT
CCTCCCTGCAGGACGGCGAGTTCATCTACAAGGTGAAGCTGCGCGGCACCAACTTCCC
CTCCGACGGCCCCGTAATGCAGAAGAAGACGATGGGCTGGGAGGCCTCCTCCGAGCG
GATGTACCCCGAGGACGGCGCCCTGAAGGGCGAGATCAAGCAGAGGCTGAAGCTGAA
GGACGGCGGCCACTACGACGCTGAGGTCAAGACCACCTACAAGGCCAAGTAGCCCGT
GCAGCTGCCCGGCGCCTACAACGTCAACATCAAGTTGGACATCACCTCCCACAACGAG
GACTACACCATCGTGGAACAGTACGAACGCGCCGAGGGCCGCCACTCCACCGGCGGC
ATGGACGAGCTGTACAAGCATCATCATCATCATCATTAAAGATCCTAAGGTACCTAATTG
CCTAGAAAACATGAGGATCACCCATGTCTGCAGGTCGACTCTAGAAAACATGAGGATCA
CCCATGT (SEQ ID NO:181)
Protein: (X indicates non-canonical amino acid)

MGRLESTPPKKKRKVEDSASVSKGEEDNMAIIKEFMRFKVHMEGSVNGHEFEIEGEGEGRP
YEGTQTAKLKVTKGGPLPFAWDILSPQFMYGSKAYVKHPADIPDYLKLSFPEGFKWERVMN
FEDGGVVTVTQDSSLQDGEFIYKVKLRGTNFPSDGPVMQKKTMGWEASSERMYPEDGALK
GEIKQRLKLKDGGHYDAEVKTTYKAKXPVQLPGAYNVNIKLDITSHNEDYTIVEQYERAEGR
HSTGGMDELYKHHHHHH (SEQ ID NO:182)

mCherry^{185TAG}-4xBoxB (mCherry^{185TAG} with 4 BoxB-loops)
DNA: (BoxB-stem loops and Amber codon underlined) 
ATGGGCCGCCTGGAAAGCACCCCGCCGAAAAAAAAACGCAAAGTGGAAGATAGCGCGA
GCGTGAGCAAGGGCGAGGAGGATAACATGGCCATCATCAAGGAGTTCATGCGCTTCAA
GGTGCACATGGAGGGCTCCGTGAACGGCCACGAGTTCGAGATCGAGGGCGAGGGCGA
GGGCCGCCCCTACGAGGGCACCCAGACCGCCAAGCTGAAGGTGACCAAGGGTGGCCC
CCTGCCCTTCGCCTGGGACATCCTGTCCCCTCAGTTCATGTACGGCTCCAAGGCCTAC
GTGAAGCACCCCGCCGACATCCCCGACTACTTGAAGCTGTCCTTCCCCGAGGGCTTCA
AGTGGGAGCGCGTGATGAACTTCGAGGACGGCGGCGTGGTGACCGTGACCCAGGACT
CCTCCCTGCAGGACGGCGAGTTCATCTACAAGGTGAAGCTGCGCGGCACCAACTTCCC
CTCCGACGGCCCCGTAATGCAGAAGAAGACGATGGGCTGGGAGGCCTCCTCCGAGCG
GATGTACCCCGAGGACGGCGCCCTGAAGGGCGAGATCAAGCAGAGGCTGAAGCTGAA
GGACGGCGGCCACTACGACGCTGAGGTCAAGACCACCTACAAGGCCAAGTAGCCCGT
GCAGCTGCCCGGCGCCTACAACGTCAACATCAAGTTGGACATCACCTCCCACAACGAG
GACTACACCATCGTGGAACAGTACGAACGCGCCGAGGGCCGCCACTCCACCGGCGGC
ATGGACGAGCTGTACAAGCATCATCATCATCATCATTAAAGATCCTAAGGTACCGCCCT
GAAAAGGGCTCGAGCCCTGAAAAGGGCAATTGCCCTGAAAAGGGCGTCGACGCCC
TGAAAAGGGC (SEQ ID NO:183)
Protein: (X indicates non-canonical amino acid)

MGRLESTPPKKKRKVEDSASVSKGEEDNMAIIKEFMRFKVHMEGSVNGHEFEIEGEGEGRP
YEGTQTAKLKVTKGGPLPFAWDILSPQFMYGSKAYVKHPADIPDYLKLSFPEGFKWERVMN
FEDGGVVTVTQDSSLQDGEFIYKVKLRGTNFPSDGPVMQKKTMGWEASSERMYPEDGALK
GEIKQRLKLKDGGHYDAEVKTTYKAKXPVQLPGAYNVNIKLDITSHNEDYTIVEQYERAEGR
HSTGGMDELYKHHHHHH (SEQ ID NO:184)

GFP^{39TAA}-2xMS2 (GFP with Ochre-encoded amino acid position 39 and 2 MS2 RNA stem-loops)
DNA: (MS2 stem-loops and Ochre codon underlined) 
ATGGGCCGCCTGGAAAGCACCCCGCCGAAAAAAAAACGCAAAGTGGAAGATAGCGCGA
GCGATTACAAAGATGATGATGATAAAGTGAGCAAGGGCGAGGAGCTGTTCACCGGGGT
GGTGCCCATCCTGGTCGAGCTGGACGGCGACGTAAACGGCCACAAGTTCAGCGTGTCC
GGCGAGGGCGAGGGCGATGCCACCTAAGGCAAGCTGACCCTGAAGTTCATCTGCACC
ACCGGCAAGCTGCCCGTGCCCTGGCCCACCCTCGTGACCACCCTGACCTACGGCGTG
CAGTGCTTCAGCCGCTACCCCGACCACATGAAGCAGCACGACTTCTTCAAGTCCGCCAT
GCCCGAAGGCTACGTCCAGGAGCGCACCATCTTCTTCAAGGACGACGGCAACTACAAG
ACCCGCGCCGAGGTGAAGTTCGAGGGCGACACCCTGGTGAACCGCATCGAGCTGAAG
GGCATCGACTTCAAGGAGGACGGCAACATCCTGGGGCACAAGCTGGAGTACAACTACA
ACAGCCACAACGTCTATATCATGGCCGACAAGCAGAAGAACGGCATCAAGGCCAACTTC
 
AAGATCCGCCACAACATCGAGGACGGCAGCGTGCAGCTCGCCGACCACTACCAGCAGA
ACACCCCCATCGGCGACGGCCCCGTGCTGCTGCCCGACAACCACTACCTGAGCACCCA
GTCCGCCCTGAGCAAAGACCCCAACGAGAAGCGCGATCACATGGTCCTGCTGGAGTTC
GTGACCGCCGCCGGGATCACTCTCGGCATGGACGAGCTGTACAAGCATCACCATCACC
ATCACTGAGGATCCTAAGGTACCTAATTGCCTAGAAAACATGAGGATCACCCATGTCTG
CAGGTCGACTCTAGAAAACATGAGGATCACCCATGT (SEQ ID NO:185)
Protein: (X indicates non-canonical amino acid)

MGRLESTPPKKKRKVEDSASDYKDDDDKVSKGEELFTGVVPILVELDGDVNGHKFSVSGE
GEGDATXGKLTLKFICTTGKLPVPWPTLVTTLTYGVQCFSRYPDHMKQHDFFKSAMPEGYV
QERTIFFKDDGNYKTRAEVKFEGDTLVNRIELKGIDFKEDGNILGHKLEYNYNSHNVYIMADK
QKNGIKANFKIRHNIEDGSVQLADHYQQNTPIGDGPVLLPDNHYLSTQSALSKDPNEKRDHM
VLLEFVTAAGITLGMDELYKHHHHHH (SEQ ID NO:186)

mCherry^{185TAA}-2xMS2 (mCherry with Ochre-encoded amino acid position 185 and 2 MS2 RNA stem-loops)
DNA: (MS2 stem-loops and Ochre codon underlined) 
ATGGGCCGCCTGGAAAGCACCCCGCCGAAAAAAAAACGCAAAGTGGAAGATAGCGCGA
GCGTGAGCAAGGGCGAGGAGGATAACATGGCCATCATCAAGGAGTTCATGCGCTTCAA
GGTGCACATGGAGGGCTCCGTGAACGGCCACGAGTTCGAGATCGAGGGCGAGGGCGA
GGGCCGCCCCTACGAGGGCACCCAGACCGCCAAGCTGAAGGTGACCAAGGGTGGCCC
CCTGCCCTTCGCCTGGGACATCCTGTCCCCTCAGTTCATGTACGGCTCCAAGGCCTAC
GTGAAGCACCCCGCCGACATCCCCGACTACTTGAAGCTGTCCTTCCCCGAGGGCTTCA
AGTGGGAGCGCGTGATGAACTTCGAGGACGGCGGCGTGGTGACCGTGACCCAGGACT
CCTCCCTGCAGGACGGCGAGTTCATCTACAAGGTGAAGCTGCGCGGCACCAACTTCCC
CTCCGACGGCCCCGTAATGCAGAAGAAGACGATGGGCTGGGAGGCCTCCTCCGAGCG
GATGTACCCCGAGGACGGCGCCCTGAAGGGCGAGATCAAGCAGAGGCTGAAGCTGAA
GGACGGCGGCCACTACGACGCTGAGGTCAAGACCACCTACAAGGCCAAGTAACCCGT
GCAGCTGCCCGGCGCCTACAACGTCAACATCAAGTTGGACATCACCTCCCACAACGAG
GACTACACCATCGTGGAACAGTACGAACGCGCCGAGGGCCGCCACTCCACCGGCGGC
ATGGACGAGCTGTACAAGCATCATCATCATCATCATTGAAGATCCTAAGGTACCTAATTG
CCTAGAAAACATGAGGATCACCCATGTCTGCAGGTCGACTCTAGAAAACATGAGGATCA
CCCATGT (SEQ ID NO:187)
Protein: (X indicates non-canonical amino acid)

MGRLESTPPKKKRKVEDSASVSKGEEDNMAIIKEFMRFKVHMEGSVNGHEFEIEGEGEGRP
YEGTQTAKLKVTKGGPLPFAWDILSPQFMYGSKAYVKHPADIPDYLKLSFPEGFKWERVMN
FEDGGVVTVTQDSSLQDGEFIYKVKLRGTNFPSDGPVMQKKTMGWEASSERMYPEDGALK

GEIKQRLKLKDGGHYDAEVKTTYKAKXPVQLPGAYNVNIKLDITSHNEDYTIVEQYERAEGR
HSTGGMDELYKHHHHHH (SEQ ID NO:188)

GFP^{39TGA}-2xMS2 (GFP with Opal-encoded amino acid position 39 and 2 MS2 RNA stem-loops)
DNA: (MS2 stem-loops and Opal codon underlined) 
ATGGGCCGCCTGGAAAGCACCCCGCCGAAAAAAAAACGCAAAGTGGAAGATAGCGCGA
GCGATTACAAAGATGATGATGATAAAGTGAGCAAGGGCGAGGAGCTGTTCACCGGGGT
GGTGCCCATCCTGGTCGAGCTGGACGGCGACGTAAACGGCCACAAGTTCAGCGTGTCC
GGCGAGGGCGAGGGCGATGCCACCTGAGGCAAGCTGACCCTGAAGTTCATCTGCACC
ACCGGCAAGCTGCCCGTGCCCTGGCCCACCCTCGTGACCACCCTGACCTACGGCGTG
CAGTGCTTCAGCCGCTACCCCGACCACATGAAGCAGCACGACTTCTTCAAGTCCGCCAT
GCCCGAAGGCTACGTCCAGGAGCGCACCATCTTCTTCAAGGACGACGGCAACTACAAG
ACCCGCGCCGAGGTGAAGTTCGAGGGCGACACCCTGGTGAACCGCATCGAGCTGAAG
GGCATCGACTTCAAGGAGGACGGCAACATCCTGGGGCACAAGCTGGAGTACAACTACA
ACAGCCACAACGTCTATATCATGGCCGACAAGCAGAAGAACGGCATCAAGGCCAACTTC
AAGATCCGCCACAACATCGAGGACGGCAGCGTGCAGCTCGCCGACCACTACCAGCAGA
ACACCCCCATCGGCGACGGCCCCGTGCTGCTGCCCGACAACCACTACCTGAGCACCCA
GTCCGCCCTGAGCAAAGACCCCAACGAGAAGCGCGATCACATGGTCCTGCTGGAGTTC
GTGACCGCCGCCGGGATCACTCTCGGCATGGACGAGCTGTACAAGCATCACCATCACC
ATCACTAAGGATCCTAAGGTACCTAATTGCCTAGAAAACATGAGGATCACCCATGTCTG
CAGGTCGACTCTAGAAAACATGAGGATCACCCATGT (SEQ ID NO:189)
Protein: (X indicates non-canonical amino acid)

MGRLESTPPKKKRKVEDSASDYKDDDDKVSKGEELFTGVVPILVELDGDVNGHKFSVSGE
GEGDATXGKLTLKFICTTGKLPVPWPTLVTTLTYGVQCFSRYPDHMKQHDFFKSAMPEGYV
QERTIFFKDDGNYKTRAEVKFEGDTLVNRIELKGIDFKEDGNILGHKLEYNYNSHNVYIMADK
QKNGIKANFKIRHNIEDGSVQLADHYQQNTPIGDGPVLLPDNHYLSTQSALSKDPNEKRDHM
VLLEFVTAAGITLGMDELYKHHHHHH (SEQ ID NO:190)

mCherry^{185TGA}-2xMS2 (mCherry with Opal-encoded amino acid position 185 and 2 MS2 RNA stem-loops)
DNA: (MS2 stem-loops and Opal codon underlined) 
ATGGGCCGCCTGGAAAGCACCCCGCCGAAAAAAAAACGCAAAGTGGAAGATAGCGCGA
GCGTGAGCAAGGGCGAGGAGGATAACATGGCCATCATCAAGGAGTTCATGCGCTTCAA
GGTGCACATGGAGGGCTCCGTGAACGGCCACGAGTTCGAGATCGAGGGCGAGGGCGA
GGGCCGCCCCTACGAGGGCACCCAGACCGCCAAGCTGAAGGTGACCAAGGGTGGCCC
 
CCTGCCCTTCGCCTGGGACATCCTGTCCCCTCAGTTCATGTACGGCTCCAAGGCCTAC
GTGAAGCACCCCGCCGACATCCCCGACTACTTGAAGCTGTCCTTCCCCGAGGGCTTCA
AGTGGGAGCGCGTGATGAACTTCGAGGACGGCGGCGTGGTGACCGTGACCCAGGACT
CCTCCCTGCAGGACGGCGAGTTCATCTACAAGGTGAAGCTGCGCGGCACCAACTTCCC
CTCCGACGGCCCCGTAATGCAGAAGAAGACGATGGGCTGGGAGGCCTCCTCCGAGCG
GATGTACCCCGAGGACGGCGCCCTGAAGGGCGAGATCAAGCAGAGGCTGAAGCTGAA
GGACGGCGGCCACTACGACGCTGAGGTCAAGACCACCTACAAGGCCAAGTGACCCGT
GCAGCTGCCCGGCGCCTACAACGTCAACATCAAGTTGGACATCACCTCCCACAACGAG
GACTACACCATCGTGGAACAGTACGAACGCGCCGAGGGCCGCCACTCCACCGGCGGC
ATGGACGAGCTGTACAAGCATCATCATCATCATCATTAAAGATCCTAAGGTACCTAATTG
CCTAGAAAACATGAGGATCACCCATGTCTGCAGGTCGACTCTAGAAAACATGAGGATCA
CCCATGT (SEQ ID NO:191)
Protein: (X indicates non-canonical amino acid)

MGRLESTPPKKKRKVEDSASVSKGEEDNMAIIKEFMRFKVHMEGSVNGHEFEIEGEGEGRP
YEGTQTAKLKVTKGGPLPFAWDILSPQFMYGSKAYVKHPADIPDYLKLSFPEGFKWERVMN
FEDGGVVTVTQDSSLQDGEFIYKVKLRGTNFPSDGPVMQKKTMGWEASSERMYPEDGALK
GEIKQRLKLKDGGHYDAEVKTTYKAKXPVQLPGAYNVNIKLDITSHNEDYTIVEQYERAEGR
HSTGGMDELYKHHHHHH (SEQ ID NO:192)

Nup153 (*Homo sapiens* Nucleoporin 153; Uniprot: P49790)
DNA: 
ATGGCGTCTGGTGCTGGCGGTGTTGGTGGAGGAGGTGGGGGTAAAATTCGTACTCGTC
GCTGTCATCAAGGTCCGATTAAACCGTATCAGCAGGGACGTCAGCAACATCAGGGTATT
CTGAGCCGTGTGACCGAAAGCGTGAAAAACATTGTGCCGGGTTGGCTGCAACGTTATTT
CAACAAAAATGAGGATGTGTGTTCGTGTTCTACCGATACCAGTGAAGTTCCTCGTTGGC
CGGAAAACAAAGAAGATCACCTGGTGTATGCCGATGAAGAATCGAGCAATATCACCGAT
GGCCGTATTACTCCTGAACCGGCGGTTAGTAACACTGAAGAACCGTCAACCACAAGCAC
AGCATCGAACTATCCAGATGTCCTGACTCGCCCTTCTCTGCACCGTTCTCACCTGAACT
TTAGCATGCTGGAATCACCAGCTCTGCATTGTCAGCCGTCTACCAGTAGTGCCTTCCCG
ATTGGCTCTAGTGGCTTTTCGCTGGTCAAAGAGATCAAAGACTCGACCTCTCAACATGA
CGATGATAACATTAGCACGACCTCGGGTTTTAGTAGCCGTGCCTCCGATAAAGACATTA
CCGTGAGCAAAAACACCTCTCTGCCGCCTCTGTGGAGTCCTGAAGCCGAACGCTCTCA
TAGTCTGTCTCAGCACACAGCCACCAGTTCCAAAAAACCAGCCTTCAACCTGAGCGCCT
TTGGTACACTGTCACCGAGCCTGGGAAATTCCTCTATCCTGAAAACATCACAGCTGGGC
GATAGTCCGTTTTATCCGGGCAAAACGACGTATGGTGGTGCCGCTGCTGCTGTTCGCC
AGTCTAAACTGCGTAACACTCCGTATCAAGCTCCAGTCCGTCGCCAAATGAAAGCAAAA
 
CAACTGTCGGCCCAGTCTTATGGTGTGACAAGCTCTACAGCTCGTCGTATCCTGCAAAG
TCTGGAGAAAATGTCATCTCCGCTGGCAGATGCCAAACGTATTCCGTCCATTGTGAGCA
GTCCGCTGAATAGCCCGCTGGACCGTAGTGGGATCGATATCACCGACTTCCAAGCCAA
ACGTGAGAAAGTGGATAGCCAGTATCCGCCTGTACAACGTCTGATGACCCCGAAACCG
GTTTCAATTGCCACGAATCGTAGCGTGTATTTCAAACCGTCACTGACCCCTAGTGGTGA
GTTTCGTAAAACAAATCAGCGTATCGACAACAAATGCTCTACCGGGTATGAAAAAACAT
GACGCCGGGACAGAATCGTGAACAACGTGAATCTGGCTTCTCTTATCCGAACTTTAGTC
TGCCGGCAGCAAATGGTCTGAGTAGCGGTGTAGGAGGTGGTGGGGGCAAAATGCGCC
GTGAACGTCACGCCTTTGTGGCCTCTAAACCTCTGGAAGAAGAGATGGAGGTTCCT
GTACTGCCGAAAATCAGTCTGCCTATCACCTCTTCAAGTCTGCCGACCTTCAACTTTTCT
AGTCCGGAAATCACAACCTCTAGCCCGTCACCGATTAATAGCAGTCAAGCACTGACGAA
TAAAGTCCAAATGACCTCACCGAGTTCTACGGGTTCTCCGATGTTCAAATTCTCTAGTCC
TATCGTGAAATCAACCGAAGCGAACGTCCTGCCTCCTTCTAGTATTGGGTTCACCTTTAG
CGTCCCAGTGGCCAAAACAGCTGAACTGAGCGGTAGCAGTAGTACTCTGGAACCGATT
ATCAGCTCAAGCGCCCATCATGTCACTACCGTGAATAGCACAAACTGTAAAAAACGCC
GCCTGAGGACTGTGAAGGACCGTTTCGTCCTGCCGAAATCCTGAAAGAAGGTTCCGTC
CTGGACATTCTGAAATCTCCGGGATTTGCCTCTCCTAAAATCGACTCTGTTGCCGCTCAA
CCAACTGCCACATCACCGGTGGTTTATACTCGTCCGGCGATTAGCAGTTTTAGCAGTAG
TGGCATCGGTTTTGGTGAATCCCTGAAAGCTGGCTCATCTTGGCAGTGTGACACCTGCC
TGCTGCAAAACAAAGTGACCGATAACAAATGTATTGCCTGTCAGGCCGCCAAACTGTCT
CCTCGTGATACAGCCAAACAGACCGGCATCGAAACCCCTAATAAAGCGGGAAAACGA
CCCTGTCAGCAAGTGGTACGGGATTTGGGGACAAATTCAAACCTGTGATCGGCACATG
GGACTGTGACACTTGTCTGGTACAGAACAAACCAGAAGCGATCAAATGTGTGGCCTGTG
AAACGCCTAAACCTGGAACATGTGTGAAACGTGCCCTGACTCTGACTGTTGTGTCAGAA
AGCGCCGAAACCATGACGGCAAGCAGCTCATCCTGTACTGTGACTACCGGGACTCTGG
GATTTGGTGACAAATTCAAACGCCCGATTGGTTCCTGGGAATGCTCCGTGTGTTGTGTG
AGCAATAATGCCGAGGACAACAAATGTGTGTCCTGTATGAGCGAGAAACCTGGCAGCTC
TGTTCCTGCTAGCAGCTCTAGCACAGTTCCTGTTAGTCTGCCTAGTGGTGGTTCTCTGG
GTCTGGAAAAATTCAAAAAACCTGAAGGAAGCTGGGATTGTGAGCTGTGCCTGGTACAG
AATAAAGCGGATAGCACGAAATGTCTGGCCTGTGAGTCAGCCAAACCAGGGACTAAAA
GCGGCTTTAAAGGCTTCGACACGTCGAGCAGTTCTAGTAACAGCGCCGCCTCATCATCT
TTCAAATTTGGGGTGAGCAGCTCCTCTAGTGGTCCTAGTCAAACACTGACCTCTACCGG
AAACTTCAAATTCGGCGATCAGGGTGGCTTCAAAATTGGTGTCTCCTCTGATTCGGGTA
GCATTAACCCGATGAGTGAGGGGTTCAAATTCAGCAAACCAATTGGCGATTTCAAATTC
GGTGTGTCGTCTGAATCCAAACCTGAAGAAGTCAAAAAAGACAGCAAAAACGACAATTT
CAAATTCGGCCTGTCTAGTGGTCTGTCTAATCCGGTTAGCCTGACCCCGTTTCAGTTCG
 
GGGTGTCTAATCTGGGTCAGGAAGAGAAAAAGAGGAGCTGCCTAAAAGTTCATCTGCC
GGGTTCAGTTTTGGTACAGGCGTGATCAATAGCACTCCAGCACCAGCCAATACAATCGT
GACGAGCGAGAACAAATCGAGCTTCAACCTGGGGACAATCGAAACGAAAAGCGCCAGT
GTAGCGCCATTCACGTGTAAAACCTCCGAGGCAAAAAAGAAGAGATGCCGGCCACAA
AAGGTGGATTCTCATTCGGCAACGTGGAACCGGCTAGCCTGCCATCAGCAAGCGTGTT
TGTACTGGGCCGTACCGAGGAGAAACAGCAGGAACCTGTTACTAGCACCAGTCTGGTC
TTTGGTAAAAAGCCGACAACGAAGAACCGAAATGTCAGCCAGTGTTCAGCTTCGGCAA
TAGCGAACAGACGAAAGACGAAAACAGCAGCAAATCGACGTTCAGCTTCAGTATGACGA
AACCGAGCGAAAAAGAAAGTGAGCAGCCAGCAAAAGCAACGTTCGCCTTTGGAGCACA
GACATCAACCACAGCCGATCAAGGAGCAGCGAAACCAGTTTTCAGTTTTCTGAATAACA
GCTCAAGCAGCAGTTCTACACCAGCAACCTCAGCAGGTGGTGGGATCTTTGGATCAAG
CACCTCATCCAGCAATCCGCCAGTGGCAACATTCGTGTTTGGCCAGAGCAGTAATCCG
GTGTCATCTTCAGCATTTGGGAATACCGCCGAGAGTAGCACATCACAGTCTCTGCTGTT
CTCACAGGACTCTAAACTGGCAACCACCTCTTCTACTGGTACAGCGGTTACCCCGTTTG
TGTTCGGTCCGGGAGCATCATCCAATAATACCACGACGTCGGGCTTTGGGTTTGGTGC
CACGACAACAAGCAGTAGCGCTGGTAGCAGCTTTGTCTTTGGCACAGGTCCTTCAGCAC
CTTCTGCTTCACCAGCTTTCGGAGCCAATCAGACTCCGACATTCGGACAGTCACAGGGT
GCCTCTCAACCAAATCCTCCGGGTTTTGGCAGTATTAGCAGTAGTACCGCCCTGTTCCC
GACCGGTAGTCAACCGGCACCGCCAACATTTGGAACGGTTAGCAGTAGTAGTCAGCCT
CCGGTGTTTGGACAACAACCGAGCCAGAGCGCCTTCGGATCAGGAACGACCCCTAATA
GTAGCAGTGCCTTCCAGTTCGGTAGCAGTACCACCAACTTCAACTTCACGAACAATAGC
CCGTCAGGTGTGTTCACGTTTGGCGCCAATTCTTCTACCCCAGCGGCAAGTGCTCAACC
TTCAGGCTCAGGTGGATTTCCTTTCAACCAGTCACCAGCAGCGTTTACTGTTGGTTCTAA
CGGGAAAAACGTTTTCAGTAGCAGCGGCACCTCGTTTTCTGGTCGTAAAATCAAAACGG
CCGTTCGTCGCCGTAAA (SEQ ID NO:193)
Protein:

MASGAGGVGGGGGGKIRTRRCHQGPIKPYQQGRQQHQGILSRVTESVKNIVPGWLQRYF
NKNEDVCSCSTDTSEVPRWPENKEDHLVYADEESSNITDGRITPEPAVSNTEEPSTTSTAS
NYPDVLTRPSLHRSHLNFSMLESPALHCQPSTSSAFPIGSSGFSLVKEIKDSTSQHDDDNIS
TTSGFSSRASDKDITVSKNTSLPPLWSPEAERSHSLSQHTATSSKKPAFNLSAFGTLSPSLG
NSSILKTSQLGDSPFYPGKTTYGGAAAAVRQSKLRNTPYQAPVRRQMKAKQLSAQSYGVT
SSTARRILQSLEKMSSPLADAKRIPSIVSSPLNSPLDRSGIDITDFQAKREKVDSQYPPVQRL
MTPKPVSIATNRSVYFKPSLTPSGEFRKTNQRIDNKCSTGYEKNMTPGQNREQRESGFSYP
NFSLPAANGLSSGVGGGGGKMRRERHAFVASKPLEEEEMEVPVLPKISLPITSSSLPTFNFS
SPEITTSSPSPINSSQALTNKVQMTSPSSTGSPMFKFSSPIVKSTEANVLPPSSIGFTFSVPV
AKTAELSGSSSTLEPIISSSAHHVTTVNSTNCKKTPPEDCEGPFRPAEILKEGSVLDILKSPGF

ASPKIDSVAAQPTATSPVVYTRPAISSFSSSGIGFGESLKAGSSWQCDTCLLQNKVTDNKCI
ACQAAKLSPRDTAKQTGIETPNKSGKTTLSASGTGFGDKFKPVIGTWDCDTCLVQNKPEAIK
CVACETPKPGTCVKRALTLTVVSESAETMTASSSSCTVTTGTLGFGDKFKRPIGSWECSVC
CVSNNAEDNKCVSCMSEKPGSSVPASSSSTVPVSLPSGGSLGLEKFKKPEGSWDCELCLV
QNKADSTKCLACESAKPGTKSGFKGFDTSSSSSNSAASSSFKFGVSSSSSGPSQTLTSTGN
FKFGDQGGFKIGVSSDSGSINPMSEGFKFSKPIGDFKFGVSSESKPEEVKKDSKNDNFKFG
LSSGLSNPVSLTPFQFGVSNLGQEEKKEELPKSSSAGFSFGTGVINSTPAPANTIVTSENKS
SFNLGTIETKSASVAPFTCKTSEAKKEEMPATKGGFSFGNVEPASLPSASVFVLGRTEEKQQ
EPVTSTSLVFGKKADNEEPKCQPVFSFGNSEQTKDENSSKSTFSFSMTKPSEKESEQPAKA
TFAFGAQTSTTADQGAAKPVFSFLNNSSSSSSTPATSAGGGIFGSSTSSSNPPVATFVFGQ
SSNPVSSSSAFGNTAESSTSQSLLFSQDSKLATTSSTGTAVTPFVFGPGASSNNTTTSGFGF
GATTTSSSAGSSFVFGTGPSAPSASPAFGANQTPTFGQSQGASQPNPPGFGSISSSTALFP
TGSQPAPPTFGTVSSSSQPPVFGQQPSQSAFGSGTTPNSSSAFQFGSSTTNFNFTNNSPS
GVFTFGANSSTPAASAQPSGSGGFPFNQSPAAFTVGSNGKNVFSSSGTSFSGRKIKTAVRR
RK (SEQ ID NO:194)

Vim^{116TAG} (*Homo sapiens* Vimentin with Amber-encoded amino acid position 116; Uniprot: P08670)
DNA: (Amber codon underlined) 
ATGTCCACCAGGTCCGTGTCCTCGTCCTCCTACCGCAGGATGTTCGGCGGCCCGGGCA
CCGCGAGCCGGCCGAGCTCCAGCCGGAGCTACGTGACTACGTCCACCCGCACCTACA
GCCTGGGCAGCGCGCTGCGCCCCAGCACCAGCCGCAGCCTCTACGCCTCGTCCCCGG
GCGGCGTGTATGCCACGCGCTCCTCTGCCGTGCGCCTGCGGAGCAGCGTGCCCGGGG
TGCGGCTCCTGCAGGACTCGGTGGACTTCTCGCTGGCCGACGCCATCAACACCGAGTT
CAAGAACACCCGCACCAACGAGAAGGTGGAGCTGCAGGAGCTGAATGACCGCTTCGCC
TAGTACATCGACAAGGTGCGCTTCCTGGAGCAGCAGAATAAGATCCTGCTGGCCGAGC
TCGAGCAGCTCAAGGGCCAAGGCAAGTCGCGCCTGGGGGACCTCTACGAGGAGGAGA
TGCGGGAGCTGCGCCGGCAGGTGGACCAGCTAACCAACGACAAAGCCCGCGTCGAGG
TGGAGCGCGACAACCTGGCCGAGGACATCATGCGCCTCCGGGAGAAATTGCAGGAGG
AGATGCTTCAGAGAGAGGAAGCCGAAAACACCCTGCAATCTTTCAGACAGGATGTTGAC
AATGCGTCTCTGGCACGTCTTGACCTTGAACGCAAAGTGGAATCTTTGCAAGAAGAGAT
TGCCTTTTTGAAGAAACTCCACGAAGAGGAAATCCAGGAGCTGCAGGCTCAGATTCAGG
AACAGCATGTCCAAATCGATGTGGATGTTTCCAAGCCTGACCTCACGGCTGCCCTGCGT
GACGTACGTCAGCAATATGAAAGTGTGGCTGCCAAGAACCTGCAGGAGGCAGAAGAAT
GGTACAAATCCAAGTTTGCTGACCTCTCTGAGGCTGCCAACCGGAACAATGACGCCCTG
CGCCAGGCAAAGCAGGAGTCCACTGAGTACCGGAGACAGGTGCAGTCCCTCACCTGTG
 
AAGTGGATGCCCTTAAAGGAACCAATGAGTCCCTGGAACGCCAGATGCGTGAAATGGA
AGAGAACTTTGCCGTTGAAGCTGCTAACTACCAAGACACTATTGGCCGCCTGCAGGATG
AGATTCAGAATATGAAGGAGGAAATGGCTCGTCACCTTCGTGAATACCAAGACCTGCTC
AATGTTAAGATGGCCCTTGACATTGAGATTGCCACCTACAGGAAGCTGCTGGAAGGCGA
GGAGAGCAGGATTTCTCTGCCTCTTCCAAACTTTTCCTCCCTGAACCTGAGGGAAACTA
ATCTGGATTCACTCCCTCTGGTTGATACCCACTCAAAAAGGACACTTCTGATTAAGACGG
TTGAAACTAGAGATGGACAGGTTATCAACGAAACTTCTCAGCATCACGATGACCTTGAA
(SEQ ID NO:195)
Protein: (X indicates non-canonical amino acid)

MSTRSVSSSSYRRMFGGPGTASRPSSSRSYVTTSTRTYSLGSALRPSTSRSLYASSPGGV
YATRSSAVRLRSSVPGVRLLQDSVDFSLADAINTEFKNTRTNEKVELQELNDRFAXYIDKVR
FLEQQNKILLAELEQLKGQGKSRLGDLYEEEMRELRRQVDQLTNDKARVEVERDNLAEDIM
RLREKLQEEMLQREEAENTLQSFRQDVDNASLARLDLERKVESLQEEIAFLKKLHEEEIQEL
QAQIQEQHVQIDVDVSKPDLTAALRDVRQQYESVAAKNLQEAEEWYKSKFADLSEAANRN
NDALRQAKQESTEYRRQVQSLTCEVDALKGTNESLERQMREMEENFAVEAANYQDTIGRL
QDEIQNMKEEMARHLREYQDLLNVKMALDIEIATYRKLLEGEESRISLPLPNFSSLNLRETNL
DSLPLVDTHSKRTLLIKTVETRDGQVINETSQHHDDLE (SEQ ID NO:196)

INSR^{676TAG} (*Homo sapiens* insulin receptor; Uniprot: P06213)
DNA: (Amber codon underlined) 
ATGGGCACCGGGGGCCGGCGGGGGCGGCGGCCGCGCCGCTGCTGGTGGCGGTGG
CCGCGCTGCTACTGGGCGCCGCGGGCCACCTGTACCCCGGAGAGGTGTGTCCCGGCA
TGGATATCCGGAACAACCTCACTAGGTTGCATGAGCTGGAGAATTGCTCTGTCATCGAA
GGACACTTGCAGATACTCTTGATGTTCAAAACGAGGCCCGAAGATTTCCGAGACCTCAG
TTTCCCCAAACTCATCATGATCACTGATTACTTGCTGCTCTTCCGGGTCTATGGGCTCGA
GAGCCTGAAGGACCTGTTCCCCAACCTCACGGTCATCCGGGGATCACGACTGTTCTTTA
ACTACGCGCTGGTCATCTTCGAGATGGTTCACCTCAAGGAACTCGGCCTCTACAACCTG
ATGAACATCACCCGGGGTTCTGTCCGCATCGAGAAGAACAATGAGCTCTGTTACTTGGC
CACTATCGACTGGTCCCGTATCCTGGATTCCGTGGAGGATAATTACATCGTGTTGAACA
AAGATGACAACGAGGAGTGTGGAGACATCTGTCCGGGTACCGCGAAGGGCAAGACCAA
CTGCCCCGCCACCGTCATCAACGGGCAGTTTGTCGAACGATGTTGGACTCATAGTCACT
GCCAGAAAGTTTGCCCGACCATCTGTAAGTCACACGGCTGCACCGCCGAAGGCCTCTG
TTGCCACAGCGAGTGCCTGGGCAACTGTTCTCAGCCCGACGACCCCACCAAGTGCGTG
GCCTGCCGCAACTTCTACCTGGATGGCAGGTGTGTGGAGACCTGCCCGCCCCCGTACT
ACCACTTCCAGGACTGGCGCTGTGTGAACTTCAGCTTCTGCCAGGACCTGCACCACAAA
TGCAAGAACTCGCGGAGGCAGGGCTGCCACCAGTACGTCATTCACAACAACAAGTGCA
 
TCCCTGAGTGTCCCTCCGGGTACACGATGAATTCCAGCAACTTGCTGTGCACCCCATGC
CTGGGTCCCTGTCCCAAGGTGTGCCACCTCCTAGAAGGCGAGAAGACCATCGACTCGG
TGACGTCTGCCCAGGAGCTCCGAGGATGCACCGTCATCAACGGAGTCTGATCATCAA
CATTCGAGGAGGCAACAATCTGGCAGCTGAGCTAGAAGCCAACCTCGGCCTCATTGAA
GAAATTTCAGGGTATCTAAAAATCCGCCGATCCTACGCTCTGGTGTCACTTTCCTTCTTC
CGGAAGTTACGTCTGATTCGAGGAGAGACCTTGGAAATTGGGAACTACTCCTTCTATGC
CTTGGACAACCAGAACCTAAGGCAGCTCTGGGACTGGAGCAAACACAACCTCACCATC
ACTCAGGGGAAACTCTTCTTCCACTATAACCCCAAACTCTGCTTGTCAGAAATCCACAAG
ATGGAAGAAGTTTCAGGAACCAAGGGGCGCCAGGAGAGAAACGACATTGCCCTGAAGA
CCAATGGGGACCAGGCATCCTGTGAAAATGAGTTACTTAAATTTTCTTACATTCGGACAT
CTTTTGACAAGATCTTGCTGAGATGGGAGCCGTACTGGCCCCCCGACTTCCGAGACCT
CTTGGGGTTCATGCTGTTCTACAAAGAGGCCCCTTATCAGAATGTGACGGAGTTCGACG
GGCAGGATGCATGTGGTTCCAACAGTTGGACGGTGGTAGACATTGACCCACCCCTGAG
GTCCAACGACCCCAAATCACAGAACCACCCAGGGTGGCTGATGCGGGGTCTCAAGCCC
TGGACCCAGTATGCCATCTTTGTGAAGACCCTGGTCACCTTTTCGGATGAACGCCGGAC
CTATGGGGCCAAGAGTGACATCATTTATGTCCAGACAGATGCCACCAACCCCTCTGTGC
CCCTGGATCCAATCTCAGTGTCTAACTCATCATCCCAGATTATTCTGAAGTGGAAACCAC
CCTCCGACCCCAATGGCAACATCACCCACTACCTGGTTTTCTGGGAGAGGCAGGCGGA
AGACAGTGAGCTGTTCGAGCTGGATTATTGCCTCTAGGGGCTGAAGCTGCCCTCGAGG
ACCTGGTCTCCACCATTCGAGTCTGAAGATTCTCAGAAGCACAACCAGAGTGAGTATGA
GGATTCGGCCGGCGAATGCTGCTCCTGTCCAAAGACAGACTCTCAGATCCTGAAGGAG
CTGGAGGAGTCCTCGTTTAGGAAGACGTTTGAGGATTACCTGCACAACGTGGTTTTCGT
CCCCAGGCCATCTCGGAAACGCAGGTCCCTTGGCGATGTTGGGAATGTGACGGTGGCC
GTGCCCACGGTGGCAGCTTTCCCCAACACTTCCTCGACCAGCGTGCCCACGAGTCCGG
AGGAGCACAGGCCTTTTGAGAAGGTGGTGAACAAGGAGTCGCTGGTCATCTCCGGCTT
GCGACACTTCACGGGCTATCGCATCGAGCTGCAGGCTTGCAACCAGGACACCCCTGAG
GAACGGTGCAGTGTGGCAGCCTACGTCAGTGCGAGGACCATGCCTGAAGCCAAGGCT
GATGACATTGTTGGCCCTGTGACGCATGAAATCTTTGAGAACAACGTCGTCCACTTGAT
GTGGCAGGAGCCGAAGGAGCCCAATGGTCTGATCGTGCTGTATGAAGTGAGTTATCGG
CGATATGGTGATGAGGAGCTGCATCTCTGCGTCTCCCGCAAGCACTTCGCTCTGGAAC
GGGGCTGCAGGCTGCGTGGGCTGTCACCGGGGAACTACAGCGTGCGAATCCGGGCCA
CCTCCCTTGCGGGCAACGGCTCTTGGACGGAACCCACCTATTTCTACGTGACAGACTAT
TTAGACGTCCCGTCAAATATTGCAAAAATTATCATCGGCCCCCTCATCTTTGTCTTTCTCT
TCAGTGTTGTGATTGGAAGTATTTATCTATTCCTGAGAAAGAGGCAGCCAGATGGGCCG
CTGGGACCGCTTTACGCTTCTTCAAACCCTGAGTATCTCAGTGCCAGTGATGTGTTTCC
ATGCTCTGTGTACGTGCCGGACGAGTGGGAGGTGTCTCGAGAGAAGATCACCCTCCTT
 
CGAGAGCTGGGGCAGGGCTCCTTCGGCATGGTGTATGAGGGCAATGCCAGGGACATC
ATCAAGGGTGAGGCAGAGACCCGCGTGGCGGTGAAGACGGTCAACGAGTCAGCCAGT
CTCCGAGAGCGGATTGAGTTCCTCAATGAGGCCTCGGTCATGAAGGGCTTCACCTGCC
ATCACGTGGTGCGCCTCCTGGGAGTGGTGTCCAAGGGCCAGCCCACGCTGGTGGTGA
TGGAGCTGATGGCTCACGGAGACCTGAAGAGCTACCTCCGTTCTCTGCGGCCAGAGGC
TGAGAATAATCCTGGCCGCCCTCCCCCTACCCTTCAAGAGATGATTCAGATGGCGGCA
GAGATTGCTGACGGGATGGCCTACCTGAACGCCAAGAAGTTTGTGCATCGGGACCTGG
CAGCGAGAAACTGCATGGTCGCCCATGATTTTACTGTCAAAATTGGAGACTTTGGAATG
ACCAGAGACATCTATGAAACGGATTACTACCGGAAAGGGGGCAAGGGTCTGCTCCCTG
TACGGTGGATGGCACCGGAGTCCCTGAAGGATGGGGTCTTCACCACTTCTTCTGACAT
GTGGTCCTTTGGCGTGGTCCTTTGGGAAATCACCAGCTTGGCAGAACAGCCTTACCAAG
GCCTGTCTAATGAACAGGTGTTGAAATTTGTCATGGATGGAGGGTATCTGGATCAACCC
GACAACTGTCCAGAGAGAGTCACTGACCTCATGCGCATGTGCTGGCAATTCAACCCCAA
CATGAGGCCAACCTTCCTGGAGATTGTCAACCTGCTCAAGGACGACCTGCACCCCAGC
TTTCCAGAGGTGTCGTTCTTCCACAGCGAGGAGAACAAGGCTCCCGAGAGTGAGGAGC
TGGAGATGGAGTTTGAGGACATGGAGAATGTGCCCCTGGACCGTTCCTCGCACTGTCA
GAGGGAGGAGGCGGGGGCCGGGATGGAGGGTCCTCGCTGGGTTTCAAGCGGAGCT
ACGAGGAACACATCCCTTACACACACATGAACGGAGGCAAGAAAAACGGGCGGATTCT
GACCTTGCCTCGGTCCAATCCTTCCT (SEQ ID NO:197)
Protein: (X indicates non-canonical amino acid)

MGTGGRRGAAAAPLLVAVAALLLGAAGHLYPGEVCPGMDIRNNLTRLHELENCSVIEGHLQI
LLMFKTRPEDFRDLSFPKLIMITDYLLLFRVYGLESLKDLFPNLTVIRGSRLFFNYALVIFEMVH
LKELGLYNLMNITRGSVRIEKNNELCYLATIDWSRILDSVEDNYIVLNKDDNEECGDICPGTA
KGKTNCPATVINGQFVERCWTHSHCQKVCPTICKSHGCTAEGLCCHSECLGNCSQPDDPT
KCVACRNFYLDGRCVETCPPPYYHFQDWRCVNFSFCQDLHHKCKNSRRQGCHQYVIHNN
KCIPECPSGYTMNSSNLLCTPCLGPCPKVCHLLEGEKTIDSVTSAQELRGCTVINGSLIINIRG
GNNLAAELEANLGLIEEISGYLKIRRSYALVSLSFFRKLRLIRGETLEIGNYSFYALDNQNLRQ
LWDWSKHNLTITQGKLFFHYNPKLCLSEIHKMEEVSGTKGRQERNDIALKTNGDQASCENE
LLKFSYIRTSFDKILLRWEPYWPPDFRDLLGFMLFYKEAPYQNVTEFDGQDACGSNSWTVV
DIDPPLRSNDPKSQNHPGWLMRGLKPWTQYAIFVKTLVTFSDERRTYGAKSDIIYVQTDATN
PSVPLDPISVSNSSSQIILKWKPPSDPNGNITHYLVFWERQAEDSELFELDYCLXGLKLPSRT
WSPPFESEDSQKHNQSEYEDSAGECCSCPKTDSQILKELEESSFRKTFEDYLHNVVFVPRP
SRKRRSLGDVGNVTVAVPTVAAFPNTSSTSVPTSPEEHRPFEKVVNKESLVISGLRHFTGY
RIELQACNQDTPEERCSVAAYVSARTMPEAKADDIVGPVTHEIFENNVVHLMWQEPKEPNG
LIVLYEVSYRRYGDEELHLCVSRKHFALERGCRLRGLSPGNYSVRIRATSLAGNGSWTEPT
YFYVTDYLDVPSNIAKIIIGPLIFVFLFSVVIGSIYLFLRKRQPDGPLGPLYASSNPEYLSASDVF

PCSVYVPDEWEVSREKITLLRELGQGSFGMVYEGNARDIIKGEAETRVAVKTVNESASLRE
RIEFLNEASVMKGFTCHHVVRLLGVVSKGQPTLVVMELMAHGDLKSYLRSLRPEAENNPGR
PPPTLQEMIQMAAEIADGMAYLNAKKFVHRDLAARNCMVAHDFTVKIGDFGMTRDIYETDYY
RKGGKGLLPVRWMAPESLKDGVFTTSSDMWSFGVVLWEITSLAEQPYQGLSNEQVLKFVM
DGGYLDQPDNCPERVTDLMRMCWQFNPNMRPTFLEIVNLLKDDLHPSFPEVSFFHSEENK
APESEELEMEFEDMENVPLDRSSHCQREEAGGRDGGSSLGFKRSYEEHIPYTHMNGGKK
NGRILTLPRSNPS (SEQ ID NO:198)

Nup153-EGFP^{149TAG}
DNA: (Amber codon underlined) 
ATGGCGTCTGGTGCTGGCGGTGTTGGTGGAGGAGGTGGGGGTAAAATTCGTACTCGTC
GCTGTCATCAAGGTCCGATTAAACCGTATCAGCAGGGACGTCAGCAACATCAGGGTATT
CTGAGCCGTGTGACCGAAAGCGTGAAAACATTGTGCCGGGTTGGCTGCAACGTTATTT
CAACAAAAATGAGGATGTGTGTTCGTGTTCTACCGATACCAGTGAAGTTCCTCGTTGGC
CGGAAAACAAAGAAGATCACCTGGTGTATGCCGATGAAGAATCGAGCAATATCACCGAT
GGCCGTATTACTCCTGAACCGGCGGTTAGTAACACTGAAGAACCGTCAACCACAAGCAC
AGCATCGAACTATCCAGATGTCCTGACTCGCCCTTCTCTGCACCGTTCTCACCTGAACT
TTAGCATGCTGGAATCACCAGCTCTGCATTGTCAGCCGTCTACCAGTAGTGCCTTCCCG
ATTGGCTCTAGTGGCTTTTCGCTGGTCAAAGAGATCAAAGACTCGACCTCTCAACATGA
CGATGATAACATTAGCACGACCTCGGGTTTTAGTAGCCGTGCCTCCGATAAAGACATTA
CCGTGAGCAAAAACACCTCTCTGCCGCCTCTGTGGAGTCCTGAAGCCGAACGCTCTCA
TAGTCTGTCTCAGCACACAGCCACCAGTTCCAAAAAACCAGCCTTCAACCTGAGCGCCT
TTGGTACACTGTCACCGAGCCTGGGAAATTCCTCTATCCTGAAAACATCACAGCTGGGC
GATAGTCCGTTTTATCCGGGCAAAACGACGTATGGTGGTGCCGCTGCTGCTGTTCGCC
AGTCTAAACTGCGTAACACTCCGTATCAAGCTCCAGTCCGTCGCCAAATGAAAGCAAAA
CAACTGTCGGCCCAGTCTTATGGTGTGACAAGCTCTACAGCTCGTCGTATCCTGCAAAG
TCTGGAGAAAATGTCATCTCCGCTGGCAGATGCCAAACGTATTCCGTCCATTGTGAGCA
GTCCGCTGAATAGCCCGCTGGACCGTAGTGGGATCGATATCACCGACTTCCAAGCCAA
ACGTGAGAAAGTGGATAGCCAGTATCCGCCTGTACAACGTCTGATGACCCCGAAACCG
GTTTCAATTGCCACGAATCGTAGCGTGTATTTCAAACCGTCACTGACCCCTAGTGGTGA
GTTTCGTAAAACAAATCAGCGTATCGACAACAAATGCTCTACCGGGTATGAAAAAACAT
GACGCCGGGACAGAATCGTGAACAACGTGAATCTGGCTTCTCTTATCCGAACTTTAGTC
TGCCGGCAGCAAATGGTCTGAGTAGCGGTGTAGGAGGTGGTGGGGGCAAAATGCGCC
GTGAACGTCACGCCTTTGTGGCCTCTAAACCTCTGGAAGAAGAGATGGAGGTTCCT
GTACTGCCGAAAATCAGTCTGCCTATCACCTCTTCAAGTCTGCCGACCTTCAACTTTTCT
AGTCCGGAAATCACAACCTCTAGCCCGTCACCGATTAATAGCAGTCAAGCACTGACGAA
 
TAAAGTCCAAATGACCTCACCGAGTTCTACGGGTTCTCCGATGTTCAAATTCTCTAGTCC
TATCGTGAAATCAACCGAAGCGAACGTCCTGCCTCCTTCTAGTATTGGGTTCACCTTTAG
CGTCCCAGTGGCCAAAACAGCTGAACTGAGCGGTAGCAGTAGTACTCTGGAACCGATT
ATCAGCTCAAGCGCCCATCATGTCACTACCGTGAATAGCACAAACTGTAAAAAACGCC
GCCTGAGGACTGTGAAGGACCGTTTCGTCCTGCCGAAATCCTGAAAGAAGGTTCCGTC
CTGGACATTCTGAAATCTCCGGGATTTGCCTCTCCTAAAATCGACTCTGTTGCCGCTCAA
CCAACTGCCACATCACCGGTGGTTTATACTCGTCCGGCGATTAGCAGTTTTAGCAGTAG
TGGCATCGGTTTTGGTGAATCCCTGAAAGCTGGCTCATCTTGGCAGTGTGACACCTGCC
TGCTGCAAAACAAAGTGACCGATAACAAATGTATTGCCTGTCAGGCCGCCAAACTGTCT
CCTCGTGATACAGCCAAACAGACCGGCATCGAAACCCCTAATAAAGCGGGAAAACGA
CCCTGTCAGCAAGTGGTACGGGATTTGGGGACAAATTCAAACCTGTGATCGGCACATG
GGACTGTGACACTTGTCTGGTACAGAACAAACCAGAAGCGATCAAATGTGTGGCCTGTG
AAACGCCTAAACCTGGAACATGTGTGAAACGTGCCCTGACTCTGACTGTTGTGTCAGAA
AGCGCCGAAACCATGACGGCAAGCAGCTCATCCTGTACTGTGACTACCGGGACTCTGG
GATTTGGTGACAAATTCAAACGCCCGATTGGTTCCTGGGAATGCTCCGTGTGTTGTGTG
AGCAATAATGCCGAGGACAACAAATGTGTGTCCTGTATGAGCGAGAAACCTGGCAGCTC
TGTTCCTGCTAGCAGCTCTAGCACAGTTCCTGTTAGTCTGCCTAGTGGTGGTTCTCTGG
GTCTGGAAAAATTCAAAAAACCTGAAGGAAGCTGGGATTGTGAGCTGTGCCTGGTACAG
AATAAAGCGGATAGCACGAAATGTCTGGCCTGTGAGTCAGCCAAACCAGGGACTAAAA
GCGGCTTTAAAGGCTTCGACACGTCGAGCAGTTCTAGTAACAGCGCCGCCTCATCATCT
TTCAAATTTGGGGTGAGCAGCTCCTCTAGTGGTCCTAGTCAAACACTGACCTCTACCGG
AAACTTCAAATTCGGCGATCAGGGTGGCTTCAAAATTGGTGTCTCCTCTGATTCGGGTA
GCATTAACCCGATGAGTGAGGGGTTCAAATTCAGCAAACCAATTGGCGATTTCAAATTC
GGTGTGTCGTCTGAATCCAAACCTGAAGAAGTCAAAAAAGACAGCAAAAACGACAATTT
CAAATTCGGCCTGTCTAGTGGTCTGTCTAATCCGGTTAGCCTGACCCCGTTTCAGTTCG
GGGTGTCTAATCTGGGTCAGGAAGAGAAAAAAGAGGAGCTGCCTAAAAGTTCATCTGCC
GGGTTCAGTTTTGGTACAGGCGTGATCAATAGCACTCCAGCACCAGCCAATACAATCGT
GACGAGCGAGAACAAATCGAGCTTCAACCTGGGGACAATCGAAACGAAAAGCGCCAGT
GTAGCGCCATTCACGTGTAAAACCTCCGAGGCAAAAAAAGAAGAGATGCCGGCCACAA
AAGGTGGATTCTCATTCGGCAACGTGGAACCGGCTAGCCTGCCATCAGCAAGCGTGTT
TGTACTGGGCCGTACCGAGGAGAAACAGCAGGAACCTGTTACTAGCACCAGTCTGGTC
TTTGGTAAAAAAGCCGACAACGAAGAACCGAAATGTCAGCCAGTGTTCAGCTTCGGCAA
TAGCGAACAGACGAAAGACGAAAACAGCAGCAAATCGACGTTCAGCTTCAGTATGACGA
AACCGAGCGAAAAAGAAAGTGAGCAGCCAGCAAAAGCAACGTTCGCCTTTGGAGCACA
GACATCAACCACAGCCGATCAAGGAGCAGCGAAACCAGTTTTCAGTTTTCTGAATAACA
GCTCAAGCAGCAGTTCTACACCAGCAACCTCAGCAGGTGGTGGGATCTTTGGATCAAG
 
CACCTCATCCAGCAATCCGCCAGTGGCAACATTCGTGTTTGGCCAGAGCAGTAATCCG
GTGTCATCTTCAGCATTTGGGAATACCGCCGAGAGTAGCACATCACAGTCTCTGCTGTT
CTCACAGGACTCTAAACTGGCAACCACCTCTTCTACTGGTACAGCGGTTACCCCGTTTG
TGTTCGGTCCGGGAGCATCATCCAATAATACCACGACGTCGGGCTTTGGGTTTGGTGC
CACGACAACAAGCAGTAGCGCTGGTAGCAGCTTTGTCTTTGGCACAGGTCCTTCAGCAC
CTTCTGCTTCACCAGCTTTCGGAGCCAATCAGACTCCGACATTCGGACAGTCACAGGGT
GCCTCTCAACCAAATCCTCCGGGTTTTGGCAGTATTAGCAGTAGTACCGCCCTGTTCCC
GACCGGTAGTCAACCGGCACCGCCAACATTTGGAACGGTTAGCAGTAGTAGTCAGCCT
CCGGTGTTTGGACAACAACCGAGCCAGAGCGCCTTCGGATCAGGAACGACCCCTAATA
GTAGCAGTGCCTTCCAGTTCGGTAGCAGTACCACCAACTTCAACTTCACGAACAATAGC
CCGTCAGGTGTGTTCACGTTTGGCGCCAATTCTTCTACCCCAGCGGCAAGTGCTCAACC
TTCAGGCTCAGGTGGATTTCCTTTCAACCAGTCACCAGCAGCGTTTACTGTTGGTTCTAA
CGGGAAAAACGTTTTCAGTAGCAGCGGCACCTCGTTTTCTGGTCGTAAAATCAAAACGG
CCGTTCGTCGCCGTAAAGCGGATCCACCGGTCGCCACGAGAGTGAGCAAGGGCGAGG
AGCTGTTCACCGGGGTGGTGCCCATCCTGGTCGAGCTGGACGGCGACGTAAACGGCC
ACAAGTTCAGCGTGTCCGGCGAGGGCGAGGGCGATGCCACCTACGGCAAGCTGACCC
TGAAGTTCATCTGCACCACCGGCAAGCTGCCCGTGCCCTGGCCCACCCTCGTGACCAC
CCTGACCTACGGCGTGCAGTGCTTCAGCCGCTACCCCGACCACATGAAGCAGCACGAC
TTCTTCAAGTCCGCCATGCCCGAAGGCTACGTCCAGGAGCGCACCATCTTCTTCAAGGA
CGACGGCAACTACAAGACCCGCGCCGAGGTGAAGTTCGAGGGCGACACCCTGGTGAA
CCGCATCGAGCTGAAGGGCATCGACTTCAAGGAGGACGGCAACATCCTGGGGCACAA
GCTGGAGTACAACTACAACAGCCACTAGGTCTATATCATGGCCGACAAGCAGAAGAACG
GCATCAAGGTGAACTTCAAGATCCGCCACAACATCGAGGACGGCAGCGTGCAGCTCGC
CGACCACTACCAGCAGAACACCCCCATCGGCGACGGCCCCGTGCTGCTGCCCGACAA
CCACTACCTGAGCACCCAGTCCGCCCTGAGCAAAGACCCCAACGAGAAGCGCGATCAC
ATGGTCCTGCTGGAGTTCGTGACCGCCGCCGGGATCACTCTCGGCATGGACGAGCTGT
ACAAGTAA (SEQ ID NO:199)
Protein: (X indicates non-canonical amino acid)

MASGAGGVGGGGGGKIRTRRCHQGPIKPYQQGRQQHQGILSRVTESVKNIVPGWLQRYF
NKNEDVCSCSTDTSEVPRWPENKEDHLVYADEESSNITDGRITPEPAVSNTEEPSTTSTAS
NYPDVLTRPSLHRSHLNFSMLESPALHCQPSTSSAFPIGSSGFSLVKEIKDSTSQHDDDNIS
TTSGFSSRASDKDITVSKNTSLPPLWSPEAERSHSLSQHTATSSKKPAFNLSAFGTLSPSLG
NSSILKTSQLGDSPFYPGKTTYGGAAAAVRQSKLRNTPYQAPVRRQMKAKQLSAQSYGVT
SSTARRILQSLEKMSSPLADAKRIPSIVSSPLNSPLDRSGIDITDFQAKREKVDSQYPPVQRL
MTPKPVSIATNRSVYFKPSLTPSGEFRKTNQRIDNKCSTGYEKNMTPGQNREQRESGFSYP
NFSLPAANGLSSGVGGGGGKMRRERHAFVASKPLEEEEMEVPVLPKISLPITSSSLPTFNFS

SPEITTSSPSPINSSQALTNKVQMTSPSSTGSPMFKFSSPIVKSTEANVLPPSSIGFTFSVPV
AKTAELSGSSSTLEPIISSSAHHVTTVNSTNCKKTPPEDCEGPFRPAEILKEGSVLDILKSPGF
ASPKIDSVAAQPTATSPVVYTRPAISSFSSSGIGFGESLKAGSSWQCDTCLLQNKVTDNKCI
ACQAAKLSPRDTAKQTGIETPNKSGKTTLSASGTGFGDKFKPVIGTWDCDTCLVQNKPEAIK
CVACETPKPGTCVKRALTLTVVSESAETMTASSSSCTVTTGTLGFGDKFKRPIGSWECSVC
CVSNNAEDNKCVSCMSEKPGSSVPASSSSTVPVSLPSGGSLGLEKFKKPEGSWDCELCLV
QNKADSTKCLACESAKPGTKSGFKGFDTSSSSSNSAASSSFKFGVSSSSSGPSQTLTSTGN
FKFGDQGGFKIGVSSDSGSINPMSEGFKFSKPIGDFKFGVSSESKPEEVKKDSKNDNFKFG
LSSGLSNPVSLTPFQFGVSNLGQEEKKEELPKSSSAGFSFGTGVINSTPAPANTIVTSENKS
SFNLGTIETKSASVAPFTCKTSEAKKEEMPATKGGFSFGNVEPASLPSASVFVLGRTEEKQQ
EPVTSTSLVFGKKADNEEPKCQPVFSFGNSEQTKDENSSKSTFSFSMTKPSEKESEQPAKA
TFAFGAQTSTTADQGAAKPVFSFLNNSSSSSSTPATSAGGGIFGSSTSSSNPPVATFVFGQ
SSNPVSSSAFGNTAESSTSQSLLFSQDSKLATTSSTGTAVTPFVFGPGASSNNTTTSGFGF
GATTTSSSAGSSFVFGTGPSAPSASPAFGANQTPTFGQSQGASQPNPPGFGSISSSTALFP
TGSQPAPPTFGTVSSSSQPPVFGQQPSQSAFGSGTTPNSSSAFQFGSSTTNFNFTNNSPS
GVFTFGANSSTPAASAQPSGSGGFPFNQSPAAFTVGSNGKNVFSSSGTSFSGRKIKTAVRR
RKADPPVATRVSKGEELFTGVVPILVELDGDVNGHKFSVSGEGEGDATYGKLTLKFICTTGK
LPVPWPTLVTTLTYGVQCFSRYPDHMKQHDFFKSAMPEGYVQERTIFFKDDGNYKTRAEVK
FEGDTLVNRIELKGIDFKEDGNILGHKLEYNYNSHXVYIMADKQKNGIKVNFKIRHNIEDGSV
QLADHYQQNTPIGDGPVLLPDNHYLSTQSALSKDPNEKRDHMVLLEFVTAAGITLGMDELY
K (SEQ ID NO:200)

Nup153-EGFP^{149TAG}-MS2
DNA: (MS2stem-loops and Amber codon underlined) 
ATGGCGTCTGGTGCTGGCGGTGTTGGTGGAGGAGGTGGGGGTAAAATTCGTACTCGTC
GCTGTCATCAAGGTCCGATTAAACCGTATCAGCAGGGACGTCAGCAACATCAGGGTATT
CTGAGCCGTGTGACCGAAAGCGTGAAAAACATTGTGCCGGGTTGGCTGCAACGTTATTT
CAACAAAAATGAGGATGTGTGTTCGTGTTCTACCGATACCAGTGAAGTTCCTCGTTGGC
CGGAAAACAAAGAAGATCACCTGGTGTATGCCGATGAAGAATCGAGCAATATCACCGAT
GGCCGTATTACTCCTGAACCGGCGGTTAGTAACACTGAAGAACCGTCAACCACAAGCAC
AGCATCGAACTATCCAGATGTCCTGACTCGCCCTTCTCTGCACCGTTCTCACCTGAACT
TTAGCATGCTGGAATCACCAGCTCTGCATTGTCAGCCGTCTACCAGTAGTGCCTTCCCG
ATTGGCTCTAGTGGCTTTTCGCTGGTCAAAGAGATCAAAGACTCGACCTCTCAACATGA
CGATGATAACATTAGCACGACCTCGGGTTTTAGTAGCCGTGCCTCCGATAAAGACATTA
CCGTGAGCAAAAACACCTCTCTGCCGCCTCTGTGGAGTCCTGAAGCCGAACGCTCTCA
TAGTCTGTCTCAGCACACAGCCACCAGTTCCAAAAAACCAGCCTTCAACCTGAGCGCCT
 
TTGGTACACTGTCACCGAGCCTGGGAAATTCCTCTATCCTGAAAACATCACAGCTGGGC
GATAGTCCGTTTTATCCGGGCAAAACGACGTATGGTGGTGCCGCTGCTGCTGTTCGCC
AGTCTAAACTGCGTAACACTCCGTATCAAGCTCCAGTCCGTCGCCAAATGAAAGCAAAA
CAACTGTCGGCCCAGTCTTATGGTGTGACAAGCTCTACAGCTCGTCGTATCCTGCAAAG
TCTGGAGAAAATGTCATCTCCGCTGGCAGATGCCAAACGTATTCCGTCCATTGTGAGCA
GTCCGCTGAATAGCCCGCTGGACCGTAGTGGGATCGATATCACCGACTTCCAAGCCAA
ACGTGAGAAAGTGGATAGCCAGTATCCGCCTGTACAACGTCTGATGACCCCGAAACCG
GTTTCAATTGCCACGAATCGTAGCGTGTATTTCAAACCGTCACTGACCCCTAGTGGTGA
GTTTCGTAAAACAAATCAGCGTATCGACAACAAATGCTCTACCGGGTATGAAAAAACAT
GACGCCGGGACAGAATCGTGAACAACGTGAATCTGGCTTCTCTTATCCGAACTTTAGTC
TGCCGGCAGCAAATGGTCTGAGTAGCGGTGTAGGAGGTGGTGGGGGCAAAATGCGCC
GTGAACGTCACGCCTTTGTGGCCTCTAAACCTCTGGAAGAAGAGATGGAGGTTCCT
GTACTGCCGAAAATCAGTCTGCCTATCACCTCTTCAAGTCTGCCGACCTTCAACTTTTCT
AGTCCGGAAATCACAACCTCTAGCCCGTCACCGATTAATAGCAGTCAAGCACTGACGAA
TAAAGTCCAAATGACCTCACCGAGTTCTACGGGTTCTCCGATGTTCAAATTCTCTAGTCC
TATCGTGAAATCAACCGAAGCGAACGTCCTGCCTCCTTCTAGTATTGGGTTCACCTTTAG
CGTCCCAGTGGCCAAAACAGCTGAACTGAGCGGTAGCAGTAGTACTCTGGAACCGATT
ATCAGCTCAAGCGCCCATCATGTCACTACCGTGAATAGCACAAACTGTAAAAAAACGCC
GCCTGAGGACTGTGAAGGACCGTTTCGTCCTGCCGAAATCCTGAAAGAAGGTTCCGTC
CTGGACATTCTGAAATCTCCGGGATTTGCCTCTCCTAAAATCGACTCTGTTGCCGCTCAA
CCAACTGCCACATCACCGGTGGTTTATACTCGTCCGGCGATTAGCAGTTTTAGCAGTAG
TGGCATCGGTTTTGGTGAATCCCTGAAAGCTGGCTCATCTTGGCAGTGTGACACCTGCC
TGCTGCAAAACAAAGTGACCGATAACAAATGTATTGCCTGTCAGGCCGCCAAACTGTCT
CCTCGTGATACAGCCAAACAGACCGGCATCGAAACCCCTAATAAAGCGGGAAAACGA
CCCTGTCAGCAAGTGGTACGGGATTTGGGGACAAATTCAAACCTGTGATCGGCACATG
GGACTGTGACACTTGTCTGGTACAGAACAAACCAGAAGCGATCAAATGTGTGGCCTGTG
AAACGCCTAAACCTGGAACATGTGTGAAACGTGCCCTGACTCTGACTGTTGTGTCAGAA
AGCGCCGAAACCATGACGGCAAGCAGCTCATCCTGTACTGTGACTACCGGGACTCTGG
GATTTGGTGACAAATTCAAACGCCCGATTGGTTCCTGGGAATGCTCCGTGTGTTGTGTG
AGCAATAATGCCGAGGACAACAAATGTGTGTCCTGTATGAGCGAGAAACCTGGCAGCTC
TGTTCCTGCTAGCAGCTCTAGCACAGTTCCTGTTAGTCTGCCTAGTGGTGGTTCTCTGG
GTCTGGAAAAATTCAAAAAACCTGAAGGAAGCTGGGATTGTGAGCTGTGCCTGGTACAG
AATAAAGCGGATAGCACGAAATGTCTGGCCTGTGAGTCAGCCAAACCAGGGACTAAAA
GCGGCTTTAAAGGCTTCGACACGTCGAGCAGTTCTAGTAACAGCGCCGCCTCATCATCT
TTCAAATTTGGGGTGAGCAGCTCCTCTAGTGGTCCTAGTCAAACACTGACCTCTACCGG
AAACTTCAAATTCGGCGATCAGGGTGGCTTCAAAATTGGTGTCTCCTCTGATTCGGGTA
 
GCATTAACCCGATGAGTGAGGGGTTCAAATTCAGCAAACCAATTGGCGATTTCAAATTC
GGTGTGTCGTCTGAATCCAAACCTGAAGAAGTCAAAAAAGACAGCAAAAACGACAATTT
CAAATTCGGCCTGTCTAGTGGTCTGTCTAATCCGGTTAGCCTGACCCCGTTTCAGTTCG
GGGTGTCTAATCTGGGTCAGGAAGAGAAAAAAGAGGAGCTGCCTAAAAGTTCATCTGCC
GGGTTCAGTTTTGGTACAGGCGTGATCAATAGCACTCCAGCACCAGCCAATACAATCGT
GACGAGCGAGAACAAATCGAGCTTCAACCTGGGGACAATCGAAACGAAAAGCGCCAGT
GTAGCGCCATTCACGTGTAAAACCTCCGAGGCAAAAAAAGAAGAGATGCCGGCCACAA
AAGGTGGATTCTCATTCGGCAACGTGGAACCGGCTAGCCTGCCATCAGCAAGCGTGTT
TGTACTGGGCCGTACCGAGGAGAAACAGCAGGAACCTGTTACTAGCACCAGTCTGGTC
TTTGGTAAAAAAGCCGACAACGAAGAACCGAAATGTCAGCCAGTGTTCAGCTTCGGCAA
TAGCGAACAGACGAAAGACGAAAACAGCAGCAAATCGACGTTCAGCTTCAGTATGACGA
AACCGAGCGAAAAAGAAAGTGAGCAGCCAGCAAAAGCAACGTTCGCCTTTGGAGCACA
GACATCAACCACAGCCGATCAAGGAGCAGCGAAACCAGTTTTCAGTTTTCTGAATAACA
GCTCAAGCAGCAGTTCTACACCAGCAACCTCAGCAGGTGGTGGGATCTTTGGATCAAG
CACCTCATCCAGCAATCCGCCAGTGGCAACATTCGTGTTTGGCCAGAGCAGTAATCCG
GTGTCATCTTCAGCATTTGGGAATACCGCCGAGAGTAGCACATCACAGTCTCTGCTGTT
CTCACAGGACTCTAAACTGGCAACCACCTCTTCTACTGGTACAGCGGTTACCCCGTTTG
TGTTCGGTCCGGGAGCATCATCCAATAATACCACGACGTCGGGCTTTGGGTTTGGTGC
CACGACAACAAGCAGTAGCGCTGGTAGCAGCTTTGTCTTTGGCACAGGTCCTTCAGCAC
CTTCTGCTTCACCAGCTTTCGGAGCCAATCAGACTCCGACATTCGGACAGTCACAGGGT
GCCTCTCAACCAAATCCTCCGGGTTTTGGCAGTATTAGCAGTAGTACCGCCCTGTTCCC
GACCGGTAGTCAACCGGCACCGCCAACATTTGGAACGGTTAGCAGTAGTAGTCAGCCT
CCGGTGTTTGGACAACAACCGAGCCAGAGCGCCTTCGGATCAGGAACGACCCCTAATA
GTAGCAGTGCCTTCCAGTTCGGTAGCAGTACCACCAACTTCAACTTCACGAACAATAGC
CCGTCAGGTGTGTTCACGTTTGGCGCCAATTCTTCTACCCCAGCGGCAAGTGCTCAACC
TTCAGGCTCAGGTGGATTTCCTTTCAACCAGTCACCAGCAGCGTTTACTGTTGGTTCTAA
CGGGAAAAACGTTTTCAGTAGCAGCGGCACCTCGTTTTCTGGTCGTAAAATCAAAACGG
CCGTTCGTCGCCGTAAAGCGGATCCACCGGTCGCCACGAGAGTGAGCAAGGGCGAGG
AGCTGTTCACCGGGGTGGTGCCCATCCTGGTCGAGCTGGACGGCGACGTAAACGGCC
ACAAGTTCAGCGTGTCCGGCGAGGGCGAGGGCGATGCCACCTACGGCAAGCTGACCC
TGAAGTTCATCTGCACCACCGGCAAGCTGCCCGTGCCCTGGCCCACCCTCGTGACCAC
CCTGACCTACGGCGTGCAGTGCTTCAGCCGCTACCCCGACCACATGAAGCAGCACGAC
TTCTTCAAGTCCGCCATGCCCGAAGGCTACGTCCAGGAGCGCACCATCTTCTTCAAGGA
CGACGGCAACTACAAGACCCGCGCCGAGGTGAAGTTCGAGGGCGACACCCTGGTGAA
CCGCATCGAGCTGAAGGGCATCGACTTCAAGGAGGACGGCAACATCCTGGGGCACAA
GCTGGAGTACAACTACAACAGCCACTAGGTCTATATCATGGCCGACAAGCAGAAGAACG
 
GCATCAAGGTGAACTTCAAGATCCGCCACAACATCGAGGACGGCAGCGTGCAGCTCGC
CGACCACTACCAGCAGAACACCCCCATCGGCGACGGCCCCGTGCTGCTGCCCGACAA
CCACTACCTGAGCACCCAGTCCGCCCTGAGCAAAGACCCCAACGAGAAGCGCGATCAC
ATGGTCCTGCTGGAGTTCGTGACCGCCGCCGGGATCACTCTCGGCATGGACGAGCTGT
ACAAGTAAAGCGGCCGCGACTCTAGATCATAATCAGCACATGAGGATCACCCATGTCTG
CAGGTCGACTCTAGAAAACATGAGGATCACCCATGT (SEQ ID NO:201)
Protein: (X indicates non-canonical amino acid)

MASGAGGVGGGGGGKIRTRRCHQGPIKPYQQGRQQHQGILSRVTESVKNIVPGWLQRYF
NKNEDVCSCSTDTSEVPRWPENKEDHLVYADEESSNITDGRITPEPAVSNTEEPSTTSTAS
NYPDVLTRPSLHRSHLNFSMLESPALHCQPSTSSAFPIGSSGFSLVKEIKDSTSQHDDDNIS
TTSGFSSRASDKDITVSKNTSLPPLWSPEAERSHSLSQHTATSSKKPAFNLSAFGTLSPSLG
NSSILKTSQLGDSPFYPGKTTYGGAAAAVRQSKLRNTPYQAPVRRQMKAKQLSAQSYGVT
SSTARRILQSLEKMSSPLADAKRIPSIVSSPLNSPLDRSGIDITDFQAKREKVDSQYPPVQRL
MTPKPVSIATNRSVYFKPSLTPSGEFRKTNQRIDNKCSTGYEKNMTPGQNREQRESGFSYP
NFSLPAANGLSSGVGGGGGKMRRERHAFVASKPLEEEEMEVPVLPKISLPITSSSLPTFNFS
SPEITTSSPSPINSSQALTNKVQMTSPSSTGSPMFKFSSPIVKSTEANVLPPSSIGFTFSVPV
AKTAELSGSSSTLEPIISSSAHHVTTVNSTNCKKTPPEDCEGPFRPAEILKEGSVLDILKSPGF
ASPKIDSVAAQPTATSPVVYTRPAISSFSSSGIGFGESLKAGSSWQCDTCLLQNKVTDNKCI
ACQAAKLSPRDTAKQTGIETPNKSGKTTLSASGTGFGDKFKPVIGTWDCDTCLVQNKPEAIK
CVACETPKPGTCVKRALTLTVVSESAETMTASSSSCTVTTGTLGFGDKFKRPIGSWECSVC
CVSNNAEDNKCVSCMSEKPGSSVPASSSSTVPVSLPSGGSLGLEKFKKPEGSWDCELCLV
QNKADSTKCLACESAKPGTKSGFKGFDTSSSSSNSAASSSFKFGVSSSSSGPSQTLTSTGN
FKFGDQGGFKIGVSSDSGSINPMSEGFKFSKPIGDFKFGVSSESKPEEVKKDSKNDNFKFG
LSSGLSNPVSLTPFQFGVSNLGQEEKKEELPKSSSAGFSFGTGVINSTPAPANTIVTSENKS
SFNLGTIETKSASVAPFTCKTSEAKKEEMPATKGGFSFGNVEPASLPSASVFVLGRTEEKQQ
EPVTSTSLVFGKKADNEEPKCQPVFSFGNSEQTKDENSSKSTFSFSMTKPSEKESEQPAKA
TFAFGAQTSTTADQGAAKPVFSFLNNSSSSSSTPATSAGGGIFGSSTSSSNPPVATFVFGQ
SSNPVSSSAFGNTAESSTSQSLLFSQDSKLATTSSTGTAVTPFVFGPGASSNNTTTSGFGF
GATTTSSSAGSSFVFGTGPSAPSASPAFGANQTPTFGQSQGASQPNPPGFGSISSSTALFP
TGSQPAPPTFGTVSSSSQPPVFGQQPSQSAFGSGTTPNSSSAFQFGSSTTNFNFTNNSPS
GVFTFGANSSTPAASAQPSGSGGFPFNQSPAAFTVGSNGKNVFSSSGTSFSGRKIKTAVRR
RKADPPVATRVSKGEELFTGVVPILVELDGDVNGHKFSVSGEGEGDATYGKLTLKFICTTGK
LPVPWPTLVTTLTYGVQCFSRYPDHMKQHDFFKSAMPEGYVQERTIFFKDDGNYKTRAEVK
FEGDTLVNRIELKGIDFKEDGNILGHKLEYNYNSHXVYIMADKQKNGIKVNFKIRHNIEDGSV
QLADHYQQNTPIGDGPVLLPDNHYLSTQSALSKDPNEKRDHMVLLEFVTAAGITLGMDELY
K (SEQ ID NO:202)

Vim^{116TAG}-mOrange
DNA: (Amber codon underlined) 
ATGTCCACCAGGTCCGTGTCCTCGTCCTCCTACCGCAGGATGTTCGGCGGCCCGGGCA
CCGCGAGCCGGCCGAGCTCCAGCCGGAGCTACGTGACTACGTCCACCCGCACCTACA
GCCTGGGCAGCGCGCTGCGCCCCAGCACCAGCCGCAGCCTCTACGCCTCGTCCCCGG
GCGGCGTGTATGCCACGCGCTCCTCTGCCGTGCGCCTGCGGAGCAGCGTGCCCGGGG
TGCGGCTCCTGCAGGACTCGGTGGACTTCTCGCTGGCCGACGCCATCAACACCGAGTT
CAAGAACACCCGCACCAACGAGAAGGTGGAGCTGCAGGAGCTGAATGACCGCTTCGCC
TAGTACATCGACAAGGTGCGCTTCCTGGAGCAGCAGAATAAGATCCTGCTGGCCGAGC
TCGAGCAGCTCAAGGGCCAAGGCAAGTCGCGCCTGGGGGACCTCTACGAGGAGGAGA
TGCGGGAGCTGCGCCGGCAGGTGGACCAGCTAACCAACGACAAAGCCCGCGTCGAGG
TGGAGCGCGACAACCTGGCCGAGGACATCATGCGCCTCCGGGAGAAATTGCAGGAGG
AGATGCTTCAGAGAGAGGAAGCCGAAAACACCCTGCAATCTTTCAGACAGGATGTTGAC
AATGCGTCTCTGGCACGTCTTGACCTTGAACGCAAAGTGGAATCTTTGCAAGAAGAGAT
TGCCTTTTTGAAGAAACTCCACGAAGAGGAAATCCAGGAGCTGCAGGCTCAGATTCAGG
AACAGCATGTCCAAATCGATGTGGATGTTTCCAAGCCTGACCTCACGGCTGCCCTGCGT
GACGTACGTCAGCAATATGAAAGTGTGGCTGCCAAGAACCTGCAGGAGGCAGAAGAAT
GGTACAAATCCAAGTTTGCTGACCTCTCTGAGGCTGCCAACCGGAACAATGACGCCCTG
CGCCAGGCAAAGCAGGAGTCCACTGAGTACCGGAGACAGGTGCAGTCCCTCACCTGTG
AAGTGGATGCCCTTAAAGGAACCAATGAGTCCCTGGAACGCCAGATGCGTGAAATGGA
AGAGAACTTTGCCGTTGAAGCTGCTAACTACCAAGACACTATTGGCCGCCTGCAGGATG
AGATTCAGAATATGAAGGAGGAAATGGCTCGTCACCTTCGTGAATACCAAGACCTGCTC
AATGTTAAGATGGCCCTTGACATTGAGATTGCCACCTACAGGAAGCTGCTGGAAGGCGA
GGAGAGCAGGATTTCTCTGCCTCTTCCAAACTTTTCCTCCCTGAACCTGAGGGAAACTA
ATCTGGATTCACTCCCTCTGGTTGATACCCACTCAAAAAGGACACTTCTGATTAAGACGG
TTGAAACTAGAGATGGACAGGTTATCAACGAAACTTCTCAGCATCACGATGACCTTGAA
GGGGATCCACCGGTCGCCACCATGGTGAGCAAGGGCGAGGAGAATAATATGGCCATCA
TCAAGGAGTTCATGCGCTTCAAGGTGCGCATGGAGGGCACCGTGAACGGCCACGAGTT
CGAGATCGAGGGCGAGGGCGAGGGCCGCCCCTACGAGGGCTTTCAGACCGCTAAGCT
GAAGGTGACCAAGGGCGGCCCCCTGCCCTTCGCCTGGGACATCCTGTCCCCTCTCTTC
ACCTACGGCTCCAAGGCCTACGTGAAGCACCCCGCCGACATCCCCGACTACTTCAAGC
TGTCCTTCCCCGAGGGCTTCAAGTGGGAGCGCGTGATGAACTACGAGGACGGCGGCG
TGGTGACCGTGACCCAGGACTCCTCACTGCAGGACGGCGAGTTCATCTACAAGGTGAA
GATGCGCGGCACCAACTTCCCCTCCGACGGCCCCGTGATGCAGAAGAAGACCATGGG
CTGGGAGGCCTCCTCCGAGCGGATGTACCCCGAGGACGGCGCCCTGAAGGGCGAGAT
 
CAGGATGAGGCTGAAGCTGAAGGACGGCGGCCACTACACCTCCGAGGTCAAGACCAC
CTACAAGGCCAAGAAGTCCGTGCAGCTGCCCGGCGCCTACATCGTCGGCATCAAGCTG
GACATCACCTCCCACAACGAGGACTACACCATCGTGGAACAGTACGAACGCGCCGAGG
GCCGCCACTCCACCGGCGGCATGGACGAGCTGTACAAGTAA (SEQ ID NO:203)
Protein: (X indicates non-canonical amino acid)

MSTRSVSSSSYRRMFGGPGTASRPSSSRSYVTTSTRTYSLGSALRPSTSRSLYASSPGGV
YATRSSAVRLRSSVPGVRLLQDSVDFSLADAINTEFKNTRTNEKVELQELNDRFAXYIDKVR
FLEQQNKILLAELEQLKGQGKSRLGDLYEEEMRELRRQVDQLTNDKARVEVERDNLAEDIM
RLREKLQEEMLQREEAENTLQSFRQDVDNASLARLDLERKVESLQEEIAFLKKLHEEEIQEL
QAQIQEQHVQIDVDVSKPDLTAALRDVRQQYESVAAKNLQEAEEWYKSKFADLSEAANRN
NDALRQAKQESTEYRRQVQSLTCEVDALKGTNESLERQMREMEENFAVEAANYQDTIGRL
QDEIQNMKEEMARHLREYQDLLNVKMALDIEIATYRKLLEGEESRISLPLPNFSSLNLRETNL
DSLPLVDTHSKRTLLIKTVETRDGQVINETSQHHDDLEGDPPVATMVSKGEENNMAIIKEFM
RFKVRMEGTVNGHEFEIEGEGEGRPYEGFQTAKLKVTKGGPLPFAWDILSPLFTYGSKAYV
KHPADIPDYFKLSFPEGFKWERVMNYEDGGVVTVTQDSSLQDGEFIYKVKMRGTNFPSDG
PVMQKKTMGWEASSERMYPEDGALKGEIRMRLKLKDGGHYTSEVKTTYKAKKSVQLPGAY
IVGIKLDITSHNEDYTIVEQYERAEGRHSTGGMDELYK (SEQ ID NO:204)

Vim^{116TAG}-mOrange-MS2
DNA: (MS2 stem-loops and Amber codon underlined) 
ATGTCCACCAGGTCCGTGTCCTCGTCCTCCTACCGCAGGATGTTCGGCGGCCCGGGCA
CCGCGAGCCGGCCGAGCTCCAGCCGGAGCTACGTGACTACGTCCACCCGCACCTACA
GCCTGGGCAGCGCGCTGCGCCCCAGCACCAGCCGCAGCCTCTACGCCTCGTCCCCGG
GCGGCGTGTATGCCACGCGCTCCTCTGCCGTGCGCCTGCGGAGCAGCGTGCCCGGGG
TGCGGCTCCTGCAGGACTCGGTGGACTTCTCGCTGGCCGACGCCATCAACACCGAGTT
CAAGAACACCCGCACCAACGAGAAGGTGGAGCTGCAGGAGCTGAATGACCGCTTCGCC
TAGTACATCGACAAGGTGCGCTTCCTGGAGCAGCAGAATAAGATCCTGCTGGCCGAGC
TCGAGCAGCTCAAGGGCCAAGGCAAGTCGCGCCTGGGGGACCTCTACGAGGAGGAGA
TGCGGGAGCTGCGCCGGCAGGTGGACCAGCTAACCAACGACAAAGCCCGCGTCGAGG
TGGAGCGCGACAACCTGGCCGAGGACATCATGCGCCTCCGGGAGAAATTGCAGGAGG
AGATGCTTCAGAGAGAGGAAGCCGAAAACACCCTGCAATCTTTCAGACAGGATGTTGAC
AATGCGTCTCTGGCACGTCTTGACCTTGAACGCAAAGTGGAATCTTTGCAAGAAGAGAT
TGCCTTTTTGAAGAAACTCCACGAAGAGGAAATCCAGGAGCTGCAGGCTCAGATTCAGG
AACAGCATGTCCAAATCGATGTGGATGTTTCCAAGCCTGACCTCACGGCTGCCCTGCGT
GACGTACGTCAGCAATATGAAAGTGTGGCTGCCAAGAACCTGCAGGAGGCAGAAGAAT
GGTACAAATCCAAGTTTGCTGACCTCTCTGAGGCTGCCAACCGGAACAATGACGCCCTG
 
CGCCAGGCAAAGCAGGAGTCCACTGAGTACCGGAGACAGGTGCAGTCCCTCACCTGTG
AAGTGGATGCCCTTAAAGGAACCAATGAGTCCCTGGAACGCCAGATGCGTGAAATGGA
AGAGAACTTTGCCGTTGAAGCTGCTAACTACCAAGACACTATTGGCCGCCTGCAGGATG
AGATTCAGAATATGAAGGAGGAAATGGCTCGTCACCTTCGTGAATACCAAGACCTGCTC
AATGTTAAGATGGCCCTTGACATTGAGATTGCCACCTACAGGAAGCTGCTGGAAGGCGA
GGAGAGCAGGATTTCTCTGCCTCTTCCAAACTTTTCCTCCCTGAACCTGAGGGAAACTA
ATCTGGATTCACTCCCTCTGGTTGATACCCACTCAAAAAGGACACTTCTGATTAAGACGG
TTGAAACTAGAGATGGACAGGTTATCAACGAAACTTCTCAGCATCACGATGACCTTGAA
GGGGATCCACCGGTCGCCACCATGGTGAGCAAGGGCGAGGAGAATAATATGGCCATCA
TCAAGGAGTTCATGCGCTTCAAGGTGCGCATGGAGGGCACCGTGAACGGCCACGAGTT
CGAGATCGAGGGCGAGGGCGAGGGCCGCCCCTACGAGGGCTTTCAGACCGCTAAGCT
GAAGGTGACCAAGGGCGGCCCCCTGCCCTTCGCCTGGGACATCCTGTCCCCTCTCTTC
ACCTACGGCTCCAAGGCCTACGTGAAGCACCCCGCCGACATCCCCGACTACTTCAAGC
TGTCCTTCCCCGAGGGCTTCAAGTGGGAGCGCGTGATGAACTACGAGGACGGCGGCG
TGGTGACCGTGACCCAGGACTCCTCACTGCAGGACGGCGAGTTCATCTACAAGGTGAA
GATGCGCGGCACCAACTTCCCCTCCGACGGCCCCGTGATGCAGAAGAAGACCATGGG
CTGGGAGGCCTCCTCCGAGCGGATGTACCCCGAGGACGGCGCCCTGAAGGGCGAGAT
CAGGATGAGGCTGAAGCTGAAGGACGGCGGCCACTACACCTCCGAGGTCAAGACCAC
CTACAAGGCCAAGAAGTCCGTGCAGCTGCCCGGCGCCTACATCGTCGGCATCAAGCTG
GACATCACCTCCCACAACGAGGACTACACCATCGTGGAACAGTACGAACGCGCCGAGG
GCCGCCACTCCACCGGCGGCATGGACGAGCTGTACAAGTAAAGCGGCCGCGACTCTA
GATCATAATCAGCACATGAGGATCACCCATGTCTGCAGGTCGACTCTAGAAAACATGAG
GATCACCCATGT (SEQ ID NO:205)
Protein: (X indicates non-canonical amino acid)

MSTRSVSSSSYRRMFGGPGTASRPSSSRSYVTTSTRTYSLGSALRPSTSRSLYASSPGGV
YATRSSAVRLRSSVPGVRLLQDSVDFSLADAINTEFKNTRTNEKVELQELNDRFAXYIDKVR
FLEQQNKILLAELEQLKGQGKSRLGDLYEEEMRELRRQVDQLTNDKARVEVERDNLAEDIM
RLREKLQEEMLQREEAENTLQSFRQDVDNASLARLDLERKVESLQEEIAFLKKLHEEEIQEL
QAQIQEQHVQIDVDVSKPDLTAALRDVRQQYESVAAKNLQEAEEWYKSKFADLSEAANRN
NDALRQAKQESTEYRRQVQSLTCEVDALKGTNESLERQMREMEENFAVEAANYQDTIGRL
QDEIQNMKEEMARHLREYQDLLNVKMALDIEIATYRKLLEGEESRISLPLPNFSSLNLRETNL
DSLPLVDTHSKRTLLIKTVETRDGQVINETSQHHDDLEGDPPVATMVSKGEENNMAIIKEFM
RFKVRMEGTVNGHEFEIEGEGEGRPYEGFQTAKLKVTKGGPLPFAWDILSPLFTYGSKAYV
KHPADIPDYFKLSFPEGFKWERVMNYEDGGVVTVTQDSSLQDGEFIYKVKMRGTNFPSDG
PVMQKKTMGWEASSERMYPEDGALKGEIRMRLKLKDGGHYTSEVKTTYKAKKSVQLPGAY
IVGIKLDITSHNEDYTIVEQYERAEGRHSTGGMDELYK (SEQ ID NO:206)

INSR^{676TAG}-EGFP
DNA: (Amber codon underlined) 
ATGGGCACCGGGGGCCGGCGGGGGCGGCGGCCGCGCCGCTGCTGGTGGCGGTGG
CCGCGCTGCTACTGGGCGCCGCGGGCCACCTGTACCCCGGAGAGGTGTGTCCCGGCA
TGGATATCCGGAACAACCTCACTAGGTTGCATGAGCTGGAGAATTGCTCTGTCATCGAA
GGACACTTGCAGATACTCTTGATGTTCAAAACGAGGCCCGAAGATTTCCGAGACCTCAG
TTTCCCCAAACTCATCATGATCACTGATTACTTGCTGCTCTTCCGGGTCTATGGGCTCGA
GAGCCTGAAGGACCTGTTCCCCAACCTCACGGTCATCCGGGGATCACGACTGTTCTTTA
ACTACGCGCTGGTCATCTTCGAGATGGTTCACCTCAAGGAACTCGGCCTCTACAACCTG
ATGAACATCACCCGGGGTTCTGTCCGCATCGAGAAGAACAATGAGCTCTGTTACTTGGC
CACTATCGACTGGTCCCGTATCCTGGATTCCGTGGAGGATAATTACATCGTGTTGAACA
AAGATGACAACGAGGAGTGTGGAGACATCTGTCCGGGTACCGCGAAGGGCAAGACCAA
CTGCCCCGCCACCGTCATCAACGGGCAGTTTGTCGAACGATGTTGGACTCATAGTCACT
GCCAGAAAGTTTGCCCGACCATCTGTAAGTCACACGGCTGCACCGCCGAAGGCCTCTG
TTGCCACAGCGAGTGCCTGGGCAACTGTTCTCAGCCCGACGACCCCACCAAGTGCGTG
GCCTGCCGCAACTTCTACCTGGATGGCAGGTGTGTGGAGACCTGCCCGCCCCCGTACT
ACCACTTCCAGGACTGGCGCTGTGTGAACTTCAGCTTCTGCCAGGACCTGCACCACAAA
TGCAAGAACTCGCGGAGGCAGGGCTGCCACCAGTACGTCATTCACAACAACAAGTGCA
TCCCTGAGTGTCCCTCCGGGTACACGATGAATTCCAGCAACTTGCTGTGCACCCCATGC
CTGGGTCCCTGTCCCAAGGTGTGCCACCTCCTAGAAGGCGAGAAGACCATCGACTCGG
TGACGTCTGCCCAGGAGCTCCGAGGATGCACCGTCATCAACGGGAGTCTGATCATCAA
CATTCGAGGAGGCAACAATCTGGCAGCTGAGCTAGAAGCCAACCTCGGCCTCATTGAA
GAAATTTCAGGGTATCTAAAAATCCGCCGATCCTACGCTCTGGTGTCACTTTCCTTCTTC
CGGAAGTTACGTCTGATTCGAGGAGAGACCTTGGAAATTGGGAACTACTCCTTCTATGC
CTTGGACAACCAGAACCTAAGGCAGCTCTGGGACTGGAGCAAACACAACCTCACCATC
ACTCAGGGGAAACTCTTCTTCCACTATAACCCCAAACTCTGCTTGTCAGAAATCCACAAG
ATGGAAGAAGTTTCAGGAACCAAGGGGCGCCAGGAGAGAAACGACATTGCCCTGAAGA
CCAATGGGGACCAGGCATCCTGTGAAAATGAGTTACTTAAATTTTCTTACATTCGGACAT
CTTTTGACAAGATCTTGCTGAGATGGGAGCCGTACTGGCCCCCCGACTTCCGAGACCT
CTTGGGGTTCATGCTGTTCTACAAAGAGGCCCCTTATCAGAATGTGACGGAGTTCGACG
GGCAGGATGCATGTGGTTCCAACAGTTGGACGGTGGTAGACATTGACCCACCCCTGAG
GTCCAACGACCCCAAATCACAGAACCACCCAGGGTGGCTGATGCGGGGTCTCAAGCCC
TGGACCCAGTATGCCATCTTTGTGAAGACCCTGGTCACCTTTTCGGATGAACGCCGGAC
CTATGGGGCCAAGAGTGACATCATTTATGTCCAGACAGATGCCACCAACCCCTCTGTGC
CCCTGGATCCAATCTCAGTGTCTAACTCATCATCCCAGATTATTCTGAAGTGGAAACCAC
 
CCTCCGACCCCAATGGCAACATCACCCACTACCTGGTTTCTGGGAGAGGCAGGCGGA
AGACAGTGAGCTGTTCGAGCTGGATTATTGCCTCTAGGGGCTGAAGCTGCCCTCGAGG
ACCTGGTCTCCACCATTCGAGTCTGAAGATTCTCAGAAGCACAACCAGAGTGAGTATGA
GGATTCGGCCGGCGAATGCTGCTCCTGTCCAAAGACAGACTCTCAGATCCTGAAGGAG
CTGGAGGAGTCCTCGTTTAGGAAGACGTTTGAGGATTACCTGCACAACGTGGTTTTCGT
CCCCAGAAAAACCTCTTCAGGCACTGGTGCCGAGGACCCTAGGCCATCTCGGAAACGC
AGGTCCCTTGGCGATGTTGGGAATGTGACGGTGGCCGTGCCCACGGTGGCAGCTTTCC
CCAACACTTCCTCGACCAGCGTGCCCACGAGTCCGGAGGAGCACAGGCCTTTTGAGAA
GGTGGTGAACAAGGAGTCGCTGGTCATCTCCGGCTTGCGACACTTCACGGCTATCGC
ATCGAGCTGCAGGCTTGCAACCAGGACACCCCTGAGGAACGGTGCAGTGTGGCAGCCT
ACGTCAGTGCGAGGACCATGCCTGAAGCCAAGGCTGATGACATTGTTGGCCCTGTGAC
GCATGAAATCTTTGAGAACAACGTCGTCCACTTGATGTGGCAGGAGCCGAAGGAGCCC
AATGGTCTGATCGTGCTGTATGAAGTGAGTTATCGGCGATATGGTGATGAGGAGCTGCA
TCTCTGCGTCTCCCGCAAGCACTTCGCTCTGGAACGGGGCTGCAGGCTGCGTGGGCTG
TCACCGGGGAACTACAGCGTGCGAATCCGGGCCACCTCCCTTGCGGGCAACGGCTCTT
GGACGGAACCCACCTATTTCTACGTGACAGACTATTTAGACGTCCCGTCAAATATTGCA
AAAATTATCATCGGCCCCCTCATCTTTGTCTTTCTCTTCAGTGTTGTGATTGGAAGTATTT
ATCTATTCCTGAGAAAGAGGCAGCCAGATGGGCCGCTGGGACCGCTTTACGCTTCTTCA
AACCCTGAGTATCTCAGTGCCAGTGATGTGTTTCCATGCTCTGTGTACGTGCCGGACGA
GTGGGAGGTGTCTCGAGAGAAGATCACCCTCCTTCGAGAGCTGGGGCAGGGCTCCTTC
GGCATGGTGTATGAGGGCAATGCCAGGGACATCATCAAGGGTGAGGCAGAGACCCGC
GTGGCGGTGAAGACGGTCAACGAGTCAGCCAGTCTCCGAGAGCGGATTGAGTTCCTCA
ATGAGGCCTCGGTCATGAAGGGCTTCACCTGCCATCACGTGGTGCGCCTCCTGGGAGT
GGTGTCCAAGGGCCAGCCCACGCTGGTGGTGATGGAGCTGATGGCTCACGGAGACCT
GAAGAGCTACCTCCGTTCTCTGCGGCCAGAGGCTGAGAATAATCCTGGCCGCCCTCCC
CCTACCCTTCAAGAGATGATTCAGATGGCGGCAGAGATTGCTGACGGGATGGCCTACC
TGAACGCCAAGAAGTTTGTGCATCGGGACCTGGCAGCGAGAAACTGCATGGTCGCCCA
TGATTTTACTGTCAAAATTGGAGACTTTGGAATGACCAGAGACATCTATGAAACGGATTA
CTACCGGAAAGGGGGCAAGGGTCTGCTCCCTGTACGGTGGATGGCACCGGAGTCCCT
GAAGGATGGGGTCTTCACCACTTCTTCTGACATGTGGTCCTTTGGCGTGGTCCTTTGGG
AAATCACCAGCTTGGCAGAACAGCCTTACCAAGGCCTGTCTAATGAACAGGTGTTGAAA
TTTGTCATGGATGGAGGGTATCTGGATCAACCCGACAACTGTCCAGAGAGAGTCACTGA
CCTCATGCGCATGTGCTGGCAATTCAACCCCAACATGAGGCCAACCTTCCTGGAGATTG
TCAACCTGCTCAAGGACGACCTGCACCCCAGCTTTCCAGAGGTGTCGTTCTTCCACAGC
GAGGAGAACAAGGCTCCCGAGAGTGAGGAGCTGGAGATGGAGTTTGAGGACATGGAG
AATGTGCCCCTGGACCGTTCCTCGCACTGTCAGAGGGAGGAGGCGGGGGGCCGGGAT
 
GGAGGGTCCTCGCTGGGTTTCAAGCGGAGCTACGAGGAACACATCCCTTACACACACA
TGAACGGAGGCAAGAAAAACGGGCGGATTCTGACCTTGCCTCGGTCCAATCCTTCCTG
GGCCCGGGATCCACCGGTCGCCACCATGGTGAGCAAGGGCGAGGAGCTGTTCACCGG
GGTGGTGCCCATCCTGGTCGAGCTGGACGGCGACGTAAACGGCCACAAGTTCAGCGT
GTCCGGCGAGGGCGAGGGCGATGCCACCTACGGCAAGCTGACCCTGAAGTTCATCTG
CACCACCGGCAAGCTGCCCGTGCCCTGGCCCACCCTCGTGACCACCCTGACCTACGG
CGTGCAGTGCTTCAGCCGCTACCCCGACCACATGAAGCAGCACGACTTCTTCAAGTCC
GCCATGCCCGAAGGCTACGTCCAGGAGCGCACCATCTTCTTCAAGGACGACGGCAACT
ACAAGACCCGCGCCGAGGTGAAGTTCGAGGGCGACACCCTGGTGAACCGCATCGAGC
TGAAGGGCATCGACTTCAAGGAGGACGGCAACATCCTGGGGCACAAGCTGGAGTACAA
CTACAACAGCCACAACGTCTATATCATGGCCGACAAGCAGAAGAACGGCATCAAGGTGA
ACTTCAAGATCCGCCACAACATCGAGGACGGCAGCGTGCAGCTCGCCGACCACTACCA
GCAGAACACCCCCATCGGCGACGGCCCCGTGCTGCTGCCCGACAACCACTACCTGAG
CACCCAGTCCGCCCTGAGCAAAGACCCCAACGAGAAGCGCGATCACATGGTCCTGCTG
GAGTTCGTGACCGCCGCCGGGATCACTCTCGGCATGGACGAGCTGTACAAGTAA (SEQ
ID NO:207)
Protein: (X indicates non-canonical amino acid)

MGTGGRRGAAAAPLLVAVAALLLGAAGHLYPGEVCPGMDIRNNLTRLHELENCSVIEGHLQI
LLMFKTRPEDFRDLSFPKLIMITDYLLLFRVYGLESLKDLFPNLTVIRGSRLFFNYALVIFEMVH
LKELGLYNLMNITRGSVRIEKNNELCYLATIDWSRILDSVEDNYIVLNKDDNEECGDICPGTA
KGKTNCPATVINGQFVERCWTHSHCQKVCPTICKSHGCTAEGLCCHSECLGNCSQPDDPT
KCVACRNFYLDGRCVETCPPPYYHFQDWRCVNFSFCQDLHHKCKNSRRQGCHQYVIHNN
KCIPECPSGYTMNSSNLLCTPCLGPCPKVCHLLEGEKTIDSVTSAQELRGCTVINGSLIINIRG
GNNLAAELEANLGLIEEISGYLKIRRSYALVSLSFFRKLRLIRGETLEIGNYSFYALDNQNLRQ
LWDWSKHNLTITQGKLFFHYNPKLCLSEIHKMEEVSGTKGRQERNDIALKTNGDQASCENE
LLKFSYIRTSFDKILLRWEPYWPPDFRDLLGFMLFYKEAPYQNVTEFDGQDACGSNSWTVV
DIDPPLRSNDPKSQNHPGWLMRGLKPWTQYAIFVKTLVTFSDERRTYGAKSDIIYVQTDATN
PSVPLDPISVSNSSSQIILKWKPPSDPNGNITHYLVFWERQAEDSELFELDYCLXGLKLPSRT
WSPPFESEDSQKHNQSEYEDSAGECCSCPKTDSQILKELEESSFRKTFEDYLHNVVFVPRK
TSSGTGAEDPRPSRKRRSLGDVGNVTVAVPTVAAFPNTSSTSVPTSPEEHRPFEKVVNKES
LVISGLRHFTGYRIELQACNQDTPEERCSVAAYVSARTMPEAKADDIVGPVTHEIFENNVVHL
MWQEPKEPNGLIVLYEVSYRRYGDEELHLCVSRKHFALERGCRLRGLSPGNYSVRIRATSL
AGNGSWTEPTYFYVTDYLDVPSNIAKIIIGPLIFVFLFSVVIGSIYLFLRKRQPDGPLGPLYASS
NPEYLSASDVFPCSVYVPDEWEVSREKITLLRELGQGSFGMVYEGNARDIIKGEAETRVAVK
TVNESASLRERIEFLNEASVMKGFTCHHVVRLLGVVSKGQPTLVVMELMAHGDLKSYLRSL
RPEAENNPGRPPPTLQEMIQMAAEIADGMAYLNAKKFVHRDLAARNCMVAHDFTVKIGDFG

MTRDIYETDYYRKGGKGLLPVRWMAPESLKDGVFTTSSDMWSFGVVLWEITSLAEQPYQG
LSNEQVLKFVMDGGYLDQPDNCPERVTDLMRMCWQFNPNMRPTFLEIVNLLKDDLHPSFP
EVSFFHSEENKAPESEELEMEFEDMENVPLDRSSHCQREEAGGRDGGSSLGFKRSYEEHI
PYTHMNGGKKNGRILTLPRSNPSWARDPPVATMVSKGEELFTGVVPILVELDGDVNGHKFS
VSGEGEGDATYGKLTLKFICTTGKLPVPWPTLVTTLTYGVQCFSRYPDHMKQHDFFKSAMP
EGYVQERTIFFKDDGNYKTRAEVKFEGDTLVNRIELKGIDFKEDGNILGHKLEYNYNSHNVYI
MADKQKNGIKVNFKIRHNIEDGSVQLADHYQQNTPIGDGPVLLPDNHYLSTQSALSKDPNEK
RDHMVLLEFVTAAGITLGMDELYK (SEQ ID NO:208)

INSR^{676TAG}-EGFP-MS2
DNA: (MS2 stem-loops and Amber codon underlined) 
ATGGGCACCGGGGGCCGGCGGGGGCGGCGGCCGCGCCGCTGCTGGTGGCGGTGG
CCGCGCTGCTACTGGGCGCCGCGGGCCACCTGTACCCCGGAGAGGTGTGTCCCGGCA
TGGATATCCGGAACAACCTCACTAGGTTGCATGAGCTGGAGAATTGCTCTGTCATCGAA
GGACACTTGCAGATACTCTTGATGTTCAAAACGAGGCCCGAAGATTTCCGAGACCTCAG
TTTCCCCAAACTCATCATGATCACTGATTACTTGCTGCTCTTCCGGGTCTATGGGCTCGA
GAGCCTGAAGGACCTGTTCCCCAACCTCACGGTCATCCGGGGATCACGACTGTTCTTTA
ACTACGCGCTGGTCATCTTCGAGATGGTTCACCTCAAGGAACTCGGCCTCTACAACCTG
ATGAACATCACCCGGGGTTCTGTCCGCATCGAGAAGAACAATGAGCTCTGTTACTTGGC
CACTATCGACTGGTCCCGTATCCTGGATTCCGTGGAGGATAATTACATCGTGTTGAACA
AAGATGACAACGAGGAGTGTGGAGACATCTGTCCGGGTACCGCGAAGGGCAAGACCAA
CTGCCCCGCCACCGTCATCAACGGGCAGTTTGTCGAACGATGTTGGACTCATAGTCACT
GCCAGAAAGTTTGCCCGACCATCTGTAAGTCACACGGCTGCACCGCCGAAGGCCTCTG
TTGCCACAGCGAGTGCCTGGGCAACTGTTCTCAGCCCGACGACCCCACCAAGTGCGTG
GCCTGCCGCAACTTCTACCTGGATGGCAGGTGTGTGGAGACCTGCCCGCCCCCGTACT
ACCACTTCCAGGACTGGCGCTGTGTGAACTTCAGCTTCTGCCAGGACCTGCACCACAAA
TGCAAGAACTCGCGGAGGCAGGGCTGCCACCAGTACGTCATTCACAACAACAAGTGCA
TCCCTGAGTGTCCCTCCGGGTACACGATGAATTCCAGCAACTTGCTGTGCACCCCATGC
CTGGGTCCCTGTCCCAAGGTGTGCCACCTCCTAGAAGGCGAGAAGACCATCGACTCGG
TGACGTCTGCCCAGGAGCTCCGAGGATGCACCGTCATCAACGGGAGTCTGATCATCAA
CATTCGAGGAGGCAACAATCTGGCAGCTGAGCTAGAAGCCAACCTCGGCCTCATTGAA
GAAATTTCAGGGTATCTAAAAATCCGCCGATCCTACGCTCTGGTGTCACTTTCCTTCTTC
CGGAAGTTACGTCTGATTCGAGGAGAGACCTTGGAAATTGGGAACTACTCCTTCTATGC
CTTGGACAACCAGAACCTAAGGCAGCTCTGGGACTGGAGCAAACACAACCTCACCATC
ACTCAGGGGAAACTCTTCTTCCACTATAACCCCAAACTCTGCTTGTCAGAAATCCACAAG
ATGGAAGAAGTTTCAGGAACCAAGGGGCGCCAGGAGAGAAACGACATTGCCCTGAAGA
 
CCAATGGGGACCAGGCATCCTGTGAAAATGAGTTACTTAAATTTTCTTACATTCGGACAT
CTTTTGACAAGATCTTGCTGAGATGGGAGCCGTACTGGCCCCCCGACTTCCGAGACCT
CTTGGGGTTCATGCTGTTCTACAAAGAGGCCCCTTATCAGAATGTGACGGAGTTCGACG
GGCAGGATGCATGTGGTTCCAACAGTTGGACGGTGGTAGACATTGACCCACCCCTGAG
GTCCAACGACCCCAAATCACAGAACCACCCAGGGTGGCTGATGCGGGGTCTCAAGCCC
TGGACCCAGTATGCCATCTTTGTGAAGACCCTGGTCACCTTTTCGGATGAACGCCGGAC
CTATGGGGCCAAGAGTGACATCATTTATGTCCAGACAGATGCCACCAACCCCTCTGTGC
CCCTGGATCCAATCTCAGTGTCTAACTCATCATCCCAGATTATTCTGAAGTGGAAACCAC
CCTCCGACCCCAATGGCAACATCACCCACTACCTGGTTTTCTGGGAGAGGCAGGCGGA
AGACAGTGAGCTGTTCGAGCTGGATTATTGCCTCTAGGGGCTGAAGCTGCCCTCGAGG
ACCTGGTCTCCACCATTCGAGTCTGAAGATTCTCAGAAGCACAACCAGAGTGAGTATGA
GGATTCGGCCGGCGAATGCTGCTCCTGTCCAAAGACAGACTCTCAGATCCTGAAGGAG
CTGGAGGAGTCCTCGTTTAGGAAGACGTTTGAGGATTACCTGCACAACGTGGTTTTCGT
CCCCAGAAAAACCTCTTCAGGCACTGGTGCCGAGGACCCTAGGCCATCTCGGAAACGC
AGGTCCCTTGGCGATGTTGGGAATGTGACGGTGGCCGTGCCCACGGTGGCAGCTTTCC
CCAACACTTCCTCGACCAGCGTGCCCACGAGTCCGGAGGAGCACAGGCCTTTTGAGAA
GGTGGTGAACAAGGAGTCGCTGGTCATCTCCGGCTTGCGACACTTCACGGGCTATCGC
ATCGAGCTGCAGGCTTGCAACCAGGACACCCCTGAGGAACGGTGCAGTGTGGCAGCCT
ACGTCAGTGCGAGGACCATGCCTGAAGCCAAGGCTGATGACATTGTTGGCCCTGTGAC
GCATGAAATCTTTGAGAACAACGTCGTCCACTTGATGTGGCAGGAGCCGAAGGAGCCC
AATGGTCTGATCGTGCTGTATGAAGTGAGTTATCGGCGATATGGTGATGAGGAGCTGCA
TCTCTGCGTCTCCCGCAAGCACTTCGCTCTGGAACGGGGCTGCAGGCTGCGTGGGCTG
TCACCGGGGAACTACAGCGTGCGAATCCGGGCCACCTCCCTTGCGGGCAACGGCTCTT
GGACGGAACCCACCTATTTCTACGTGACAGACTATTTAGACGTCCCGTCAAATATTGCA
AAAATTATCATCGGCCCCCTCATCTTTGTCTTTCTCTTCAGTGTTGTGATTGGAAGTATTT
ATCTATTCCTGAGAAAGAGGCAGCCAGATGGGCCGCTGGGACCGCTTTACGCTTCTTCA
AACCCTGAGTATCTCAGTGCCAGTGATGTGTTTCCATGCTCTGTGTACGTGCCGGACGA
GTGGGAGGTGTCTCGAGAGAAGATCACCCTCCTTCGAGAGCTGGGGCAGGGCTCCTTC
GGCATGGTGTATGAGGGCAATGCCAGGGACATCATCAAGGGTGAGGCAGAGACCCGC
GTGGCGGTGAAGACGGTCAACGAGTCAGCCAGTCTCCGAGAGCGGATTGAGTTCCTCA
ATGAGGCCTCGGTCATGAAGGGCTTCACCTGCCATCACGTGGTGCGCCTCCTGGGAGT
GGTGTCCAAGGGCCAGCCCACGCTGGTGGTGATGGAGCTGATGGCTCACGGAGACCT
GAAGAGCTACCTCCGTTCTCTGCGGCCAGAGGCTGAGAATAATCCTGGCCGCCCTCCC
CCTACCCTTCAAGAGATGATTCAGATGGCGGCAGAGATTGCTGACGGGATGGCCTACC
TGAACGCCAAGAAGTTTGTGCATCGGGACCTGGCAGCGAGAAACTGCATGGTCGCCCA
TGATTTTACTGTCAAAATTGGAGACTTTGGAATGACCAGAGACATCTATGAAACGGATTA
 
CTACCGGAAAGGGGGCAAGGGTCTGCTCCCTGTACGGTGGATGGCACCGGAGTCCCT
GAAGGATGGGGTCTTCACCACTTCTTCTGACATGTGGTCCTTTGGCGTGGTCCTTTGGG
AAATCACCAGCTTGGCAGAACAGCCTTACCAAGGCCTGTCTAATGAACAGGTGTTGAAA
TTTGTCATGGATGGAGGGTATCTGGATCAACCCGACAACTGTCCAGAGAGAGTCACTGA
CCTCATGCGCATGTGCTGGCAATTCAACCCCAACATGAGGCCAACCTTCCTGGAGATTG
TCAACCTGCTCAAGGACGACCTGCACCCCAGCTTTCCAGAGGTGTCGTTCTTCCACAGC
GAGGAGAACAAGGCTCCCGAGAGTGAGGAGCTGGAGATGGAGTTTGAGGACATGGAG
AATGTGCCCCTGGACCGTTCCTCGCACTGTCAGAGGGAGGAGGCGGGGGCCGGGAT
GGAGGGTCCTCGCTGGGTTTCAAGCGGAGCTACGAGGAACACATCCCTTACACACACA
TGAACGGAGGCAAGAAAAACGGGCGGATTCTGACCTTGCCTCGGTCCAATCCTTCCTG
GGCCCGGGATCCACCGGTCGCCACCATGGTGAGCAAGGGCGAGGAGCTGTTCACCGG
GGTGGTGCCCATCCTGGTCGAGCTGGACGGCGACGTAAACGGCCACAAGTTCAGCGT
GTCCGGCGAGGGCGAGGGCGATGCCACCTACGGCAAGCTGACCCTGAAGTTCATCTG
CACCACCGGCAAGCTGCCCGTGCCCTGGCCCACCCTCGTGACCACCCTGACCTACGG
CGTGCAGTGCTTCAGCCGCTACCCCGACCACATGAAGCAGCACGACTTCTTCAAGTCC
GCCATGCCCGAAGGCTACGTCCAGGAGCGCACCATCTTCTTCAAGGACGACGGCAACT
ACAAGACCCGCGCCGAGGTGAAGTTCGAGGGCGACACCCTGGTGAACCGCATCGAGC
TGAAGGGCATCGACTTCAAGGAGGACGGCAACATCCTGGGGCACAAGCTGGAGTACAA
CTACAACAGCCACAACGTCTATATCATGGCCGACAAGCAGAAGAACGGCATCAAGGTGA
ACTTCAAGATCCGCCACAACATCGAGGACGGCAGCGTGCAGCTCGCCGACCACTACCA
GCAGAACACCCCCATCGGCGACGGCCCCGTGCTGCTGCCCGACAACCACTACCTGAG
CACCCAGTCCGCCCTGAGCAAAGACCCCAACGAGAAGCGCGATCACATGGTCCTGCTG
GAGTTCGTGACCGCCGCCGGGATCACTCTCGGCATGGACGAGCTGTACAAGTAAAGCG
GCCGCGCGGCCGCGACTCTAGATCATAATCAGCACATGAGGATCACCCATGTCTGCAG
GTCGACTCTAGAAAACATGAGGATCACCCATGT (SEQ ID NO:209)
Protein: (X indicates non-canonical amino acid)

MGTGGRRGAAAAPLLVAVAALLLGAAGHLYPGEVCPGMDIRNNLTRLHELENCSVIEGHLQI
LLMFKTRPEDFRDLSFPKLIMITDYLLLFRVYGLESLKDLFPNLTVIRGSRLFFNYALVIFEMVH
LKELGLYNLMNITRGSVRIEKNNELCYLATIDWSRILDSVEDNYIVLNKDDNEECGDICPGTA
KGKTNCPATVINGQFVERCWTHSHCQKVCPTICKSHGCTAEGLCCHSECLGNCSQPDDPT
KCVACRNFYLDGRCVETCPPPYYHFQDWRCVNFSFCQDLHHKCKNSRRQGCHQYVIHNN
KCIPECPSGYTMNSSNLLCTPCLGPCPKVCHLLEGEKTIDSVTSAQELRGCTVINGSLIINIRG
GNNLAAELEANLGLIEEISGYLKIRRSYALVSLSFFRKLRLIRGETLEIGNYSFYALDNQNLRQ
LWDWSKHNLTITQGKLFFHYNPKLCLSEIHKMEEVSGTKGRQERNDIALKTNGDQASCENE
LLKFSYIRTSFDKILLRWEPYWPPDFRDLLGFMLFYKEAPYQNVTEFDGQDACGSNSWTVV
DIDPPLRSNDPKSQNHPGWLMRGLKPWTQYAIFVKTLVTFSDERRTYGAKSDIIYVQTDATN

PSVPLDPISVSNSSSQIILKWKPPSDPNGNITHYLVFWERQAEDSELFELDYCLXGLKLPSRT
WSPPFESEDSQKHNQSEYEDSAGECCSCPKTDSQILKELEESSFRKTFEDYLHNVVFVPRK
TSSGTGAEDPRPSRKRRSLGDVGNVTVAVPTVAAFPNTSSTSVPTSPEEHRPFEKVVNKES
LVISGLRHFTGYRIELQACNQDTPEERCSVAAYVSARTMPEAKADDIVGPVTHEIFENNVVHL
MWQEPKEPNGLIVLYEVSYRRYGDEELHLCVSRKHFALERGCRLRGLSPGNYSVRIRATSL
AGNGSWTEPTYFYVTDYLDVPSNIAKIIIGPLIFVFLFSVVIGSIYLFLRKRQPDGPLGPLYASS
NPEYLSASDVFPCSVYVPDEWEVSREKITLLRELGQGSFGMVYEGNARDIIKGEAETRVAVK
TVNESASLRERIEFLNEASVMKGFTCHHVVRLLGVVSKGQPTLVVMELMAHGDLKSYLRSL
RPEAENNPGRPPPTLQEMIQMAAEIADGMAYLNAKKFVHRDLAARNCMVAHDFTVKIGDFG
MTRDIYETDYYRKGGKGLLPVRWMAPESLKDGVFTTSSDMWSFGVVLWEITSLAEQPYQG
LSNEQVLKFVMDGGYLDQPDNCPERVTDLMRMCWQFNPNMRPTFLEIVNLLKDDLHPSFP
EVSFFHSEENKAPESEELEMEFEDMENVPLDRSSHCQREEAGGRDGGSSLGFKRSYEEHI
PYTHMNGGKKNGRILTLPRSNPSWARDPPVATMVSKGEELFTGVVPILVELDGDVNGHKFS
VSGEGEGDATYGKLTLKFICTTGKLPVPWPTLVTTLTYGVQCFSRYPDHMKQHDFFKSAMP
EGYVQERTIFFKDDGNYKTRAEVKFEGDTLVNRIELKGIDFKEDGNILGHKLEYNYNSHNVYI
MADKQKNGIKVNFKIRHNIEDGSVQLADHYQQNTPIGDGPVLLPDNHYLSTQSALSKDPNEK
RDHMVLLEFVTAAGITLGMDELYK (SEQ ID NO:210)

INSR^{676TAG}-mOrange-MS2
DNA: (MS2 stem-loops and Amber codon underlined) 
ATGGGCACCGGGGGCCGGCGGGGGCGGCGGCCGCGCCGCTGCTGGTGGCGGTGG
CCGCGCTGCTACTGGGCGCCGCGGGCCACCTGTACCCCGGAGAGGTGTGTCCCGGCA
TGGATATCCGGAACAACCTCACTAGGTTGCATGAGCTGGAGAATTGCTCTGTCATCGAA
GGACACTTGCAGATACTCTTGATGTTCAAAACGAGGCCCGAAGATTTCCGAGACCTCAG
TTTCCCCAAACTCATCATGATCACTGATTACTTGCTGCTCTTCCGGGTCTATGGGCTCGA
GAGCCTGAAGGACCTGTTCCCCAACCTCACGGTCATCCGGGGATCACGACTGTTCTTTA
ACTACGCGCTGGTCATCTTCGAGATGGTTCACCTCAAGGAACTCGGCCTCTACAACCTG
ATGAACATCACCCGGGGTTCTGTCCGCATCGAGAAGAACAATGAGCTCTGTTACTTGGC
CACTATCGACTGGTCCCGTATCCTGGATTCCGTGGAGGATAATTACATCGTGTTGAACA
AAGATGACAACGAGGAGTGTGGAGACATCTGTCCGGGTACCGCGAAGGGCAAGACCAA
CTGCCCCGCCACCGTCATCAACGGGCAGTTTGTCGAACGATGTTGGACTCATAGTCACT
GCCAGAAAGTTTGCCCGACCATCTGTAAGTCACACGGCTGCACCGCCGAAGGCCTCTG
TTGCCACAGCGAGTGCCTGGGCAACTGTTCTCAGCCCGACGACCCCACCAAGTGCGTG
GCCTGCCGCAACTTCTACCTGGATGGCAGGTGTGTGGAGACCTGCCCGCCCCCGTACT
ACCACTTCCAGGACTGGCGCTGTGTGAACTTCAGCTTCTGCCAGGACCTGCACCACAAA
TGCAAGAACTCGCGGAGGCAGGGCTGCCACCAGTACGTCATTCACAACAACAAGTGCA
 
TCCCTGAGTGTCCCTCCGGGTACACGATGAATTCCAGCAACTTGCTGTGCACCCCATGC
CTGGGTCCCTGTCCCAAGGTGTGCCACCTCCTAGAAGGCGAGAAGACCATCGACTCGG
TGACGTCTGCCCAGGAGCTCCGAGGATGCACCGTCATCAACGGAGTCTGATCATCAA
CATTCGAGGAGGCAACAATCTGGCAGCTGAGCTAGAAGCCAACCTCGGCCTCATTGAA
GAAATTTCAGGGTATCTAAAAATCCGCCGATCCTACGCTCTGGTGTCACTTTCCTTCTTC
CGGAAGTTACGTCTGATTCGAGGAGAGACCTTGGAAATTGGGAACTACTCCTTCTATGC
CTTGGACAACCAGAACCTAAGGCAGCTCTGGGACTGGAGCAAACACAACCTCACCATC
ACTCAGGGGAAACTCTTCTTCCACTATAACCCCAAACTCTGCTTGTCAGAAATCCACAAG
ATGGAAGAAGTTTCAGGAACCAAGGGGCGCCAGGAGAGAAACGACATTGCCCTGAAGA
CCAATGGGGACCAGGCATCCTGTGAAAATGAGTTACTTAAATTTTCTTACATTCGGACAT
CTTTTGACAAGATCTTGCTGAGATGGGAGCCGTACTGGCCCCCCGACTTCCGAGACCT
CTTGGGGTTCATGCTGTTCTACAAAGAGGCCCCTTATCAGAATGTGACGGAGTTCGACG
GGCAGGATGCATGTGGTTCCAACAGTTGGACGGTGGTAGACATTGACCCACCCCTGAG
GTCCAACGACCCCAAATCACAGAACCACCCAGGGTGGCTGATGCGGGGTCTCAAGCCC
TGGACCCAGTATGCCATCTTTGTGAAGACCCTGGTCACCTTTTCGGATGAACGCCGGAC
CTATGGGGCCAAGAGTGACATCATTTATGTCCAGACAGATGCCACCAACCCCTCTGTGC
CCCTGGATCCAATCTCAGTGTCTAACTCATCATCCCAGATTATTCTGAAGTGGAAACCAC
CCTCCGACCCCAATGGCAACATCACCCACTACCTGGTTTTCTGGAGAGGCAGGCGGA
AGACAGTGAGCTGTTCGAGCTGGATTATTGCCTCTAGGGGCTGAAGCTGCCCTCGAGG
ACCTGGTCTCCACCATTCGAGTCTGAAGATTCTCAGAAGCACAACCAGAGTGAGTATGA
GGATTCGGCCGGCGAATGCTGCTCCTGTCCAAAGACAGACTCTCAGATCCTGAAGGAG
CTGGAGGAGTCCTCGTTTAGGAAGACGTTTGAGGATTACCTGCACAACGTGGTTTTCGT
CCCCAGGCCATCTCGGAAACGCAGGTCCCTTGGCGATGTTGGGAATGTGACGGTGGCC
GTGCCCACGGTGGCAGCTTTCCCCAACACTTCCTCGACCAGCGTGCCCACGAGTCCGG
AGGAGCACAGGCCTTTTGAGAAGGTGGTGAACAAGGAGTCGCTGGTCATCTCCGGCTT
GCGACACTTCACGGGCTATCGCATCGAGCTGCAGGCTTGCAACCAGGACACCCCTGAG
GAACGGTGCAGTGTGGCAGCCTACGTCAGTGCGAGGACCATGCCTGAAGCCAAGGCT
GATGACATTGTTGGCCCTGTGACGCATGAAATCTTTGAGAACAACGTCGTCCACTTGAT
GTGGCAGGAGCCGAAGGAGCCCAATGGTCTGATCGTGCTGTATGAAGTGAGTTATCGG
CGATATGGTGATGAGGAGCTGCATCTCTGCGTCTCCCGCAAGCACTTCGCTCTGGAAC
GGGGCTGCAGGCTGCGTGGGCTGTCACCGGGGAACTACAGCGTGCGAATCCGGGCCA
CCTCCCTTGCGGGCAACGGCTCTTGGACGGAACCCACCTATTTCTACGTGACAGACTAT
TTAGACGTCCCGTCAAATATTGCAAAAATTATCATCGGCCCCCTCATCTTTGTCTTTCTCT
TCAGTGTTGTGATTGGAAGTATTTATCTATTCCTGAGAAAGAGGCAGCCAGATGGGCCG
CTGGGACCGCTTTACGCTTCTTCAAACCCTGAGTATCTCAGTGCCAGTGATGTGTTTCC
ATGCTCTGTGTACGTGCCGGACGAGTGGGAGGTGTCTCGAGAGAAGATCACCCTCCTT
 
CGAGAGCTGGGGCAGGGCTCCTTCGGCATGGTGTATGAGGGCAATGCCAGGGACATC
ATCAAGGGTGAGGCAGAGACCCGCGTGGCGGTGAAGACGGTCAACGAGTCAGCCAGT
CTCCGAGAGCGGATTGAGTTCCTCAATGAGGCCTCGGTCATGAAGGGCTTCACCTGCC
ATCACGTGGTGCGCCTCCTGGGAGTGGTGTCCAAGGGCCAGCCCACGCTGGTGGTGA
TGGAGCTGATGGCTCACGGAGACCTGAAGAGCTACCTCCGTTCTCTGCGGCCAGAGGC
TGAGAATAATCCTGGCCGCCCTCCCCCTACCCTTCAAGAGATGATTCAGATGGCGGCA
GAGATTGCTGACGGGATGGCCTACCTGAACGCCAAGAAGTTTGTGCATCGGGACCTGG
CAGCGAGAAACTGCATGGTCGCCCATGATTTTACTGTCAAAATTGGAGACTTTGGAATG
ACCAGAGACATCTATGAAACGGATTACTACCGGAAAGGGGGCAAGGGTCTGCTCCCTG
TACGGTGGATGGCACCGGAGTCCCTGAAGGATGGGGTCTTCACCACTTCTTCTGACAT
GTGGTCCTTTGGCGTGGTCCTTTGGGAAATCACCAGCTTGGCAGAACAGCCTTACCAAG
GCCTGTCTAATGAACAGGTGTTGAAATTTGTCATGGATGGAGGGTATCTGGATCAACCC
GACAACTGTCCAGAGAGAGTCACTGACCTCATGCGCATGTGCTGGCAATTCAACCCCAA
CATGAGGCCAACCTTCCTGGAGATTGTCAACCTGCTCAAGGACGACCTGCACCCCAGC
TTTCCAGAGGTGTCGTTCTTCCACAGCGAGGAGAACAAGGCTCCCGAGAGTGAGGAGC
TGGAGATGGAGTTTGAGGACATGGAGAATGTGCCCCTGGACCGTTCCTCGCACTGTCA
GAGGGAGGAGGCGGGGGCCGGGATGGAGGGTCCTCGCTGGGTTTCAAGCGGAGCT
ACGAGGAACACATCCCTTACACACACATGAACGGAGGCAAGAAAAACGGGCGGATTCT
GACCTTGCCTCGGTCCAATCCTTCCTGGCCCGGGATCCACCGGTCGCCACCGTGAGC
AAGGGCGAGGAGAATAATATGGCCATCATCAAGGAGTTCATGCGCTTCAAGGTGCGCA
TGGAGGGCACCGTGAACGGCCACGAGTTCGAGATCGAGGGCGAGGGCGAGGGCCGC
CCCTACGAGGGCTTTCAGACCGCTAAGCTGAAGGTGACCAAGGGCGGCCCCCTGCCCT
TCGCCTGGGACATCCTGTCCCCTCTCTTCACCTACGGCTCCAAGGCCTACGTGAAGCA
CCCCGCCGACATCCCCGACTACTTCAAGCTGTCCTTCCCCGAGGGCTTCAAGTGGGAG
CGCGTGATGAACTACGAGGACGGCGGCGTGGTGACCGTGACCCAGGACTCCTCACTG
CAGGACGGCGAGTTCATCTACAAGGTGAAGATGCGCGGCACCAACTTCCCCTCCGACG
GCCCCGTGATGCAGAAGAAGACCATGGGCTGGGAGGCCTCCTCCGAGCGGATGTACC
CCGAGGACGGCGCCCTGAAGGGCGAGATCAGGATGAGGCTGAAGCTGAAGGACGGCG
GCCACTACACCTCCGAGGTCAAGACCACCTACAAGGCCAAGAAGTCCGTGCAGCTGCC
CGGCGCCTACATCGTCGGCATCAAGCTGGACATCACCTCCCACAACGAGGACTACACC
ATCGTGGAACAGTACGAACGCGCCGAGGGCCGCCACTCCACCGGCGGCATGGACGAG
CTGTACAAGTAAAGCGGCCGCGACTCTAGATCATAATCAGCACATGAGGATCACCCATG
TCTGCAGGTCGACTCTAGAAAACATGAGGATCACCCATGT (SEQ ID NO:211)
Protein: (X indicates non-canonical amino acid)

MGTGGRRGAAAAPLLVAVAALLLGAAGHLYPGEVCPGMDIRNNLTRLHELENCSVIEGHLQI
LLMFKTRPEDFRDLSFPKLIMITDYLLLFRVYGLESLKDLFPNLTVIRGSRLFFNYALVIFEMVH

LKELGLYNLMNITRGSVRIEKNNELCYLATIDWSRILDSVEDNYIVLNKDDNEECGDICPGTA
KGKTNCPATVINGQFVERCWTHSHCQKVCPTICKSHGCTAEGLCCHSECLGNCSQPDDPT
KCVACRNFYLDGRCVETCPPPYYHFQDWRCVNFSFCQDLHHKCKNSRRQGCHQYVIHNN
KCIPECPSGYTMNSSNLLCTPCLGPCPKVCHLLEGEKTIDSVTSAQELRGCTVINGSLIINIRG
GNNLAAELEANLGLIEEISGYLKIRRSYALVSLSFFRKLRLIRGETLEIGNYSFYALDNQNLRQ
LWDWSKHNLTITQGKLFFHYNPKLCLSEIHKMEEVSGTKGRQERNDIALKTNGDQASCENE
LLKFSYIRTSFDKILLRWEPYWPPDFRDLLGFMLFYKEAPYQNVTEFDGQDACGSNSWTVV
DIDPPLRSNDPKSQNHPGWLMRGLKPWTQYAIFVKTLVTFSDERRTYGAKSDIIYVQTDATN
PSVPLDPISVSNSSSQIILKWKPPSDPNGNITHYLVFWERQAEDSELFELDYCLXGLKLPSRT
WSPPFESEDSQKHNQSEYEDSAGECCSCPKTDSQILKELEESSFRKTFEDYLHNVVFVPRP
SRKRRSLGDVGNVTVAVPTVAAFPNTSSTSVPTSPEEHRPFEKVVNKESLVISGLRHFTGY
RIELQACNQDTPEERCSVAAYVSARTMPEAKADDIVGPVTHEIFENNVVHLMWQEPKEPNG
LIVLYEVSYRRYGDEELHLCVSRKHFALERGCRLRGLSPGNYSVRIRATSLAGNGSWTEPT
YFYVTDYLDVPSNIAKIIIGPLIFVFLFSVVIGSIYLFLRKRQPDGPLGPLYASSNPEYLSASDVF
PCSVYVPDEWEVSREKITLLRELGQGSFGMVYEGNARDIIKGEAETRVAVKTVNESASLRE
RIEFLNEASVMKGFTCHHVVRLLGVVSKGQPTLVVMELMAHGDLKSYLRSLRPEAENNPGR
PPPTLQEMIQMAAEIADGMAYLNAKKFVHRDLAARNCMVAHDFTVKIGDFGMTRDIYETDYY
RKGGKGLLPVRWMAPESLKDGVFTTSSDMWSFGVVLWEITSLAEQPYQGLSNEQVLKFVM
DGGYLDQPDNCPERVTDLMRMCWQFNPNMRPTFLEIVNLLKDDLHPSFPEVSFFHSEENK
APESEELEMEFEDMENVPLDRSSHCQREEAGGRDGGSSLGFKRSYEEHIPYTHMNGGKK
NGRILTLPRSNPSWARDPPVATVSKGEENNMAIIKEFMRFKVRMEGTVNGHEFEIEGEGEG
RPYEGFQTAKLKVTKGGPLPFAWDILSPLFTYGSKAYVKHPADIPDYFKLSFPEGFKWERVM
NYEDGGVVTVTQDSSLQDGEFIYKVKMRGTNFPSDGPVMQKKTMGWEASSERMYPEDGA
LKGEIRMRLKLKDGGHYTSEVKTTYKAKKSVQLPGAYIVGIKLDITSHNEDYTIVEQYERAEG
RHSTGGMDELYK (SEQ ID NO:212)

INSR^{676TAG}-iRFP-MS2
DNA: (MS2 stem-loops and Amber codon underlined) 
ATGGGCACCGGGGGCCGGCGGGGGCGGCGGCCGCGCCGCTGCTGGTGGCGGTGG
CCGCGCTGCTACTGGGCGCCGCGGGCCACCTGTACCCCGGAGAGGTGTGTCCCGGCA
TGGATATCCGGAACAACCTCACTAGGTTGCATGAGCTGGAGAATTGCTCTGTCATCGAA
GGACACTTGCAGATACTCTTGATGTTCAAAACGAGGCCCGAAGATTTCCGAGACCTCAG
TTTCCCCAAACTCATCATGATCACTGATTACTTGCTGCTCTTCCGGGTCTATGGGCTCGA
GAGCCTGAAGGACCTGTTCCCCAACCTCACGGTCATCCGGGGATCACGACTGTTCTTTA
ACTACGCGCTGGTCATCTTCGAGATGGTTCACCTCAAGGAACTCGGCCTCTACAACCTG
ATGAACATCACCCGGGGTTCTGTCCGCATCGAGAAGAACAATGAGCTCTGTTACTTGGC
 
CACTATCGACTGGTCCCGTATCCTGGATTCCGTGGAGGATAATTACATCGTGTTGAACA
AAGATGACAACGAGGAGTGTGGAGACATCTGTCCGGGTACCGCGAAGGGCAAGACCAA
CTGCCCCGCCACCGTCATCAACGGGCAGTTTGTCGAACGATGTTGGACTCATAGTCACT
GCCAGAAAGTTTGCCCGACCATCTGTAAGTCACACGGCTGCACCGCCGAAGGCCTCTG
TTGCCACAGCGAGTGCCTGGGCAACTGTTCTCAGCCCGACGACCCCACCAAGTGCGTG
GCCTGCCGCAACTTCTACCTGGATGGCAGGTGTGTGGAGACCTGCCCGCCCCCGTACT
ACCACTTCCAGGACTGGCGCTGTGTGAACTTCAGCTTCTGCCAGGACCTGCACCACAAA
TGCAAGAACTCGCGGAGGCAGGGCTGCCACCAGTACGTCATTCACAACAACAAGTGCA
TCCCTGAGTGTCCCTCCGGGTACACGATGAATTCCAGCAACTTGCTGTGCACCCCATGC
CTGGGTCCCTGTCCCAAGGTGTGCCACCTCCTAGAAGGCGAGAAGACCATCGACTCGG
TGACGTCTGCCCAGGAGCTCCGAGGATGCACCGTCATCAACGGGAGTCTGATCATCAA
CATTCGAGGAGGCAACAATCTGGCAGCTGAGCTAGAAGCCAACCTCGGCCTCATTGAA
GAAATTTCAGGGTATCTAAAAATCCGCCGATCCTACGCTCTGGTGTCACTTTCCTTCTTC
CGGAAGTTACGTCTGATTCGAGGAGAGACCTTGGAAATTGGGAACTACTCCTTCTATGC
CTTGGACAACCAGAACCTAAGGCAGCTCTGGGACTGGAGCAAACACAACCTCACCATC
ACTCAGGGGAAACTCTTCTTCCACTATAACCCCAAACTCTGCTTGTCAGAAATCCACAAG
ATGGAAGAAGTTTCAGGAACCAAGGGGCGCCAGGAGAGAAACGACATTGCCCTGAAGA
CCAATGGGGACCAGGCATCCTGTGAAAATGAGTTACTTAAATTTTCTTACATTCGGACAT
CTTTTGACAAGATCTTGCTGAGATGGGAGCCGTACTGGCCCCCCGACTTCCGAGACCT
CTTGGGGTTCATGCTGTTCTACAAAGAGGCCCCTTATCAGAATGTGACGGAGTTCGACG
GGCAGGATGCATGTGGTTCCAACAGTTGGACGGTGGTAGACATTGACCCACCCCTGAG
GTCCAACGACCCCAAATCACAGAACCACCCAGGGTGGCTGATGCGGGGTCTCAAGCCC
TGGACCCAGTATGCCATCTTTGTGAAGACCCTGGTCACCTTTTCGGATGAACGCCGGAC
CTATGGGGCCAAGAGTGACATCATTTATGTCCAGACAGATGCCACCAACCCCTCTGTGC
CCCTGGATCCAATCTCAGTGTCTAACTCATCATCCCAGATTATTCTGAAGTGGAAACCAC
CCTCCGACCCCAATGGCAACATCACCCACTACCTGGTTTTCTGGGAGAGGCAGGCGGA
AGACAGTGAGCTGTTCGAGCTGGATTATTGCCTCTAGGGGCTGAAGCTGCCCTCGAGG
ACCTGGTCTCCACCATTCGAGTCTGAAGATTCTCAGAAGCACAACCAGAGTGAGTATGA
GGATTCGGCCGGCGAATGCTGCTCCTGTCCAAAGACAGACTCTCAGATCCTGAAGGAG
CTGGAGGAGTCCTCGTTTAGGAAGACGTTTGAGGATTACCTGCACAACGTGGTTTTCGT
CCCCAGGCCATCTCGGAAACGCAGGTCCCTTGGCGATGTTGGGAATGTGACGGTGGCC
GTGCCCACGGTGGCAGCTTTCCCCAACACTTCCTCGACCAGCGTGCCCACGAGTCCGG
AGGAGCACAGGCCTTTTGAGAAGGTGGTGAACAAGGAGTCGCTGGTCATCTCCGGCTT
GCGACACTTCACGGGCTATCGCATCGAGCTGCAGGCTTGCAACCAGGACACCCCTGAG
GAACGGTGCAGTGTGGCAGCCTACGTCAGTGCGAGGACCATGCCTGAAGCCAAGGCT
GATGACATTGTTGGCCCTGTGACGCATGAAATCTTTGAGAACAACGTCGTCCACTTGAT
 
GTGGCAGGAGCCGAAGGAGCCCAATGGTCTGATCGTGCTGTATGAAGTGAGTTATCGG
CGATATGGTGATGAGGAGCTGCATCTCTGCGTCTCCCGCAAGCACTTCGCTCTGGAAC
GGGGCTGCAGGCTGCGTGGGCTGTCACCGGGGAACTACAGCGTGCGAATCCGGGCCA
CCTCCCTTGCGGGCAACGGCTCTTGGACGGAACCCACCTATTTCTACGTGACAGACTAT
TTAGACGTCCCGTCAAATATTGCAAAAATTATCATCGGCCCCCTCATCTTTGTCTTTCTCT
TCAGTGTTGTGATTGGAAGTATTTATCTATTCCTGAGAAAGAGGCAGCCAGATGGGCCG
CTGGGACCGCTTTACGCTTCTTCAAACCCTGAGTATCTCAGTGCCAGTGATGTGTTTCC
ATGCTCTGTGTACGTGCCGGACGAGTGGGAGGTGTCTCGAGAGAAGATCACCCTCCTT
CGAGAGCTGGGGCAGGGCTCCTTCGGCATGGTGTATGAGGGCAATGCCAGGGACATC
ATCAAGGGTGAGGCAGAGACCCGCGTGGCGGTGAAGACGGTCAACGAGTCAGCCAGT
CTCCGAGAGCGGATTGAGTTCCTCAATGAGGCCTCGGTCATGAAGGGCTTCACCTGCC
ATCACGTGGTGCGCCTCCTGGGAGTGGTGTCCAAGGGCCAGCCCACGCTGGTGGTGA
TGGAGCTGATGGCTCACGGAGACCTGAAGAGCTACCTCCGTTCTCTGCGGCCAGAGGC
TGAGAATAATCCTGGCCGCCCTCCCCCTACCCTTCAAGAGATGATTCAGATGGCGGCA
GAGATTGCTGACGGGATGGCCTACCTGAACGCCAAGAAGTTTGTGCATCGGGACCTGG
CAGCGAGAAACTGCATGGTCGCCCATGATTTTACTGTCAAAATTGGAGACTTTGGAATG
ACCAGAGACATCTATGAAACGGATTACTACCGGAAAGGGGGCAAGGGTCTGCTCCCTG
TACGGTGGATGGCACCGGAGTCCCTGAAGGATGGGGTCTTCACCACTTCTTCTGACAT
GTGGTCCTTTGGCGTGGTCCTTTGGGAAATCACCAGCTTGGCAGAACAGCCTTACCAAG
GCCTGTCTAATGAACAGGTGTTGAAATTTGTCATGGATGGAGGGTATCTGGATCAACCC
GACAACTGTCCAGAGAGAGTCACTGACCTCATGCGCATGTGCTGGCAATTCAACCCCAA
CATGAGGCCAACCTTCCTGGAGATTGTCAACCTGCTCAAGGACGACCTGCACCCCAGC
TTTCCAGAGGTGTCGTTCTTCCACAGCGAGGAGAACAAGGCTCCCGAGAGTGAGGAGC
TGGAGATGGAGTTTGAGGACATGGAGAATGTGCCCCTGGACCGTTCCTCGCACTGTCA
GAGGGAGGAGGCGGGGGCCGGGATGGAGGGTCCTCGCTGGGTTTCAAGCGGAGCT
ACGAGGAACACATCCCTTACACACACATGAACGGAGGCAAGAAAAACGGGCGGATTCT
GACCTTGCCTCGGTCCAATCCTTCCTGGGCCCGGGATCCACCGGTCGCCACCGCGGAA
GGATCCGTCGCCAGGCAGCCTGACCTCTTGACCTGCGACGATGAGCCGATCCATATCC
CCGGTGCCATCCAACCGCATGGACTGCTGCTCGCCCTCGCCGCCGACATGACGATCGT
TGCCGGCAGCGACAACCTTCCCGAACTCACCGGACTGGCGATCGGCGCCCTGATCGG
CCGCTCTGCGGCCGATGTCTTCGACTCGGAGACGCACAACCGTCTGACGATCGCCTTG
GCCGAGCCCGGGGCGGCCGTCGGAGCACCGATCACTGTCGGCTTCACGATGCGAAAG
GACGCAGGCTTCATCGGCTCCTGGCATCGCCATGATCAGCTCATCTTCCTCGAGCTCG
AGCCTCCCCAGCGGGACGTCGCCGAGCCGCAGGCGTTCTTCCGCCGCACCAACAGCG
CCATCCGCCGCCTGCAGGCCGCCGAAACCTTGGAAAGCGCCTGCGCCGCCGCGGCGC
AAGAGGTGCGGAAGATTACCGGCTTCGATCGGGTGATGATCTATCGCTTCGCCTCCGA
 
CTTCAGCGGCGAAGTGATCGCAGAGGATCGGTGCGCCGAGGTCGAGTCAAAACTAGG
CCTGCACTATCCTGCCTCAACCGTGCCGGCGCAGGCCCGTCGGCTCTATACCATCAAC
CCGGTACGGATCATTCCCGATATCAATTATCGGCCGGTGCCGGTCACCCCAGACCTCA
ATCCGGTCACCGGGCGGCCGATTGATCTTAGCTTCGCCATCCTGCGCAGCGTCTCGCC
CGTCCATCTGGAATTCATGCGCAACATAGGCATGCACGGCACGATGTCGATCTCGATTT
TGCGCGGCGAGCGACTGTGGGGATTGATCGTTTGCCATCACCGAACGCCGTACTACGT
CGATCTCGATGGCCGCCAAGCCTGCGAGCTAGTCGCCCAGGTTCTGGCCTGGCAGATC
GGCGTGATGGAAGAGTAAGCGGCCGCGACTCTAGATCATAATCAGCACATGAGGATCA
CCCATGTCTGCAGGTCGACTCTAGAAAACATGAGGATCACCCATGT (SEQ ID NO:213)
Protein: (X indicates non-canonical amino acid)

MGTGGRRGAAAAPLLVAVAALLLGAAGHLYPGEVCPGMDIRNNLTRLHELENCSVIEGHLQI
LLMFKTRPEDFRDLSFPKLIMITDYLLLFRVYGLESLKDLFPNLTVIRGSRLFFNYALVIFEMVH
LKELGLYNLMNITRGSVRIEKNNELCYLATIDWSRILDSVEDNYIVLNKDDNEECGDICPGTA
KGKTNCPATVINGQFVERCWTHSHCQKVCPTICKSHGCTAEGLCCHSECLGNCSQPDDPT
KCVACRNFYLDGRCVETCPPPYYHFQDWRCVNFSFCQDLHHKCKNSRRQGCHQYVIHNN
KCIPECPSGYTMNSSNLLCTPCLGPCPKVCHLLEGEKTIDSVTSAQELRGCTVINGSLIINIRG
GNNLAAELEANLGLIEEISGYLKIRRSYALVSLSFFRKLRLIRGETLEIGNYSFYALDNQNLRQ
LWDWSKHNLTITQGKLFFHYNPKLCLSEIHKMEEVSGTKGRQERNDIALKTNGDQASCENE
LLKFSYIRTSFDKILLRWEPYWPPDFRDLLGFMLFYKEAPYQNVTEFDGQDACGSNSWTVV
DIDPPLRSNDPKSQNHPGWLMRGLKPWTQYAIFVKTLVTFSDERRTYGAKSDIIYVQTDATN
PSVPLDPISVSNSSSQIILKWKPPSDPNGNITHYLVFWERQAEDSELFELDYCLXGLKLPSRT
WSPPFESEDSQKHNQSEYEDSAGECCSCPKTDSQILKELEESSFRKTFEDYLHNVVFVPRP
SRKRRSLGDVGNVTVAVPTVAAFPNTSSTSVPTSPEEHRPFEKVVNKESLVISGLRHFTGY
RIELQACNQDTPEERCSVAAYVSARTMPEAKADDIVGPVTHEIFENNVVHLMWQEPKEPNG
LIVLYEVSYRRYGDEELHLCVSRKHFALERGCRLRGLSPGNYSVRIRATSLAGNGSWTEPT
YFYVTDYLDVPSNIAKIIIGPLIFVFLFSVVIGSIYLFLRKRQPDGPLGPLYASSNPEYLSASDVF
PCSVYVPDEWEVSREKITLLRELGQGSFGMVYEGNARDIIKGEAETRVAVKTVNESASLRE
RIEFLNEASVMKGFTCHHVVRLLGVVSKGQPTLVVMELMAHGDLKSYLRSLRPEAENNPGR
PPPTLQEMIQMAAEIADGMAYLNAKKFVHRDLAARNCMVAHDFTVKIGDFGMTRDIYETDYY
RKGGKGLLPVRWMAPESLKDGVFTTSSDMWSFGVVLWEITSLAEQPYQGLSNEQVLKFVM
DGGYLDQPDNCPERVTDLMRMCWQFNPNMRPTFLEIVNLLKDDLHPSFPEVSFFHSEENK
APESEELEMEFEDMENVPLDRSSHCQREEAGGRDGGSSLGFKRSYEEHIPYTHMNGGKK
NGRILTLPRSNPSWARDPPVATAEGSVARQPDLLTCDDEPIHIPGAIQPHGLLLALAADMTIV
AGSDNLPELTGLAIGALIGRSAADVFDSETHNRLTIALAEPGAAVGAPITVGFTMRKDAGFIG
SWHRHDQLIFLELEPPQRDVAEPQAFFRRTNSAIRRLQAAETLESACAAAAQEVRKITGFDR
VMIYRFASDFSGEVIAEDRCAEVESKLGLHYPASTVPAQARRLYTINPVRIIPDINYRPVPVTP

DLNPVTGRPIDLSFAILRSVSPVHLEFMRNIGMHGTMSISILRGERLWGLIVCHHRTPYYVDL
DGRQACELVAQVLAWQIGVMEE (SEQ ID NO:214)

## Claims

1. An assembler fusion protein (AFP) comprising:
(a) at least one first polypeptide segment acting as assembler (AP) that is selected from:
(a1) a polypeptide segment derived from an intracellular targeting polypeptide (IC-TP segment), wherein said intracellular targeting polypeptide targets, and thus becomes locally enriched at, an intracellular structural element within or directly adjacent to the cytoplasm; and
(a2) a polypeptide segment derived from a phase separation polypeptide (PSP segment), wherein said phase separation polypeptide has the ability to undergo self-association in the cytoplasm of a cell so as to create sites of high local concentration in the cytoplasm, and
(b) at least one second polypeptide segment acting as an effector (EP) that is selected from:
b1) an RNA-targeting polypeptide (RNA-TP) segment, and
b2) an orthogonal aminoacyl tRNA synthetase (O-RS) segment;
wherein said polypeptide segments are functionally linked in said AFP.

2. An assembler fusion protein (AFP) combination comprising at least two AFPs of claim 1.

3. A fusion protein (RNA-TP/O-RS fusion protein) comprising:
(i) at least one RNA-targeting polypeptide (RNA-TP) segment; and
(ii) at least one orthogonal aminoacyl tRNA synthetase (O-RS) segment,
wherein said polypeptide segments are functionally linked in said RNA-TP/O-RS fusion protein.

4. A nucleic acid molecule, or a combination of two or more nucleic acid molecules, comprising:
(i) a nucleotide sequence that encodes at least one AFP of claim 1, or at least one AFP combination of claim 2, or
(ii) a nucleic acid sequence complementary to the nucleotide sequence of (i).
(iii) both of (i) and (ii).

5. A nucleic acid molecule, or a combination of two or more nucleic acid molecules, comprising:
(i) a nucleotide sequence that encodes at least one RNA-TP/O-RS fusion protein of claim 3, or
(ii) a nucleic acid sequence complementary to (i), or
(iii) both of (i) and (ii).

6. An expression cassette comprising the nucleotide sequence of the nucleic acid molecule, or the combination of nucleic acid molecules, of claim 4 or claim 5.

7. An expression vector comprising at least one expression cassette of claim 6.

8. A cell comprising at least one nucleic acid molecule, or combination of nucleic acid molecules, of claim 4 or claim 5, at least one expression cassette of claim 6, or at least one expression vector of claim 7.

9. The cell of claim 8 comprising a nucleotide sequence that encodes, or is complementary to a nucleotide sequence encoding, at least one AFP of claim 1 comprising (i) at least one EP selected from RNA-TP segments and (ii) at least one EP selected from O-RS segments.

10. The cell of claim 8 comprising a nucleotide sequence that encodes, or is complementary to a nucleotide sequence encoding, a combination of at least two AFPs of claim 1, wherein one of the at least two AFPs comprises at least one RNA-TP segment and another one of the at least two AFPs comprises at least one O-RS segment.

11. The cell of claim 8 comprising a nucleotide sequence that encodes, or is complementary to a nucleotide sequence encoding at least one RNA-TP/O-RS fusion protein of claim 3.

12. A method for preparing a polypeptide of interest (POI) comprising in its amino acid sequence one or more non-canonical amino acid (ncAA) residues, wherein the method comprises expressing the POI in a cell of claim 9 or claim 10 in the presence of said one or more ncAAs, wherein the cell comprises:
(i) a POI-encoding nucleotide sequence (CS^{POI}) wherein said one or more ncAA residues of the POI are encoded by selector codon(s),
(ii) a targeting nucleotide sequence (TN) that is functionally linked to the CS^{POI} and is able to interact with an RNA-TP segment of at least one of the AFPs in the cell;
(iii) one or more orthogonal tRNA^{ncAA} (O-tRNA^{ncAA}) molecules which carry the antico-don(s) complementary to the selector codon(s) of the CS^{POI}, and wherein said O-tRNA^{ncAA} molecules together with one or more O-RS segments of at least one of the AFPs in the cell form one or more orthogonal O-RS/O-tRNA^{ncAA} pairs which allow for the introduction of said one or more ncAA residues into the amino acid sequence of the POI;
and wherein the method optionally further comprises recovering the expressed POI.

13. A method for preparing a polypeptide of interest (POI) comprising in its amino acid sequence one or more non-canonical amino acid (ncAA) residues, wherein the method comprises expressing the POI in a cell of claim 11 in the presence of said one or more ncAAs, wherein the cell comprises:
(iv) a POI-encoding nucleotide sequence (CS^{POI}) wherein said one or more ncAA residues of the POI are encoded by selector codon(s),
(v) a targeting nucleotide sequence (TN) that is functionally linked to the CS^{POI} and is able to interact with an RNA-TP segment of at least one of the RNA-TP/O-RS fusion proteins in the cell;
(vi) one or more orthogonal tRNA^{ncAA} (O-tRNA^{ncAA}) molecules which carry the antico-don(s) complementary to the selector codon(s) of the CS^{POI}, and wherein said O-tRNA^{ncAA} molecules together with one or more O-RS segments of the RNA-TP/O-RS fusion proteins in the cell form one or more orthogonal O-RS/O-tRNA^{ncAA} pairs which allow for the introduction of said one or more ncAA residues into the amino acid sequence of the POI;
and wherein the method optionally further comprises recovering the expressed POI.

14. A nucleic acid molecule comprising:
(i) a nucleotide sequence (CS^{POI}) that encodes a polypeptide of interest (POI), said POI comprising one or more non-canonical amino acid (ncAA) residues which are encoded in the CS^{POI} by selector codons, and
(ii) a targeting nucleotide sequence (TN), wherein an RNA molecule comprising said TN is able to interact via said TN with an RNA-targeting polypeptide (RNA-TP).

15. A kit for preparing a polypeptide of interest (POI) having at least one non-canonical amino acid (ncAA) residue, the kit comprising:
- at least one ncAA, or salt thereof, corresponding to the at least one ncAA residue of the POI, and
- at least one expression vector of claim 7.
